(19) Europäisches Patentamt / European Patent Office / Office européen des brevets

(11) EP 2 162 145 B1

(12) **EUROPEAN PATENT SPECIFICATION**

(45) Date of publication and mention of the grant of the patent:
**04.05.2016 Bulletin 2016/18**

(21) Application number: **08750724.0**

(22) Date of filing: **29.05.2008**

(51) Int Cl.:
***A61K 36/58*** (2006.01) ***A61K 31/366*** (2006.01)
***A61P 15/10*** (2006.01) ***C07D 493/22*** (2006.01)

(86) International application number:
**PCT/GB2008/001825**

(87) International publication number:
**WO 2008/145996 (04.12.2008 Gazette 2008/49)**

(54) **NOVEL COMPOUNDS AND PHARMACEUTICAL PREPARATIONS FROM NEOBEGUEA SPEC**

NEUE VERBINDUNGEN UND PHARMAZEUTISCHE ZUBEREITUNGEN AUS NEOBEGUEA ARTEN

NOUVEAUX COMPOSÉS ET NOUVELLES PRÉPARATIONS PHARMACEUTIQUES PROVENANT DES ESPÈCES NEOBEGUEA

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **29.05.2007 SE 0701283**
**06.08.2007 SE 0701821**
**12.11.2007 SE 0702472**

(43) Date of publication of application:
**17.03.2010 Bulletin 2010/11**

(73) Proprietor: **Dicotyledon AB**
**193 35 Sigtuna (SE)**

(72) Inventors:
- **WIKBERG, Jarl, Erik, Sylvester**
  **S-193 35 Sigtuna (SE)**
- **RASOANAIVO, Philippe**
  **101- Antananarivo (MG)**
- **BENOIT, Rasolondratovo**
  **Tolaria (601) (MG)**
- **RAZAFIMAHEFA, Andriantiaray, Solofoniaina**
  **Antananarivo 101 (MG)**

(74) Representative: **Webber, Philip Michael et al**
**Dehns**
**St Bride's House**
**10 Salisbury Square**
**London**
**EC4Y 8JD (GB)**

(56) References cited:
- **PARIS R R ET AL: "ON THE POLY PHENOLS PHENOLIC ACIDS FLAVONOIDS IN LEAVES OF 2 MELIACEAE SPECIES OF MADAGASCAR CEDRELOPSIS-GREVEI AND NEOBEGUEA-MAHAFALENSIS" PLANTES MEDICINALES ET PHYTOTHERAPIE, ANGERS, FR, vol. 6, no. 4, 1 January 1972 (1972-01-01), pages 311-319, XP009120341 ISSN: 0032-0994 cited in the application**
- **DATABASE WPI Week 200555 Thomson Scientific, London, GB; AN 2005-537835 XP002538721 & JP 2005 213202 A (MARUHA CORP) 11 August 2005 (2005-08-11) cited in the application**
- **COOMBES PHILIP H ET AL: "Phragmalin limonoids from the Madagascan Meliaceae Neobeguea leandreana." JOURNAL OF NATURAL PRODUCTS JUN 2003, vol. 66, no. 6, June 2003 (2003-06), pages 735-738, XP002538717 ISSN: 0163-3864**
- **NAIDOO D ET AL: "Limonoids and triterpenoids from the seed of Neobeguea mahafalensis." BIOCHEMICAL SYSTEMATICS AND ECOLOGY, vol. 31, no. 9, September 2003 (2003-09), pages 1047-1050, XP002538718 ISSN: 0305-1978 cited in the application**
- **RANDRIANARIVELOJOSIA M ET AL: "A limonoid from Neobeguea mahafalensis" PHYTOCHEMISTRY, PERGAMON PRESS, GB, vol. 52, no. 6, 1 November 1999 (1999-11-01), pages 1141-1143, XP004291127 ISSN: 0031-9422 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MULHOLLAND D A ET AL: "LIMONOID EXTRACTIVES FROM THE GENERA CAPURONIANTHUS NEOBEGUEA AND QUIVISIANTHE" PHYTOCHEMISTRY (OXFORD), vol. 27, no. 6, 1988, pages 1741-1744, XP002538719 ISSN: 0031-9422 cited in the application**
- **LIAO, SHANG-GAO ET AL: "Plant orthoesters" CHEMICAL REVIEWS (WASHINGTON, DC, UNITED STATES) , 109(3), 1092-1140 CODEN: CHREAY; ISSN: 0009-2665, 2009, XP002538720**
- **KOTSOS ET AL: 'EXTRACTIVES FROM NEOBEGUEA MAHAFALENSIS AND CEDRELOPSIS GREVEI (THESIS)', [Online] 01 January 1997, page 213PP, XP009124534 Retrieved from the Internet: <URL:http://hdl.handle.net/10413/278> [retrieved on 2009-10-22]**

## Description

## Background Art

Extracts and pharmaceuticals from *Neobeguea mahafalensis* root tissue, the procedures for their preparation, and their use for eliciting sexual enhancing effect and for treatment of sexual dysfunction, in particular erectile dysfunction and hypoactive desire disorders are disclosed. The structures of the chemical compounds in these extracts causing sexual enhancing effect, the preparation of these compounds and the pharmaceuticals prepared from them are disclosed.

The invention thus relates to sexual function and the normal sexual response cycle may be divided into four phases. a) The desire phase, which consists typically of fantasies about and the desire to have sexual activity. b) The excitement phase, which is characterized by the subjective sense of sexual pleasure and accompanying physiological changes, namely penile tumescence and erection in men; and pelvic congestion, swelling of the external genitalia, and vaginal lubrication and expansion in women, c) The orgasmic phase, where sexual pleasure peaks with the release of sexual tension and rhythmic contraction of the perineal muscles and reproductive organs. In men, the sensation of ejaculatory inevitability is followed by the ejaculation of semen. In woman, contractions of the outer third of the vaginal wall occur. d) The final phase, resolution, which is characterized by a sense of muscular relaxation and general well-being. Men are physiologically refractory to erection and orgasm for a variable period, whereas women may be able to respond to further stimulation (1) (numbers within parenthesis refer throughout this patent to references given in the reference list, i.e. listed under "REFERENCES"). Disorders of the sexual response can occur at one or more of these phases a-d (1), and are common among the populations.

In men disorders of sexual function (i.e., sexual disorders or sexual dysfunction) include erectile dysfunctions, ejaculatory dysfunctions and hypoactive sexual desire disorders.

Erectile dysfunction in men is defined as the inability to obtain and/or maintain penile erection sufficient for satisfactory sexual performance (2). Reasons for erectile dysfunctions are many and include (but are not limited to) psychological factors, endocrine factors, neurogenic factors (e.g. neural trauma, spinal cord injury), vascular factors, age factors (being common at old age), disease conditions (e.g. hypertension, diabetes) and external factors such as passive and active smoking. Moreover, it is seen as a side effect upon treatment with many drugs (e.g. antidepressants, antipsychotics and certain antihypertensives) and surgery (e.g., prostatectomy) (see also references 1-6).

Erectile dysfunction is commonly also referred to as impotence, and for the sake of the present invention erectile dysfunction and impotence is *mutatis mutandis* intended to mean the same thing.

Ejaculatory dysfunctions comprise premature ejaculation, retarded ejaculation, retrograde ejaculation, anejaculation, aspermia, haemospermia, low volume ejaculate, painful ejaculation and anhedonia (i.e., lack of pleasure) (see reference 7).

Hypoactive sexual desire disorders (HSDD) in men are often mistaken for erectile dysfunction. However, HSDD is defined as the persistent or recurrent absence or deficit of sexual fantasies and desire for sexual activity. It may be caused by psychological, psychiatric and endocrine factors (see reference 8).

Disorders of sexual function in women (also referred to as sexual disorder or sexual dysfunction) are also common and are in fact an underestimated problem. However, there is an increased awareness of the existence of sexual dysfunction in woman. It has been considered to comprise four major components: decreased desire to have sex (also known as HSDD in women); decreased arousal (with respect to e.g. blood flow to the genitals and lubrication); pain during intercourse; and difficulty to have orgasms (9). In particular HSDD in women has attracted interest. The latter has also been labeled "Women's Sexual Interest/Desire Disorder" (10) and is defined as the "persistent or recurring lack of or diminished feelings of sexual interest or desire, absent sexual thoughts or fantasies and/or desire for or receptivity to sexual activity" (11). Motivations for attempting to become sexually aroused could be scarce or absent (10,11).

Irrespectively of the type of sexual dysfunction, in man or woman, it can be very disabling for the patient as well as it may seriously affect the relation with his/her partner, or even the ability to attract a partner. It is therefore desired to find means to treat these conditions.

Various regimes are available for treatment of sexual dysfunction in men, and include agents that act as vasodilators on erectile tissue (e.g., papaverine, $\alpha$-adrenoceptor blocking agents, prostaglandins, organic nitrates, minoxidil, potassium channel openers, phosphodiesterase inhibitors) and drugs that are supposedly acting centrally in the brain or spinal cord such as yohimbine, opioid receptor antagonists, dopamine receptor agonists, antidepressants and melanocortin receptor agonists (5).

For women some drugs have also been tried to ameliorate sexual dysfunction, as well as such drugs are in development, and include testosterone, melanocortin receptor agonists, serotonin receptor agonists and antagonists, and others (9).

To assess the severity of sexual dysfunction, and the effect of treatments, various approaches are in use, e.g. direct measurement of penile erection strength (e.g., nocturnal tumescence and rigidity values) and frequency of erection,

e.g. using devices such as RigiScan (Timm Medical Technologies, Eden, Prairie, MN, USA; see reference 31).

However, another widely used approach to assess severity of sexual dysfunction is self-assessment using self-report techniques. This approach is sometimes considered more satisfactory than direct measurement of penile erection strength in men, or at least giving complementary information (31). For men the "International Index of Erectile Function" (IIEF) was developed (13). It can assess five modalities of sexual function: erectile function, orgasmic function, sexual desire, intercourse satisfaction, and overall satisfaction (12). A reduced set of IIEF, called IIEF-5, is in wide use for assessment of erectile dysfunction (13). For women similar self-reporting approaches are also in use (14).

Plants and materials of animal origin have been used since long time for supposed aphrodisiac effects (15) as well as for treatment of various medical conditions. However, remedies of this type are mostly poorly defined. Thus, while e.g. plants have been used in traditional medicines for very long times the exact effect of a particular plant is not known unless the plant has been properly examined and documented by use of scientific methods. There are many reasons for this. One is that as a rule traditionally plant medicines utilize mixtures derived from different plants and it is therefore not possible to ascribe a claimed effect of a traditional remedy to a specific plant. Another is that effect of plants or mixtures of plants that are used traditionally lacks, in the general case, proper scientific documentation. Instead effects are exaggerated, ascribed magic proportions, and described wrongly. The uses of the plants are as a rule mixed up with different ill-defined conditions, or simply told without any grounded fact in reality. Yet another reason is that the preparation of the plant is not known (i.e., kept secret) and it can't therefore be ascertained in what way the plant was prepared, which portion of it was used and if a particular preparation of a plant provided any useful activity or not. In addition dosages and dosing schedules are as a rule not known.

A large number of plants in Madagascar have been mentioned in the literature to affect sexual functions. For example Madagascan species mentioned to be aphrodisiacs are *Croton crocodilorium, Vanilla madagascariensis, Phyllanthus bojerianus, Carissa edulis, Pandaca retusa, Thylachium sumangui, Stadmannia glauca, Phyllanthus decaryanus, Xylopia danguyella, Canavalia ensiformis, Lissochilus madagascariensis, Phyllanthus erythroxyloides, Cedrelopsis grevei, Cynosorchis purpurascens, Oldelandia lancifolia, Thylachium angustifolium, Thylachium panduriforme, Oldenlandia caffra, Exacum quinquenervium, Thylacium seyrigii, Pandaca callosa, Hypoestes sp., Woodfordia fruticosa, Carapa moluccensis, Crataeva greveana, Fagaropsis velutina, Vepris elliotti, Pandaca debrayi, Psiadia altissima, Mollugo sp., Cabucala eryhtrocarpa, Potameia ovata, Muntafara sessilifolia, Cabucala striolata*, *Boscia longifolia, Evodia belahe, Uvaria manjensis, Pandaca mocquerysii, Potameia eglandulosa, Cabucala sp., Pluchea grevei, Maerua filiformis, Vernonia poissonii, Habenaria elliotii, Boscia plantefolii, Thylachium laburnoides, Boscia madagascariensis, Phyllanthus seyrigii*, *Cynosorchis graminea, Amorphophallus hildebranditii, Exacum emirnense, Vernonia leucolepis, Cartopodium plantagineum, Xylocarpus moluccensis, Exacum sp., Tachiadenus carinatus, Satyrium perrieri, Rhynchosia sp., Danais sp., Vanilla decaryana, Capparis sepiaria, Solanum erythrocarpum, Lagenaria siceraria, Solanum indicum, Solanum macrocarpon*, *Solanum erythracanthum, Cyperus alboviridis, Stenocline incana, Hedvchium coronarium, Hedychium peregrinum, Mucuna pruriens, Pandanus utilis, Fluggea microcarpa, Persea americana, Arachis hypogaea, Zingiber officinale, Rhipsalis cassytha, Brachylaena ramiflora, Tribulus terrestris, Neobeguea mahafaliensis, Turraea sericae, Mystroxylon aegypticum*, *Thylachium monophyllum*, *Tina striata, Psychotria sp., Potameia thouarsii, Potanzeia thouarsiana*, *Maerua nuda, Cabucala cryptophlebia, Cyperus esculentus*, *Lawsonia alba, Lycopoditern cernuum*, *Oxalis sessilis, Nicandra physaloides, Persea gratissima*, *Rhinacanthus osmospermus, Solanum macrocarpum, Ficus baroni, Pandaca crassifolia, Ceiba pentandra, Torenia sp., Thymus vulgaris, Cyperus articulatus, Apium graveolens, Mentha piperita, Vanilla planifolia, Byttneria voulily, Vernonia grandis, Vernonia scariosa, Samadera madagascariensis*, *Pinus pinaster*, *Arundo madagascariensis, Woodfordia floribunda, Catharanthus roseus, Argyreia speciosa, Asparagus racemosus*, *Cananga odorata, Mucuna urens, Pandanus edulis*, *and Nuxia sphaerocarpa* (list compiled by Professor Philippe Rasonaivo based on literature data; 16).

It seems highly unlikely that such a large number of plants as in the list above would all in reality be capable of providing aphrodisiac effects. In fact, a plant can't be used based on such type of claims as above for any practical treatment purpose. This is due to the following:

Firstly, in practically all instances the source of information has been from traditional healers or local persons, which as a rule use mixtures of plants for treatment of various ill-specified conditions. Thus, there is here a great risk of mixing up the actions of plants claimed by the healer or local person.

Secondly, the information on the action of a plant was as a rule based on vernacular names told by a traditional healer or local person - not based on identification from exact botanical field studies. This gives a great risk of confusion of which species is intended. For example, the vernacular name 'Hazomena', which means red tree, is used among the Madagascan population to describe species that have red stem barks, namely *Nesogordonia normandy, Khaya madagascarensis, Securinega perrieri, Securinega capuroni, Erythroxylum nitidum*, *Stadnzannia grevei, Tina gelonium*, *Ochnella madagascariensis*, *Symphonic microphylla*, *Phyllanthus decaryanus, Weinmannia bojeriana, Securinega capuronii, Weinmannia rutenbergii*, *Weinmannia sp., Carphalea geayi, Cordyla madagas-*

*cariensis*, *Neobeguea mahafaliensis, Securinega seyrigii, Phyllantus rhontboidalis* and *Homalium sp.* (16).

Thirdly, actions of plants are ascribed according to the physical appearance of the plant by local healers. This includes, for example, shape and taste. Thus, e.g., if a plant has a bitter taste it is ascribed to have antimalarial (17) or aphrodisiac effects among Madagascan healers (18). However, bitter taste is a common property among plants, and bears of course no relation to any beneficial effect on any particular medical condition.

Fourthly, the medical condition for use of a plant is usually not defined in the publicly available sources in such a way that it is known in a true medical sense for what disease or condition the plant should be applied.

Thus, reports on presumed medical effects of plants based on indirect information obtained from local traditional healers and alike is highly unreliable and can't be used in any practical sense for treatment of medical conditions.

As earlier literature reports *vis-a-vis* usefulness of Madagascan plants were deemed useless in the search for principles useful for treatment of sexual dysfunction we instead elected to enter into a screening programme where plants were carefully botanically identified, parts of them collected, drugs and extracts properly prepared and tested on animals, and subsequently these materials were evaluated on human subjects which were properly medically examined for sexual function before and after the treatments. In these attempts we found surprisingly strong and long-lasting effects of extracts of the root from the species *Neobeguea mahafalensis* (*Neobeguea mahafaliensis*) on sexual functions in the male rat and male mouse in vivo. Prompted by these exciting results we made clinical trials where pharmaceutical preparations of extracts from the root of *Neobeguea mahafalensis* were given to male patients with sexual dysfunction of the type erectile dysfunction and/or hypoactive sexual desire disorder. In these trials surprisingly strong and long-lasting beneficial actions on sexual ability were noted in previously sexually incapacitated patients.

Although these surprising strong and long-lasting effects could not have been anticipated from prior art we retrospectively investigated the literature on uses of plants that might be referred to *Neobeguea mahafalensis.* Upon these investigations we found only fragmentary notes. However, it must be stated that as the information was based on vernacular names it can't with certainty be stated that the plant referred to in the literature is indeed *Neobeguea mahafalensis.* Nevertheless, the information available based on indirect information told by local healers or local population, is as follows:

Debray wrote '*Neobeguea mahafalensis* Leroy .... Le décocté d'écorce, très amèr, est utilise en ingestion pour combattre les douleurs rhumatismales et serai aphrodiasaque'. (English translation: 'The decoction of the stem bark, which is very bitter, is used in oral administration to treat rheumatic pains, and might be aphrodisiac') (19, page 21).

Paris and Debray wrote regarding *Neobeguea mahafalensis* Leroy: 'Le decocte d'ecorces, très amer, est repute antirhumatismal et aphrodisiaque' (English translation: The infusion of stem bark, very bitter, is said to be antirheumatic and aphrodisiac) (20, page 316).

Centre de Formation Professionnelle Forestiere ' FOFAMPIALA' de Morondava wrote regarding the traditional uses of *Neobeguea mahafaliensis*:'- Tonificant; contre les etats de fatigue generate et les maux de dos: l'écorce de cet arbre est seche et râpée; on la laisse ensuite macerer quelques heures dans de l'eau froide, puis on boit la solution ainsi obtenue. - Pour la construction des meubles'. (English translation: The infusion of scrapped stem barks is used as tonic, as well as for the treatment of general tiredness and back pains) (21).

Ralantonirina wrote: 'Handy ou NEOBEGUEA MAHAFALENSIS STS Leroy (Meliacées). Ecorces .... (UTILISATION SUR PLACE): Asthenie sexuelle.

Douleur et ballonnement abominal. Epigastralgie. Gastrite Lombalgiqie. Rhumatisme, Psycho-stimulant.' (English translation: Stem bark .... (UTILISATION ON THE SITE): Sexual asthenia, abdominal pains, epigastralgia, gastritis, lumbago, rheumatism. Psychostimulant) (22, table on page 58).

Ralantonirina further more wrote regarding traditional uses of *Neobeguea mahafaliensis:* 'Les ecorces son utilisées pour traiter: lástenié sexuelle, la douleur et le ballonement abdominal, l'épigatralgie, la fastrite, la lombalgie, le rhumatisme, Elles son psycho-stimulants. (English translation: The stem bark is used to treat sexual asthenia, abdominal pains, epigastralgia, gastritis, lumbago, rheumatism. They are psychostimulants) (22).

Direction des Eaux et Forêts, Ministère de l'Agriculture et du Développement Rural wrote in a review covering traditional uses of 200 species, among these stems of *Neobeguea mahafaliensis*: 'Bois utilisé pour la menuiserie, l'ébénisterie, le tournage et même pour lest parquets mosaic de luxe, les revêtements d'interieur, les pieux des cases et les pare à bouefs, Ecorce contenant des principes aphrodisiaques. Utilisée pour traiter les maladies de reins, les douleurs rhumatismale et lombaire liées à la blennorragie chronique' (23, page 381). (English translation: Wood used in furniture, carvings, floor plank, fencing for houses and zebu parks. Stem bark containing aphrodisiac principles. Used to treat kidney diseases, rheumatoid and lumbar pains linked to chronic blennorragia).

Boiteau wrote regarding ethnomedical uses of *Neobeguea mahafaliensis:* 'Ecorce très amèr; sa decoction est administrée contre les douleurs lombaires surtout chez les homes atteints de blennorragie chronique' (English translation: 'Very bitter stem bark; its decoction is administered against lumbar pains, especially in the men who have chronic gonorrhea') (24).

Boiteau further wrote 'HANDY (tandr.). *Neobeguea mahafalensis* J.F. Leroy (Méliacées). Décocté d'écorce très amer utlilisé en boisson pour combattre les douleours rhumatismales, notamment les douleurs de la colonne vertébrale. Passe pour restaurer aussi les functions genitalez chez les vieillards d' après Debray*). Mais il faut tenir compte du fait que tout ce qui amer passe dans la symbolique malagache pour exalter les qualites viriles.' (English translation: 'Decoction of stem bark very bitter used to treat rheumatic pains, especially pains of the spinal column. Claimed to restore genital functions in the old men (according to Debray). However, it should be kept in mind that bitter principles are claimed in the Malagasy culture to exalt virile functions.') (24).

In interpreting these citations comprising the scanty and fragmentary information available on traditional uses of *Neobeguea mahafalensis* it shall be noted that all the studies were based either on interviews with no certification of botanical identity, or on indirect citations from the studies based on these interviews. In interpreting them it should also be noted that the vernacular name 'Handy' refers to both *Neobeguea mahafalensis* or *Neobeguea sp.* (16), thus meaning that the identity of'Handy' is uncertain. It shall further be noted that local healers administer remedies based on 'Handy' together with several other plants such as Cedrelopsis grevei, Vanilla madagascariensis, Tamarindus indica (22, page 40). Accordingly it is not ascertained that a particular effect is tied with a particular plant. Furthermore, the bitter taste of *Neobeguea mahafalensis* extracts is a reason that it might exalt virile functions according to local beliefs.

Accordingly, in view of the above the cultural use of plant medicines in Madagascar, the studies cited above could not have led anyone skilled in the art of plant ethnomedicine (or arts related thereto) that *Neobeguea mahafalensis* possesses any particularly useful properties *vis-à-vis* treatment of sexual dysfunctions (*c.f.* the list above on the large number of Madagascan plants claimed to be aphrodisiacs). (One may compare this with the sayings, well known among the western populations, that oysters, chocolate, rhino-horn and egg yolk possesses sexually stimulating effect. Still it is clear to anyone that these sayings are of no real medical practical use).

Additionally Liao et al. discloses "Plant orthoesters" (Chemical Reviews (Washington DC. USA) 109(3), pages 1092-1140); and a PhD thesis by Kotsos (1997; XP009124534; http://hdl.handle.net110413/278) discloses "Extractives from Neobeguea mahafalensis and Cedrelopsis grevei".

On top of all this the investigations preceding the present disclosure failed to demonstrate any appreciable activity of preparation obtained from the stem bark of *Neobeguea mahafalensis* on sexual behaviour activity in animal test system, whereas preparations from the root of *Neobeguea mahafalensis* showed remarkable high activity in this respect. Accordingly these findings makes it evident that previous fragmentary notes regarding uses of stem bark of *Neobegarea mahafalensis* were based on beliefs and loose sayings rather than on grounded facts in reality.

Thus, investigations of the present invention found surprisingly that extracts prepared from roots of *Neobeguea mahafalensis* elicit strong sexual enhancing effect, and the present invention provides therefore the root of *Neobeguea mahafaliensis* and materials derived therefrom for use in treatment of sexual dysfunction.

Otherwise extracts of *Neobeguea mahafalensis* have been described (20,28) as well as various compounds have been isolated from them (29,38,39), albeit with unknown effects on humans and animals. Extracts of *Neobeguea mahafalensis* have been used for preparation of lotions, creams and skin-makeup (and alike) for use in cosmetics as anti-ageing principle, anti-oxidant principle, antiradical principle, collagenase inhibitory action and anti-inflammatory principle for skin treatment (28).

Based on the review above of the fragmentary and uncertain documentation on *Neobeguea mahafalensis* it can thus not be held that anyone skilled in the art would have known prior to the disclosure of the present invention that preparations from *Neobeguea mahafalensis,* in particular preparations from its root, possesses extraordinary long-lasting and dramatic effects on sexual dysfunctions in humans, as well as similar strong long-lasting effects in animal *in vivo* models of sexual function. Moreover, the specific processes for preparation of the extracts eliciting such effects, as well as the specific pharmaceutical preparations of *Neobeguea mahafalensis* for systemic use to be used for efficient administration, or the treatment schedules to properly treat sexual dysfunctions and conditions related thereto based on pharmaceutical preparations of *Neobeguea mahafalensis* were not known prior to the disclosure of the present invention.

Moreover, prior art did not describe the chemical substances of *Neobeguea mahafalensis* that causes sexual enhancing effect and which are useful for treatment of sexual dysfunction.

The present invention thus provides novel chemical substances, which among other disclosed uses, can be used for treatment of sexual dysfunction, and procedures for obtaining these chemical substances.

Also disclosed is the use of drugs, extracts, components, compounds, substantially pure compounds, chemical substances and pharmaceuticals for treatment of sexual dysfunction, in particular erectile dysfunction and hypoactive sexual desire disorders.

Further disclosed are extracts, drugs, components, compounds, substantially pure compounds, chemical substances and pharmaceuticals derived from *Neobeguea mahafcalensis,* and procedures for their preparation, which are

in particular useful for systemic use and/or for the treatment of sexual dysfunction, in particular erectile dysfunction and hypoactive sexual desire disorders in men and woman.

Further disclosed are procedures for collecting *Neobeguea mahafalensis,* as well as procedures for cultivating *Neobegatea mahafalensis,* for use in the preparation of extracts, drugs, components, compounds, substantially pure compounds, chemical structures and pharmaceuticals which are in particular intended for systemic use and/or treatment of sexual dysfunction, in particular erectile dysfunction and hypoactive sexual desire disorders (HSDD).

Also disclosed are treatment schedules for treating sexual dysfunction, in particular erectile dysfunction and hypoactive sexual desire disorders, based on the use of extracts, drugs, components, pro-components, compounds, pro-compounds, substantially pure compounds, chemical substances and pharmaceuticals bearing a relation with *Neobeguea mahafalensis.*

Further disclosed are procedures for assaying the sexual activity enhancing activity of extracts, drugs, components, pro-components, compounds, pro-compounds, substantially pure compounds, chemical structures and pharmaceuticals bearing a relation with *Neobeguea mahafalensis,* procedures which are useful to judge the suitability of extracts, drugs, components, compounds, substantially pure compounds, chemical structures and pharmaceuticals bearing a relation with *Neobeguea mahafalensis* for the treatment of sexual dysfunction, in particular erectile dysfunction and hypoactive sexual desire disorders, as well as said procedures finds use to determine the proper dosages and treatment schedules for extracts, drugs, components, compounds, substantially pure compounds, chemical substances and pharmaceuticals bearing a relation with *Neobeguea mahafalensis* when they are to be given to humans or animals in the treatment of sexual dysfunction, in particular erectile dysfunction and hypoactive sexual desire disorders.

Further disclosed are characteristic mass-peaks, which can be used in conjunction with mass-spectrometry to determine whether or not extracts, drugs, components, pro-components, compounds, pro-compounds, substantially pure compounds, chemical substance and pharmaceuticals derived or prepared from *Neobeguea mahafalensis,* or prepared by other means, are part of the invention. Also disclosed are procedures for isolation of components and compounds from *Neobeguea mahafalensis* which are surprisingly potent enhancers of sexual activity with a surprisingly long duration of action, and the uses of these components and compounds for preparation of pharmaceuticals and the treatment of sexual dysfunction, as well as for other uses.

Also disclosed are structures of chemical substances bearing a relation with *Neobeguea mahafalensis* which are surprisingly potent enhancers of sexual activity with a surprisingly long duration of action, and the uses of these chemical substances for preparation of pharmaceuticals and the treatment of sexual dysfunction, as well as for other uses.

Extract: By 'extract' is herein defined a material obtained from *Neobeguea mahafalensis,* or a part of *Neobeguea mahafalensis,* or the drug of the invention (as defined herein below) by extraction with solvent, or obtained in a liquid form by other means (e.g., by applying pressure to part(s) of *Neobeguea mahafalensis* using a mechanical device, e.g. a screw press, or by using other means, such as steam distillation). In a further sense by extract is defined a material or residue obtained by removal of the solvent or liquid of the extract obtained as said in the foregoing sentence (e.g., by using evaporation, freeze drying, spray drying, or by using other means of removing the solvent or liquid). By 'extract' is also in a further sense defined a material processed further from the extract obtained by any one of the above processes for obtaining a further extract, e.g. by applying further solvent extraction(s) and/or treatment(s) with chemicals and/or applying fractionation(s) and/or applying other operation(s) or procedure(s).

**Drug:** By 'drug' is herein defined a raw material obtained from *Neobeguea mahafalensis.* A drug in this sense may constitute the plant in whole or in part. The raw material may be obtained by manipulation of *Neobeguea mahafalensis,* such as cutting, crushing, grinding, sieving, drying, freezing, thawing, extraction, modification, derivatization, treatment with chemicals, treatment with solvents, and alike, the thus so prepared *Neobeguea mahafalensis* comprising the drug of the present invention. In another sense by 'drug' is also intended the extract or pharmaceutical obtained or prepared with *Neobeguea mahafalensis* as a starting material, as is evident from the context term 'drug' is applied herein.

**Plant part or Part:** By'plant part' or 'part' is herein defined a portion of *Neobeguea mahafalensis* that may be selected from (but not limited to) root, stem bark, flower, fruit, leaf, stem, trunk or branch, with root and stem bark being preferred, and with root being most preferred. Wherever reference is made to 'plant' herein *Neobeguea mahafalensis* is intended. Wherever reference is made to 'plant part' herein a part of *Neobeguea mahafalensis* is intended. Wherever reference is made to 'part of plant' herein a part of *Neobeguea mahafalensis* is intended.

**Compound:** By'compound' or 'compound of the invention' is herein in one meaning defined a chemical compound obtained from or being obtainable from *Neobeguea mahafalensis.* However, in another meaning by 'compound' or 'compound of the invention' is *mutatis mutandis* intended the same thing as 'chemical substance of the invention'.

**Substantially pure compound:** By'substantially pure compound' is herein intended a single compound obtained from or prepared from *Neobeguea ntahafalensis* that is by weight at least 10%, more preferable at least 20%, even more preferable at least 30%, even more preferable at least 40%, even more preferable at least 50%, even more preferable at least 60%, even more preferable at least 70%, even more preferable at least 80%, even more preferable at least 90%, even more preferable at least 95%, even more preferable at least 98%, and most preferably at least 99% pure. (E.g. by 99 % pure means in this context that the single compound obtained from or being obtainable from

*Neobeguea mahafalensis* exists in a form where it takes at least 99% of the weight, while the remainder comprises other material(s)). By'substantially pure compound' is herein in an alternative mode intended a single compound obtained from or prepared from *Neobeguea mahafalensis* that can be determined by use of chromatographic separation in conjunction with UV absorbance detection at 210 nm to be at least 10%, more preferable at least 20%, even more preferable at least 30%, even more preferable at least 40%, even more preferable at least 50%, even more preferable at least 60%, even more preferable at least 70%, even more preferable at least 80%, even more preferable at least 90%, even more preferable at least 95%, even more preferable at least 98%, and most preferably at least 99% pure. (E.g. by 99 % pure means in this mode that the single compound obtained from or being obtainable from *Neobeguea mahafalensis* exists in a form where it takes at least 99% of UV absorbance at 210 nm (disregarding the solvent's UV absorbance), while the remainder of the UV absorbance at 210 nm (disregarding the solvent's UV absorbance) arises from other material(s) as determined by chromatography together with UV absorbance detection).

**Structure of the invention:** By 'structure of the invention' is herein defined as a compound as claimed herein. For all cases herein the stereochemistry of chemical structures are disregarded and a structure of the invention can confine (have) any one and all possible stereoconfigurations of the chemical structures given herein. In some embodiments of the invention the chemical structure of neobeguin (CAS registry number 260794-07-8) and/or the chemical structure with CAS registry number 98379-63-6 and/or the chemical structure of pseudrelone $A_2$ (compound 3 of reference 39) and/or leandreanin A (CAS registry number 561307-81-1) and/or leandreanin B (CAS registry number 561307-82-2) and/or leandreanin C (CAS registry number 561307-83-3) and/or 2-hydroxy-6-deoxyswietine (compound 1 of reference 39) and/or 2-hydroxy-6-deoxyswietonolide tiglate (compound 2 of reference 39) is specifically excluded from being a structure of the invention.

**Chemical substance of the invention:** By 'chemical substance' or 'chemical substance of the invention' is herein intended a chemical substance or chemical compound having the structure of the invention.

**Heavy atom:** By 'heavy atom' is herein defined an atom which chemical atomic weight is at least 6.9, i.e. Lithium and any and all atoms heavier than Lithium. Preferred heavy atoms are nitrogen, oxygen, sulphur, phosphorous and halogen.

**Halogen:** By'halogen' or'halogen atom' is herein intended fluorine, chlorine, bromine or iodine atom.

**Chemical group:** By 'chemical group' is herein referred two or more atoms connected together by covalent chemical bond(s) so as to form a group that can be attached to atom of other molecule by single, double or triple bond, the chemical group preferably containing between 1 to 60 atoms, more preferably between 1 to 40 atoms, even more preferably between 1 to 30 atoms even more preferably between 1 to 25 atoms, more preferably between 1 to 20 atoms, and most preferably between 1 to 16 atoms, the chemical group comprising a linear, branched and/or cyclic structure.

**Sexual enhancing effect:** By 'sexual enhancing effect' or 'sexual enhancing activity' is herein defined the effect of a material, extract, substance, compound etc. ('Material') that can be observed after administration to male mice as an increase in sexual activity when the male mice are introduced to sexual receptive female mice. One can then observe the sexual enhancing effect as an increase in the number of mounts of the male mouse performed over a definite period of time (i.e. 1, 2, 3 or 4 hours) following the introduction of the sexual receptive female to the male. In order for a Material to be classified to having a sexual enhancing effect the number of mounts of the mice shall be increased at least 50 % compared to control mice, i.e. compared to mice that were not administered the Material, but otherwise treated in the same way. The percent increase in mounts is calculated as follows: Let the average number of mounts for the control mice during the elected test period be X, and the average number of mounts for the treated mice during the same length test period be Y, then the percent increase in mounts is 100 x (Y-X)/X. (For example, if the control mice show on the average X=18 mounts during the first hour after introduction to the female, and the treated mice show on the average Y=31 mounts during the first hour after their introduction to the female, then the percent increase in the number of mounts is 100 x (31-18)/18 = 72.22 %; the Material hence showing a sexual enhancing effect). It is moreover preferred that a test is repeated using at least 3 different couples in each group of control and treated animals, and moreover and that the effect is statistically significant, at least $p<0.05$, when assessed using an appropriate statistical method (unpaired two tailed t-test being preferred) in order for it to be classified as a sexual enhancing effect. Any suited dose of Material can be given to the males; it is preferred though that a treatment group is given the same dose per mass of the males' bodyweight. The Material can be administered either orally or subcutaneously. The Material is preferably administered to each male mouse once each day for three consecutive days, whereafter on the fourth day a female mouse is introduced to each male mouse. It is preferred that the number of mounts is counted during the first hour after introduction of the female mouse. As an alternative rats can be used instead of mice. In this case the sexual enhancing effect can be observed as an increase in the number of active couples or as an increase in the copulatory efficiency. It is then preferred that either one of number of active couples or increase in the copulatory efficiency is increased at least 50 % compared to controls, in order for the effect to be classified as being a sexual enhancing effect. To assess whether or not a Material has a sexual enhancing effect mice are preferred over rats. In the most specific sense a sexual enhancing activity of an extract, fraction, component, compound or substance is being present when the systemic administration of said extract, fraction, component, compound or substance to male mice is giving an at

least 50 % increase in the number of mounts being observable during a period of one hour following the introduction of the male mice to sexually receptive female mice, when compared to controls where the male mice were untreated.

**Sexual enhancing activity:** By 'sexual enhancing activity' is *mutatis mutandis* intended the same thing as 'sexual enhancing effect'.

**Long-lasting effect:** By 'long-lasting effect' is herein intended a treatment effect that is seen after an initial period of administration of an agent (i.e. during a period when the administration of said agent is entirely discontinued), where said treatment effect is being observable preferably at least three days, more preferably at least one week, event more preferably at least two weeks, even more preferable at least one month after the discontinuation of the administration of the agent. In a further sense by'long-lasting effect' is in this context intended that the long-lasting effect is directed towards amelioration or cure of a sexual dysfunction, preferably erectile dysfunction and/or impotence and/or ejaculatory dysfunction and/or hypoactive sexual desire disorder, HSDD. In another sense by'long-lasting effect' is intended that the long-lasting effect is directed to a sexual enhancing effect.

**Water solution:** By 'water solution' is intended water in which water soluble material(s) has been solubilised. The materials solubilised include (but are not limited to) salts, buffer materials, buffer constituents, acids, bases, organic compounds and solvents.

**Hydrophilic solvent:** By 'hydrophilic solvent' is intended water, water solution or organic solvent that (preferably at room temperature, more preferably at a temperature between 10 to 30°C, more preferably at a temperature between 20 to 25°C and most preferably at a temperature of 20°C) is in any and all proportions entirely miscible with distilled water (i.e. phase separation is not forming). Examples of hydrophilic solvents (but not limited to) are glycerol, methanol, ethanol, acetone, glycol, acetonitrile, N-methylpyrrolidone. Preferred hydrophilic solvents are water, methanol, ethanol, acetonitrile, propionitrile, propanol, propan-1-ol, propan-2-ol, dimethyl sulfoxide, formamide, dimethylformamide, acetone, tetrahydrofurane, glycol, glycerol, dioxane, 1,4-dioxane, formic acid, acetic acid, propionic acid, butyric acid, 2-methylpropanoic acid, 3-oxobutanamide, N,N-diethylacetamide, N,N-diethyl acetoacetamide, propylene glycol, methylsulfonylmethane, ethanol amine, *tert*-butyl alcohol, diethylene glycol, 1,2-dimethoxy-ethane, ethylene glycol, hexamethylphosphoramide, hexamethylphosphorous triamide, pyridine, 2-methyltetrahydrofuran, 3-methyltetrahydropyran, 2-methylpyridine, 1,3-propanediol, sulfolane, triethylene glycol, tetraethylene glycol, tetrahydrofurfuryl alcohol, triethanolamine, triethyl phosphate, triethylene glycol, triethylene glycol dimethyl ether, triethylene glycol monomethyl ether, N-methylpyrrolidone, liquid carbon dioxide, and mixtures thereof.

**Lipophilic solvent:** By 'lipophilic solvent' is intended organic solvent that (preferably at room temperature, more preferably at a temperature between 10 to 30°C, more preferably at a temperature between 20 to 25°C, and most preferably at a temperature of 20°C) is not at all, or only in some but not all proportions, miscible with distilled water (i.e. phase separations can be seen in at least some proportions). Examples of lipophilic solvents (but not limited to) are chloroform, methylene chloride (dichloromethane), benzene, toluene, carbon tetrachloride, tricholoethylene, petroleum ether, ethylacetate, hexane, octan-1-ol. Preferred lipophilic solvents are pentane, n-pentane, 2-methylbutane, 2,2-dimethylpropane, hexane, n-hexane, 2-methylpentane, 3-methylpentane, 2,3-dimethylbutane, 2,2-dimethylbutane, heptane, n-heptane, 2-methylhexane, 3-methylhexane, 2,2-dimethylpentane, 2,3-dimethylpentane, 2,4-dimethylpentane, 3,3-dimethylpentane, 3-ethylpentane, 2,2,3-trimethylbutane, octane, n-octane, 2-methylheptane, 3-methylheptane, 4-methylheptane, 3-ethylhexane, 2,2-dimethylhexane, 2,3-dimethylhexane, 2,4-dimethylhexane, 2,5-dimethylhexane, 3,3-dimethylhexane, 3,4-dimethylhexane, 2-methyl-3-ethylpentane, 3-methyl-3-ethylpentane, 2,2,3-trimethylpentane, 2,2,4-trimethylpentane, isooctane, 2,3,3-trimethylpentane, 2,3,4-trimethylpentane, 2,2,3,3-tetramethylbutane, cyclohexane, benzonitrile, chlorobenzene, diethyl ether, methyl-tert-butyl ether, methylenechloride, dichloromethane, chloroform, carbon tetrachloride, 1,2-dichloroethane, perchloroethylene, trichloroethylene, 1,1,1-trichloroethane, trichloroethene, perchloroethylene, tetrachloroethene, vinylchloride, ethylacetate, methylethylketone, propyl acetate, iso-propyl acetate, butyl lactate, n-butyl lactate, iso-butyl lactate, tert-butyl lactate, sec-butyl lactate, butyl acetate, n-butyl acetate, iso-butyl acetate, tert-butyl acetate, sec-butyl acetate, triacetin, 1,2,3-triacetoxypropane, diacetin, glycerol 1,3-diacetate, glycerol 1,2-diacetate, benzene, toluene, xylene, o-xylene, m-xylene, p-xylene, 2,2,4-trimethylpentane, butanone, 2-butanone, pentan-3-one, pentan-2-one, 3-pentanone, 2-pentanone, cyclopentanone, butan-1-ol, butan-2-ol, n-butanol, 1-butanol, sec-butanol, 2-butanol, isobutanol, 2-methyl-1-propanol, 2-methyl-2-propanol, pentanol, 1-pentanol, 3-methyl-1-butanol, 2-methyl-1-butanol, 2,2-dimethyl-1-propanol, 3-pentanol, 2-pentanol, 3-methyl-2-butanol, 3-methyl-2-butanol, 2-methyl-2-butanol, hexanol, heptanol, octanol, monohydroxylated hydrocarbon(s), diethylene glycol, dimethyl sulfoxide, methyl t-butyl ether, N-methyl-2-pyrrolidinone, nitromethane, tetrahydrofuran, triethyl amine, benzylalcohol, carboxylic acid methyl ester, carboxylic acid ethyl ester, fatty acid methyl ester, vegetable oil, animal oil, triglyceride(s), mineral oil, wood turpentine, petroleum ether, naphtha, hydrocarbon solvent, chlorinated hydrocarbon solvent, fluorinated hydrocarbon solvent, halogenated hydrocarbon solvent, freon, 1-bromo-3-chloropropane, [2-(2-butoxyethoxy)ethyl]acetate, 2-butoxyethyl acetate, cumene, cyclohexanol, cyclohexanone, decahydronaphthalene, n-decane, dibenzyl ether, 1,2-dichlorobenzene, 1,3-dichlorobenzene, 1,4-dichlorobenzene, 1,2-dichloroethane, dimethyl carbonate, diethylene glycol dibutyl ether, diethylene glycol diethyl ether, diethylene glycol mono-n-hexyl ether, diethylene glycol monobutyl ether, diethylene glycol monoethyl ether, diethylene glycol monomethyl ether, ethylbenzene, ethyl formate, 2-ethyl-1-hexanol, dimethyl

sulfoxide, 1,1,1,3,3,3-hexafluoro-2-propanol, isoamyl acetate, 2-methylbutyl acetate, 3-methylbutyl acetate, isoamyl butyrate, isobutyl acetate, isopropyl acetate, isopropyl methyl ketone, 1,3,5-trimethylbenzene, (1-methoxy-2-propyl)acetate, methyl acetate, methylcyclohexane, methylcyclohexanol, 5-methyl-3-heptanone, 3-methyltetrahydropyran, 2-methylpentane, 2-methyl-1-butanol, *n*-nonane, nitroethane, propylacetate, 1,2-propylene glycol diacetate, propylene carbonate, tetrahydrofuran, tetrahydrofurfuryl alcohol, 1,2,3,4-tetrahydronaphthalene, triethylene glycol, dimethyl sulphoxide, dimethyl ether, methyl ethyl ether, liquid ethane, liquid ethene, liquid propane, liquid propylene, liquid *n*-butane, liquid *iso*-butane, liquid carbon dioxide, liquid trifluoromethane, liquid chlorotrifluoromethane, liquid trichlorofluoromethane, liquid ammonia and mixtures thereof.

**Hydrophobic solvent:** By 'hydrophobic solvent' is *mutatis mutandis* intended the same as 'lipophilic solvent'.

**Pro-component:** A 'pro-component', is defined as a component that upon systemic administration to an animal or human (i.e. upon systemic use) by virtue of the metabolism of said animal or human is converted to a component obtained from or being obtainable from *Neobeguea mahafalensis.* In a further sense by pro-component is intended that the pro-component upon systemic administration in addition also increases sexual activity in the animal or human. Pro-components may be obtained from *Neobeguea mahafalensis* or they may be manufactured in other ways, e.g. from another species or by chemical synthesis. A pro-component can be used for the same purposes as described herein for a component derived from *Neobeguea mahafalensis.*

**Pro-compound:** A'pro-compound', is defined as a compound that upon systemic administration to an animal or human (i.e. upon systemic use) by virtue of the metabolism of said animal or human is converted to a compound obtained from or being obtainable from *Neobeguea mahafalensis* and/or to a chemical substance of the invention. In a further sense by pro-compound is intended that the pro-compound upon systemic administration in addition also increases sexual activity in the animal or human, or causes a sexual enhancing effect. A pro-compound may be obtained from *Neobeguea mahafalensis* or it may be manufactured in other systems or by other means, e.g. by isolation from another species or by chemical synthesis. A pro-compound can be used for the same purposes as described herein for a (pure or substantially pure) compound derived from *Neobeguea mahafalensis* and/or the chemical substance of the invention.
**Prodrug:** By term prodrug is defined as a compound that when administered to a living organism is converted to a chemical substance of the invention.

**Root:** By term 'root' is intended the root of *Neobeguea mahafalensis,* i.e. the portions of the *Neobeguea rnahafalensis* extending from the bottom of the trunk and residing (growing) below ground.

**Stem bark:** By term 'stem bark' is intended the stem bark of *Neobeguea mahafalensis,* i.e. the bark of the trunk of *Neobeguea mahafalensis;* in other words the bark of the *Neobeguea mahafaliensis* obtained from the portions of *Neobeguea mahafalensis* residing (growing) above ground.

**CAS registry number:** By'CAS registry number' is intended a unique number referring to a substance in the CAS REGISTRY database of CAS of the American Chemical Society, the Chemical Abstracts Service, 2540 Olentangy River Road, Columbus, OH 43202, USA.

**Neobeguea mahafalensis and Neobeguea mahafaliensis:** By terms 'Neobeguea mahafalensis' and 'Neobeguea mahafaliensis' is mutatis mutandis intended to mean the same plant.

**Neobeguea leandriana and Neobeguea leandreana:** By terms Neobeguea leandriana' and 'Neobeguea leandreana' is mutatis mutandis intended to mean the same plant.

**Sexual disorder and sexual dysfunction:** Terms 'sexual disorder' and 'sexual dysfunction' are in this patent *mutatis mutandis* herein referring to the same thing.

**Food supplement:** Term 'food supplement' is herein intended to mean a material derived from or being obtainable from *Neobeguea mahafaliensis* which is taken in addition to normal diet.

**Dietary supplement:** Term 'dietary supplement' is herein defined a material derived from or being obtainable from *Neobeguea mahafaliensis* that can serve as a dietary supplement as defined under the Dietary Supplement Health and Education Act of 1994, public Law 103-417 103rd Congress, US Food and Drug Administration, USA.

The species *Neobeguea mahafalensis* of the family Meliaceae (i.e. belonging to the Mahogany family) of the genus Neobeguea was described by Leroy (25, 26). It is a tree, where specimens identified in the field are described as follows:

Specimen 1 data: Tree c. 7 m; trunk 15 cm diameter; bark grey, smooth, cambium beige; leaves compound alternate; leaflets dark green above, pale green below; fruit terminal, 3-4 (rounded) angled, golden brown with beige speckling; seeds winged, dehiscing to leave 3-4 angled woody, central columella; in freshly cut fruit, central columella pink red, wings held erect; fruit c. 3-4 cm by 4 cm.

Specimen 2 data: Common spreading tree to 10 m; bark falling in pieces (Platanus-like); young leaves dark red, laminae then axes becoming green; many flowers galled; sepals and petals pale green; staminal tube pale dull pink, shading to orange at base; anthers pale yellow, style and stigma pale green.

Specimen 3 data: Sparingly branched tree to 7 m. DBH 25 cm. Bark silvery-grey with broad shallow furrows and ridges, red pustulate, minutely flakey. Fruits abundant, brown with grey speckles, globular, leaving three angled stalk and three winged seeds at maturity.

Specimen 4 data: Tree 6 m high, DBH: 24 cm. Fruit hard, spheroid. Leaves blue-green below. Bark fine, falling off in patches to give mottled appearance.

Specimen 5 data: Small tree 6-7 m tall. Leaves green above, light grey-green below. Leaf axis tinged magenta. Sepals and petals greenish-rose white, anthers yellow-green.

Vernacular names for *Neobeguea mahafalensis* are as follows: Andipasy (Mahafaly), Andy (Sakalava, Mahafaly), Bemahova (Sakalava), Handy (Tandroy), Hazolava (Sakalava, Mahafaly, Tandroy), Fipy, Handibohitsy, Hazomena.

Referring to Figure 1 parts of *Neobeguea mahafalensis* are shown schematically. Shown at 101 is a branch with fruit; at 102 an inflorescence; at 103 and 104 flowers after regression of the perianth; at 105 petal; at 106 and 107 anther; at 108 anther; at 109 schematic cross-section of pistil; at 110 immature fruit; at 111 adult fruit; at 112 flower bud in its place; at 113 down.

For the sake of the present invention whenever name *Neobeguea mahafalensis* is used it is *mutatis mutandis* intended to mean the same thing as *Neobeguea mahafaliensis.* Species *Neobeguea mahafaliensis* (*Neobeguea mahafalensis*) was described by J.F. Leroy [see Compt. Rend. Acad. Sci. Paris ccxlvi 2640 (1958), Journ. Agric. & Bot. Appliq. v 504 (1958), and references 25 and 26].

For the sake of the present invention by *Neobeguea mahafalensis* is also intended a 'part' or 'plant part' of *Neobeguea mahafalensis.*

When a reference herein is made to *Neobeguea mahafalensis,* also species closely related to *Neobeguea mahafalensis* (*Neobeguea mahafaliensis*) are, in some embodiments of the invention, also intended namely *Neobeguea leandriana, Neobeguea leandreana, Neobeguea ankaranensis* and *Neobeguea sp.,* with *Neobeguea mahafalensis* being preferred.

For reasons given below in this patent further below in all embodiments of the present invention whenever reference is made to *Neobeguea mahafalensis,* the *Neobeguea mahafalensis* can, if so desired, be substituted for anyone of *Neobeguea mahafaliensis, Neobeguea leandriana, Neobeguea leandreana, Neobeguea ankaranensis* and *Neobeguea sp.*

For the sake of the present invention whenever name *Neobeguea leandriana* is used it is *mutatis mutandis* meaning the same thing as *Neobeguea leandreana.* Species *Neobeguea ankaranensis* was described by J.-F. Leroy [see Comp. Rend. Acad. Sci. Paris ccxlvi. 2640 (1958) and Journ. Agric. & Bot. Appliq. v 504 (1958)].

Species *Neobeguea leandriana* was described by J.F. Leroy [see Adansonia 16(2): 172 (1976)].

For the sake of the present invention whenever term *Neobeguea sp.* or *Neobeguea spp.* is used is intended any one of *Neobeguea mahafalensis, Neobeguea leandreana, Neobeguea ankaranensis*.

For the sake of the present invention the plant or parts of it can be collected in the wild, preferably from its natural habitat.

However, collection from other areas is also possible. The species of *Neobeguea mahafalensis* is endemic to the South and the South-East region of Madagascar, where it is preferably collected.

For the sake of the present invention *Neobeguea mahafalensis* can be propagated and/or cultivated. Methods for cultivation of *Neobeguea mahafalensis* are described (27). Seeds are used for germination. There are approximately 15,000 seeds in 1 kg of dry material. Seeds can be kept in a metallic box for 30 months before use (27).

Other methods well known in the art of cultivation can be used for establishing growth of *Neobeguea mahafalensis,* such as (but not limited to) cuttings and shoots. Cells from *Neobeguea mahafalensis* can also be grown in cell culture. Tissue taken from *Neobeguea mahafalensis* can be grown in tissue culture. Explants can be derived from *Neobeguea mahafalensis* and used in tissue culture. Cells from *Neobeguea mahafalensis* can be used to produce zygotic embryos. Such embryos can further be used for propagation of *Neobeguea mahafalensis.* Hybrid cells can be produced by fusing cell(s) from *Neobeguea mahafalensis* with other cell(s). Such somatic hybrids can be used to produce new genotypes that are grown in different ways including producing new hybrid plants and growing in culture. Tissue or explants from *Neobeguea mahafalensis* can be multiplied by micropropagation *in vitro.* Cells from *Neobeguea mahafalensis* can be cloned to produce genetically identical assemblages of individuals that are propagated by vegetative means or grown in culture. Auxiliary shoot proliferation (auxiliary buds) can be induced by cytokine treatment. Such shoots and buds can also be used for propagation of *Neobeguea mahafalensis.* Tissue, shoots, clones, buds and parts from *Neobeguea mahafalensis* can be propagated by transplantation. Crosses of *Neobeguea mahafalensis* with other species can be produced. Cuttings, buddings or parts of *Neobeguea mahafalensis* can be transplanted onto another plant and the *Neobeguea mahafalensis* grown attached on the other plant. For the sake of the present invention cells, hybrid cells, clones, explants, genetic assemblies, crosses, buds, cultures, tissue, tissue cultures, cell cultures, shoots,

transplants, propagations and micropropagations derived as said above can be substituted for *Neobeguea mahafalensis,* or a part of *Neobeguea mahafalensis.* For sake of the present invention the cultivated *Neobeguea mahafalensis* can be used in the same way as the wild *Neobeguea mahafalensis.*

A very important aspect of the invention is the selection of specimens of *Neobeguea mahafalensis* which are rich in content of sexual enhancing activity. In particular the contents of the sexual enhancing component R306 can be assayed in extracts from different specimens of *Neobeguea mahafalensis* using the procedures described in Example 48. Specimens with high content of R306 are very desired for use with practically all aspects of the present invention. Particularly desired are specimens which content of R306 in their root is high; in particular specimens which give an R2C extract with high content of R306 (see Example 48 how to perform the assay). Such specimens with high content of R306 can be used as source for breeding *Neobeguea mahafalensis.* Vegetative propagation of such specimens assures high content of sexual enhancing effect in the plant as well as systematic selection and cross-breeding of such specimens can substantially increase the content of R306 and sexual enhancing activity being obtainable from the plant. Cultivation conditions and collection of the plant can be optimized (e.g. selecting the best time of the year for collection) in order to assure high content of R306 and sexual enhancing activity of the *Neobeguea mahafalensis* sample.

Disclosed herein are *Neobeguea mahafalensis* roots for use for treatment of medical condition for treatment of sexual dysfunction and/or veterinary condition as defined herein and/or for use as food supplement and/or dietary supplement.

However, it is a known fact in plant physiology that trees often grow in symbiosis with other organisms, such as fungi. Thus, for example, mycorrhizae are symbiotic associations that form between the roots of most plant species and fungi. These symbioses are characterized by bi-directional movement of nutrients and compounds between the plant and fungi.

Symbiosis includes mycorrhizal associations that vary widely in form and function. These include (but are not limited to) ectomycorrhizal fungi, which are mostly basidiomycetes that grow between root cortical cells of many tree species, forming a so called Hartig net. Others include (but are not limited to) arbuscular mycorrhizal fungi that belong to the order *Glomales* forming highly branched structures called arbuscules within root cortical cells of many herbaceous and woody plant species.

It is therefore contemplated that a component or compound obtained from or being obtainable from *Neobeguea mahafalensis* is also being obtainable from, or possible to derive from, an organism that lives in symbiosis with *Neobeguea mahafalensis.* Such symbiotic organisms include (but are not limited to), fungus, mycorrhizae, ectomycorrhizal fungi, basidiomycetes, arbuscular mycorrhizal fungi. For the sake of the present invention therefore an organism capable of living in symbiosis with *Neobeguea mahafalensis* can be substituted for *Neobeguea mahafalensis* for all aspects of the present invention. If said symbiotic organism grows or is grown in the absence of *Neobeguea mahafalensis* it still serves as a substitute for *Neobeguea mahafalensis* for all aspects of the present invention.

It is also well known that many plant components or compounds, such as e.g. terpenoids, vary in content during physiological and pathophysiological events of the plant. For example it is well known that terpenoids of many species increase substantially during stress conditions, e.g. fungal infection, herbivore attack of the plant, and alike.

In our studies preceding this invention we surprisingly found that extracts, drugs and pharmaceuticals prepared from some specimens of *Neobeguea mahafalensis* root tissue show very good treatment effect on sexual dysfunction in humans, as well as they show high activity in the sexual function assays in animals described herein (see Examples 17, 22, 23, 30, 32, and 34), while extracts, drugs and pharmaceuticals prepared from other specimens of *Neobeguea mahafalensis* show very low activity in these respects, even to the extent that these extracts, drugs and pharmaceuticals are practically inactive. It is contemplated that the preparations from the specimens showing such low activity are lacking sufficient amounts of the component(s), compound(s) having one or several of the characteristic mass-peaks described in Example 24 and further below and/or having too low contents of the chemical substance(s) of the invention. It is contemplated that external factors may be a reason, such as differing stress conditions subjected to the specimen prior to its collection. It is therefore contemplated that subjecting *Neobeguea mahafalensis* to stress prior to collecting it is beneficial to increase the sexual stimulating activity of, as well as promote the existence and/or demonstrability of one or several characteristic mass-peaks in, the extracts, drugs, components, compounds, substantially pure compounds, chemical substances of the invention and pharmaceuticals prepared for the purposes of the present invention, as well as to increase the amount of the chemical substance of the invention being possible to prepare from *Neobeguea mahafalensis.* In particular the chemical substances of the invention that can be increased in such ways are those with the chemical structures of any of R306, R310A and R310B. (See Example 48 for a method how to assay the content of R306 in different specimens of *Neobeguea mahafalensis*). Stressed specimens of *Neobeguea mahafalensis* can be selected in the wild (e.g., specimens that are stressed due to infection with parasites such as fungi, or stressed due to herbivore attack(s), or stressed due to mechanical damage such cutting of branches, removal of stem bark in part or in whole, and alike). It is also contemplated that stress can be induced artificially to *Neobeguea mahafalensis* prior to collecting it. Stress can be induced by various means such as (but not limited to) mechanical means (e.g. by cutting the plant or by partly removing stem bark or by other means subjecting the plant to mechanical damage), by chemical means

(e.g. subjecting the plant to toxic chemical agent, e.g. herbicide, defoliant) or by biological means (e.g. by infecting the plant with parasite, such as fungus).

A drug can be prepared from *Neobeguea mahafalensis* root tissue by separating the parts of the tree so as to provide in separate portions root, stem, trunk, stem bark, fruit, leaves, seeds, or branches.

In the preparation of a drug, a part of *Neobeguea mahafalensis* is usually cut into small pieces and/or ground before further use. A grinding device is usually applied to break it up. A commonly applied device is a hammer mill, but any type of grinder or mill can be used (e.g., ball mill, vibrating mill, air pressure (jet) mill, planetary centrifugal mill). Before cutting or grinding the part of the plant may be optionally dried or frozen. Crushing and grinding can be done at ambient temperatures or at low temperatures to maintain the material in frozen state.

In the preparation of the drug sieves can optionally be used to fractionate the ground (or correspondingly prepared) *Neobeguea mahafalensis* into suitable particle sizes. The materials can be repeatedly ground and/or sieved until a desired particle size is obtained. Suitable particle sizes for the drug of the present invention range (but are not limited to) from 30 μm to 10 mm, with 0.2 - 4 mm being preferred.

In the preparation of a drug, a part of the *Neobeguea mahafalensis,* or the cut or ground (and optionally sieved) material obtained from *Neobeguea mahafalensis,* can be preserved until further use by drying and/or freezing and/or by addition of preservative.

Drying of the drug can be done in the open air at ambient temperatures, or by applying controlled temperatures, usually temperatures between plus 10 to 200°C are being preferred, with plus 20 to 70°C being more preferred, and with plus 22 to 35°C being most preferred.

Passing air or an inert gas, such as nitrogen or argon, over or through the cut or ground material derived from *Neobeguea mahafalensis* can accelerate drying and preserve the activity of the drug. Nitrogen is a preferred gas in this context in order to avoid oxidation, but air is also satisfactory. The temperature of the drying gas is preferably controlled keeping it constant or varying in a cycle to provide a gentle drying.

Special devices for drying are well known in the art and can be applied. These include (but are not limited to) spreading the materials on shelves, using drying cabinets, vacuum drying cabinets, and/or fluidized bed dryers.

The drug can also be prepared by freeze-drying (i.e., lyophilization). In such a case the desired part of the plant is usually first cut into pieces, or ground, then frozen and subsequently placed in a freeze-dryer (a vacuum device equipped with a cold-trap). Vacuum is applied and the vacuum is maintained until the sample is dry. Alternatively the plant part is first frozen and then cut or ground whereupon it is placed in the freeze dryer, vacuum is applied and the vacuum is maintained until the sample is dry.

The drug prepared by grinding the plant, or part of the plant, followed by optional sieving and/or optional drying, can be used directly for the preparation of a pharmaceutical and/or a food supplement and/or a dietary supplement, as will be detailed below.

The drug or plant part may be sterilized before use and/or storage to render it free from microorganisms. Sterilization for these purposes can be done with procedures well known in the art such as (but not limited to) autoclaving, radiation sterilization and ozone treatment. In e.g. ozone treatment the drug or plant part is placed in an atmosphere supplemented with a suited concentration of ozone (preferably generated with an ozone generator) for a suited period of time. In radiation sterilization the drug or plant part is radiated with gamma rays, e.g. using a Cobalt-60 gamma source. Radiation sterilization is a preferred method for sterilization. Radiation sterilization can also be done by subjecting the drug or plant part to radiation with an electron beam. Chemiclaving and heating by microwave irradiation are yet other processes for sterilization that can be used for.

The drug can be further processed by applying extraction. Prior to the extraction the drug may be processed by cutting and/or grinding with optional drying and/or sieving. However, in some embodiments the drug is prepared from the plant or plant part directly and used essentially uncut.

Extraction is usually performed by placing the (grinded or powdered) drug in a suited solvent (or solvent mixture) and allowing the extraction to proceed for a suited length of time, using a suited temperature with optional stirring or agitation. The extraction time is usually from 1 minute to 7 days, most preferably 1 to 24 hours. In many embodiments of the invention the extraction process is accelerated by heating. The temperature applied during extraction usually varies from -30°C to +200°C, more preferably 0 - 100°C, more preferably 10 - 50°C. However, in many preferred embodiments of the invention the extraction proceeds at the boiling temperature, or just below the boiling temperature, of the solvent or solvent mixture used in the extraction procedure. Extraction can be performed by procedures well known in the art, such as by maceration and percolation. Continuous extraction, where the drug is moved continuously in the opposite direction of the flow of the extracting solution (i.e., applying the principle of counter current), can also be applied.

Any type of solvent can be used for the extraction of the drug. In one important embodiment extraction of the drug is performed by using water, with distilled water being preferred. The plant material is placed in cold or heated water. A preferred procedure is to place the drug in boiling water and continue the boiling for a period of time (usually a few minutes) whereafter the mixture is allowed to cool down, allowing the extraction to be continued for an additional period of time (usually several hours).

The extraction of the drug may be performed using organic solvent(s). Any type of organic solvents can be used including hydrophilic and/or lipophilic solvents, such as ethylacetate, acetone, chloroform, methylene-chloride (dichloromethane), ethylmethylketone, ethanol, petroleum ether, acetonitrile, glycerol, with dichloromethane, chloroform, ethylacetate, acetone and ethanol being most preferred. Mixtures of organic solvents can be used as well, such as mixtures of ethanol and water. Acids, such as hydrochloric acid (usually diluted in water) can also be applied. Alkaline solutions, such as sodium hydroxide, sodium carbonate or sodium bicarbonate in water solution are also useful, as well as other water solution and/or buffered solution (e.g. sodium phosphate buffer or ammonium acetate buffer) can be used. Preferred are extracts produced by lipophilic solvents or combinations of the use of hydrophilic and lipophilic solvents.

Herein extracts of root and stem bark of *Neobeguea mahafalensis* produced by extraction of chloroform are termed RCH and SCH, respectively. When produced by ethanol they are termed REtOH and SEtOH, respectively. When produced by acetone they are termed RT and ST, respectively. The solution produced by extraction of a portion of *Neobeguea mahafalensis* with ethanol or water/ethanol mixture is termed tincture and is in the case root of *Neobeguea mahafalensis* being used herein termed R-tinct and in case of stem bark of *Neobeguea mahafalensis* being used termed S-tinct.

A preferred embodiment of the invention comprises placing the ground root of *Neobeguea mahafalensis,* in an organic solvent (whether hydrophilic solvent or lipophilic solvent), such as chloroform, dichloromethane, ether, petroleum ether, ethylacetate, acetone, with chloroform being preferred and followed by extensive shaking (usually from several hours to days) and then filtering the materials, collecting the organic solvent and evaporating until a dry extract is obtained. The thus obtained extract (termed 'RCH' in case of starting with root of *Neobeguea mahafalensis*) is highly useful for preparation of a pharmaceutical, food or dietary supplement of the invention as well as for further fractionation to obtain compounds, substantially pure compounds and chemical substances of the invention. A specific example of this embodiment of the invention is given in Example 42. Sequential extraction is also used where the ground *Neobeguea mahafalensis* materials is extracted first with one solvent and then again with another solvent. Using different solvents (whether hydrophilic or lipophilic) in sequence increases the yield of the extraction process, as is clearly shown in Example 50.

It is advantageous to subject the drug to sequential extractions using different solvents as this increases the efficiency of the extraction process. In one specific embodiment the ground plant root tissue of *Neobeguea mahafalensis* is first extracted with a lipophilic solvent to produce one extract and then the remaining plant tissue is extracted with a hydrophilic solvent to produce yet another extract. In another embodiment the ground plant root tissue of *Neobeguea mahafalensis* is first extracted with a hydrophilic solvent to produce one extract and the remaining plant tissue is then extracted with a lipophilic solvent to produce yet another extract. Extractions according to this embodiment of the invention can proceed in any order using several hydrophilic and lipophilic solvents. Extracts produced by sequential extractions according to this embodiment of the invention can be united to produce combined extracts. Moreover, extracts produced according to this embodiment of the invention can be processed further using any one other procedure(s) described in this patent, such as chromatography, or e.g. by dissolving it in water and partitioning (i.e. extracting or exhausting it) with a liphopilic solvent, or dissolving it in a lipophilic solvent and partitioning (i.e. extracting or exhausting it) with water. The extract produced according to this embodiment of the invention is also used both in preparation of the lipid soluble fraction of the invention, as well as the high molecular weight fraction of the invention. A specific example of this embodiment of the invention is given in Example 50.

For the sake of the present patent preferred lipophilic solvents are any one of (or even mixtures of) pentane, n-pentane, 2-methylbutane, 2,2-dimethylpropane, hexane, n-hexane, 2-methylpentane, 3-methylpentane, 2,3-dimethylbutane, 2,2-dimethylbutane, heptane, n-heptane, 2-methylhexane, 3-methylhexane, 2,2-dimethylpentane, 2,3-dimethylpentane, 2,4-dimethylpentane, 3,3-dimethylpentane, 3-ethylpentane, 2,2,3-trimethylbutane, octane, n-octane, 2-methylheptane, 3-methylheptane, 4-methylheptane, 3-ethylhexane, 2,2-dimethylhexane, 2,3-dimethylhexane, 2,4-dimethylhexane, 2,5-dimethylhexane, 3,3-dimethylhexane, 3,4-dimethylhexane, 2-methyl-3-ethylpentane, 3-methyl-3-ethylpentane, 2,2,3-trimethylpentane, 2,2,4-trimethylpentane, isooctane, 2,3,3-trimethylpentane, 2,3,4-trimethylpentane, 2,2,3,3-tetramethylbutane, cyclohexane, benzonitrile, chlorobenzene, diethyl ether, methyl-tert-butyl ether, methylenechloride, dichloromethane, chloroform, carbon tetrachloride, 1,2-dichloroethane, perchloroethylene, trichloroethylene, 1,1,1-trichloroethane, trichloroethene, perchloroethylene, tetrachloroethene, vinylchloride, ethylacetate, methylethylketone, propyl acetate, iso-propyl acetate, butyl lactate, n-butyl lactate, iso-butyl lactate, tert-butyl lactate, sec-butyl lactate, butyl acetate, n-butyl acetate, iso-butyl acetate, tert-butyl acetate, sec-butyl acetate, triacetin, 1,2,3-triacetoxypropane, diacetin, glycerol 1,3-diacetate, glycerol 1,2-diacetate, benzene, toluene, xylene, o-xylene, m-xylene, p-xylene, 2,2,4-trimethylpentane, butanone, 2-butanone, pentan-3-one, pentan-2-one, 3-pentanone, 2-pentanone, cyclopentanone, butan-1-ol, butan-2-ol, n-butanol, 1-butanol, sec-butanol, 2-butanol, isobutanol, 2-methyl-1-propanol, 2-methyl-2-propanol, pentanol, 1-pentanol, 3-methyl-1-butanol, 2-methyl-1-butanol, 2,2-dimethyl-1-propanol, 3-pentanol, 2-pentanol, 3-methyl-2-butanol, 3-methyl-2-butanol, 2-methyl-2-butanol, hexanol, heptanol, octanol, monohydroxylated hydrocarbon(s), diethylene glycol, dimethyl sulfoxide, methyl t-butyl ether, N-methyl-2-pyrrolidinone, nitromethane, tetrahydrofuran, triethyl

amine, benzylalcohol, carboxylic acid methyl ester, carboxylic acid ethyl ester, fatty acid methyl ester, vegetable oil, animal oil, triglyceride(s), mineral oil, wood turpentine, petroleum ether, naphtha, hydrocarbon solvent, chlorinated hydrocarbon solvent, fluorinated hydrocarbon solvent, halogenated hydrocarbon solvent, freon, 1-bromo-3-chloropropane, [2-(2-butoxyethoxy)ethyl]acetate, 2-butoxyethyl acetate, cumene, cyclohexanol, cyclohexanone, decahydronaphthalene, n-decane, dibenzyl ether, 1,2-dichlorobenzene, 1,3-dichlorobenzene, 1,4-dichlorobenzene, 1,2-dichloroethane, dimethyl carbonate, diethylene glycol dibutyl ether, diethylene glycol diethyl ether, diethylene glycol mono-n-hexyl ether, diethylene glycol monobutyl ether, diethylene glycol monoethyl ether, diethylene glycol monomethyl ether, ethylbenzene, ethyl formate, 2-ethyl-1-hexanol, dimethyl sulfoxide, 1,1,1,3,3,3-hexafluoro-2-propanol, isoamyl acetate, 2-methylbutyl acetate, 3-methylbutyl acetate, isoamyl butyrate, isobutyl acetate, isopropyl acetate, isopropyl methyl ketone, 1,3,5-trimethylbenzene, (1-methoxy-2-propyl)acetate, methyl acetate, methylcyclohexane, methylcyclohexanol, 5-methyl-3-heptanone, 3-methyltetrahydropyran, 2-methylpentane, 2-methyl-1-butanol, *n*-nonane, nitroethane, propylacetate, 1,2-propylene glycol diacetate, propylene carbonate, tetrahydrofuran, tetrahydrofurfuryl alcohol, 1,2,3,4-tetrahydronaphthalene, triethylene glycol, dimethyl sulphoxide, dimethyl ether, methyl ethyl ether, liquid ethane, liquid propane, liquid butane, liquid *iso*-butane, liquid carbon dioxide, liquid trifluoromethane, liquid chlorotrifluoromethane, liquid trichlorofluoromethane, liquid ammonia. The most preferred lipophilic solvents are dichloromethane, chloroform and ethylacetate.

For the sake of the present patent preferred hydrophilic solvents are any one of (or even mixtures of) water, methanol, ethanol, acetonitrile, propionitrile, propanol, propan-1-ol, propan-2-ol, dimethyl sulfoxide, formamide, dimethylformamide, acetone, tetrahydrofurane, glycol, glycerol, dioxane, 1,4-dioxane, formic acid, acetic acid, propionic acid, butyric acid, 2-methylpropanoic acid, 3-oxobutanamide, N,N-diethylacetamide, N,N-diethyl acetoacetamide, propylene glycol, methylsulfonylmethane, ethanol amine, *tert*-butyl alcohol, diethylene glycol, 1,2-dimethoxy-ethane, ethylene glycol, hexamethylphosphoramide, hexamethylphosphorous triamide, pyridine, 2-methyltetrahydrofuran, 3-methyltetrahydropyran, 2-methylpyridine, 1,3-propanediol, sulfolane, triethylene glycol, tetraethylene glycol, tetrahydrofurfuryl alcohol, triethanolamine, triethyl phosphate, triethylene glycol, triethylene glycol dimethyl ether, triethylene glycol monomethyl ether, N-Methyl-2-pyrrolidone, liquid carbon dioxide. The most preferred hydrophilic solvents are water, acetonitrile, ethanol and acetone.

The extraction of the drug with lipophilic solvent, hydrophilic solvent, organic solvent, water, water solution, alkaline solution, acid solution or buffered solution, can proceed in the cold or at elevated temperatures. Suited temperatures are from - 20°C to +100°C, with temperatures of +20°C to +60°C preferred, and +20°C to +35°C most preferred. The boiling temperature of the solvent is often a suited temperature.

After the extraction a solution comprising the extract is obtained by decanting, filtering or centrifugation, or any other suited means that removes the non-solubilized portions, as may be required.

Another preferred extraction procedure for the preparation of the extract of the present invention is using Soxhlet procedure (32). Typically, the drug (i.e., ground root of *Neobeguea mahafalensis)* is placed inside a 'thimble' made from filter paper (or other suited materials) which is loaded into the Soxhlet extractor. The extractor is attached to a flask containing a solvent such as methylenechloride, diethyl ether or petroleum ether with methylenechloride being preferred, and a condenser. The solvent is heated, causing it to evaporate. The hot solvent vapour travels up to the condenser, where it cools and drips down onto the drug. The chamber containing the drug slowly fills with warm solvent until it is almost full, when it is emptied by a siphon action back down to the flask. This cycle is preferably allowed to repeat many times. During each cycle, a further extraction is achieved. The repeated extraction with clean solvent increases the efficiency of the extraction. Moreover, vacuum can be applied to reduce the temperature during the extraction with the Soxhlet extractor, which is preferred in some embodiments of the present invention. In addition an inert gas can be introduced into the Soxhlet extractor, e.g. nitrogen or argon, in order to prevent oxidation.

Examples of the extraction of *Neobeguea mahafalensis* using Soxhlet procedure are given in Examples 27 and 28.

Soxhlet extraction is in some embodiments of the invention a quite preferred procedure for the preparation of the extract of the invention, as a part of the process of preparing the pharmaceutical of the invention, due to the efficiency of the Soxhlet extraction procedure. Most preferred is the application of the Soxhlet procedure for preparing extracts of the root and stem bark of *Neobeguea mahafalensis,* with root being most preferred.

However, in most embodiments of the invention Soxhlet extraction is specifically excluded because the procedure is too harsh and extracts many non-desired components from the *Neobeguea mahafalensis*.

Another extraction procedure, well known in the art, which can be used to prepare the extract of the present invention is high pressure supercritical fluid extraction, with the preferred solvent being fluid (liquid) carbon dioxide although any other fluidized gas can be used as well (33,34).

According to this procedure the gas is compressed into a liquid. This liquid is then pumped through a cylinder or container containing the drug (e.g., ground plant part; preferably prepared from stem bark or root of *Neobeguea mahafalensis* with root being most preferred). From there, the extract laden liquid may be pumped into a separation chamber where the extract is separated from the gas by decompression, yielding the desired extract in solid form. (The gas may optionally thereafter be recovered for re-use).

Yet another extraction procedure, well known in the art, which can be used to prepare the extract of the present invention, is ultrasound-assisted extraction (35, 36). Typically, the drug of the invention is inserted in an extraction chamber (e.g. made from stainless steel) and drained by the extraction fluid (e.g. organic solvent such as hexane, chloroform, ethylacetate). The chamber is then immersed in a water bath in which an ultrasonic probe is immersed. (Other means of sonication may be applied, as well). Fresh extraction fluid may be pumped into the extraction chamber during the sonication process, while the old fluid is removed, which may increase the efficiency of the extraction.

Any one of the extraction procedures of the present invention can be scaled up by using bigger extraction vessels and/or devices and larger volumes of extracting liquids. In typical industrial settings between 100 g to 1000 kg of the drug is extracted in each batch, or the extraction process is run continuously typically processing from 10 g to 1000 kg drug per hour.

Following the extraction procedures the obtained solution (i.e., extract) can be administered as is to a human or animal, accordingly then in a sense the extract comprising a pharmaceutical and/or food supplement and/or dietary supplement. Preferred solvents for this embodiment of the invention are water and ethanol. Examples of embodiments of the invention where the extraction proceeds using water and/or ethanol are given in Examples 1-5. Moreover, the tea-bag of the present disclosure (see below) can be placed in water or ethanol or a water/ethanol mixture to produce a solution (i.e., extract) which can be ingested as a pharmaceutical and/or food supplement and/or dietary supplement.

However, in most embodiments of the invention it is preferred to process the extract further in order to produce the food supplement, dietary supplement, pharmaceutical, compound, substantially pure compound of the invention as well as for producing the chemical substance of the invention. Thus, following the extraction the solvent is usually removed in order to obtain a residue. The residue (herein also termed extract) can be administered as it is to a human or an animal, thus in a sense then being a pharmaceutical, or it can be processed further in the manufacture of the pharmaceutical, compound, substantially pure compound of the invention. Also the processing the extract further can isolate the chemical substance of the invention.

Removal of the solvent of the extract can be done by, e.g., evaporation, evaporation at reduced pressure (e.g., using a Rotavapor), spray drying, or freeze-drying. When water is used as solvent, freeze drying and spray-drying are preferred procedures.

Removing the solvent often results in oily wax-like residue which can often be converted to a powder by triturating, e.g. using a spatula. Removing the solvent with freeze drying can also preferably be applied as this avoids oily or wax-like residues. An oily or wax-like residue can be converted to a fluffy powder after dissolving the oily or wax-like extract in suited solvent (e.g. acetonitrile) and then freeze drying it. The extract of the invention in form of powder, in particular a fluffy powder, comprises a preferred embodiment of the invention.

A highly preferred extract of the invention is made from the water extract of the drug, usually prepared by grinding *Neobeguea mahafalensis* root tissue and placing the ground drug in water (usually hot or boiling water is used, which is followed by optionally cooling the mixture), followed by filtering or removing the solid portion by other means. This is then followed by removing the water from the water extract, preferably by freeze-drying or spray-drying. This procedure results in a fluffy powder, which is highly desired in the manufacture of the food and dietary supplement and pharmaceutical of the invention. This is due to that the powder obtained has very good physical properties, being highly suited for preparation of capsules or tablets, and the other types of pharmaceuticals and food and dietary supplements of the invention described herein, as well as the powder has increased stability compared to water solutions or other solutions. Evaporating the water with other means than lyophilization or spray drying such as using a rotavapor may lead to oily residue which is less desired in the manufacture of the pharmaceutical and food and dietary supplement of the invention. (However, such oily residue can be converted into powder or fluffy powder as described above and are then more useful). An example how to prepare a lyophilised water extract from *Neobeguea mahafalensis* is given in Example 8, this type of extract being termed 'RW' when the procedure is applied with the root of *Neobeguea mahafaliensis* as starting materials and 'SW' in case that the procedure is applied with stem bark of *Neobeguea mahafalensis* as starting materials. RW is most preferred for the preparation of pharmaceuticals and food and dietary supplements. This is because of the high water solubility of these extracts, their ease of preparation and their suitability for preparation of orally administrable pharmaceuticals and food and dietary supplements of the invention. In the preparation of RW freeze drying and spray-drying can be used with essentially the same result.

An alternative way of removing water is to passing the water extract of *Neobeguea mahafalensis* through a resin such as Diaon SP-207 (Mitsubishi Kasei Corporation) (i.e. this is the process of applying chromatography). The compounds and components of the invention will then attach to the resin and can be retrieved in a small volume by passing a suited organic solvent through the resin.

In the extraction procedure used for preparation of RW water can be exchanged for water solution or hydrophilic solvent or hydrophilic solvent mixtures or water/hydrophilic solvent mixtures (e.g., but not limited to, acetonitrile, ethanol, ethanol/water mixture, acetone and acetone/water mixture) and still yielding the RW extracts of the invention (or corresponding when starting with other parts of the plant).

In the present invention by 'fluffy powder' is intended that the powder contains a substantial amount of air (or

gas), the content of air being at least 5 %, preferably at least 10 %, more preferably at least 20 %, even more preferably at least 30 %, even more preferably at least 50 %, even more preferably at least 80 % and most preferably at least 90 % by volume. (I.e. by 80 % by volume is in this context intended that the powder contains 80 % air (or gas) and 20 % solid material, both by volume).

However, in most embodiments of the invention the fluffiness of a powder is instead characterized by the density of the powder (i.e., weight per volume). Therefore in a further sense by 'fluffy powder' is intended that the density of the powder is less than 500 mg/ml, more preferably less than 400 mg/ml, even more preferably less than 300 ml/mg, even more preferably less than 200 mg/ml, even more preferably less than 150 mg/ml, and most preferably less than 150 mg/ml.

However, after one has obtained the fluffy powder of the invention it may be compacted, e.g. by applying pressure to it or grinding it. It may also be granulated by procedures well known in the art. Such compacted powders or granulates are also highly useful in the preparation of pharmaceutical and food and dietary supplement of the invention and can be substituted for the any extract and/or fluffy powder of the invention.

Another type of extract highly preferred extracts are made by first extracting the ground root of *Neobeguea mahafalensis* with a hydrophilic solvent to produce a first extract or fraction, and then subjecting the first extract or fraction to extraction with a lipophilic solvent to produce a second extract. The second extract thereby obtained comprises the highly desired lipid soluble fraction (extract) of the invention. A very specific highly preferred enriched extract according to this embodiment of the invention is obtained by extraction of the ground root of *Neobeguea mahafalensis* with hot water followed by filtering, followed by optional drying of the water solution (usually freeze drying or spray drying) resulting in a brown powder (or optionally just concentrating the water extract removing part of the water), dissolving the brown powder in a small volume of water (if required) and partitioning the solution in between chloroform-water, followed by collecting the chloroform phase and evaporating the chloroform phase to dryness; the procedure yielding a white powder, termed 'R2C' when the starting materials is the root of *Neobeguea mahafalensis.*

Other extracts similar to R2C can be prepared in an analogous fashion as for the preparation of R2C. Such extracts then also comprise useful enriched extracts of the invention. Moreover, in the extraction procedure used for preparation of R2C water can be exchanged for hydrophilic solvent or water/hydrophilic solvent mixtures (e.g., but not limited to, acetonitrile, ethanol, ethanol/water mixtures, acetone and acetone/water mixtures), while chloroform can be exchanged for any lipophilic solvent or lipophilic solvent mixtures (e.g., but not limited to, methylenechloride, hexane, petroleumether, ether, ethyl acetate) and still yielding essentially the R2C extracts (or corresponding when starting with other parts of the plant).

R2C are highly preferred enriched extracts of the invention, with R2C being one of the most preferred enriched extracts of the invention. R2C, which are highly useful for preparing the pharmaceuticals of the invention, are also highly useful as starting points for further fractionations in order to obtain the further enriched extracts, components, compounds and/or substantially pure compounds of the invention. An example how to specifically perform this procedure of the invention is given in Examples 9 and 33. Results of assays of the sexual enhancing activity of R2C are given in Examples 22 and 34.

Combinations of the procedures described above and in the following may also be performed in any suited order. E.g. the RWExh extract (see below) may be first prepared and after removing of all solvents the dried extract is dissolved in water and then subjected to extraction with chloroform or other lipophilic solvent in the same fashion as for the preparation of R2C. This creates also a variant of R2C which is also a highly preferred embodiment of the invention. A variant of R2C prepared in this fashion can be exchanged with R2C for any purpose of the present invention.

Yet another highly useful embodiment of the invention is the extraction ground root of *Neobeguea mahafalensis,* with a lipophilic solvent and then exhausting the lipophilic solvent fraction with a hydrophilic solvent and then retrieving the materials remaining in the lipophilic solvent in order to produce a lipid soluble fraction (extract). One specific example of this embodiment of the invention is the extraction of *Neobeguea mahafalensis* root with ethyl acetate (a lipophilic solvent), filtering off the non-solubilized materials, collecting the ethylacetate solution, and then adding water and shaking vigorously in order to 'exhaust' the ethyl acetate phase, and after phase separation collecting the ethyl acetate fraction and evaporating off the ethyl acetate in order to obtain a residue, the residue being the desired lipid soluble extract of the invention.

Another highly useful very specific enriched extract of the invention is prepared by extraction of the ground root of *Neobeguea mahafalensis* with boiling water usually followed by cooling of the decoction while the drug is present in the water, followed by removing the solid from the decoction (usually by filtering), and subsequently extracting the water solute with an organic solvent (preferably the organic solvent being partially soluble in water) by the process herein referred to as 'exhausting' (ethylacetate is being preferred, but other solvents partially miscible with water, such as, but not limited to, butanol, benzyl alcohol, butyl lactate or triacetin may be used). After phase separation the water phase is collected and evaporated to dryness, e.g. using a Rotavapor, spray dryer or by freeze drying; the resulting enriched extract being a preferred extract of the invention; in case of the starting materials is root of *Neobeguea mahafalensis* the extract manufactured according the to this procedure being termed 'RWExh' and constituting a particularly preferred enriched extract of the invention. This is because the procedure removes unwarranted potentially toxic principles from

the initial water extract, compared to using many other procedures for extraction. Examples how to prepare RWExh are given in Examples 29 and 31. An example how to perform an assay of the sexually enhancing activity of RWExh is described in Example 30.

By term 'exhausting' means herein that a drug, extract, fraction or solution is extracted with a solvent with the purpose to remove components being solvable by said solvent from the drug, extract, fraction or solution, and then collecting the remainder of drug, extract, fraction or solution, said drug, extract, fraction or solution thus having become exhausted of some of its components.

A similar procedure is to extract the ground root of *Neobeguea mahafalensis,* with a hydrophilic organic solvent, evaporating the hydrophilic organic solvent to produce a first extract and then solubilising the extract in a further hydrophilic and/or lipophilic solvent, and then partitioning the extract in between hydrophilic and lipophilic solvents (i.e. the process comprising 'exhausting' the first extract), and isolating the lipophilic solvent phase in order to produce a lipid soluble second extract and/or isolating the water phase to produce yet another second extract. A specific example of this embodiment of the invention comprises the extraction of the ground root of *Neobegueci mahafalensis* with acetone, filtering off the non-solubilised materials collecting the acetone solution, evaporating off the acetone to produce a first extract, and then solubilising the first extract in water and/or chloroform and partitioning the solution between chloroform and water, then isolating the chloroform phase and evaporating off the chloroform to produce a residue comprising the desired lipid soluble second extract.

Any extract of the invention, whether produced by hydrophilic or lipophilic solvents or the combinations thereof, according to any procedure of the invention, may be further fractionated using procedures well known in the art. The extract may be subjected to sequential solvent extractions using different solvents, or buffers of different compositions, ionic strength and pH. It may be subjected to fractionation by subsequent and/or sequential procedures involving (in any selected order(s) and/or repetition(s)) one or several of solvent extraction, partitioning between solvents of different polarity, chromatography; in particular liquid chromatography including: preparative chromatography, flash chromatography, thin layer chromatography, paper chromatography, ion-exchange chromatography, normal phase chromatography, polar interaction chromatography, hydrophilic interaction chromatography, reversed phase chromatography, high pressure liquid chromatography, medium pressure liquid chromatography, hydroxyapatite chromatography, silica gel chromatography, aluminia chromatography, chiral chromatography, achiral chromatography, gel filtration, Sephadex LH-20 chromatography, molecular sieving and molecular size exclusion chromatography, as well as electrophoresis such as free flow electrophoresis, zone electrophoresis, tachophoresis, iso-tachophoresis, isoelectric focusing, or dialysis, crystallization, filtration, or other fractionation method. Such procedures aim to enrich the activity of the preparation and remove unwarranted materials. Often several different fractionation methods are combined in sequence in order to obtain higher purity. E.g. solvent extraction(s) is first applied which is followed by chromatographic procedure(s) and/or solvent extraction by partition in hydrophilic (usually water, buffer or water solution) and lipophilic solvent. A preferred procedure is first to apply water extraction of the dried plant part where usually heated water is used in the extraction. This is followed by (optional) lyophilization of the water extract or drying by other means to obtain a dry power. The thus obtained powder, oil or solid is dissolved in a small quantity of water, or the water extract is applied directly without lyophilization or drying, and organic solvent is added (usually chloroform or any other suited solvent, e.g., butanol, methylethylketone, ethylacetate, methylenechloride, petroleum ether, hexane, benzene, carbontetrachloride, trichloroethylene). (Lipophilic solvents are preferred with chloroform being most preferred). After thorough mixing and phase separation the organic phase is collected and the solvent evaporated. (Usually there is a precipitate formed which is discarded along with the water-phase). In yet other embodiments of the invention the root of *Neobeguea mahafalensis* is extracted directly with organic solvent such as acetone or chloroform followed by the removal of the organic solvent by evaporation. The thus obtained extract (i.e. 'enriched extract') obtained after evaporation of the organic solvent according to any one of the foregoing procedures is a preferred embodiment of the invention because such extracts have removed unwarranted materials from the extract and concentrated the active principle, and are therefore highly desired in the preparation of the pharmaceutical and/or food supplement and/or dietary supplement of the invention. Other enriched extracts are prepared by Soxhlet procedure and are good starting materials for further enrichment by other fractionation methods, e.g. chromatography. Yet another method for obtaining an enriched extract is by absorption of an extract to a suited solid support such as Diaon SP-207 and then performing sequential eluting with organic solvents of increasing lipophilicity. Enriched extract(s) (or any other extract(s) derived from *Neobeguea mahafalensis)* can thus be further processed by fractionation, e.g. by reversed phase chromatography, normal phase chromatography, HPLC and alike; the purpose being to make the extract even more pure; even eventually obtaining the substantially pure compound of the invention. Silica gel is another very useful solid support for chromatography according to this embodiment of the invention. In a general sense chromatographic supports for liquid chromatography can exploit hydrophobic interactions and are then termed hydrophobic interaction columns, as well as they can alternatively exploit polar interactions and is then termed polar interaction column. Hydrophobic liquid interaction chromatography is also termed reversed phase chromatography while polar liquid interaction chromatography is also called normal phase chromatography.

Throughout this patent by term hydrophobic interaction liquid chromatography is *mutates mutandis* intended

the same thing as hydrophobic interaction chromatography which is furthermore *mutatis mutandis* intended to mean the same thing as reversed phase chromatography.

Throughout this patent by term polar interaction liquid chromatography is *mutatis mutandis* intended the same thing as polar interaction chromatography which is furthermore *mutatis mutandis* intended to mean the same thing as normal phase chromatography.

In the case of hydrophobic interaction chromatography compounds will attach to the non-polar stationary phase by hydrophobic interactions while the mobile phase used is largely polar. The materials bound to the stationary phase can be eluted using a mobile phase with a suited balance of hydrophilic and lipophilic properties; the most polar compounds elute first with the most non-polar eluting last; retention increases as the amount of polar solvent (like e.g. water) in the mobile phase increases. Examples of the stationary support for hydrophobic interaction columns are silica derivatised with hydrophobic alkyl chains (such as $C_{18}H_{37}$ or $C_8H_{17}$) that interact with the analyte. There are three common chain lengths, C4, C8, and C18, but other lengths are possible too. The mobile phase can be mixtures like acetonitrile/water, isopropanol/water, methanol/water. Sephadex derivitized with hydrophobic groups such as hydroxypropylated Sephadex and acetylated Sephadex also find use as stationary support for hydrophobic interaction chromatography.

In case of polar interaction chromatography the stationary phase is polar and the mobile phase is largely non-polar. The compound attaches to the stationary phase with polar interactions and can be eluted by using a mobile phase with a suited balance of hydrophilic and lipophilic properties; the most non-polar compounds elute first and the most polar elute last. The mobile phase generally consists of a very nonpolar solvent like hexane or heptane, or slightly polar solvents like dichloromethane, which are mixed with more polar solvents like isopropanol, methanol, ethyl acetate, chloroform or dioxane. Retention increases as the amount of non-polar solvent in the mobile phase increases. Examples of stationary phases for polar interaction chromatography are silica (e.g. silica 60 from Merck KGaA, Germany, LSP Processing, D-64271 Darmstadt, Germany), alumina (aluminium oxide), cellulose, hydroxyapatite, vinyl alcohol bonded silica, polyamine bonded silica. Silica is a particularly desired support for polar interaction chromatography for the purpose of the present invention as it allows the preparation of very large batches of the extract of the invention, even in amounts up to the range of 1-1000 kg. (See Example 50 how to apply polar interaction chromatography for this aspect of the invention).

Polar interaction chromatography also includes hydrophilic interaction chromatography, HILIC (see Grumbach et al., Hydrophilic interaction chromatography using silica columns for the retention of polar analytes and enhanced ESI-MS sensitivity, LCGS Asia Pacific, vol. 7, number 4, November 2004). HILIC can use as stationary phase simple unbonded silica, silanol or diol bonded phases, amino or anionic bonded phases, amide bonded phases, cationic bonded phases and zwitterionic bonded phases. For the sake of the present invention HILIC can be used in the same way as polar interaction chromatography, i.e. in the same way as normal phase chromatography.

Gas chromatography is also well known in the art. For the purpose of the present invention gas chromatography can be used to analyze the presence and to quantify the amount of the compound of the invention in biological samples, extracts and alike.

In the widest sense in this patent by chromatography is intended a process wherein components held in solution are attached to a solid support whereafter subsequently solvents are applied to detach the components from said solid support. The solid support for chromatography can be placed in columns, the thus formed arrangement being termed chromatographic column; the solution with components are introduced to the chromatographic column in one end and the components are allowed to attach to the column's solid support. The components are thereafter eluted by introduction of solvent(s) into the column. However, it is also possible to perform the process batchwise. In this case the components are introduced to the solid support by mixing the component solution with a solid support. Elution is then made by adding solvent(s) to the support, mixing the solvent(s) and support, and then separating the solvent(s) from the support. For the sake of the present invention such a batch process is also included into the process of chromatography; introducing the solution to the solid support has the same meaning as the process of applying a fraction or extract to a chromatographic column. Other variations around this theme are also possible, such as thin layer chromatography (TLC), where the solid support is attached to a surface and wherein the solvent(s) are allowed to move by capillary force; TLC thus being included in the process of chromatography. In the widest sense any process where a solution of components are contacted with solid support and then removing the components from the support by any suited process so as to obtain an extract is herein included into 'chromatography'; the process of contacting components with a solid support is included into the process of 'applying a fraction or extract to a chromatographic column'.

The material obtained, purified or fractionated from an extract of the invention is also termed extract in the sense of this invention and are useful for all aspects of the present invention whenever term extract is used. The material obtained, purified or fractionated from an extract of the invention is also termed enriched extract in the sense of this invention and are also useful for all aspects of the present invention in the same way the extract of the invention can be used.

Due to the fact that the most desired components (including the chemical substances) of the invention that are responsible for causing the sexual enhancing effect are lipid soluble, the most important embodiments of the invention

use at least once somewhere in the preparation of the extract of the invention a lipophilic solvent. However, since extractions can proceed in many different ways the use of the lipophilic solvent can be placed at many different steps in the extraction procedures of the many procedures included into the invention. One typical example is to start the extraction of the ground root of *Neobeguea mahafalensis* with a lipophilic solvent (e.g. chloroform, dichloromethane, hexane, petroleum ether, ether, etc.). The extract thus formed can be subjected to further fractionations as disclosed herein. However, it may be advantageous to once again, in an optional step, extract the remainder of the lipophilic solvent extracted ground root (i.e. 'lipophilic solvent exhausted' root) with a further solvent, like a hydrophilic organic solvent like acetone or ethanol, or even water. This produces a further extract which also contains the active principles of the invention, like R306 and R310. Sequential extraction in this way with lipophilic solvent and hydrophilic solvent (in any order) (and even with mixtures of lipophilic and hydrophilic solvents) thus increases the yield for the extraction of the components causing sexual enhancing effect, including those for R306 and R310. Often the so obtained extract is processed further in different ways, and extracts may also be combined before being processed further, e.g. before being subjecting them to normal phase chromatography (i.e. applying the extract to a polar interaction column) or to reversed phase chromatography. In Example 50 the use of such a procedure is described, wherein root of *Neobeguea mahafalensis* is first extracted with dichloromethane to produce an extract, then the root is again extracted with acetone to produce another extract, and after solvent evaporations the dichloromethane and acetone extracts are combined into a combined extract and the combined extract is subjected to polar interaction chromatography using silica gel chromatography (i.e. normal phase chromatography) using dichloromethane and mixtures of dichloromethane and methanol as eluents in order to produce 'D-Ac1', a lipid soluble extract which is a very desired embodiment of the invention due to the fact that D-Ac1 can substitute for R2C for practically all embodiments of the present invention. Moreover, the order of subjecting the root to hydrophilic and hydrophobic solvent does not matter; i.e. in case of producing D-Ac1 the root can first be extracted with acetone, then with dichloromethane, the extracts combined and then subjected to silica gel chromatography. On top of that the root can be extracted with only a hydrophilic solvent and then the materials is subjected to further fractionation preferably using an polar interaction chromatography (normal phase chromatography) wherein the elution proceeds with lipophilic solvent and lipophilic/hydrophilic solvent mixtures. E.g. the root is first extracted with acetone and the thus formed extract is then applied to a silica gel column and the desired extract, in essence being similar do D-Ac1, is obtained by stepwise elution with dichloromethane and dichloromethane/methanol mixtures. On top of the root can be extracted with only a lipophilic solvent (e.g. chloroform) and the this obtained extract is subjected to further fractionation preferably using an polar interaction chromatography in the same way as above in this paragraph, which will also produce an extract similar to D-Ac1.

Another very useful approach for the fractionation of the extract of the invention is Sephadex LH-20 chromatography. Sephadex LH-20 is hydroxypropylated Sephadex G-25 (available from GE Healthcare Europe GmbH, Munzinger Strasse 5, D-79111 Freiburg, Germany). Eluents are organic solvent and mixtures thereof, like 70 % methanol/30 % dichloromethane. An extract prepared by further purification of D-Ac1 on LH-20 in this way was termed D-Ac12 and was found to exert strong sexual enhancing effect. LH-20 chromatography exploits hydrophobic interactions and therefore belongs to the category of hydrophobic interaction chromatography.

Another very specific further enriched extract of the invention is prepared by molecular size fractionation. In this embodiment a water extract is first prepared from the ground root of *Neobeguea mahafalensis*) by water extraction and then fractionating the solubilised extract according to molecular weight enriching the high molecular weight components of the water extract. (The drug can also first be extracted with lipophilic solvent such as dichloromethane prior to extraction with water and then isolating the high molecular weight components from the water fraction). In some embodiments of the invention the water extract is first subjected to partition with organic solvent partially soluble in water, such as ethylacetate in order to remove unwarranted materials (i.e. essentially using the procedure for preparation of RWExh and SWExh) and after phase separation the water phase is subjected to molecular size fractionation. The thus enriched high molecular weight components are dried or lyophilized and used in the preparation of the pharmaceutical of the invention. The preferred molecular weight (MW) of the components from *Neobeguea mahafalensis* enriched by the procedure is higher than 50 000 dalton, more preferable higher than 20 000 dalton, even more preferably higher than 10 000 dalton, even more preferably higher than 5000 dalton even more preferably higher than 3000, even more preferably higher than 2500, even more preferably higher than 2000 dalton, even more preferably higher than 1800 dalton, even more preferably higher than 1500 dalton, even more preferably higher than 1200 dalton, even more preferably higher than 1000 dalton and most preferably higher than 900 dalton. However, in other embodiments of the invention the most preferred molecular weight is 5000 dalton and higher. Procedures for molecular size fractionation are well known in the art and include (but are not limited to) molecular size exclusion chromatography, gel filtration, dialysis and ultrafiltration. In the case of gel filtration Sephadex G-25 chromatography (chromatography medium G-25 and G25 is *mutatis mutandis* herein meaning the same thing) is a preferred approach. However, Sephadex G-10, Sephadex G-15 or Sephadex G-50 can also be used. A specific example how to prepare the enriched extract RW1 according to this embodiment of the invention is given in Example 31. An example how to perform an assay of the sexually enhancing activity of RW1 is described in Example 32. The enriched extract prepared by molecular size fractionation constitutes

a particularly preferred enriched extract of the invention, with RW1 being most preferred. This is because the molecular size fractionation procedure removes unwarranted potentially toxic principles compared to other procedures for extraction, as well as it results in highly water soluble extracts that are highly suited for preparation of the orally administrable pharmaceuticals, food or dietary supplements of the invention. This embodiment of the invention can be combined with exhaustion of the water phase with lipophilic solvent that is preferably partially miscible with water (e.g. ethylacetate), essentially as described above for preparation of RWExh; the molecular size fractionation of the water extract resulting after solvent exhaustion results in highly enriched high molecular weight extracts that are low in content of toxic materials while their sexually enhancing activity is high. The reason that high molecular weight materials prepared as described herein is active is that high molecular weight components of *Neobeguea mahafalensis* have low molecular weight components attached maintaining the latter usually highly water insoluble components in water solution. Such extracts then also comprise useful enriched extracts of the invention. Moreover, extracts according to this embodiment of the invention (including the RW1 extracts), can be demonstrated to yield the characteristic mass-peak(s) of the invention upon application of mass-spectrometry, as well as they contain the R306 and R310 compounds.

RW1 extracts (when dissolved in water) can also be extracted with lipophilic solvents such as chloroform to produce a variant of the R2C extracts, as described above, which are also highly desired embodiments of the invention. A variant of R2C prepared in this fashion can be exchanged with R2C for any purpose of the present invention.

Yet other very important embodiment of the invention comprises the enriched extract of the drug prepared by reversed phase chromatography. Typically, the preparation of this type of enriched extract proceeds by preparing an initial extract of the drug by solvent extraction(s). The initial extract is then applied to a reversed phase column and elution proceeds with suited solvent mixture (usually organic solvent/water mixture), usually applied as a gradient with increasing proportion of organic solvent, or applied by stepwise elution with eluent containing increasing proportion of organic solvent. A very specific embodiment of such an enriched extract of the invention comprises enriched extract RB. RB is prepared by first essentially preparing extract RW (or an extract similar to RW) followed by preparation of extract R2C by chloroform-water partition (or applying similar procedure with hydrophobic solvent-water partition) using the ground root of *Neobeguea mahafalensis* as starting materials. Extract R2C (or corresponding) is then applied to LiChroprep RP-18 (Merck Chemical Co., Germany) at 30% acetonitrile in water (+ optional 0.1% trifluoroacetic acid) and then stepwise eluted with 40%, 50%, 60 % and 70% acetonitrile (+ optional 0.1% trifluoroacetic acid), preferably at a temperature of +5°C. The desired RB fractions will be eluted in the middle of the chromatogram and the eluate can be collected and subsequently dried removing the solvent obtaining a white powder. A prototypic column for preparing RB comprises a diameter of 33 mm and a length of 420 mm. In a prototypic preparation 190 mg of R2C (or corresponding) is dissolved in 15 ml acetonitrile followed by addition of 35 ml water. The turbulent solution obtained is applied to the column filled with LiChroprep RP-18 in 30% acetonitrile in water (+ optional 0.1% trifluoroacetic acid) at +5°C. Elution is performed with a step-wise gradient at a flow-rate of approximately 1.5 ml/min comprising (in order): 1 liter of 30% acetonitrile in water (+ optional 0.1% trifluoroacetic acid), then 1 liter of acetonitrile 40% (+ optional 0.1% trifluoroacetic acid), 1 liter of acetonitrile 50% (+ optional 0.1% trifluoroacetic acid), 1 liter of acetonitrile 60 % in water (+ optional 0.1% trifluoroacetic acid), and finally with 1.5 liter of 70 % acetonitrile in water (+ optional 0.1 % trifluoroacetic acid). The first 3084 ml are collected and freeze dried, yielding the extract RA. The subsequent 1104 ml are then collected and freeze dried, yielding the desired enriched the extract RB. The subsequent 1168 ml are also collected and freeze dried, yielding the extract RC. However, in another version of this embodiment of the invention the first 2500 ml are collected and freeze dried, yielding the extract RA. The subsequent 1600 ml are then collected and freeze dried, yielding the desired enriched the extract RB. The remaining eluent is also collected and freeze dried, yielding the extract RC. However, in yet another version of this embodiment of the invention the first 2500 ml are collected and freeze dried, yielding the extract RA. The subsequent 1800 ml are then collected and freeze dried, yielding the desired enriched the extract RB. The remaining eluent is also collected and freeze dried, yielding the extract RC.

In the procedure of the foregoing paragraph 0.1% trifluoroacetic acid can be present or excluded, still yielding essentially the same results.

In a wider sense extracts RA, RB and RC are herein defined the R2C extract subfractionated with reversed phase chromatography. R2C extract is thus applied to a reversed phase chromatographic set up and elution proceeds with a gradient (in a continuous or step-wise fashion) starting with solvents or solvent mixtures with low lipophilicity towards higher lipophilicity and RA, RB and RC is collected in the eluate with solvent of, respectively, low, medium and high lipophilicity.

In the preparation of RA, RB and RC by reversed phase chromatography any reversed phase chromatographic support may be used that includes an elution procedure used in liquid chromatography in which the mobile phase is significantly more polar than the stationary phase, the stationary phase comprising e.g. (but not limited to) a microporous silica-based material with chemically bonded alkyl chains of different length such as between 4 to 22 carbons e.g. C4, C8, C12, C14, C16, C18, C20, C22. The mobile phase can be composed of water and solvent mixtures, the solvents for example comprising (but not limited to) methanol, ethanol, acetonitrile. The pH of the eluant may be adjusted in a wide range from pH 1 - 14.

It is possible to collect a very narrow portion (i.e. small fraction) of the eluent from the reversed phase column in order to enclose any one of RA, RB or RC. Accordingly e.g. a RB fraction will contain only a few components (compounds) of the original R2C mixture and hence comprise a very enriched extract of the invention. Such an enriched RB extract can be assayed for sexual enhancing activity as described herein and is among the most desired extract of the invention. RB extracts can be combined with other RB extracts so as to afford a mix of sexually enhancing activity compounds (components) and likely also non-active compounds (components). Such mixes of extracts are herein also termed extracts and are also part of the invention.

In a wider sense extracts RA, RB and RC are herein also defined as a chloroform extract of root, sub fractionated with reversed phase chromatography which also gives these extracts. The RCH extract is thus applied to a reversed phase chromatographic set up and elution proceeds with a gradient (in a continuous or step-wise fashion) starting with solvents or solvent mixtures with low lipophilicity towards higher lipophilicity and RA, RB and RC is collected in the eluate with solvent of, respectively, low, medium and high lipophilicity. In a further wider sense in this embodiment of the invention RCH or R2C can be exchanged for any lipophilic solvent and/or hydrophilic solvent extract of the root, as well as extracts obtained by other chromatographic procedures or fractionation procedures, such as the D-Ac1 or dichloromethane (DCM) and/or acetone (01DG2) extracts of roots of Example 50 and still giving essentially the same end result producing the RA, RB and RC extracts.

Extracts similar to RA, RB, and RC, can be prepared using variations of the procedures described herein. E.g. the R2C extract which is to be applied to LiChroprep RP-18 may be exchanged for any another extract, e.g. even obtained from the water extract of the root of *Neobeguea mahafalensis* concentrated on resin such as Diaon SP-207 as detailed above. LiChroprep RP-18 may be exchanged by chromatographic support with corresponding properties and essentially still essentially leading to the RA, RB and RC extracts.

Components may be isolated by extensive fractionation of extracts and/or enriched extracts. An example of the isolation of 10 different components by extensive fractionation of the RB extract, along with their chromatographic and UV-absorbing properties, as well as their characterization according to characteristic mass-peaks, is given in Example 39 (listed under "Peak 1-10" of the table of Figures 8 and 9). The components comprising to any of Peak 1 - 10 of Example 39 comprise highly desired embodiments of the invention. (Herein respective Peak 1 - 10 of Example 39 are also termed RB1, RB2, RB3, RB4, RB5, RB6, RB7, RB8, RB9 and RB10). Such isolated components, and other components isolated in a similar fashion from *Neobeguea mahafalensis,* are highly desired embodiments of the invention as these components contain sexually activity enhancing principles; i.e. the chemical substance(s) of the invention in crude form.

Also substantially pure compounds may be isolated by extensive fractionation of *Neobeguea mahafalensis.* An example of the isolation of 10 different substantially pure compounds by extensive fractionation of the RB extract, along with their chromatographic and UV-absorbing properties, as well as their characterization according to characteristic mass-peaks, is given in Example 39 (listed under "Peak 1-10" of the table of Figures 8 and 9; i.e. RB1, RB2, RB3, RB4, RB5, RB6, RB7, RB8 , RB9 and RB10). The substantially pure compounds comprising to any of Peak 1 - 10 of Example 39 comprise highly desired embodiments of the invention. Such isolated substantially pure compounds, and other substantially pure compounds isolated in the similar fashion from *Neobeguea mahafalensis,* are highly desired embodiments of the invention as they contain sexually activity enhancing principles; i.e. the chemical substance(s) of the invention in crude form.

In the widest sense the most desired extract of the invention can be defined as a fraction obtained from the root of *Neobeguea mahafalensis* as the starting material for said fraction which is either lipid soluble or of high molecular weight. For the case of a lipid soluble fraction the fraction shall preferably be entirely soluble with an amount of at least 1 mg/ml, more preferably at least 2 mg/ml, even more preferably at least 4 mg/ml, even more preferably at least 8 mg/ml, even more preferably at least 12 mg/ml, even more preferably at least 16 mg/ml, and most preferably at least 18 mg/ml in sun flower oil and/or in octan-1-ol at the temperature 20°C, for said fraction to be regarded as being lipid soluble. By a lipid soluble fraction is with the same meaning herein intended a lipid soluble extract. For the case of a high molecular weight fraction the molecular weights of its components shall be higher than 50 000 dalton, more preferable higher than 20 000 dalton, even more preferably higher than 10 000 dalton, even more preferably higher than 5000 dalton, even more preferably higher than 3000 dalton, even more preferably higher than 2500 dalton, even more preferably higher than 2000 dalton, even more preferably higher than 1800 dalton, even more preferably higher than 1500 dalton, even more preferably higher than 1200 dalton, even more preferably higher than 1000 dalton and most preferably higher than 900 dalton. By a high molecular weight fraction is with the same meaning herein intended a high molecular weight extract.

The solubility of the R2C extract was found to be 21 mg/mL in oil and 30 mg/mL in octan-1-ol at 20°C (Example 51). The solubility of D-Ac1 extract was moreover found to be higher than 20 mg/mL in sun flower oil.

For sake of information DNA was isolated from leaves of *Neobeguea spp.* (Muellner AN et al.: Molecular phylogenetics of Meliaceae (Sapindales) based on nuclear and plastid DNA sequences. Am. J. Botany. 2003, 90(3): 471-480). Although DNA is of high molecular weight, DNA is obviously not the source of the sexual enhancing effect of the present invention and DNA from *Neobeguea mahafalensis* is therefore specifically excluded from the high molecular

weight material (fraction) (or for that sake any other useful fraction or extract) of the present invention. Moreover, as leaves of *Neobeguea mahafalensis* do not contain sexual enhancing effect, leaves of *Neobeguea spp.* are specifically excluded from the present invention.

Moreover, in the widest sense the most desired extract of the invention is defined as a fraction obtained from the root of *Neobeguea mahafalensis* as the starting material for said fraction which is capable of inducing a sexual enhancing effect *(vide supra* for the definition of sexual enhancing effect). However, for the sake of the present invention one can substitute the assay of a sexual enhancing effect by the assaying of the presence of the chemical substance of the invention *(vide supra* for the definition of the compound of the invention as well as in the claims for the claimed chemical compounds of this patent). In particular an extract that contains at least (by weight) 0.05 %, more preferably at least 0.1 %, even more preferably at least 0.15 %, even more preferably at least 0.2 %, and even more preferably at least 0.25 %, and most preferably at least 0.3 % of the compound of the invention is an extract comprised by the invention. Even more specifically the extract containing at least (by weight) at least 0.05 %, more preferably at least 0.1 %, even more preferably at least 0.15 %, even more preferably at least 0.2 %, and even more preferably at least 0.25 %, and most preferably at least 0.3 % of R306 is comprised by the invention. Moreover, compound R306 can be substituted with R310 (assaying the sum of its tautomeric forms) for this embodiment of the invention. For the sake of the present invention whenever term "selection of a fraction having sexual enhancing effect" this term also includes the assay of the presence and content of the compound of the invention as defined in this paragraph. For an example how to assay an extract for its presence and contents of R306 see Example 48.

Investigations preceding to the present studies showed that many water soluble compounds of small molecular weight were present in water extracts of *Neobeguea mahafalensis.* Many of these were flavonoids and one of those identified chemically by use of HPLC and NMR was epicatechin. However, in the lipid soluble fractions and high molecular weight fraction epicatechin was removed. Therefore, in some embodiments of the invention, the extract, fraction or composition of the invention does not comprise substantial amounts of epicatechin, i.e. preferably such preparations containing (by weight) less than 5 %, more preferably less than 2 %, more preferably less than 1 %, more preferably less than 0.5 %, more preferably less than 0.2 % and most preferably less than 0.1 % of epicatechin.

Earlier studies have identified compounds pseudrelone $A_2$, β-amyrin, stigmasterol, neobeguin, sapelin C, sapelin E acetate, grandifoliolenone, methyl angolensate, mexicanolide, khayasin, leandreanin A, leandreanin B and leandreanin C in samples from *Neobeguea mahafalensis* and *Neobeguea leandreana* (29, 38, 39, 46). Many of these compounds were also identified in crude fractions and extracts prepared herein, and they are there less desirous as they seem to have negative influence on sexual activity. However, many of these compounds were specifically removed from many of the fractions and extracts of the present invention; in particular they were removed from the high molecular weight fractions of the invention, as well as many of the fractions prepared by chromatography. Therefore, in some embodiments of the invention, the extract, fraction or composition of the invention does not comprise substantial amounts of any one of pseudrelone $A_2$, β-amyrin, stigmasterol, neobeguin, sapelin C, sapelin E acetate, grandifoliolenone, methyl angolensate, mexicanolide, khayasin, leandreanin A, leandreanin B and leandreanin C, i.e. preferably such preparations containing (by weight) less than 5 %, more preferably less than 2 %, more preferably less than 1 %, more preferably less than 0.5 %, more preferably less than 0.2 % and most preferably less than 0.1 % of any one of pseudrelone $A_2$, β-amyrin, stigmasterol, neobeguin, sapelin C, sapelin E acetate, grandifoliolenone, methyl angolensate, mexicanolide, khayasin, leandreanin A, leandreanin B and leandreanin C.

On top of this the high molecular weight extracts and lipophilic extracts of the invention have essentially removed salts of sodium, potassium and magnesium. As these salts can have negative effect on particular pharmaceutical preparations, e.g. solutions in oil which then do not become fully solubilised which is not desirous in many cases, therefore, in some embodiments of the invention, the extract, fraction or composition of the invention does not comprise substantial amounts of any one of the ions of sodium, potassium or magnesium, i.e. such preparations containing (by weight) less than less than 1 %, more preferably less than 0.5 %, more preferably less than 0.1 %, more preferably less than 0.05 % and most preferably less than 0.01 % of any one of sodium, potassium or magnesium.

Moreover, some embodiments of the invention, the extract, fraction or composition of the invention does not comprise one or more of the following: a water or ethanolic extract from *Cedrelopsis grevei* or even any other extract from *Cedrelopsis grevei* [Cf. the patent JP2005-213202]. This is because for the sake of the present invention extracts from *Cedrelopsis grevei* are of no benefit or even cause negative action on sexual activity.

Applying extensive fractionation substantially pure compounds R306 and R310 can be isolated from the root of *Neobeguea mahafalensis.* R306 and R310 can be isolated using the procedure described in Example 41. R306 may also be isolated using the procedure described in Example 42. However, other fractionation methods also lead to R306 and R310 and they can also be isolated from stem bark of *Neobeguea mahafalensis,* as well as any other part of *Neobeguea mahafalensis.* R306 and R310 are characterized from their characteristic mass-peaks, their molecular composition, their UV-absorbing and chromatographic properties, as described herein below and in Examples 41 and 42, as well as by their sexual enhancing effect as exemplified in Example 43, and as described further below, as well as by their NMR spectral characteristics (see Example 47).

Herein a compound is regarded as substantially pure when it (by weight) is at least 10%, more preferable at least 20%, even more preferable at least 30%, even more preferable at least 40%, even more preferable at least 50%, even more preferable at least 60%, even more preferable at least 70%, even more preferable at least 80%, even more preferable at least 90%, even more preferable at least 95%, even more preferable at least 98%, and most preferably at least 99% pure. (E.g. by 99 % pure means in this context that a single compound exists in a form where it takes at least 99% of the weight, while the remainder comprises other material(s)). In order to determine whether or not a compound is substantially pure it can be subjected to reversed phase chromatography preferably using a hydrophobic interaction column, preferably the hydrophobic interaction column being derivatized with C18 groups such as Lichrospher C18, or using a chiral interaction column such as Chirobiotic V. The compound is according to this determination process solubilised in a suited solvent such as isopropanol/water and injected onto the chromatography column and then the column is eluted with suited solvent, such as isopropanol/water mixture(s), while monitoring continuously the UV absorbance of the eluent with a photo diode detector covering wavelengths at least between 205 - 350 nm. The eluent is collected until no materials is any longer eluted from the column; the fractions containing the peak corresponding to the compound which purity is about to be determined is specifically collected (starting immediately after a deflection of the UV absorbance from the solvent background UV absorbance baseline can be observed, and ending just before the UV absorbance returns to the solvent background UV absorbance base line) and combined into a first fraction while the remainder of the fractions are combined into a second fraction. The fractions are then lyophilized and the residual materials is weighed. The purity (in %) is calculated as follows: 100*(weight of residual materials in first fraction)/((weight of residual materials in first fraction)+(weight of residual materials in second fraction)). (In formula of previous sentence sign "*" is intended to mean a multiplication operator). It is preferred that both a C18 and chiral column is used in separate assays to ascertain that the chromatographic procedure is able to separate of all compounds of the sample; the purity is then taken as the lowest one of the values for purity obtained by using the C18 and chiral columns. Methods for chromatography for this aspect of the invention are essentially as given in Examples 41 and 42.

In a simpler variant herein a compound is regarded as substantially pure when its is at least 10%, more preferable at least 20%, even more preferable at least 30%, even more preferable at least 40%, even more preferable at least 50%, even more preferable at least 60%, even more preferable at least 70%, even more preferable at least 80%, even more preferable at least 90%, even more preferable at least 95%, even more preferable at least 98%, and most preferably at least 99% pure as can be estimated from the UV absorbance being observable at 210 nm during reversed phase chromatography using a hydrophobic interaction column derivatized with C18 groups such as Lichrospher C18 or a chiral interaction column such as Chirobiotic V. The compound is in this version of the procedure solubilised in a suited solvent such as isopropanol/water and injected onto the column and the column is subsequently eluted with suited solvent, such as isopropanol/water mixture(s), while monitoring the eluent with a UV detector at the wavelength 210 nm. The area under the curve of the UV absorbing peak corresponding to the compound which purity is to be checked is obtained, starting immediately after a clear deflection from the UV absorbance of the absorbance of the solvent base line is observed and ending immediately before the return of the UV absorbance to the absorbance of the solvent base line. The area under the curve for all other UV absorbing peaks are also obtained in the same way and these areas are summed up together. The purity (in %) is thereafter calculated as follows:

100*(area under curve for the UV absorbance peak corresponding to the compound

of interest)/((area under curve for the UV absorbance peak corresponding to the compound of interest)+(sum of areas under absorbance peaks for all other UV absorbing peaks)). (In formula of previous sentence sign "*" is intended to mean a multiplication operator). It is preferred that both a C18 and chiral column is used to ascertain separation of all compounds in the sample under investigation; the purity is then taken as the lowest one of the values for purity obtained by using the C18 and chiral columns. Methods for chromatography for this aspect of the invention are essentially as given in Examples 41 and 42.

The structures of a substantially pure compound isolated from *Neobeguea mahafalensis* can be determined using NMR and/or X-ray crystallography, using procedures well know in the art. In addition high-resolution mass-spectrometry is useful to give ideas in the structural determination of the substantially pure compound of the invention. Elucidating the structures of substantially pure compounds is a highly desired embodiment of the invention as many of these compounds comprise similar sexual enhancing activity as R306 and R310. Moreover, many compounds having structural similarity to R306 and R310 exist in *Neobeguea mahafalensis* as well as in other species, in particular many other species of Meliaceae, which also have similar sexual enhancing activity as R306 and R310, as well as they can be synthesized (e.g. by using so called semi-synthesis) and which are also comprised by the invention.

Using high resolution time of flight mass spectrometry R306 yielded a mass of 699.2991 ($M+H^+$) which corresponded to the unprotonated summary formula $C_{37}H_{46}O_{13}$. Using 600 MHz NMR (see Example 47) the structure of R306 was determined to be as follows:

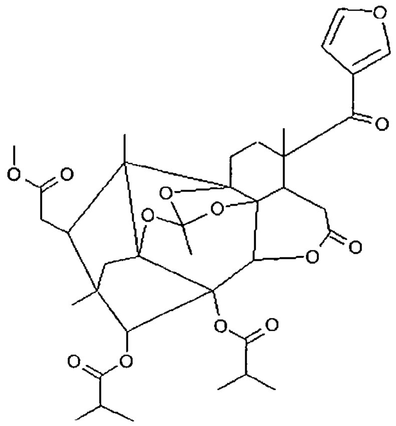

R306, which represents a novel structure, has been found to be surprisingly potent in eliciting a sexual enhancing effect as well as having a surprisingly long-lasting effect (see Example 43). The chemical substance comprising the structure of R306 is therefore a highly desired chemical substance of the invention.

Using high resolution time of flight mass spectrometry R310 yielded a mass of 715.2953 ($M+H^+$) which corresponded to the unprotonated summary formula: $C_{37}H_{46}O_{14}$.

It is thus contemplated that R310 is highly structurally similar to R306 because R310 is having only one additional oxygen atom compared to R306. Also R310 has been found to be surprisingly potent in eliciting a sexual enhancing effect as well as having a surprisingly long-lasting effect (see Example 43). Interestingly R310 has the same summary formula as neobeguin (38) but the NMR spectrum of R310 is distinctly different from that of neobeguin previously isolated from *Neobeguea mahafalensis* (38). Therefore R310 is distinct from neobeguin. Moreover, both the NMR and chromatographic properties of R310 indicates that it exists in slowly interconverting tautomeric forms. Due to its different molecular mass R310 is also different from pseudrelone $A_2$, that was also previously isolated from *Neobegacea mahafalensis* (38,39). Moreover, neither neobeguin nor pseudrelone $A_2$ are known to share the same striking sexual enhancing effect of R310. Thus, accordingly R310 is also a novel highly desired chemical substance of the invention.

Using 600 MHz NMR the contemplations of the preceding paragraph proved to be entirely correct as the structure of the two tautomeric forms of R310, **R310A** and **R310B,** were determined (Example 47); the structure of R310A thus being as follows:

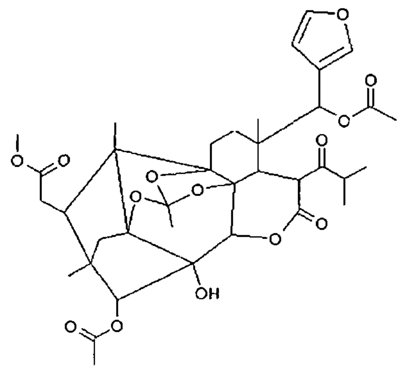

And the structure of R310B being as follows:

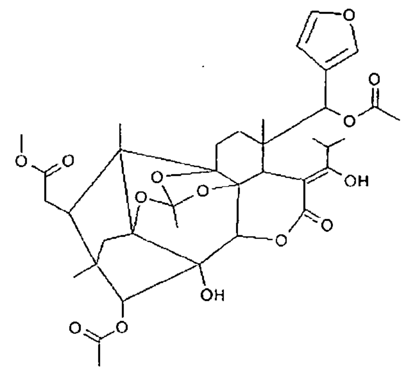

In the following term R310 will collectively mean both or one or the other of the structures R310A and R310B, as well as that R310A and R310B may represent tautomeric (spontaneously interconverting) molecular forms of R310.

It is obvious that structures R306, R310A and R310B can be chemically modified into new structures using hydrolysis and esterification reactions. Processes for performing such hydrolyzing and esterifying reactions can follow the teachings of Guex and Tamm in reference (40) directed to the busseins which are limonoids with a closely similar structure as the chemical substances of the present invention.

Thus, using an acetylation procedure essentially as taught in ref 40, p. 530, Experimenteller Teil 2 for acetylation of bussein A (or any another suited acetylation procedure) the acetylation of R310B with acetic anhydride (e.g. with base like pyridine or other suited base and solvent) yields compound R310B1 as follows:

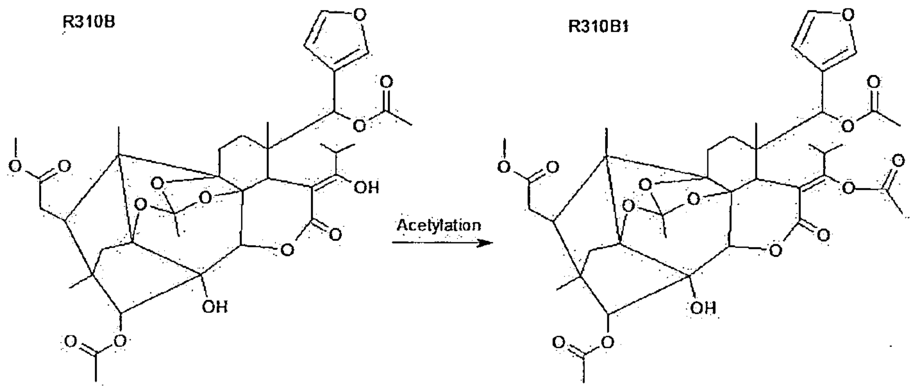


Moreover, using an acylation procedure essentially as taught in ref 40, p. 530, Experimenteller Teil 3 for acylation of bussein A, acylation of R310B with chloroaceticanhydride (e.g. with base like pyridine or other suited base and solvent) yields compound R310B2 as follows:

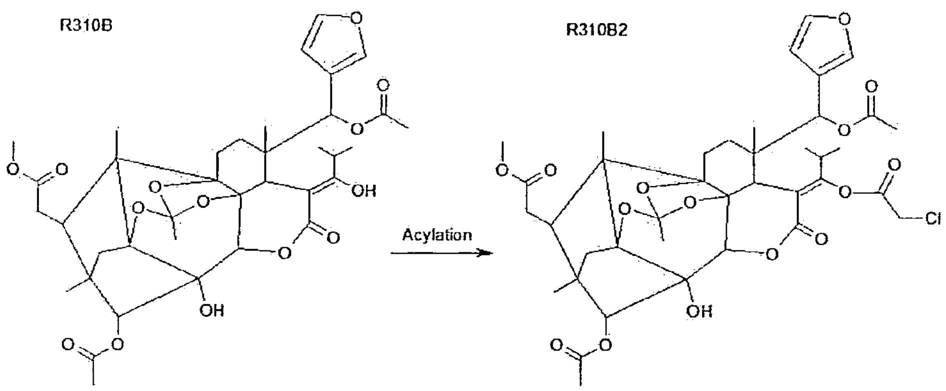


Furthermore, using an acylation procedure essentially as taught in ref 40, p. 530, Experimenteller Teil 4 for acylation of bussein A, acylation of R310B or R310A with acetylchloride (e.g. with pyridine additive in suited solvent like chloroform) yields compound R310A3 and R310B3 as follows:

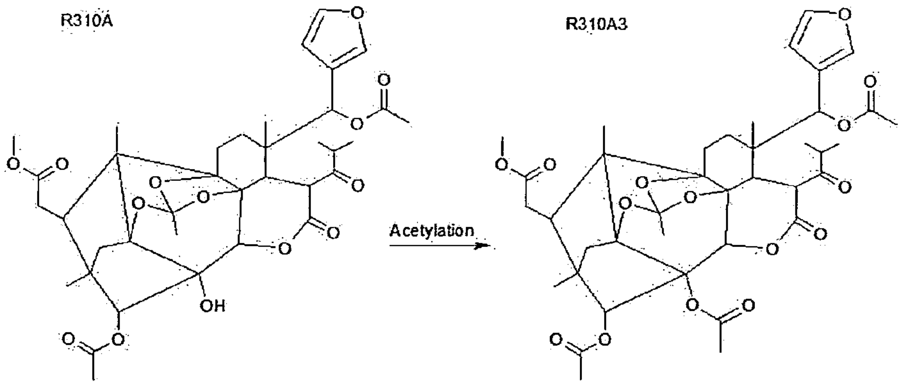


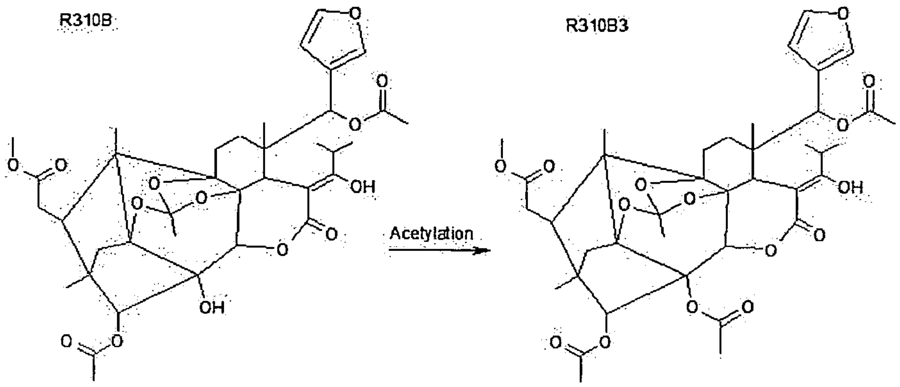


Even furthermore, using an acylation procedure essentially as taught in ref 40, p. 530-531, Experimenteller Teil 5 for acylation of bussein A, acylation of R310A or R310B with chloroacetylchloride (e.g. with pyridine additive in suited solvent like chloroform) yields compound R310A4 and R310B4 as follows:

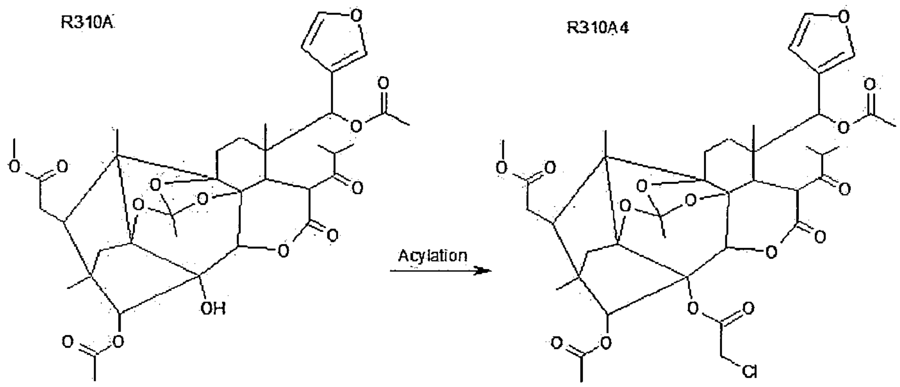

R310B R310B4

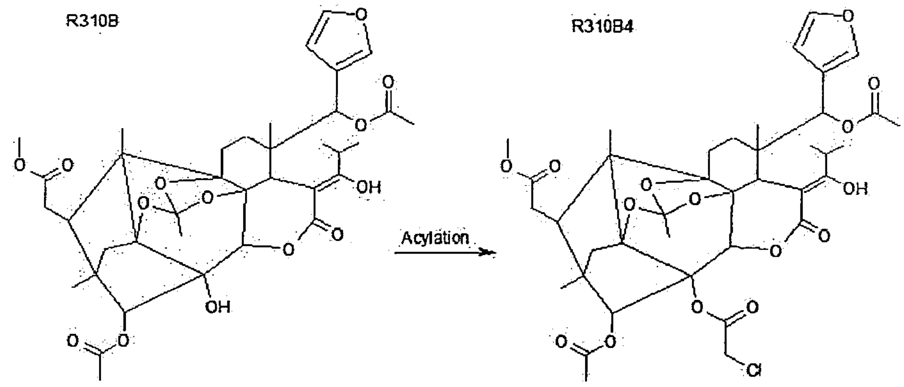


Even furthermore, applying acid hydrolysis essentially as taught in ref 40, p. 531, Experimentelller Teil 6 and 7 for hydrolysis of busseins A and B, acid hydrolysis of R310A or R310B (e.g. $H_2SO_4$ in methanol or [water-complexed] $H_2SO_4$ in tetrahydrofurane) yields among several other products compound R310A5 or R310B5 as follows:

R310A R310A5

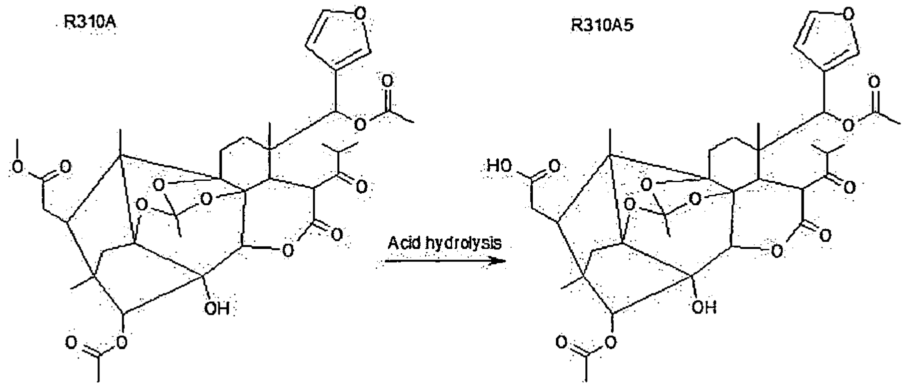


R310B R310B5

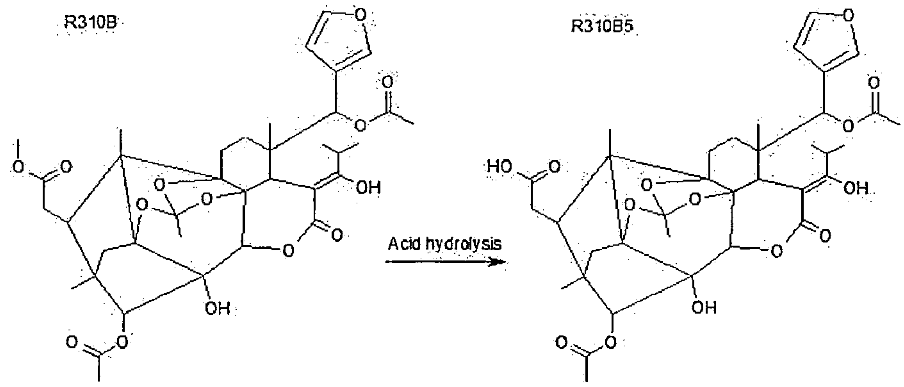


Moreover, acid hydrolysis in the way taught in ref 40, p. 531, Experimenteller Teil 6 and 7 for hydrolysis of busseins A and B (e.g. $H_2SO_4$ in methanol or [water-complexed] $H_2SO_4$ in tetrahydrofurane) gives for R306 among several other products compound R306E as follows:

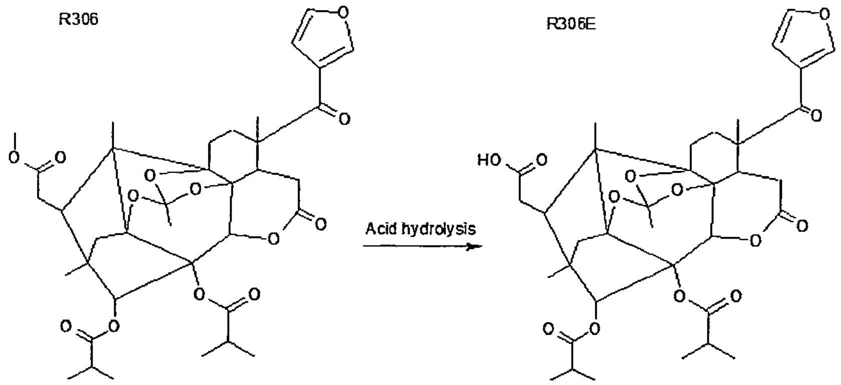


Furthermore, alkaline hydrolysis as taught in ref 40 p. 531-532, Experimenteller Teil 8 and 9 for hydrolysis of busseins A and B, the alkaline hydrolysis of R310B (e.g. $NH_3$ in methanol or NaOH in methanol/$H_2O$) yields among several other products compound R310B6 as follows:

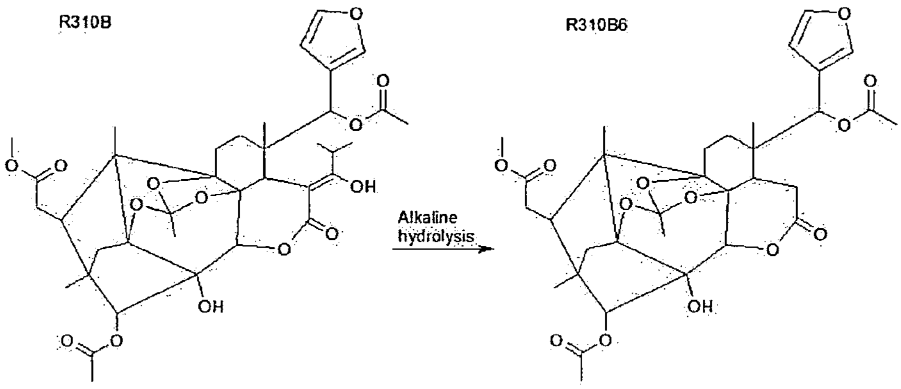


(The type of reaction for alkaline hydrolysis of R310B by cleavage of the double bond of the 1-hydroxy-2-methylpropylidene group, leading to the forming of R310B6 is confirmed in Bernasconi et al., J. Am. Chem. Soc. Vol. 103, No. 16, 1981, p. 4852, Scheme I and under "Mechanism of hydrolysis in basic solution", hydrolytic cleavage of activated olefins; the reference Bernasconi et al., J. Am. Chem. Soc. Vol. 103, No. 16, 1981, p 4850-4860 and methods therein).

Alkaline hydrolysis as taught in ref 40 p. 531-532, Experimenteller Teil 8 and 9 for hydrolysis of busseins A and B, alkaline hydrolysis of R310B (e.g. $NH_3$ in methanol or NaOH in methanol/$H_2O$) yields among several other products also compounds R310B7 and R310B8 as follows:

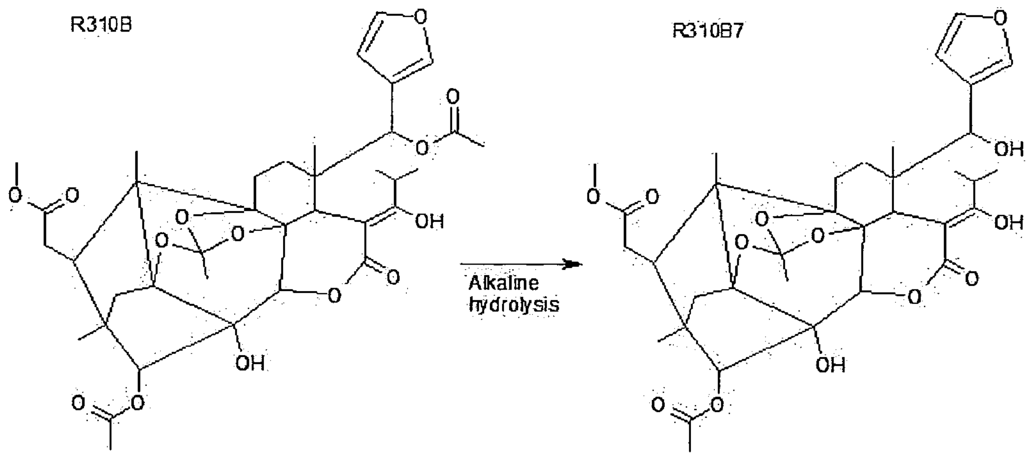

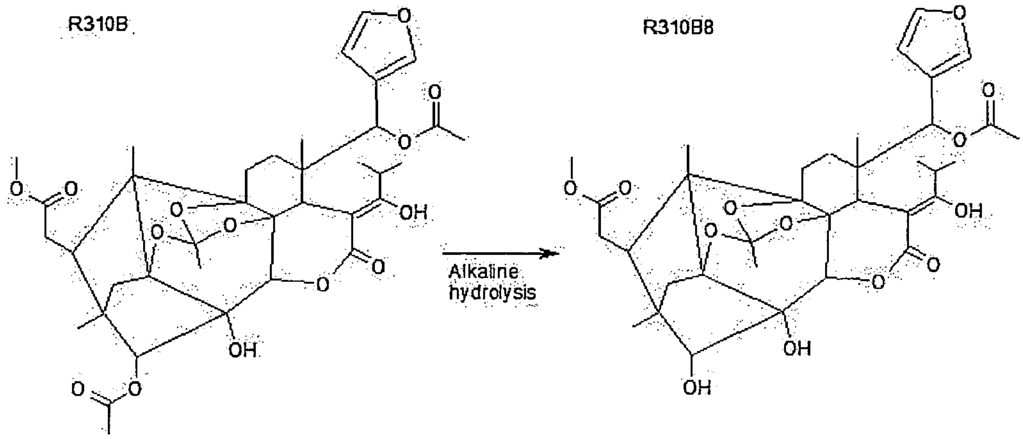


as well as hydrolysis products where two or more of the acetyl and the 1-hydroxy-2-methylpropylidene groups are hydrolysed, including the hydrolysis products R310B9:

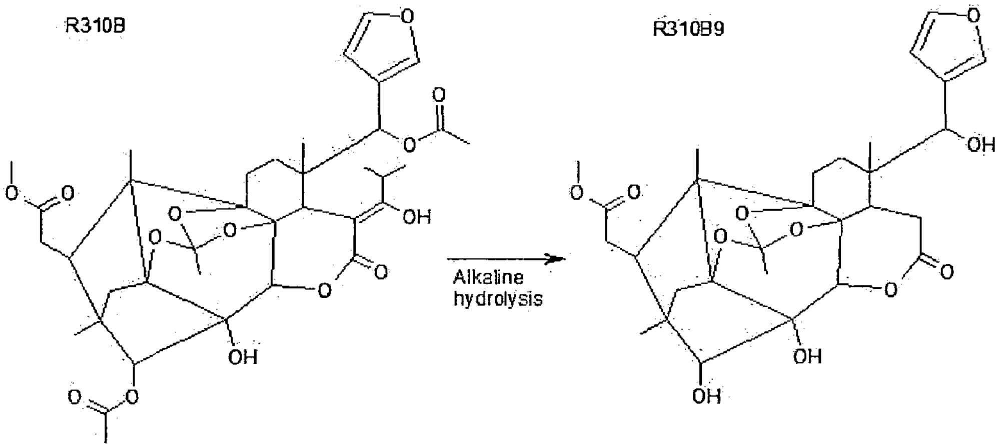


as well as the complete hydrolysis product R31 OB 11:

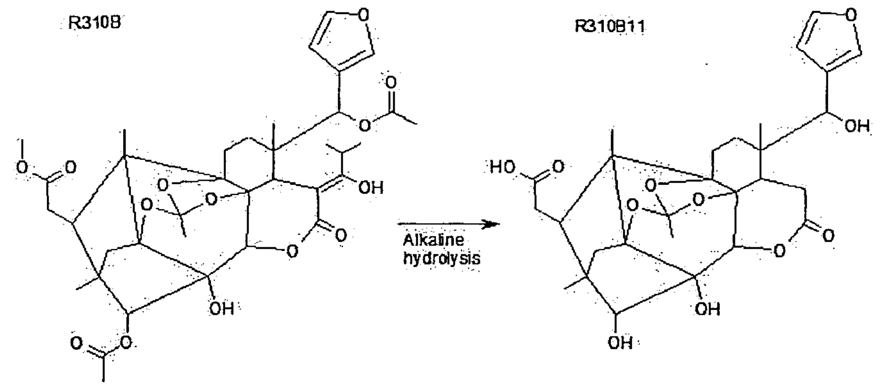


Application of alkaline hydrolysis in a similar way as taught in ref 40, 531-532, Experimenteller Teil 8 and 9 for hydrolysis of busseins A and B, the alkaline hydrolysis of R306 (e.g. $NH_3$ in methanol or NaOH in methanol/$H_2O$) yields among other products compounds R306AB, R306BA and R306D as follows:

R306 R306AB

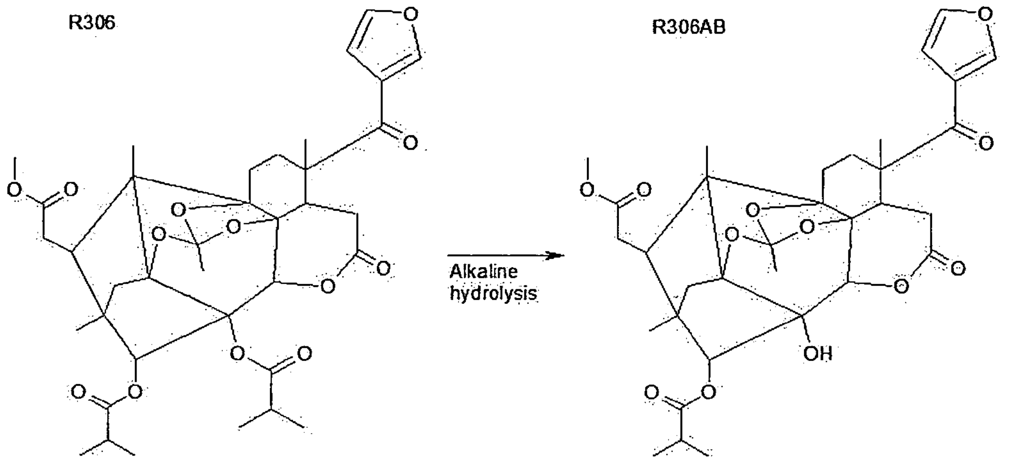


R306 R306BA

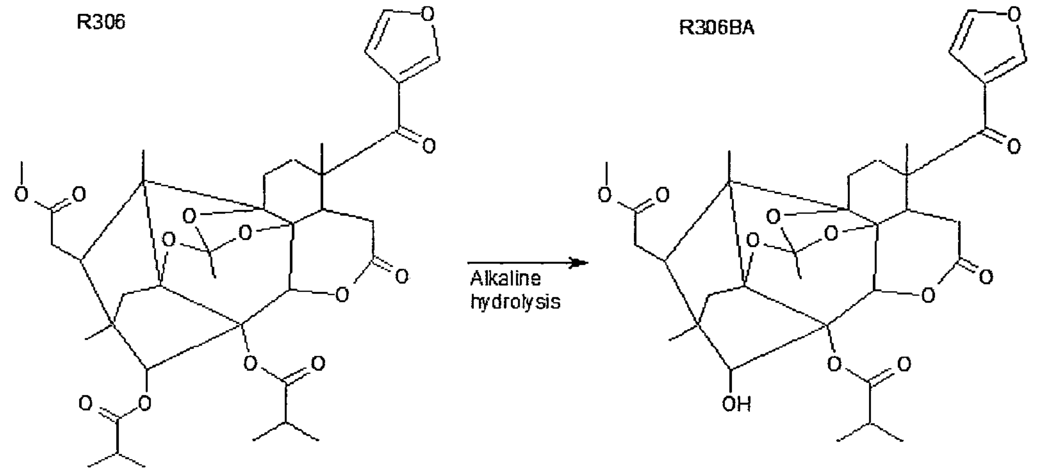


R306 R306D

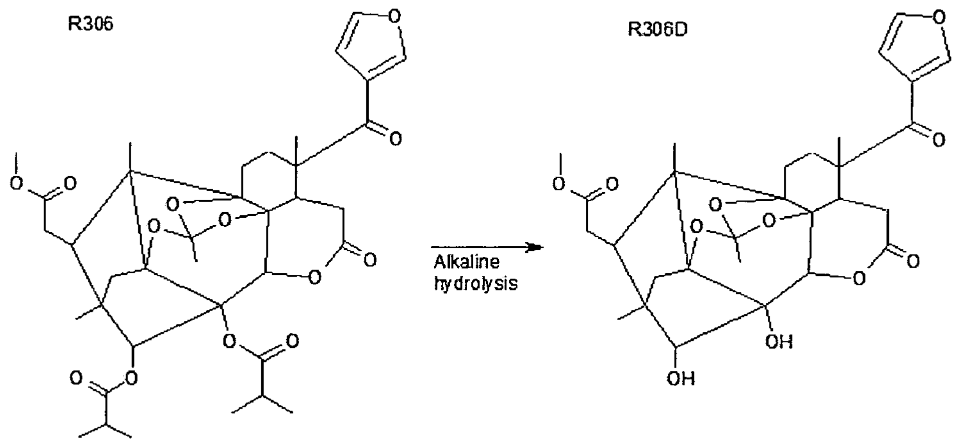


On top of this compound R310B9 can be oxidized to yield R306D:

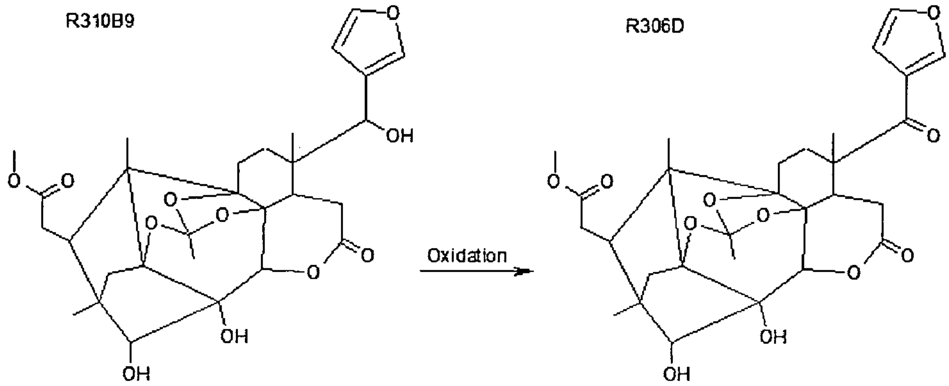


Oxidation of R310B9 to yield R306D can be afforded e.g. using Dess-Martin oxidation (Dess, DB, Martin, JC: J. Am. Chem. Soc. 1991, 113, 7277-7287); see Miller et al. J. Am. Chem. Soc. 2006, 128 (51), 17057-17062 for analogous case. The reaction can also be afforded using periodoacid, $HIO_4$, as oxidizing agent; see Turkish J Chemistry 2005, 29(6), 635-639 for analogous case, or mangan oxide, $MnO_2$, as oxidizing agent; see Synlett. 2005, (8), 2826-2828 for analogous case.

R310B10 can then be acylated with isopropionic anhydride (see Dálaigh et al., Org. Biomol. Chem. 2006, 4, 2785-2793 and MacKay and Vedejs, J. Org. Chem, 2004, 69, 6934-6937 for the analagous cases) to give R306, one of the most desired compounds of the invention.

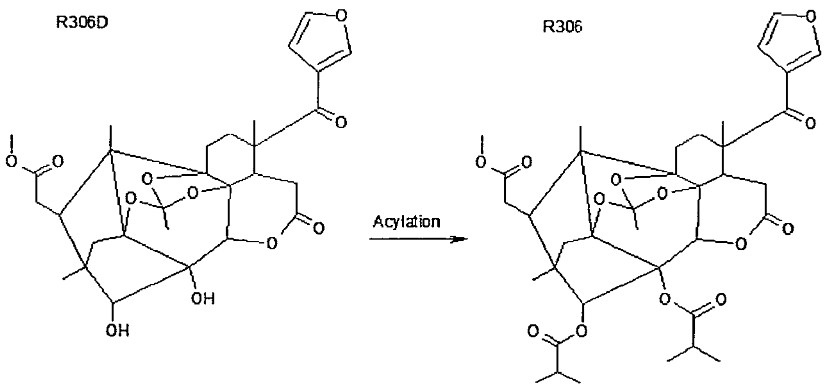


Using an acylation procedure essentially as taught in ref 40, p. 530, Experimenteller Teil 5 for acylation of bussein A, acylation of R310B9 with acetyl chloride (e.g. with pyridine additive in suited solvent like chloroform) or by acetylation with acetic anhydride yields among other compounds R310B12 as follows:

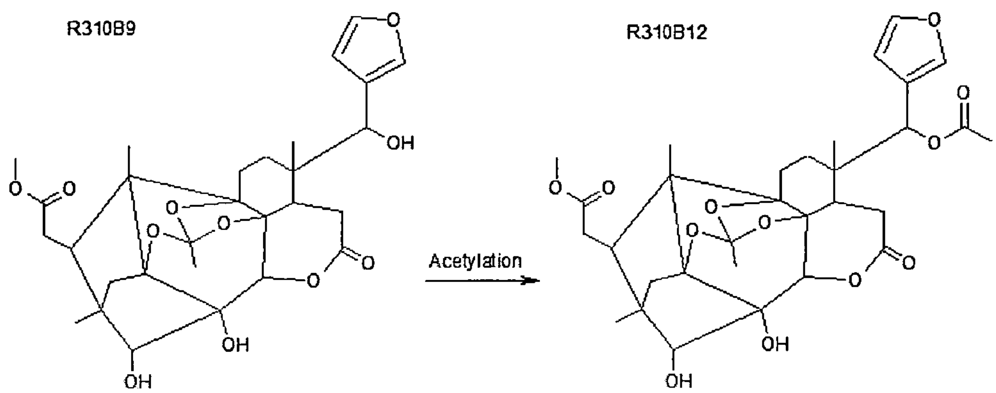


Using an acylation procedure essentially as taught in ref 40, p. 530, Experimenteller Teil 5 for acylation of bussein A, acylation of R310B9 with acetyl chloride (e.g. with pyridine additive in suited solvent like chloroform) or by acylation with chloroacetic anhydride yields among other compounds R310B10 as follows:

R310B9 R310B10

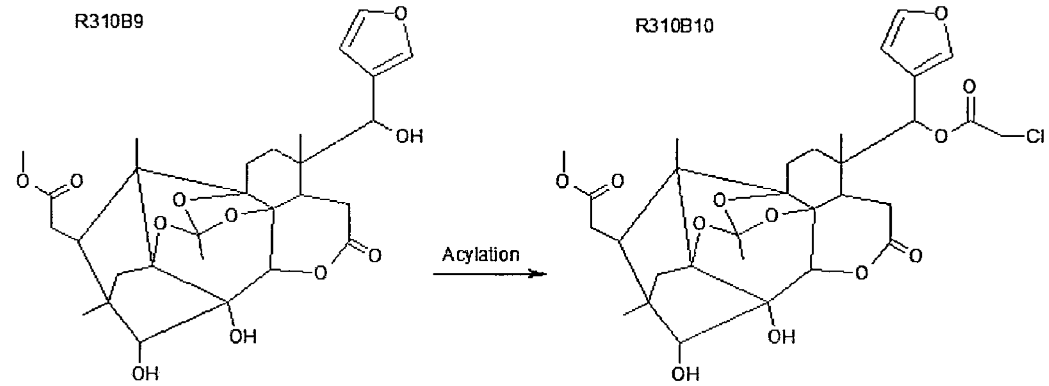


Many other derivatives and variants of the compound of the invention can be produced along the lines of the above procedures substituting acetic anhydride, chloroaceticanhydride, acetylchloride or chloroacetylchloride with other acylation reagent such as other anhydrides of other carboxylic acids or other alkylchloroformates.

On top of this, processes for chemical methylation is well known in the art and can be afforded using methylation reagents like methyl iodide $CH_3I$ (see Master et al., Bioorganic & Medicinal Chemistry Letters 13 (2003) 1249-1251), dimethyl carbonate (see Ouk et al., Green Chemistry, 2002, 4, 431-435), and dimethyl sulfate (see Tamburlin-Thumin et al, Eur. J. Med. Chem. 36 (2001) 561-568). Using methylation with such reagents along such principles methylation products can be formed from many of the compounds of the invention, e.g. R310B can yield R310B13, R310B14 and R310B15, as follows:

R310B R310B13

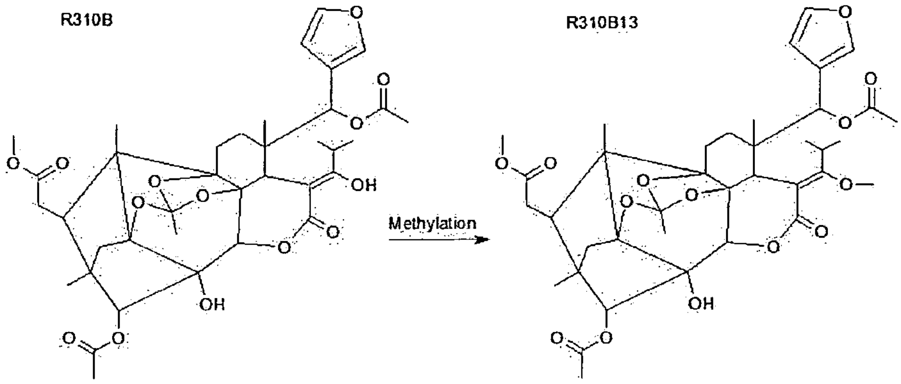


R310B R310B14

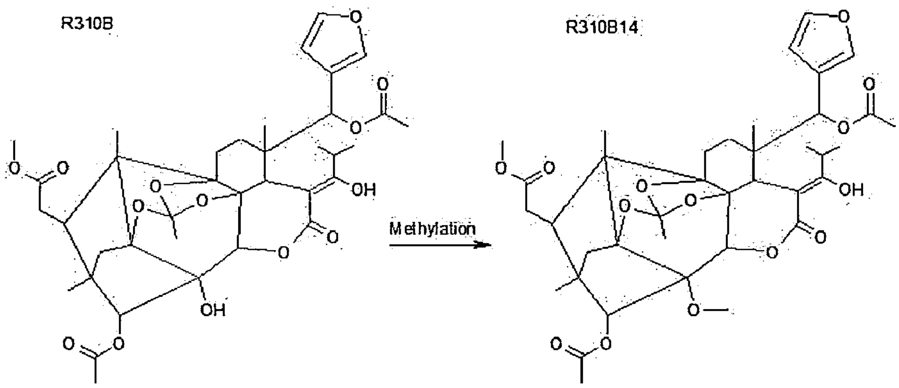

R310B R310B15

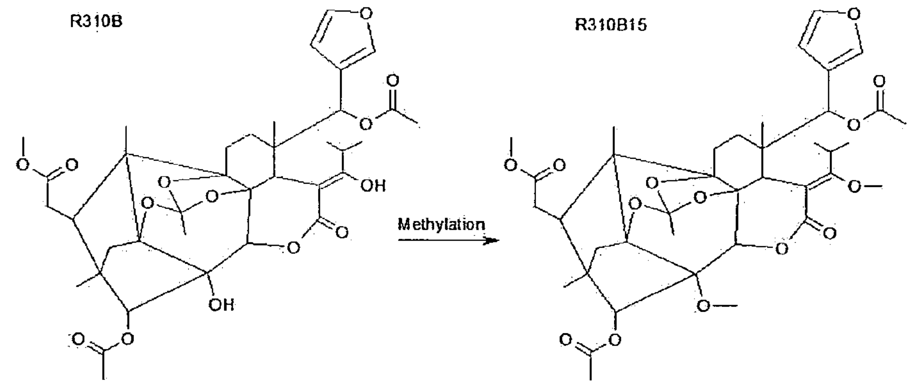


As well as methylation products can be formed from R310B9 yielding among other products R310B16, R310B17 and R310B18, as follows:

R310B9 R310B16

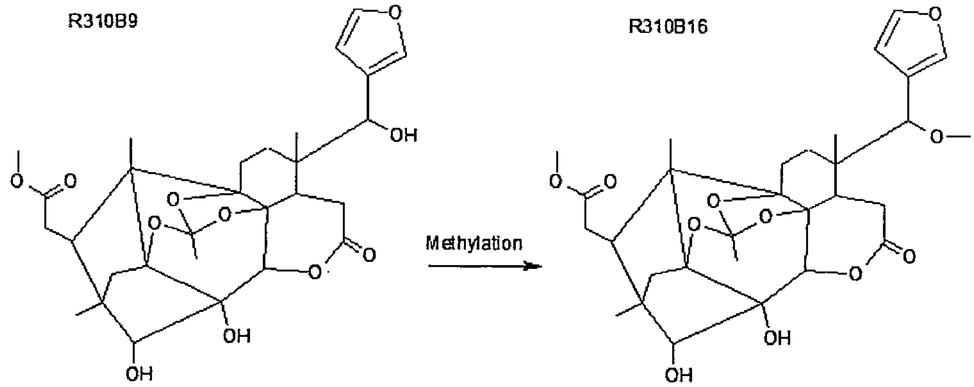


R310B9 R310B17

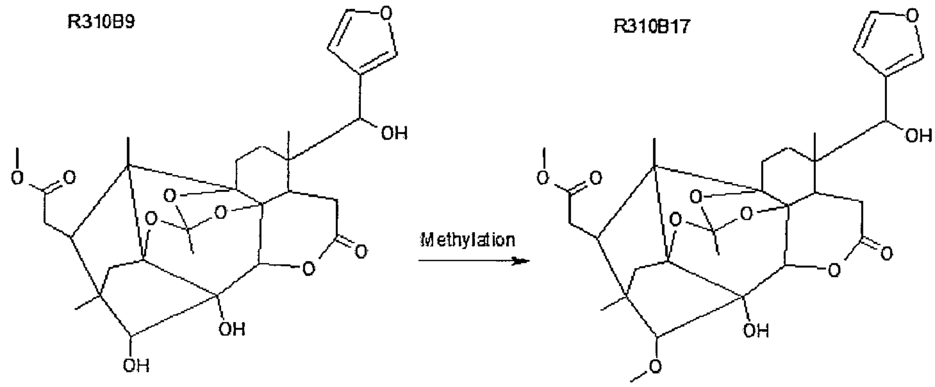


R310B9 R310B18

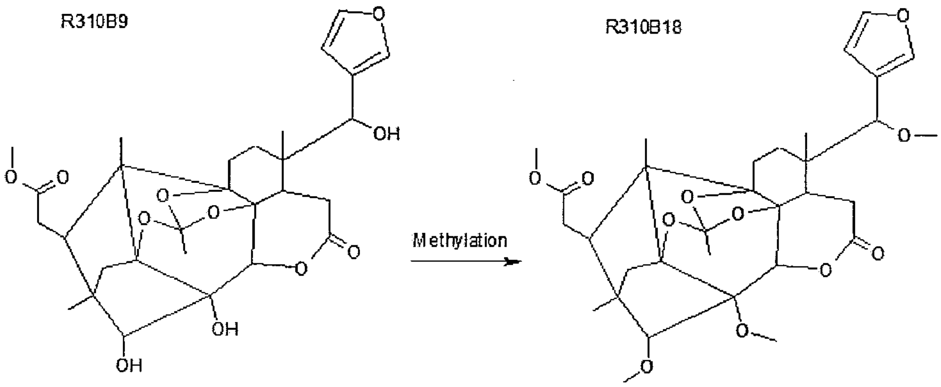

The introduction of other functions is possible as well, using alkylation according to the same or similar principles.

Processes for esterification of carboxylic function are also well known in the art. For example compound R306E can yield R306F by esterification with ethanol:

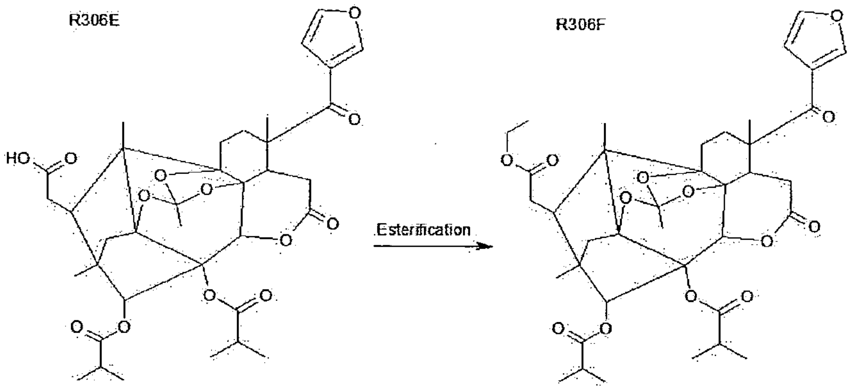


This reaction can e.g. be afforded with dicyclohexylcarbodiimide and dimethylaminopyridine as catalyst in suited solvent like dichloromethane or dimethylformamide; i.e. Steglich esterification (see B. Neises, W. Steglich: Simple Method for the Esterification of Carboxylic Acids, Angew. Chem. Int. Ed., 1978, 17, 522-524. The corresponding esterification with other alcohols is possible as well.

In pharmacology and medicinal chemistry it is well known that chemical substance with structural similarities often cause similar pharmacological actions. In elaborate experimentation involving extensive subfractionation of extracts from *Neobeguea mahafalensis* many differing fractions were found to have similar sexual enhancing effect as R306 and R310. It is accordingly thus obvious that there are many compounds in *Neobeguea mahafalensis* that bear structural similarity with R306 and/or R310A and/or R310B, and which also have sexual enhancing effects. Moreover, as disclosed above simple chemistry allows the preparation of new derivatives from R306, R310A and R310B having closely similar pharmacological and chemical properties. On top of that, in view of the general chemistry of limonoids among the Meliaceae family (45) it is obvious that comprised by the invention is a chemical substance (chemical compound) having the chemical structure III:

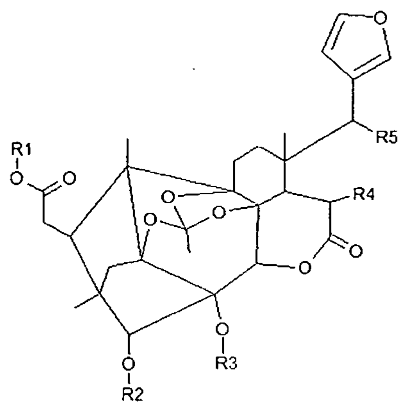

wherein

R1 is a substituent having from one to 30 atoms of any type(s), more preferably one to 16 atoms, more preferably one to 12 atoms and most preferably one to 10 atoms, with hydrogen, oxygen, carbon, sulphur, nitrogen, phosphorous and halogen atoms being preferred; the R1 substituent being a hydrogen and/or linear, branched and/or cyclic structure; the R1 substituent preferably comprising of from zero to 10 heavy atoms, even more preferably zero to 6 heavy atoms, even more preferably zero to 4 heavy atoms, even more preferably zero to 3 heavy atoms, and most preferably zero to 2 heavy atoms; most preferably the substituent being comprised of any one of hydrogen, methyl, ethyl, propyl, isopropyl, butyl, isobutyl, cyclopropyl, cyclopropenyl, cyclobutyl, cyclopentyl, cyclohexyl, alkyl, cyclic alkyl, halogenated alkyl, halogenated cyclic alkyl, propenyl, halogenated propenyl, alkenyl, halogenated alkenyl, halogenated cyclic alkenyl, alkynyl, halogenated alkynyl, aryl, halogenated aryl, with methyl being most preferred,

and wherein R2 is a substituent having from one to 30 atoms of any type(s), more preferably one to 20 atoms, and most preferably one to 16 atoms, with hydrogen, oxygen, carbon, sulphur, nitrogen, phosphorous and halogen being preferred; the R2 substituent being a hydrogen or a linear, branched and/or cyclic structure; the R2 substituent preferably comprising of from zero to 10 heavy atoms, even more preferably zero to 8 heavy atoms, even more preferably zero to 6 heavy atoms, even more preferably zero to 5 heavy atoms; most preferably the substituent being composed of any one of hydrogen, methyl, ethyl, propyl, isopropyl, cyclopropyl, cyclopropenyl, butyl, isobutyl, cyclobutyl, alkyl, halogenated alkyl, cyclic alkyl, halogenated cyclic alkyl, propenyl, halogenopropenyl, alkenyl, halogenated alkenyl, cyclic alkenyl, halogenated cyclic alkenyl, alkynyl, halogenated alkynyl, aryl, halogenated aryl, acetyl, halogenoacetyl, propionyl, halogenopropionyl, butyryl, halogenobutyryl, isobutyryl, halogenoisobutyryl, alkyryl, halogenated alkyryl, cyclic alkyryl, halogenated cyclic alkyryl, benzoyl, aryryl, with a hydrogen or acetyl group or isobutyryl group being most preferred,

and wherein R3 is a substituent having from one to 30 atoms of any type(s), more preferably one to 20 atoms, and most preferably one to 16 atoms, with hydrogen, oxygen, carbon, sulphur, nitrogen, phosphorous and halogen being preferred; the R3 substituent being a hydrogen or a linear, branched and/or cyclic structure; the R3 substituent preferably comprising of from zero to 8 heavy atoms, even more preferably zero to 10 heavy atoms, even more preferably zero to 6 heavy atoms, even more preferably zero to 5 heavy atoms; most preferably the substituent being composed of any one of hydrogen, methyl, ethyl, propyl, isopropyl, cyclopropyl, cyclopropenyl, butyl, isobutyl, cyclobutyl, alkyl, halogenated alkyl, cyclic alkyl, halogenated cyclic alkyl, propenyl, halogenopropenyl, alkenyl, halogenated alkenyl, cyclic alkenyl, halogenated cyclic alkenyl, alkynyl, halogenated alkynyl, aryl, halogenated aryl, acetyl, halogenoacetyl, propionyl, halogenopropionyl, butyryl, halogenobutyryl, isobutyryl, halogenoisobutyryl, alkyryl, halogenated alkyryl, cyclic alkyryl, halogenated cyclic alkyryl, benzoyl, aryryl, with a hydrogen or acetyl group or isobutyryl group being most preferred,

and wherein R4 is a substituent connected by single or double bond having from one to 32 atoms, more preferably one to 18 atoms, more preferably one to 15 atoms more preferably one to 12 atoms, and most preferably one to 9 atoms with hydrogen, oxygen, carbon, sulphur, nitrogen, phosphorous and halogen atoms being preferred; the R4 substituent preferably being a hydrogen or a linear or branched and/or cyclic structure; the R4 substituent preferably comprising between 0 to 12 heavy atoms, even more preferably between 0 to 11 heavy atoms, even more preferably between 0 to 10 heavy atoms, even more preferably between 0 to 9 heavy atoms, even more preferably between 0 to 8 heavy atoms, even more preferably between 0 to 7 heavy atoms, even more preferably between 0 to 6 heavy atoms, and most preferably between 0 to 5 heavy atoms; most preferably the substituent being comprised of any of hydrogen, halogeno, oxo, hydroxy, methoxy, ethoxy, propoxy, isopropoxy, butoxy, isobutoxy, alkoxy, halogenated alkoxy, ethenyloxy, propenyloxy, alkenyloxy, halogenated alkenyloxy, methyl, ethyl, propyl, isopropyl, butyl, isobutyl, alkyl, halogenated alkyl, hydroxymethyl, hydroxyethyl, hydroxypropyl, hydroxyispropyl, hydroxybutyl, hydroxyisobutyl, hydroxyalkyl, halogenated alkyl, methylene, ethenyl, propenyl, isopropenyl, butenyl, isobutenyl, alkenyl, halogenated alkenyl, acetyl, halogenoacetyl, propionyl, butyryl, isobutyryl, alkyryl, halogenated alkyryl, acetyloxy, halogenoacetyloxy, propionyloxy, isopropionyloxy, butyryloxy, isobutyryloxy, alkyryloxy, halogenated alkyryloxy, 2-oxy-2-methyl-ethyl, hydroxy-oxomethyl, 2-hydroxy-2-oxoethyl, 3-hydroxy-3-oxopropionyl, methoxy-oxomethyl, ethoxy-oxomethyl, propoxy-oxomethyl, isopropoxy-oxomethyl, butoxy-oxomethyl, isobutoxy-oxomethyl, alkoxy-oxomethyl, 2-methoxy-2-oxoethyl, 2-ethoxy-2-oxoethyl, 2-propoxy-2-oxoethyl, 2-isopropoxy-2-oxoethyl, 2-butoxy-2-oxoethyl, 2-isobutoxy-2-oxoethyl, 2-alkoxy-2-oxoethyl, hydroxymethylene, 1-hydroxyethylidene, 1-hydroxypropylidene, 1-hydroxy-2-methylpropylidene, 1-acetyloxy-2-methylpropylidene, 1-halogenoacetyloxy-2-methylpropylidene, 1-alkyryloxy-2-methylpropylidene, 1-halogenoalkyryloxy-2-methylpropylidene, with hydrogen or isobutyryl or 1-hydroxy-2-methylpropylidene being most preferred,

and wherein R5 is a substituent connected by single or double bond having from one to 32 atoms, more preferably one to 18 atoms, more preferably one to 15 atoms, more preferably one to 12, more preferably or one to 10 atoms, more preferably one to 8 atoms and most preferably one to 7 atoms, with hydrogen, oxygen, carbon, sulphur, nitrogen, phosphorous and halogen atoms being preferred; the R5 substituent preferably being a hydrogen or an oxygen or a linear or branched and/or cyclic structure; the R5 substituent preferably comprising between 0 to 12 heavy atoms, even more preferably between 0 to 11 heavy atoms, even more preferably between 0 to 10 heavy atoms, even more preferably between 0 to 9 heavy atoms, even more preferably between 0 to 8 heavy atoms, even more preferably between 0 to 7 heavy atoms, even more preferably between 0 to 6 heavy atoms, more preferably between 1 to 5 heavy atoms and most preferably between 1 to 4 heavy atoms; most preferably the substituent being comprised of any of hydrogen, halogeno, oxo, hydroxy, methoxy, ethoxy, propoxy, butoxy, isobutoxy, alkoxy, halogenated alkoxy, ethenyloxy, propenyloxy, alkenyloxy, halogenated alkyloxy, methyl, ethyl, propyl, isopropyl, butyl, isobutyl, alkyl, halogenated alkyl, hydroxymethyl, hydroxyethyl, hydroxypropyl, hydroxyisopropyl, hydroxybutyl, hydroxyisobutyl, hydroxyalkyl, methylene, ethenyl, propenyl, isopropenyl, butenyl, isobutenyl, alkenyl, halogenated alkenyl, acetyl, halogenoacetyl, propionyl, isopropionyl, butyryl, isobutyryl, alkyryl, halogenated alkyryl, acetyloxy, halogenoacetyloxy, propionyloxy, halogenopropionyloxy, butyryloxy, halogenobutyryloxy, isobutyryloxy, halogenoisobutyryloxy, alkyryloxy, halogenated alkyryloxy, 2-oxy-2-methylethyl, hydroxy-oxomethyl, 2-hydroxy-2-oxoethyl, 3-hydroxy-3-oxopropionyl, methoxy-oxomethyl, ethoxy-oxomethyl, propoxy-oxomethyl, isopropoxy-oxomethyl, butoxy-oxomethyl, isobutoxy-oxomethyl, alkoxy-oxomethyl, 2-methoxy-2-oxoethyl,2-ethoxy-2-oxoethyl, 2-propoxy-2-oxoethyl, 2-isopropoxy-2-oxoethyl, 2-butoxy-2-oxoethyl, 2-isobutoxy-2-oxoethyl, 2-alkoxy-2-oxoethyl, hydroxymethylene, 1-hydroxyethylidene, 1-hydroxypropylidene, 1-hydroxy-2-methylpropylidene, with oxo and acetyloxy being preferred,

and which is optionally capable of eliciting a sexual enhancing effect.

However, in more restricted sense compounds of the invention have the structure according to general structure III:

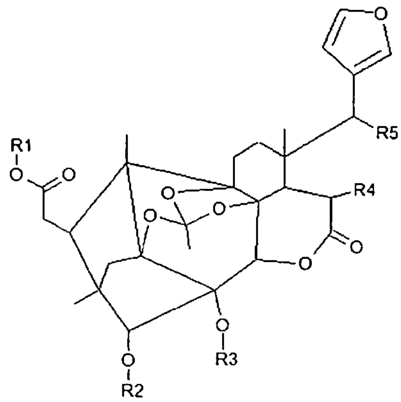

wherein

R1 is a substituent of any one of hydrogen, methyl or ethyl, with methyl being most preferred,

and wherein R2 is a substituent of any one of hydrogen, methyl, ethyl, acetyl, halogenoacetyl, propionyl, butyryl, isobutyryl, with a hydrogen or acetyl group or isobutyryl group being most preferred,

and wherein R3 is a substituent of any one of hydrogen, methyl, ethyl, acetyl, halogenoacetyl, propionyl, butyryl, isobutyryl, with a hydrogen or acetyl group or isobutyryl group being most preferred,

and wherein R4 is a substituent of any one of hydrogen, isobutyryl, 1-hydroxyl-2-methylpropylidene, 1-methoxy-2-methylpropylidene, 1-acetyloxy-2-methylpropylidene, 1-halogenoacetyloxy-2-methylpropylidene, with hydrogen or isobutyryl or 1-hydroxy-2-methylpropylidene being most preferred,

and wherein R5 is a substituent of any one of oxo, hydroxy, methoxy, ethoxy, acetyloxy, halogenoacetyloxy, propionyloxy, butyryloxy, isobutyryloxy, with oxo and acetyloxy being preferred,

and which chemical substance (chemical compound) is optionally capable of eliciting a sexual enhancing effect.

However, in the widest sense R1, R2, R3, R4 and R5 each comprise any chemical group as defined herein *(vide supra).*

In view of that R306 and R310 confines ester bonds they may be hydrolyzed forming hydrolysis products, as is exemplified for the partial hydrolysis of R306 to R306AB:

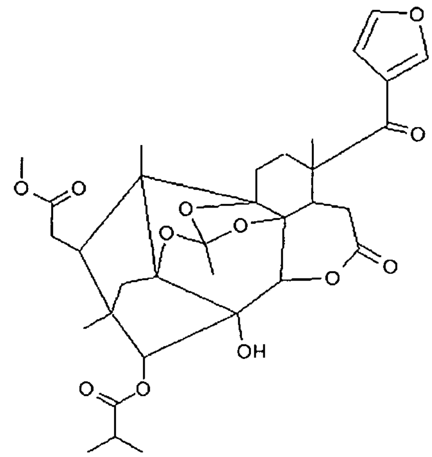

or to R306BA:

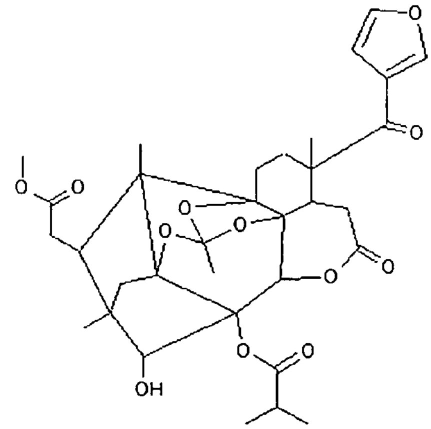

as well as to R306C:

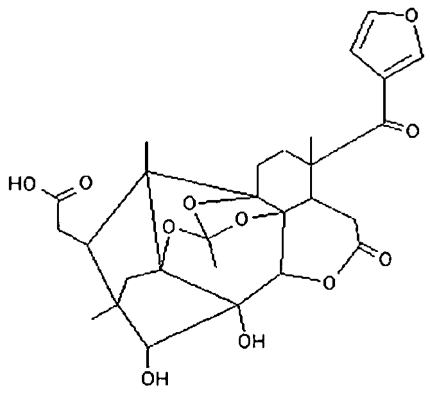

In an analogous fashion as described above for the hydrolysis of R306, hydrolysis products of R310 may be formed which are also capable of eliciting sexual enhancing effects. In fact, the process for hydrolyzing R306 and/or R310 can be carried in an essentially similar fashion as described for closely similar compounds busseins in reference (40).

It is further contemplated that compounds with any of the structures R306A, R306AB, R306BA, R306B, R306C, other hydrolysis products of R306, as well as hydrolysis products of R310, are capable of eliciting sexual enhancing effect.

Compounds derived from R306 or R310 by hydrolysis exposing hydroxyl or carboxyl group, as well as the free hydroxyl group already present in R310, may be esterified by proper chemical reaction thereby forming esterification products of R306 or R310 as well as forming esterification products from the hydrolysis products of R306 or R310. This can be afforded e.g. using carboxylic acid anhydride such as acetic acid anhydride, or other proper reagent, e.g. an acyl

chloride such as acetyl chloride, propylchloride, or other similar and proper reagent well known in the art, which leads to compounds according to the general structure III. In fact, the processes for hydrolyzing and esterifying R306 and R310, can be carried in an essentially similar fashion as described for hydrolyzing and esterifying the closely similar compounds busseins as is thoroughly described in reference (40); and the methods described in (40) can be directly applied onto R306 and R310 in order to form hydrolysis products and esterification products on R306 and R310.

Structures of the invention are defined by the claims. A structure of the invention may be comprised by a chemical substance having a substantial structural similarity with R306 or R310. Such a chemical substance can be derived from *Neobeguea mahafalensis* as well as it can be obtained from any other plant or biological source, in particular from species closely related to *Neobeguea mahafalensis* as well as from many species of the Meliaceae family, as well as it can be obtained by synthetic chemistry. It is in particular possible to derive them by so called semi-synthesis (partial synthesis), where part of the starting material in the synthesis is a chemical compound isolated from a natural source. A typical natural source as starting materials in synthesis of the chemical substance of the invention is a limonoid or terpenoid or terpene isolated from a species of the Meliaceae family.

In order to decide upon whether or not a structure has substantial structural similarity to R306 or R310 one may find the largest common substructure in the structure of interest that is also present in the structure of R306 or R310. One thus first finds the largest contiguous fragment(s) of connected atoms in the structure of interest that is also present in R306 or R310. (Substructures are well defined concept of chemoinformatics; see e.g. Chemoinformatics, eds. Johan Gasteiger and Thomas Engel, Wiley-VCH GmbH & Co. KGaA, ISBN 3-527-30681-1). For example, the structure of neobeguin (38) is as follows:

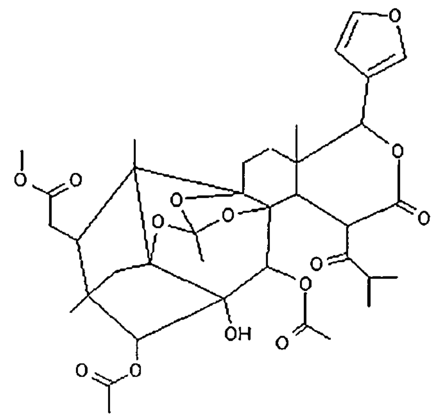

In order to find the largest common substructure of R306 and neobeguin one would traverse all carbon and oxygen atoms of R306 once and look for the largest substructure(s) of connected atoms that exactly matches substructure(s) of connected carbon and oxygen atoms in neobeguin. (Assessing substructures is a well known procedure in the art and can be performed e.g. by computer based substructure searches, e.g. using the SciFinder software by the American Chemical Society (SciFinder is available from Chemical Abstracts Service, 2540 Olentangy River Road, Columbus, OH 43202, U.S.A). In this example two such largest substructures (both being equally large) are found, namely A:

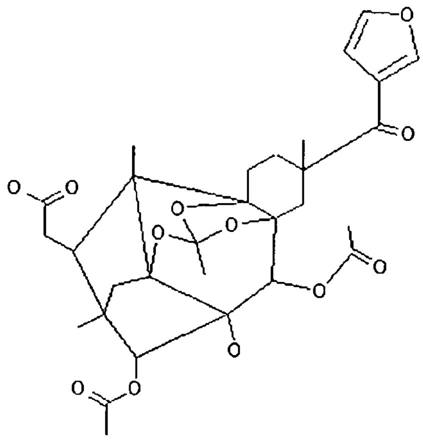

and B:

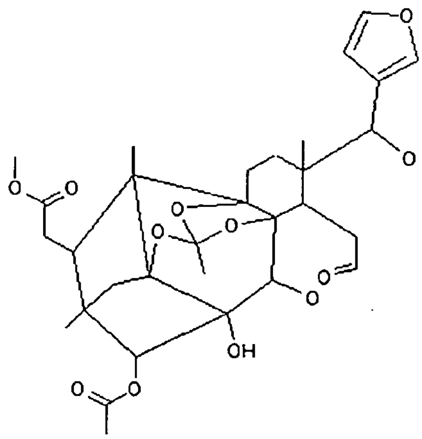

where both A and B have the equal number of carbon and oxygen atoms, namely each having 31 carbon atoms and 12 oxygen atoms. A comparison is then made with R306 by first summing up the count of carbon and oxygen atoms in the largest substructure, which in this example amounts to 31+12=43.

In the next step the number of carbon and oxygen atoms in R306 are counted and summed up, which yields 27+14=50.

In the last step the carbon-oxygen atom count for the largest substructure in the structure under investigation shared with R306 is divided with the carbon-oxygen atom count for R306, which defines the R306 similarity index, which in this example is 43/50 = 0.86.

Based on the computation of R306 similarity indices a structure of the invention is defined as a structure having an R306 similarity index of at least 0.6, more preferably at least 0.62, even more preferably at least 0.64, even more preferably at least 0.64, even more preferably at least 0.66, even more preferably at least 0.66, even more preferably at least 0.68, even more preferably at least 0.70, even more preferably at least 0.72, even more preferably at least 0.74, even more preferably at least 0.76, even more preferably at least 0.78, even more preferably at least 0.80, even more preferably at least 0.82, even more preferably at least 0.84, even more preferably at least 0.86, even more preferably at least 0.88, even more preferably at least 0.90, even more preferably at least 0.92, even more preferably at least 0.94, even more preferably at least 0.96, even more preferably at least 0.98, even more preferably at least 1.00.

In a further sense a structure is defined as a structure having an R306 similarity index of at least 0.6, more preferably at least 0.62, even more preferably at least 0.64, even more preferably at least 0.64, even more preferably at least 0.66, even more preferably at least 0.66, even more preferably at least 0.68, even more preferably at least 0.70, even more preferably at least 0.72, even more preferably at least 0.74, even more preferably at least 0.76, even more preferably at least 0.78, even more preferably at least 0.80, even more preferably at least 0.82, even more preferably at least 0.84, even more preferably at least 0.86, even more preferably at least 0.88, even more preferably at least 0.90, even more preferably at least 0.92, even more preferably at least 0.94, even more preferably at least 0.96, even more preferably at least 0.98, even more preferably at least 1.00, when the chemical substance with said structure is also

capable of eliciting a sexual enhancing effect.

In a further sense it is required that a chemical substance is derived from *Neobegasea mahafalensis* and its structure is having an R306 similarity index of at least 0.6, more preferably at least 0.62, even more preferably at least 0.64, even more preferably at least 0.64, even more preferably at least 0.66, even more preferably at least 0.66, even more preferably at least 0.68, even more preferably at least 0.70, even more preferably at least 0.72, even more preferably at least 0.74, even more preferably at least 0.76, even more preferably at least 0.78, even more preferably at least 0.80, even more preferably at least 0.82, even more preferably at least 0.84, even more preferably at least 0.86, even more preferably at least 0.88, even more preferably at least 0.90, even more preferably at least 0.92, even more preferably at least 0.94, even more preferably at least 0.96, even more preferably at least 0.98, even more preferably at least 1.00, as well as it optionally is also capable of eliciting a sexual enhancing effect, in order for the chemical substance to be structually similar to R306.

A similarity index with R310A or R310B can be computed in an analogous fashion as described above for computation of the R306 similarity index.

A structure is defined as a structure an R310A or R310B similarity index of at least 0.6, more preferably at least 0.62, even more preferably at least 0.64, even more preferably at least 0.64, even more preferably at least 0.66, even more preferably at least 0.66, even more preferably at least 0.68, even more preferably at least 0.70, even more preferably at least 0.72, even more preferably at least 0.74, even more preferably at least 0.76, even more preferably at least 0.78, even more preferably at least 0.80, even more preferably at least 0.82, even more preferably at least 0.84, even more preferably at least 0.86, even more preferably at least 0.88, even more preferably at least 0.90, even more preferably at least 0.92, even more preferably at least 0.94, even more preferably at least 0.96, even more preferably at least 0.98, even more preferably at least 1.00.

In a further sense a structure is defined as a structure having an R310A or R310B similarity index of at least 0.6, more preferably at least 0.62, even more preferably at least 0.64, even more preferably at least 0.64, even more preferably at least 0.66, even more preferably at least 0.66, even more preferably at least 0.68, even more preferably at least 0.70, even more preferably at least 0.72, even more preferably at least 0.74, even more preferably at least 0.76, even more preferably at least 0.78, even more preferably at least 0.80, even more preferably at least 0.82, even more preferably at least 0.84, even more preferably at least 0.86, even more preferably at least 0.88, even more preferably at least 0.90, even more preferably at least 0.92, even more preferably at least 0.94, even more preferably at least 0.96, even more preferably at least 0.98, even more preferably at least 1.00, when the chemical substance with said structure is also capable of eliciting a sexual enhancing effect.

In a further sense it is required that a chemical substance is derived from *Neobeguea mahafalensis* and its structure is having an R310 similarity index of at least 0.6, more preferably at least 0.62, even more preferably at least 0.64, even more preferably at least 0.64, even more preferably at least 0.66, even more preferably at least 0.66, even more preferably at least 0.68, even more preferably at least 0.70, even more preferably at least 0.72, even more preferably at least 0.74, even more preferably at least 0.76, even more preferably at least 0.78, even more preferably at least 0.80, even more preferably at least 0.82, even more preferably at least 0.84, even more preferably at least 0.86, even more preferably at least 0.88, even more preferably at least 0.90, even more preferably at least 0.92, even more preferably at least 0.94, even more preferably at least 0.96, even more preferably at least 0.98, even more preferably at least 1.00, as well at it optionally is also capable of eliciting a sexual enhancing effect, in order for the chemical substance to be structurally similar to R310.

In a further sense it is required that the chemical substance is derived from *Neobeguea mahafalensis* but that neobeguin (38), pseudrelone $A_2$ (39), Leandreanin A, Leandreanin B and Leandreanin C (41), 2-hydroxy-6-deoxyswietine and 2-hydroxy-6-deoxyswietonolide tiglate (39) are specifically excluded, and its structure is having an R306 and/or R310 similarity index of at least 0.6, more preferably at least 0.62, even more preferably at least 0.64, even more preferably at least 0.64, even more preferably at least 0.66, even more preferably at least 0.66, even more preferably at least 0.68, even more preferably at least 0.70, even more preferably at least 0.72, even more preferably at least 0.74, even more preferably at least 0.76, even more preferably at least 0.78, even more preferably at least 0.80, even more preferably at least 0.82, even more preferably at least 0.84, even more preferably at least 0.86, even more preferably at least 0.88, even more preferably at least 0.90, even more preferably at least 0.92, even more preferably at least 0.94, even more preferably at least 0.96, even more preferably at least 0.98, even more preferably at least 1.00, as well at it is optionally required that the substance is capable of eliciting a sexual enhancing effect.

In most embodiments it is required that the R306 and/or R310 similarity index for a structure is at least 0.67, more preferably at least 0.81, even more preferably at least 0.87, even more preferably at least 0.90 and for it to fall within the claims in order for it to be comprised by the invention.

In addition minor modifications of the structures of any of general structures III, R306, R306AB, R306BA, R306C, R306D, R306E, R306F, R310A, R310A3, R310A4, R310A5, R310B, R310B1, R310B2, R310B3, R310B4, R310B5, R310B6, R310B7, R310B8, R310B9, R310B10, R310B11, R310B12, R310B13, R310B14, R310B15, R310B16, R310B17, R310B18, R310A can be done and the compound with such modified structures still being a

chemical substance of the invention (provided that it falls within the claims). Such modifications include opening of ring, closing of side chain to form cyclic structure and replacing any hydrogen or side chain with other group, e.g. methyl, ethyl, halogen. Such modifications can be done in any set of combinations.

A way of helping to ensure that a structure is a structure of the invention is to consider the presence of specific R306 substructures. This is because the presence of a specific substructure confined in R306 in a chemical compound may be sufficient to afford a sexual enhancing effect of the chemical compound of the invention. The substructures defined below are useful as starting points for synthesising synthetic compounds sharing similar pharmacological sexual enhancing activity as R306. Assessing the presence of substructures is a well known procedure in the art and can be performed e.g. by computer based substructure searches, e.g. using the SciFinder software by the American Chemical Society (SciFinder is available from Chemical Abstracts Service, 2540 Olentangy River Road, Columbus, OH 43202,U.S.A).

Substructure-306(1):

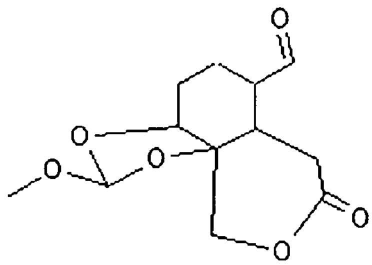

Substructure-306(2):

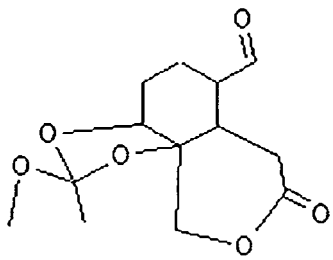

Substructure-306(3):

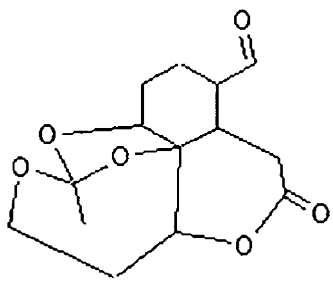

Substructure-306(4):

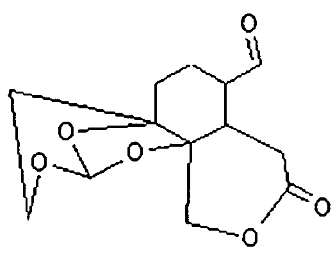

Substructure-306(5):

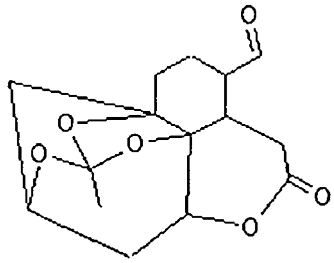

Substructure-306(6):

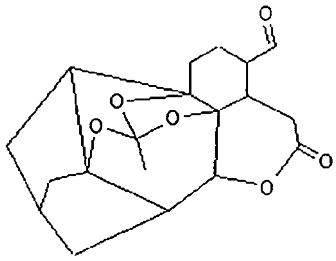

Substructure-306(7):

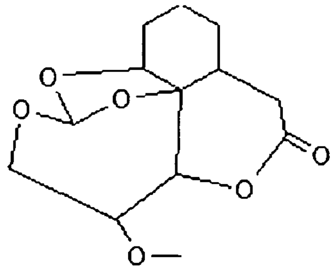

Substructure-306(8):

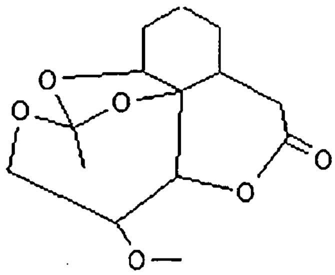

Substructure-306(9):

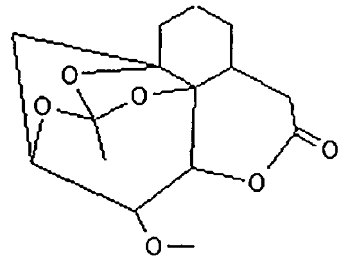

Substructure-306(10):

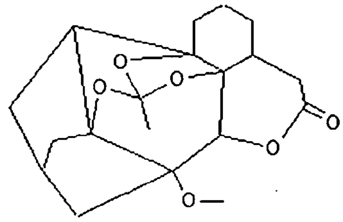

Substructure-306(11):

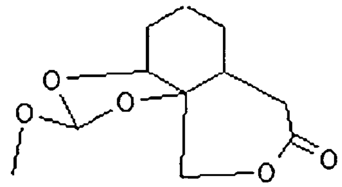

Substructure-306(12):

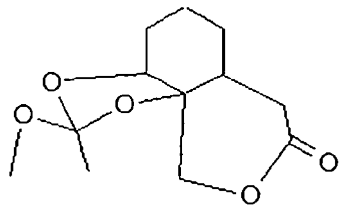

Substructure-306(13):

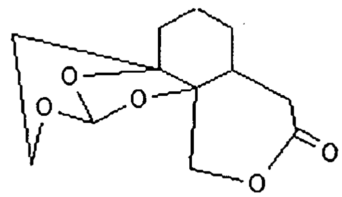

Substructure-306(14):

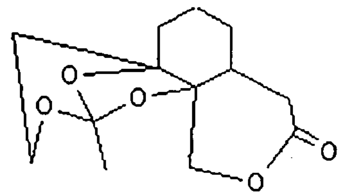

Substructure-306(15):

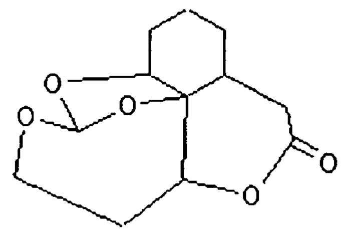

Substructure-306(16):

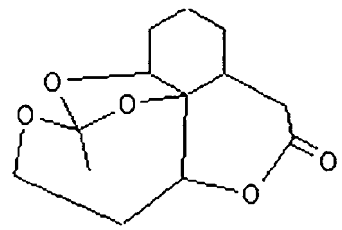

Substructure-306(17):

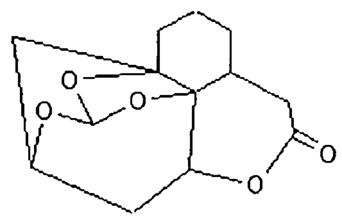

Substructure-306(18):

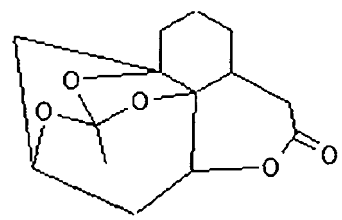

Substructure-306(19):

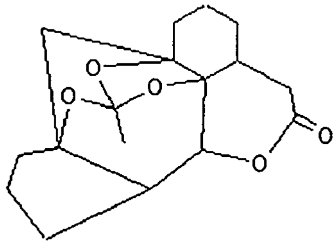

Substructure-306(20):

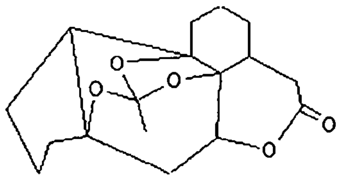

Substructure-306(21):

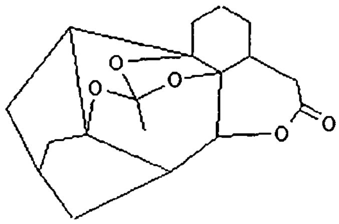

excluding the compound with registry number 98379-63-6 of the Chemical Abstracts Service (CAS) of the American Chemical Society. the compound with registry number 98379-63-6 of the Chemical Abstracts Service (CAS) of the American Chemical Society does not form part of the invention. This is because the compound 98379-63-6 is devoid of sexual enhancing effect.

The chemical substance of the invention may be obtained in radioactive form, e.g. by semisynthesis, by exchanging a suited group(s) or atom(s) in the structure of the invention with radioactive atom(s) or with group(s) in which one or several atom(s) are radioactive. Preferred radioactive atoms for the purpose of this embodiment of the invention are $^{3}H$, $^{14}C$, $^{18}O$, $^{125}I$ and $^{131}I$. Manufacturing of radioactive compounds in this way comprise procedures well known in the art.

Comprised by the invention is the salt of the chemical substance of the invention; in particular the pharmaceutically acceptable salt(s); i.e. those salts of compounds of the invention that are safe and effective for systemic use in mammals and is particularly desired for those substances of the invention that are capable of forming salts.

The phrase "pharmaceutically acceptable salt(s)", as used herein, means those salts of compounds of the invention that are safe and effective for systemic use in mammals and that possess the desired biological activity. Pharmaceutically acceptable salts include salts of acidic or basic groups present in compounds of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Certain compounds of the invention can form pharmaceutically acceptable salts with various amino acids. Suitable base salts include, but are not limited to, aluminum, calcium, lithium, magnesium, potassium, sodium, zinc, and diethanolamine salts. For a review on pharmaceutically acceptable salts see BERGE ET AL., 66 J. PHARM. SCI. 1-19 (1977), incorporated herein by reference.

Comprised by the invention are also the structures of the tautomeric forms wherein one of the structures of the tautomers is comprised by the structure of the invention. In fact, the thorough investigations underlying this patent have shown that R310 exists in slowly interconverting tautomeric keto and enol forms. Thus, e.g. (but not limited to) R310A3, R310B3, R310A4, R310B4, R310A5, R310B5, R310B7, R310B8 and R310B14 interconvert in between keto and enol forms in an analogous fashion as R310A and R310B and both the keto and enol forms are included into the structures of the inventions for these compounds.

The structure of the invention contains many chiral carbons. Comprised by the invention are therefore all possible stereoisomeric forms of the compound of the invention.

In detailed comparison of the sexual enhancing effects of extract prepared from root and stem bark of *Neobeguea mahafalensis* it has surprisingly been found that the stem bark yields extracts that are inactive, whereas root gives highly active extracts (see Example 40). Accordingly, due to this finding extracts of stem bark are specifically excluded from being part of the invention as their administration would risk administering potentially toxic principles (e.g. toxic saponins are present in many of the extract derived from stem bark as is evident from the foamy appearance of many of these extracts, in particular water extracts of the stem bark of *Neobeguea mahafalensis)* as well as stem bark yields very bitter extracts that show low patient compliance. Thus, the drug and/or extract and/or component and/or compound and/or substantially pure compound and/or pharmaceutical and/or food supplement and/or dietary supplement prepared from stem bark of *Neobeguea mahafalensis* is specifically excluded from some embodiments of the invention.

On top of that extract S2C from stem bark of *Neobeguea mahafalensis* has been found to be essentially devoid of chemical substance R306 while extracts R2C from root of *Neobeguea mahafalensis* may contain up to 2 % of R306 (see further below). This is also one further reason that using the root of *Neobeguea mahafalensis* is preferred in most embodiments of the present invention over the stem bark as well at is a further reason that stem bark of *Neobeguea mahafalensis* is specifically excluded from all embodiments of the present invention.

The activity of the extract(s) isolated by fractionation of *Neobeguea mahafalensis* can be followed by observing the sexual enhancing effect of the extracts. Observing the sexual enhancing effect is usually done by administering the extract to humans or animals, with animals being preferred, and then studying the change of any physiological or behavioral effect related to sexual function on said humans or animals. Extracts with higher activity are preferred. (By higher activity is intended that a lower amount of the extract can be administered to the animal or human and elicit an effect comparable with another extract that requires a higher amount to elicit the same or similar degree of effect in the animal or human). Preferred assays for determining activity and examples of assaying are described in Examples 17, 22, 23, 30, 32, 34, 40 and 43 and further below herein. Preparation of preferred enriched extracts from *Neobeguea mahafalensis* are described in Example 9, 29, 31 and 33. Highly preferred enriched extracts of the present invention comprises extracts R2C, RCH, RT, REtOH, DCM, 01DG2, D-Ac1, D-Ac12, RW, RWExh, RW1, RB and RXM. Further purification of such enriched extracts (usually with chromatographic procedures) will result in pure compound(s) and/or substance(s)) and/or substantially pure compounds and/or substantially pure substance(s)), such as described in Examples 41 and 42 for the preparation of R306 and R310; which can be assayed for activity on sexual functions in animals, e.g. using the approaches described in Examples 17, 22, 23, 30, 32, 34 and 43, or by using clinical trial, e.g. as detailed in Examples 18-21. Pure compound(s) or substantially pure compound(s) isolated in this way from *Neobeguea mahafalensis* can for the sake of the present invention be substituted for the extract of *Neobeguea mahafalensis* and be used for all the purposes stated herein for the extract or enriched extract derived or prepared from *Neobeguea mahafalensis.* Moreover, a compound identified in *Neobeguea mahafalensis* can be manufactured by other means than by extraction from *Neobeguea mahafalensis.* Example of such other method(s) for manufacture comprise (but are not limited to) chemical synthesis, derivatisation of compound isolated from other sources than *Neobeguea mahafalensis,* by direct isolation from other source(s) than *Neobeguea mahafalensis,* and by production in other system(s). Compound(s) manufactured in such way(s) can for the sake of the present invention be substituted for the extract or enriched extract of *Neobeguea mahafalensis* and be used for all the purposes stated herein for the extract or enriched extract derived from *Neobeguea mahafalensis.* Any component or active component (active compound) derived from *Neobeguea mahafalensis* that is manufactured in other system or synthesized can for the sake of the present invention be substituted for the extract (enriched extract) of *Neobeguea mahafalensis* and be used for all the purposes stated herein for the extract (enriched extract) derived from *Neobeguea mahafalensis.* By active component (active compound) is herein intended a material derived from *Neobeguea mahafalensis* where said component (compound) shows activity on sexual function(s) or conditions of sexual dysfunctions, e.g., as can be demonstrated using the tests detailed in Examples 17, 22, 23, 30, 32, 34 and 43, or using clinical trial, e.g. as detailed in Examples 18-21, or is having a sexual enhancing effect, and/or yielding at least one mass-peak in a mass spectrometer having the molecular weight of the characteristic mass-peak of the invention.

A substance of the invention is often formed artificially during the isolation of it from *Neobeguea mahafalensis.* Thus it is in this case not originally comprised in the living *Neobeguea mahafalensis.* The chemical substance formed from a compound or component comprised in *Neobeguea mahafalensis* during its isolation is also comprised by the invention when its structure is also comprised by the structure of the invention as defined herein above.

It is contemplated that species related to *Neobeguea mahafalensis,* namely primarily those belonging to the Meliaceae family (i.e. the Mahogany family) due to their relatedness and biological similarities with *Neobeguea mahafalensis* also contain principles with enhancing activity on sexual function, as well as chemical substances with chemical structures closely similar to those of *Neobeguea mahafalensis* which are also capable of eliciting sexual enhancing effects; in particular it is contemplated that they contain chemical substances having close structural similarities to R306 and R310 and therefore also elicit sexual enhancing effects. Accordingly they are therefore useful substitutes for *Ne-*

*obeguea mahafalensis* for all the embodiments of the present invention described herein for *Neobeguea mahafalensis.* Of the many species of the Meliaceae family *Neobeguea leandriana, Neobeguea leandreana, Neobeguea ankaranensis* and *Neobeguea sp.* Are particularly useful in this respect due to that they are among the closest relatives to *Neobeguea mahafalensis.*

Accordingly a drug, extract, compound, substantially pure compound, structure of the invention, chemical substance of the invention, pharmaceutical, tea-bag, food supplement and/or dietary supplement prepared from and/or prepared by use of and/or obtained from and/or being obtainable from a species belonging to the Meliaceae family, in particular *Neobeguea leandriana, Neobeguea leandreana, Neobegzzea ankaranensis* and *Neobeguea sp.* can be used in the same way as described in herein for the drug, extract, compound, substantially pure compound, structure of the invention, chemical of the invention, pharmaceutical, tea-bag, food supplement and or dietary supplement prepared from and/or prepared by use of and/or derived from and/or obtained from and/or being obtainable from *Neobeguea mahafalensis.*

It is furthermore contemplated that *Neobeguea leandreana* (*Neobeguea leandriana*) is a useful substitute for *Neobeguea mahafalensis* for all embodiments of the present invention. Thus, substances with similar structures as R306 were isolated by Coombes et al. from Neobeguea leandreana, namely the phragmalin limonoids leandreanin A (CAS registry number 561307-81-1), leandreanin B (CAS registry number 561307-82-2) and leandreanin C (CAS registry number 561307-83-3) (41). While the pharmacological actions of leandreanin A, leandreanin B and leandreanin C are completely unknown it is contemplated that *Neobeguea leandreana* by virtue of the biochemical metabolism of the plant would contain the chemical structures of the present invention or that such chemical structures can be derived from *Neobeguea leandreana* applying any one of the processes described herein for *Neobeguea mahafalensis,* such as heating, drying, oxidation, etc. Accordingly *Neobeguea leandreana (Neobeguea leandriana*) serves as an excellent substitute for *Neobeguea mahafalensis* for all aspects of the present invention in particular for preparation of extracts, compounds, components, substantially pure components, pharmaceuticals, pharmaceuticals for treatment of sexual dysfunction, tea-bags, food supplements and dietary supplements.

Compounds derived from *Neobeguea leandreana (Neobeguea leandriana*), in particular phragmalins, in particular leandreanin A, leandreanin B and leandreanin C, can be used as raw-materials in the preparation of the compound of the invention by use of chemical synthesis; i.e. by applying so called semi-synthesis.

It is also contemplated that leandreanin A, leandreanin B and/or leandreanin C by virtue of their structural similarity with R306 and R310 are capable of eliciting a similar sexual stimulating effect as R306/R310. Therefore, also disclosed is the use of any one of leandreanin A, leandreanin B and leandreanin C for the treatment of sexual dysfunction, in particular erectile dysfunction, ejaculatory dysfunction and hypoactive sexual desire disorders in both men and woman. Moreover, due to the fact that minor variations in its chemical structure is allowed for a chemical compound and still retaining or even enhancing its pharmacological activity minor chemical variations of leandreanin A, leandreanin B and leandreanin C still lead to compounds that have sexual stimulating effects. Specifically, exchange of any of one, two, three, four, five, six, seven, eight, nine or ten methyl groups with hydrogen, and/or exchange of any of one or two methoxy groups with hydrogen, and/or hydrolysis (i.e. removal of) any of one, two, three or four acetyl groups, and/or exchange of any one of one, two three, four, five, six, seven, eight, nine, ten, eleven, twelve, thirteen, fourteen or fifteen hydrogen atoms with any combination(s) of hydroxy, methyl, trifluoromethyl, methoxy, ethyl, ethoxy, propyl, isopropyl, fluorine, chlorine, bromine or iodine in any one of leandreanin A, leandreanin B and leandreanin C still leads to compounds for use for the treatment of sexual dysfunction, in particular erectile dysfunction, ejaculatory dysfunction and hypoactive sexual desire disorders in both men and women.

It is also contemplated that neobeguin (CAS registry number 260794-07-8) being previously isolated from *Neobegueca mahafalensis* by virtue of its structural similarity with R306 is capable of eliciting a similar sexual stimulating effect as R306/R310. Therefore, also disclosed is the use of neobeguin for the treatment of sexual dysfunction, in particular erectile dysfunction, ejaculatory dysfunction and hypoactive sexual desire disorders in both men and woman. Moreover, due to the fact that minor variations in its chemical structure is allowed for a chemical compound and still retaining or even enhancing its pharmacological activity minor chemical variations of neobeguin still lead to compounds that have sexual stimulating effects. Specifically, exchange of any of one, two, three, four, five, six, seven, eight, nine or ten methyl groups with hydrogen, and/or exchange of a methoxy group with hydrogen, and/or hydrolysis (i.e. removal of) any of one or two acetyl groups, and/or substitution of any one of one, two three, four, five, six, seven, eight, nine, ten, eleven, twelve, thirteen, fourteen or fifteen hydrogen atoms with any combination(s) of hydroxy, methyl, trifluoromethyl, ethyl, acetyl, chloroacetyl, propionyl, butynyl, isobutynyl, propyl, isopropyl, fluorine, chlorine, bromine or iodine in neobeguin still leads to compounds for use for the treatment of sexual dysfunction, in particular erectile dysfunction, ejaculatory dysfunction and hypoactive sexual desire disorders in both men and woman.

It is also contemplated that psedurelone $A_2$ being previously isolated from *Neobeguea mahafalensis* (39) by virtue of its structural similarity with R306 is capable of eliciting a similar sexual stimulating effect as R306. Therefore, also disclosed is the use of neobeguin for the treatment of sexual dysfunction, in particular erectile dysfunction, ejaculatory dysfunction and hypoactive sexual desire disorders in both men and women.

Moreover, due to the fact that minor variations in its chemical structure is allowed for a chemical compound and still retaining or even enhancing its pharmacological activity minor chemical variations of neobeguin still lead to compounds that have sexual stimulating effects. Specifically, exchange of any of one, two, three, four, five, six, seven, eight, nine or ten methyl groups with hydrogen, and/or exchange of a methoxy group with hydrogen, and/or hydrolysis (i.e. removal of) any of one or two isobutynyl groups, and/or substitution of any one of one, two three, four, five, six, seven, eight, nine, ten, eleven, twelve, thirteen, fourteen or fifteen hydrogen atoms with any combination(s) of hydroxy, methyl, trifluoromethyl, ethyl, acetyl, chloroacetyl, propionyl, butynyl, isobutynyl, propyl, isopropyl, fluorine, chlorine, bromine or iodine in psedurelone $A_2$ still leads to compounds for use for the treatment of sexual dysfunction, in particular erectile dysfunction, ejaculatory dysfunction and hypoactive sexual desire disorders in both men and women.

Yet another important species disclosed herein is Entandrophragma bussei Harms which also belongs the Meliaceae family, being a member of the genus Entandrophragma (Harms ex Engl. Publication: in Engl. Pflanzenw. Afr. iii. 1. (Engl. & Drude, Veg. der Erde, ix.) 807 (1915), in obs.). This is because tetranortriterpenes termed busseins were isolated from it which have structures that closely resembles that of R306. These include busseins A, B, C, D, E, F, G, H, J, K, L and M (40,42) and compounds 3, 4, 5, 6, 9, 10, 11, 12, 13, 14, 15 and 16 listed on p. 523 in reference 40 obtained by semi-synthesis from naturally occurring busseins; all these listed compounds herein collectively being termed busseins. It is thus contemplated that busseins by virtue of their structural similarities with R306 are capable of eliciting similar sexual stimulating effect as R306. Therefore, also disclosed is the use of a bussein for the treatment of sexual dysfunction, in particular erectile dysfunction, ejaculatory dysfunction and hypoactive sexual desire disorders in both men and women. Moreover, due to the fact that minor variations in the chemical structure is allowed for a chemical compound and still retaining or even enhancing its pharmacological activity minor chemical variations of a bussein still lead to compounds that have sexual stimulating effects. Specifically, exchange of any of one, two, three, four, five, six, seven, eight, nine or ten methyl groups with hydrogen, and/or exchange of a methoxy group with hydrogen, and/or hydrolysis (i.e. removal of) any of one or two acetyl groups, and/or substitution of any one of one, two three, four, five, six, seven, eight, nine, ten, eleven, twelve, thirteen, fourteen or fifteen hydrogen atoms with any combination(s) of hydroxy, methyl, trifluoromethyl, ethyl, acetyl, chloroacetyl, propionyl, butynyl, isobutynyl, propyl, isopropyl, fluorine, chlorine, bromine or iodine in a bussein still lead to compounds for use for the treatment of sexual dysfunction, in particular erectile dysfunction, ejaculatory dysfunction and hypoactive sexual desire disorders in both men and women.

Busseins isolated from *Entandrophragma bussei* Harms constitute furthermore important raw materials in the preparation of the compound of the invention by use of semisynthesis. This is because *Entandrophragma bussei* contains large quantities of these busseins, often in the range of one to several grams per kg of wood (42). Other species of the genus *Entandrophragma* are also useful sources as raw materials in the synthesis of the compound of the invention in particular *Entandrophragma spicatum* and *Entandrophragma caudatum* because of their rich content of limonoids, as well as are also many other species of the Meliaceae family. E.g. *Entandrophragma caudatum* is a rich source of phragmalin (see Arndt and Baarschers, The structure of phragmalin a meliacin with a norbornane part skeleton, Tetrahedron vol. 28, 1972, 2333-2340) which can be used as a precursor in the semisynthesis of the compound of the invention with the structure of R306 or a structure closely similar to R306.

Although a few compounds isolated from various species have chemical structures that contains structural elements with resemblance of structural elements in R306 the pharmacological actions of these compounds are largely not known; in particular their potential for treatment of sexual dysfunction is completely unknown. In order for a compound to be useful for the treatment of sexual dysfunction characteristic substructures confined in R306/R310 can be used, which include Substructure-306(1) ... Substructure-306(21) as well as compounds comprising the Substructure-306(U1) ... Substructure-306(U6) as follows:

Substructure-306(U1):

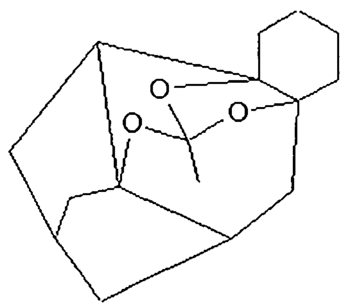

Substructure-306(U2):

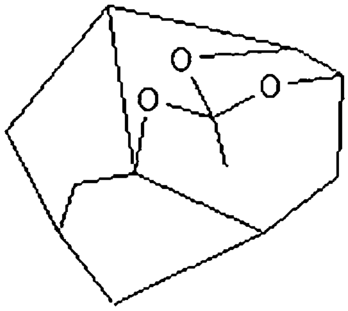

Substructure-306(U3):

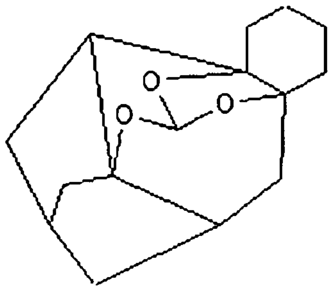

Substructure-306(U4):

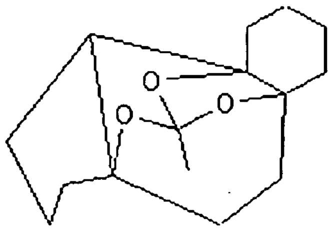

Substructure-306(U5):

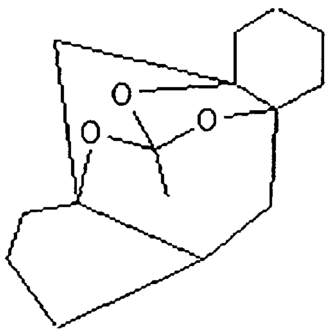

Substructure-306(U6):

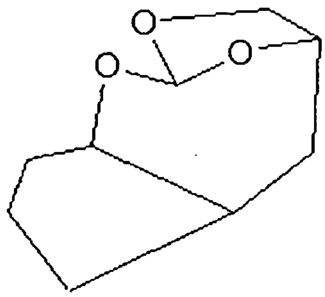

Known compounds comprised by these substructures include compounds with CAS registry numbers (with generic names given within parentheses) as follows: 939775-81-2 (Xylocarpin I), 926896-44-8 (Kotschyin A), 926896-45-9 (Kotschyin B), 926896-46-0 (Kotschyin C), 803723-28-6 (Tabulalide D), 803723-27-5, 919578-31-3 (12-O-acetyltabulalide D), 629654-41-7 (Swietenialide D), 629654-42-8 (Swietenialide E), 561307-81-1 (Leandreanin A), 561307-82-2 (Leandreanin B), 561307-83-3 (Leandreanin C), 260794-07-8 (Neobeguin), 121665-59-6, 116408-24-3, 115391-10-1, 115367-50-5, 115367-49-2, 98401-23-1, 98379-64-7, 98379-63-6, 98379-62-5, 98379-61-4, 98379-60-3, 98379-59-0, 98379-58-9, 98379-57-8, 41060-13-3 (Bussein A) 41060-14-4 (Bussein B), 90930-95-3 (Bussein C), 90930-96-4 (Bussein D), 90930-97-5 (Bussein E), 90930-98-6 (Bussein F), 90930-99-7 (Bussein G), 90931-00-3 (Bussein H), 90955-39-8 (Bussein J), 90931-01-4 (Bussein K), 90931-02-5 (Bussein L), 90931-03-6 (Bussein M), 40185-33-9, 96386-37-7, 72264-44-9 (Febrinin A), 72264-45-0 (Febrinin B), 96386-36-6 (Epoxyfebrinin B), 81584-75-0 (Spicata-2), 73702-69-9 (Pseudrelone B), 67931-05-9, 67931-04-8, 67904-58-9, 67904-57-8, 67904-53-4 (Chukrasin B), 67904-54-5 (Chukrasin C), 67904-55-6 (Chukrasin D), 67904-56-7 (Chukrasin E), 67895-40-3, 67895-39-0, 67895-38-9, 66939-94-4, 66901-32-4, 66901-31-3, 66901-30-2, 66901-32-4, 66901-31-3, 66901-30-2, 66884-81-9, 66884-80-8, 66884-79-5, 66884-78-4, 66884-77-3, 66495-42-9, 66451-22-7 (Chukrasin), 52724-62-6, 52681-81-9, 41508-26-3, 38575-45-0, 37832-02-3, 37665-93-3, 37665-92-2, 37665-91-1, 37665-90-0, 35183-64-3, 35055-81-3, however none of them are previously known to elicit a sexual enhancing effect.

Also disclosed is the use of compounds for treatment of sexual dysfunction, in particular erectile dysfunction and/or hypoactive sexual desire disorders which comprising the Substructure-306(T1) ... Substructure-306(T4) as follows:

Substructure-306(T1):

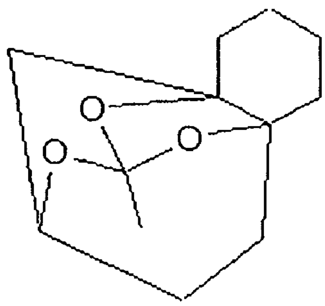

Substructure-306(T2):

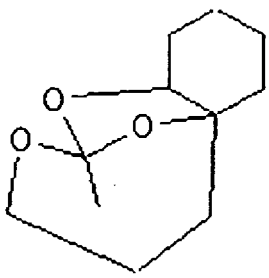

Substructure-306(T3):

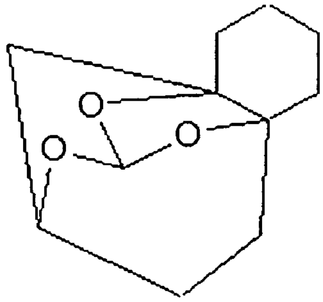

Substructure-306(T4):

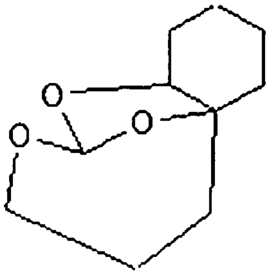

Known compounds comprised by Substructure-306(T1) ... Substructure-306(T4) in addition to those comprised by Substructure-306(U1) ... Substructure-306(U6) listed above are compounds with CAS registry numbers: 160768-97-8, 160768-98-9, 160768-99-0, 160769-00-6, 312538-39-9 and 175881-66-0 and some of these were obtained by chemical synthesis (44); however none of them were previously known to elicit sexual enhancing effects.

Compounds comprised by Substructure-306(U1) ... Substructure-306(U6) were previously claimed for use for treatment of diabetes and dyslipidemias and pharmaceuticals thereof were claimed (WO 2007/0318 30 A2), but their effects on sexual functions were previously not known. Compounds of this type were also reported to reduce feeding in larvae of insect (43).

Also disclosed is the use of a compound having a substructure of any one Substructure-306(1) ... Substructure-306(21), Substructure-306(U1) ... Substructure-306(U6) and Substructure-306(T1) ... Substructure-306(T4) for the treatment of sexual dysfunction, in particular erectile dysfunction, ejaculatory dysfunction and hypoactive sexual desire disorders is comprised by the invention.

Any drug, extract, component, compound, substantially pure compound, pharmaceutical, food supplement, dietary supplement, organic compound, synthetic compound, extract derived from plant (even extract from a plant not being *Neobeguea mahafalensis*), extract from animal, extract from microorganism, extract from body fluid, etc., can be checked whether or not it is part of the invention by analyzing the presence of characteristic mass-peaks in it. In particular a drug, extract, component, compound, substantially pure compound or pharmaceutical prepared from or derived from *Neobeguea mahafalensis* or comprising materials derived from *Neobeguea mahafalensis* otherwise, is subject to extraction using hydrophilic solvent (e.g. acetonitrile) or hydrophobic solvent (e.g. chloroform) or partitioning between water and hydrophobic solvent (e.g. chloroform). (E.g., the extract, drug, pharmaceutical, food supplement or dietary supplement is dispersed, ground or solubilised in chloroform. Water is optionally added and the mixture shaken for an appropriate period of time). After isolation of the chloroform phase by separating eventual water phase and removing unsolubilised materials, the chloroform phase is evaporated and the residue then obtained is dissolved in suited solvent, such as acetonitrile, acetonitrile and water, or acetonitrile and formic acid solution in water, or other suitable solvent, and the thus obtained solution (after removal of eventual unsolubilised or precipitated materials) is introduced into a mass-spectrometer (or optionally first introduced onto an HPLC column attached to a mass spectrometer thus introducing to the mass-spectrometer by means of chromatography) said mass-spectrometer being equipped with a suited ionizer. Preferred ionizer for most embodiments of the invention is atmospheric pressure chemical ionization (APCI) ion source due its more efficient capacity of ionization, but any suited ionizer such as electrospray ionizer (ESI) (Z-spray or nanoflow electrospray ionizer), which are also used in preferred embodiments of the invention, can be used. Atmospheric pressure photoionization, APPI, can also be used and is preferred in some embodiments of the invention.

LC/MS can be used, as well as MS/MS, as well as Time of flight (Tof) mass spectrometry, all technologies well-known in the art. Any type of mass-spectrometer can be used, however the mass-spectrometer should preferably be at least showing an accuracy of $\pm 0.2$ amu (amu=atomic mass units; i.e. 1 amu = 1/12 the weight of one carbon atom, thus 1 amu amounting to $1.66053886 \times 10^{-27}$ kg), more preferably at least showing an accuracy of $\pm 0.15$ amu and most

preferably showing an accuracy of at least ±0.1 amu.

This means accordingly that due to the inherent inability of any mass spectrometer to determine a mass weight of a compound (component or material or substance) exactly, the mass of a compound (component or material) determined by a specific mass-spectrometer should be within 0.2 amu, more preferably within 0.15 amu, and most preferably within 0.1 amu of the mass values listed herein for characteristic mass-peaks for the compound (component or material or substance) to be deemed as yielding the characteristic mass-peak of the invention. Thus, for example, if the mass of a mass-peak derived from a certain material is determined by use of a specific mass spectrometer to being as low as 305.5 or even as high as 305.9, more preferably as low as 305.55 or even as high as 305.85, and most preferably as low as 305.6 or even as high as 305.8 it shall be regarded to represent the characteristic mass-peak of the invention with the mass 305.7 (listed herein further below).

For LC/MS a preferred instrument for the purposes of determining characteristic mass-peaks of the invention is a Perkin Elmer PE SCIEX API 150EX single quadropole mass spectrometer (PerkinElmer Inc., 45 William Street, Wellesley MA 02481, USA), this mass-spectrometer preferably being equipped with atmospheric pressure chemical ionisation source (APCI).

When using time of flight mass spectrometry for determining characteristic mass-peaks of the invention a Q-Tof2 from Micromass (Macromass/Waters; Waters Corporation, 34 Maple Street, Milford Massachusetts 01757, USA) is preferred, the Q-Tof2 preferably being equipped with Micromass Z-spray (nanoflow) electrospray ionization inlet. Most preferred is a Micromass nanoflow interface together with glass capillary option.

However, any suited mass spectrometer can be used for the sake of the present invention.

Using APCI (API) and/or electrospray ionisation in some embodiments of the invention (preferably for a material derived from, but not limited to, the root of *Neobeguea mahafalensis*) the characteristic mass-peaks are, within ± 0.2 amu, more preferably within ± 0.15 amu and most preferably within ±0.1 amu, when using positive ionisation as follows (in amu):

305.7, 323.7, 324.2, 342.2, 378.3, 378.7, 383.2, 384.2, 391.2, 391.7, 408.2, 413.2, 413.7, 417.2, 425.7, 432.7, 441.7, 442.2, 465.3, 477.3, 481.3, 491.3, 507.3, 523.3, 537.3, 539.3, 540.3, 551.3, 565.3, 567.3, 579.3, 620.3, 639.3, 652.4, 655.4, 658.4, 662.4, 667.4, 671.4, 683.4, 694.4, 699.3, 699.4, 700.4, 703.4, 706.4, 710.4, 713.4, 715.3, 715.4, 716.4, 717.4, 727.4, 730.4, 731.4, 732.4, 732.9, 734.4, 741.4, 743.4, 744.4, 745.4, 759.4, 761.4, 762.4, 775.4, 785.4, 788.4, 789.4, 803.4, 819.4,

and when using negative ionisation as follows (in amu):

311.2, 335.2, 346.7, 347.2, 352.7, 353.2, 359.2, 364.7, 373.2, 388.7, 389.2, 401.2, 406.7, 407.2, 412.7, 418.7, 419.2, 425.2, 428.7, 450.7, 460.8, 463.3, 465.3, 485.3, 497.3, 498.8, 510.8, 513.3, 522.8, 525.3, 541.3, 551.3, 553.4, 559.3, 569.3, 573.3, 574.8, 575.3, 585.3, 587.3, 588.8, 599.3, 601.3, 603.3, 611.3, 617.3, 629.3, 633.3, 643.3, 645.3, 655.4, 657.4, 659.4, 661.4, 669.4, 671.4, 675.4, 681.4, 685.4, 687.4, 691.4, 695.4, 697.4, 701.4, 703.4, 706.9, 712.9, 713.4, 715.4, 719.4, 721.4, 725.4, 727.4, 729.4, 734.9, 738.9, 739.4, 741.4, 745.4, 748.9, 755.4, 759.4, 761.4, 772.9, 773.4, 787.4.

Since some materials may be more pure, while other materials may be less pure, it is required in some embodiments of the invention that at least one, more preferably at least two, more preferably at least three, more preferably at least four, more preferably at least five, more preferably at least six, more preferably at least seven, more preferably at least eight, more preferably at least nine, more preferably at least 10, more preferably at least 15, more preferably at least 20, more preferably at least 40, even more preferably at least 60, even more preferably at least 80, even more preferably at least 100, even more preferably at least 120, even more preferably at least 140, even more preferably at least 160, even more preferably 180, even more preferably at least 200, and most preferably all above listed characteristic mass-peaks found in root are found in the materials being analysed in order for it to be part of the invention.

Moreover, the mass-peaks being common for root pose special interest. Thus, for more specific embodiments of the invention the characteristic mass-peaks common for root of *Neobeguea mahafalensis* are, within ± 0.2 amu, more preferably within ± 0.15 amu and most preferably within ± 0.1 amu, when using positive ionisation as follows (in amu):

378.3, 413.2, 465.3, 477.3, 507.3, 523.3, 539.3, 639.3, 655.4, 662.4, 694.4, 699.3, 699.4, 703.4, 706.4, 715.3, 715.4, 716.4, 717.4, 731.4, 732.4, 734.4, 745.4, 775.4, 803.4, 819.4,

and when using negative ionisation as follows (in amu):

373.2, 497.3, 551.3, 553.4, 573.3, 585.3, 601.3, 611.3, 629.3, 655.4, 657.4, 661.4, 669.4, 671.4, 675.4, 685.4, 687.4, 697.4, 703.4, 713.4, 719.4, 721.4, 727.4, 745.4, 748.9.

Due to their importance and ubiquitous presence in *Neobeguea mahafalensis* the characteristic mass-peaks common to root and stem bark of *Neobeguea mahafalensis,* listed above, are of special interest in characterizing any drug, extract, pharmaceutical, tea-bag, food supplement or dietary supplement, in particular (but not limited to) materials prepared from or derived from *Neobeguea mahafalensis.* Therefore, in preferred embodiments of the invention a material that is characterized by the presence of at least one, more preferably at least two, even more preferably at least tree, even more preferably at least four, even more preferably at least five, even more preferably at least six, even more preferably at least seven, even more preferably at least eight, even more preferably at least ten, even more preferably at least 15, even more preferably at least 20, even more preferably at least 25, even more preferably at least 30, even more preferably at least 40, and most preferably all of the mass-peaks common to root and stem bark, listed above, is part of the invention.

However, due to the fact that extracts of stem bark from *Neobeguea mahafalensis* is unable to enhance the sexual activity in sexual behaviour test while the extract from the root of *Neobeguea mahafalensis* is highly active in this respect (Example 40) it is contemplated that it is only the characteristic mass-peaks unique to root that should be used to characterise the materials of the invention. Accordingly, in some embodiments the characteristic mass-peaks of the invention are, within ± 0.2 amu, more preferably swithin ± 0.15 amu and most preferably within ± 0.1 amu, when using positive ionisation as follows (in amu), selected from the list of:

305.7, 323.7, 324.2, 342.2, 378.3, 383.2, 384.2, 391.2, 391.7, 408.2, 413.7, 417.2, 425.7, 432.7, 441.7, 442.2, 481.3, 491.3, 537.3, 540.3, 551.3, 565.3, 567.3, 579.3, 620.3, 652.4, 655.4, 658.4, 667.4, 671.4, 683.4, 700.4, 710.4, 713.4, 727.4, 730.4, 732.9, 741.4, 743.4, 744.4, 759.4, 761.4, 762.4, 785.4, 788.4, 789.4,

and when using negative ionisation as follows (in amu):

311.2, 335.2, 346.7, 347.2, 352.7, 353.2, 359.2, 364.7, 388.7, 389.2, 401.2, 406.7, 407.2, 412.7, 418.7, 419.2, 425.2, 428.7, 450.7, 460.8, 463.3, 465.3, 485.3, 498.8, 510.8, 513.3, 522.8, 525.3, 541.3, 559.3, 569.3, 574.8, 575.3, 587.3, 588.8, 599.3, 603.3, 617.3, 633.3, 643.3, 645.3, 659.4, 681.4, 691.4, 695.4, 701.4, 706.9, 712.9, 715.4, 725.4, 729.4, 734.9, 738.9, 739.4, 741.4,755.4, 759.4, 761.4, 772.9, 773.4, 787.4.

Due to their importance the mass-peaks present in root but not stem bark of *Neobeguea mahafalensis,* listed above, are of special interest in characterizing any drug, extract, pharmaceutical, tea-bag, food supplement or dietary supplement, in particular (but not limited to) materials prepared from or derived from *Neobeguea mahafalensis.* Therefore, in preferred embodiments of the invention a material that is characterized by the presence of at least one, more preferably at least two, even more preferably at least tree, even more preferably at least four, even more preferably at least five, even more preferably at least six, even more preferably at least seven, even more preferably at least eight, even more preferably at least ten, even more preferably at least 15, even more preferably at least 20, even more preferably at least 25, even more preferably at least 30, even more preferably at least 40, even more preferably at least 50, even more preferably at least 60, even more preferably at least 70, even more preferably at least 80, even more preferably at least 90, even more preferably at least 100, and most preferably all of the mass-peaks present in root but not stem bark, listed above, is part of the invention.

Moreover, extremely important mass-peaks are those mass-peaks corresponding to R306 and R310, namely 699.3 and 715.3, which is due to the fact that substantially pure compounds R306 and R310 are particular effective in causing sexual enhancement and ameliorating sexual dysfunction when administered systemically (see Example 43). Accordingly, in some highly specific embodiments of the invention it is required that the drug, extract, enriched extract or pharmaceutical within ± 0.2 amu, more preferably swithin ± 0.15 amu and most preferably within ± 0.1 amu when using positive ionisation contains any one or both of the characteristic mass-peaks (in amu) 699.3 and 715.3.

Interestingly as the characteristic mass-peaks 699.3 and 715.3 are common to both extracts from root and stem-bark of *Neobeguea mahafalensis* most embodiments of the invention prefer extracts, compounds substantially pure compounds prepared from either one or both of root or stem-bark of *Neobeguea mahafalensis.*

However, in even more preferred embodiments of the invention high-resolution mass spectrometers are used to determine mass-peaks. It is thus preferred to use a mass spectrometer capable of determining mass-peaks with an accuracy of at least 20 ppm, more preferably at least 10 ppm and most preferably at least 5 ppm, to be useful to characterise a material so as to be comprised by the invention.

This means accordingly that it is preferred to use a high resolution mass spectrometer that is capable of determining mass-peak values so that they fall within at least 0.002 %, more preferably within 0.001% , and most preferably within 0.0005% of the true mass value of the compound or chemical introduced into the mass spectrometer. E.g. if the accuracy is 10 ppm (0.001%) a compound having the true mass 687.2684 the mass of that compound could be determined by the mass spectrometer to be as low as 687.2684 - (687.2684 x 0.001 /100) = 687.2615273 amu, or even as high as 687.2684 + (687.2684 x 0.001 /100) = 687.2752727 amu.

Although any high resolution mass-spectrometer can be used, a preferred high resolution mass spectrometer for the purposes of the present invention is Q-Tof2 from Micromass (Macromass/Waters; Waters Corporation, 34 Maple Street, Milford Massachusetts 01757, USA).

Although most ion sources can be used it is preferred in some embodiments of the invention that the high resolution mass spectrometer is equipped with electro spray ionization source, such Z-spray electrospray ionization inlet or nanoflow electrospray ion source. Most preferred is a Micromass nanoflow interface together with glass capillary option.

However, as atmospheric pressure photoionization (APPI) is a novel, alternative ionization method for mass spectrometric analysis, which was developed in order to broaden the group of compounds that can be analyzed towards less polar compounds compared to electrospray, it is preferred in other embodiments of the invention that an APPI ion source is used.

The ionization process in APPI is initiated by photons, emitted from a light source, typically a krypton discharge lamp. The photons ionize compounds that have ionization energies below their energy (10 eV), which include most of larger molecules, but leaving out most of the typically used gases and solvents. Therefore, the analytes can be ionized selectively, with minimum background interference. Furthermore, as the ionization of the analytes is dependent on their ionization energy, rather than their proton affinity, like with electro spray ionization (ECI) and atmospheric pressure ionisation (APCI), ionization of molecules of low polarity by APPI is possible. A preferred APPI for the specific embodiment of the invention when using a Q-Tof2 is PhotoMate® photoionization source from Syagen Technologies Inc. (1411 Warner Ave, Tustin, CA 92780, USA), which is preferably used together with Waters IonSABRE™APCI. (In this embodiment of the invention a Micromass IonSabre APCI unit is combined with the 'Fishbowl' APPI source from Syagen and the solution of the mixture of analyte with is introduced using flow injection.) Moreover as the enriched extract RB, being one of the most preferred extract of the invention, serves among the best sources to determine characteristic mass-peaks which can be used to determine whether or not an extract, drug, component, compound, substantially pure compound, active principle, or pharmaceutical is part of the invention. Accordingly enriched extract RB (prepared according to Example 33) was assayed to determine its mass-peaks with high resolution (see Example 39) which clearly defines the characteristic mass-peaks of the invention which,
when using electrospray ionisation (ESI) with positive ionisation, within 20 ppm, more preferably within 10 ppm and most preferably within 5 ppm amu, are as follows (in amu):

687.2684, 697.2883, 699.2991, 699.3026, 703.2987, 713.2837, 715.2953, 715.2982, 727.2975, 727.3017, 729.2780, 757.3043, 759.2906, 761.3022, 777.3041, 781.2695,

and when using electrospray ionisation (ESI) with negative ionisation, within 20 ppm, more preferably within 10 ppm and most preferably within 5 ppm amu, are as follows (in amu):

655.2738, 671.2746, 671.2945, 697.2562, 697.2562, 697.2579, 699.2670, 713.2837, 713.2840, 713.2840, 713.2845, 713.2880, 715.2991, 717.2317, 725.2812, 729.2712, 743.2921,

and when using atmospheric pressure photo ionization (APPI) with positive ionisation, within 20 ppm, more preferably within 10 ppm and most preferably within 5 ppm amu, are as follows (in amu):

583.2175, 607.5648, 629.3130, 663.4538, 683.2740, 687.2720, 697.2856, 699.2991, 699.2992, 703.2965, 713.2867, 715.2943, 715.2953, 727.2964, 727.3070, 729.2770, 731.2950, 741.3110, 757.3044, 759.2868, 759.2897, 761.3002, 761.3231, 777.2954, 815.3124, 833.2825,

and when using atmospheric pressure photo ionization (APPI) with negative ionisation, within 20 ppm, more preferably within 10 ppm and most preferably within 5 ppm amu, are as follows (in amu):

549.2480, 583.2593, 601.2740, 610.2440, 615.2123, 631.2452, 635.2375, 643.2410, 645.2507, 647.2702, 663.2630, 677.2615, 681.2554, 685.2584, 685.2657, 685.2828, 701.2443, 701.2740, 701.2825, 703.2686, 713.2805, 715.2625, 717.2990, 727.2664, 729.2802, 729.2827, 731.2914, 743.2920, 745.2903, 759.2914.

Accordingly, as an example, a drug, extract, enriched extract, component, compound, substantially pure compound, pharmaceutical, tea-bag, food supplement, dietary supplement, biological sample or material otherwise is deemed to yield the characteristic mass-peak of the invention with the value 687.2684 amu if it is determined by the use of a mass spectrometer to yield a mass-peak that falls within 20 ppm (0.002 %) from the value 687.2684 amu; that is the value falls somewhere between 687.2546546 and 687.2821454 amu (i.e., 687.2684 $\pm$ 687.2684 x 0.002/100 amu), however it is more preferred that the value is determined to be 10 ppm (0.001 %) from the value 687.2684 amu; that is the value falls somewhere between 687.2615273 and 687.2752727 amu (i.e., 687.2684 $\pm$ 687.2684 x 0.001/100 amu),

however it is most preferred that the value is determined to be 5 ppm (0.0005 %) from the value 687.2684 amu; that is the value falls somewhere between 687.2649637 and 687.2718363 amu (i.e., 687.2684 ± 687.2684 x 0.0005/100 amu).

Accordingly due to their importance and ubiquitous presence in *Neobeguea mahafalensis* the characteristic mass-peaks present in extract RB, listed above, are of special interest in characterizing a drug, extract, enriched extract, compound, substantially pure compound, component, pharmaceutical, tea-bag, food supplement, dietary supplement, biological sample or material otherwise, in particular (but not limited to) materials or samples prepared from or derived from *Neobeguea mahafalensis*. Therefore, in preferred embodiments of the invention a material or sample that is characterized by the presence of at least one, more preferably at least two, even more preferably at least tree, even more preferably at least four, even more preferably at least five, even more preferably at least six, even more preferably at least seven, even more preferably at least eight, even more preferably at least ten, even more preferably at least 15, even more preferably at least 20, even more preferably at least 25, even more preferably at least 30, even more preferably at least 40, even more preferably 50, even more preferably 60 and most preferably all of the mass-peaks present in RB, as listed above, is part of the invention provided that it falls within the appended claims.

However, in another preferred embodiments of the invention a material or sample that is characterized by the presence of at least one, more preferably at least two, even more preferably at least tree, even more preferably at least four, even more preferably at least five, even more preferably at least six, even more preferably at least seven, even more preferably at least eight, even more preferably at least ten, even more preferably at least 15, even more preferably at least 20, even more preferably at least 25, and most preferably at least 27 of the mass-peaks present in RB determined by use of ESI, as listed above, is part of the invention provided that it falls within the appended claims.

However, in yet another preferred embodiments of the invention a material or sample that is characterized by the presence of at least one, more preferably at least two, even more preferably at least tree, even more preferably at least four, even more preferably at least five, even more preferably at least six, even more preferably at least seven, even more preferably at least eight, even more preferably at least ten, even more preferably at least 15, even more preferably at least 20, even more preferably at least 25, even more preferably at least 30, and more preferably at least 40, and most preferably all of the mass-peaks present in RB determined by use of APPI, as listed above, is part of the invention provided that it falls within the appended claims.

Also disclosed is a drug, extract, compound, substantially pure compound, pharmaceutical, tea-bag, food supplement or dietary supplement derived from *Neobeguea mahafalensis* in which the characteristic mass-peak(s) can be determined with a high-resolution mass-spectrometer within 20 ppm, more preferably within 10 ppm and most preferably within 5 ppm amu to being any one or both of (in amu) 699.2991 and 715.2953; electrospray ionisation (ESI) with positive ionisation being preferred but any other method generally used for ionization in mass-spectrometry may be applied; e.g. APPI. Accordingly a drug, extract, compound, substantially pure compound or pharmaceutical being derived from *Neobeguea mahafalensis* and determined as said in the present paragraph to having one or both of the characteristic mass-peaks 715.2953 and/or 699.2991 is disclosed herein.

A very specific embodiment of the invention is the drug, extract, enriched extract, compound, substantially pure compound or pharmaceutical which contains a chemical compound with the structure of R306 and/or or R310.

Another very specific embodiment of the invention is the drug, extract, enriched extract, component, substantially pure compound, pharmaceutical and/or chemical which contains a compound having the structural formula of R306 and/or R310.

Also disclosed is the drug, extract, enriched extract, compound, substantially pure compound, pharmaceutical and/or chemical substance preferably derived from (but not limited to) *Neobeguea mahafalensis* which contains a compound with the summary formula $C_{37}H_{46}O_{13}$, and which is capable of eliciting a sexual enhancing effect.

Also disclosed is the drug, extract, enriched extract, compound, substantially pure compound, pharmaceutical and/or chemical substance preferably derived from (but not limited to) *Neobeguea mahafalensis* which contains a compound with the summary formula of $C_{37}H_{46}O_{14}$, and which is capable of eliciting a sexual enhancing effect.

Examples of a separation of root and stem bark extracts by HPLC in the context of characterisation by characteristic mass-peaks are given in Example 24, and Figures 4 and 5 and Example 39, Figures 8 and 9.

For the sake of the present invention the extract, enriched extract, compound, substantially pure compound and/or chemical substance of the invention may be mixed with other extract(s), enriched extract(s), compound(s), substantially pure compound(s) and/or chemical substance(s) of the invention in any desired combination and proportions in order to obtain a mix with desired properties. Such mixtures are particularly useful in many embodiments in the preparation of the pharmaceutical of the invention. A typical example of such a mix is the mix of chemical substances (substantially pure compounds) R306 and R310 in 50:50 % proportion. Such mixtures can be substituted for any constituent in the manufacture of the pharmaceutical of the invention.

A highly preferred embodiment of the invention comprises the pharmaceutical, in particular a pharmaceutical intended for systemic administration, manufactured from the drug, extract, enriched extract, compound, substantially pure compound and/or chemical substance claimed herein. A pharmaceutical preparation is usually obtained by manufacturing a pharmaceutical composition comprising the drug, extract, enriched extract, component, compound, substantially pure compound and/or chemical substance and an optional pharmaceutically acceptable carrier, excipient, dissolvant and/or diluent. In most embodiments of the invention the solvent has been evaporated entirely from an extract of the invention and a residue has been obtained in solid form, as oil or as powder. A powder, in particular a fluffy powder, is particularly desired in the manufacture of capsules and tablets. The residue (dry extract of the invention), enriched extract, component, compound, substantially pure compound and/or substance of the invention may be ground and passed through sieves to obtain suited particle sizes (or fractionated by other means according to particle size) or granulated before combined with the carrier, excipient and/or diluent. In the case the extract is oil it may be triturated to obtain it in crystalline form or it may be dissolved in a suited solvent (e.g. acetonitrile) followed by freeze drying, or spray drying or drying by other means, which usually results in a suited powder very useful in preparing the pharmaceutical of the invention. Alternatively the oil may be dissolved in suited carrier and used in the further preparation of the pharmaceutical.

Most preferred extracts and/or enriched extracts for preparation of the pharmaceutical of the invention comprise RW, RW1, RWExh, RXM, R2C, RCH, RT, REtOH, RB, RB1, RB2, RB3, RB4, RB5, RB6, RB7, RB8, RB9, RB10, R3004, DCM, 01DG2, D-Ac1 D-Ac12, and/or any one of the extracts comprising Peak 1-10 according to Example 39.

Substantially pure compounds preferred for preparation of the pharmaceutical of the invention comprise any one of the components of Peak 1-10 according to Example 39 (i.e. RB1, RB2, RB3, RB4, RB5, RB6, RB7, RB8, RB9, RB10), substantially pure R306 (whether grade of R306 is Crude, Grade 3, Grade 4, Crystalline, or Grade 4 CH) (e.g. prepared as described in any of Examples 41 or 42), and/or substantially pure R310, preferably prepared as described in Example 41.

The preferred chemical substance for use in the preparation of the pharmaceutical of the invention is comprised by R306. R310 is also a highly preferred substance for use in the preparation of the pharmaceutical of the invention. However, any chemical substance of the invention is useful for the preparation of the pharmaceutical of the invention. In the same way any compound having the structure of the invention is equally useful in the preparation of the pharmaceutical of the invention.

Due to the poor water solubility the extract, enriched extract, component, compound, substantially pure compound or chemical substance of the invention they are often subjected to micronization prior to its use for the preparation of the pharmaceutical of the invention. Micronization is a technology well known in the art, which has the purpose to disintegrate a material into fine particles, usually of between 1 to 30 microns of size, e.g. by using a jet-mill, but even finer particles can be made, e.g. nanoparticles, e.g. by applying $CO_2$ supercritical fluid precipitation. Micronization is particularly preferred for (enriched) extracts R2C, RXM, RB, DCM, 01DG2, D-Ac1, D-Ac12, RCH, RT, REtOH and any one of the enriched extracts comprising Peak 1-10 according to Example 39 (i.e. RB1, RB2, RB3, RB4, RB5, RB6, RB7, RB8, RB9, RB10) as well as substantially pure compounds R306 and/or R310, and/or the chemical substance R306 and/or the chemical substance of the invention. The thus micronized sample comprises an important embodiment of the invention.

Also disclosed are capsules. These can be prepared for (but are not limited to) oral use. They are usually prepared by mixing the drug, extract, enriched extract, component, compound, substantially pure compound or chemical substance of the invention with a suited excipient or diluent (filler). Optionally to the mixture is also added preservative(s) and/or stabilizers. In a preferred embodiment of the invention a pharmaceutically acceptable flavouring is also added. (A useful flavouring is ground dried root of ginger; e.g. 0.1-10% ginger, with 0.5-3 % being preferred). Capsules are then filled with the mixture and usually thereafter sealed. In a quite preferred embodiment of the invention the capsules are filled with the extract of the invention as it is, without any additional adding. Following their filling the capsules are often cleaned and polished.

Any type of capsule well known in the art can be used (see e.g. 30). However, two types of capsules are preferred, namely 1) hard capsules and 2) soft gelatin capsules, with hard capsules being most preferred in many embodiments of the invention. Hard capsules are in particular preferred for preparations containing one or several of extracts RW, RW1, RT, REtOH and RWExh. Hard capsules may be manufactured from gelatin but vegetarian capsules are also in use, often manufactured from plant cellulose; usually by modification of cellulose to hydroxypropyl methyl cellulose and can be used for the sake of the pharmaceutical of the invention. Flavoured and coloured capsules are also in use, which can be applied for the sake of the present invention. Any type of capsule suited for systemic administration can be used.

Capsules can be filled directly with the ground dried plant part of *Neobeguea mahafalensis* (i.e. the drug of the invention), with the root and stem bark being preferred, and the root being most preferred. Capsules are usually manufactured so as to contain of between 5 - 1000 mg of the drug of the invention, but other amounts are also possible.

Capsules can alternatively be filled with the extract of the invention, that is the extract from *Neobeguea mahafalensis* after removal of solvent or liquid, together with optional filler, carrier, excipient and/or diluent. Capsules are usually manufactured so as to contain between 0.1 - 1000 mg of the extract of the invention, but other amounts are also possible.

A highly preferred embodiment of the invention is achieved by filling capsules with the enriched extract of the

invention, optionally together with pharmaceutically acceptable filler, carrier, excipient and/or diluent. Capsules are usually manufactured so as to contain between 0.01 - 1000 mg of the enriched extract, but other amounts are also possible.

Capsules can also be filled with the component, pure compound or substantially pure compound of the invention, optionally together with filler, carrier, excipient and/or diluent. Capsules are usually manufactured so as to contain between 0.01 - 100 mg of the component, pure compound or substantially pure compound, but other amounts are also possible.

Capsules can also be filled with the chemical substance of the invention, optionally together with filler, carrier, excipient and/or diluent. Capsules are usually manufactured so as to contain between 0.01 - 100 mg of the component, pure compound or substantially pure compound, but other amounts are also possible.

Before filling capsules the ground drug, extract, enriched extract, component, compound , substantially pure compound and/or chemical substance of the invention can optionally be mixed with pharmaceutically acceptable filler such as (but not limited to) lactose, sucrose or starch. The ground drug, extract, enriched extract, component, compound, substantially pure compound and/or chemical substance of the invention can also be mixed with lubricant/glidant such as (but not limited to) magnesium stearate, stearic acid, talc and/or silicon dioxide if desired. This is particularly useful when a capsule filling machine is used as it eases the filling of the capsules. Wetting agent, such as (but not limited to) lauryl sulphate can be added, as well as disintegrants, such as (but not limited to) sodium starch glycolate, if desired. Antioxidant agents may also be added. Antifungal and/or antimicrobial preservatives may also be added.

In some embodiments of the invention the drug, extract, enriched extract, component, compound, substantially pure compound and/or chemical substance of the invention is suspended in pharmaceutically acceptable suited liquid, such as (but not limited to) pharmaceutically acceptable oil (e.g., peanut oil, corn oil, sesame oil, cotton oil and/or olive oil), glycerol, polysorbate 80, polyethylene glycol, propylene glycol, isopropyl alcohol, to ease the filling of the capsule. This is in particular useful embodiment of the invention when soft gelatin capsules are manufactured. Antioxidant agents may also be added.

One of the most important embodiments of the invention is the pharmaceutical comprising the drug, extract, enriched extract, component, compound, substantially pure compound and/or chemical substance of the invention is solubilised in pharmaceutically acceptable dissolvant(s), e.g. suited pharmaceutically acceptable oil or other pharmaceutically acceptable suited hydrophobic liquid, such as (but not limited to) oil (e.g., vegetable oil, peanut oil, corn oil, sesame oil, sunflower oil, cotton oil, olive oil), triacetin, diacetin, glycerol, polysorbate 80, polyethylene glycol, propylene glycol, isopropyl alcohol. Such pharmaceutical preparations may be used for oral administration. This is because such formulations give good oral bioavailability of the active compound (s) of the invention. This is particularly useful when making pharmaceutical preparations of extract RXM, R2C, DCM, 01DG2, D-Ac 1, D-Ac 12, RCH, RT, REtOH, RB, RB1, RB2, RB3, RB4, RB5, RB6, RB7, RB8, RB9, RB10, R3004, any one of the extracts comprising Peak 1-10 according to Example 39, and compounds R306 and R310. Such solution can be ingested orally and which give good oral bioavailability. This is in particular useful embodiment of the invention when soft gelatin capsules are manufactured. Moreover, affording a solution according to this embodiment of the invention in the same way as stated in this paragraph is used in the manufacture of an injectable for parenteral administration, e.g. the pharmaceutical for subcutaneous injection. Most suited for manufacture of such injectables are any of the extracts RXM, R2C, DCM, RCH, RT, REtOH, 01DG2, D-Ac1, D-Ac12, RB, SB, RB1, RB2, RB3, RB4, RB5, RB6, RB7, RB8, RB9, RB10, R3004, any one of the extracts comprising Peak 1-10 according to Example 39, and compounds R306 and R310 dissolved in vegetable oil such as peanut oil, corn oil, sesame oil, sunflower oil, cotton oil, olive oil. The injectable according to this embodiment of the invention is also an extremely important embodiment of the invention.

Moreover, in a very important embodiment of the invention the extract, enriched extract, component, substantially pure compound, compound and/or chemical substance of the invention is dissolved in pharmaceutically acceptable oil detergent mixture, in oil surfactant mixture, in emulsifiable oil or in self-emulsifying system (see 37) before filling the capsule (preferably a soft gelatin capsule). This formulation is advantageous for many of the enriched extracts of the invention as they have been found to be poorly taken up orally when prepared in other forms. This holds in particular true for enriched extracts where the extraction has proceeded with use of a hydrophobic solvent. Accordingly pharmaceutical preparations for oral use comprising extracts of the invention prepared, at least in part, with the use of hydrophobic solvent(s) (e.g. chloroform), in particular extracts R2C, DCM, 01DG2, D-Ac1, D-Ac12, RB, RXM, RCH, RB as well as substantially pure R306 and R310, the chemical substance R306, and components according to Peak 1-10 of Example 39, as well as the chemical substance of the invention, as well even extracts RT, and REtOH, are preferably manufactured by solubilising or dispersing it in oil such as triglyceride oil (e.g. olive oil, sun flower oil, cotton oil, peanut oil, Neobee M5; tri caprylic/capric triglyceride ester, Miglyol 810, propyleneglycol dicaprylate; Sefsol 228, ethoxylated plant fats, ethanol, or soybean oil, or any other suited oil) and optionally adding or mixing with detergent or surfactant (e.g. polyethylene glycol; e.g. PEG 500, PEG1500 or any other molecular size PEG, polyvinyl pyrrolidone, PVP, of suited molecular weight, e.g. 14 000 or 44 000, Tween, e.g. Tween-20; polyoxyethylenesorbitan monolaurate) and/or pharmaceutically acceptable solvents such as triacetin or diacetin.

Another approach which is highly suited is to use a fat which is solid at room temperature. The fat is liquefied

by heating and the extract or compound of the invention is then solubilised in the fat. The fat is then allowed to stiffen by cooling down and it can then be molded into tablets, casted into capsules, or granulated and used for the preparation of e.g. capsules, tablets, suppositories. Any synthetic, vegetable or animal fat can be used for this embodiment of the invention and include, but are not limited to cocoa butter, coconut fat, hydrogenated fat, hydrogenated animal or vegetable oil, solid triglycerides, solid fat, malleable fat as well as Fattibase™ from Paddock Laboratories, Inc, 3940 Quebec Avenue North Minneapolis, Minnesota 55427, USA, Wecobee™ bases or Witespol™ bases. Additives like cetyl ester wax, beeswax, antioxidants, flavours, colors, and others, and pharmaceutically acceptable solubilisers such as triacetin and diacetin, can be used. Most suited for this embodiment of the invention are extract RXM, R2C, DCM, 01DG2, D-Ac1, D-Ac12, RCH, RT, REtOH, RB, RB1, RB2, RB3, RB4, RB5, RB6, RB7, RB8, RB9, RB10, R3004, any one of the extracts comprising Peak 1-10 according to Example 39, and compounds R306 and R310.

Pharmaceutically acceptable antioxidant agents may also be added. Pharmaceutically acceptable antifungal and/or antimicrobial preservatives may also be added.

Examples of antioxidative agents that can be used as additives in the pharmaceutical of the invention are (but is not limited to): Ascorbic acid, ascorbyl palmitate, butylated hydroxyanisole, butylated hydroxytoluene, hypophosphorous acid, monothioglycerol, propyl gallate, sodium ascorbate, sodium bisulphite, sodium formaldehyde sulfoxylate and sodium meta bisulphite.

Examples of antifungal agents that can be used as additives in the pharmaceutical of the invention are (but is not limited to): Butylparaben, ethyl paraben, benzoic acid, propylparaben, sodium benzoate and sodium propionate.

Examples of antimicrobial agents that can be used as additives in the pharmaceutical of the invention are (but is not limited to): Benzalkonium chloride, benzethonium chloride, benzyl alcohol, cetylpyridinium chloride, chlorobutanol, phenol, phenylethyl alcohol, phenylmercuric nitrate and thiomersal.

The exact amount filled into a capsule depends on the size of the capsules, the properties of the mixture, the method of filling, the degree of packing and other factors, and may be thus subject to variation depending on the conditions used and methods applied, but usually ranges from between 50 to 2500 mg total weight per capsule.

Also disclosed are tablets. These can be manufactured for (but are not limited to) oral, sublingual or buccal use. Tablets are usually manufactured by mixing the drug, extract, enriched extract, component, substantially pure compound and/or chemical substance of the invention with one or several of diluents or fillers, binders or adhesives, disintegrants or disintegrating agents, antiadherents, glidants, lubricants or lubricating agents, and other adjuncts such as colorants and flavourings. Tablets are usually manufactured with one of three basic methods: wet granulation, dry granulation and direct compression (see 31 for an overview of these processes).

Molding is yet another process for manufacture of tablets, which is useful for the sake of the present invention (see 31).

Tablets are usually manufactured so as to contain of between 0.01 - 1000 mg of drug, extract, enriched extract, component, compound substantially pure compound and/or chemical substance of the invention, but other amounts are also possible.

Examples of diluents or fillers for the sake of the present invention, in particular for manufacture of tablets, are (but is not limited to): Lactose, microcrystalline cellulose, starch, powdered sucrose, and calcium phosphate.

Examples of binders or adhesives for the sake of the present invention, in particular for manufacture of tablets, are (but is not limited to): Acacia, alginic acid, carboxymethylcellulose sodium, compressible sugar (e.g., Nu-Tab), ethylcellulose, gelatine, liquid glucose, methylcellulose, povidone, and pregelatinized starch.

Examples of disintegrants or disintegrating agents for the sake of the present invention, in particular for manufacture of tablets, are (but is not limited to): Crosscarmellose, corn, potato starches, sodium starch glycolate, sodium carboxymethyl cellulose, polyvinyl polypyrolodine (PVP), crospovidone, cation-exchange resins and alginic acid.

Examples of glidants for the sake of the present invention, in particular for manufacture of tablets, are (but is not limited to): Colloidal silica, cornstarch and talk.

Examples of lubricants or lubricating agents for the sake of the present invention, in particular for manufacture of tablets, are (but is not limited to): Calcium stearate, magnesium stearate, mineral oil, stearic acid, stearic acid.

Examples of colorants for the sake of the present invention, in particular for manufacture of tablets, are (but is not limited to): FD&C Red No. 3, FD&C Red No. 20, FD&C Yellow No. 2, FD&C Blue No. 2, FD&C Green No. 5, FD&C Orange No. 5, FD&C Red No. 8, Caramel and ferric oxide.

Examples of flavourings for the sake of the present invention, in particular for manufacture of tablets, are (but is not limited to): Anis oil, cinnamon oil, cocoa, menthol, orange oil, peppermint oil, vanillin and ginger. A useful flavouring for the sake of manufacturing a tablet of the invention is dried ground root of ginger; a formulation usually containing between 0.1-10% ginger, with 0.5-3 % being preferred.

Tablets are optionally coated using procedures well known in the art (see 30 for overviews of tablet coating processes).

Yet another very important embodiment of the invention comprises an injectable formulation of the extract, enriched extract, component, compound, substantially pure compound and/or chemical substance of the invention; the

preferred extract for the sake of this embodiment of the invention comprising one or more of R2C, DCM, 01DG2, D-Ac1, D-Ac12, RCH, RT, REtOH, RB, components comprising Peak 1-10 of Example 39 (i.e. RB1, RB2, RB3, RB4, RB5, RB6, RB7, RB8, RB9, RB10), substantially pure R306, substantially pure R310 and/or the chemical substance of R306 and/or R310 and/or the chemical substance of the invention. In a highly preferred embodiment of the invention this pharmaceutical is manufactured by dissolving the extract, enriched extract, component, compound or substantially pure compound of the invention in pharmaceutically acceptable oil (e.g. hydrogenated caster oil, olive oil, peanut oil or any other oil) with an optional hydrophobic carrier (e.g. triacetin, benzyl benzoate or ethyl oleate or a combination thereof), and with optional addition of acylated monoglycerides, propyl dicaprylates/dicaprates, caprylic/capric acid triglycerides, or a combination thereof. Such preparations are useful for (but not limited to) subcutaneous and/or intramuscular injection.

Also disclosed is a solvent-permeable container holding the drug of the invention. Such a container can for example be made from water permeable paper, e.g. making up a 'tea-bag'. The drug of the invention is enclosed in the permeable container. Prior to administration to a human the container is immersed in solvent (e.g. hot or cold water) for a period of time (usually from a few minutes to several days), with optional gentle agitation from time to time. The container is then removed, or the fluid is decanted, and the thus obtained solution is ingested orally. A procedure found to work efficiently is to immerse the solvent-permeable container with the drug of the invention in water at room temperature for several days, e.g. 3-4 days with occasional gentle shaking, thereafter decanting the water extract formed and subsequently ingesting the water extract orally; usually a portion of the water extract is ingested orally every day over a number of subsequent days; e.g. once daily every day for 2-7 days, with 3-4 days of treatment preferred, where after the treatment is stopped. This gives a good treatment effect for sexual dysfunction in both men and woman, and is useful for treatment of (but not limited to) erectile dysfunction, impotence, ejaculatory dysfunction, hypoactive sexual desire disorder and anhedonia, even over a prolonged period of time. The treatment can be repeated with intervals, e.g. every week, every second week, every month, every second month and every 3 month or even every year, with repeated treatments every month being preferred. A solvent extract prepared according to this embodiment of the invention is also particularly useful as a food supplement or dietary supplement, e.g. to increase general well-being and quality of life. The quantity of drug (i.e. ground plant part of *Neobeguea mahafalensis*) placed in a water-permeable container usually ranges (but are not limited to) between 0.1 to 20 g, usually 1 to 5 g and the particle sizes usually vary from (but are not limited to) 0.1 mm to 5 mm, with about 0.3 to 2 mm preferred. Any suited solvent can be used for the purpose of this embodiment of the invention such as (but not limited to) water, purified water, sterilized water, distilled water, acidified water, alkalinized water, ethanol and ethanol/water mixtures, addition of organic acid, citric acid, fruit juice, minced fruit e.g. from fruit like tamarind (*Tamarindus indica*) and/or flavouring. Moreover, in to prevent growth of microorganisms may preservative may be added to the solution, such as sodium benzoate, e.g. water solution from between 250 - 1000 mg/litre of sodium benzoate is suited. The solvent-permeable container can be manufactured using any suited solvent permeable material like (but not limited to) paper, fibres, metal, plastic. Tea-bag paper made from cellulose fibres, paper made of synthetic fibres, or a blend of cellulose and synthetic fibres, is a preferred material for use in the manufacture of the tea-bag. Heat sealable tea-bag paper is also preferred which makes the manufacture of the tea-bag of the invention a simple process.

A solvent-permeable container is illustrated in Figure 13. Such a solvent-permeable container is prepared from heat sealable tea-bag paper by folding the paper doubly with the sealable surfaces contacting each other at the paper's edges and enclosing the ground drug of the invention in between a portion of the two paper surfaces thus formed, and subsequently applying heat to the paper's edges, e.g. by placing the folded paper onto a flat surface and pressing the paper's edges with a hot iron in order to form a seal. Heat-sealable tea-bag paper made of cellulose fibres (e.g. obtained from Changzhou Kakoo Tea Foodstuff Co.,Ltd ., Changzhou, Jiangsu, China) have been found to work highly satisfactory for this purpose. Water-permeable containers manufactured according said principles comprise accordingly highly desired embodiments of the invention. Another specific embodiment is also given in Example 37.

Also disclosed is a solvent non-permeable container enclosing the solvent-permeable container containing the drug of the invention as depicted in Figure 14A. As shown in the figure, the solvent non-permeable container **1403** containing the solvent-permeable container with the drug **1404** is usually equipped with a cap **1402** and an optional seal **1401.** Optionally an additional container with solvent is added so as to form a set of containers as depicted in Figure 14B. The additional container will contain the solvent to be added to the container **1404** holding the solvent-permeable container. The non-water permeable containers are preferably made from (but not limited to) glass or plastic, the cap from rubber or plastic, and the seal from metal (e.g. aluminium) or plastic. The solvent **1406** usually comprises water, buffer, purified water, sterilized water, distilled water, ethanol or ethanol/water mixtures. To the solvent **1406** can be added additives such as (but not limited to) flavouring(s) and/or antioxidant(s) and/or preservative(s). One container **1403** may contain from one to several solvent-permeable containers 1404, with one to three preferred.

Any other pharmaceutical preparation for the systemic administration of the drug, extract, enriched extract, component, substantially pure compound, compound and/or chemical substance of the invention to a human or animal can be used and include (but are not limited to) powders, granulates, suppositories, inserts, lozenges, troches, buccal tablets, sublingual tablets, compressed tablets, multiple compressed tablets, molded tablets, chewable tablets, effervescent tablets, tablet triturates, sugar coated tablets, film-coated tablets, gelatine-coated tablets, enteric-coated tablets, dispensing tablets, hypodermic tablets, extended release tablets, instant disintegrating tablets, immediate release tablets, dermal formulations, ointments, creams, gels, transdermal formulations, solutions, tinctures, injectables, parenterals, implants, formulations for urethral administration, formulations for topical administration, ophthalmic solutions, vaginal formulations, inhalants, disperse systems, emulsions, multitablet systems, microencapsulated drug systems, osmotic pumps, subdermal implants, ocular systems, parenteral systems, vaginal systems, coated bead systems, granules, microspheres, modified-release systems, extended-release systems, delayed-release systems, repeat action systems, targeted release systems, ion-exchange resin systems, slowly eroding or hydrophilic matrix system, inert plastic matrix embedded systems, pills, ointments, creams, gels, water solutions, solutions, mixtures.

There are many options to produce a pharmaceutical from the drug, extract, enriched extract, component, substantially pure compound, compound and/or chemical substance of the invention. Methods suited therefore are described in Ansel, H.C., L.V. Allen, Jr, N.G. Popovich: Pharmaceutical Dosage forms and drug delivery systems (seventh edition). Lippincott Williams & Wilkins. (1999) ISBN 0-683-30572-7, methods and procedures therein.

When preparing tinctures for the sake of the pharmaceutical of the present invention an ethanol concentration of between 40 - 60 % is preferred, with about 50 % being most preferred, which can be used for preparation of tinctures of the drug and/or extract of the invention. However, any proportion of ethanol/water can be used in the preparation of a tincture for the sake of the present invention.

In many embodiments of the present invention preparations or pharmaceuticals comprising only a part and/or a ground part of *Neobeguea mahafalensis,* and/or which comprises only the water solution formed by soaking a part or ground part of *Neobeguea mahafalensis* in cold and/or hot water and/or comprising materials derived from stem bark of *Neobeguea mahafalensis* are specifically excluded. This is because such simple preparations or pharmaceuticals possess toxic principles, as well as they have strong bitter taste and are difficult to handle by the patient and show therefore inferior patient compliance as well as they are potentially harmful to the patient an accordingly not desired in medical treatment.

A pharmaceutical preparation of the present invention can in addition to materials or active principles derived from *Neobeguea mahafalensis* also contain other active principles, such as other pharmacologically active compounds or agents, other drugs, plants, plant parts, plant medicines, herbs, herbal parts, herbal medicines.

Due to the fact that the extract, fraction and preparation of the invention is intended for systemic use preparations intended for topical use are specifically excluded from the invention. Therefore skin make-up is specifically excluded, as well as lotions, creams, ointments or gels, in particular when such are intended for topical or local use, or topical or local treatment. Moreover, all topical, local and dermatological uses of the extract, fraction, preparation, medicament, component, compound, pharmaceutical of the invention are specifically excluded from the invention.

Also disclosed is the use of the drug, extract, enriched extract, component, substantially pure compound, compound, chemical substance of the invention of the invention, and/or pharmaceutical of the invention for the treatment of sexual dysfunction in men and/or woman. Surprisingly, such treatment is very efficacious and affords a very long-lasting effect.

Hereinafter whenever term 'preparation' or 'preparation' of the invention' is used the drug, extract, enriched extract, component, substantially pure compound, pro-component, pro-compound, compound, chemical substance, food supplement, dietary supplement, tea-bag or pharmaceutical falling within the claims is intended.

The sexual enhancing effect of the preparation can be demonstrated in open field studies in the rat or mice, e.g. as is detailed in Examples 17 and 30. In such open field tests male rats or male mice are, respectively, introduced to sexually receptive female rats or mice and a number of sexual behaviours are recorded with respect to number, frequency, latency and/or duration (see Examples 17 and 30 for details).

In such tests very remarkably the preparation of the invention does not give any immediate sexual enhancing effect following its administration. Thus, after 1 hr following e.g. a single oral or subcutaneous dose of it usually no effect is seen. However, after several days of treatment with the preparation a marked sexual enhancing effect is observed. Among rats the sexual enhancing effect can be seen as an increase in the number of couples that engage in sexual activity among treated animals compared with non-treated controls; i.e. the fraction of couples that engage in sexual activity is increased by the treatment with the preparation. In the rat there is also an increase in the copulatory efficacy - i.e., the number of finished copulatory cycles; that is the number of cycles that end with ejaculation is increased. Moreover in the rat there may be a decrease in the length if the copulatory cycle, as well as a decrease or increase in the number of mounts and intromissions in the copulatory cycles (Examples 17 and 22). However, in the mice the sexual enhancing effect of the preparation is distinctly seen as an increase in the number of mounts counted over a period of time, e.g. counting the number of mounts over a period of one, two, three, or four hours following the introduction of a sexual receptive female mouse to the male mouse (Example 30).

The so observed sexual enhancing effect of the preparation is also very long-lasting, as the effect can usually be seen more than one week following the discontinuation of the administration of the preparation; it is often even after two weeks or more following the discontinuation of the treatment.

Even more remarkable efficacious and long-lasting is the sexual enhancing effect of the preparation of the invention in humans upon its systemic administration, in particular when conditions of sexual dysfunction is prevailing, in which case the sexual functions are ameliorated or restored. Even in normal subjects a marked increase in performance (i.e. constituting a marked sexual enhancing effect) can be seen. Most remarkable is also the long duration of the effect. Following a treatment schedule that usually means administering the preparation of the invention every day from one to seven days a positive treatment effect and/or sexual enhancing effect can be noticed even for weeks, months and years following such a single treatment period. Even up to three years following a single period of treatment a positive treatment effect and/or sexual enhancing effect may be seen. (The reason for this long lasting effect may at least in part be psychological).

Positive treatment effects and/or sexual enhancing effects are seen of the drug, extract, enriched extract, component, substantially pure compound, compound, chemical substance and/or pharmaceutical of the invention on erectile functions. In the case of erectile dysfunction or impotence the ability to obtain erection of an incapacitated individual is improved or restored. Effects are seen on the ability to obtain erection, as well as the frequency of erections is increased, and the strength and duration of erections are increased. The treatment thus restores a previous insufficient ability to obtain erection of sufficient strength to allow penetration during intercourse, and it restores the ability to maintain erection to allow completion of intercourse. The frequency of intercourse is also increased and the satisfaction is increased and sexual self-confidence is improved. During sexual activity frequency of ejaculation, orgasm or climax is improved. Sexual desire is also improved. Over all sexual performance and satisfaction is restored; in particular these positive treatment effects and sexual enhancing effects are seen in conditions of erectile dysfunction and conditions related thereto. These positive treatment effects are seen irrespectively of the cause of the erectile dysfunction, whether it is caused by psychological factors, endocrine factors, neurogenic factors, vascular factors, age factors, disease conditions or external factors such as passive and active smoking, or as the side effect upon treatment with medicaments, surgery or other factor(s).

Surprisingly the drug, extract, enriched extract, component, substantially pure compound, compound, chemical substance and pharmaceutical of the invention has also a positive treatment effect on ejaculatory dysfunctions such as premature ejaculation, retarded ejaculation, retrograde ejaculation, anejaculation, aspermia, haemospermia, low volume ejaculate, painful ejaculation and anhedonia. In these cases the malfunctions are ameliorated or abolished. In particular positive treatment effects are seen on premature ejaculation and anhedonia.

Positive treatment effects and/or sexual enhancing effects of the drug, extract, enriched extract, component, substantially pure compound, chemical substance and pharmaceutical of the invention are also seen on hypoactive sexual desire disorders (HSDD). In these conditions the drug restores and/or increases and/or improves sexual fantasies, thoughts and/or desire. Interest for and receptivity to sexual activity is also usually increased. Arousal is usually improved or restored, eventual pain during intercourse may be diminished or abolished, ability to have orgasm is as a rule increased or restored, sexual feelings and receptivity for sexual activity are as a rule increased. Motivation for having sexual activity is usually increased or restored. Moreover, they are seen irrespectively if the cause is due to psychological, psychiatric, endocrine or genetic factors, or other factors. These positive effects are seen in both man and woman.

The sexual enhancing effect of the preparation of the invention can in humans be assessed using the 'International Index of Erectile Function' (IIEF) or the reduced version of IIEF, IIEF-5 questionnaires (see reference 13). An increase in the scores of these questionnaires demonstrates the sexual enhancing effect of the preparation of the invention. Also direct methods for assessing frequency and/or strength of erections can be used, such as Rigiscan; an increase in frequency and/or strength of erection upon treatment with the preparation of the invention demonstrates its sexual enhancing effect.

As a result of its sexual enhancing effect the over all improvement of sexual performance and fertility is improved or restored with the administration of the preparation of the invention.

The sexual enhancing effect of the preparation of the invention can also been demonstrated clinically in males, as is further detailed in Examples 18-21.

A further use of the drug, extract, enriched extract, component, substantially pure compound, compound, chemical substance and/or pharmaceutical of the invention is to restore or improve fertility in animals. This is desired in animal breeding to increase production and also in saving endangered species. It is a common finding that animals kept in unnatural environment loose their interest to engage in sexual activity and due to this reason it can become difficult and even impossible to obtain an offspring from them. Administration of the preparation of the invention can in these cases be beneficial and improve the ability to obtain offspring from the animals.

A most important embodiment of the present invention is to afford a long-lasting effect on sexual disorders. After the treatment with the preparation of the invention, using appropriate dosage schedules, restoration of sexual functions and amelioration of the dysfunction(s) are seen over extended periods of time lasting from weeks, months and even years, even after a single treatment occasion (e.g. even after scheduling the drug in single or divided doses e.g. over one, two, three days or a week).

The drug of the invention is usually administered in dosages referring to the dry weight of the plant part used.

In humans it is usually administered in single or divided doses amounting to between 1 mg and 100 g, more preferably between 10 mg and 10 g, even more preferably between 100 mg and 1 g of the dried material.

However, when the extract of the invention has been prepared the dosage is usually referred back to the original dry weight of the plant part used for the extraction. Thus, for example, if the dry weight of the original plant part used for the extraction was 5 g and the water or solvent extraction or by extract obtained other means yields a residue with a weight of 1 g, the amount given of the extract should be divided by 5 compared to the weight of the dried plant part. Accordingly the preferred dosage of such an extract is between 0.02 mg and 20 g, more preferably between 2 mg and 2 g, even more preferably between 20 mg and 0.5 g. Such doses are administered in single or divided doses.

However, yet another way of basing dosage is to perform a bio-assay of the materials intended to be administered to a human or animal (in this context termed 'preparation'). A standard bioassay to assess the activity of a preparation is, in principle performed as follows:

Sexually naïve male rats (220-250g) are divided into groups of at least 6 rats in each group. In each group each rat is treated with the same oral dose of the preparation each day on three consecutive days. The different groups are given different doses of the preparation (usually the preparation is dissolved or dispersed in a small volume of water or other liquid which is given orally by a syringe). However, in many embodiments of this part of the invention administration is preferably done by subcutaneous injection after solubilisation of the preparation in oil (e.g. sun flower oil). (Hereinafter the solvent/dispersant for the preparation is termed 'vehicle'). However, such oil solutions may also be administered orally with essentially the same results. Usually a schedule using logarithmically increasing dosages, according to a pattern (in principle) .... 0.1, 0.3, 1, 3, 10, 30 mg/kg body weight of the rats... etc, is used. A dosage schedule like (in principle) ... 0.004, 0.04, 0.4, 4, 40, 400 ... mg/kg may also be used. Moreover, a control group of at least 6 rats is given an equal volume of the vehicle for the preparation. In the case a drug, pharmaceutical preparation, tea-bag, food-supplement or dietary supplement is being assayed it can also be directly administered to the rats, or a proper extraction procedure can be applied on to it in advance prior to the administration.

Thus, for example, in group 1 each one of eight rats are given vehicle only (i.e. without preparation serving as a control) once a day on three consecutive days, in group 2 each one of eight rats are given 1 mg/kg of a preparation once a day on three consecutive days, in group 3 each one of eight rats are given 3 mg/kg of the preparation once a day on three consecutive days, in group 4 each one of eight rats are given 10 mg/kg of the preparation once a day on three consecutive days, etc. (This dosing needs to be adjusted according to the actual activity of the preparation applied. Thus for a high activity preparation lower doses is used albeit but with the same ratio of dosing interval. For a lower activity preparation higher doses with the same ratio of dosing interval is used).

On the 4th, 7th or 14th day following the first administration of the preparation each male rat is placed individually into a clear plastic observation chamber (dimensions usually approximately 36 x 24 x 33 cm, length x width x height) for a 30 min adaptation period prior to the introduction of one sexually naive female rat (220-250g), said female rat having been brought to sexual receptivity by sequential treatment with estradiol benzoate (12 $\mu$g/rat; 48 h pre-session injection time) and progesterone (500 $\mu$g/rat; 4.5 h pre-session injection time).

The couple is then observed for 30 minutes for engagement of sexual activity using sexual behaviour test (SBT) as is detailed in Example 17. The percentage of sexually active couples are calculated in % for each group (i.e., by sexually active couples are intended males engaging in any of mounting the female from the rear, intromission and ejaculation; see 'Recorded elements of SBT' under Example 17). Alternatively, the copulatory efficacy index is computed (by copulatory efficacy index is intended the average number of finished copulatory cycles, i.e., cycles that end with ejaculation, in each group; see under' Recorded elements of SBT' under Example 17).

A dose response curve is then constructed and the effective dose 50 (ED50) is calculated by visually reading from the curve, or e.g. as is further detailed in Example 23. The estimated dose in mg/kg rat body weight capable of inducing a 50 % increase of the maximally attainable increase in sexual activity by high dose(s) of the preparation (i.e., the 100 % level, in other words the level where increasing the dose does not substantially yield an increase in the effect) over the base line (i.e., the % sexually active couples in the control group, or copulatory efficacy index for the control group) is then estimated (this is further clarified in Example 23). Using the ED50 (expressed in mg/kg rat body weight) the Activity of 1 mg of the preparation expressed as Units (U) is then defined from the formula:

$$Activity = \frac{1}{ED_{50}}$$

Thus, one (1) mg of a preparation has an Activity of one (1) U when this preparation gives an $ED_{50}$ of 1 mg/kg in the above-described bio-assay. In another example, if a preparation yields an $ED_{50}$ of 25 mg/kg one (1) mg of this preparation has an Activity of 1/25 = 0.04 . In yet another example if a preparation yields an $ED_{50}$ of 0.02 mg/kg one (1)

mg of it has an Activity of 1/0.02 = 50 U.

In a further sense a preparation where 1 mg shows an activity of e.g. 3 U (i.e. 3 U/mg), 500 mg of that preparation corresponds to an Activity of 3 x 500 = 1500 U. In yet another example, a preparation where one mg shows an Activity of 0.05 U (i.e. 0.05 U/mg), 700 mg of that preparation corresponds to 0.05 x 700 = 35 U.

In order to distinguish units computed based on $ED_{50}$ from the % sexually active couples from that based on copulatory efficacy index the U measure is often affixed a subscript such that $U_{sac}$ refers to units computed from $ED_{50}$ values obtained using the sexually active couples measure, and $U_{cei}$ refers to units computed from $ED_{50}$ measures based on copulatory efficacy indices. While $U_{cei}$ is very useful, generally $U_{sac}$ is preferred.

However, in many cases it is preferred that mice are used instead or rats in the bioassay described above under 'standard bioassay'. In this case rats are substituted for mice and the assay performed essentially in the same way as described for Example 23 and above for the standard bioassay with use of rats, except that the number of mounts during an one, two, three or four hour period is counted using the approach, pre-treatments and schedules essentially as described in Example 30. The activity is then calculated and obtained in units, U, in the same way as described under the standard bioassay, when using rats. However, sometimes the units obtained when using mice are termed $U_{mnt}$, or simply U, as it is usually clear from the context that mice were used in the determination of the activity. For example, using this approach 1 mg of substantially pure R306 was determined to yield an activity of 15 U (Example 43). In fact for the sake of the present invention bioassay using mice is preferred as this species gives a very clear signal which is easy to record (i.e. the count of mounts over a period of 1, 2, 3 or 4 hours). Also in mice the assays are performed 4, 7 or 14 days following the start of the treatment comprising 3 doses of the same amount given to each male mouse once daily for 3 days.

Accordingly using bioassay the dosage of a drug, extract, enriched extract, component, compound, substantially pure compound or pharmaceutical according to the invention can be based on Units, U, $U_{sac}$, or $U_{mnt}$. For the sake of the present invention 'U', 'Units' and 'Units of activity' is *mutatis mutandis* intended to mean the same thing, but the species used in the assay (i.e. mouse or rat) needs to be specified. Moreover, for the sake of the present invention when deciding upon suited dosages the units for doses give herein, U can be substituted with any of $U_{sac}$, $U_{cei}$, or $U_{mnt}$ with essentially the same result.

In order to assess the sexual enhancing effect of a material of the present invention the assay where mice are used is preferred. Accordingly for most embodiments of the present invention the unit activity U refers to $U_{mnt}$.

The drug, extract, enriched extract, component, compound, substantially pure compound, chemical substance, compound or pharmaceutical of the invention is thus usually administered in single or divided doses comprising between 0.01 to 1000 U, more preferably between 0.1 to 100 U, even more preferably between 0.2 to 40 U, but other dosages may also be applied.

The compound of the invention (chemical substance of the invention), such as R306 or R310, is usually administered in doses ranging 400 to 0.004 mg/kg body weight, even more preferably 400 to 0.004 mg/kg body weight, even more preferably 40 to 0.004 mg/kg body weight, even more preferably 4 to 0.004 mg/kg body weight and most preferably 4 to 0.04 mg/kg body weight.

In the rat and mice the preferred dosages are usually 10-fold higher than in humans. For other species the dosages may be adjusted in order to obtain an optimal effect but would in most cases be somewhere between or close to the dosages required in rat, mice and humans.

In view of the long-lasting effect of the preparation of the invention it is usually administered in intervals with long durations in between. A common treatment schedule is to give one dose each day for three consecutive days ('priming period'), where after no treatment is given for a prolonged period of time ('no-treatment period'). Other treatment schedules are also possible ranging from one single dose to several divided doses; these doses being given in one day, or over several days, or over one week or several weeks ('priming period'). After this time a prolonged period of time follows when no treatment is given ('no-treatment period'). This usually affords a desired sexual enhancing effect that last for several weeks, even for several months, even very for one year, or even for several years. (Due to this property the preparation of the invention is said to have a long-lasting effect.) Usually the effects declines slowly during the no-treatment period and the treatment can then be iterated at suited time points to restore the desired effect on sexual functions. For the sake of the present patent this type of treatment is termed 'intermittent treatment', which comprise treatment 'priming period' followed by 'no-treatment period' in an iterated fashion.

In other treatment regimes a very low dose is given continuously (e.g., every day, every week or every month). In such a case a long-term sexual enhancing effect of the preparation of the invention is obtained. However, for such a treatment schedule to be effective usually the dosage is kept very low (often in order of 10 - 1000 fold lower than for the intermittent treatment regime). Herein this type of treatment is termed 'continuous treatment'.

Sometimes the two treatment regimes are combined. Thus, initially a high single 'loading dose' or a couple of high single loading doses are given over a period of 1 day to one week. This is then followed by maintenance doses given at divided intervals, e.g., every day, every week, every second week, every month, every second month, every fourth month, every half-year, every year, even every second year, or even every third year. In these cases the preferred

loading dose(s) (referring to the original dry weight of the plant part) is between 1 mg and 100 g, more preferably between 10 mg and 10g, even more preferably between 100 mg and 1 g, while the maintenance dose is usually in the order of 10 - 1000-fold lower than the loading dose(s). When expressed in units U the loading dose is usually 500 - 50 000 U while the maintenance dose is usually 10 - 1000 fold lower than this.

Also disclosed is a procedure for finding an optimal treatment regime in humans or animals comprising giving the drug, extract, enriched extract, component, compound, substantially pure compound, chemical substance or pharmaceutical of the invention in different doses for different period of times and then comparing the efficiency of the treatments. Also disclosed is a method to find the optimal composition of the extract of the invention (i.e., with respect to procedure of manufacture, purity, presence of characteristic mass-peaks, etc.) as well as the procedure of manufacture of a pharmaceutical, food supplement or dietary supplement thereof comprising administering different versions of the extracts, pharmaceuticals, food supplements or dietary supplements of the invention prepared in different ways and then administering them to humans or animals and comparing groups of humans or animals that have obtained different extracts or pharmaceuticals of the invention. In humans a preferred method of assessing the efficiency of the treatment in man for the purpose of such studies is the use of the 'International Index of Erectile Function' (IIEF) or the reduced version of IIEF, IIEF-5 (see 13). Also direct methods for assessing frequency and/or strength of erections can be used, such as Rigiscan. However, also other qualities rather than only measuring sexual qualities, e.g. relating to toxicity, safety and pharmacovigilance need to be assessed to assure the safety of the treatment.

As the material of the present invention is intended for systemic use an extract, enriched extract, component, substantially pure compound, compound, chemical substance, pharmaceutical, organic compound, synthetic compound, extract derived from plant (even extract from a plant not being *Neobeguea mahafalensis*), extract from animal and extract from microorganism can be checked whether or not it is part of the invention by analysing the occurrence of characteristic mass-peaks in the body fluids (e.g. serum, plasma, urine, cerebrospinal fluid) of an animal or human following the systemic administration of the extract, enriched extract, component, pro-component, substantially pure compound, compound, pro-compound, pharmaceutical, organic compound, synthetic compound, extract derived from plant (even extract from a plant not being *Neobeguea mahafalensis,* extract from animal and extract from microorganism. This is typically performed by collecting the body fluid (whole blood or blood plasma being preferred, with blood plasma being most preferred) from the human or animal some time after the systemic administration (per oral administration or subcutaneous injection being preferred, with subcutaneous injection being most preferred) of the material under investigation to the human or animal (usually from 1 to 24 hours is suited) and then extracting the body fluid using suited solvent (e.g. acetonitrile), concentrating the solvent or evaporating the solvent entirely. Usually this is followed by further preparation procedures, e.g. diluting the concentrated body fluid extract or dissolving the dried body fluid extract in a suited new solvent (e.g. hexane, ethylacetate, methylenechloride or mixtures thereof) and subjecting the solution to single or repeated partition with water or water solution (e.g. sodium hydroxide solution or hydrochloride solution) and after phase separation collecting the organic phase and optionally concentrating it, or drying it to completeness and again diluting or dissolving the material in organic solvent (e.g. hexane, ethylacetate, methylenechloride or mixtures thereof), and then optionally subjecting it to a new partition with water or water solution (e.g. sodium hydroxide solution or hydrochloride solution) and after phase separation collecting the organic phase and concentrating it, or drying it to completeness, and again dissolving in organic solvent (e.g. hexane, ethylacetate, methylenechloride or mixtures thereof), and finally injecting it into a HPLC column connected to a mass-spectrometer (or directly injecting it into the mass-spectrometer skipping the HPLC step). The chromatogram (mass-spectra) is then observed for the occurrence of mass-peaks falling in the range of those listed above for characteristic mass-peaks. It is preferred that at least one, more preferably at least two, more preferably at least three, more preferably at least four, more preferably at least five, more preferably at least six, more preferably at least seven, more preferably at least eight, more preferably at least nine, more preferably at least ten, more preferably at least 15, more preferably at least 20, more preferably at least 23, more preferably at least 27 characteristic mass-peaks are then detected in the biological sample in order for the extract, enriched extract, component, pro-component, substantially pure compound, compound, pro-compound, tea-bag, food supplement, dietary supplement, pharmaceutical, organic compound, synthetic compound, extract derived from plant, extract from animal and extract from microorganism to be comprised by the invention. It is preferred for this embodiment of the invention that the accuracy of the mass spectrometer is at least 20 ppm, more preferably at least 10 ppm and most preferably at least 5 ppm. Solid phase extraction of the body fluid is also a method, well known in the art, which can be used in conjunction with above methods for detection of characteristic mass-peaks.

The drug, extract, enriched extract, component, compound, substantially pure compound and chemical substance of the invention comprise a new principle in pharmacology and are therefore highly desired tools for investigating the new principle's mechanism of action for eliciting a sexually enhancing effect. Investigating the mechanism of action of the new principle is highly desired in the further work of finding novel compounds, whether such are natural or synthetic, even such that are structurally distinct from the chemical substance of the invention, but that can still substitute for the chemical substance of the invention as they elicit the same type of effect in the human or animal.

In such investigations the drug, extract, enriched extract, component, compound, substantially pure compound

and chemical substance of the invention, with the chemical substance of the invention being preferred, can be used as such or it can first be made radioactive or it can be derivatised by chemical means; e.g. adding additional chemical groups or markers to it, or attaching it to a solid support (e.g. affinity chromatography media).

Any one of Substructure-306(1) ... Substructure-306(21), Substructure-306(U1) ... Substructure-306(U6), Substructure-306(T1) ... Substructure-306(T4) defined herein above provide a substructure which can be incorporated into novel chemical compounds. After synthesis of such compounds the new compounds are assayed for possible sexual enhancing activity and the synthesis is iterated until compound with desired sexual enhancing activity is found. Such novel synthetic compounds may find important uses as they may be easy and cheap to prepare while still eliciting the same sexual enhancing activity as the chemical substance of the present invention.

The chemical substance of the invention is also highly desired for investigating its ADMET properties (i.e. absorption, distribution, metabolism, excretion and toxicology). This is because it is highly desired to screen and select the chemical substance of the invention with good (preferably the best) ADEMET properties for use in treatment of sexual dysfunction.

Further uses and embodiments of the invention are evident from the attached examples and amended claims, which are not intended to limit the invention in any way.

**Brief description of figures**

**Figure 1:** Parts of *Neobeguea mahafalensis* shown schematically. Shown at **101** is a branch with fruit; at **102** an inflorescence; at **103** and **104** flowers after regression of the perianth; at **105** petal; at **106** and **107** anther; at **108** anther; at **109** schematic cross-section of pistil; at **110** immature fruit; at **111** adult fruit; at **112** flower bud in its place; at **113** down.

**Figure 2:** Schematic depiction of Soxhlet apparatus and procedure for Soxhlet extraction of stem bark of *Neobeguea mahafalensis.* Stem bark can substitute for any portion of *Neobeguea mahafalensis* with root being preferred.

**Figure 3:** HPLC profiles of extract RA, RB, RC and R2C using a LiChroprep RP-18 4.6 x 250 mm column, eluent - linear gradient from 20% acetonitrile in 5 mM ammonium acetate to 80% acetonitrile in 5 mM ammonium acetate in 60 min, flow rate 1.0 ml/ min, UV detection at 260 nm. Indicated by the separations (dashed lines) on the R2C chromatogram is the preferred peaks to be comprised in, respectively, extracts RA, RB and RC.

**Figure 4:** Exemplary mass spectra of root extract R2C from *Neobeguea mahafalensis* obtained with APCI ion source. Top pan: Summary ion intensity of LC/MS experiment. Middle pan: positive ions observed at 54.55 min. Bottom pan: negative ions observed at 54.58 min.

**Figure 5:** Exemplary mass spectra of stem bark extract S2C from *Neobeguea mahafalensis* obtained with APCI ion source. Top pan: Summary ion intensity of LC/MS experiment. Middle pan: positive ions observed at marked time interval. Bottom pan: negative ions observed at marked time interval.

**Figure 6:** Exemplary electrospray mass spectra of root extract R2C from *Neobeguea mahafalensis,* positive ionization. Spectra obtained on a Q-Tof2.

**Figure 7:** Exemplary electrospray mass spectra of stem bark extract S2C from *Neobeguea mahafalensis,* negative ionization. Spectra obtained on a Q-Tof2.

**Figure 8:** Retention times of components of the RB extract when analyzed an HPLC column (4.6 x 250 mm) filled with LiChrosorb RP18, particle size 5 microns, eluted by linear gradient from 20 to 80% acetonitrile in water + 0.1% trifluoroacetic acid, flow rate 1.0 ml/min (listed under the "Retention time, min" column), UV absorbent properties of these components recorded as wavelength in nanometers for UV maxima (listed in the "UV maxima (nm)" column) and the mass spectral data of these components obtained on Q-Tof2 with positive and negative ionization using ESI and APPI ion sources. The mass peaks for the latter are listed in the "Positive ionization (%)" and "Negative ionization (%)", "ESI" and APPI", columns, and are given in atomic mass units, amu, together with the intensities of the mass peaks given in % of the intensity of the most intense peak for each respective analysis mode for the cases that several mass-peaks were detected for a particular component. Figure 8 shows the data for 7 different components numbered 1-7 as indicated in the "Peak number" column. The table is continued in Figure 8 shown in additionally 3 components, labelled 8-10. (For further details see Example 39).

**Figure 9:** Table continued from figure 8 showing the data for components 8-10. The order of columns and their labels are the same as in Figure 8.

**Figure 10:** Analytical HPLC of R306 (Grade 4) on LISPRP18-5-3627 using isocratic water/2-propanol (60:40) as eluent (isocratic regime) at flow rate - 0.7 mL/min; detection at 220 nm

**Figure 11:** Absorption maxima were seen at 198, 215 and 261 nm. UV spectrum of R306 (Grade 4) recorded on-line with a photodiode array detector from the HPLC run shown in Figure 10. Absorption maxima were seen at 198, 215 and 261 nm

**Figure 12:** Assay of sexual enhancing effects of different doses of substantially pure compounds R306 and R310 in male mice at different post-treatment time points. The number of mounts were in these tests counted during a 3 hour period following the introduction of the sexually receptive female mice.

**Figure 13:** Schematic depiction of solvent permeable container containing the drug of the invention, manufactured from heat-sealable teabag paper; **1302** top view; **1301** and **1307** cross-section side views. The container is manufactured by folding heat-sealable tea-bag paper so as to enclose a portion of the ground drug of the invention (placement if drug illustrated at **1303, 1306** and **1309)** and sealing at the paper edges. The seal is formed by applying heat at the paper's edges;

indicated at **1302, 1305** and **1308).**

**Figure 14:** Schematic drawings of containers containing the drug of the invention. A. Outer non-solvent permeable container containing within it a solvent permeable container, the latter which contains the drug of the invention. At **1401** optional seal, at **1402** cap, at 1403 solvent non-permeable container, at **1404** solvent permeable container enclosing a portion of the ground drug of the invention. B. Set of containers. At **1404** the capped and sealed container of Figure 14A. At **1405** an optional additional container containing solvent **1406.**

**Figure 15:** $^{1}H$ NMR spectrum for R306.

**Figure 16:** Expanded $^{1}H$ NMR spectrum for R306.

**Figure 17:** HMBC 2D NMR spectrum for R306.

**Figure 18:** NOESY 2D NMR spectrum for R306.

**Figure 19:** $^{13}C$ NMR spectrum for R306.

**Figure 20:** $^{1}H$ NMR spectrum for R310.

**Figure 21:** Expanded $^{1}H$ NMR spectrum for R310.

**Figure 22:** Expanded $^{1}H$ NMR spectrum for R310.

**Figure 23:** Expanded $^{1}H$ NMR spectrum for R310.

**Figure 24:** HMBC 2D NMR spectrum for R310.

**Figure 25:** NOESY 2D NMR spectrum for R310.

**Figure 25:** COSY 2D NMR spectrum for R310.

**Figure 26:** TOCSY 2D NMR spectrum for R310.

**Figure 27:** $^{13}C$ NMR spectrum for R310.

**Figure 28:** Diagnostic $^{1}H \rightarrow {}^{13}C$ HMBC connectivities in compound R306.

**Figure 29:** Diagnostic $^{1}H \rightarrow {}^{13}C$ HMBC HMBC connectivities in compound R310A.

**Figure 30:** Diagnostic $^{1}H \rightarrow {}^{13}C$ HMBC HMBC connectivities in compound R310B.

**Figure 31:** Diagnostic NOE $^{1}H$-$^{1}H$ interactions in compound R306.

**Figure 32:** Diagnostic NOE $^{1}H$-$^{1}H$ interactions in compound R310A.

**Figure 33:** Diagnostic NOE $^{1}H$-$^{1}H$ interactions in compound R310B.

**Figure 34:** Assays for the contents of R306 in root and stem-bark extracts from *Neobeguea mahafalensis* using LC/MS. Samples were injected onto a LiChrosorb RP18-5 (2.1 mm x 100 mm, 5 $\mu$m) HPLC column (Merck Chemical Co., Germany) attached to a Perkin Elmer PE SCIEX API 150EX mass spectrometer and eluted with a gradient formed from water and acetonitrile (from 20% to 90% acetonitrile) with a 5 mM ammonium acetate additive during a 60 min period; flow rate 0.2 mL/min. Detection of ion-currents versus time at 699.4 atomic mass units occurring around 36.8 min following the injections. **a** - **c)** Responses from the indicated amounts of pure R306 injected onto the HPLC column. **d** - **f)** Responses of, respectively, 20, 10 or 4 $\mu$g of an R2C extract from root of *Neobeguea mahafalensis*; the content of R306 in this sample being estimated to be 1.8 %. g) Response to 20 $\mu$g of an S2C extract from stem-bark of *Neobeguea mahafalensis.* A minor response around 36.99 min is observed; the eventual content of R306 in the S2C sample, if present, is from this recording estimated to be less than 0.1 %. Note that the number above each peak refer to the time point for HPLC peak maximum being detected by the spectrometer; the number to the right of this number corresponds to the ion current at peak maximum; i.e. for panel a) the elution time was 36.90 min with the peak maximum ion current amounting to $3.89 \times 10^{6}$ cps (counts per second).

**Figure 35:** Overview of an extraction procedure for obtaining an enriched extract of *Neobeguea mahafalensis.*

**Figure 36:** Number of active couples (in %) after RW treatment. (The Y-axis is expressed in % active couples in the respective group). * = p<0.05 vs control, Chi-square test.

**Figure 37:** Copulatory efficacy in rats treated with RW. # = P < 0.05 vs corresponding group day 0.

**Figure 38:** Effect of RW on copulatory cycle length. # P < 0.05 vs corresponding group day 0.

**Figure 39:** Copulatory efficacy in rats treated with R2W, R2C or R2P for 3 days. The copulatory efficacy was estimated on day 7 following the start of the experiment. C=Control.

**Figure 40:** Number of mounts in cycle for rats treated with R2W, R2C or R2P for 3 days. The number of mounts was estimated on day 7 following the start of the experiment. C=Control. Note that the R2P group did not finish any full cycle during the evaluation. * P < 0.05 vs. control group.

**Figure 41:** Data for the number of active couples plotted in a semi logarithmic diagram.

**Figure 42:** Data for the copulatory efficacy index plotted in a semi logarithmic diagram.

**Figure 43:** Assay of root extracts of *Neobeguea mahafalensis* in mice sexual behaviour test.

**Figure 44:** Assay of enriched extract of RW1 from *Neobeguea mahafalensis* in mice sexual behaviour test.

**Figure 45:** Assay of enriched extracts prepared from *Neobeguea mahafalensis* on sexual behaviour in mice.

**Figure 46:** Comparison of sexual activity enhancing activity of extracts from stem bark and root of *Neobeguea mahafalensis.* *** =p<0.0005 Student's non-paired two-tailed test.

**Figure 47:** Determination of the content of R306 in R2C and S2C preparations.

**Figure 48:** R2C oral treatment - 4th day.

**Figure 49:** R2C oral treatment - 7th day.

**Figure 50:** R2C subcutaneous treatment.

**Figure 51:** Assay of sexual enhancing effect of DCM, 01DG2 and D-Ac1 extracts.

## EXAMPLES

All samples of *Neobegua mahfalensis* used herein were collected from Madagascar with permit from Ministere de l'evironment, des eaux et forets et du tourisme, Repoblikan'i Madagaskara, B.P. 243 -Nanisana - ANTANANARIVO - 101, Madagascar.

### Example 1: Water extract of stem bark of *Neobeguea mahafalensis*

50 - 100 g of stem bark from *Neobeguea mahafalensis* was placed in 200-1000 ml of water at room temperature for 3 days. The water turned brown. After removing the water by decantation the water solution was ingested orally as a pharmaceutical.

### Example 2: Tincture of root of *Neobeguea mahafalensis*

0.5 g of ground root from *Neobeguea mahafalensis* was placed in 2 - 4.5 ml of ethanol/water (between 50 - 99 % ethanol was used) and allowed stand for 3 days with gentle shaking. The root was sedimented and the supernatant decanted and filtered. The sediment was pressed to yield additional solution, which was also filtered. Portions of the combined supernatant solution (i.e. REtOH extract) were ingested orally as a pharmaceutical.

### Example 3: Ethanol extract of root of *Neobeguea mahafalensis*

<u>Example **3a**</u>

0.5 g of ground root from *Neobeguea mahafalensis* was placed in 4.5 ml of 50 % aqueous ethanol and gently shaken for 3 days at room temperature. The mixture was then filtered through a lump of cotton wool placed in a conical funnel; afterwards the extracted root on the lump was rinsed with the same solvent (2 x 1.5 ml). The combined filtrates were evaporated to dryness; the residue was dissolved in 30 % acetonitrile in water (10 ml) and freeze-dried. A brown, fluffy powder formed. Yield 133.2 mg. The residual root remaining after extraction was dried in the air keeping it on an open Petri dish. Residual dried root weighed 332 mg.

<u>Example 3b</u>

0.5 g of ground root from *Neobeguea mahafalensis* was placed in 4.0 ml of 70 % aqueous ethanol and gently shaken for 3 days at room temperature. The procedures following were then as described for Example 3a. Yield of lyophilized powder was 127.5 mg. Residual dried root weighed 352 mg.

<u>Example 3c</u>

0.5 g of ground root from *Neobeguea mahafalensis* was placed in 2.5 ml of commercial 95.5% ethanol and gently shaken for 3 days at room temperature. The procedure following was then as described for Example 3a. Yield of lyophilized powder was 57.5 mg. Residual dried root weighed 425 mg.

Herein ethanol extracts according to Examples 3a, 3b and 3c are collectively termed REtOH.

### Example 4: Acetone extract of root of *Neobeguea mahafalensis*

0.5 g of ground root from *Neobeguea mahafalensis* was placed in 2.5 ml of acetone and gently shaken for 3 days at room temperature. The mixture was then filtered through a lump of cotton wool placed in a conical funnel; afterwards the extracted root on the lump was rinsed with the same solvent (2 x 1.5 ml). The combined filtrates were evaporated to dryness; the residue was dissolved in 30 % acetonitrile in water (10 ml) and freeze-dried. powder, herein termed extract RT, was 28.7 mg. Residual root weighed 458 mg.

**Example 5: Ethanol extract of stem bark of *Neobeguea mahafalensis***

Example 5a

0.5 g of ground stem bark from *Neobeguea mahafalensis* was placed in 2.5 ml of 50% aqueous ethanol and gently shaken for 3 days at room temperature. The mixture was filtered through a circle of filter paper (diameter about 10 mm) placed in a glass filtering funnel; afterwards the extracted stem bark on the filter was rinsed with the same solvent (2 x 1 ml). The combined filtrates were evaporated to dryness, the residue dissolved in 30% acetonitrile in water (10 ml) and freeze-dried. A brown, fluffy powder formed. Yield 99.1 mg. The residual stem bark after extraction was dried in the air keeping it on an open Petri dish. Residual dried root weighed 366 mg.

Example 5b

0.5 g of ground stem bark from *Neobeguea mahafalensis* was placed in 2.5 ml of 70% aqueous ethanol and gently shaken for 3 days at room temperature. The procedure following was then as described for Example 5a. Yield of lyophilized powder was 82.9 mg. Residual dried stem bark weighed 391 mg.

Example 5c

0.5 g of ground stem bark from *Neobeguea mahafalensis* was placed in 2.5 ml of commercial 95.5% ethanol and gently shaken for 3 days at room temperature. The procedure following was then as described for Example 5a. Yield of lyophilized powder was 44.0 mg. Residual dried stem bark weighed 439 mg.

Example 5d

0.5 g of ground stem bark from *Neobeguea mahafalensis* was placed in 2.5 ml of commercial 99.5% ethanol and gently shaken for 3 days at room temperature. The procedure following was then as described for Example 5a. Yield of lyophilized powder was 35.1 mg. Residual dried stem bark weighed 450 mg.

Herein ethanol extracts according to Examples 5a, 5b, 5c and 5d are collectively termed SEtOH.

**Example 6: Chloroform extract of root of *Neobeguea mahafalensis***

0.5 g of ground root from *Neobeguea mahafalensis* was placed in 10 ml of chloroform and gently shaken for 3 days at room temperature. The mixture was filtered through a lump of cotton wool placed in a conical funnel; afterwards the extracted root on the lump was rinsed with the same solvent (2 x 5 ml). The combined filtrates (colourless) were evaporated to dryness. A wax-like residue, herein termed extract RCH, formed. Yield 18 mg. The root after extraction was dried in the air keeping it on an open Petri dish. Residual root weighed 480 mg.

**Example 7: Chloroform extract of stem bark of *Neobeguea mahafalensis***

0.5 g of ground stem bark from *Neobeguea mahafalensis* was placed in 10 ml of chloroform and gently shaken for 3 days at room temperature. The mixture was filtered through a lump of cotton wool placed in a conical funnel; afterwards the extracted stem bark on the lump was rinsed with the same solvent (2 x 5 ml). The combined filtrates (slightly yellow-green) were evaporated to dryness. A wax-like residue, herein termed SCH, formed. Yield 40 mg. The stem bark after extraction was dried in the air keeping it on open Petri dish. Residual stem bark weighed 460 mg.

**Example 8: Freeze dried water extract of root of *Neobeguea mahafalensis***

A tablespoon of dried ground roots (5.0 g) from *Neobeguea mahafalensis* was put in 0.5 litre of boiling water. The mixture was boiled for 2 min, then left to cool down (slowly, overnight). It was then filtered (through a wool plug placed into a glass funnel under water jet pump vacuum) into two round-bottom flasks. The filtrates were then liquid shell frozen and freeze-dried, yielding 1.45 g of reddish-brown fluffy powder (herein termed 'RW' extract). The reddish-brown powder was used further in the manufacture of pharmaceuticals or used directly without further processing as a pharmaceutical.

**Example 9: Enriched extract from root of *Neobeguea mahafalensis***

Dried ground root (5.0 g, 1 table spoon) from *Neobeguea mahafalensis* was put in boiling water (500 ml). The

mixture was boiled for 2 min, and then allowed to cool down for 2-3 h. The concoction was filtered, and the filtrate lyophilized. A light-brown powder (RW extract) formed. Yield 1.50 g.

The powder was placed in a separation funnel; water (100 ml) was first added, solubilising the RW powder, and chloroform (100 ml) was then added. The mixture was intensely shaken for 2 min, then left to stand for 20 h (separation of phases proceeds very slowly, part of the mixture remains as an unseparated zone containing a precipitate between the water and chloroform layers). The chloroform and water layers were collected in separate portions, and the remaining intermediate zone was placed in plastic tubes resistant to chloroform and centrifuged at 4000 rpm for 30 min. Chloroform and water layers formed in the tubes after the centrifugation and were separately collected by use of a pipette. 20 ml each of chloroform and water were equally divided and added to all the tubes, the tubes were vortexed, centrifugation repeated, and the new chloroform and water layers were collected. This procedure of addition of solvents, vortexing, centrifugation and collection of layers was repeated twice.

The unresolved central layer was evaporated. A brown-red powder (R2P) remained. Yield 28.0 mg.

The united chloroform extracts were evaporated to dryness, the residue was dissolved in acetonitrile-water (volume proportion 4 : 6; 50 ml), and freeze-dried. A white powder (R2C) formed. Yield 32.3 mg.

The united water phases were freeze-dried. A brown, fluffy powder (R2W) formed. Yield 1.09 g.

The separation procedure is further clarified in Figure 35.

**Reference Example 10: Enriched extract from stem bark of *Neobeguea mahafalensis***

Dried and ground stem bark (6.0 g, 1 table spoon) was put into boiling water (500 ml). The mixture was boiled for 2 min, and then allowed to cool down for 2-3 h. The concoction was filtered, and the filtrate was lyophilized. A light-brown powder (herein termed 'SW') formed. Yield 1.16 g.

All of the above SW the powder was placed in a separation funnel and water (100 ml) and chloroform (100 ml) were added. The mixture formed was intensely shaken for 2 min, then left to stand for 20 h (separation of phases proceeded very slowly, part of the mixture remains as an unseparated zone containing a precipitate between the water and chloroform layers). The chloroform and water layers, as well as the intermediate zone were collected separately. The intermediate zone was placed in plastic tubes resistant to chloroform and centrifuged at 4000 rpm for 30 min. The chloroform and water layers, formed in the tubes after the centrifugation, were collected separately using a pipette. 20 ml each of chloroform and water were equally divided and added to the tubes, the tubes were vortexed, the centrifugation repeated, and the new chloroform and water layers were again collected. This addition of solvents, vortexing, centrifugation and collection of layers was repeated twice. The unresolved central layer was evaporated. A dirty-yellow powder (herein termed 'S2P') remained. Yield 35 mg. The united chloroform extracts were evaporated to dryness, the residue was dissolved in acetonitrile-water (volume proportion 4 : 6, 50 ml), and freeze-dried. A white powder (herein termed 'S2C') formed. Yield 69 mg. The united water phases were freeze-dried. A light brown, fluffy powder (herein termed 'S2W') formed. Yield 0.90 g.

**Reference Example 11: Capsules prepared from stem bark of *Neobeguea mahafalensis***

Dried coarsely ground stem bark of *Neobeguea mahafalensis* was carefully further ground in a mortar into a fine powder. The powder was then directly filled into vegetarian capsules obtained from Capsuline, Inc, P.O. Box 667260, Pompano Beach, FL 33066, USA.

a) Size 0 capsules were filled with the stem bark powder, the powder was gently packed and the capsules were closed with their caps. Each capsule contained 300 mg of stem bark.

b) Size 00 capsules were filled with the stem bark powder, the powder was gently packed and the capsules were closed with their caps. Each capsule contained 465 mg of stem bark.

Following their filling the capsules were stored in capped plastic vessels.

**Example 12: Capsules prepared from root of *Neobeguea mahafalensis***

Dried coarsely ground root of *Neobeguea mahafalensis* was carefully further ground in a mortar into a fine powder. The powder was then directly packed into vegetarian capsules obtained from Capsuline, Inc, P.O. Box 667260, Pompano Beach, FL 33066, USA.

a) Size 0 capsules were filled with the root powder, the powder was gently packed and the capsules were closed with their caps. Each capsule contained 220 mg of root.

b) Size 00 capsules were filled with the root powder, the powder was gently packed and the capsules were closed with their caps. Each capsule contained 350 mg of root.

Following their filling the capsules were stored in capped glass vessels.

**Reference Example 13: Capsules prepared from water extract SW of stem bark of *Neobeguea mahafalensis***

The freeze-dried water extract SW of stem bark of *Neobeguea mahafalensis* comprising a fluffy powder (prepared as described in Example 10) was carefully ground to a fine compacted powder in a mortar. The fine compacted powder was then directly packed into vegetarian capsules from Capsuline, Inc, P.O. Box 667260, Pompano Beach, FL 33066, USA, as follows;

a) Size 0 capsules were filled with the fine compacted powder, the fine compacted powder was gently packed and the capsules were closed with their caps. Each capsule contained 105 mg of SW extract.

b) Size 00 capsules were filled with the fine compacted powder, the fine compacted powder was gently packed and the capsules were closed with their caps. Each capsule contained 160 mg of SW extract.

Following their filling the capsules were stored in capped glass vessels.

**Example 14: Capsules prepared from water extract RW of root of *Neobeguea mahafalensis***

The freeze-dried water extract RW of root of *Neobeguea mahafalensis* comprising a fluffy powder (prepared as described in Example 8) was carefully ground to a fine compacted powder in a mortar. The powder was then directly packed into vegetarian capsules from Capsuline, Inc, P.O. Box 667260, Pompano Beach, FL 33066, USA, as follows:

a) Size 0 capsules were filled with the fine compacted powder, the fine compacted powder was gently packed and the capsules were closed with their caps. Each capsule contained 70 mg of RW extract.

b) Size 00 capsules were filled with the fine compacted powder, the fine compacted powder was gently packed and the capsules were closed with their caps. Each capsule contained 100 mg of RW extract.

Following the filling capsules were stored in capped glass vessels.

**Reference Example 15: Capsules prepared from enriched extract of stem bark of *Neobeguea mahafalensis***

1 g of the S2C extract of stem bark from *Neobeguea mahafalensis* was prepared according to Example 10 and thoroughly mixed with 30 g corn starch. Size 0 vegetarian capsules (Capsuline, Inc, P.O. Box 667260, Pompano Beach, FL 33066, USA) were filled with the powder mixture, the powder was gently packed and the capsules were closed with their caps. Each capsule contained 10 mg of S2C extract.

**Example 16: Capsules prepared from enriched extract of root of *Neobeguea mahafalensis***

1 g of the R2C extract from root from *Neobeguea mahafalensis* was prepared according to Example 9 and thoroughly mixed with 30 g corn starch. Size 0 vegetarian capsules (Capsuline, Inc, P.O. Box 667260, Pompano Beach, FL 33066, USA) were filled with the powder mixture, the powder was gently packed and the capsules were closed with their caps. Each capsule contained 10 mg of R2C extract.

**Example 17: Influence of extract from *Neobeguea mahafalensis* on sexual activity in male rats**

Methods

Behavioral tests were conducted during the dark phase of the day/night cycle (16.00 - 20.00 h) under dim light illumination. Sexually naïve Wistar rats (220-250g) of both sexes were housed under standard conditions. After a 7-day adaptation period they were used in the tests.

On the time for evaluation each male rat was placed individually into a clear plastic observation chamber (36 x 24 x 33 cm, length x width x height) for a 30 min adaptation period prior to the introduction of a receptive, sexually inexperienced female.

Before use the female rats were brought into full sexual receptivity by sequential treatment with estradiol benzoate (12 μg/rat; 48 h pre-session injection time) and progesterone (500 μg/rat; 4.5 h pre-session injection time). Estradiol and progesterone were prepared in olive oil and injected subcutaneously in a volume of 0.1 ml/rat.

Sexual behaviour test (SBT)

The following indices of male sexual behavior were registered:

(1) Mounting the female. The male normally mounts from the rear, sometimes posing his forelegs over the female's back, and makes rapid anteroposterior pelvic thrusts for about 300 ms. He then dismounts rather slowly. After a mount, the male frequently licks his genital region. Mounts are performed in bouts with short (5 - 10 s) inter-mount intervals. A bout is defined as a sequence of mounts, with or without vaginal penetration, uninterrupted by any behavior that is not oriented towards the female, except genital self-grooming. The number of mounts within a bout varies usually between 1 and 5 (mean number about 2). Between each bout there is a longer (20 - 80 seconds) pause, during which time the male may engages in other behaviors. Before, at the beginning of, or during the mount the female assumes a lordosis posture. If no lordosis is displayed, the female is immediately replaced. An inexperienced male, and sometimes also an experienced male, may mount the female with incorrect orientation, e.g. on the flanks, the head, etc. Such mounts are not scored.

(2) Intromission (vaginal penetration). This behavior starts with a mount, but suddenly the male makes a deep thrust forward and stops pelvic thrusting. He then vigorously withdraws and always licks his genitals. A male will never mount again immediately after an intromission.

(3) Ejaculation. This behavior starts with an intromission, but after vaginal penetration (the deep forward thrust) the male remains on the female for 1 - 3 s. Rhythmic contraction of the posterior abdomen are clearly visible. He then slowly rises with his forelegs held open. At ejaculation, it is the female that moves away from the male. He then licks his genitals and remains inactive for several (4-7) min.

Recorded elements of SBT

The elements recorded from the sexual behavior tests were used to calculate all standard measures of sexual behavior, providing a general characterization of the copulatory cycle. The following were thus recorded:

1. Latencies of the first mount and intromission
2. Total number of ejaculations
3. Series in a complete copulatory cycle.

Additionally, for each copulatory series (n, number of series), the following measures were calculated:

1. Copulatory efficacy - average number of finished copulatory cycles (i.e., cycles that end with ejaculation) in the group
2. Duration of the series (DSn, time interval between the first mount or intromission to the ejaculation; often labeled as ejaculatory
3. Number of mounts and intromissions preceding each successive ejaculation (NMn and Nin).
4. Post-ejaculatory interval (the time interval between the ejaculation and first successive mount/intromission).
5. Number of active couples (also termed number of active rats). As active couples are counted all couples that started sexual activity during each test period.

(Note that herein by 'Copulatory efficacy' and 'Copulatory efficacy index' is *mutatis mutandis* intended to mean the same thing.)

The screening tests are normally ended at the first ejaculation (if there no special reason for recording the post-ejaculatory interval). If a male does not intromit within 30 min, the test is ended for that male and he is considered sexually inactive. If a male has intromitted but not ejaculated within 30 min, he is given 30 min after the first intromission to ejaculate. Otherwise he is considered inactive.

Test protocol

Experimental groups: Control group and RW-group (8 male rats each). The RW extract of Example 8, was

dissolved in a small amount of water and administered *per os* at a dose of 60 mg/kg/day for 3 consecutive days. Controls received an equal volume of water *per os.* The experimental protocol is clarified in Table 1, Example 17:

**Table 1, Example 17**

| Day | Procedure | Comments |
|---|---|---|
| 0 | Sexual behavior test (SBT) | Assess normal (basal) level |
| 1 | **Extract of Example 8** (**RW**) dose 60 mg/kg, or Control treatment, SBT after ~1hr | Test if there is any 'viagra' type activity |
| 2 | **RW** 60 mg/kg or Control treatment | |
| 3 | **RW** 60 mg/kg or Control treatment | |
| 4 | SBT | Evaluation 24 hrs after $3^{rd}$ dose |
| 7 | SBT | Evaluation 4 days $3^{rd}$ dose |
| 14 | SBT | Test for long-term effect |

Results

One hour after administration of the first RW-dose there was no appreciable influence on the sexual activity among the male rats. After 3 days of administration of RW the sexual activity was tested again on day 4, 24 hrs after the last RW administration. The results show that the number of active couples in the RW-treated group (given in % of the respective proper test group and occasion) was statistically significantly ($p<0.05$) higher on the 4th day compared to the respective control (Fig. 36):

On the 7th and 14th day there were also statistically significant ($p<0.05$) increases in the number of active couples (Fig. 36). This is very note-worthy as the male rats had not received any further treatment after the 3rd dose of RW. As seen from the figure these effects were very marked indeed, as an increase in the number of active couples occurred from 25-38 % to 88 % (i.e. well more than a doubling), and without the effect showing any tendency to decline even at the 14th day.

The copulatory efficacy, or the average number of finished copulatory cycles in the group, was also measured on the 4th, 7th and 14th day (Fig. 37). As can be seen from Figure 37, the copulatory efficacy index increased for the RW-treated rats, although a statistically significant increase ($p<0.05$; versus the corresponding group on day 0) was seen only on the 14th day. (It has to be mentioned that on the 1st day no rats had full copulatory cycles in the control group.)

Furthermore the copulatory cycle length decreased on the 1st and 14th day as compared to the control group and corresponding 0th day group in the RW-treated group (Figure 38).

We also observed that the number of mounts and intromissions decreased on the 7th and 14th day in the RW-treated rats (Table 2, Example 17).

The full summary of the data from this test series is given in Table 2, Example 17. Conclusions

No effect of RW was observed after a single administration (test performed 1 hr after RW administration). However, following the 3:d day of administration of RW a significant increase in the number of sexually active couples was seen; the effect was statistically significant even at the 14th day of the experiment (i.e., 11 days after the last administration of RW). On the other hand, RW decreased the length of the copulatory cycle, as well as the number of mounts and intromissions in the cycle, but this effect was statistically significant only versus the level on day 0 of the corresponding group.

In conclusion, after treatment with RW rats are more sexually active, but the length of copulatory cycle is shorter (less intromissions and mounts). Thus, RW has a sexual enhancing effect.

## Table 2, Example 17

| № | Sexual behavior | Control | RW | Control | RW | Control | RW | Control | RW | Control | RW |
|---|---|---|---|---|---|---|---|---|---|---|---|
| | | 0th day | | lth day | | 4th day | | 7th day | | 14th day | |
| | | Mean | Mean | Mean | Mean | Mean | Mean | Mean | Mean | Mean | Mean |
| 1 | Copulatory Efficacy, index | 0.4 | 0.4 | 0.0 | 0.4 | 0.8 | 1.9 | 0.6 | 1.6 | 0.9 | 2.4 |
| | StErr | 0.4 | 0.4 | 0.0 | 0.4 | 0.5 | 0.8 | 0.4 | 0.5 | 0.6 | 0.7 |
| | ANOVA | | | | | | | | | | |
| 9 | Number of active rats, % | 25% | 38% | 25% | 63% | 38% | 88% | 38% | 88% | 38% | 88% |
| 2 | The Copulatory Cycles lenght, sec | 1980.0 | 1691.3 | | 1016.7 | 1756.3 | 1603.5 | 911.6 | 1423.8 | 1415.0 | 891.4 |
| | StErr | 395.1 | 64.9 | | 124.6 | 421.3 | 212.5 | 229.0 | 241.4 | 225.5 | 86.6 |
| | ANOVA | | | | | | | | | | |
| 3 | Latencies of the first Mount or Intermission, sec | 160.5 | 210.7 | 495.0 | 648.6 | 193.0 | 271.6 | 32.3 | 109.1 | 24.0 | 27.1 |
| | StErr | 64.5 | 70.0 | 365.0 | 454.0 | 156.0 | 117.0 | 23.9 | 37.8 | 10.5 | 10.3 |
| | ANOVA | | | | | | | | | | |
| 4 | The Number of Mounts in the Cycle | 3.0 | 4.3 | | 4.3 | 2.7 | 4.4 | 2.0 | 2.1 | 3.0 | 1.4 |
| | StErr | 0.6 | 0.3 | | 0.9 | 0.9 | 0.5 | 0.9 | 0.5 | 0.6 | 0.3 |
| | ANOVA | | | | | | | | | | |
| 5 | The Number of Intromissions in the Cycle | 43.7 | 32.7 | | 21.3 | 23.0 | 28.3 | 20.4 | 20.0 | 17.3 | 13.7 |
| | StErr | 7.2 | 3.7 | | 1.9 | 7.5 | 2.5 | 6.8 | 2.8 | 1.9 | 1.1 |
| | ANOVA | | | | | | | | | | |
| 6 | The Number of Mounts and Intromissions in the Cycle | 46.7 | 37.0 | | 25.7 | 27.2 | 33.2 | 24.7 | 21.7 | 20.3 | 15.2 |
| | StErr | 7.7 | 3.5 | | 2.7 | 4.5 | 3.3 | 13.7 | 2.6 | 2.4 | 1.2 |
| | ANOVA | | | | | | | | | | |
| 7 | Postejaculatory interval, sec | 452.0 | 472.0 | | 591.5 | 412.2 | 714.7 | 638.4 | 660.7 | 811.0 | 671.3 |
| | StErr | 88.0 | 55.0 | | 133.5 | 112.4 | 74.8 | 108.6 | 82.0 | 156.4 | 79.3 |
| | ANOVA | | | | | | | | | | |
| 8 | The first Copulatory Cycle lenght, sec | 1502.0 | 1792.0 | | 1261.0 | 2424.0 | 2016.8 | 1218.0 | 1625.4 | 1767.5 | 1261.8 |
| | StErr | | | | | 200.0 | 472.9 | 123.0 | 291.5 | 105.5 | 209.9 |
| | ANOVA | | | | | | | | | | |

Table 2, Example 17

P<0.05 vs corresponding day 0th day, t-test
(dotted) P<0.05 vs Control, t-test
(black) P < 0.05 vs Control, Chi-Square Test

### Example 18: Effect of the pharmaceutical preparation prepared from an extract of *Neobeguea mahafalensis* in a 40 year old male

The subject is married and has two children, 3 and 5 years old. He is healthy and he takes no medicines.

His sexual performances had declined gradually over the years, during the period before treatment. He had also since long time been afraid to have sex with a woman due to fear of losing erection. He volunteered to take totally nine capsules 00 prepared according to Example 14 each capsule comprising 100 mg of the RW extract according to Example 8. (Each capsule corresponding to about 300 mg of dried root). The subject was told that the capsules contained extract from Madagascan plant but he was not revealed from which species. He took 3 capsules each day for 3 consecutive days.

Already at the first day of treatment, after having taken the first capsule, the subject noticed a positive effect on his sexual ability. During the 2nd and 3rd day this effect increased even more. He describes the effect as an increase in sexual excitement when he wants to have sex.

He also describes the effect as an improved sexual performance and that he is no longer afraid to have sex. He obtains a stronger erection that is maintained during the whole period of intercourse. He also experiences an increased feeling of pleasure while making love. He also has increased the duration of the intercourse to approximately 30 minutes; while earlier it was about 15 min.

Remarkably, the effect of the treatment remains still after 3 ½ years.

The subject states that he has not experienced any other effect of the drug. His memory is not affected; he experiences no change of sleeping pattern and no change in motor behaviour. He has no problems of urination.

**Example 19: Effect of the pharmaceutical preparation prepared from an extract of *Neobeguea mahafalensis* in a 37 year old male**

The subject is married and has three children, 4, 6 and 8 years old. He states that he is healthy and that he takes no medicines.

The subject did not earlier have any erection problems. He volunteered to take totally 12 capsules 00 prepared according to Example 14 each capsule comprising 100 mg of the RW extract according to Example 8. (Each capsule corresponding to about 300 mg of dried root). The subject was told that the capsules contained extract from Madagascan plant but he was not revealed from which species. He took 3 capsules each day for 4 consecutive days.

The subject states that after the treatment he experiences a stronger sexual appetite when he is together with a woman. During intercourse he experiences now a longer time before ejaculation. After ejaculation he experiences now also a shorter period of sleepiness than before taking the drug. Moreover, at the day following having sex he also feels stronger than earlier, being capable to renewed sexual activity to an extent that he was not capable of prior to obtaining the treatment.

However, if he tries to force the ejaculation during intercourse he experiences exhaustion, as he finds it more difficult to ejaculate fast than earlier. However, if he allows it to take the time it needs he does not experience this type of exhaustion.

Since he obtained the capsules for almost 1 ½ year ago the effect still remains, although there has been some progressive reduction in the extent of the effect. In particular the prolongation of the time for ejaculation that he experienced in the beginning has be come less pronounced.

The subject states that he does not have any problems to urinate, and that he never experienced any other noticeable effects from the treatment. He does not have any problems with memory, sleeping or performing motor tasks.

**Example 20: Effect of the pharmaceutical preparation of the extract of *Neobeguea mahafalensis* in a 55 year old male volunteer**

The subject is married and has 15 children, the youngest is only a few months while the oldest is 33 years. He states that he is over all healthy and that he takes no medicines. However, he had experienced a decreased sexual ability being able to make love only 3 times per months, or even being totally unable. Due to his incapacity he lost his relation with his woman. Earlier he had had many wives (in accordance with local tradition) and got many children. However, the 14 first children were born when he was young, i.e. while he was aged 21 to 35 years and due to his increasing incapacity did not receive any more children for long time.

He volunteered to take totally nine capsules 00 prepared according to Example 14 each capsule comprising 100 mg of the RW extract according to Example 8. (Each capsule corresponding to about 300 mg of dried root). The subject was told that the capsules contained extract from Madagascan plant but he was not revealed from which species. He took 3 capsules each day for 3 consecutive days.

After the treatment he started to notice a sexual excitement during closeness with a woman that he was not able to feel before the treatment. After the treatment he became able to make love once a day. He obtains more rapid erections now compared to what he was able to obtain earlier before the treatment (this effect is still seen more than one year after the treatment). He now also obtains spontaneous erections, something that he did not have before the treatment. The volume of his ejaculations is larger than they were before the treatment. He sexual performance is now even better than it was when he was young. The duration of intercourse is now usually 5-10 minutes and this duration has not changed since before the treatment. The effect is still, since slightly more than one year following the treatment, clearly noticeable and shows only slight decline.

Shortly after the treatment, due to his regain of sexual ability, he married a young wife who became pregnant and recently gave him a child.

He states that he has not experienced any other effects of the drug. He sleeps normally, has no problems with the memory and no change in motor behaviour. He does not experience any problems with urination.

**Example 21: Effect of the pharmaceutical preparation of the extract of *Neobeguea mahafalensis* in a 57 year old male volunteer**

The subject is an academic holding an administrative position. He is married and has 4 children, the youngest is 20 and the oldest is 30 years old.

The subject states that he is overall healthy although he suffers from a slightly elevated blood pressure that is under control by medication.

The subject had noticed a progressive decline in his sexual performance from the age he was 45 years old. His erections had since then become progressively weaker and he required more sexual stimulation to obtain erection. Still he had been able to have sex with a woman but to a much lesser extent than when he was young.

He volunteered to take totally 14 capsules 0 prepared according to Example 14 each capsule comprising 70 mg of the RW extract according to Example 8. (Each capsule corresponding to about 200 mg of dried root). The subject was told that the capsules contained extract from Madagascan plant but he was not revealed from which species. He took 2 capsules each day for 7 consecutive days.

Already at the first day of treatment he noticed an effect as a sensation of the desire to make love. At the second day that desire was further increased and in contact with a woman he obtained a strong erection. Immediately after the completion of the treatment he gained a dramatic increase in sexual performance being able to have sex with a woman 3 times a day. He also now experienced an increased time for ejaculation during intercourse. Earlier it took him a few minutes to ejaculate; now this time had increased to 5-10 minutes. The subject also describes the effect from the drug as the 'batteries has become charged again'. He experiences an increase sexual desire; the drugs effect reminds him of the performance in his youth.

Following the treatment the subject did not receive any further treatment during a period of 3 years. Even after this long time, however, the effect remains although it has gradually declined since this time. After three years following the treatment he has sex about 3 times per week. However, his performance is still better compared to the situation before the treatment.

The drug has not given him any noticeable side effects. His memory is good, he sleeps well and his motor functions are normal. His work capacity is normal and was not affected by the drug. He does not experience any problems with urination.

**Example 22: Evaluation of behavioral effects of R2W, R2P and R2C extracts from *Neobeguea mahafalensis***

The activity of the extracts R2W, R2P and R2C of Example 9 were evaluated using essentially the same method as detailed in Example 17. The sexual behaviour test (SBT) and recorded elements of SBT were the same as in Example 17.

Test protocol

The experimental groups comprised: Control, R2W, R2C and R2P groups. Each group contained 6 male rats. Each extract was administered *per os* to the rats, suspended or dissolved in a small volume of water (R2W dissolved in water, whereas R2C and R2P formed suspensions). Control rats were given the same volume of water.

The extracts were administered as follow: R2W - 43 mg/kg body weight; R2C - 4 mg/kg body weight; and R2P - 4 mg/kg body weight. These doses were administered once a day for 3 consecutive days. The protocol is further detailed in Table 1, Example 22:

| Day | Procedure |
|---|---|
| 1 | Drug *per os* administration at the doses: R2W (6 rats) - 43 mg/kg; R2C (6 rats) - 4 mg/kg; (6 rats) - 4 mg/kg; Controls received water *per os* only |
| 2 | Drug administration - same as for day 1 |
| 3 | Drug administration - same as for day 1 |
| 7 | Sexual behavior test (SBT) |

Results

Four days following the last administration (i.e., on day 7) the sexual activity was tested. Extracts R2W (43 mg/kg, p.o.) and R2P (4 mg/kg, p.o.) did not appreciably increase any of the recorded indices of sexual activity. For example, for the R2W group the copulatory efficacy (i.e., the average number of finished copulatory cycles in the group) was similar to that of the control group; for the R2P group it was even lover than in control group (Fig. 39). By contrast R2C caused a clear increase in the copulatory efficacy (Fig. 39):

The number of mounts was drastically and statistically significantly increased in R2C group as is illustrated in Fig. 40.

The full data from this series test is given in Table 2, Example 22. (Note that while rats in the R2P group started activity and therefore are included in the 'Number of active rats' none of them completed any cycles; i.e., none of the rats ejaculated. Therefore the copulatory cycle length, number of mounts in the cycle, number of intromission in the cycle, number of mounts and intromissions in the cycle, post-ejaculatory interval and first copulatory cycle length are left blank for the R2P group.)

Conclusion

R2W and R2P do not influence male rat sexual behavior at the doses used. Extract R2C induces an increase of sexual activity. Thus, R2C has a sexual enhancing effect.

**Table 2, Example 22**

| № | Sexual behavior | Control | R2W | R2C | R2P |
|---|---|---|---|---|---|
| | | | 43mg/kg | 4mg/kg | 4mg/kg |
| | | **Mean** | **Mean** | **Mean** | **Mean** |
| 1 | **Copulatory Efficacy, index** | **0.5** | **0.8** | **2.0** | **0.0** |
| | **StErr** | 0.5 | 0.8 | 0.7 | 0.0 |
| | ANOVA | | | | **0.2967** |
| 2 | **Number of active rats, %** | **33%** | **33%** | **67%** | **50%** |
| 3 | **The Copulatory Cycles lenght, sec** | **1540.0** | **1560.0** | **1522.9** | |
| | **StErr** | 429.6 | 229.4 | 256.8 | |
| | ANOVA | | | | **0.9961** |
| 4 | **Latencies of the first Mount or Intermission, sec** | **104.0** | **669.0** | **226.3** | **509.0** |
| | **StErr** | 47.0 | 513.0 | 49.4 | 415.5 |
| | ANOVA | | | | **0.2809** |
| 5 | **The Number of Mounts in the Cycle** | **0.3** | **0.4** | 2.3 | |
| | **StErr** | 0.3 | 0.2 | 0.4 | |
| | ANOVA | | | | **0.0062** |
| 6 | **The Number of Intromissions in the Cycle** | **16.0** | **17.4** | **17.1** | |
| | **StErr** | 3.1 | 3.1 | 2.3 | |
| | ANOVA | | | | **0.9648** |
| 7 | **The Number of Mounts and Intromissions in the Cycle** | **16.3** | **17.8** | **19.4** | |
| | **StErr** | 3.3 | 3.2 | 2.5 | |
| | ANOVA | | | | **0.8173** |
| 8 | **Postejaculatory interval, sec** | **778.0** | **592.8** | **619.2** | |
| | **StErr** | 83.2 | 24.4 | 34.3 | |
| | ANOVA | | | | **0.0809** |
| 9 | **The first Copulatory Cycle lenght, sec** | **1759.0** | **1806.0** | **2502.0** | |
| | **StErr** | | | 279.8 | |
| | ANOVA | | | | |

2.3 P < 0.05 vs Control, Chi-Square Test

**Example 23: Assay of activity of water extract RW from root of *Neobeguea mahafalensis***

The activity of the extract RW, prepared as described in Example 8, was assayed for activity in essence using the same method as detailed in Example 17. The sexual behaviour test (SBT) and recorded elements of SBT were the same as in Example 17.

Test protocol

The test protocol comprised 5 groups of rats, wherein each group contained 8 rats. The test drug (RW) comprised

the extract of Example 8. Prior to administration it was dissolved in a small volume of water. On day 1 group 1 was given 3 mg/kg body weight (bw) of RW *per os*; group 2, 10 mg/kg bw; group 3, 30 mg/kg bw; group 4, 100 mg/kg bw. Group 5 was given water *per os* only. This administration was repeated on day 2 and 3. Sexual behaviour test was performed on day 7.

**Table 1, Example 23:**

| Day | Procedure |
|---|---|
| 1 | **Drug:** Extract RW of Example 8<br>a) 1 mg/kg body weight (8 male rats)<br>b) 3 mg/kg body weight (8 male rats)<br>c) 10 mg/kg body weight (8 male rats);<br>d) 30 mg/kg (8 male rats)<br>e) 100 mg/kg body weight (8 male rats)<br>**Control:** Water (8 male rats) |
| 2 | Drug administration - same as for day 1 |
| 3 | Drug administration - same as for day 1 |
| 7 | Sexual behavior test (SBT) |

Results

Of the recorded element of SBT the number of active couples (in %) and the copulatory efficacy index for each group was as follows:

**Table 2, Example 23:**

| Group | Dose (mg/kg bw) | Number of active couples (%) | Copulatory efficacy index |
|---|---|---|---|
| 1 | 1 | 25 | 0.0 |
| 2 | 3 | 38 | 0.8 |
| 3 | 10 | 50 | 0.8 |
| 4 | 30 | 75 | 1.9 |
| 5 | 100 | 88 | 2.0 |
| 6 | 0 | 38 | 0.4 |

The data for the number of active couples was then plotted in a semi logarithmic diagram as shown in Fig. 41. (The control level was assumed to occur also at a low level of administration that was selected so low to be reasonably assumed to be essentially inactive; in this case it was set to 0.01 mg/mg body weight).

Non-linear curve fitting of the data to the logistic equation:

$$Y = B + \frac{A-B}{1+\left(\frac{X}{K}\right)^{P}}$$

where Y is the number of active couples, A is the starting level of the dose response curve (i.e., Y level without drug), B the ending level of the dose response curve (i.e., the maximally possible attainably Y level with high doses of the drug), K the $ED_{50}$, and P the Hill coefficient (i.e., slope of the curve) was afforded by using GraphPad Prism software (GraphPad Software, Inc.11452 El Camino Real, #215San Diego, CA 92130 USA).

The results were as follows:

A = 32 %
B = 91 %
K ($ED_{50}$) = 16.3 mg/kg body weight

P = 1.67

Accordingly using the Equation:

$$Activity = \frac{1}{ED_{50}}$$

where $ED_{50}$ is expressed in mg/kg body weight, the activity of the RW extract of Example 8 can be calculated to 1/16.3 = 0.061 U/mg. (These units correspond to $U_{sac}$; i.e. the activity of the extract was estimated to be $0.061_{sac}$/mg). (It shall be observed that the activity obtained and the data obtained that underlies this activity is given only as an example with the purpose to illustrate the method for assaying activity. The exact activity obtained of a sample may be subjected to variation due to experimental error, variation due to sample size and variation due to variations in extraction procedures and variation due to the different specimens of *Neobeguea mahafalensis* and differences in collections and storage of the specimen, drug and extract thereof).

The data for the copulatory efficacy index was also plotted in a semi logarithmic diagram as shown in Fig. 42. (The control level was assumed to occur also at a low level of administration that was selected so low to be reasonably assumed to be essentially inactive; in this case it was set to 0.01 mg/mg body weight).

The data was fitted to the logistic equation using the same approach as above. The results were as follows:

A = 0.36
B = 2.08
K ($ED_{50}$) = 14.1 mg/kg body weight
P = 2.19

Applying the same approach as above the activity of the extract could then be computed as 1/14.1 = 0.071 U/mg (i.e. 0.071 $U_{cei}$/mg). (It shall be observed that the activity obtained and the data obtained that underlies this activity is given only as an example with the purpose to illustrate the method for assaying activity. The exact activity obtained of a sample maybe subjected to variation due to experimental error, variation due to sample size and variation due to variations in extraction procedures and variation due to the different specimens of *Neobeguea mahafalensis* and differences in collections and storage of the specimen, drug and extract thereof).

**Example 24: Identification of characteristic mass-peaks in extracts of *Neobeguea mahafalensis***

Introduction

Extracts prepared from *Neobeguea mahafalensis* were analysed by mass spectrometry in order to demonstrate the presence of characteristic mass-peaks. Two instruments were applied:

One instrument was a PE SCIEX API 150EX single quadrupole mass spectrometer (Perkin Elmer, 45 William Street, Wellesley, MA 02481-4078, USA). This is a general purpose instrument with a mass range up to 3000 amu (amu= atomic mass units), which can be used as a mass detector, when connected to HPLC. A choice of inlets is available, including atmospheric pressure chemical ionisation (APCI) ion source (Heated Nebulizer). This ion source provides a better ionization than the more common electrospray source for less polar analytes. API 150EX allows fast, reliable, sensitive and fully automated data acquisition and analysis.

The other instrument was a Q-Tof2 from Micromass (Macromass/Waters; Waters Corporation, 34 Maple Street, Milford Massachusetts 01757, USA). This is a high resolution hybrid quadrupole time-of-flight mass spectrometer, equipped with Z-spray electrospray ionization inlet (i.e., the Micromass nanoflow interface together with glass capillary option was used). This instrument utilizes a high performance, research grade quadrupole mass analyzer (MS1) and an orthogonal acceleration time-of-flight mass measuring part (MS2). A hexapole collision cell, placed between two mass analysers, is useful to induce fragmentation, which is necessary for structural investigations; (however, the hexapole collision cell option was not active in the experiments of this example). Ions coming from mass analysers are detected by a microchannel plate detector and ion counting system.

Methods

Using the PE SCIEX API 150EX single quadrupole mass spectrometer the R2C sample of root prepared

according to Example 9, and the S2C sample from stem bark prepared according to Example 10 were analysed as follows:

The R2C or S2C sample (1.0 mg) was dissolved in acetonitrile (0.3 ml), diluted with water to 1.0 ml volume and centrifuged. The clear supernatant (50 μl) was introduced into the LC/MS system, containing a 4 x 250 mm Silasorb C18 HPLC column. Analysis time was 80 minutes using a linear gradient made from solution A (5% acetonitrile in water with 5 mM ammonium acetate additive) and solution B (94% acetonitrile in water with 5 mM ammonium acetate additive). Upon starting the analysis the eluent contained 80% solution A and 20% solution B; at the end of the 80 minutes the eluent was composed of 20% solution A and 80% solution B. The eluate from the column was introduced into an APCI ion source integrated with the PE SCIEX API 150EX mass spectrometer at a flow rate of 1.0 ml/min.

Using the Q-Tof2 mass spectrometer the R2C and S2C samples were prepared similarly as above (i.e., 0.2 mg of each was dissolved in 0.2 ml acetonitrile then diluted with 1% formic acid to 1.0 ml volume and centrifuged) and were then introduced via an electrospray needle and infused into the Micromass Q-Tof2 mass spectrometer. Change of polarity was applied to induce positive or negative ionization. Analysis of raw data was attained using MassLynx V4.0 software.

Results

Examples of mass spectra of root extract R2C using the PE SCIEX API 150EX spectrometer with atmospheric pressure chemical ionisation are shown in Figure 4. Shown are the summary ion intensities from the LC/MS run between 0 - 80 min, and separate mass scans at selected times during the LC/MS run with, respectively, positive and negative ionization.

Upon analyzing all separate scans between 0 - 80 min the following characteristic root mass-peaks were identified:

Positive ionization, root extract R2C (accuracy approximately ± 0.1 amu):

305.7, 323.7, 324.2, 342.2, 378.3, 378.7, 383.2, 384.2, 391.2, 391.7, 408.2, 413.2, 413.7, 417.2, 425.7, 432.7, 441.7, 442.2, 465.3 , 477.3, 481.3 , 491.3, 507.3, 523.3, 537.3, 539.3, 540.3, 551.3 , 565.3, 567.3, 579.3, 620.3, 639.3, 652.4, 655.4, 658.4, 662.4, 667.4, 671.4, 683.4 , 694.4, 699.4 , 700.4, 703.4, 706.4, 710.4,713.4, 715.4, 716.4, 717.4, 727.4, 730.4, 731.4, 732.4, 732.9, 734.4, 741.4, 743.4, 744.4, 745.4, 759.4, 761.4, 762.4, 775.4, 785.4, 788.4, 789.4, 803.4 , 819.4

Negative ionization, root extract R2C (accuracy approximately ± 0.1 amu):

311.2, 335.2, 346.7, 347.2, 352.7, 353.2, 359.2, 364.7, 373.2, 388.7, 389.2, 401.2, 406.7, 407.2, 412.7, 418.7, 419.2, 425.2, 428.7, 450.7, 460.8, 463.3, 465.3, 485.3, 497.3, 498.8, 510.8, 513.3, 522.8, 525.3, 541.3, 551.3, 553.4, 559.3 , 569.3, 573.3, 574.8, 575.3, 585.3, 587.3, 588.8, 599.3, 601.3 , 603.3, 611.3, 617.3 , 629.3, 633.3, 643.3,645.3, 655.4,657.4,659.4,661.4,669.4,671.4,675.4, 681.4,685.4, 687.4,691.4,695.4, 697.4, 701.4, 703.4, 706.9, 712.9, 713.4, 715.4, 719.4, 721.4, 725.4, 727.4, 729.4, 734.9, 738.9, 739.4 , 741.4, 745.4, 748.9, 755.4, 759.4 , 761.4, 772.9, 773.4 , 787.4

Examples of mass spectra of root extract S2C using the PE SCIEX API 150EX with atmospheric pressure chemical ionisation are shown in Figure 5. Shown are the summary ion intensities from the LC/MS run between 0 - 80 min, and separate mass scans at selected times of the LC/MS run with, respectively, positive and negative ionization.

Analyzing all separate scans between 0 - 80 min the following characteristic stem bark mass-peaks were identified:

Positive ionization, stem bark extract S2C (accuracy approximately ±0.1 amu): 359.7,378.3, 413.2,465.3,477.3,507.3,509.3, 523.3,539.3,555.3, 569.3, 577.3, 595.3, 599.3, 639.3 , 646.3, 655.4, 657.4, 659.4, 662.4, 672.4, 675.4, 687.4, 688.4, 689.4, 694.4, 699.4, 703.4 , 704.4, 706.4, 715.4 , 716.4, 717.4, 722.4, 731.4, 732.4 , 733.4, 734.4, 738.4, 745.4, 746.4, 747.4, 748.4, 749.4, 759.4, 761.4, 764.4, 765.4, 773.4, 775.4, 776.4, 777.4, 790.4, 792.4, 803.4, 819.4, 833.4, 850.5

Negative ionization, stem bark extract S2C (accuracy approximately ± 0.1 amu):

337.2, 373.2, 411.2, 451.2, 457.3, 467.3, 497.3, 499.3, 543.3, 551.3, 553.4, 573.3, 585.3, 601.3 , 611.3, 629.3, 637.3, 641.3, 655.4, 657.4, 661.4, 669.4, 671.4 , 675.4, 685.4, 687.4, 697.4, 703.4, 713.4 , 719.4, 721.4, 727.4,

745.4 , 748.9, 849.5

Examples of mass spectra of root extract R2C using the Q-Tof2 mass spectrometer are shown in drawing section Figure 6.
Examples of mass spectra of stem bark extract S2C using the Q-Tof2 mass spectrometer are shown in drawing section Figure 7.
In the above lists, mass-peaks were detected with electrospray ionization on Q-Tof2 as well and are shown in bold face, underlined. The lower number of mass-peaks detected is attributed to the lower efficiency of ionization with the electrospray ionization compared to atmospheric pressure chemical ionisation.

**Example 25: Example of a suitable tablet formulation**

| Constituent | Per tablet |
|---|---|
| R2C extract, prepared according Example 9 | 10 mg |
| Potato starch | 90 mg |
| Colloidal silica | 10 mg |
| Talc | 20 mg |
| Magnesium stearate | 2 mg |
| 5 % aqueous solution of gelatin | 25 mg |
| **Final weight of tablet** | **135 mg** |

**Example 26: Example of a formulation of an injectable (suspension for subcutaneous injection)**

| Constituent | Per 1 ml |
|---|---|
| R2C extract, prepared according Example 9 | 10 mg |
| Sodium chloride | 4 mg |
| Aqua ad inject q.s. | |

**Example 27: Extract of stem bark of *Neobeguea mahafalensis* by use of Soxhlet procedure**

16.04 g of stem bark prepared from *Neobeguea mahafalensis* by grinding was placed in a Soxhlet apparatus and extracted with boiling methylene chloride (200 ml) for 15 h, as schematically shown in drawing section, Figure 2. The extract was evaporated to dryness, and the residue triturated with a spatula. Green powder (the desired extract) formed. Yield 1.0 g.
(The remaining materials of stem bark after extraction weighed 14.3 g, after thorough drying.)

**Example 28: Extract of root of *Neobeguea mahafalensis* by use of Soxhlet procedure**

8.0 g of root materials derived from *Neobeguea mahafalensis* by grinding was placed in a Soxhlet apparatus and extracted with boiling methylene chloride (200 ml) for 15 h essentially as schematically shown in Figure 2. The extract was evaporated to dryness in a rotavapor whereupon 204 mg of slightly yellow oily material (the desired extract) was obtained.
(The remaining materials of root after extraction weighed 7.56 g after thorough drying.)

**Example 29: Enriched extract of root of *Neobeguea mahafalensis* prepared by combined water and ethylacetate extraction**

Twenty gram of dried root materials derived from *Neobeguea mahafalensis* by grinding was poured into boiling

water (1 litre), the mixture boiled for 2 min, then allowed to cool down and left for 3 hours at room temperature. The mixture was filtered and divided into two portions, about 500 ml each. One of the 500 ml portion was evaporated to dryness using a Rotavapor, resulting in 'Crude aqueous extract' (RW). The other 500 ml portion was extracted ('exhausted') with ethyl acetate (500 ml). After separation of phases the ethyl acetate fraction was evaporated to dryness using a Rotavapor, resulting in 'EtOAc fraction' (RWEtOAc). After separation of phases the water phase was also evaporated to dryness, resulting in 'Exhausted aqueous fraction' (RWExh), comprising the desired enriched root extract of *Neobeguea mahafalensis.*

**Example 30: Assay of root extracts of *Neobeguea mahafalensis* in mice sexual behaviour test**

Animals

Mice of 9 weeks age weighing 30 ± 2 g were used throughout the study. They were all sexually unskilled. At 4 weeks of age they had been separated from their parents and males and females had been placed in separated cages. The mice were fed with pellets enriched with proteins and lipids.

Sexual behaviour test

Prior to the tests, male and female mice received specific treatments, as follows: Each male mouse received one of the extracts of Example 29, 'Crude aqueous extract', 'EtOAc fraction' and 'Exhausted aqueous fraction' (i.e., respectively, RW, EWEtOAc and RWExh extracts) by oral administration using a syringe and a cannula for esophageal insertion. Each extract was given at a dose of 60 mg/kg dissolved in a small amount of water during three consecutive days (D-1, D-2 and D-3) at the same time of the day (3.00 pm). Male control mice received the identical treatment schedule with water only.

Female mice were first treated with 50 μg/kg β-estradiol 48 hours before the test, and then with 2 mg/kg progesterone 4 ½ hours before the test, each by subcutaneous injection (compounds dissolved in olive oil) at a volume of 1 ml/kg, in order to make them sexually receptive.

The behavioural test was performed in a dark room at ambient temperature, during the last enlightened period of the day (3.00 pm) and the first dark period of the night (7.00 pm). Each observation period lasted 4 hours.

At the fourth, seventh and fourteenth day (D-4, D-7 and D-14), the male control mice and the treated male mice were individually placed in Plexiglass cages, 30 min before the start of the test.

At 3.00 pm, a female mouse was introduced into each cage hosting a male mouse. The number of mounts of the male mouse, defined as a tentative of lateral or dorsal mounting on the female mouse with or without penis intromission, was then counted throughout the 4 hour observation period and used as a simple measure of sexual behaviour activity.

In case a female was sexually non-receptive it was immediately replaced with a new female mouse.

Results

The results are summarized in Fig. 43.

The number of mounts during the four hour test period amounted to 45 on an average in non-treated male mice and is represented in the figure as the four bars of equal height at day 0.

At day four (D-4) and seven (D-7) the number of mounts increased for both the Crude aqueous extract (RW) and the Exhausted aqueous fraction (RWExh) treated mice, compared to the control. For the EtOAc fraction (RWEtOAc) treated mice the number of mounts was substantially lower compared with the control at D-4, whereas it was not substantially different from the control at D-7.

Also at day 14 (D-14) the RW and RWExh treated mice showed a higher number of mounts than the control and RWEtOAc treated mice.

The sexual enhancing effect peaked for RWExh at D-7 when it amounted to 138 mounts during the 4 hour test period - an increase by a factor of three compared to the control. Accordingly RWExh is a highly preferred extract of the invention.

**Example 31: Preparation of enriched extract RW1 from root from *Neobeguea mahafalensis* by molecular size fractionation using G-25 chromatography**

One gram of dried ground root of *Neobeguea mahafalensis* was poured into boiling water (50 ml) and the mixture was allowed to boil for 2 min, then allowed to cool down to room temperature and then left overnight at +5°C. The mixture was filtered (weight of residual roots after extraction, filtration and drying was 0.66 g), and the filtrate obtained was extracted with ethyl acetate (3 x 50 ml). (To improve separation of layers, the water-ethyl acetate emulsions were

transferred to glass tubes with conical bottom and centrifuged.) The obtained clear organic extracts were then evaporated giving 14.9, 5.6 and 4.2 mg of dark brown oily residues for the $1^{st}$, $2^{nd}$ and $3^{rd}$ extractions, respectively. The remaining water layer was evaporated to 20 ml volume (using a Rotavapor) and introduced onto a transparent plastic column (3.3 x 88.5 cm) filled with Sephadex G-25 in aqueous suspension. The column was eluted with distilled water under hydrostatic pressure, flow rate 3.4 ml/min. The high molecular fraction, i.e. the dark brown zone, emerging after about 280 ml of colourless eluate had passed, corresponding to the void volume of the G-25 column, was collected and dried by freeze drying until completely dried. A brown fluffy powder, belonging to the type RW1, was obtained.

**Example 32: Assay of enriched extract RW1 from *Neobeguea mahafalensis* in mice sexual behaviour test**

The activity if RW1 prepared according to Example 31 was assayed for sexual enhancing activity essentially using the method describe in Example 30, the only difference being that the mice were treated per orally with 60 mg/kg RW1 dissolved in a small amount of water for three consecutive days. Controls were subject to the same treatment schedule but given water only per os.

The number of mounts increased substantially when assayed on the fourth (D-4) and seventh day (D-7) of the experiment, as shown schematically in Fig. 44.

The sexual enhancing effect of RW1 is seen as an increase in the number of mounts counted during the 4 hour observation period.

**Example 33: Enriched extract from root of *Neobeguea mahafalensis* prepared by solvent extractions and reversed phase chromatography**

<u>Boiling water extraction</u>

Twenty five grams (25.0 g) of dried ground root from *Neobeguea mahafalensis* were put in boiling water (2500 ml). The mixture was boiled for 2 min, and then allowed to slowly cool and the mixture left for 16 h. The concoction was then filtered. The filtrate was freeze-dried. A light-brown fluffy powder (RW) formed, but to save the time the process was interrupted when about 10% of ice from start was still present. [As a control the residual solid residue (RR) was also collected and dried in the open air until constant weight (15.8 g); however RR was not used further].

<u>Chloroform extractions</u>

The partly freeze dried filtrate RW was thawed and water was added to 500 ml final volume. Chloroform (500 ml) was then added and the mixture was put at stand to allow the residual ice to melt. The mixture was then placed into a separation funnel, intensely shaken for 2 min, then left to stand for 20 h (separation of phases proceeded very slowly, part of the mixture remained as an unseparated zone between water and chloroform layers, containing precipitate). The chloroform and water layers were separately collected. The intermediate zone was placed in plastic tubes resistant to chloroform and centrifuged at 3000 rpm for 15 min. Chloroform and water layers, as well as an unresolved layer in between the chloroform and water layers formed in the tubes. After completion of the centrifugation the chloroform and water layers were separately collected by pipette and united with the previous water and chloroform phases, whereas the unresolved middle layer was retained in the tube. Twenty ml each of water and chloroform were then added to each tube, the tubes were again vortexed, the centrifugation repeated, and the new chloroform and water layers were again collected, again retaining the middle layer. Addition of solvents, vortexing, centrifugation, collection of layers and uniting the chloroform and water layers with the previous chloroform and water layers was repeated four times. The unresolved middle layers were also united.

The combined water layers were placed in a separation funnel and fresh chloroform (500 ml) was added and the mixture intensely shaken for 2 minutes. After separation three layers formed as before, and were collected.

The middle layer was combined with the previous united middle layers. After evaporation the united middle layers weigh 195 mg comprising a brown-red powder (R2P). R2P was not used further.

The chloroform phase was united with all the previous chloroform phases and evaporated to dryness, the residue treated with acetonitrile (40 ml), trying to dissolve it. Some part of the residue (a wax-like material) was not soluble, and was removed upon decantation. To the decantate (i.e., the acetonitrile solution) water (60 ml) was added. An oily precipitate formed. The mixture was filtered, and the filtrate was freeze-dried. A white powder, R2C (yield 190 mg) formed.

The wax-like material (R2X1) (constituting only a small fraction) and the oily precipitate (R2X2; obtained from the chloroform phase after having filtrated of R2C and evaporating off the chloroform) were first dried under vacuum in presence of $P_2O_5$, then dissolved in chloroform (10 ml for each sample), the solutions combined and evaporated. Residual colorless oil (R2X) weighed 30 mg. (R2X was not used further in this example).

United water phases obtained after extraction with chloroform were freeze-dried. A brown, fluffy powder (R2W) formed. Yield 6.39 g. (R2W was not used further in this example).

Further fractionation of R2C using reversed phase liquid chromatography

R2C (190 mg) was dissolved in acetonitrile (15 ml), and then water (35 ml) was added. A slightly turbulent solution formed. The turbulent solution was applied to a glass column (33 x 420 mm) filled with LiChroprep RP-18 (Merck Chemical Co., Germany) equilibrated with cool (+5°C) 30% acetonitrile in water + 0.1% trifluoroacetic acid. The column (which was maintained in a cold room with all eluents at +5°C) was eluted first with 30% acetonitrile in water +0.1% trifluoroacetic acid (1 liter), then with similar solutions where the acetonitrile content was sequentially changed to respectively 40%, 50%, 60 % (volume of each eluent portion 1 liter) and finally to 70 % (1.5 liter). Flow rate was about 1.5 ml/min. Eluate fractions of 12 ml volumes each were collected in glass tubes (each tube being given an ordinal number in the order they were collected starting from tube 1 and ending with tube 440). 30% acetonitrile content in eluent corresponded to the eluted 1-84 fractions (i.e. tube 1-84), 40% - to 85-179 (i.e. tube 85-179), 50% - to 180-267 (i.e. tube 180-267), 60% - to 268-345 (i.e. tube 268-345), and 70% - to 346-440 fractions (i.e. tube 346-440).

Every 10th fraction (i.e. content of every 10th tube) was then analyzed by HPLC (LiChroprep RP-18 2.1 x 100 mm column, eluent - linear gradient from 20% acetonitrile in 5 mM ammonium acetate to 80% acetonitrile in 5 mM ammonium acetate in 60 min, flow rate 0.2 ml/ min, detection at 220 and 260 nm). (Later on some fractions were analyzed further to determine the partition of certain UV absorbing peaks between fractions).

Three bigger fractions were then created by pooling the contents of tubes 1 to 257, 258 to 350 and 351 to 440, respectively. The three pools were freeze dried. The three freeze dried portions were then dissolved in 50 % (for the pool of tubes 1 to 257), 50% (for the pool of tubes 258-350) and 70% (for the tubes 351-440) acetonitrile in water (volume of each solution 100 ml) and the solution formed were again freeze dried. Extracts RA (43.5 mg) (for pool of tube 1-257), RB (83.9 mg) (for pool of tube 258-350) and RC (54.6 mg) (for pool of tube 351-440) were obtained as white fluffy powders. (Besides, an additional portion of RA (9.6 mg) was contaminated with material used in the laboratory practice (i.e. dye from marker used for labelling on glass surface) and collected separately and measured for its weight; adding up this materials with the RA collected the yield of RA was 53.1 mg.) (The weights above for RA, RB and RC are approximate, as the samples were weighed in plastic tubes, not on small pieces of paper or aluminum foil, because of suspected hygroscopic properties and static electricity).

Exemplary analytical HPLC chromatograms of extracts

RA, RB and RC, along with the starting material R2C, are shown in Drawing section Figure 3.

Assay of the sexual enhancing activities of extracts R2C, RA, RB and RC in mice is given in Example 34. Assay of the sexually enhancing activity of R2C was given in Example 22. Extracts R2C and RB are particularly preferred sexual enhancing extracts of the invention.

**Example 34: Assay of enriched extracts prepared from *Neobeguea mahafalensis* on sexual behavior in mice**

The activity of extract R2C, RA, RB and RC, prepared according to Example 33, was assayed for sexual enhancing activity essentially using the method describe in Example 30, the only difference being that the mice were treated with subcutaneous injections of extracts R2C, RA, RB or RC for three consecutive days at a dose of 4 mg/kg for each day. For the sake of administration extracts R2C, RB and RC were dissolved in sun flower oil and injected subcutaneously in a volume of 1 ml/kg of mice body weight. RA was dissolved in water and injected subcutaneously in a volume of 1 ml/kg. Controls received the same amount of sun flower oil subcutaneously for three consecutive days.

As seen from the Fig. 45, the number of mounts increased substantially for R2C and RB treated mice compared to the controls, both at the fourth and seventh day following the start of treatments. For extracts RA and RC there was no appreciable difference compared to the controls. Accordingly enriched extracts R2C and RB comprise extracts with sexual enhancing effects and are highly desired embodiments of the invention.

**Example 35: A formulation of a pharmaceutical prepared from extract RB and R2C from *Neobeguea mahafalensis* comprising oil solution for intramuscular or subcutaneous injection**

A)

| | |
|---|---|
| RB extract, prepared according Example 33 | 20 mg |

(continued)

| | |
|---|---|
| Cotton oil | ad 1 ml |

B)

| | |
|---|---|
| R2C extract, prepared according Example 33 | 20 mg |
| Cotton oil | 1 ml |

**Example 36: Formulation of a pharmaceutical comprising oil solution of extract RB and R2C from *Neobeguea mahafalensis* for soft gelatin capsules intended for per oral administration**

A)

| | |
|---|---|
| RB extract, prepared according Example 33 | 20 mg |
| Peanut oil | ad 1 ml |

The oil solution was filled into an open soft gelatin capsule and the capsule was sealed by adding a drop of hot liquidized gelatin to the opening and allowing the drop to cool down.
B)

| | |
|---|---|
| R2C extract, prepared according Example 33 | 20 mg |
| Peanut oil | ad 1 ml |

The oil solution was filled into an open soft gelatin capsule and the capsule was sealed by adding a drop of hot liquidized gelatin to the opening and allowing the drop to cool down.

**Example 37: Tea bag with ground root of *Neobeguea mahafalensis***

Heat-sealable tea bag paper was obtained from Glatfelter Corporation - Schoeller Hoesch (Papierfabrik Schoeller & Hoesch, GmbH & Co. KG, Postfach 1155, D-76584 Gernsbach, Germany). The paper was cut to 14 x 8 cm and was folded in the middle of the long direction so as to yield a double paper layer of 14 x 4 cm. The paper was then sealed by applying heat along the open long edge so as to form a tube 14 x 4 cm.
Root of *Neobeguea mahafalensis* was carefully ground into a fine powder and the tube was filled with 2 g of the powder. The tube was then folded again in the middle along the short direction thus forming a double layered tube with the powder inside and the openings to the same side. The tube was then closed in its open ends by pursing them together and sealing with a stapler. A string with a small paper label attached to its opposite was also attached under the staple to allow immersing and stirring the bag in hot water.

**Example 38: Preparation of biological sample for assay of characteristic mass-peaks**

Minipigs (Ellegaard Göttingen Minipigs ApS, Göttingen, Germany) were treated with 4, 40 or 400 mg/kg of extract R2C (prepared as described in Example 33) or the same quantities of extract RB (prepared as described in example 33) by subcutaneous injection dissolved in sun flower oil. 24 hours post-injection blood was drawn into heparinized Vacutainer glass tubes (Becton Dickinson Co.) and the plasma immediately prepared by centrifugation and stored at -20°C until time of further sample preparation.
Prior to use, all glassware was silanized by treatment with dimethyldichlorosilane and then rinsed with toluene and dried. Since the characteristic mass-peak arise from different compounds (components) several different extraction procedures were used in the preparation of samples in order to extract them optimally.

Preparation procedure 1

Ten ml aliquots of plasma were transferred to glass tubes with teflon-lined screw caps. Twenty ml of acetonitrile

were added to each tube, the samples vortexed for 30 seconds and then centrifuged at 2,000 rpm for 10 minutes. The supernatants were carefully decanted into clean tubes and the volumes reduced to less than 0.1 ml by evaporation at 40°C under a dry nitrogen stream for use in subsequent mass-spectrometric analysis.

Preparation procedure 2:

Ten ml aliquots of plasma were transferred to glass tubes with teflon-lined screw caps. Twenty ml of acetonitrile were added to each tube, the samples vortexed for 30 seconds and then centrifuged at 2,000 rpm for 10 minutes. The supernatants were carefully decanted into clean tubes and the volume reduced to less than 1 ml by evaporation at 40°C under a dry nitrogen stream. Ten ml of 0.2 N NaOH and 20 ml of a hexane-ethylacetate mixture (9:1, v/v) were added to each tube. The tubes were shaken for 30 minutes on a reciprocating shaker at 60 cycles/minute followed by centrifugation at 2,000 rpm for 5 minutes. The organic phases were combined and evaporated to dryness. The samples were dissolved in small volumes of acetonitrile for use in subsequent HPLC and/or mass-spectrometric analysis.

Preparation procedure 3:

Ten ml aliquots of plasma were transferred to glass tubes with teflon-lined screw caps. Twenty ml of acetonitrile were added to each tube, the samples vortexed for 30 seconds and then centrifuged at 2,000 rpm for 10 minutes. The supernatants were carefully decanted into clean tubes and the volume reduced to less than 1 ml by evaporation at 40°C under a dry nitrogen stream. Ten ml of 0.1 N HCI and 20 ml of a hexane-ethylacetate mixture (9:1, v/v) were added to each tube. The tubes were shaken for 30 minutes on a reciprocating shaker at 60 cycles/minute followed by centrifugation at 2,000 rpm for 5 minutes. The organic phases were combined and evaporated to dryness. The samples were dissolved in small volumes of acetonitrile for use in subsequent HPLC and/or mass-spectrometric analysis.

Preparation procedure 4:

Ten ml aliquots of plasma were transferred to glass tubes with teflon-lined screw caps. Twenty ml of acetonitrile were added to each tube, the samples vortexed for 30 seconds and then centrifuged at 2,000 rpm for 10 minutes. The supernatants were carefully decanted into clean tubes and the volume reduced to less than 1 ml by evaporation at 40°C under a dry nitrogen stream. Ten ml of 0.2 N NaOH and 20 ml of a hexane-ethylacetate mixture (9:1, v/v) were added to each tube. The tubes were shaken for 30 minutes on a reciprocating shaker at 60 cycles/minute followed by centrifugation at 2,000 rpm for 5 minutes. The organic phases were transferred to a clean glass tubes and 10 ml of 0.1 N HCI added. The tube was again agitated (15 minutes) and again centrifuged (10 minutes). The organic phases were combined and evaporated to dryness. The samples were dissolved in small volumes of acetonitrile for use in subsequent HPLC and/or mass-spectrometric analysis.

Preparation procedure 5:

Ten ml aliquots of plasma were transferred to glass tubes with teflon-lined screw caps. Twenty ml of acetonitrile were added to each tube, the samples vortexed for 30 seconds and then centrifuged at 2,000 rpm for 10 minutes. The supernatants were carefully decanted into clean tubes and the volume reduced to less than 1 ml by evaporation at 40°C under a dry nitrogen stream. Ten ml of distilled water and 20 ml of chloroform were added to each tube. The tubes were shaken for 30 minutes on a reciprocating shaker at 60 cycles/minute followed by centrifugation at 2,000 rpm for 5 minutes. The samples were dissolved in small volumes of acetonitrile for use in subsequent HPLC and/or mass-spectrometric analysis.

Preparation procedure 6:

500 μl plasma samples were diluted with 700 μl of acetonitrile. After vortex mixing during 30 s, the samples were ultra centrifuged for 8 min at 10,500 rpm. The supernatant was mixed in a 5 ml glass tube, with 800 μl of 1-octanosulfonic acid sodium salt solution at 0.2 M during 30 s.

The extraction procedure of the obtained solution was preformed on solid-phase extraction (SPE) cartridges using a vacuum manifold. Three milliliter per hundred milligrams Upti-Clean CN-S (INTERCHIM, Asniere sur seine, France) cartridges were used.

The mixture was loaded under vacuum on an extraction cartridge previously activated with 2 ml of methanol and 2 ml of water. The cartridge was then washed with 2 ml of water and dried under pressure for 5 min. Elution was carried out with 2 × 0.4 ml of methanol (sometimes ethylacetate or chloroform was used as eluent). The eluant was evaporated under a stream of nitrogen in water bath at 40 °C. The residue was reconstituted in 200 μl aliquot of acetonitrile.

A 50 μl aliquot was then analyzed in subsequent HPLC and/or mass-spectrometric analysis.

Sample analysis:

Samples prepared according to preparation procedures 1-6 were introduced into Q-Tof2 mass spectrometer using flow injection approach. The instrument was configured corresponding to either ESI or APPI analysis. Injection volume was 10 ml, flow rate - 100 ml/min. Alternatively, an HPLC column was connected to Q-Tof2 in order to perform LC/MS. The analysis was performed as described under Example 24 and 39. Characteristic set of mass peaks, corresponding to those listed in Examples 24 and 39, were detected.

**Example 39: Accurate mass measurements of RB extract using electrospray ionization and APPI on a Q-Tof2**

To analyze the presence of characteristic mass-peaks in the RB extract, the RB extract prepared according to Example 33 was subjected to repeated fractionation on a semi-preparative (10 x 250 mm) HPLC column filled with LiChrosorb RP18, particle size 10 microns (Merck Chemical Co., Germany). Eluent for this fractionation was 52% acetonitrile in water + 0.1% trifluoroacetic acid. Eluate fractions, containing ten different separated components were further investigated by analytical HPLC performed on an HPLC column (4.6 x 250 mm) filled with LiChrosorb RP18, particle size 5 microns, eluted by linear gradient from 20 to 80% acetonitrile in water + 0.1 %trifluoroacetic acid, flow rate 1.0 ml/min. A photo diode array (PDA) detection during HPLC analysis allowed us also to obtain characteristic UV spectra of separated RB components (obtained data for retention times on the HPLC column and UV maxima of the separate components are shown in the table in the drawing section, Figures 8 and 9). Ten fractions were collected in this way from the root extract RB, which were further freeze-dried and the powders obtained representing 10 different components from the root of *Neobeguea mahafalensis* were used for exact mass measurements on Q-Tof2. For ESI a Micromass nanoflow interface together with glass capillary option was used. For APPI the PhotoMate® photoionization source ('Fishbowl') from Syagen Technologies Inc., 1411 Warner Ave, Tustin, CA 92780, USA, combined with Waters IonSABRE™APCI (Waters Corporation, 34 Maple Street, Milford, MA 01757, USA); the analyte being introduced using flow injection.

Each one of the ten freeze dried components were dissolved separately to yield an analyte concentration of about 1 ng/mL in acetonitrile-water-formic acid (50:50:0.2). The solution was then mixed with a similar solution containing a reference substance with known molecular mass (preferably the molecular mass of the known substance was close to the mass peak of the analyte), and the mixture was introduced into the Q-Tof2 mass spectrometer using electrospray needle. ESI spectrum of the mixture was recorded. Composition of the mixture was optimized in terms of proportions of the components and their concentration, trying to obtain peaks of equal intensity comprising about 200 counts in one scan. When such a situation was attained the mass spectrum was recorded. The mass-peak value of the reference substance was used to correct the masses of the analytes, which was done automatically by the built in software of the Q-Tof2: MassLynx. The thus obtained mass values, representing the masses of the characteristic mass-peaks associated with the respective component of RB and are listed in the table of Figures 8 and 9 of the drawing section in the column labelled, positive ionization, ESI column.

Accurate mass measurements using electrospray ionization in negative mode were made in a similar fashion as above for each of the 10 components, however the solvent used for the components was acetonitrile-water (50:50) without the addition of formic acid. The mass values thus obtained, representing the masses of the characteristic mass-peaks associated with the respective component of RB, are listed in the table of Figures 8 and 9 of the drawing section in the column labelled, negative ionization ionization, ESI.

Accurate mass measurements using atmospheric pressure photo ionization (APPI) were made in the similar fashion as above, by injecting 10 μL of a solution of each of the ten freeze dried components mixed a with reference substance in methanol-toluene (95:5) using a syringe pump (flow rate 100 μL/min). The mass-peaks thus obtained are presented in the table of Figure 8 and 9 of the drawing section under Positive and Negative ionization, APPI.

**Example 40: Comparison of sexual activity enhancing activity of extracts from stem bark and root of *Neobeguea mahafalensis***

The activity of extracts R2C and S2C, prepared in essentially the identical fashion as described to Examples 9 and 10, from respectively stem bark and root of *Neobeguea mahafalensis* were assayed for sexual enhancing activity essentially using the method describe in Example 30, the only difference being that the mice were treated with subcutaneous injections with either of the extracts R2C or S2C at a dose of 4 mg/kg for each day for 3 consecutive days. Extracts R2C and S2C were dissolved in sunflower oil and injected in a volume of 1 ml/kg of mice body weight. Controls received the same injection schedule of sunflower oil. Each of the treatment group comprised 4 male mice. The sexual activity test was performed only on D-4 (i.e., fourth day after start of treatment of the male mice).

As seen from Figure 46, the number of mounts increased substantially for R2C treated mice compared to the control; the effect being highly significantly different from the control ($p<0.0005$; Student's two-tailed t-test). However, surprisingly S2C was completely inactive; i.e. not significantly different from the control ($p>0.38$; Student's two-tailed t-test). (Error bars in Figure 46 represent the standard error of the mean).

Conclusion

The extract of root from *Neobeguea mahafalensis* is highly active in enhancing sexual activity and thus comprise a highly desired embodiment of the invention - i.e. being an extract comprising sexual enhancing effect. However, the extract of the stem bark from *Neobeguea mahafalensis* is inactive and accordingly not very desired for administration to animal or human.

Because of the finding that S2C is devoid of sexual enhancement activity, S2C is not a desired part of the present invention. Accordingly the use of stem bark of *Neobeguea mahafalensis* is specifically excluded from all aspects of the present invention.

### Example 41: Preparation of R306 and R310 from root of *Neobeguea mahafalensis* by hot water and chloroform extraction and chromatography

Dried ground root (722 g) from *Neobeguea mahafalensis* was divided into 144 portions (about 5 g in each). Each portion was put in boiling water (500 ml). The mixtures were boiled for 2 min, and then allowed to slowly cool down for 16 h. The concoctions were filtered through cotton wool plugs placed into glass funnels. The solid residues (RR) were collected, united and dried on the open air until constant weight (405.4 g). The united filtrates were freeze-dried. A light-brown powder (RW) formed, but to save time the process was interrupted when about 10% of starting ice was still present.

Extraction procedure:

To about 1/15 part of the above material water (to 500 ml volume) and chloroform (500 ml) were added allowing melting the residual ice. The mixture was placed into a separation funnel and intensely shaken for 2 min. Then it was left to stand for 20 h (separation of phases proceeded very slowly, part of the mixture remained as an unseparated zone between water and chloroform layers containing precipitate). The chloroform and water layers were separately collected, but the intermediate zone was placed in plastic tubes resistant to chloroform and centrifuged at 3000 rpm for 15 min. Chloroform and water layers formed in the tubes after the centrifugation were separately collected by pipette. 20 ml of each solvent were equally divided and added to the tubes, tubes were vortexed, centrifugation repeated, and the new chloroform and water layers were again collected. Addition of water and chloroform solvents, vortexing, centrifugation, collection of layers was repeated four times. The unresolved central layer was evaporated. A brown-red powder (R2P) remained.

The procedure was repeated using combined water layers from the previous extraction, adding a fresh chloroform portion (500 ml). A new R2P portion was collected, which was combined with the previous one.

All 15 portions of RW were fractionated this way; summary yield of R2P was 14.4 g.

The united chloroform extracts were evaporated to dryness, the residue treated with acetonitrile (1000 ml), trying to dissolve it. Some part of residue (R2X, a wax-like material) was not soluble. The acetonitrile solution was separated by decantation and evaporated to dryness. Yield of oily residue (R2C) was 6.68 g. R2X weighed 146 mg.

United water phases obtained after extraction with chloroform were freeze-dried. A brown, fluffy powder (R2W) formed. Yield 101.1 g.

Fractionation of R2C using reversed phase liquid chromatography

R2C (6.68 g) was dissolved in acetonitrile (500 ml), and water (1000 ml) was added. A slightly turbulent solution formed. The turbulent solution was applied to a cooled (+5°C) glass column (10 x 24.5 cm) filled with LiChroprep RP-18 (Merck) and equilibrated with 30% acetonitrile in water + 0.1% trifluoroacetic acid. The column was then placed in the cold room and eluted first with 30% acetonitrile in water + 0.1% trifluoroacetic acid (4 liters), then with similar solutions, changing the acetonitrile content subsequently to 40%, 50%, 60 % and 70 % (volume of each eluent portion 4 liter). Flow rate was about 10 ml/min. Just after starting of application of the 70 % eluent about 60 ml of eluate were collected separately for further isolation of substantially pure compounds. This eluate fraction was diluted with an equal volume of water, frozen and freeze dried. A white powder (R3004) was obtained. Yield 250 mg. The column was regenerated by washing with isopropanol (4 liter) and before storage 40% ethanol (1 liter) was passed through it.

Preparation of crude R306 and R310

R3004 (about 20 mg) was placed into 1.5 ml volume centrifuge tube. 0.7 ml of acetonitrile were added, and vortexed until dissolution. Then 0.7 ml of water were added, the tube shaken and centrifuged. The clear supernatant obtained was introduced into a semipreparative Lichrosorb C18 column (10 x 250 mm) (Merck) previously equilibrated with 62 % acetonitrile in water. The column was eluted with the same buffer, flow rate 5 ml/min, UV detection at 270 nm. The eluate fraction corresponding to the peak that follows the highest peak in the peak group (retention time 30.6 min) was separately collected in order to obtain crude R306, and similarly eluent fraction corresponding to the last, remotely placed peak (retention time 46.8 min) was collected in order to obtain crude R310. In such a way R3004 was repeatedly applied to the Lichrosorb C18 column and the eluate fractions containing crude R306 and R310 were pooled, diluted with an equal volume of water, frozen and freeze-dried. Fluffy, white powders formed. Yield 58 mg of powder for crude R306 and 24 mg of powder for crude R310.

Preparation of substantially pure R306 (Grade 3)

Crude R306 from the previous step (about 2 mg) was placed into 1.5 ml volume centrifuge tubes, 0.5 ml isopropanol was added and the tubes were vortexed until dissolution. 1.0 ml of water was added, the mixture was shaken and centrifuged. The clear supernatant obtained was introduced into a semipreparative Chirobiotic V column (10 x 250 mm) (Astec; obtained from Supelco/Sigma-Aldrich, 595 N. Harrison Road, Bellefonte, PA 16823-0048, USA) previously equilibrated with 34 % isopropanol in water. The column was eluted with the same buffer, flow rate 1 ml/min, UV detection at 220 nm. The eluate fraction corresponding the central part of the main peak was separately collected. In such a way 20 mg of crude R306 were applied for purification. The appropriate eluate fractions were pooled and freeze-dried. Yield of substantially pure R306 (Grade 3) (white, fluffy powder) was 12 mg.

Preparation of substantially pure 306 (Grade 4)

R306 (Grade 3) from the previous step (about 1.2 mg) was placed into 1.5 ml volume centrifuge tubes, 0.5 ml isopropanol was added and the tubes were vortexed until dissolution of the substance. 1.0 ml of water was added, the mixture was shaken and centrifuged. The clear supernatant obtained was introduced into analytical Lichrospher C18 column (4.6 x 250 mm; Merck) previously equilibrated with 40 % isopropanol in water. The column was eluted with the same buffer, flow rate 1 ml/min, UV detection at 220 nm. The eluate fraction corresponding to the central part of the main peak was separately collected. In such a way 12 mg of R306 (Grade 3) were repeatedly applied for purification. The appropriate eluate fractions were pooled and freeze-dried. According to HPLC and mass-spectrometry a 100% pure product R306 (Grade 4) was obtained as a white, fluffy powder. Yield 7.6 mg. High-resolution mass-spectrometry 699.2991 ($M+H^+$).

Analytical HPLC of substantially pure R306 (Grade 4) was performed on a Waters system (Millenium32 Workstation, 2690 Separation Module, 996 Photodiode Array Detector) equipped with LISPRP18-5-3627 column (Hichrom Ltd, England) using water/2-propanol (60:40) as eluent (isocratic regime) at flow rate - 0.7 mL/min and detection at 220 nm yielded one single peak with retention time 9.957 min (Figure 9). The UV spectrum of R306 was recorded on-line with the diode array detector yielding absorption maxima at 198, 215 and 261 nm (Drawing section, Figure 10).

Preparation of substantially pure R306 (Crystalline)

R306 (Grade 4) (1.3 mg) was placed into a small thick-wall glass vial. Isopropanol (300 microlitres) was added, the vial was closed with a polypropylene cap and vortexed until dissolution. The solvent was then evaporated under a gentle argon stream till dryness. Formation of a white, crystalline residue occurred. Isopropanol (100 microlitres) was then added and the vial was placed in the refrigerator overnight. Then liquid was removed by pipette and the remaining crystals were dried in a desiccator. Yield 1.0 mg.

Preparation of substantially pure R310

Crude R310 obtained above was subjected to purification essentially as described above for R306 in Example 41 by first applying the crude R310 into the semipreparative Chirobiotic V column (10 x 250 mm) previously equilibrated with 34 % isopropanol in water and eluted with the same buffer, flow rate 1 ml/min, and after pooling and freeze-drying the main peak the materials was further applied into a Lichrospher C18 4.6 x 250 mm column equilibrated with water/2-propanol (65:35) and eluted with the same buffer (isocratic regime) at a flow rate of 1 mL/min and detection at 268 nm, which yielded essentially only one peak of substantially pure R310 eluted at the retention time 37.1 min, which was collected and freeze-dried. High-resolution mass-spectrometry 715.2953 ($M+H^+$). The UV spectrum of R310 was recorded on-line with the diode array detector yielding absorption maxima at 210 and 270 nm. In addition the thus obtained R310 was further purified on Lichrospher C18 column (4.6 x 250 mm; Merck) using essentially the same procedure as for R306 above, under "Preparation of substantially pure 306 (Grade 4)". The very pure R310 thus obtained was used for NMR determination of the structures the tautomeric forms R310A and R310B, of R310.

Analysis of substantial pure R310 by HPLC and UV-spectrometry

The sample of the very pure R310 from the previous step was analyzed by HPLC. It was then observed that the compound gradually formed two peaks on the chromatographs, which correspond to the keto and enol forms R310A and R310B, respectively; the keto form being the initial one purified and also residing in larger quantity in the equilibrium formed.

On LiChrosorb RP18-5 (2.1 mm x 100 mm, 5 $\mu$m) using a gradient formed from water and acetonitrile (from 20% to 90% MeCN) with 5 mM ammonium acetate additive; flow rate 0.2 mL/min during 60 min; detection at 220 nm, two peaks formed with retention times 37.0 (presumed R310B) and 42.1 (presumed R310A) min.

On LiChrospher RP18-5 (4.6 mm x 250 mm, 5 $\mu$m), using a Millenium32 Workstation, 2690 Separation Module, 996 Photodiode Array Detector, using acetonitrile (30:70) with 0.1% trifluoroacetic acid additive a eluent; flow rate 0.7 mL/min during 35 min (isocratic regime); detection at 220 nm, two peaks formed at 11.8 minutes (presumed R310B) and 16.4 minutes (presumed R310A).The UV spectra of the peaks were recorded with the Photodiode Array Detector; the presumed R310A form of R310 showing two UV absorption maxima at 208 and 269 nm, and the presumed R310B form of R310 showing two UV absorption maxima at 191 and 209 nm.

**Example 42: Preparation of R306 from root of *Neobeguea mahafalensis* by chloroform extraction and chromatography**

To dried ground root of *Neobeguea mahafalensis* (124 g) chloroform (1.2 liter) was added. The mixture was placed in the cold room (+5°C) and shaken overnight. The suspension was then filtered under vacuum through a porous glass filter, and the material on the filter was washed with chloroform (300 ml). The united filtrates were evaporated on rotary evaporator till dryness. A glass like material RCH (3.59 g) formed. The glass like material was agitated with acetonitrile (300 ml) for 30 min, trying to dissolve it. A precipitate formed. The mixture was vacuum filtered through a cotton wool plug placed into a glass funnel. The filtrate was diluted with an equal volume of water, and the emulsion formed was applied to a cooled (+5°C) glass column (10 x 24.5 cm) filled with LiChroprep RP-18 and equilibrated with 60% acetonitrile in water. The column was then placed in the cold room (+5°C) and eluted, first with 60% acetonitrile in water (4 liters), then with 70 % acetonitrile in water (4 liters). Flow rate was about 10 ml/min. Just after starting of application of the 70 % eluent, 100 ml size eluate fractions were collected. The content of R306 characteristic mass-peaks in these fractions was monitored using mass spectrometer with electrospray needle, looking for ion 699.3 ($M+H^+$). Substantial content of this ion was found in fractions 6 to 10 (i.e. 600 - 1000 ml of 70% eluent). These fractions were diluted with the equal volumes of water and freeze dried. The obtained fluffy powders were placed into 1.5 ml volume centrifuge tubes, each dissolved in 1.0 ml acetonitrile, 0.5 ml water added and centrifuged. The clear supernatant obtained was introduced into two semipreparative columns connected in sequence comprised of a Lichrospher C18 column and a Lichrosorb C18 column (each 10 x 250 mm) previously equilibrated with 65 % acetonitrile in water. The columns were eluted with the same buffer, flow rate 5 ml/min, UV detection at 260 nm. The eluate fractions corresponding to the descending slope of the main peak (including shoulder, monitoring the 699.3+ ion continuously) were separately collected, united and freeze dried affording 19.5 mg of white, fluffy powder. The powder was suspended in isopropanol (1.5 ml). The suspension was distributed between 5 centrifuge tubes (1.5 ml volume) 0.3 ml in each. To each tube 0.2 ml isopropanol and 1.0 ml water were added, shaken and centrifuged. The clear supernatant obtained was introduced into a semipreparative Chirobiotic V column (10 x 250 mm) previously equilibrated with 34 % isopropanol in water. The column was eluted with the same buffer, flow rate 1 ml/min, UV detection at 220 nm. The eluate fraction corresponding the former part of the main peak (monitoring the 699.3+ ion continuously) was separately collected. The appropriate eluate fractions from the following purification runs were pooled and freeze-dried. Yield of crude R306 (white, fluffy powder) was 2.4 mg.

The crude R306 sample from the previous step (about 1.2 mg) was placed into 1.5 ml volume centrifuge tube, 0.5 ml isopropanol added and the mixture was vortexed until dissolution. 1.0 ml of water was added, mixture shaken and centrifuged. The clear supernatant obtained was introduced into an analytical Lichrospher C18 column (4.6 x 250 mm) previously equilibrated with 40 % isopropanol in water. The column was eluted with the same buffer, flow rate 0.7 ml/min, UV detection at 220 nm. The eluate fraction corresponding to the main peak (monitoring the 699.3+ ion continuously) was separately collected. In such a way all of the remaining crude R306 from the previous step was applied for purification. The appropriate eluate fractions were pooled and freeze-dried. Yield of according to HPLC and mass-spectrometry 100% pure product R306 (Grade CH 4) (white, fluffy powder) was 1.0 mg. Mass-spectrometry data: 699.3 ($M+H^+$), 716.3 ($M+NH4^+$), 721.3 ($M+Na^+$), 737.3 ($M+K^+$).

**Example 43: Dose and time response of R306 and R310 on sexual activity in mice**

The sexual enhancing effect of R306 (Grade CH 4; i.e. better than 99 % pure R306) from Example 42 was assessed using the method described in Example 30. Mice were injected subcutaneously with either of 0.4, 0.04 and 0.004 mg/kg of R306 (Grade CH 4) dissolved in sunflower oil each day for three consecutive days. Control mice received sun-flower oil only. Each group contained 3 mice.

The number of mounts of each male mouse onto the female mouse was then counted over a 3 hour period on respectively the 4th day (D-4), the 7th day (D-7) and the 14th day (D-14) after the first days injection of R306 for each couple and the average of number of mounts was calculated for each group.

Results are shown in Drawing section Figure 12 (top panel). As seen significant increases on the sexual activity were seen with the two highest doses of R306 as well as a tendency of increase was seen with the lowest dose tested. The effect peaked at D7, while clear effects were seen also at D14. The number of mounts counted during the 3 hour assessment period increased about 3.5 fold on D4 at the highest dose of R306 evaluated (0.4 mg/kg).

Based on these data, using the 7th day as a basis for computation, the activity of the substantially pure R306 was estimated to be about 15 U/mg (i.e. 15 $U_{mnt}$/mg; i.e. computed by fitting the mounting data of Figure 12A to the logistic function as described in Example 23, which determined the $EC_{50}$ of R306 to be about 0.06625 mg/kg; hence 1/0.06625 = 15.09434).

The sexual enhancing activity of R310 obtained as described in Example 41 was assessed using the same method as described in Example 30. Mice were injected subcutaneously with 4, 0.4 or 0.04 mg/kg of R310 dissolved in sunflower oil each day for three consecutive days. Control mice received sunflower oil only. Each group contained 3 mice.

The number of mounts of the female over a 3 hour period were then counted on day 4 (D-4), day 7 (D-7) and day 14 (D-14) following the first injection of R310. Results are shown in Drawing section Figure 12 bottom panel of the drawings section. Significant increases on the sexual activity were seen at the highest dose of R310 evaluated on all days tested. For the two lower doses of R310 sexual activity was significantly increased only on D-4.

Significances in this example were assessed with Student's t-test, two-tailed.

**Example 44: Pharmaceutical preparations of R306**

Preparation of R306 for oral use:

| | |
|---|---|
| R306 (Grade 4) | 15 mg |
| Cotton oil | ad 1 ml |

Preparation of R306 for subcutaneous injection:

| | |
|---|---|
| R306 (Grade 4) | 5 mg |
| Peanut oil | ad 1 ml |

**Example 45: Pharmaceutical preparation of R310**

Preparation of R310 for oral use:

| | |
|---|---|
| Substantially pure R310 prepared according to example 41 | 15 mg |
| Cotton oil | ad 1 ml |

Preparation of R310 for subcutaneous injection:

| | |
|---|---|
| Substantially pure R310 prepared according to example 41 | 5 mg |
| Peanut oil | ad 1 ml |

**Example 46: Pharmaceutical preparation containing a mix of R306 and R310 for injection**

| | |
|---|---|
| R306 (Grade 4) | 5 mg |
| Substantially pure R310 prepared according to example 41 | 10 mg |
| Cotton oil | ad 1 ml |

**Example 47: Determination of the structure of R306, R310A and R310B by NMR**

METHODS

NMR spectra were recorded with a Varian UNITY INOVA 600 MHz spectrometer equipped with a cryoprobe in $CDCl_3$ solution at 25°C. Chemical shifts are reported in ppm relative to residual solvent signal ($\delta(^1H)$ 7.25 ppm, $\delta(^{13}C)$ 70.0 ppm). Two-dimensional spectra recorded included DQF-COSY, NOESY, TOCSY, sensitivity-enhanced $^{13}C$-HSQC and $^{13}C$-$^1H$ HMBC. Pulsed-field gradients were used for all $^{13}C$ correlation spectra. The NOESY mixing time was 800 ms, the TOCSY one 70 ms. $^{13}C$-HMBC spectra were recorded with coupling evolution delay for the generation of multiple-bond correlations set to 62.5 ms. All 2D spectra were run with 4096*1024 points data matrix, giving $\tau_{2max}$=250ms for $^1H$ nucleus in acquisition dimension and $\tau_{1max}$=100ms for $^1H$ or $\tau_{1max}$=50ms for $^{13}C$ for indirect dimension. Prior to Fourier transform the data matrix was zero-filled twice and multiplication by shifted sine-bell window function applied. For $^1H$-$^{13}C$ HMBC the magnitude spectra were calculated. Graphs from the spectra recorded are shown in drawing section figures 15 - 27.

RESULTS

The structure elucidations were accomplished on the basis of two-dimensional $^1H$-$^1H$ and $^1H$-$^{13}C$ spectra of the compounds taken in $CDCl_3$ solutions. According to the spectra R306 was stable during several weeks, but R310 consisted of two isomers which slowly interchanged. One of the R310 isomers had a very low field signal at 13.84 ppm being characteristic for the intramolecularly chelated OH proton in β-diketones when the enolized proton is hydrogen-bonded to the ester function. Thus, the initial proposal was that R310 should contain a β-diketone moiety that gives two forms in the spectrum due to slow keto-enol equilibrium. Analysis of low field part of the $^1H$ spectrum showed that both of the R306 and R310 compounds contain a 3-substituted furane cycle. For R306 this furane cycle was attached to the rest of molecule through a carbonyl function in position 17, whereas in R310 instead of a carbonyl group there was a 17-CH($OCOCH_3$) substituent.

Careful analysis of the $^1H$-$^{13}C$ HMBC spectra allowed us to propose the structure depicted in the drawing section figures 28, 29 and 30 for the carbon skeleton, for respectively compounds R306, R310A and R310B. The numbering and the relevant HMBC correlations are shown in these figures too. For structure R306 all $^1H$-$^{13}C$ HMBC correlations are shown in these figures, as well. For R310A and R310B are shown only those ones conforming to the position and type of substituents. The measured chemical shifts are collected in Table 1, Example 47 below. Both molecules R306 and R310 share a highly similar carbon skeleton, resembling limonoid orthoacetates; differences in the compounds are due to the side chains. In R306 the hydroxyl functions in positions 2 and 3 are acylated by isopropylcarboxy groups, whereas for R310 there one of these positions is acetylated with an acetyl group. The relative stereochemical positions of substituents and rings were established on the basis of NOESY data. The diagnostic NOE correlations are shown in drawing section Figures 31, 32 and 33 for, respectively compounds R306, R310A and R310B; they closely follow the stereochemical orientations typical for limonoid ortho esters. Interestingly, in both molecules R306 and R310 there is new six-membered cycle formed by carboxyl-16 with oxygen at carbon-30, not earlier found in other limonoids. The relative orientation of this new cycle comes from the stereochemistry of 14-CH and 30-CH. The α-configuration of the 14-CH bond was deduced from strong NOEs between 14-CH and 18-$CH_3$, but 30-CH has a β-orientation and a very strong NOE with 5-CH as expected. Thus, the new ring has trans- fusion relative to limonoid cycle B and cis- fusion relative to ring C. The β-orientation of the furane substituent was established by NOEs to methylester group in both molecules.

In R310A and R310B the β-diketone moiety is incorporated as side chain in position-15. Unequal populations of keto- and enol- forms allowed us to make the assignments of $^1H$ and $^{13}C$ resonances for both forms of R310 separately (Table 1, Example 47). The R-configuration of 17-CH in R310A and R310B was deduced on basis of very strong NOEs to 30-CH and 5-CH, as well as NOEs between the furane ring and 12-CH2 and NOEs between 17-OAc methyl and isopropyl group.

Table 1. $^{1}H$ (600 MHz) and $^{13}C$ (150.9 MHz) NMR Data for Compounds 306 and 310 AB in $CDCl_3$

| No. | --------R306-------------- | | -----R310A(keto)------- | | -----R310B(enol)------- | |
|---|---|---|---|---|---|---|
| | δ($^{1}H$), (*J*,inHz) | δ($^{13}C$) | δ($^{1}H$), (*J*,inHz) | δ($^{13}C$) | δ($^{1}H$), (*J*,inHz) | δ($^{13}C$) |
| 1 | - | 84.4 | - | 84.0 | - | 84.0 |
| 2 | - | 83.3 | - | 78.2 | - | 77.7 |
| 3 | 5.37 s | 80.4 | 4.83 s | 82.2 | 4.84 s | 82.9 |
| 4 | - | 46.2 | - | 45.5 | - | 45.4 |
| 5 | 2.54dd (2.4 ; 9.8) | 35.9 | 2.84dd (3.2;8.8) | 37.1 | 3.00dd (2.3;9.7) | 37.0 |
| 6 | pro-R2.08dd (9.8;15.5) | 33.6 | 2.37dd (3.2;15.5) | 34.1 | 2.26dd (3.2;16.7) | 33.7 |
| | pro-S 2.32dd (2.4;15.5) | | 2.44dd (8.8;15.5) | | 2.46dd (9.7;16.7) | |
| 7 | - | 172.3 | - | 172.6 | - | 172.5 |
| 8 | - | 79.7 | - | 79.4 | - | 80.5 |
| 9 | - | 83.7 | - | 83.8 | - | 84.8 |
| 10 | - | 46.6 | - | 46.0 | - | 45.9 |
| 11 | pro-R 1.39 m | 25.0 | 1.96 m | 24.3 | 1.92 m | 23.3 |
| | pro-S 2.17 m | | 2.19 m | | 2.03 m | |
| 12 | pro-R 1.83 m | 31.4 | 1.50 m | 32.0 | 1.50 m | 30.9 |
| | pro-S 2.11 m | | 1.42 m | | 1.16 m | |
| 13 | - | 51.6 | - | 38.7 | - | 39.8 |
| 14 | 1.96dd (1.7;8.1) | 49.1 | 2.79 d (0.8) | 50.6 | 2.66 s | 44.8 |
| 15 | 2.71;2.73 m | 28.4 | 3.80 d (0.8) | 52.1 | - | 90.8 |
| 16 | - | 169.0 | - | 167.2 | - | 170.5 |
| 17 | - | 199.0 | 6.00 s | 69.6 | 5.84 s | 70.6 |
| 18 | 1.48 s | 24.5 | 1.18 s | 21.4 | 1.22 s | 21.6 |
| 19 | 1.10 s | 16.0 | 1.15 s | 15.3 | 1.15 s | 15.3 |
| 20 | - | 124.6 | - | 121.4 | - | 122.1 |
| 21 | 8.06dd (1.4;0.8) | 147.1 | 7.55dd (1.7;0.8) | 141.3 | 7.49dd (1.7;0.8) | 140.7 |
| 22 | 6.79dd (1.9;0.8) | 110.1 | 6.35dd (2.0;0.8) | 109.3 | 6.39dd (2.0;0.8) | 109.7 |
| 23 | 7.41dd (1.9;1.4) | 143.4 | 7.35dd (2.0;1.7) | 142.8 | 7.32dd (2.0;1.7) | 142.5 |
| 28 | 0.90 s | 15.0 | 0.96 s | 14.8 | 0.95 s | 14.6 |
| 29 | pro-R 2.00 (11.1) | 40.8 | 1.96 d (10.9) | 39.6 | 1.92 d (10.9) | 39.4 |
| | pro-S 1.80 (11.1) | | 1.79 d (10.9) | | 1.81 d (10.9) | |
| 30 | 5.87 s | 74.8 | 5.25 s | 75.8 | 5.32 s | 74.4 |
| 31 | - | 118.6 | - | 118.6 | - | 118.5 |
| 32 | 1.62 s | 21.3 | 1.55 s | 20.7 | 1.57 s | 20.9 |

(continued)

| No. | --------R306-------------- | | -----R310A(keto)------- | | -----R310B(enol)------- | |
|---|---|---|---|---|---|---|
| | δ($^1$H), (*J*,inHz) | δ($^{13}$C) | δ($^1$H), (*J*,inHz) | δ($^{13}$C) | δ($^1$H), (*J*,inHz) | δ($^{13}$C) |
| $CH_3O$- | 3.51 s | 51.7 | 3.73 s | 52.0 | 3.69 s | 51.9 |
| | 2- isopropilcarboxyl- | | 2- acetyl- | | 2-acetyl- | |
| C= O | - | 175.3 | C=O | 170.6 | C=O | 170.4 |
| CH | 2.59 septet(6.9) | 35.1 | $CH_3$ 2.22 s | 20.3 | $CH_3$ 2.27 s | 20.5 |
| $CH_3$ | 1.19 d(6.9) | 19.0 | | | | |
| | 1.16 d(6.9) | 18.5 | 17-acetyl- | | 17-acetyl- | |
| | 3- isopropilcarboxyl- | | C=O | 169.5 | C=O | 169.3 |
| C=O | - | 175.8 | $CH_3$ 2.01 s | 21.1 | $CH_3$ 1.97 s | 20.5 |
| CH | 2.78 septet(7.4) | 34.8 | | | | |
| $CH_3$ | 1.38 d(7.4) | 18.6 | 15-C(O)CH($CH_3$)$_2$ | | 15- =C(OH)CH(CH3)2 | |
| | 1.37 d(7.4) | 18.9 | C=O - | 208.4 | =C(OH) 13.84 | 182.8 |
| | | | CH 3.15 septet(6.8) | 37.9 | CH 2.94 septet(6.8) | 29.9 |
| | | | $CH_3$ 1.16 d(6.8) | 19.5 | $CH_3$ 1.26 d(6.8) | 18.2 |
| | | | 1.04 d(6.8) | 18.9 | 1.12 d(6.8) | 20.4 |

**Example 48: Determination of the content of R306 in R2C and S2C preparations**

All experiments were carried out using a Perkin Elmer PE SCIEX API 150EX LC/MS mass spectrometer equipped with a turboionspray ion source (PerkinElmer Life and Analytical Sciences); samples were injected onto a LiChrosorb RP18-5 (2.1 mm x 100 mm, 5 μm) HPLC column (Merck Chemical Co., Germany) attached to the mass spectrometer and eluted with a gradient formed from water and acetonitrile (from 20% to 90% acetonitrile) with a 5 mM ammonium acetate additive during a 60 min period at a flow rate of 0.2 mL/min; eluted peaks were detected by monitoring for the positive ion around 699.3 amu corresponding to the computed molecular mass of R306. In addition peaks were also detected by UV detection at 220 nm, besides monitoring with the mass spectrometer. R306 eluted with a peak at about 36.8 min. (Note that in the following all ion-currents were detected at 699.4 amu - the difference to the 699.3 amu calculated from the R306 structure being due to the inability of the mass spectrometer to determine molecular masses at higher resolution than about ± 0.1 amu.)

***Standard curve***

The accurate weight of 0.106 mg of pure R306 (prepared according to Example 41) was dissolved in 0.707 mL of a mixture of acetonitrile/water (1:1) thereby yielding a stock solution with a concentration of 0.15 mg/mL of R306. Using this solution six experiments were curried out by injecting different amounts of the R306 sample onto the HPLC column and detecting the response occurring at 699.4 amu about 36.8 min following the injection.

Experiment 1: 20 μL of the R306 stock solution was injected onto the HPLC column; i.e. 3.0 μg of R306 was loaded onto the column.

Experiment 2: 10 μL of the R306 stock solution was injected onto the HPLC column; i.e. 1.5 μg of R306 was loaded onto the column.

Experiment 3: The above stock solution of R306 was diluted two-fold with a mixture of acetonitrile/water (1:1) yielding a solution of 0.075 mg/mL of R306, and 10 μL of this solution was injected onto the HPLC column; i.e. 0.75 μg of R306 was loaded onto the column.

Experiment 4: The diluted R306 solution of Experiment 3 was diluted further two-fold with a mixture of acetonitrile/water (1:1) and 10 μL of this solution was injected; i.e. 0.375 μg of R306 was loaded onto the column.

Experiment 5: The diluted R306 solution of Experiment 4 was diluted further two-fold with a mixture of acetonitrile/water (1:1) and 10 μL of this solution was injected; i.e. 0.1875 μg of R306 was loaded onto the column.

Experiment 6: The diluted R306 solution of Experiment 5 was diluted further two-fold with a mixture of acetonitrile/water (1:1) and 10 μL of this solution was injected; i.e. 0.09375 μg of R306 was loaded onto the column.

The peak responses from the mass spectrometer above the base-line at 699.4 amu occurring at about 36.8 min following the injections were monitored in cps (counts per seconds) and were determined as follows:

Results from assays of R306, pure sample:

| Experiment | Weight of R306 injected (μg) | Peak response for positive ion at 699.4 amu (cps) |
|---|---|---|
| 1 | 3.0 | $6.84 \times 10^6$ |
| 2 | 1.5 | $5.64 \times 10^6$ |
| 3 | 0.75 | $4.89 \times 10^6$ |
| 4 | 0.375 | $3.89 \times 10^6$ |
| 5 | 0.1875 | $2.89 \times 10^6$ |
| 6 | 0.09375 | $1.99 \times 10^6$ |

(Exemplary recordings from these assays are also shown graphically in the Drawing section, Figure 34). From these data a standard curve was constructed by plotting on millimeter paper. The curve looked essentially as shown in Figure 47:

Although the curve is non-linear the amount of R306 in a sample can thus be estimated by using the standard curve from the measured peak ion-current at 699.4 amu occurring close to about 36.8 min following the sample's injection onto the HPLC column.

***Experiments with R2C***

Two samples of R2C were prepared from roots of *Neobeguea mahafalensis* collected from two different trees. Sample 1 was from an old stressed tree with heavily damaged stem bark collected in November 2005 and Sample 2 was from a young healthy tree obtained on February 8, 2008. The R2C samples were prepared using the procedure described in Example 9. (Exemplary recordings from these assays are shown in Drawing section, Figure 34).

<u>Sample 1 of R2C</u>

R2C (0.304 mg) was dissolved in 0.760 mL of a mixture of acetonitrile/water (1:1) yielding a concentration of 0.4 mg/mL of R2C. Three experiments were carried out as follows:

Experiment 1: 50 μL of the R2C solution (0.4 mg/mL) was injected onto the HPLC column; i.e. 20 μg of R2C was loaded onto the column.

Experiment 2: 25 μL of the R2C solution (0.4 mg/mL) was injected; i.e. 10 μg of R2C was loaded onto the column.

Experiment 3: 10 μL of the R2C solution (0.4 mg/mL) was injected; i.e. 4 μg of R2C was loaded onto the column.

The following results were obtained and after using the above standard curve the content of R306 in R2C was determined:

| Weight of R2C injected, μg | Peak response for positive ion at 699.4 amu at about 36.8 min in cps | Total amount of R306 in injected sample estimated by reading from standard curve, in μg | Content of R306 in R2C, μg/mg (%) |
|---|---|---|---|
| 20 | $3.95 \times 10^6$ | 0.39 | 19.5 (1.95) |
| 10 | $2.85 \times 10^6$ | 0.19 | 19.0 (1.90) |
| 4 | $1.35 \times 10^6$ | 0.065 | 16.25 (1.625) |

The average content of R306 in Sample 1 of R2C is (19.5 + 19 + 16.25)/3 = 18.25 μg/mg; i.e. Sample 1 of R2C contained 1.825 % R306.

<u>Sample 2 of R2C</u>

R2C (0.320 mg) from Sample 2 was dissolved in 0.8 mL of a mixture of acetonitrile/water (1:1) yielding a concentration of 0.4 mg/mL of R2C. The solution was injected onto the HPLC column in three experiments in the same way as for Sample 1 above; the results obtained were as follows:

| Weight of R2C injected, μg | Peak response for positive ion at 699.3 amu at about 36.8 min in cps | Total amount of R306 in injected sample estimated by reading from standard curve, in μg | Content of R306 in R2C, μg/mg (%) |
|---|---|---|---|
| 20 | 2.19e6 | 0.12 | 6.0 (0.6) |
| 10 | 1.28e6 | 0.057 | 5.7 (0.57) |
| 4 | 0.63e6 | 0.027 | 6.75 (0.65) |

The average content of R306 in Sample 1 of R2C is (6 + 5.7 + 6.75)/3 = 6.15 μg/mg; i.e. 0.615 %.

Conclusion: Because of the difference in contents of the active component R306 in R2C samples from different specimens of *Neobeguea mahafalensis* a very important aspect of the invention is to determine the content of R306 in extracts prior to their use for preparation of pharmaceutical and prior to administration to human subjects. This assures quality control and is used to determine the dosage of extract to be administered to human subjects as well as *Neobeguea mahafalensis* specimens can be collected which contain large amounts of R306.

***Experiments with S2C***

S2C was prepared from stem-bark of *Neobeguea mahafalensis* as described in Example 10. 20 μg of the S2C sample was injected onto the HPLC column and assayed as for the other samples. The results from these assays are exemplified in Drawing section Figure 34, panel g. As seen there is essentially no ion current at 699.4 amu at the elution time for R306. Therefore S2C is essentially devoid of R306 (i.e. it contains at least less than 0.1 % of R306). Because of this finding S2C is not desired part of the present invention. Accordingly the use of stem bark of *Neobeguea mahafalensis* is specifically excluded from all aspects of the present invention.

**Example 49: Comparison of the sexual enhancing effect of oral and subcutaneous administration of R2C to male mice**

The enriched extract R2C was prepared using the procedure described in Example 9 and assayed for its content of R306 using the procedure of Example 48, which was determined to be 0.62 %.

R2C was dissolved in olive oil and administered to male mice, either orally or by subcutaneous injection for three consecutive days and the sexual enhancing activity was then assessed on the 4th and 7th day following the start of the experiment essentially using the method describe in Example 30. Controls were subject to the same treatment schedule but given the same amounts of olive oil - per os or by subcutaneous injection, respectively. Each group of treatment comprised 3 animals.

For oral treatments the doses given each of the tree days were 0.4, 4 and 40 mg/kg. At the 4th day the sexual behaviour was assessed; all doses increased the number of mountings during a 3 hour period with the effect being highly significant at 4 and 40 mg/kg. These results are illustrated in Figure 48.

At the 7th day following the start of treatments an enhancement of the mountings were also observable; the increase in number of mounts being significant at all doses of R2C administered. The results are illustrated in Figure 49.

The subcutaneous administration of R2C was assessed at a dose of 4 mg/kg. A significant enhancement of the number of mounts was seen both at the 4th and 7th day, as illustrated in Figure 50.

Assessing the effects at 4 mg/kg for oral and subcutaneous administration at the 4th and 7th day there was no statistical difference in the increase in the number of mounts seen by the two different modes of administration of R2C.

Conclusion: Oral and subcutaneous administration of R2C when administered dissolved in vegetable oil are equally effective modes of administration for achieving a sexual enhancing effect.

Note: Statistics were by Student's non-paired t-test, two tailed

<u>Estimation of sexual enhancing activity of R2C</u>

Based on these data, using the 7th day of oral administration of R2C as a basis for computation the $EC_{50}$ of R2C was estimated to be about 0.91 mg/kg. This gives an activity for this R2C preparation (which in fact contains 0.61 % R306) amounting to 1.1 $U_{mnt}$/mg; i.e. 1/0.91 = 1.098901.

**Example 50: Preparation of DCM, 01DG2 and D-Ac1 extracts**

500 g of dried and powdered roots of *Neobeguea mahafalensis* were extracted with 1 L of dichloromethane during 1 h by shaking at room temperature. The dichloromethane solution was filtered off and the remainder of the roots were once again extracted with 1 L dichloromethane in the same way, and the two filtrates were pooled and evaporated to dryness under reduced pressure, yielding 7.8 g of crude dichloromethane extract, herein termed "DCM". The remainder of the "dichloromethane exhausted" roots were extracted twice with 1 L acetone using the same procedure; after evaporation of the acetone 5.2 g of crude acetone extract (herein termed "01DG2") had been obtained.

Minor samples of the DCM and 01DG2 extracts were taken away for testing of their sexual enhancing effects (see further below) while the major parts of these extracts were combined (i.e. yielding in total 13 g) and 8 g of this combined extract was dissolved in 5 mL dichloromethane and applied to a silica gel column (length 42 cm, internal diameter 2.5 cm; 97 g of silica 60 from Merck, Germany).

Elutions then proceeded in steps applying 300 mL of solvent or solvent mixture with increasing polarity (i.e.

dichloromethane and methanol/dichloromethane mixtures) in each step, collecting 10 mL fractions and subjecting these fractions to silica gel thin layer chromatography (TLC; using Merck TLC aluminium sheets Silica gel 60 $F_{254}$ no. 1.05554.0001) using 1 % methanol in dichloromethane as mobile phase and searching after a TLC profile matching that of the R2C extract prepared according to Example 9. Specifically three major UV absorbing spots with $R_f$-values 0.14, 0.32 and 0.40 which are clearly detectable in R2C samples by illumination with a UV lamp at 254 nm were looked for (the spots with $R_f$ 0.14 and 0.40 being quite narrow while that of 0.32 being quite broad). After each step the column was washed with additionally 100 mL of the same solvent or solvent mixture and then elutions proceeded with the next step.

Step 1 comprised elutions with 300 mL of dichloromethane only and none of the fractions collected yield the desired TLC profile; all spots showing up having too high $R_f$ values as being detectable on the TLC, and these eluents were therefore discarded; the column was then washed with additionally 100 mL of dichloromethane and the resulting eluent was also discarded.

Step 2 comprised elutions with 300 mL of 1 % methanol in dichloromethane and did also not yield the desired TLC profile, with spots of too high $R_f$ values being detectable on the TLC, and these eluents were accordingly discarded; the column was then washed with additionally 100 mL of 1 % methanol in dichloromethane and the eluent discarded.

Step 3 comprised elutions with 300 mL of 2 % methanol in dichloromethane and did also not yield the desired TLC profile with spots of too high $R_f$ values being detectable on the TLC and these eluents were also discarded; the column was then washed with additionally 100 mL of 2 % methanol in dichloromethane and the eluent discarded.

Step 4 comprised elutions with 300 mL of 3 % methanol in dichloromethane and collecting 10 mL fractions. Starting from fraction 7 and onward to fraction 18 the UV absorbing TLC spots with the desired $R_f$ values 0.14, 0.32 and 0.40, with a pattern closely resembling that of R2C, in parallel TLC runs, became detectable and these fractions were then combined and the solvent evaporated under reduced pressure yielding the desired D-Ac1 extract at a final yield of 4.6029 g.

Measurement of contents of R306 in DCM, 01DG2 and D-Ac1 extracts

The contents of R306 were measured using the method described in Example 48 and were found to be as follows: DCM 0.37%, 01DG2 0.57% and D-Ac1 0.55%. By calculation the average content of R306 in the combined DCM+O1DG2 extract was 0.45 %.

Assay of sexual enhancing effect of DCM, 01DG2 and D-Ac1 extracts

The sexual enhancing effects of DCM, 01DG2 and D-Ac1 were assessed essentially using the method described in Example 30. Moreover, for comparison a dichloromethane extract of the stem bark of *Neobeguea mahafalensis* had been prepared in exactly the same way as the dichloromethane extract of the root, DCM, prepared above.

The extracts were dissolved in olive oil and administered subcutaneously, D-Ac1 at 0.4 mg/mg; all the others at 4 mg/mg. Mountings were recorded during 1 h following introduction of the male mice the female mice. Each group comprised 3-6 male mice. Results were as shown in Figure 51; each bar representing the average ± S.E.M.:

Significances were computed using Student's t-test for non-paired samples, treated vs. Control and were computed two-tailed. All extracts except the stem bark yielded statistically significant sexual enhancing effect. As seen all root extracts showed sexual enhancing activity according to the definition herein. However, the dichloromethane stem bark extract was devoid of sexual enhancing activity.

**Example 51: Solubility of R2C extract**

The solubility of R2C extract prepared according to Example 9 was tested for its solubility in sunflower oil, octan-1-ol and hexane as follows.

Sunflower oil:

R2C, 1 mg, was added to 0.1 ml sunflower oil at 20°C. A clear solution was obtained. Additionally 1 mg R2C was added. A clear solution was formed. Additionally 0.1 mg of R2C was added and a clear solution was formed. When adding additionally 0.1 mg of R2C some unsolubilised material remained.

*Conclusion*: R2C is soluble up to a concentration of 21 mg/ml in sunflower oil at 20°C.

Octan-1-ol:

R2C, 0.75 mg, was added to 0.05 mL octan-1-ol at 20°C. A clear solution was obtained. Additionally 0.75 mg of R2C was added and a clear solution was again obtained.

*Conclusion*: R2C is soluble in octan-1-ol at 20°C up to a concentration of at least 30 mg/ml.

Hexane:

R2C, 0.5 mg, was added to 0.5 mL *n*-hexane at 20°C. A turbulent solution was obtained. Addition of additional 0.5 mL *n*-hexane did not still give a clear solution. T

*Conclusion*: R2C is only partially soluble in *n*-hexane.

**REFERENCES**

1. Baldwin, DS: Sexual dysfunction associated with antidepressant drugs. Expert Opinion Drug Safety 2004, 3(5), 457-470.

2. Shabbir, M., D.M. Mihailidis and R.J. Morgan: Erectile dysfunction: an underdiagnosed condition associated with multiple risk factors. Current Medical Research and Opinions: 2004, 20(5), 603-606.

3. Hafez, ESE and S.D. Hafez. Erectile dysfunction: Anatomical parameters, etiology, diagnosis and therapy. Archives of Andrology. 2005, 51, 15-31.

4. Giuliano, F., and O. Rampin: Neural control of erection. Physiology & Behaviour. 2004, 83, 189-201.

5. Andersson, K-E.: Pharmacology of penile erection. Pharmacological Reviews. 2001, 53(3), 417-450.

6. Kendirci, M., S. Nowfar and W.J.G. Hellstrom: The impact of vascular risk factors on erectile function. Drugs of Today. 005, 41(1), 65-74.

7. Ralph, D.J. and K.R. Wylie: Ejaculatory disorders and sexual function. BJU International. 2005 Jun;95(9):1181-1186.

8. Meuleman, E.J.H and J.J.D.M. Lankveld: Hypoactive sexual desire disorder: an underestimated condition in men. BJU International. 2005, 95, 291-295.

9. Enserink, M: Let's talk about sex - and drugs. Science. 2005, 308, 1578-1580.

10. Bolour, S and G. Braunstein: Testosterone thrapy in women: a review. International Journal of Impotence Research. 2005, 1-10 (doi: 10.1038/sj.ijir.3901334).

11. Basson, R.: Female sexual response: the role of drugs in the management of sexual dysfunction. Obstet. Gynecol. 2001, 98(2), 350-353.

12. Rosen, R.C., A. Riley, G. Wagne, I.H. Osterloh, J. Kirkpatrick, A. Mishra: The international index of erectile function (IIEF): a multidimensional scale for assessment of erectile dysfunction. Urology. 1997 Jun;49(6):822-30.

13. Cappelleri, J.C. and R.C. Rosen: The sexual health inventory for men (SHIM): a 5-year review of research and clinical experience. International Journal of Impotense research. 2005, 17, 307-319.

14. Davis , S.R., S.L. Davison, S. Donath and R.J. Bell: Circulating androgen levels and self-reported sexual function in women. JAMA 2005, 294 (1), 91-96.

15. Sandroni, P.: Aphrodisiacs past and present: a historical review. Clin Auton Res. 2001 Oct;11(5:303-7.

16. Philippe Rasoanaivo, monthly up-dated database of ethnomedical data of Madagascan plants (based on literature

data), with 6,178 plants recorded hitherto.

17. Philippe Rasoanaivo, Alain Petitjean, Suzanne Ratsimamanga-Urverg, Albert Rakoto-Ratsimamanga (1992) Medicinal plants used to treat malaria in Madagascar, Journal of Ethnopharmacology, 37, 117-127.

18. Davidra H. Rajaonatahina, 'Médecine traditionnelle, les croyances, la tradition et les maladies transmissibles' , These de Doctorat en Médecine 1992, N° 2693.

19. M. Debray. Contribution à l'inventaire des plantes médicinales de Madagascar: Region Sud-Ouest. Documents de l'ORSTOM, 1971, p. 21.

20. Paris, RR and Debray, M: Sur les polypenols (acides-phénols, flavinoides) des feulles de deuc mélicées malagaches: Cedrelopis grevei Baillon et Neobeguea mahafalensis Leroy. Plantes medicinales et phytothérapie 1972, Tome VI, no 4, p. 311-319.

21. Centre de Formation Professionnelle Forestiere' FOFAMPIALA' de Morondava, Octobre 1991, Fiches dendrologiques, Cours N° 5, no page.

22. Diana Ralantonirina: Apercu sur les plantes medicinales dans le sud de Madagascar. Etude faite sur les adultes dans le péromètre de la reserve spéciale de Bexa - Mahafaly (These pour l'obtention du doctorat en medicine). Univesite D'Antanarivo, Faculte de Medecine. (1993).

23. Direction des Eaux et Forêts, Ministère de l'Agriculture et du Développement Rural, 'Recueil Botanique de 200 espèces Forestières' 1996 .

24. Pierre Boiteau, Dictionnaire des Noms Malgaches des Végétaux, Editions Alzieu, Grenoble, 1999, Vol I.

25. Leroy, J.-F. (1958) Contributions à l'étude des forets de Madagascar, I. Les acajous de Madagascar (Khaya et Neobeguea), Journal d'Agronomie Tropicale et de Botanique Appliquée, 5: 593-595.

26. Leroy, J.-F. (1976) Essais de taxonomie syncrétique, 1. Etude sur les Meliaceae de Madagascar, Adansonia, 16(2): 167-203.

27. Centre de Formation Professionnelle Forestière' FOFAMPIALA ' de Morondava, Octobre 1991, Fiches dendrologiques, Cours N° 5.

28. Miyake, Yasuo; Yamamoto, Shin; Hoshino, Wakun; Yoshikai, Kazutaka: Oxidation inhibitor, anti-aging medicine and antiinflammatory agent, and skin make-up charge. Japanese patent application JP2005-213202.

29. D. Naidoo, D. A. Mulholland, M. Randrianarivelojosia, P. H. Coombes: Limonoids and triterpenoids from the seed of Neobegueaea mahafalensis, Biochemical Systematics and Ecology, 2003, 31, 1047-1050.

30. Ansel, H.C., L.V. Allen, Jr, N.G. Popovich: Pharmaceutical Dosage forms and drug delivery systems (seventh edition). Lippincott Williams & Wilkins. (1999) ISBN 0-683-30572-7.

31. Yang, CC, Porter, MP, Penson, DF: Comparison of the international index of erectile function erectile domain scores and nocturnal penile tumescence and rigidity measurements: does one predict the other? BJU Int. 2006 Jul;98(1):105-9; doi:10.1111/j.1464-410X.2006.06246.

32. von Soxhlet, FR: Die gewichtsanalytische Bestimmung des Milchfettes, Polytechnisches J. (Dingler's) 1879, 232, 461.

33. Mansoori, G.A. "Phase Equilibrium of Mixtures Consisting of Molecules with Large Size and Shape Differences (Thermodynamic Modeling of Supercritical Fluid Extraction and Retrograde Condensation, SFE/RC)", GRI Document Number: GRI-88/0360 , 88p, Gas Research Institute, Chicago, IL USA, August 1988.

34. Mansoori, G.A., Schulz, K. and Martinelli, E. "Bioseparation Using Supercritical Fluid Extraction Retrograde Condensation", BIO/TECHNOLOGY, Vol. 6, pp.393-396, 1988.

35. Ruiz-Jimenez J et al., Anal Chim Acta 2004, 502, 75.

36. Ruiz-Jimenez J et al., Anal Chim Acta 2004, 525, 159.

37. Charman SA, Charman WN, Rogge MC, Wilson TD, Dutko FJ, Pouton CW: Self-emulsifying drug delivery systems: formulation and biopharmaceutic evaluation of an investigational lipophilic compound. Pharmaceutical Research, 1992 Jan;9(1):87-93.

38. Randrianarivelojosia, M, Kotsos, MP and Mulholland, DA: A limonoid from Neobeguea mahafalensis. Phytochemistry (1999) 52, 1141-1143.

39. Mulholland, DA and Taylor, DAH: Limonoid extractives from the genera Capuronanthus, Neobeguea and Quivisanthe. (1988) Phytochemistry 27, 1741-1743.

40. Guex, Matthias; Tamm, Christoph. Selective reactions of the tetranortriterpenes busseins A and B. Helvetica Chimica Acta (1985), 68(2), 522-33. CODEN: HCACAV ISSN:0018-019X. CAN 103:160723 AN 1985:560723 CAPLUS.

41. Coombes, PH, DA Mulholland and M Randrianarivelojosia: Phragmalin limonoids from the Madagascan Meliaceae Neobeguea leandreana, Journal of Natural Products, 2003,66, 735-738.

42. Guex, M and Tamm, C: Die busseine C, D, E, F, G, H, J, K, L und M, zehn neue tetratriterpene aus Entandrophramga bussei Harms, Helvetica chimica Acta vol. 67, Fasc. 3 1984, Nr. 99, p. 885-901.

43. Abdelgakeil, SAM, Makanti, M: Antifeeding activity of limonoids from Khaya psenegalesis (Meliaceae). J. Appl. Ent. 127, 236-239 (2003).

44. Boto, A, Betabcor, C, Suárez, E: Hypervalent iodine reagents: Synthesis of a steroidal orthoacetate by a radical reaction. Tetrahedron Letters, vol. 35, no 37., pp. 6933-6936, 1994.

45. Mulholland, SA, Parel, B and Coombes, PH: The chemistry of the meliaeae and ptaeroxulaceae of southern and eastern africa and Madagascar. Curent Organic Chemistry, 2000, 4, 1011-1054.

46. Naidoo, D., D.A. Mulholland, M. Randrianarivelojosia, P.H. Coombes: Limonoids and triterpenoids from the seed of Neobeguea mahafalensis, Biochemical Systematics and Ecology 31 (2003) 1047-1050.

## Claims

**1.** A process for producing an extract from a *Neobeguea spp.* root tissue, the process for producing the extract involving at least one of the steps:

(i) isolating a lipid soluble fraction, and
(ii) isolating a high molecular weight fraction.

**2.** The process according to claim 1 wherein a lipophilic solvent is used at least once, the lipophilic solvent preferably being selected from the group of pentane, n-pentane, 2-methylbutane, 2,2-dimethylpropane, hexane, n-hexane, 2-methylpentane, 3-methylpentane, 2,3-dimethylbutane, 2,2-dimethylbutane, heptane, n-heptane, 2-methylhexane, 3-methylhexane, 2,2-dimethylpentane, 2,3-dimethylpentane, 2,4-dimethylpentane, 3,3-dimethylpentane, 3-ethylpentane, 2,2,3-trimethylbutane, octane, n-octane, 2-methylheptane, 3-methylheptane, 4-methylheptane, 3-ethylhexane, 2,2-dimethylhexane, 2,3-dimethylhexane, 2,4-dimethylhexane, 2,5-dimethylhexane, 3,3-dimethylhexane, 3,4-dimethylhexane, 2-methyl-3-ethylpentane, 3-methyl-3-ethylpentane, 2,2,3-trimethylpentane, 2,2,4-trimethylpentane, isooctane, 2,3,3-trimethylpentane, 2,3,4-trimethylpentane, 2,2,3,3-tetramethylbutane, cyclohexane, benzonitrile, chlorobenzene, diethyl ether, methyl-tert-butyl ether, methylenechloride, dichloromethane, chloroform, carbon tetrachloride, 1,2-dichloroethane, perchloroethylene, trichloroethylene, 1,1,1-trichloroethane, trichloroethene, perchloroethylene, tetrachloroethene, vinylchloride, ethylacetate, methylethylketone, propyl acetate, iso-propyl acetate, butyl lactate, n-butyl lactate, iso-butyl lactate, tert-butyl lactate, sec-butyl lactate, butyl acetate, n-butyl acetate, iso-butyl acetate, tert-butyl acetate, sec-butyl acetate, triacetin, 1,2,3-triacetoxypropane, diacetin, glycerol 1,3-diacetate, glycerol 1,2-diacetate, benzene, toluene, xylene, o-xylene, m-xylene, p-xylene, 2,2,4-trimethylpentane, butanone, 2-butanone, pentan-3-one, pentan-2-one, 3-pentanone, 2-pentanone, cyclopentanone, butan-1-ol, butan-2-ol, n-butanol, 1-butanol, sec-butanol, 2-butanol, isobutanol, 2-methyl-1-propanol, 2-methyl-2-propanol, pentanol, 1-pentanol, 3-methyl-1-butanol, 2-methyl-1-butanol, 2,2-dimethyl-1-propanol, 3-pentanol, 2-pentanol, 3-methyl-2-butanol, 3-methyl-2-butanol, 2-methyl-2-butanol, hexanol, heptanol, octanol, monohydroxylated hydrocarbon(s), diethylene glycol, dimethyl sulfoxide, methyl t-butyl ether, N-methyl-2-pyrrolidinone, nitromethane, tetrahydrofuran, triethyl amine, benzylalcohol, carboxylic acid methyl ester, carboxylic acid ethyl ester, fatty acid methyl ester, vegetable oil, animal oil, triglyceride(s), mineral oil, wood turpentine, petroleum ether, naphtha, hydrocarbon solvent, chlorinated hydrocarbon solvent, fluorinated hydrocarbon solvent, halogenated hydrocarbon solvent, freon, 1-bromo-3-chloropropane, [2-(2-butoxyethoxy)ethyl]acetate, 2-butoxyethyl acetate, cumene, cyclohexanol, cyclohexanone, decahydronaphthalene, n-decane, dibenzyl ether, 1,2-dichlorobenzene, 1,3-dichlorobenzene, 1,4-dichlorobenzene, 1,2-dichloroethane, dimethyl carbonate, diethylene glycol dibutyl ether, diethylene glycol diethyl ether, diethylene glycol mono-n-hexyl ether, diethylene glycol monobutyl ether, diethylene glycol monoethyl ether, diethylene glycol monomethyl ether, ethylbenzene, ethyl formate, 2-ethyl-1-hexanol, dimethyl sulfoxide, 1,1,1,3,3,3-hexafluoro-2-propanol, isoamyl acetate, 2-methylbutyl acetate, 3-methylbutyl acetate, isoamyl butyrate, isobutyl acetate, isopropyl acetate, isopropyl methyl ketone, 1,3,5-trimethylbenzene, (1-methoxy-2-propyl)acetate, methyl acetate, methylcyclohexane, methylcyclohexanol, 5-methyl-3-heptanone, 3-methyltetrahydropyran, 2-methylpentane, 2-methyl-1-butanol, *n*-nonane, nitroethane, propylacetate, 1,2-propylene glycol diacetate, propylene carbonate, tetrahydrofuran, tetrahydrofurfuryl alcohol, 1,2,3,4-tetrahydronaphthalene, triethylene glycol, dimethyl ether, methyl ethyl ether, liquid ethane, liquid ethene, liquid propane, liquid *n*-butane, liquid *iso*-butane, liquid carbon dioxide, liquid trifluoromethane, liquid chlorotrifluoromethane, liquid trichlorofluoromethane, liquid ammonia.

**3.** The process according to any one of claims 1 or 2 for producing extracts from a *Neobeguea spp.* root tissue, the process comprising the steps:

(A)

(i) extracting the *Neobeguea spp.* root tissue with a lipophilic solvent to produce a lipid soluble first extract, and
(ii) optionally extracting the *Neobeguea spp.* root tissue from step (i) with a hydrophilic solvent to produce a second extract,
(iii) optionally combining the extracts from steps (i) and (ii) to produce a united extract; or

(B)

(i) extracting the *Neobeguea spp.* root tissue with a hydrophilic solvent to produce a first extract, and
(ii) extracting the *Neobeguea spp.* root tissue from step (i) with a lipophilic solvent to produce a lipid soluble second extract,
(iii) optionally combining the extracts from steps (i) and (ii) to produce a united extract; or

(C)

(i) extracting a *Neobeguea spp.* root tissue with a hydrophilic solvent to produce a first extract,
(ii) extracting the first extract with a lipophilic solvent to produce a lipid soluble second extract; or

(D)

(i) extracting the *Neobeguea spp.* root tissue with a lipophilic solvent to produce a lipophilic first fraction, and
(ii) exhausting the lipophilic first fraction with a hydrophilic solvent and isolating the materials not being solubilized by the hydrophilic solvent, thereby producing a lipid soluble extract.

**4.** The process according to any one of claims 1 or 2 for producing extracts from a *Neobeguea spp.* root tissue, the process comprising the steps:

(A)

(i) extracting a *Neobeguea spp.* root tissue with water to produce a water soluble fraction, and
(ii) exhausting the water soluble fraction with a lipophilic solvent that is preferably partially miscible with water, and then isolating the water phase to produce a water soluble extract, and

(iii) optionally isolating the high molecular weight fraction from the water soluble extract of step (ii) to produce a high molecular weight fraction, and/or
(iv) optionally extracting either the water soluble extract isolated in step (ii) or the high molecular weight fraction isolated in step (iii) with a lipophilic solvent to produce a lipid soluble fraction; or

(B)

(i) optionally extracting a *Neobeguea spp.* root tissue with a lipophilic solvent, and
(ii) extracting a *Neobeguea spp.* root tissue or the *Neobeguea spp.* root tissue from step (i) with a hydrophilic solvent, to produce a fraction, and
(iii) isolating the high molecular weight fraction from the fraction of step (ii) to produce a high molecular weight fraction, and
(iv) optionally extracting the high molecular weight fraction from step (iii) with a lipophilic solvent to produce a lipid soluble fraction.

**5.** A process as in any one of claims 1 or 4 wherein the high molecular weight fraction is a fraction with molecular weight at least above 900, even more preferably above 1000, more preferably above 1200, even more preferably above 1500, even more preferably above 1800, even more preferably above 2000, even more preferably above 2500, even more preferably above 3000 and most preferably above 5000 daltons;
preferably

(A) wherein the high molecular weight fraction is isolated by chromatography; or
(B) wherein the high molecular weight fraction is isolated using a Sephadex column, most preferably a G-25 column; or
(C) wherein the high molecular weight fraction is isolated by dialysis or ultrafiltration.

**6.** A process (A) according to any one of claims 1 or 2 for producing a fraction having sexual enhancing activity from a *Neobeguea spp.* root tissue, the process comprising the steps:

(i) extracting a *Neobeguea spp.* root tissue with a hydrophilic and/or lipophilic solvent to produce an extract or fraction, and
(ii) applying the extract or fraction of step (i) to a chromatographic column, and
(iii) eluting the bound material in fractions,

and selecting an eluted fraction having sexual enhancing activity, the eluted fraction preferably being lipid soluble; or
a process (B) for producing a fraction having sexual enhancing activity from a *Neobeguea spp.* root tissue, the process comprising the steps:

(i) applying a fraction or extract obtained by any of the processes of claims 1 to 5 to a chromatographic column, and
(ii) eluting the bound material in fractions,

and selecting an eluted fraction having sexual enhancing activity, the eluted fraction preferably being lipid soluble.

**7.** A process (A) for producing a lipid soluble fraction having sexual enhancing activity from a *Neobeguea spp.* root tissue, the process comprising the steps:

(i) applying a fraction or extract obtained by any of the processes of claims 1 to 6 to a polar interaction column, and
(ii) eluting the bound material in fractions,

and selecting an eluted fraction having sexual enhancing activity, the eluted fraction being lipid soluble; or
a process (B) according to any one of claims 1 or 2 for producing a lipid soluble fraction having sexual enhancing activity from a *Neobeguea spp.* root tissue, the process comprising the steps:

(i) extracting a *Neobeguea spp.* root tissue with a hydrophilic solvent and/or lipophilic solvent to produce a first extract, and

(ii) applying the first extract to a polar interaction column, and
(iii) eluting the bound material in fractions,

and selecting an eluted fraction having sexual enhancing activity, the eluted fraction being lipid soluble; preferably
wherein the stationary phase of the polar interaction column in (A) or (B) is selected from the group of silica, alumina, hydroxyapatite, cellulose, vinylalcohol bonded silica, polyamine bonded silica, silanol bonded silica, diol bonded silica, amino bonded silica, anionic bonded silica, amide bonded silica, cationic bonded silica, zwitterionic bonded silica, or any other bonded silica; and/or
wherein the mobile phase in (A) or (B) is comprised by a lipophilic solvent, or a mixture of lipophilic solvents, with optional additive(s),
wherein the lipophilic solvent(s) are preferably selected from the group of pentane, n-pentane, 2-methylbutane, 2,2-dimethylpropane, hexane, n-hexane, 2-methylpentane, 3-methylpentane, 2,3-dimethylbutane, 2,2-dimethylbutane, heptane, n-heptane, 2-methylhexane, 3-methylhexane, 2,2-dimethylpentane, 2,3-dimethylpentane, 2,4-dimethylpentane, 3,3-dimethylpentane, 3-ethylpentane, 2,2,3-trimethylbutane, octane, n-octane, 2-methylheptane, 3-methylheptane, 4-methylheptane, 3-ethylhexane, 2,2-dimethylhexane, 2,3-dimethylhexane, 2,4-dimethylhexane, 2,5-dimethylhexane, 3,3-dimethylhexane, 3,4-dimethylhexane, 2-methyl-3-ethylpentane, 3-methyl-3-ethylpentane, 2,2,3-trimethylpentane, 2,2,4-trimethylpentane, isooctane, 2,3,3-trimethylpentane, 2,3,4-trimethylpentane, 2,2,3,3-tetramethylbutane, cyclohexane, benzonitrile, chlorobenzene, diethyl ether, methyl-tert-butyl ether, methylenechloride, dichloromethane, chloroform, carbon tetrachloride, 1,2-dichloroethane, perchloroethylene, trichloroethylene, 1,1,1-trichloroethane, trichloroethene, perchloroethylene, tetrachloroethene, vinylchloride, ethylacetate, methylethylketone, propyl acetate, iso-propyl acetate, butyl lactate, n-butyl lactate, iso-butyl lactate, tert-butyl lactate, sec-butyl lactate, butyl acetate, n-butyl acetate, iso-butyl acetate, tert-butyl acetate, sec-butyl acetate, triacetin, 1,2,3-triacetoxypropane, diacetin, glycerol 1,3-diacetate, glycerol 1,2-diacetate, benzene, toluene, xylene, o-xylene, m-xylene, p-xylene, 2,2,4-trimethylpentane, butanone, 2-butanone, pentan-3-one, pentan-2-one, 3-pentanone, 2-pentanone, cyclopentanone, butan-1-ol, butan-2-ol, n-butanol, 1-butanol, sec-butanol, 2-butanol, isobutanol, 2-metyl-1-propanol, 2-metyl-2-propanol, pentanol, 1-pentanol, 3-methyl-1-butanol, 2-methyl-1-butanol, 2,2-dimethyl-1-propanol, 3-pentanol, 2-pentanol, 3-methyl-2-butanol, 3-methyl-2-butanol, 2-methyl-2-butanol, hexanol, heptanol, octanol, monohydroxylated hydrocarbon(s), diethylene glycol, dimethyl sulfoxide, methyl t-butyl ether, N-methyl-2-pyrrolidinone, nitromethane, tetrahydrofuran, triethyl amine, benzylalcohol, carboxylic acid methyl ester, carboxylic acid ethyl ester, fatty acid methyl ester, vegetable oil, animal oil, triglyceride(s), mineral oil, wood turpentine, petroleum ether, naphtha, hydrocarbon solvent, chlorinated hydrocarbon solvent, fluorinated hydrocarbon solvent, halogenated hydrocarbon solvent, freon, 1-bromo-3-chloropropane, [2-(2-butoxyethoxy)ethyl]acetate, 2-butoxyethyl acetate, cumene, cyclohexanol, cyclohexanone, decahydronaphthalene, n-decane, dibenzyl ether, 1,2-dichlorobenzene, 1,3-dichlorobenzene, 1,4-dichlorobenzene, 1,2-dichloroethane, dimethyl carbonate, diethylene glycol dibutyl ether, diethylene glycol diethyl ether, diethylene glycol mono-n-hexyl ether, diethylene glycol monobutyl ether, diethylene glycol monoethyl ether, diethylene glycol monomethyl ether, ethylbenzene, ethyl formate, 2-ethyl-1-hexanol, dimethyl sulfoxide, 1,1,1,3,3,3-hexafluoro-2-propanol, isoamyl acetate, 2-methylbutyl acetate, 3-methylbutyl acetate, isoamyl butyrate, isobutyl acetate, isopropyl acetate, isopropyl methyl ketone, 1,3,5-trimethylbenzene, (1-methoxy-2-propyl)acetate, methyl acetate, methylcyclohexane, methylcyclohexanol, 5-methyl-3-heptanone, 3-methyltetrahydropyran, 2-methylpentane, 2-methyl-1-butanol, *n*-nonane, nitroethane, propylacetate, 1,2-propylene glycol diacetate, propylene carbonate, tetrahydrofuran, tetrahydrofurfuryl alcohol, 1,2,3,4-tetrahydronaphthalene, triethylene glycol, dimethyl ether, methyl ethyl ether.

**8.** A process (A) for producing a lipid soluble fraction having sexual enhancing activity from a *Neobeguea spp.* root tissue, the process comprising the steps:

(i) applying a fraction or extract obtained by any of the processes of claims 1 to 7 to a hydrophobic interaction column, and
(ii) eluting the bound material in fractions,

and selecting an eluted fraction having sexual enhancing activity, the elute fraction being lipid soluble; or
a process (B) according to any one of claims 1 or 2 for producing a lipid soluble fraction having sexual enhancing activity from a *Neobeguea spp.* root tissue, the process comprising the steps:

(i) extracting a *Neobeguea spp.* root tissue with a hydrophilic solvent and/or lipophilic solvent to produce a first extract, and
(iii) applying the first extract to a hydrophobic interaction column,

(iv) eluting the bound material in fractions,

and selecting an eluted fraction having sexual enhancing activity, the eluted fraction being lipid soluble; or
a process (C) according to any one of claims 1 or 2 for producing a lipid soluble fraction having sexual enhancing activity from a *Neobeguea spp.* root tissue, the process comprising the steps:

(i) optionally extracting a *Neobeguea spp.* root tissue with a hydrophilic solvent to produce a first extract,
(ii) extracting a *Neobeguea spp.* root tissue or the first extract of step (i) with a lipophilic solvent to produce a second extract,
(iii) applying the second extract from step (ii) to a hydrophobic interaction column,
(iv) eluting the bound material in fractions,

and selecting an eluted fraction having sexual enhancing activity, the eluted fraction being lipid soluble;
preferably wherein the hydrophobic interaction column in (A), (B) or (C) is derivatized with $C_{4-22}$ groups, preferably $C_{18}$ groups.

**9.** A process according to any one of claims 3 to 8 wherein at least one of the hydrophilic solvent(s) used is selected from the group of methanol, ethanol, acetonitrile, propionitrile, propanol, propan-1-ol, propan-2-ol, dimethyl sulfoxide, formamide, dimethylformamide, acetone, tetrahydrofurane, glycol, glycerol, dioxane, 1,4-dioxane, formic acid, acetic acid, propionic acid, butyric acid, 2-methylpropanoic acid, 3-oxobutanamide, N,N-diethylacetamide, N,N-diethyl acetoacetamide, propylene glycol, methylsulfonylmethane, ethanol amine, *tert*-butyl alcohol, diethylene glycol, dimethyl ether, 1,2-dimethoxy-ethane, ethylene glycol, hexamethylphosphoramide, hexamethylphosphorous triamide, pyridine, 2-methyltetrahydrofuran, 3-methyltetrahydropyran, 2-methylpyridine, 1,3-propanediol, sulfolane, triethylene glycol, tetraethylene glycol, tetrahydrofurfuryl alcohol, triethanolamine, triethyl phosphate, triethylene glycol, triethylene glycol dimethyl ether, triethylene glycol monomethyl ether, N-methylpyrrolidone, liquid carbon dioxide; and/or preferably wherein at least one of the hydrophilic solvent(s) used is water.

**10.** A process

(A) as claimed in any one of claims 1 to 9 wherein the extract or fraction has sexual enhancing activity; or
(B) as claimed in any one of claims 1 to 9 or (A) above wherein the extract or fraction contains at least 0.05 %, more preferably at least 0.1 %, even more preferably at least 0.15 %, even more preferably at least 0.2 %, and even more preferably at least 0.25 %, and most preferably at least 0.3 % of a chemical as defined in claim 16; or
(C) as claimed in any one of claims 1 to 9 or (A) or (B) above wherein the lipid soluble fraction or extract is entirely soluble with an amount of at least 1 mg/ml, more preferably at least 2 mg/ml, even more preferably at least 4 mg/ml, even more preferably at least 8 mg/ml, even more preferably at least 12 mg/ml, even more preferably at least 16 mg/ml, and most preferably at least 18 mg/ml in sun flower oil and/or in octan-1-ol at the temperature 20°C; or
(D) as claimed in any one of claims 1 to 9 or (A) - (C) above wherein one or more of *Neobeguea spp.* seeds, branches, trunk, leaves or fruit are used instead of the root tissue.

**11.** A process for making a formulation, comprising combining an extract or fraction obtained by or obtainable by a process as claimed in any one of claims 1 to 10 with any one of a pharmaceutically acceptable filler, carrier, excipient, dissolvant, diluent, lubricant, glidant, antioxidant and/or additive, preferably those being selected from the group of pharmaceutically acceptable oil (such as peanut oil, corn oil, sesame oil, cotton oil, olive oil, soybean oil), triglyceride, diglyceride, cocoa butter, coconut fat, hydrogenated fat, hydrogenated animal oil, hydrogenated vegetable oil, solid triglycerides, solid fat, malleable fat, Fattibase, Wecobee bases, Witespol bases, cetyl ester wax, beeswax, glycerol, polysorbate 80, polyethylene glycol, propylene glycol, isopropyl alcohol, magnesium stearate, stearic acid, talc, silicon dioxide, lauryl sulphate, sodium starch glycolate, detergent, oil detergent mixture, oil surfactant mixture, emulsifiable oil, self-emulsifying system, Neobee M5, tri caprylic/capric triglyceride ester, Miglyol 810, propyleneglycol dicaprylate, Sefsol 228, ethoxylated plant fats, ethanol, polyvinyl pyrrolidone, Tween, polyoxyethylenesorbitan monolaurate, triacetin, diacetin, ascorbic acid, ascorbyl palmitate, butylated hydroxyanisole, butylated hydroxytoluene, hypophosphorous acid, monothioglycerol, propyl gallate, sodium ascorbate, sodium bisulphite, sodium formaldehyde sulfoxylate, sodium meta bisulphite, butylparaben, ethyl paraben, benzoic acid, propylparaben, sodium benzoate, sodium propionate, benzalkonium chloride, benzethonium chloride, benzyl alcohol, cetylpyridinium chloride, chlorobutanol, phenol, phenylethyl alchol, phenylmercuric nitrate, thiomersal, lactose, microcrystalline cellulose, starch, powdered sucrose, calcium phosphate, acacia, alginic acid, carboxymethylcellulose sodium, compressible sugar, ethylcellulose, gelatine, liquid glucose, methylcellulose, povidone, pregelatinized starch, crosscarmellose,

crosspovidone, cation exchange resin, silica, colloidal silica, cornstarch, calcium stearate, mineral oil, anis oil, cinnamon oil, cocoa, menthol, orange oil, peppermint oil, vanillin, ginger, colorant.

**12.** A process as claimed in any one of the previous claims wherein the content of a chemical compound as defined in claim 16 is assayed;
preferably wherein the content of the chemical substance is assayed by use of chromatography and/or mass spectrometry.

**13.** A composition

(A) comprising:

(i) a lipid soluble extract from a *Neobeguea spp.* root tissue and/or
(ii) a high molecular weight fraction from a *Neobeguea spp.* root tissue; or

(B) comprising an extract or fraction obtained by or obtainable by the process of any one of claims 1 to 12,

preferably wherein composition (A) ort (B) additionally comprises any one of a pharmaceutically acceptable filler, carrier, excipient, dissolvant, diluent, lubricant, glidant, antioxidant and/or additive, preferably those being selected from the group of pharmaceutically acceptable oil (such as peanut oil, corn oil, sesame oil, cotton oil, olive oil, soybean oil), triglyceride, diglyceride, cocoa butter, coconut fat, hydrogenated fat, hydrogenated animal oil, hydrogenated vegetable oil, solid triglycerides, solid fat, malleable fat, Fattibase, Wecobee bases, Witespol bases, cetyl ester wax, beeswax, glycerol, polysorbate 80, polyethylene glycol, propylene glycol, isopropyl alcohol, magnesium stearate, stearic acid, talc, silicon dioxide, lauryl sulphate, sodium starch glycolate, detergent, oil detergent mixture, oil surfactant mixture, emulsifiable oil, self-emulsifying system, Neobee M5, tri caprylic/capric triglyceride ester, Miglyol 810, propyleneglycol dicaprylate, Sefsol 228, ethoxylated plant fats, ethanol, polyvinyl pyrrolidone, Tween, polyoxyethylenesorbitan monolaurate, triacetin, diacetin, ascorbic acid, ascorbyl palmitate, butylated hydroxyanisole, butylated hydroxytoluene, hypophosphorous acid, monothioglycerol, propyl gallate, sodium ascorbate, sodium bisulphite, sodium formaldehyde sulfoxylate, sodium meta bisulphite, butylparaben, ethyl paraben, benzoic acid, propylparaben, sodium benzoate, sodium propionate, benzalkonium chloride, benzethonium chloride, benzyl alcohol, cetylpyridinium chloride, chlorobutanol, phenol, phenylethyl alchol, phenylmercuric nitrate, thiomersal, lactose, microcrystalline cellulose, starch, powdered sucrose, calcium phosphate, acacia, alginic acid, carboxymethylcellulose sodium, compressible sugar, ethylcellulose, gelatine, liquid glucose, methylcellulose, povidone, pregelatinized starch, crosscarmellose, crosspovidone, cation exchange resin, silica, colloidal silica, cornstarch, calcium stearate, mineral oil, anis oil, cinnamon oil, cocoa, menthol, orange oil, peppermint oil, vanillin, ginger, colorant.

**14.** A composition comprising a formulation obtained by or obtainable by the process of claim 11.

**15.** A composition as claimed in any one of claims 13 to 14 comprised in the form of any one of an oral administrable, parenteral administrable, systemically administrable, capsule, tablet, powder, granulate, suppository, insert, lozenge, troche, buccal tablet, sublingual tablet, compressed tablet, multiple compressed tablet, molded tablet, chewable tablet, effervescent tablet, tablet triturate, sugar coated tablet, film-coated tablet, gelatine-coated tablet, enteric-coated tablet, dispensing tablet, hypodermic tablet, extended release tablet, instant disintegrating tablet, immediate release tablet, dermal formulation, ointment, cream, gel, transdermal formulations, solutions, tincture, injectable, parenteral, implant, formulation for urethral administration, formulations for topical administration, ophthalmic solution, vaginal formulation, inhalant, disperse system, emulsion, multitablet system, microencapsulated drug system, osmotic pump, subdermal implant, ocular system, parenteral system, vaginal system, coated bead system, granule, microsphere, modified-release system, extended-release system, delayed-release system, repeat action system, targeted release system, ion-exchange resin system, slowly eroding and/or hydrophilic matrix system, inert plastic matrix embedded system, ointment, cream, gel, solution, water solution, tincture, oil solution, pill or infusion package, or tea bag.

**16.** A chemical compound having the general formula of structure III:

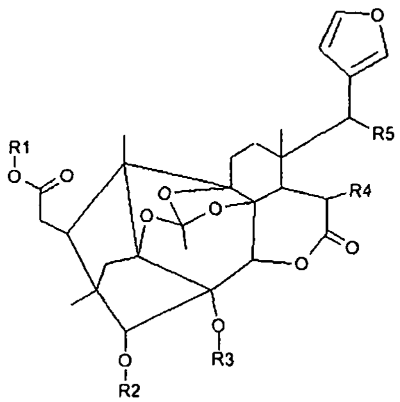

wherein R1 is a substituent having from one to 30 atoms of any type(s), more preferably one to 16 atoms, more preferably one to 12 atoms and most preferably one to 10 atoms, with hydrogen, oxygen, carbon, sulphur, nitrogen, phosphorous and halogen atoms being preferred; the R1 substituent being a hydrogen and/or linear, branched and/or cyclic structure; the R1 substituent preferably comprising of from zero to 10 heavy atoms, even more preferably zero to 6 heavy atoms, even more preferably zero to 4 heavy atoms, even more preferably zero to 3 heavy atoms, and most preferably zero to 2 heavy atoms; most preferably the substituent being comprised of any one of hydrogen, methyl, ethyl, propyl, isopropyl, butyl, isobutyl, cyclopropyl, cyclopropenyl, cyclobutyl, cyclopentyl, cyclohexyl, alkyl, cyclic alkyl, halogenated alkyl, halogenated cyclic alkyl, propenyl, halogenated propenyl, alkenyl, halogenated alkenyl, halogenated cyclic alkenyl, alkynyl, halogenated alkynyl, aryl, halogenated aryl, with methyl being most preferred,

and wherein R2 is a substituent having from one to 30 atoms of any type(s), more preferably one to 20 atoms, and most preferably one to 16 atoms, with hydrogen, oxygen, carbon, sulphur, nitrogen, phosphorous and halogen being preferred; the R2 substituent being a hydrogen or a linear, branched and/or cyclic structure; the R2 substituent preferably comprising of from zero to 10 heavy atoms, even more preferably zero to 8 heavy atoms, even more preferably zero to 6 heavy atoms, even more preferably zero to 5 heavy atoms; most preferably the substituent being composed of any one of hydrogen, methyl, ethyl, propyl, isopropyl, cyclopropyl, cyclopropenyl, butyl, isobutyl, cyclobutyl, alkyl, halogenated alkyl, cyclic alkyl, halogenated cyclic alkyl, propenyl, halogenopropenyl, alkenyl, halogenated alkenyl, cyclic alkenyl, halogenated cyclic alkenyl, alkynyl, halogenated alkynyl, aryl, halogenated aryl, acetyl, halogenoacetyl, propionyl, halogenopropionyl, butyryl, halogenobutyryl, isobutyryl, halogenoisobutyryl, alkyryl, halogenated alkyryl, cyclic alkyryl, halogenated cyclic alkyryl, benzoyl, aryryl, with a hydrogen or acetyl group or isobutyryl group being most preferred,

and wherein R3 is a substituent having from one to 30 atoms of any type(s), more preferably one to 20 atoms, and most preferably one to 16 atoms, with hydrogen, oxygen, carbon, sulphur, nitrogen, phosphorous and halogen being preferred; the R3 substituent being a hydrogen or a linear, branched and/or cyclic structure; the R3 substituent preferably comprising of from zero to 10 heavy atoms, even more preferably zero to 8 heavy atoms, even more preferably zero to 6 heavy atoms, even more preferably zero to 5 heavy atoms; most preferably the substituent being composed of any one of hydrogen, methyl, ethyl, propyl, isopropyl, cyclopropyl, cyclopropenyl, butyl, isobutyl, cyclobutyl, alkyl, halogenated alkyl, cyclic alkyl, halogenated cyclic alkyl, propenyl, halogenopropenyl, alkenyl, halogenated alkenyl, cyclic alkenyl, halogenated cyclic alkenyl, alkynyl, halogenated alkynyl, aryl, halogenated aryl, acetyl, halogenoacetyl, propionyl, halogenopropionyl, butyryl, halogenobutyryl, isobutyryl, halogenoisobutyryl, alkyryl, halogenated alkyryl, cyclic alkyryl, halogenated cyclic alkyryl, benzoyl, aryryl, with a hydrogen or acetyl group or isobutyryl group being most preferred,

and wherein R4 is a substituent connected by single or double bond having from one to 32 atoms, more preferably one to 18 atoms, more preferably one to 15 atoms more preferably one to 12 atoms, and most preferably one to 9 atoms with hydrogen, oxygen, carbon, sulphur, nitrogen, phosphorous and halogen atoms being preferred; the R4 substituent preferably being a hydrogen or a linear or branched and/or cyclic structure; the R4 substituent preferably comprising between 0 to 12 heavy atoms, even more preferably between 0 to 11 heavy atoms, even more preferably between 0 to 10 heavy atoms, even more preferably between 0 to 9 heavy atoms, even more preferably between 0 to 8 heavy atoms, even more preferably between 0 to 7 heavy atoms, even more preferably between 0 to 6 heavy atoms, and most preferably between 0 to 5 heavy atoms; most preferably the substituent being comprised of any of hydrogen, halogeno, oxo, hydroxy, methoxy, ethoxy, propoxy, isopropoxy, butoxy, isobutoxy, alkoxy, halogenated alkoxy, ethenyloxy, propenyloxy, alkenyloxy, halogenated alkenyloxy, methyl, ethyl, propyl, isopropyl, butyl, isobutyl, alkyl, halogenated alkyl, hydroxymethyl, hydroxyethyl, hydroxypropyl, hydroxyispropyl, hydroxybutyl, hydroxyisobutyl, hydroxyalkyl, halogenenated alkyl, methylene, ethenyl, propenyl, isopropenyl, butenyl, isobutenyl, alkenyl, halogenated alkenyl, acetyl, halogenoacetyl, propionyl, butyryl, isobutyryl, alkyryl, halogenated alkyryl, acetyloxy, halogenoacetyloxy, propionyloxy, isopropionyloxy, butyryloxy, isobutyryloxy, alkyryloxy, halogenated alkyryloxy, 2-oxy-2-methyl-ethyl, hydroxy-oxomethyl, 2-hydroxy-2-oxoethyl, 3-hydroxy-3-oxopropionyl, methoxy-oxomethyl, ethoxy-oxomethyl, propoxy-oxomethyl, isopropoxy-oxomethyl, butoxy-oxomethyl, isobutoxy-oxomethyl, alkoxy-oxomethyl, 2-methoxy-2-oxoethyl, 2-ethoxy-2-oxoethyl, 2-propoxy-2-oxoethyl, 2-isopropoxy-2-oxoethyl, 2-butoxy-2-oxoethyl, 2-isobutoxy-2-oxoethyl, 2-alkoxy-2-oxoethyl, hydroxymethylene, 1-hydroxyethylidene, 1-hydroxypropylidene, 1-hydroxy-2-methylpropylidene, 1-acetyloxy-2-methylpropylidene, 1-halogenoacetyloxy-2-methylpropylidene, 1-alkyryloxy-2-methylpropylidene, 1-halogenoalkyryloxy-2-methylpropylidene, with hydrogen or isobutyryl or 1-hydroxy-2-methylpropylidene being most preferred,

and wherein R5 is a substituent connected by single or double bond having from one to 32 atoms, more preferably one to 18 atoms, more preferably one to 15 atoms, more preferably one to 12, more preferably or one to 10 atoms, more preferably one to 8 atoms and most preferably one to 7 atoms, with hydrogen, oxygen, carbon, sulphur, nitrogen, phosphorous and halogen atoms being preferred; the R5 substituent preferably being a hydrogen or an oxygen or a linear or branched and/or cyclic structure; the R5 substituent preferably comprising between 0 to 12 heavy atoms, even more preferably between 0 to 11 heavy atoms, even more preferably between 0 to 10 heavy atoms, even more preferably between 0 to 9 heavy atoms, even more preferably between 0 to 8 heavy atoms, even more preferably between 0 to 7 heavy atoms, even more preferably between 0 to 6 heavy atoms, more preferably between 1 to 5 heavy atoms and most preferably between 1 to 4 heavy atoms; most preferably the substituent being comprised of any of hydrogen, halogeno, oxo, hydroxy, methoxy, ethoxy, propoxy, butoxy, isobutoxy, alkoxy, halogenated alkoxy, ethenyloxy, propenyloxy, alkenyloxy, halogenated alkyloxy, methyl, ethyl, propyl, isopropyl, butyl, isobutyl, alkyl, halogenated alkyl, hydroxymethyl, hydroxyethyl, hydroxypropyl, hydroxyisopropyl, hydroxybutyl, hydroxyisobutyl, hydroxyalkyl, methylene, ethenyl, propenyl, isopropenyl, butenyl, isobutenyl, alkenyl, halogenated alkenyl, acetyl, halogenoacetyl, propionyl, isopropionyl, butyryl, isobutyryl, alkyryl, halogenated alkyryl, acetyloxy, halogenoacetyloxy, propionyloxy, halogenopropionyloxy, butyryloxy, halogenobutyryloxy, isobutyryloxy, halogenoisobutyryloxy, alkyryloxy, halogenated alkyryloxy, 2-oxy-2-methyl-ethyl, hydroxy-oxomethyl, 2-hydroxy-2-oxoethyl, 3-hydroxy-3-oxopropionyl, methoxy-oxomethyl, ethoxy-oxomethyl, propoxy-oxomethyl, isopropoxy-oxomethyl, butoxy-oxomethyl, isobutoxy-oxomethyl, alkoxy-oxomethyl, 2-methoxy-2-oxoethyl,2-ethoxy-2-oxoethyl, 2-propoxy-2-oxoethyl, 2-isopropoxy-2-oxoethyl, 2-butoxy-2-oxoethyl, 2-isobutoxy-2-oxoethyl, 2-alkoxy-2-oxoethyl, hydroxymethylene, 1-hydroxyethylidene, 1-hydroxypropylidene, 1-hydroxy-2-methylpropylidene, with oxo and acetyloxy being preferred,

or a pharmaceutically acceptable salt thereof,

and which is optionally capable of eliciting a sexual enhancing effect; preferably wherein the chemical compound has the general formula of structure III:

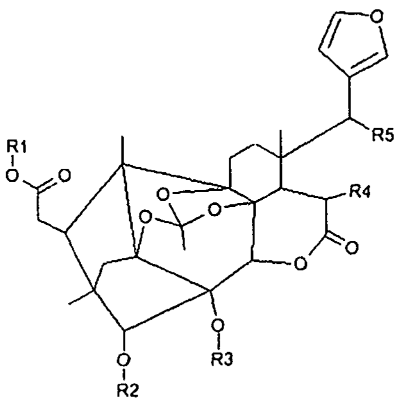

wherein R1 is any one of hydrogen, methyl or ethyl, with methyl being most preferred,

and wherein R2 is any one of hydrogen, methyl, ethyl, acetyl, halogenoacetyl, propionyl, butyryl, isobutyryl, with a hydrogen or acetyl group or isobutyryl group being most preferred,

and wherein R3 is any one of hydrogen, methyl, ethyl, acetyl, halogenoacetyl, propionyl, butyryl, isobutyryl, with a hydrogen or acetyl group or isobutyryl group being most preferred,

and wherein R4 is any one of hydrogen, isobutyryl, 1-hydroxy-2-methylpropylidene, 1-methoxy-2-methylpropylidene, 1-acetyloxy-2-methylpropylidene, 1-halogenoacetyloxy-2-methylpropylidene, with hydrogen or isobutyryl or 1-hydroxy-2-methylpropylidene being most preferred,

and wherein R5 is any one of oxo, hydroxy, methoxy, ethoxy, acetyloxy, halogenoacetyloxy, propionyloxy,

butyryloxy, isobutyryloxy, with oxo and acetyloxy being preferred,
or a pharmaceutically acceptable salt thereof,
and which chemical substance (chemical compound) is optionally capable of eliciting a sexual enhancing effect;
and more preferably wherein the compound has any one of the structures of

R306

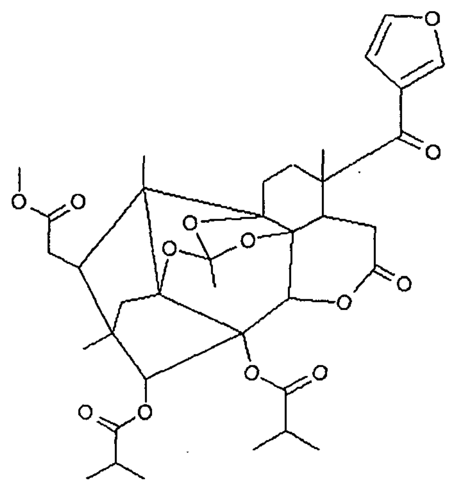

,

R310A

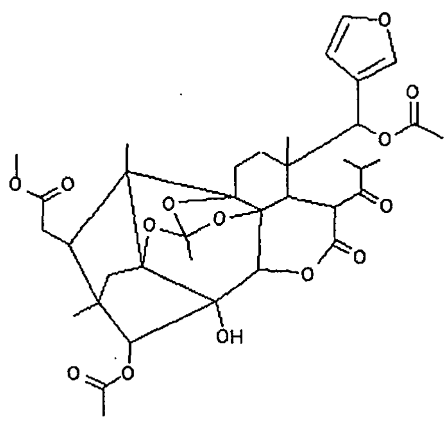

,

R310B

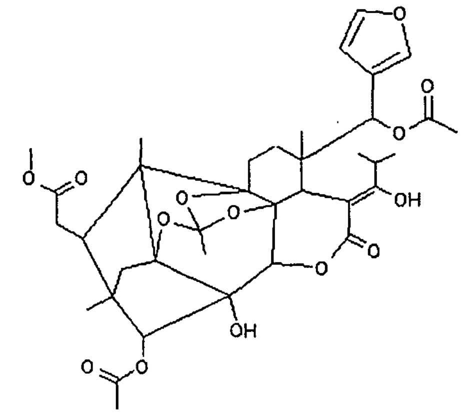

,

R310B1

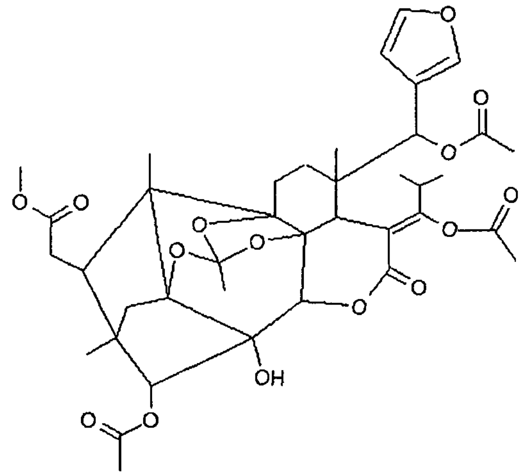

,

R310B2

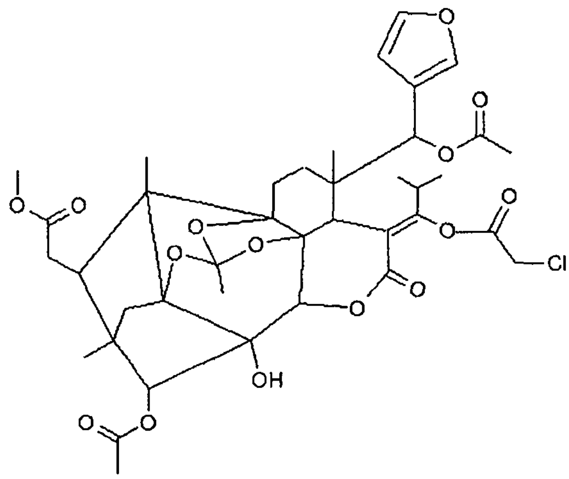

,

R310A3

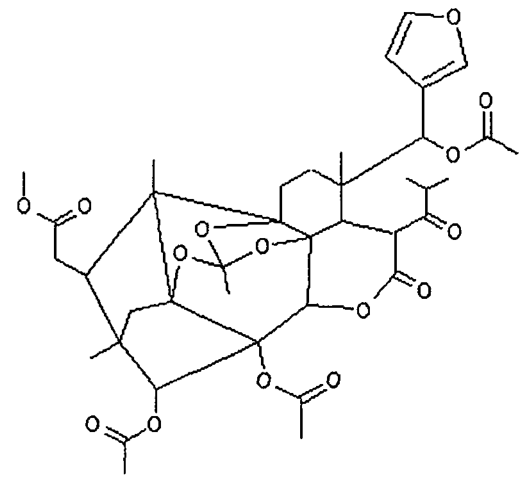

,

R310B3

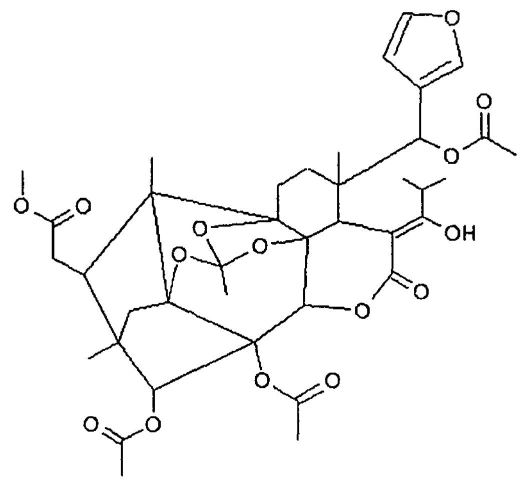

,

R310A4

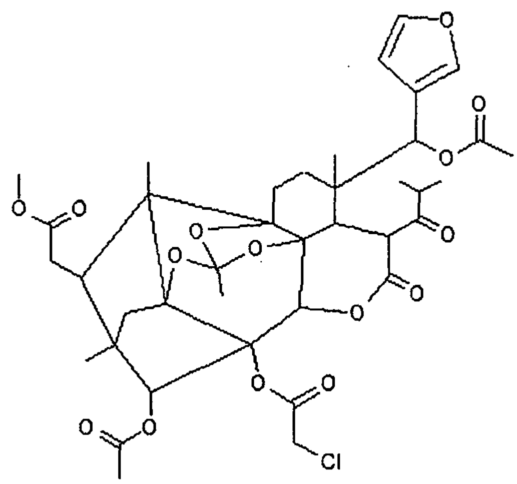

,

R310B4

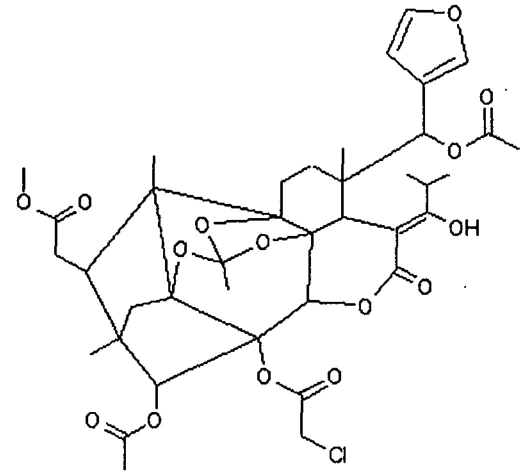

,

R310A5

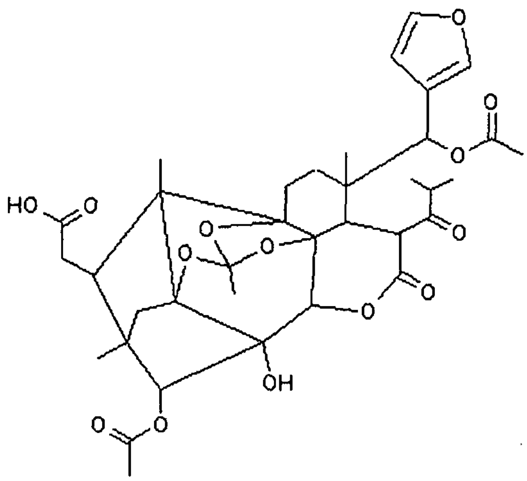

,

R310B5

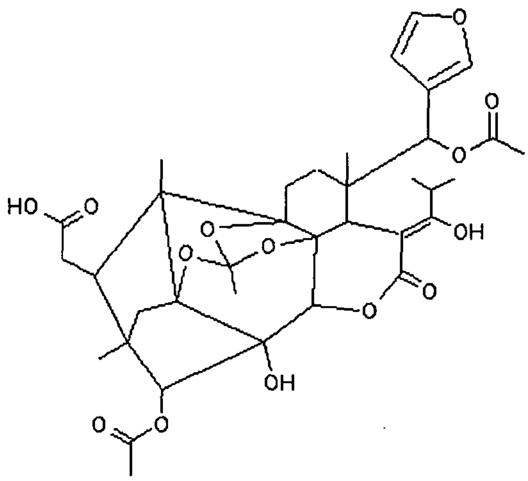

,

R310B6

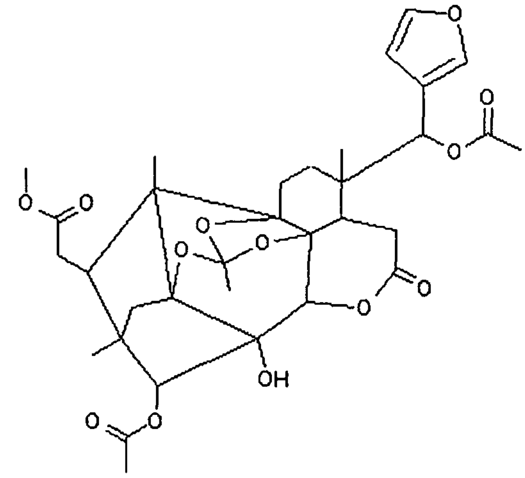

,

R310B7

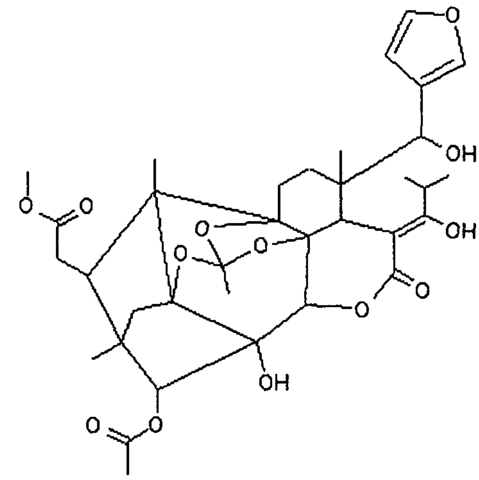

,

R310B8

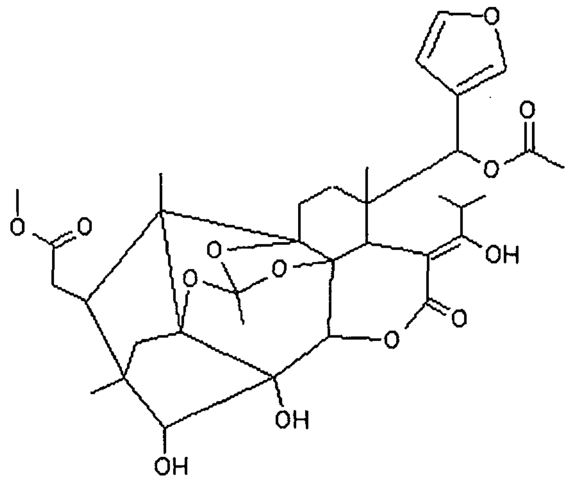

,

R310B9

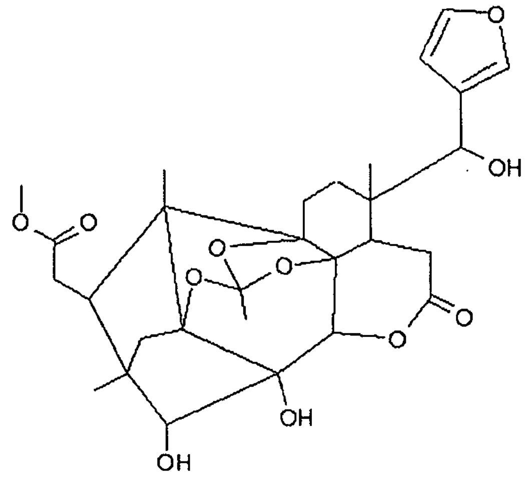

,

R306AB

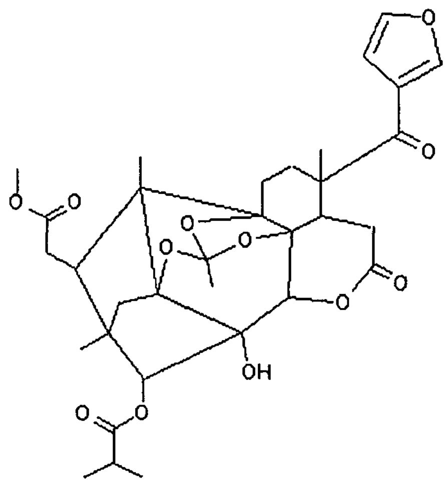

,

R306BA

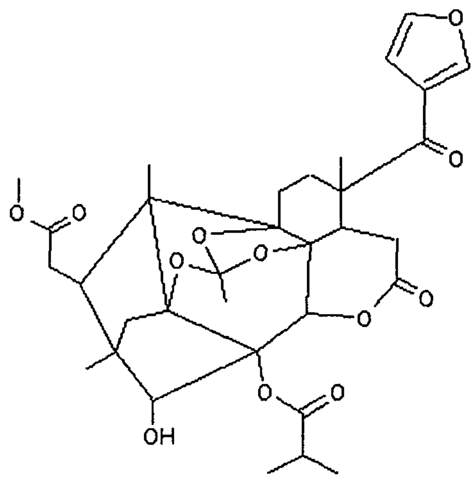

,

R306D

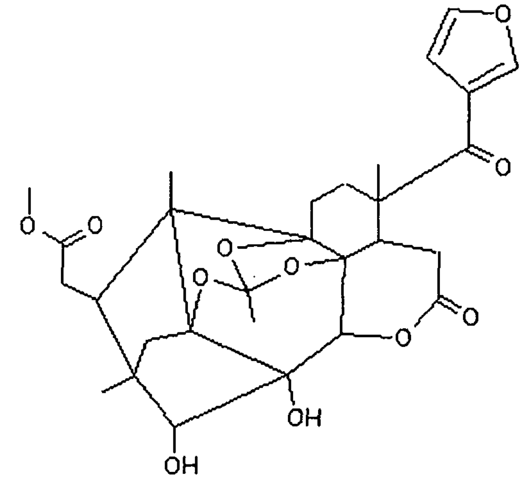

,

R306E

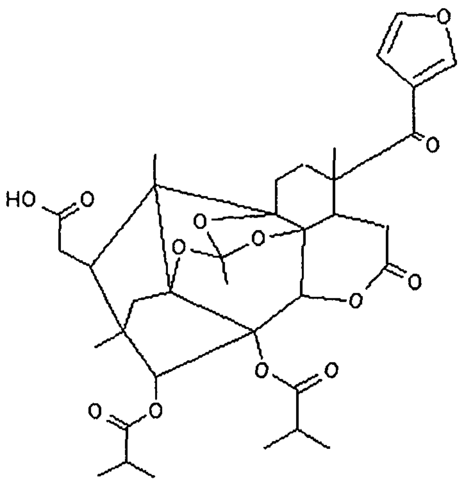

,

R306F

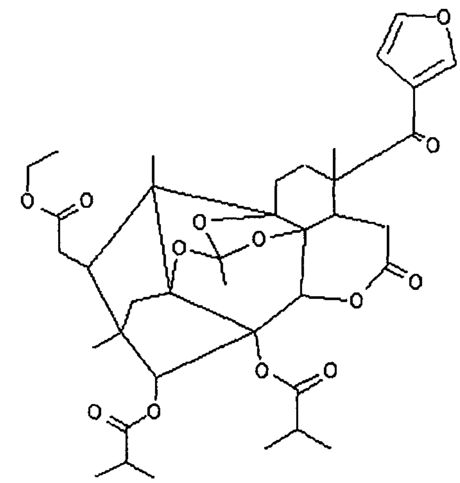

R310B10

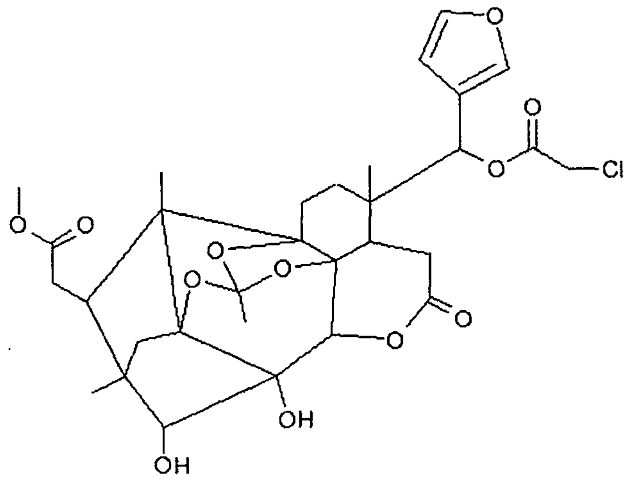

,

R310B11 (R306C)

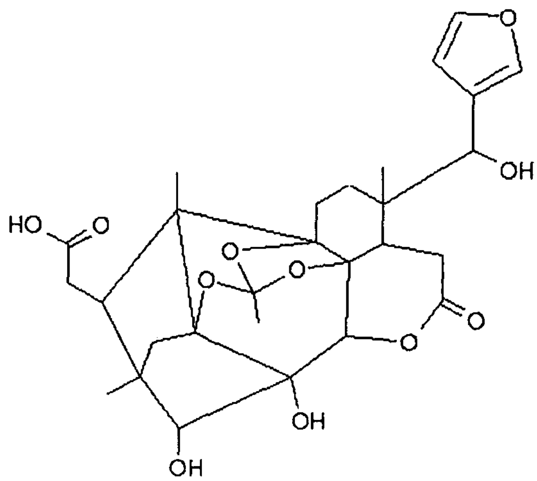

,

R310B12

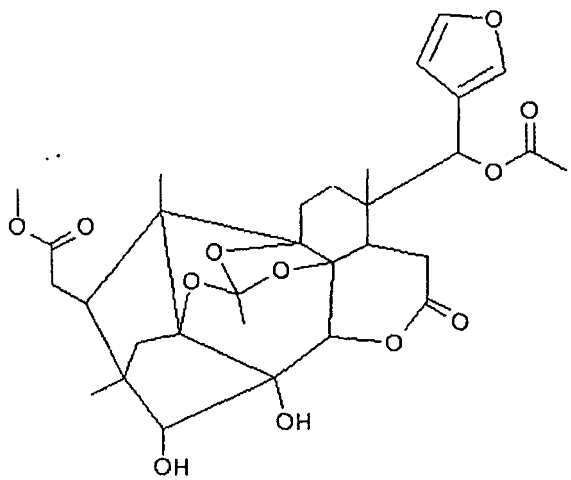

,

R310B13

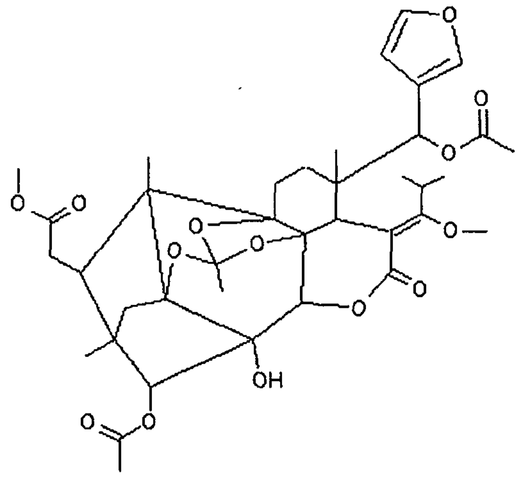

,

R310B14

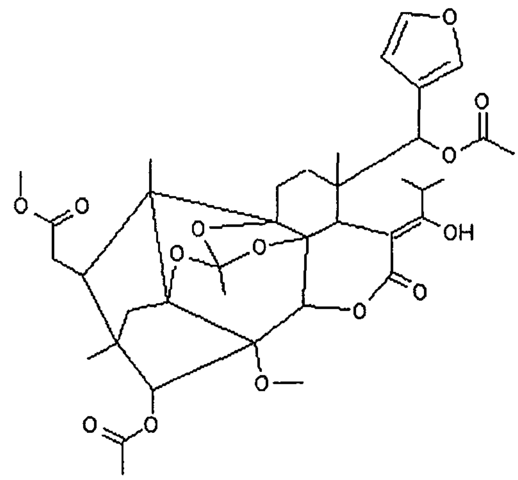

,

R310B15

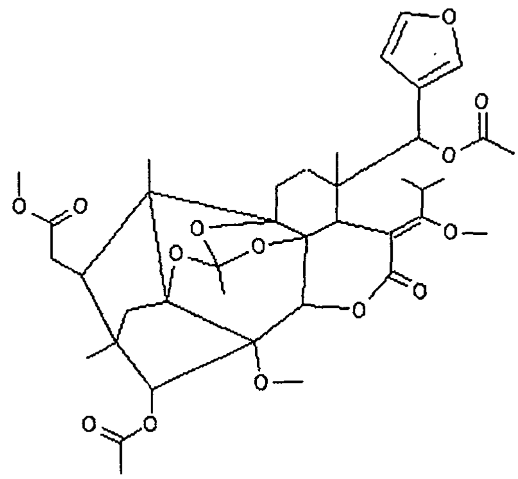

,

R310B16

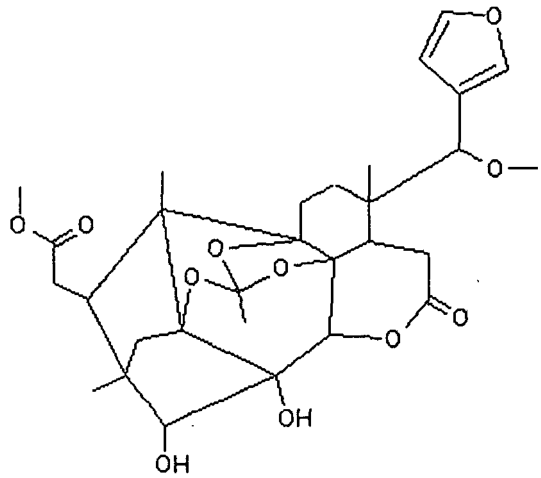

,

R310B17

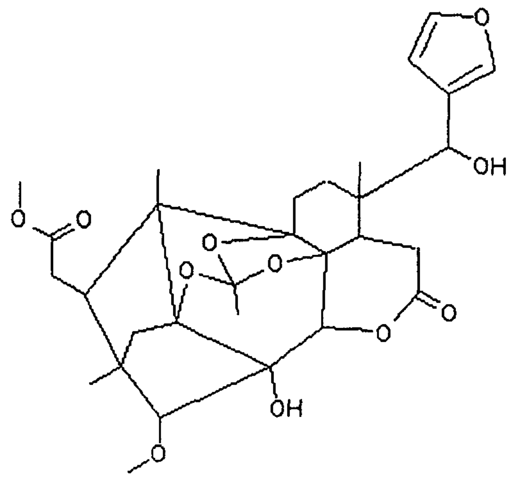

,

R310B18

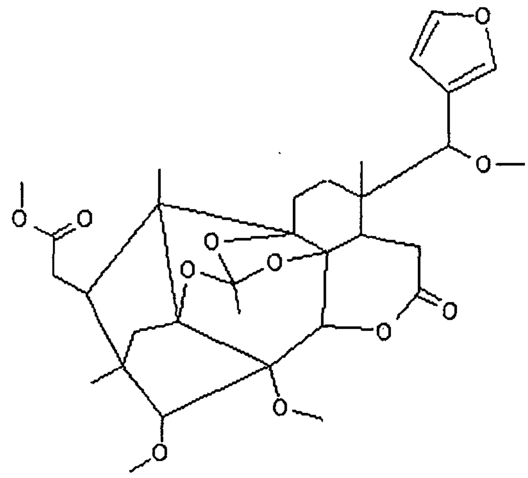

,

or a pharmaceutically acceptable salt thereof,
and which is optionally capable of eliciting a sexual enhancing effect.

**17.** A chemical compound

(A) according to claim 16 having the summary formula $C_{37}H_{46}O_{13}$ or the summary formula $C_{37}H_{46}O_{14}$, and which is optionally having sexual enhancing effect; or
(B) comprising a glycone of the chemical compound according to any of claim 16 or (A) above, and which is optionally having sexual enhancing effect; or
(C) according to any one of claim 16 or (A) - (C) above obtained from or being obtainable from *Neobeguea spp.* plant tissue, and which is optionally having sexual enhancing effect; or
(D) according to any of claim 16 or (A) - (C) above which sexual enhancing activity amounts to at least 0.1 U/mg, even more preferably to at least 0.3 U/mg, even more preferably to at least 1 U/mg, even more preferably to at least 3 U/mg and most preferably to at least 10 U/mg, the unit activity preferably being estimated as $U_{mnt}$; or
(E) comprising a pro-drug of the compound according to any of claim 16 or (A) - (D) above; or
(F) according to any one of claim 16 or (A) - (E) above of obtained from or being obtainable from a natural source, the natural source preferably being a species belonging to the Meliaceae family; or
(G) according to any one of claim 16 or (A) - (F) above obtained by or being obtainable by chemical synthesis or chemical semi-synthesis; or
(H) according to any one of claim 16 or (A) - (G) above which is synthesized starting from a raw-material originating from a natural source, the natural source preferably being a species belonging to the Meliaceae family; or
(I) according to of any one of claim 16 or (A) - (H) above obtained by chemical synthesis or chemical semi-synthesis, by a process that at least in part involves hydrolysis and/or esterification and/or alkylation; or
(J) according to any one of claim 16 or (A) - (I) above being obtained using a raw material comprising any one of a limonoid, phragmalin, neobeguin, leandreanin A, leandreanin B, leandreanin C, pseudrelone $A_2$, bussein A, B, C, D, E, F, G, H, J, K, L, M, the compound optionally having a sexual enhancing effect; or
(K) according to any one of claim 16 or (A) - (J) above in radioactive form.

**18.** A pharmaceutical composition

(A) comprising the chemical compound of any one of claims 16 to 17, optionally together with one or more pharmaceutically acceptable adjuvants, carriers, dissolvants or diluents; or
(B) comprising the chemical compound of any one of claims 16 to 17 in pharmaceutically acceptable oil, fat and/or triglyceride, which optionally in addition contains pharmaceutically acceptable additatives, adjuvants, carriers, dissolvants, preservatives or diluents;

preferably wherein the pharmaceutical composition of (A) or (B) additionally comprises any one of a pharmaceutically

acceptable filler, carrier, excipient, dissolvant, diluent, lubricant, glidant, antioxidant and/or additive, preferably those being selected from the group of pharmaceutically acceptable oil (such as peanut oil, corn oil, sesame oil, cotton oil, olive oil, soybean oil), triglyceride, diglyceride, cocoa butter, coconut fat, hydrogenated fat, hydrogenated animal oil, hydrogenated vegetable oil, solid triglycerides, solid fat, malleable fat, Fattibase, Wecobee bases, Witespol bases, cetyl ester wax,
beeswax, glycerol, polysorbate 80, polyethylene glycol, propylene glycol, isopropyl alcohol, magnesium stearate, stearic acid, talc, silicon dioxide, lauryl sulphate, sodium starch glycolate, detergent, oil detergent mixture, oil surfactant mixture, emulsifiable oil, self-emulsifying system, Neobee M5, tri caprylic/capric triglyceride ester, Miglyol 810, propyleneglycol dicaprylate, Sefsol 228, ethoxylated plant fats, ethanol, polyvinyl pyrrolidone, Tween, polyoxyethylenesorbitan monolaurate, triacetin, diacetin, ascorbic acid, ascorbyl palmitate, butylated hydroxyanisole, butylated hydroxytoluene, hypophosphorous acid, monothioglycerol, propyl gallate, sodium ascorbate, sodium bisulphite, sodium formaldehyde sulfoxylate, sodium meta bisulphite, butylparaben, ethyl paraben, benzoic acid, propylparaben, sodium benzoate, sodium propionate, benzalkonium chloride, benzethonium chloride, benzyl alcohol, cetylpyridinium chloride, chlorobutanol, phenol, phenylethyl alchol, phenylmercuric nitrate, thiomersal, lactose, microcrystalline cellulose, starch, powdered sucrose, calcium phosphate, acacia, alginic acid, carboxymethylcellulose sodium, compressible sugar, ethylcellulose, gelatine, liquid glucose, methylcellulose, povidone, pregelatinized starch, crosscarmellose, crosspovidone, cation exchange resin, silica, colloidal silica, cornstarch, calcium stearate, mineral oil, anis oil, cinnamon oil, cocoa, menthol, orange oil, peppermint oil, vanillin, ginger, colorant,
and/or wherein the pharmaceutical composition of (A) or (B) is in form of oral administrable, parenteral administrable, systemically administrable, capsule, tablet, powder, granulate, suppository, insert, lozenge, troche, buccal tablet, sublingual tablet, compressed tablet, multiple compressed tablet, molded tablet, chewable tablet, effervescent tablet, tablet triturate, sugar coated tablet, film-coated tablet, gelatine-coated tablet, enteric-coated tablet, dispensing tablet, hypodermic tablet, extended release tablet, instant disintegrating tablet, immediate release tablet, dermal formulation, ointment, cream, gel, transdermal formulations, solutions, tincture, injectable, parenteral, implant, formulation for urethral administration, formulations for topical administration, ophthalmic solution, vaginal formulation, inhalant, disperse system, emulsion, multitablet system, microencapsulated drug system, osmotic pump, subdermal implant, ocular system, parenteral system, vaginal system, coated bead system, granule, microsphere, modified-release system, extended-release system, delayed-release system, repeat action system, targeted release system, ion-exchange resin system, slowly eroding and/or hydrophilic matrix system, inert plastic matrix embedded system, ointment, cream, gel, solution, water solution, tincture, oil solution, pill.

**19.** A *Neobeguea spp.* root tissue or extract thereof for use as a medicament or for use in therapy; or
a *Neobeguea spp.* root tissue or extract thereof, for use in the treatment of any one of sexual dysfunction, erectile dysfunction, ejaculatory dysfunction or hypoactive sexual desire disorder, preferably wherein the composition is administered systemically or non-topically.

**20.** A composition as claimed in any one of claims 13 to 15 or 18 or a chemical compound as claimed in any one of claims 16 or 17 for use

(A) as a medicament or for use in therapy; or
(B) for use in treatment of any one of sexual dysfunction, erectile dysfunction, ejaculatory dysfunction or hypoactive sexual desire disorder, preferably wherein the composition of (A) or (B) is administered systemically or non-topically.

**21.** A non-medical method of eliciting a sexual enhancing effect comprising administering to a subject

(A) a pharmaceutically effective amount of a *Neobeguea spp.* root tissue or extract thereof; or
(B) a pharmaceutically effective amount of a composition as claimed in any one of claims 13 to 15 or 18 or a chemical compound as claimed in any one of claims 16 or 17;

preferably wherein the tissue, extract or composition is administered systemically or non-topically.

**22.** Use of

(A) a *Neobeguea spp.* root tissue or extract thereof, in the manufacture of a medicament for treatment of any one of sexual dysfunction, erectile dysfunction, ejaculatory dysfunction or hypoactive sexual desire disorder; or
(B) a composition as claimed in any one of claims 13 to 15 or 18 or a chemical compound as claimed in any one of claims 16 or 17 in the manufacture of a medicament for treatment of any one of sexual dysfunction,

erectile dysfunction, ejaculatory dysfunction or hypoactive sexual desire disorder,

preferably wherein the tissue, extract or composition of (A) or (B) is administered systemically or non-topically.

**23.** Use of

(A) a *Neobeguea spp.* root tissue or extract thereof, for eliciting a sexual enhancing effect, or
(B) a composition as claimed in any one of claims 13 to 15 or 18 or a chemical compound as claimed in any one of claims 16 or 17 for eliciting a sexual enhancing effect,

preferably wherein the tissue, extract or composition of (A) or (B) is administered systemically or non-topically, where the use is a non-medical use.

## Patentansprüche

**1.** Vorgang zur Herstellung eines Extrakts aus einem Wurzelgewebe von *Neobeguea spp.,* wobei der Vorgang zur Herstellung des Extrakts mindestens einen der folgenden Schritte einschließt:

(i) Isolieren einer lipidlöslichen Fraktion und
(ii) Isolieren einer hochmolekularen Fraktion.

**2.** Vorgang nach Anspruch 1, wobei ein lipophiles Lösungsmittel mindestens einmal verwendet wird, wobei das lipophile Lösungsmittel vorzugsweise aus der folgenden Gruppe ausgewählt wird: Pentan, n-Pentan, 2-Methylbutan, 2,2-Dimethylpropan, Hexan, n-Hexan, 2-Methylpentan, 3-Methylpentan, 2,3-Dimethylbutan, 2,2-Dimethylbutan, Heptan, n-Heptan, 2-Methylhexan, 3-Methylhexan, 2,2-Dimethylpentan, 2,3-Dimethylpentan, 2,4-Dimethylpentan, 3,3-Dimethylpentan, 3-Ethylpentan, 2,2,3-Trimethylbutan, Octan, n-Octan, 2-Methylheptan, 3-Methylheptan, 4-Methylheptan, 3-Ethylhexan, 2,2-Dimethylhexan, 2,3-Dimethylhexan, 2,4-Dimethylhexan, 2,5-Dimethylhexan, 3,3-Dimethylhexan, 3,4-Dimethylhexan, 2-Methyl-3-Ethylpentan, 3-Methyl-3-Ethylpentan, 2,2,3-Trimethylpentan, 2,2,4-Trimethylpentan, Isooctan, 2,3,3-Trimethylpentan, 2,3,4-Trimethylpentan, 2,2,3,3-Tetramethylbutan, Cyclohe-xan, Benzonitril, Chlorbenzol, Diethylether, Methyl-tert-butylether, Methylenchlorid, Dichlormethan, Chloroform, Kohlenstofftetrachlorid, 1,2-Dichlorethan, Perchlo-rethylen, Trichlorethylen, 1,1,1-Trichlorethan, Trichlorethen, Perchlorethylen, Tet-rachlorethen, Vinylchlorid, Ethylacetat, Methylethylketon, Propylacetat, Isopropy-lacetat, Butyllactat, n-Butyllactat, Isobutyllactat, tert-Butyllactat, sec-Butyllactat, Butylacetat, n-Butylacetat, Isobutylacetat, tert-Butylacetat, sec-Butylacetat, Triace-tin, 1,2,3-Triacetoxypropan, Diacetin, Glycerin-1,3-diacetat, Glycerin-1,2-diacetat, Benzol, Toluol, Xylol, o-Xylol, m-Xylol, p-Xylol, 2,2,4-Trimethylpentan, Butanon, 2-Butanon, Pentan-3-on, Pentan-2-on, 3-Pentanon, 2-Pentanon, Cyclopentanon, Butan-1-ol, Butan-2- ol, n-Butanol, 1-Butanol, sec-Butanol, 2-Butanol, Isobutanol, 2-Methyl-1-propanol, 2-Methyl-2-propanol, Pentanol, 1-Pentanol, 3-Methyl-1-butanol, 2-Methyl-1-butanol, 2,2-Dimethyl-1-propanol, 3-Pentanol, 2-Pentanol, 3-Methyl-2-butanol, 3-Methyl-2-butanol, 2-Methyl-2-butanol, Hexanol, Heptanol, Octanol, monohydroxylierte(r) Kohlenwasserstoff(e), Diethylenglykol, Dimethylsulfoxid, Methyl-t-butylether, N-Methyl-2-pyrrolidinon, Nitromethan, Tetrahydrofuran, Triethylamin, Benzylalkohol, Carbonsäuremethylester, Carbonsäureethylester, Fettsäuremethylester, Pflanzenöl, tierisches Öl, Triglycerid(e), Mineralöl, Holzterpentin, Petrolether, Naphtha, Kohlenwasserstofflösungsmittel, chloriertes Kohlenwasserstofflösungsmittel, fluoriertes Kohlenwasserstofflösungsmittel, halogeniertes Kohlenwasserstofflösungsmittel, Freon, 1-Brom-3-chlorpropan, [2-(2-Butoxyethoxy)ethyl]acetat, 2-Butoxyethylacetat, Cumol, Cyclohexanol, Cyclohexanon, Decahydronaphthalin, n-Decan, Dibenzylether, 1,2-Dichlorbenzol, 1,3-Dichlorbenzol, 1,4-Dichlorbenzol, 1,2-Dichlorethan, Dimethylcarbonat, Diethylenglykoldibutylether, Diethylenglykoldiethylether, Diethylenglykol-mono-n-hexylether, Diethylenglykolmonobutylether, Diethylenglykolmonoethylether, Diethylenglykolmonomethylether, Ethylbenzol, Ethylformiat, 2-Ethyl-1-hexanol, Dimethylsulfoxid, 1,1,1,3,3,3-Hexafluor-2-propanol, Isoamylacetat, 2-Methylbutylacetat, 3-Methylbutylacetat, Isoamylbutyrat, Isobutylacetat, Isopropylacetat, Isopropylmethylketon, 1,3,5-Trimethylbenzol, (1-Methoxy-2-propyl)acetat, Methylacetat, Methylcyclohexan, Methylcyclohexanol, 5-Methyl-3-heptanon, 3-Methyltetrahydropyran, 2-Methylpentan, 2-Methyl-1-butanol, *n*-Nonan, Nitroethan, Propylacetat, 1,2-Propylenglykoldiacetat, Propylencarbonat, Tetrahydrofuran, Tetrahydrofurfurylalkohol, 1,2,3,4-Tetrahydronaphthalin, Triethylenglykol, Dimethylether, Methylethylether, flüssiges Ethan, flüssiges Ethen, flüssiges Propan, flüssiges *n*-Butan, flüssiges *I*sobutan, flüssiges Kohlenstoffdioxid, flüssiges Trifluormethan, flüssiges Chlortrifluormethan, flüssiges Trichlorfluormethan, flüssiges Ammoniak.

**3.** Vorgang nach einem der Ansprüche 1 oder 2 zur Herstellung von Extrakten aus einem Wurzelgewebe von *Neobe-*

*guea spp.,* wobei der Vorgang die folgenden Schritte umfasst:

(A)

(i) Extrahieren des Wurzelgewebes von *Neobeguea spp.* mit einem lipophilen Lösungsmittel, um einen lipidlöslichen ersten Extrakt herzustellen und
(ii) gegebenenfalls Extrahieren des Wurzelgewebes von *Neobeguea spp.* aus Schritt (i) mit einem hydrophilen Lösungsmittel, um einen zweiten Extrakt herzustellen,
(iii) gegebenenfalls Kombinieren der Extrakte aus Schritt (i) und (ii), um einen vereinten Extrakt herzustellen; oder

(B)

(i) Extrahieren des Wurzelgewebes von *Neobeguea spp.* mit einem hydrophilen Lösungsmittel, um einen ersten Extrakt herzustellen und
(ii) Extrahieren des Wurzelgewebes von *Neobeguea spp.* aus Schritt (i) mit einem lipophilen Lösungsmittel, um einen lipidlöslichen zweiten Extrakt herzustellen,
(iii) gegebenenfalls Kombinieren der Extrakte aus Schritt (i) und (ii), um einen vereinten Extrakt herzustellen; oder

(C)

(i) Extrahieren eines Wurzelgewebes von *Neobeguea spp.* mit einem hydrophilen Lösungsmittel, um einen ersten Extrakt herzustellen,
(ii) Extrahieren des ersten Extrakts mit einem lipophilen Lösungsmittel, um einen lipidlöslichen zweiten Extrakt herzustellen; oder

(D)

(i) Extrahieren des Wurzelgewebes von *Neobeguea spp.* mit einem lipophilen Lösungsmittel, um eine lipophile erste Fraktion herzustellen und
(ii) Absaugen der lipophilen ersten Fraktion mit einem hydrophilen Lösungsmittel und Isolieren der Materialien, die durch das hydrophile Lösungsmittel nicht gelöst werden, wodurch ein lipidlöslicher Extrakt hergestellt wird.

**4.** Vorgang nach einem der Ansprüche 1 oder 2 zur Herstellung von Extrakten aus einem Wurzelgewebe von *Neobeguea spp.,* wobei der Vorgang die folgenden Schritte umfasst:

(A)

(i) Extrahieren eines Wurzelgewebes von *Neobeguea spp.* mit Wasser, um eine wasserlösliche Fraktion herzustellen und
(ii) Absaugen der wasserlöslichen Fraktion mit einem lipophilen Lösungsmittel, das vorzugsweise teilweise mit Wasser mischbar ist und dann Isolieren der Wasserphase, um einen wasserlöslichen Extrakt herzustellen und
(iii) gegebenenfalls Isolieren der hochmolekularen Fraktion aus dem wasserlöslichen Extrakt von Schritt (ii), um eine hochmolekulare Fraktion herzustellen und/oder
(iv) gegebenenfalls Extrahieren entweder des wasserlöslichen Extrakts, der in Schritt (ii) isoliert wurde oder der hochmolekularen Fraktion, die in Schritt (iii) isoliert wurde, mit einem lipophilen Lösungsmittel, um eine lipidlösliche Fraktion herzustellen; oder

(B)

(i) gegebenenfalls Extrahieren eines Wurzelgewebes von *Neobeguea spp.* mit einem lipophilen Lösungsmittel und
(ii) Extrahieren eines Wurzelgewebes von *Neobeguea spp.* oder des Wurzelgewebes von *Neobeguea spp.* aus Schritt (i) mit einem hydrophilen Lösungsmittel, um eine Fraktion herzustellen und
(iii) Isolieren der hochmolekularen Fraktion aus der Fraktion von Schritt (ii), um eine hochmolekulare Fraktion

herzustellen und

(iv) gegebenenfalls Extrahieren der hochmolekularen Fraktion aus Schritt (iii) mit einem lipophilen Lösungsmittel, um eine lipidlösliche Fraktion herzustellen.

**5.** Vorgang nach einem der Ansprüche 1 oder 4, wobei die hochmolekulare Fraktion eine Fraktion mit einem Molekulargewicht ist, das mindestens über 900, sogar noch vorzugsweiser über 1000, vorzugsweiser über 1200, sogar noch vorzugsweiser über 1500, sogar noch vorzugsweiser über 1800, sogar noch vorzugsweiser über 2000, sogar noch vorzugsweiser über 2500, sogar noch vorzugsweiser über 3000 und am bevorzugtesten über 5000 Dalton liegt; vorzugsweise

(A) wobei die hochmolekulare Fraktion durch eine Chromatographie isoliert wird; oder
(B) wobei die hochmolekulare Fraktion unter Verwendung einer Sephadex-Säule, am bevorzugtesten einer G-25-Säule, isoliert wird; oder
(C) wobei die hochmolekulare Fraktion durch eine Dialyse oder Ultrafiltration isoliert wird.

**6.** Vorgang (A) nach einem der Ansprüche 1 oder 2 zur Herstellung einer Fraktion mit sexuell anregender Wirkung aus einem Wurzelgewebe von *Neobeguea spp.,* wobei der Vorgang die folgenden Schritte umfasst:

(i) Extrahieren eines Wurzelgewebes von *Neobeguea spp.* mit einem hydrophilen und/oder lipophilen Lösungsmittel, um einen Extrakt oder eine Fraktion herzustellen und
(ii) Auftragen des Extrakts oder der Fraktion aus Schritt (i) auf eine chromatographische Säule und
(iii) Eluieren des gebundenen Materials in Fraktionen und Auswählen einer eluierten Fraktion, die eine sexuell anregende Wirkung aufweist, wobei die eluierte Fraktion vorzugsweise lipidlöslich ist;
oder

Vorgang (B) zur Herstellung einer Fraktion, die eine sexuell anregende Wirkung aufweist, aus einem Wurzelgewebe von *Neobeguea spp.,* wobei der Vorgang die folgenden Schritte umfasst:

(i) Auftragen einer Fraktion oder eines Extrakts, die bzw. der durch einen der Vorgänge nach den Ansprüchen 1 bis 5 gewonnen wurde, auf eine chromatographische Säule und
(ii) Eluieren des gebundenen Materials in Fraktionen und Auswählen einer eluierten Fraktion, die eine sexuell anregende Wirkung aufweist, wobei die eluierte Fraktion vorzugsweise lipidlöslich ist.

**7.** Vorgang (A) zur Herstellung einer lipidlöslichen Fraktion, die eine sexuell anregende Wirkung aufweist, aus einem Wurzelgewebe von *Neobeguea spp.,* wobei der Vorgang die folgenden Schritte umfasst:

(i) Auftragen einer Fraktion oder eines Extrakts, die bzw. der durch einen der Vorgänge nach den Ansprüchen 1 bis 6 gewonnen wurde, auf eine polare Interaktionssäule und
(ii) Eluieren des gebundenen Materials in Fraktionen und Auswählen einer eluierten Fraktion, die eine sexuell anregende Wirkung aufweist, wobei die eluierte Fraktion lipidlöslich ist;
oder

Vorgang (B) nach einem der Ansprüche 1 oder 2 zur Herstellung einer lipidlöslichen Fraktion, die eine sexuell anregende Wirkung aufweist, aus einem Wurzelgewebe von *Neobeguea spp.,* wobei der Vorgang die folgenden Schritte umfasst:

(i) Extrahieren eines Wurzelgewebes von *Neobeguea spp.* mit einem hydrophilen Lösungsmittel und/oder lipophilen Lösungsmittel, um einen ersten Extrakt herzustellen und
(ii) Auftragen des ersten Extrakts auf eine polare Interaktionssäule und
(iii) Eluieren des gebundenen Materials in Fraktionen und Auswählen einer eluierten Fraktion, die eine sexuell anregende Wirkung aufweist, wobei die eluierte Fraktion lipidlöslich ist;

vorzugsweise
wobei die stationäre Phase der polaren Interaktionssäule in (A) oder (B) aus der folgenden Gruppe ausgewählt wird: Kieselerde, Tonerde, Hydroxyapatit, Cellulose, durch Vinylalkohol gebundene Kieselerde, durch Polyamin gebundene Kieselerde, durch Silanol gebundene Kieselerde, durch Diol gebundene Kieselerde, durch Amino gebundene Kieselerde, anionisch gebundene Kieselerde, durch Amid gebundene Kieselerde, kationisch gebundene Kieselerde, zwitterionisch gebundene Kieselerde oder eine andere gebundene Kieselerde;

und/oder
wobei die mobile Phase in (A) oder (B) aus einem lipophilen Lösungsmittel oder einem Gemisch von lipophilen Lösungsmitteln mit (einem) optionalen Zusatzstoff(en) besteht,
wobei das bzw. die lipophile(n) Lösungsmittel vorzugsweise aus der folgenden Gruppe ausgewählt wird bzw. werden: Pentan, n-Pentan, 2-Methylbutan, 2,2-Dimethylpropan, Hexan, n-Hexan, 2-Methylpentan, 3-Methylpentan, 2,3-Dimethylbutan, 2,2-Dimethylbutan, Heptan, n-Heptan, 2-Methylhexan, 3-Methylhexan, 2,2-Dimethylpentan, 2,3-Dimethylpentan, 2,4-Dimethylpentan, 3,3-Dimethylpentan, 3-Ethylpentan, 2,2,3-Trimethylbutan, Octan, n-Octan, 2-Methylheptan, 3-Methylheptan, 4-Methylheptan, 3-Ethylhexan, 2,2-Dimethylhexan, 2,3-Dimethylhexan, 2,4-Dimethylhexan, 2,5-Dimethylhexan, 3,3-Dimethylhexan, 3,4-Dimethylhexan, 2-Methyl-3-Ethylpentan, 3-Methyl-3-Ethylpentan, 2,2,3-Trimethylpentan, 2,2,4-Trimethylpentan, Isooctan, 2,3,3-Trimethylpentan, 2,3,4-Trimethylpentan, 2,2,3,3-Tetramethylbutan, Cyclohexan, Benzonitril, Chlorbenzol, Diethylether, Methyl-tert-butylether, Methylenchlorid, Dichlormethan, Chloroform, Kohlenstofftetrachlorid, 1,2-Dichlorethan, Perchlorethylen, Trichlorethylen, 1,1,1-Trichlorethan, Trichlorethen, Perchlorethylen, Tetrachlorethen, Vinylchlorid, Ethylacetat, Methylethylketon, Propylacetat, Isopropylacetat, Butyllactat, n-Butyllactat, Isobutyllactat, tert-Butyllactat, sec-Butyllactat, Butylacetat, n-Butylacetat, Isobutylacetat, tert-Butylacetat, sec-Butylacetat, Triacetin, 1,2,3-Triacetoxypropan, Diacetin, Glycerin-1,3-diacetat, Glycerin-1,2-diacetat, Benzol, Toluol, Xylol, o-Xylol, m-Xylol, p-Xylol, 2,2,4-Trimethylpentan, Butanon, 2-Butanon, Pentan-3-on, Pentan-2-on, 3-Pentanon, 2-Pentanon, Cyclopentanon, Butan-1-ol, Butan-2-ol, n-Butanol, 1-Butanol, sec-Butanol, 2-Butanol, Isobutanol, 2-Methyl-1-propanol, 2-Methyl-2-propanol, Pentanol, 1-Pentanol, 3-Methyl-1-butanol, 2-Methyl-1-butanol, 2,2-Dimethyl-1-propanol, 3-Pentanol, 2-Pentanol, 3-Methyl-2-butanol, 3-Methyl-2-butanol, 2-Methyl-2-butanol, Hexanol, Heptanol, Octanol, monohydroxylierte(r) Kohlenwasserstoff(e), Diethylenglykol, Dimethylsulfoxid, Methyl-t-butylether, N-Methyl-2-pyrrolidinon, Nitromethan, Tetrahydrofuran, Triethylamin, Benzylalkohol, Carbonsäuremethylester, Carbonsäureethylester, Fettsäuremethylester, Pflanzenöl, tierisches Öl, Triglycerid(e), Mineralöl, Holzterpentin, Petrolether, Naphtha, Kohlenwasserstofflösungsmittel, chloriertes Kohlenwasserstofflösungsmittel, fluoriertes Kohlenwasserstofflösungsmittel, halogeniertes Kohlenwasserstofflösungsmittel, Freon, 1-Brom-3-chlorpropan, [2-(2-Butoxyethoxy)ethyl]acetat, 2-Butoxyethylacetat, Cumol, Cyclohexanol, Cyclohexanon, Decahydronaphthalin, n-Decan, Dibenzylether, 1,2-Dichlorbenzol, 1,3-Dichlorbenzol, 1,4-Dichlorbenzol, 1,2-Dichlorethan, Dimethylcarbonat, Diethylenglykoldibutylether, Diethylenglykoldiethylether, Diethylenglykol-mono-nhexylether, Diethylenglykolmonobutylether, Diethylenglykolmonoethylether, Diethylenglykolmonomethylether, Ethylbenzol, Ethylformiat, 2-Ethyl-1-hexanol, Dimethylsulfoxid, 1,1,1,3,3,3-Hexafluor-2-propanol, Isoamylacetat, 2-Methylbutylacetat, 3-Methylbutylacetat, Isoamylbutyrat, Isobutylacetat, Isopropylacetat, Isopropylmethylketon, 1,3,5-Trimethylbenzol, (1-Methoxy-2-propyl)acetat, Methylacetat, Methylcyclohexan, Methylcyclohexanol, 5-Methyl-3-heptanon, 3-Methyltetrahydropyran, 2-Methylpentan, 2-Methyl-1-butanol, n-Nonan, Nitroethan, Propylacetat, 1,2-Propylenglykoldiacetat, Propylencarbonat, Tetrahydrofuran, Tetrahydrofurfurylalkohol, 1,2,3,4-Tetrahydronaphthalin, Triethylenglykol, Dimethylether, Methylethylether.

**8.** Vorgang (A) zur Herstellung einer lipidlöslichen Fraktion, die eine sexuell anregende Wirkung aufweist, aus einem Wurzelgewebe von *Neobeguea spp.,* wobei der Vorgang die folgenden Schritte umfasst:

(i) Auftragen einer Fraktion oder eines Extrakts, die bzw. der durch einen der Vorgänge nach den Ansprüchen 1 bis 7 gewonnen wurde, auf eine hydrophobe Interaktionssäule und
(ii) Eluieren des gebundenen Materials in Fraktionen und Auswählen einer eluierten Fraktion, die eine sexuell anregende Wirkung aufweist, wobei die eluierte Fraktion lipidlöslich ist;
oder

Vorgang (B) nach einem der Ansprüche 1 oder 2 zur Herstellung einer lipidlöslichen Fraktion, die eine sexuell anregende Wirkung aufweist, aus einem Wurzelgewebe von *Neobeguea spp.,* wobei der Vorgang die folgenden Schritte umfasst:

(i) Extrahieren eines Wurzelgewebes von *Neobeguea spp.* mit einem hydrophilen Lösungsmittel und/oder lipophilen Lösungsmittel, um einen ersten Extrakt herzustellen und
(iii) Auftragen des ersten Extrakts auf eine hydrophobe Interaktionssäule und
(iv) Eluieren des gebundenen Materials in Fraktionen und Auswählen einer eluierten Fraktion, die eine sexuell anregende Wirkung aufweist, wobei die eluierte Fraktion lipidlöslich ist;
oder

Vorgang (C) nach einem der Ansprüche 1 oder 2 zur Herstellung einer lipidlöslichen Fraktion, die eine sexuelle anregende Wirkung aufweist, aus einem Wurzelgewebe von *Neobeguea spp.,* wobei der Vorgang die folgenden Schritte umfasst:

(i) gegebenenfalls Extrahieren eines Wurzelgewebes von *Neobeguea spp.* mit einem hydrophilen Lösungsmittel, um einen ersten Extrakt herzustellen,
(ii) Extrahieren eines Wurzelgewebes von *Neobeguea spp.* oder des ersten Extrakts aus Schritt (i) mit einem lipophilen Lösungsmittel, um einen zweiten Extrakt herzustellen,
(iii) Auftragen des zweiten Extrakts aus Schritt (ii) auf eine hydrophobe Interaktionssäule,
(iv) Eluieren des gebundenen Materials in Fraktionen und Auswählen einer eluierten Fraktion, die eine sexuell anregende Wirkung aufweist, wobei die eluierte Fraktion lipidlöslich ist;

vorzugsweise wobei die hydrophobe Interaktionssäule in (A), (B) oder (C) mit $C_{4-22}$-Gruppen, vorzugsweise $C_{18}$-Gruppen, derivatisiert wird.

**9.** Vorgang nach einem der Ansprüche 3 bis 8, wobei mindestens eines des bzw. der verwendeten Lösungsmittel(s) aus der folgenden Gruppe ausgewählt wird: Methanol, Ethanol, Acetonitril, Propionitril, Propanol, Propan-1-ol, Propan-2-ol, Dimethylsulfoxid, Formamid, Dimethylformamid, Aceton, Tetrahydrofuran, Glykol, Glycerin, Dioxan, 1,4-Dioxan, Methansäure, Ethansäure, Propansäure, Butansäure, 2-Methylpropansäure, 3-oxo-Butanamid, N,N-Diethylacetamid, N,N-Diethylacetoacetamid, Propylenglykol, Methylsulfonylmethan, Ethanolamin, *tert*-Butylalkohol, Diethylenglykol, Dimethylether, 1,2-Dimethoxyethan, Ethylenglykol, Hexamethylphosphoramid, Hexamethylphosphorsäuretriamid, Pyridin, 2-Methyltetrahydrofuran, 3-Methyltetrahydropyran, 2-Methylpyridin, 1,3-Propandiol, Sulfolan, Triethylenglykol, Tetraethylenglykol, Tetrahydrofurfurylalkohol, Triethanolamin, Triethylphosphat, Triethylenglykol, Triethylenglykoldimethylether, Triethylenglykolmonomethylether, N-Methylpyrrolidon, flüssiges Kohlenstoffdioxid;
und/oder vorzugsweise wobei mindestens eines des bzw. der verwendeten Lösungsmittel(s) Wasser ist.

**10.** Vorgang

(A) nach einem der Ansprüche 1 bis 9, wobei der Extrakt oder die Fraktion eine sexuell anregende Wirkung aufweist; oder
(B) nach einem der Ansprüche 1 bis 9 oder (A) oben stehend, wobei der Extrakt oder die Fraktion mindestens 0,05 %, vorzugsweiser mindestens 0,1 %, sogar noch vorzugsweiser mindestens 0,15 %, sogar noch vorzugsweiser mindestens 0,2 % und sogar noch vorzugsweiser mindestens 0,25 % und am bevorzugtesten mindestens 0,3 % einer Chemikalie nach Anspruch 16 enthält; oder
(C) nach einem der Ansprüche 1 bis 9 oder (A) oder (B) oben stehend, wobei die lipidlösliche Fraktion oder der Extrakt mit einer Menge von mindestens 1 mg/ml, vorzugsweiser mindestens 2 mg/ml, sogar noch vorzugsweiser mindestens 4 mg/ml, sogar noch vorzugsweiser mindestens 8 mg/ml, sogar noch vorzugsweiser mindestens 12 mg/ml, sogar noch vorzugsweiser mindestens 16 mg/ml und am bevorzugtesten mindestens 18 mg/ml in Sonnenblumenöl und/oder in Octan-1-ol bei der Temperatur von 20°C vollständig löslich ist; oder
(D) nach einem der Ansprüche 1 bis 9 oder (A) - (C) oben stehend, wobei eines oder mehrere der Samen, Zweige, Stämme, Blätter oder Früchte von *Neobeguea spp*. anstelle des Wurzelgewebes verwendet werden.

**11.** Vorgang zur Herstellung einer Formulierung, umfassend das Kombinieren eines Extrakts oder einer Fraktion, der bzw. die durch einen Vorgang nach einem der Ansprüche 1 bis 10 gewonnen wurde oder gewonnen werden kann, mit einem beliebigen eines pharmazeutisch annehmbaren Füllmittels, Trägerstoffes, Exzipienten, Lösungsmittels, Verdünnungsmittels, Schmiermittels, Gleitmittels, Antioxidationsmittels und/oder Zusatzstoffes, vorzugsweise denjenigen, die aus der folgenden Gruppe ausgewählt werden: pharmazeutisch annehmbares Öl (wie etwa Erdnussöl, Maisöl, Sesamöl, Baumwollöl, Olivenöl, Sojaöl), Triglycerid, Diglycerid, Kakaobutter, Kokosfett, gehärtetes Fett, gehärtetes Tieröl, gehärtetes Pflanzenöl, feste Triglyceride, festes Fett, formbares Fett, Fattibase, Wecobee-Grundlagen, Witespol-Grundlagen, Cetylesterwachs, Bienenwachs, Glycerin, Polysorbat 80, Polyethylenglykol, Propylenglykol, Isopropylalkohol, Magnesiumstearat, Stearinsäure, Talkum, Siliziumdioxid, Laurylsulfat, Natriumstärkeglykolat, Detergens, Öl-Detergens-Gemisch, Öl-Tensid-Gemisch, emulgierbares Öl, selbstemulgierendes System, Neobee M5, Tricapryl-/-caprintriglyceridester, Miglyol 810, Propylenglykoldicaprylat, Sefsol 228, ethoxylierte Pflanzenfette, Ethanol, Polyvinylpyrrolidon, Tween, Polyoxyethylensorbitanmonolaurat, Triacetin, Diacetin, Ascorbinsäure, Ascorbylpalmitat, Butylhydroxyanisol, Butylhydroxytoluol, Hypophosphorsäure, Monothioglycerin, Propylgallat, Natriumascorbat, Natriumbisulfit, Natriumformaldehydsulfoxylat, Natriummetabisulfit, Butylparaben, Ethylparaben, Benzoesäure, Propylparaben, Natriumbenzoat, Natriumpropionat, Benzalkoniumchlorid, Benzethoniumchlorid, Benzylalkohol, Cetylpyridiniumchlorid, Chlorbutanol, Phenol, Phenylethylalkohol, Phenylquecksilbernitrat, Thiomersal, Lactose, mikrokristalline Cellulose, Stärke, Puderzucker, Calciumphosphat, Akazie, Alginsäure, Carboxy-methylcellulosenatrium, verpressbarer Zucker, Ethylcellulose, Gelatine, flüssige Glucose, Methylcellulose, Povidon, vorverkleisterte Stärke, Croscarmellose, Crospovidon, Kationenaustauschharz, Kieselerde, kolloidale Kieselerde,

Maisstärke, Calciumstearat, Mineralöl, Anisöl, Zimtöl, Kakao, Menthol, Orangenöl, Pfefferminzöl, Vanillin, Ingwer, Farbstoff.

**12.** Vorgang nach einem der vorhergehenden Ansprüche, wobei der Inhalt einer chemischen Verbindung nach Anspruch 16 untersucht wird;
vorzugsweise wobei der Inhalt der chemischen Substanz durch die Verwendung einer Chromatographie und/oder Massenspektrometrie untersucht wird.

**13.** Zusammensetzung

(A) umfassend:

(i) einen lipidlöslichen Extrakt aus einem Wurzelgewebe von *Neobeguea spp*. und/oder
(ii) eine hochmolekulare Fraktion aus einem Wurzelgewebe von *Neobeguea spp.* oder

(B) umfassend einen Extrakt oder eine Fraktion, der bzw. die durch den Vorgang nach einem der Ansprüche 1 bis 12 gewonnen wurde oder gewonnen werden kann,

vorzugsweise wobei Zusammensetzung (A) oder (B) zusätzlich ein beliebiges eines pharmazeutisch annehmbaren Füllmittels, Trägerstoffes, Exzipienten, Lösungsmittels, Verdünnungsmittels, Schmiermittels, Gleitmittels, Antioxidationsmittels und/oder Zusatzstoffes umfasst, vorzugsweise diejenigen, die aus der folgenden Gruppe ausgewählt werden: pharmazeutisch annehmbares Öl (wie etwa Erdnussöl, Maisöl, Sesamöl, Baumwollöl, Olivenöl, Sojaöl), Triglycerid, Diglycerid, Kakaobutter, Kokosfett, gehärtetes Fett, gehärtetes Tieröl, gehärtetes Pflanzenöl, feste Triglyceride, festes Fett, formbares Fett, Fattibase, Wecobee-Grundlagen, Witespol-Grundlagen, Cetylesterwachs, Bienenwachs, Glycerin, Polysorbat 80, Polyethylenglykol, Propylenglykol, Isopropylalkohol, Magnesiumstearat, Stearinsäure, Talkum, Siliziumdioxid, Laurylsulfat, Natriumstärkeglykolat, Detergens, Öl-Detergens-Gemisch, Öl-Tensid-Gemisch, emulgierbares Öl, selbstemulgierendes System, Neobee M5, Tricapryl-/-caprintriglyceridester, Miglyol 810, Propylenglykoldicaprylat, Sefsol 228, ethoxylierte Pflanzenfette, Ethanol, Polyvinylpyrrolidon, Tween, Polyoxyethylensorbitanmonolaurat, Triacetin, Diacetin, Ascorbinsäure, Ascorbylpalmitat, Butylhydroxyanisol, Butylhydroxytoluol, Hypophosphorsäure, Monothioglycerin, Propylgallat, Natriumascorbat, Natriumbisulfit, Natriumformaldehydsulfoxylat, Natriummetabisulfit, Butylparaben, Ethylparaben, Benzoesäure, Propylparaben, Natriumbenzoat, Natriumpropionat, Benzalkoniumchlorid, Benzethoniumchlorid, Benzylalkohol, Cetylpyridiniumchlorid, Chlorbutanol, Phenol, Phenylethylalkohol, Phenylquecksilbernitrat, Thiomersal, Lactose, mikrokristalline Cellulose, Stärke, Puderzucker, Calciumphosphat, Akazie, Alginsäure, Carboxy-methylcellulosenatrium, verpressbarer Zucker, Ethylcellulose, Gelatine, flüssige Glucose, Methylcellulose, Povidon, vorverkleisterte Stärke, Croscarmellose, Crospovidon, Kationenaustauschharz, Kieselerde, kolloidale Kieselerde, Maisstärke, Calciumstearat, Mineralöl, Anisöl, Zimtöl, Kakao, Menthol, Orangenöl, Pfefferminzöl, Vanillin, Ingwer, Farbstoff.

**14.** Zusammensetzung, umfassend eine Formulierung, die durch den Vorgang nach Anspruch 11 erhalten wurde oder erhalten werden kann.

**15.** Zusammensetzung nach einem der Ansprüche 13 bis 14, die in der folgenden Form enthalten ist: oral verabreichbar, parenteral verabreichbar, systemisch verabreichbar, Kapsel, Tablette, Pulver, Granulat, Zäpfchen, Einlage, Pastille, runde Tablette, bukkale Tablette, Sublingualtablette, komprimierte Tablette, mehrfach komprimierte Tablette, Formtablette, Kautablette, Brausetablette, zerstoßene Tablette, Dragee, Filmtablette, Gelatinetablette, enterisch beschichtete Tablette, Abgabetablette, Subkutantablette, Tablette mit verlängerter Freisetzung, sofort auflösende Tablette, Tablette mit sofortiger Freisetzung, dermale Formulierung, Salbe, Creme, Gel, transdermale Formulierungen, Lösungen, Tinktur, Injektionsmittel, Parenteralium, Implantat, Formulierung für die urethrale Verabreichung, Formulierungen für die topische Verabreichung, ophthalmische Lösung, vaginale Formulierung, Inhalationsmittel, disperses System, Emulsion, Multitabletten-System, mikroverkapseltes Arzneimittelsystem, osmotische Pumpe, subdermales Implantat, okulares System, parenterales System, vaginales System, System mit beschichteten Kügelchen, Körnchen, Mikrokügelchen, System mit modifizierter Freisetzung, System mit verlängerter Freisetzung, System mit verzögerter Freisetzung, System mit wiederholter Wirkung, System mit gezielter Freisetzung, Ionenaustauschharz-System, langsam erodierendes und/oder hydrophiles Matrixsystem, inertes in eine Kunststoffmatrix eingebettetes System, Salbe, Creme, Gel, Lösung, Wasserlösung, Tinktur, Öllösung, Pille oder Aufgusseinheit oder Teebeutel.

**16.** Chemische Verbindung mit der allgemeinen Strukturformel III:

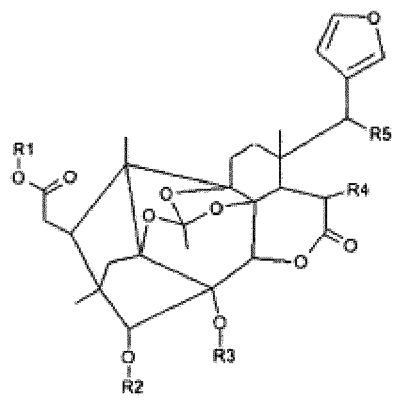

wobei R1 ein Substituent mit ein bis 30 Atomen beliebiger Art, vorzugsweiser ein bis 16 Atomen, vorzugsweiser ein bis 12 Atomen und am bevorzugtesten ein bis 10 Atomen ist, wobei Wasserstoff-, Sauerstoff-, Kohlenstoff-, Schwefel-, Stickstoff-, Phosphor- und Halogenatome bevorzugt werden; wobei der R1-Substituent eine Wasserstoff- und/oder lineare, verzweigte und/oder zyklische Struktur ist; wobei der R1-Substituent vorzugsweise null bis 10 Schweratome, sogar noch vorzugsweiser null bis 6 Schweratome, sogar noch vorzugsweiser null bis 4 Schweratome, sogar noch vorzugsweiser null bis 3 Schweratome und am bevorzugtesten null bis 2 Schweratome umfasst; am bevorzugtesten umfasst der Substituent ein beliebiges von Wasserstoff, Methyl, Ethyl, Propyl, Isopropyl, Butyl, Isobutyl, Cyclopropyl, Cyclopropenyl, Cyclobutyl, Cyclopentyl, Cyclohexyl, Alkyl, zyklischem Alkyl, halogeniertem Alkyl, halogeniertem zyklischen Alkyl, Propenyl, halogeniertem Propenyl, Alkenyl, halogeniertem Alkenyl, halogeniertem zyklischen Alkenyl, Alkynyl, halogeniertem Alkynyl, Aryl, halogeniertem Aryl, wobei Methyl am bevorzugtesten ist

und wobei R2 ein Substituent mit ein bis 30 Atomen beliebiger Art, vorzugsweiser ein bis 20 Atomen und am bevorzugtesten ein bis 16 Atomen ist, wobei Wasserstoff-, Sauerstoff-, Kohlenstoff-, Schwefel-, Stickstoff-, Phosphor- und Halogenatome bevorzugt werden; wobei der R2-Substituent eine Wasserstoff- und/oder lineare, verzweigte und/oder zyklische Struktur ist; wobei der R2-Substituent vorzugsweise null bis 10 Schweratome, sogar noch vorzugsweiser null bis 8 Schweratome, sogar noch vorzugsweiser null bis 6 Schweratome, sogar noch vorzugsweiser null bis 5 Schweratome umfasst; am bevorzugtesten besteht der Substituent aus einem beliebigen von Wasserstoff, Methyl, Ethyl, Propyl, Isopropyl, Cyclopropyl, Cyclopropenyl, Butyl, Isobutyl, Cyclobutyl, Alkyl, halogeniertem Alkyl, zyklischem Alkyl, halogeniertem zyklischen Alkyl, Propenyl, halogeniertem Propenyl, Alkenyl, halogeniertem Alkenyl, zyklischem Alkenyl, halogeniertem zyklischen Alkenyl, Alkynyl, halogeniertem Alkynyl, Aryl, halogeniertem Aryl, Acetyl, halogeniertem Acetyl, Propionyl, halogeniertem Propionyl, Butyryl, halogeniertem Butyryl, Isobutyryl, halogeniertem Isobutyryl, Alkyryl, halogeniertem Alkyryl, zyklischem Alkyryl, halogeniertem zyklischen Alkyryl, Benzoyl, Aryryl, wobei eine Wasserstoff- oder Acetylgruppe oder Isobutyrylgruppe am bevorzugtesten ist und wobei R3 ein Substituent mit ein bis 30 Atomen beliebiger Art, vorzugsweiser ein bis 20 Atomen und am bevorzugtesten ein bis 16 Atomen ist, wobei Wasserstoff-, Sauerstoff-, Kohlenstoff-, Schwefel-, Stickstoff-, Phosphor- und Halogenatome bevorzugt werden; wobei der R3-Substituent eine Wasserstoff- oder eine lineare, verzweigte und/oder zyklische Struktur ist; wobei der R3-Substituent vorzugsweise null bis 10 Schweratome, sogar noch vorzugsweiser null bis 8 Schweratome, sogar noch vorzugsweiser null bis 6 Schweratome, sogar noch vorzugsweiser null bis 5 Schweratome umfasst; am bevorzugtesten besteht der Substituent aus einem beliebigen von Wasserstoff, Methyl, Ethyl, Propyl, Isopropyl, Cyclopropyl, Cyclopropenyl, Butyl, Isobutyl, Cyclobutyl, Alkyl, halogeniertem Alkyl, zyklischem Alkyl, halogeniertem zyklischen Alkyl, Propenyl, halogeniertem Propenyl, Alkenyl, halogeniertem Alkenyl, zyklischem Alkenyl, halogeniertem zyklischen Alkenyl, Alkynyl, halogeniertem Alkynyl, Aryl, halogeniertem Aryl, Acetyl, halogeniertem Acetyl, Propionyl, halogeniertem Propionyl, Butyryl, halogeniertem Butyryl, Isobutyryl, halogeniertem Isobutyryl, Alkyryl, halogeniertem Alkyryl, zyklischem Alkyryl, halogeniertem zyklischen Alkyryl, Benzoyl, Aryryl, wobei eine Wasserstoff- oder Acetylgruppe oder Isobutyrylgruppe am bevorzugtesten ist und wobei R4 ein Substituent ist, der durch eine Einfach- oder Doppelbindung verbunden ist und ein bis 32 Atome, vorzugsweiser ein bis 18 Atome, vorzugsweiser ein bis 15 Atome, vorzugsweiser ein bis 12 Atome und am bevorzugtesten ein bis 9 Atome aufweist, wobei Wasserstoff-, Sauerstoff-, Kohlenstoff-, Schwefel-, Stickstoff-, Phosphor- und Halogenatome bevorzugt werden; wobei der R4-Substituent vorzugsweise eine Wasserstoff- oder eine lineare oder verzweigte und/oder zyklische Struktur ist; wobei der R4-Substituent vorzugsweise zwischen 0 bis 12 Schweratome, sogar noch vorzugsweiser zwischen 0 bis 11 Schweratome, sogar noch vorzugsweiser zwischen 0 bis 10 Schweratome, sogar noch vorzugsweiser zwischen 0 bis 9 Schweratome, sogar noch vorzugsweiser zwischen 0 bis 8 Schweratome, sogar noch vorzugsweiser zwischen 0 bis 7 Schweratome, sogar noch vorzugsweiser zwischen 0 bis 6 Schweratome und am bevorzugtesten zwischen null bis 5 Schweratome umfasst; am bevorzugtesten umfasst der Substituent ein beliebiges von Wasserstoff, Halogen, Oxo, Hydroxy, Methoxy, Ethoxy, Propoxy, Isopropoxy, Butoxy, Isobutoxy, Alkoxy, halogeniertem Alkoxy, Ethenyloxy, Propenyloxy, Alkenyloxy, halogeniertem Alkenyloxy, Methyl, Ethyl, Propyl, Isopropyl, Butyl, Isobutyl, Alkyl, halogeniertem Alkyl, Hydroxymethyl, Hydroxyethyl, Hydroxypropyl, Hydroxyisopropyl, Hydroxybutyl, Hydroxyisobutyl, Hydroxyalkyl, halogeniertem Alkyl, Methylen, Ethenyl, Propenyl, Isopropenyl, Butenyl, Isobutenyl, Alkenyl, halogeniertem Alkenyl, Acetyl, halogeniertem Acetyl, Propionyl, Butyryl, Isobutyryl, Alkyryl, halogeniertem Alkyryl, Acetyloxy, halogeniertem Acetyloxy, Propionyloxy, Isopropionyloxy, Butyryloxy, Isobutyryloxy, Alkyryloxy, halogeniertem Alkyryloxy, 2-oxy-2-Methyl-ethyl, Hydroxyoxomethyl, 2-Hydroxy-2-oxoethyl, 3-Hydroxy-3-oxopropionyl, Methoxy-oxomethyl, Ethoxy-oxomethyl, Propoxy-oxomethyl, Isopropoxy-oxomethyl, Butoxy-oxomethyl, Isobutoxy-oxomethyl, Alkoxy-oxomethyl, 2-Methoxy-2-oxoethyl, 2-Ethoxy-2-oxoethyl, 2-Propoxy-2-oxoethyl, 2-Isopropoxy-2-oxoethyl, 2-Butoxy-2-oxoethyl, 2-Isobutoxy-2-oxoethyl, 2-Alkoxy-2-oxoethyl, Hydroxymethylen, 1-Hydroxyethyliden, 1-Hydroxypropyliden, 1-Hydroxy-2-methylpropyliden, 1-Acetyloxy-2-methylpropyliden, 1-Halogenacetyloxy-2-methylpropyliden, 1-Alkyryloxy-2-methylpropyliden, 1-Halogenalkyryloxy-2-methylpropyliden, wobei Wasserstoff oder Isobutyryl oder 1-Hydroxy-2-methylpropyliden am bevorzugtesten ist und wobei R5 ein Substituent ist, der durch eine Einfach- oder Doppelbindung verbunden ist und ein bis 32 Atome, vorzugsweiser ein bis 18 Atome, vorzugsweiser ein bis 15 Atome, vorzugsweiser ein bis 12 Atome, vorzugsweiser ein bis 10 Atome, vorzugsweiser ein bis 8 Atome und am bevorzugtesten ein bis 7 Atome aufweist, wobei Wasserstoff-, Sauerstoff-, Kohlenstoff-, Schwefel-, Stickstoff-, Phosphor- und Halogenatome bevorzugt werden; wobei der R5-Substituent vorzugsweise eine Wasserstoff- oder eine Sauerstoff- oder eine lineare oder verzweigte und/oder zyklische Struktur ist; wobei der R5-Substituent vorzugsweise zwischen 0 bis 12 Schweratome, sogar noch vorzugsweiser zwischen 0 bis 11 Schweratome, sogar noch vorzugsweiser zwischen 0 bis 10 Schweratome, sogar noch vorzugsweiser zwischen 0 bis 9 Schweratome, sogar noch vorzugsweiser zwischen 0 bis 8 Schweratome, sogar noch vorzugsweiser zwischen 0 bis 7 Schweratome, sogar noch vorzugsweiser zwischen 0 bis 6 Schweratome, vorzugsweiser zwischen 1 bis 5 Schweratome und am bevorzugtesten zwischen 1 bis 4 Schweratome umfasst; am bevorzugtesten umfasst der Substituent ein beliebiges von Wasserstoff, Halogen, Oxo, Hydroxy, Methoxy, Ethoxy, Propoxy, Butoxy, Isobutoxy, Alkoxy, halogeniertem Alkoxy, Ethenyloxy, Propenyloxy, Alkenyloxy, halogeniertem Alkyloxy, Methyl, Ethyl, Propyl, Isopropyl, Butyl, Isobutyl, Alkyl, halogeniertem Alkyl, Hydroxymethyl, Hydroxyethyl, Hydroxypropyl, Hydroxyisopropyl, Hydroxybutyl, Hydroxyisobutyl, Hydroxyalkyl, Methylen, Ethenyl, Propenyl, Isopropenyl, Butenyl, Isobutenyl, Alkenyl, halogeniertem Alkenyl, Acetyl, halogeniertem Acetyl, Propionyl, Isopropionyl, Butyryl, Isobutyryl, Alkyryl, halogeniertem Alkyryl, Acetyloxy, halogeniertem Acetyloxy, Propionyloxy, halogeniertem Propionyloxy, Butyryloxy, halogeniertem Butyryloxy, Isobutyryloxy, halogeniertem Isobutyryloxy, Alkyryloxy, halogeniertem Alkyryloxy, 2-oxy-2-Methyl-ethyl, Hydroxyoxomethyl, 2-Hydroxy-2-oxoethyl, 3-Hydroxy-3-oxopropionyl, Methoxy-oxomethyl, Ethoxy-oxomethyl, Propoxy-oxomethyl, Isopropoxy-oxomethyl, Butoxy-oxomethyl, Isobutoxy-oxomethyl, Alkoxy-oxomethyl, 2-Methoxy-2-oxoethyl, 2-Ethoxy-2-oxoethyl, 2-Propoxy-2-oxoethyl, 2-Isopropoxy-2-oxoethyl, 2-Butoxy-2-oxoethyl, 2-Isobutoxy-2-oxoethyl, 2-Alkoxy-2-oxoethyl, Hydroxymethylen, 1-Hydroxyethyliden, 1-Hydroxypropyliden, 1-Hydroxy-2-methylpropyliden, wobei Oxo und Acetyloxy bevorzugt werden

oder ein pharmazeutisch annehmbares Salz davon

und welche gegebenenfalls dazu in der Lage ist, eine sexuell anregende Wirkung hervorzurufen;

vorzugsweise wobei die chemische Verbindung die folgende allgemeine Struktur-formel III aufweist:

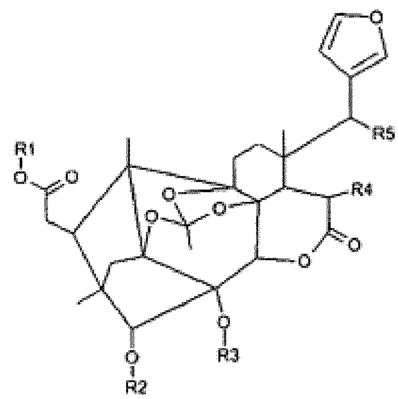

wobei R1 ein beliebiges von Wasserstoff, Methyl oder Ethyl ist, wobei Methyl am bevorzugtesten ist

und wobei R2 ein beliebiges von Wasserstoff, Methyl, Ethyl, Acetyl, halogeniertem Acetyl, Propionyl, Butyryl, Isobutyryl ist, wobei eine Wasserstoff- oder Acetylgruppe oder eine Isobutyrylgruppe am bevorzugtesten ist
und wobei R3 ein beliebiges von Wasserstoff, Methyl, Ethyl, Acetyl, halogeniertem Acetyl, Propionyl, Butyryl, Isobutyryl ist, wobei eine Wasserstoff- oder Acetylgruppe oder eine Isobutyrylgruppe am bevorzugtesten ist
und wobei R4 ein beliebiges von Wasserstoff, Isobutyryl, 1-Hydroxy-2-methylpropyliden, 1-Methoxy-2-methylpropyliden, 1-Acetyloxy-2-methylpropyliden, 1-Halogenacetyloxy-2-methylpropyliden ist, wobei Wasserstoff oder Isobutyryl oder 1-Hydroxy-2-methylpropyliden am bevorzugtesten ist
und wobei R5 ein beliebiges von Oxo, Hydroxy, Methoxy, Ethoxy, Acetyloxy, Halogenacetyloxy, Propionyloxy, Butyryloxy, Isobutyryloxy ist, wobei Oxo und Acetyloxy bevorzugt sind
oder ein pharmazeutisch annehmbares Salz davon
und wobei diese chemische Substanz (chemische Verbindung) gegebenenfalls dazu in der Lage ist, eine sexuell anregende Wirkung hervorzurufen;
und vorzugsweiser wobei die Verbindung eine beliebige der folgenden Strukturen aufweist:

R306

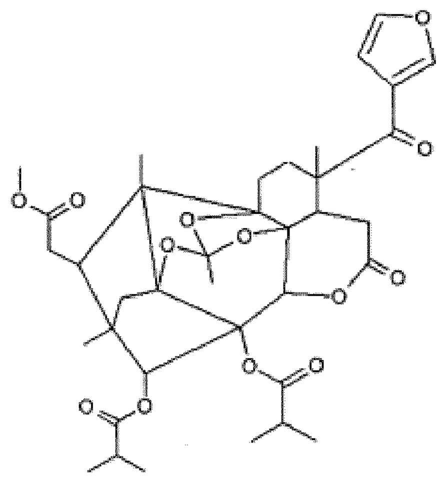

,

R310A

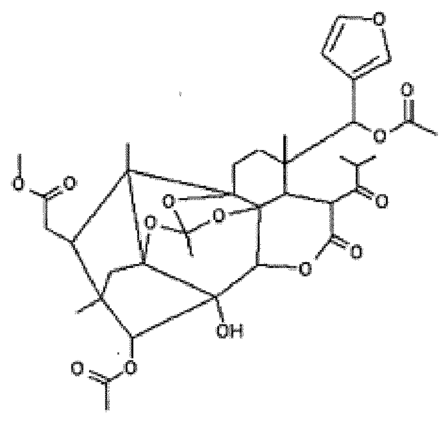

,

R310B

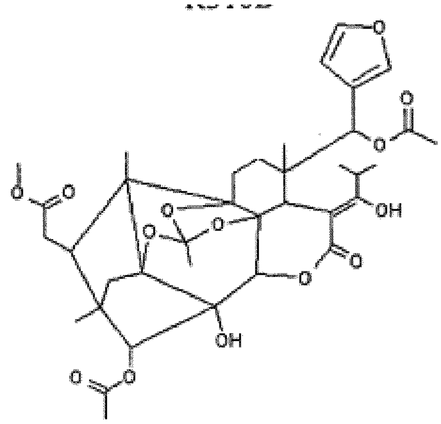

,

R310B1

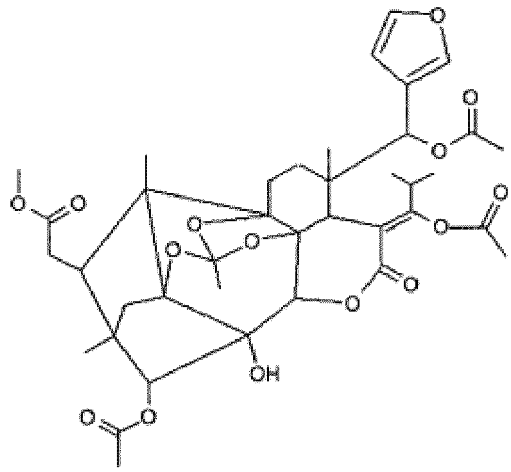

,

R310B2

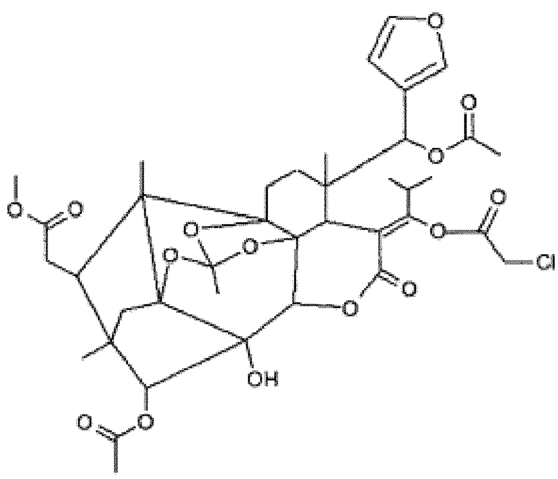

.

R310A3

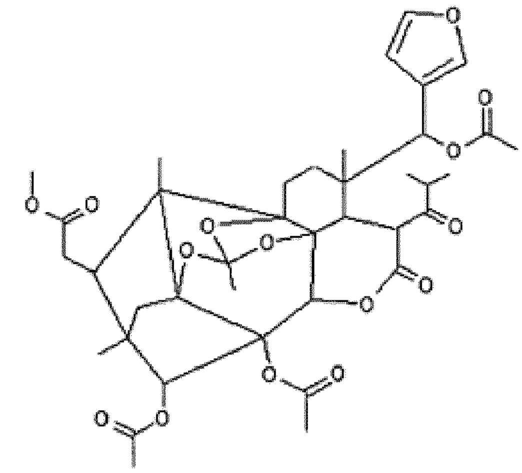

,

R310B3

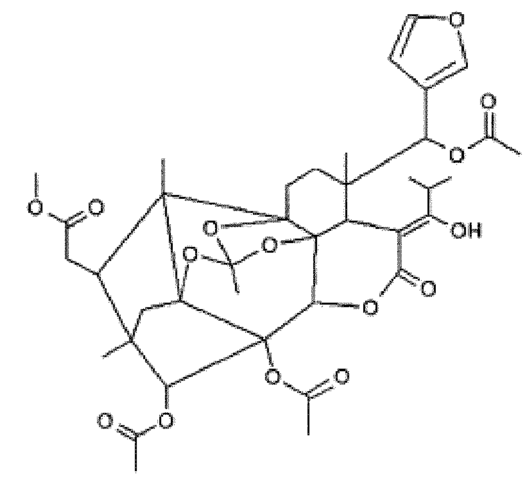

,

R310A4

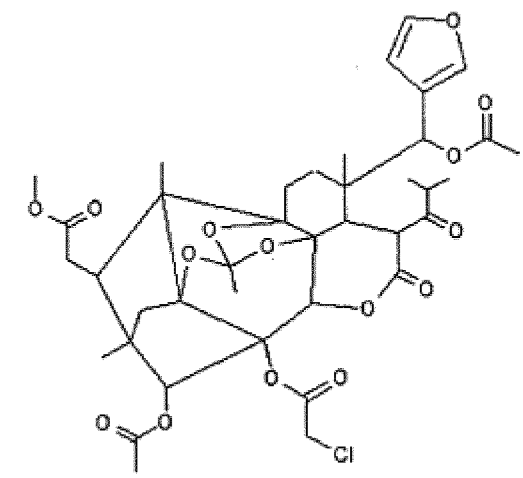

,

R310B4

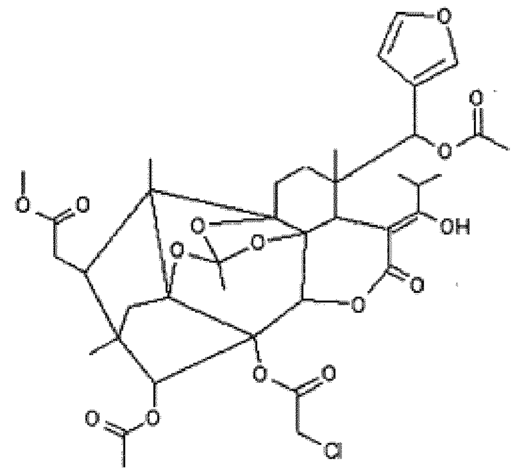

,

R310A5

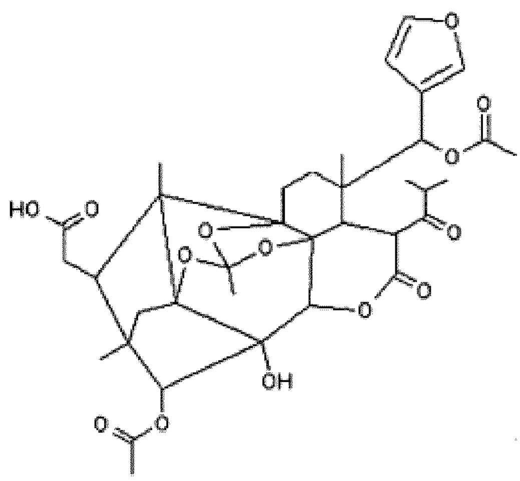

,

R310B5

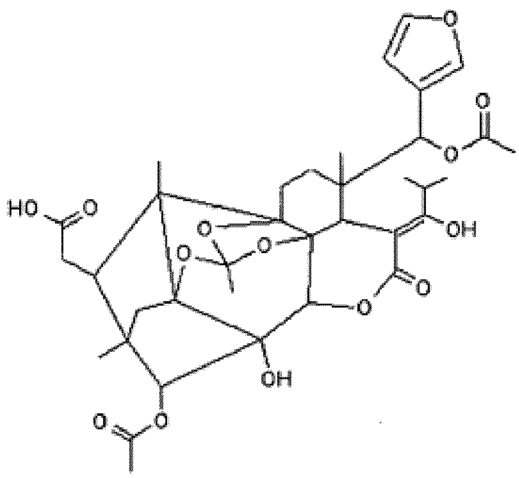

,

R310B6

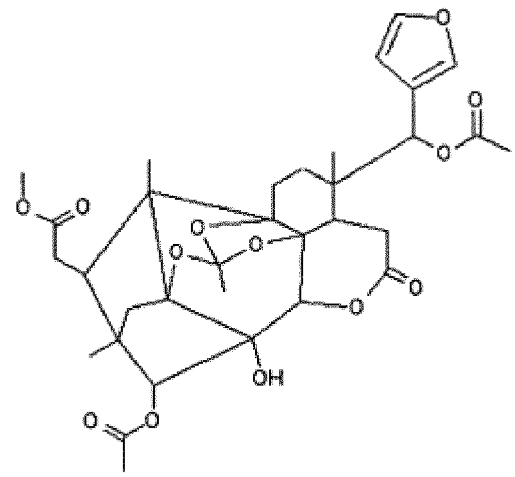

,

R310B7

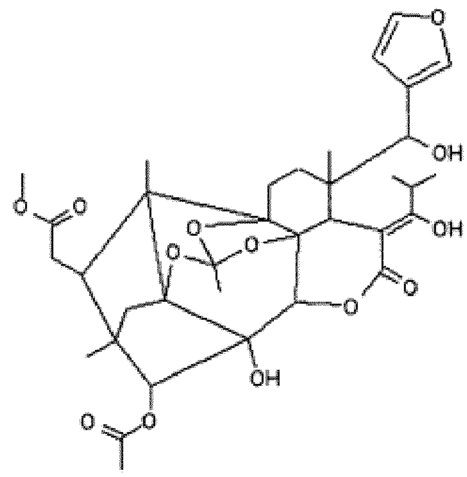

,

R310B8

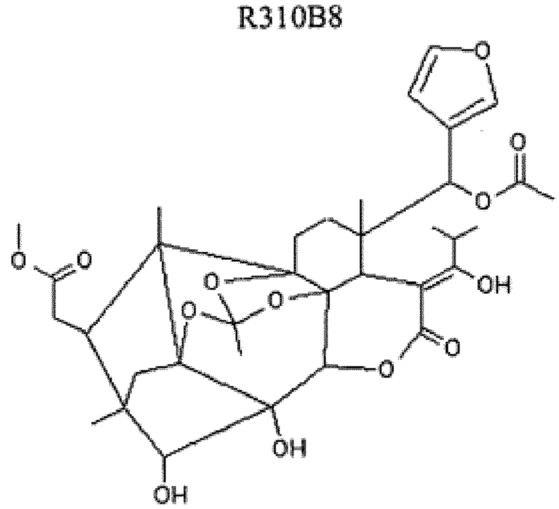

,

R310B9

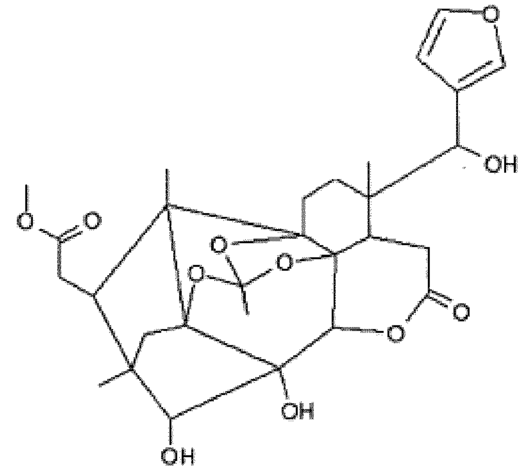

,

R306AB

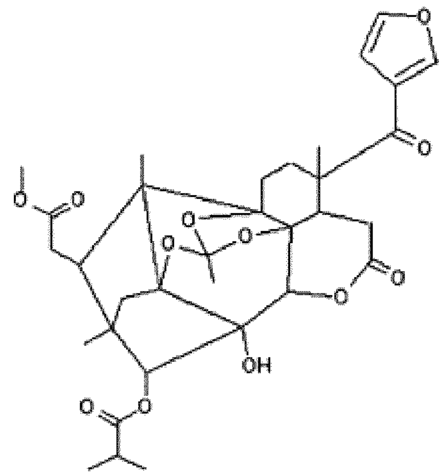

,

R306BA

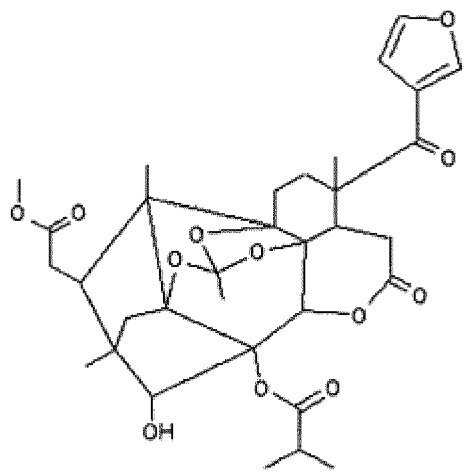

,

R306D

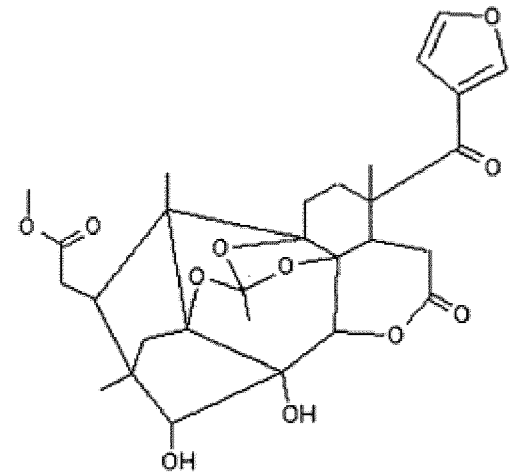

,

R306E

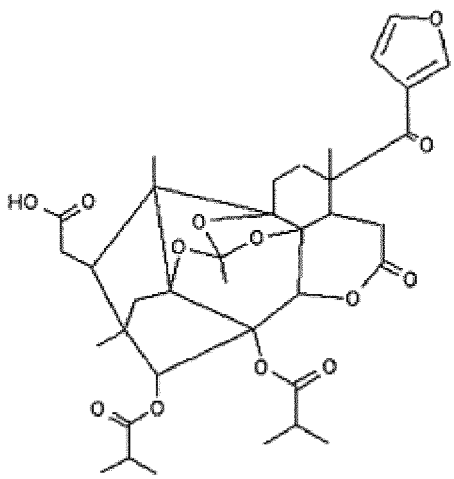

,

R306F

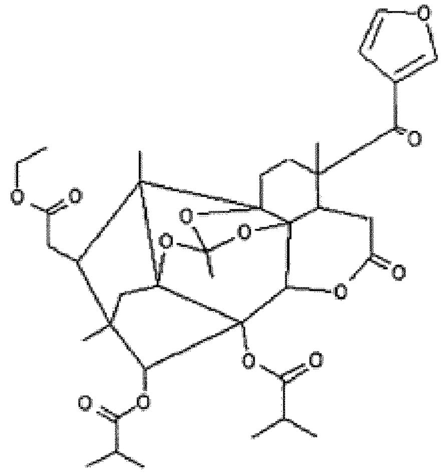

R310B10

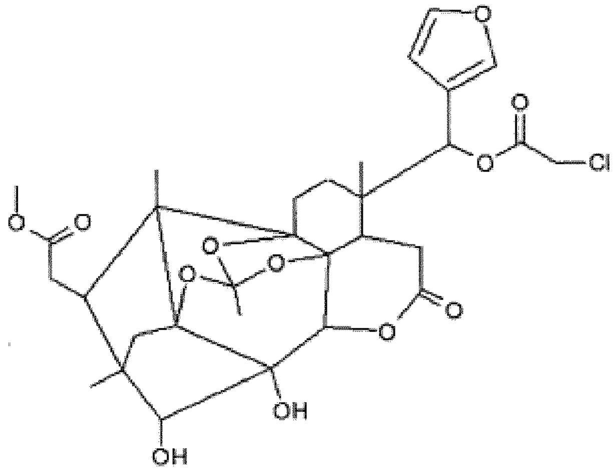

,

R310B11 (R306C)

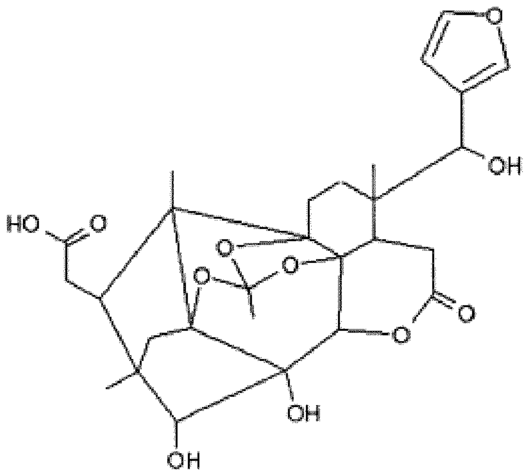

,

R310B12

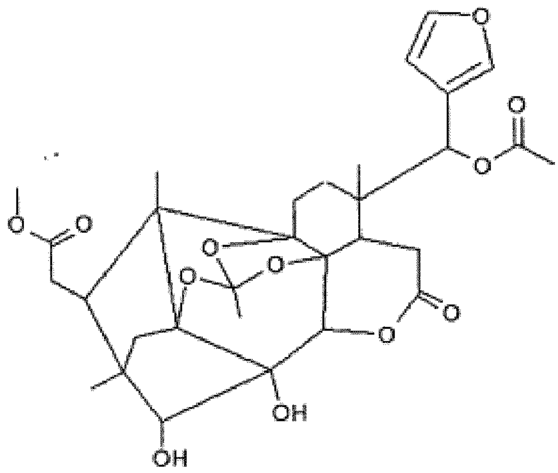

,

R310B13

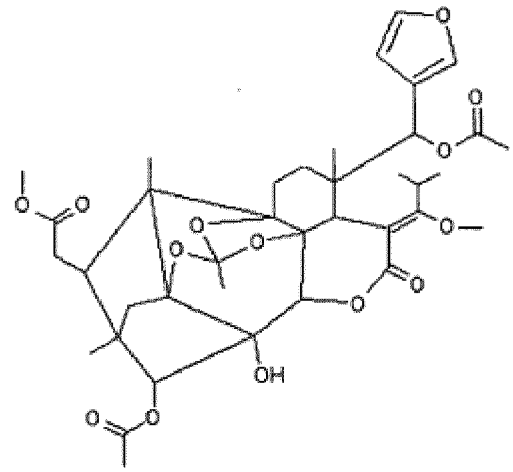

,

R310B14

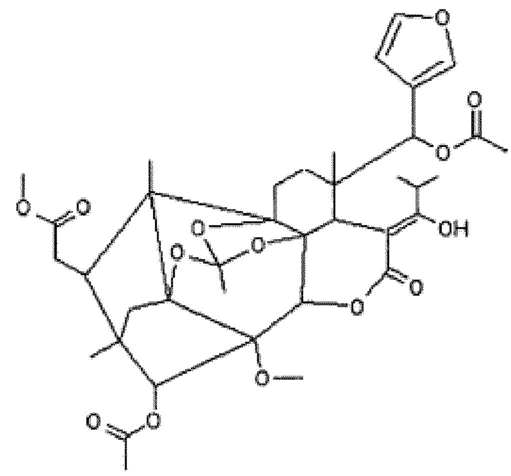

,

R310B15

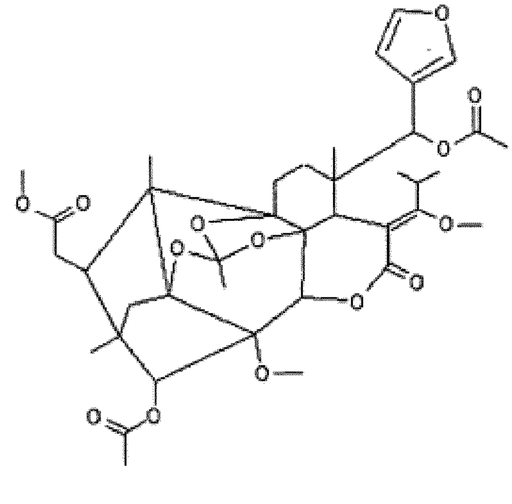

,

R310B16

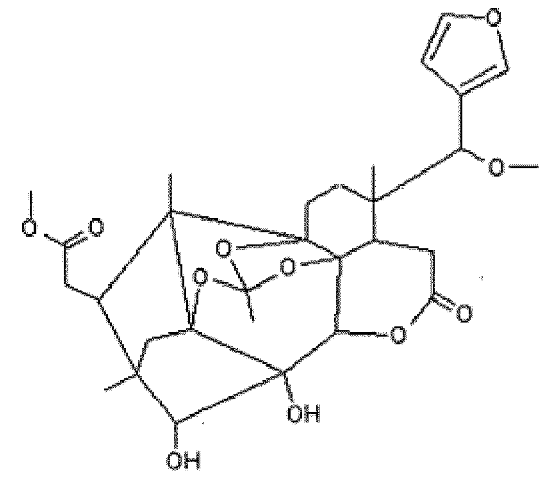

,

R310B17

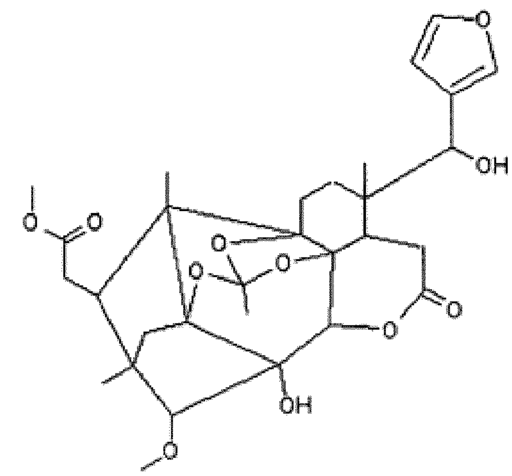

,

R310B18

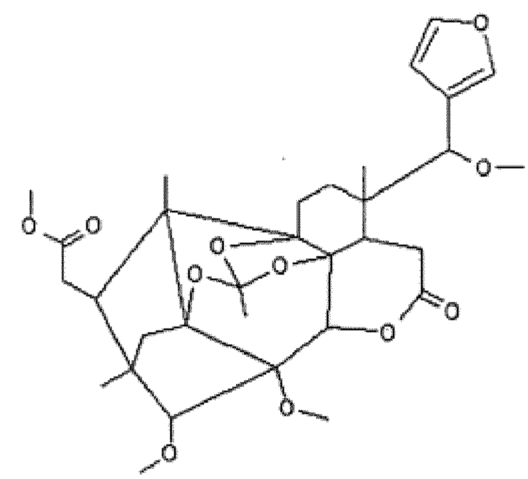

oder ein pharmazeutisch annehmbares Salz davon
und welche gegebenenfalls dazu in der Lage ist, eine sexuell anregende Wirkung hervorzurufen.

**17.** Chemische Verbindung

(A) nach Anspruch 16 mit der Summenformel $C_{37}H_{46}O_{13}$ oder der Summenformel $C_{37}H_{46}O_{14}$ und welche gegebenenfalls eine sexuell anregende Wirkung aufweist; oder
(B) umfassend ein Glycon der chemischen Verbindung nach einem beliebigen von Anspruch 16 oder (A) oben stehend und welche gegebenenfalls eine sexuell anregende Wirkung aufweist; oder
(C) nach einem von Anspruch 16 oder (A) - (C) oben stehend, die aus Pflanzengewebe von *Neobeguea spp.* erhalten wurde oder erhalten werden kann und welche gegebenenfalls eine sexuell anregende Wirkung aufweist; oder
(D) nach einem von Anspruch 16 oder (A) - (C) oben stehend, deren sexuell anregende Wirkung mindestens 0,1 U/mg, sogar noch vorzugsweiser mindestens 0,3 U/mg, sogar noch vorzugsweiser mindestens 1 U/mg, sogar noch vorzugsweiser mindestens 3 U/mg und am bevorzugtesten mindestens 10 U/mg beträgt, wobei die Einheitsaktivität vorzugsweise als $U_{mnt}$ geschätzt wird; oder
(E) umfassend eine Prodrug der Verbindung nach einem von Anspruch 16 oder (A) - (D) oben stehend; oder
(F) nach einem von Anspruch 16 oder (A) - (E) oben stehend, die aus einer natürlichen Quelle erhalten wurde oder erhalten werden kann, wobei die natürliche Quelle vorzugsweise eine Art ist, die zur Familie der Meliaceae gehört; oder
(G) nach einem von Anspruch 16 oder (A) - (F) oben stehend, die durch eine chemische Synthese oder eine chemische Semisynthese erhalten wurde oder erhalten werden kann; oder
(H) nach einem von Anspruch 16 oder (A) - (G) oben stehend, die ausgehend von einem Rohstoff synthetisiert wird, der von einer natürlichen Quelle stammt, wobei die natürliche Quelle vorzugsweise eine Art ist, die zur Familie der Meliaceae gehört; oder
(I) nach einem von Anspruch 16 oder (A) - (H) oben stehend, die durch eine chemische Synthese oder eine chemische Semisynthese, durch einen Vorgang, der zumindest teilweise eine Hydrolyse und/oder Veresterung und/oder Alkylierung einschließt, erhalten wurde; oder
(J) nach einem von Anspruch 16 oder (A) - (I) oben stehend, die unter Verwendung eines Rohstoffs erhalten wird, umfassend ein beliebiges von einem Limonoid, Phragmalin, Neobeguin, Leandreanin A, Leandreanin B, Leandreanin C, Pseudrelon $A_2$, Bussein A, B, C, D, E, F, G, H, J, K, L, M, wobei die Verbindung gegebenenfalls eine sexuell anregende Wirkung aufweist; oder
(K) nach einem von Anspruch 16 oder (A) - (J) oben stehend in radioaktiver Form.

**18.** Pharmazeutische Zusammensetzung

(A) umfassend die chemische Verbindung nach einem der Ansprüche 16 bis 17, gegebenenfalls zusammen mit einem oder mehreren pharmazeutisch annehmbaren Hilfsmitteln, Trägerstoffen, Lösungsmitteln oder Verdünnungsmitteln; oder
(B) umfassend die chemische Verbindung nach einem der Ansprüche 16 bis 17 in einem pharmazeutisch annehmbaren Öl, Fett und/oder Triglycerid, welche gegebenenfalls zusätzlich pharmazeutisch annehmbare Zusatzstoffe, Hilfsmittel, Trägerstoffe, Lösungsmittel, Konservierungsstoffe oder Verdünnungsmittel enthält; vorzugsweise wobei die pharmazeutische Zusammensetzung von (A) oder (B) zusätzlich ein beliebiges eines pharmazeutisch annehmbaren Füllmittels, Trägerstoffes, Exzipienten, Lösungsmittels, Verdünnungsmittels, Schmiermittels, Gleitmittels, Antioxidationsmittels und/oder Zusatzstoffes umfasst, vorzugsweise diejenigen, die aus der folgenden Gruppe ausgewählt werden: pharmazeutisch annehmbares Öl (wie etwa Erdnussöl, Maisöl, Sesamöl, Baumwollöl, Olivenöl, Sojaöl), Triglycerid, Diglycerid, Kakaobutter, Kokosfett, gehärtetes Fett, gehärtetes Tieröl, gehärtetes Pflanzenöl, feste Triglyceride, festes Fett, formbares Fett, Fattibase, Wecobee-Grundlagen, Witespol-Grundlagen, Cetylesterwachs, Bienenwachs, Glycerin, Polysorbat 80, Polyethylenglykol, Propylenglykol, Isopropylalkohol, Magnesiumstearat, Stearinsäure, Talkum, Siliziumdioxid, Laurylsulfat, Natriumstärkeglykolat, Detergens, Öl-Detergens-Gemisch, Öl-Tensid-Gemisch, emulgierbares Öl, selbstemulgierendes System, Neobee M5, Tricapryl-/-caprintriglyceridester, Miglyol 810, Propylenglykoldicaprylat, Sefsol 228, ethoxylierte Pflanzenfette, Ethanol, Polyvinylpyrrolidon, Tween, Polyoxyethylensorbitanmonolaurat, Triacetin, Diacetin, Ascorbinsäure, Ascorbylpalmitat, Butylhydroxyanisol, Butylhydroxytoluol, Hypophosphorsäure, Monothioglycerin, Propylgallat, Natriumascorbat, Natriumbisulfit, Natriumformaldehydsulfoxylat, Natriummetabisulfit, Butylparaben, Ethylparaben, Benzoesäure, Propylparaben, Natriumbenzoat, Natriumpropionat, Benzalkoniumchlorid, Benzethoniumchlorid, Benzylalkohol, Cetylpyridiniumchlorid, Chlorbutanol, Phenol, Phenylethylalkohol, Phenylquecksilbernitrat, Thiomersal, Lactose, mikrokristalline Cellulose, Stärke, Puderzucker, Calciumphosphat, Akazie, Alginsäure, Carboxymethylcellulosenatrium, verpressbarer Zucker, Ethylcellulose, Gelatine, flüssige Glucose, Methylcellulose, Povidon, vorverkleisterte Stärke, Croscarmellose, Crospovidon, Kationenaustauschharz, Kieselerde, kolloidale Kieselerde, Maisstärke, Calciumstearat, Mineralöl, Anisöl, Zimtöl, Kakao, Menthol, Orangenöl, Pfefferminzöl, Vanillin, Ingwer, Farbstoff
und/oder wobei die pharmazeutische Zusammensetzung von (A) oder (B) in der folgenden Form vorliegt: oral verabreichbar, parenteral verabreichbar, systemisch verabreichbar, Kapsel, Tablette, Pulver, Granulat, Zäpfchen, Einlage, Pastille, runde Tablette, bukkale Tablette, Sublingualtablette, komprimierte Tablette, mehrfach komprimierte Tablette, Formtablette, Kautablette, Brausetablette, zerstoßene Tablette, Dragee, Filmtablette, Gelatinetablette, enterisch beschichtete Tablette, Abgabetablette, Subkutantablette, Tablette mit verlängerter Freisetzung, sofort auflösende Tablette, Tablette mit sofortiger Freisetzung, dermale Formulierung, Salbe, Creme, Gel, transdermale Formulierungen, Lösungen, Tinktur, Injektionsmittel, Parenteralium, Implantat, Formulierung für die urethrale Verabreichung, Formulierungen für die topische Verabreichung, ophthalmische Lösung, vaginale Formulierung, Inhalationsmittel, disperses System, Emulsion, Multitabletten-System, mikroverkapseltes Arzneimittelsystem, osmotische Pumpe, subdermales Implantat, okulares System, parenterales System, vaginales System, System mit beschichteten Kügelchen, Körnchen, Mikrokügelchen, System mit modifizierter Freisetzung, System mit verlängerter Freisetzung, System mit verzögerter Freisetzung, System mit wiederholter Wirkung, System mit gezielter Freisetzung, Ionenaustauschharz-System, langsam erodierendes und/oder hydrophiles Matrixsystem, inertes in eine Kunststoffmatrix eingebettetes System, Salbe, Creme, Gel, Lösung, Wasserlösung, Tinktur, Öllösung, Pille.

**19.** Wurzelgewebe von *Neobeguea spp.* oder Extrakt davon zur Verwendung als ein Medikament oder zur Verwendung bei der Therapie; oder
Wurzelgewebe von *Neobeguea spp.* oder Extrakt davon zur Verwendung bei der Behandlung von einem beliebigen von sexueller Dysfunktion, erektiler Dysfunktion, ejakulatorischer Dysfunktion oder sexueller Hypoaktivität, vorzugsweise wobei die Zusammensetzung systemisch oder nicht topisch verabreicht wird.

**20.** Zusammensetzung nach einem der Ansprüche 13 bis 15 oder 18 oder chemische Verbindung nach einem der Ansprüche 16 oder 17 zur Verwendung

(A) als ein Medikament oder zur Verwendung bei der Therapie; oder
(B) zur Verwendung bei der Behandlung von einem beliebigen von sexueller Dysfunktion, erektiler Dysfunktion, ejakulatorischer Dysfunktion oder sexueller Hypoaktivität, vorzugsweise wobei die Zusammensetzung von (A) oder (B) systemisch oder nicht topisch verabreicht wird.

**21.** Nicht medizinisches Verfahren zum Hervorrufen einer sexuell anregenden Wirkung, umfassend die Verabreichung von Folgendem an einen Patienten:

(A) einer pharmazeutisch wirksamen Menge eines Wurzelgewebes von *Neobeguea spp.* oder eines Extrakts davon; oder
(B) einer pharmazeutisch wirksamen Menge einer Zusammensetzung nach einem der Ansprüche 13 bis 15 oder 18 oder einer chemischen Verbindung nach einem der Ansprüche 16 oder 17;

vorzugsweise wobei das Gewebe, der Extrakt oder die Zusammensetzung systemisch oder nicht topisch verabreicht wird.

**22.** Verwendung von

(A) einem Wurzelgewebe von *Neobeguea spp.* oder eines Extrakts davon bei der Herstellung eines Medikaments zur Behandlung von einem beliebigen von sexuel-ler Dysfunktion, erektiler Dysfunktion, ejakulatorischer Dysfunktion oder sexueller Hypoaktivität; oder
(B) einer Zusammensetzung nach einem der Ansprüche 13 bis 15 oder 18 oder einer chemischen Verbindung nach einem der Ansprüche 16 oder 17 bei der Herstellung eines Medikaments zur Behandlung von einem beliebigen von sexueller Dysfunktion, erektiler Dysfunktion, ejakulatorischer Dysfunktion oder sexueller Hypoaktivität,

vorzugsweise wobei das Gewebe, der Extrakt oder die Zusammensetzung von (A) oder (B) systemisch oder nicht topisch verabreicht wird.

**23.** Verwendung von

(A) einem Wurzelgewebe von *Neobeguea spp.* oder eines Extrakts davon zum Hervorrufen einer sexuell anregenden Wirkung oder
(B) einer Zusammensetzung nach einem der Ansprüche 13 bis 15 oder 18 oder einer chemischen Verbindung nach einem der Ansprüche 16 oder 17 zum Hervorrufen einer sexuell anregenden Wirkung,

vorzugsweise wobei das Gewebe, der Extrakt oder die Zusammensetzung von (A) oder (B) systemisch oder nicht topisch verabreicht wird, wobei die Verwendung eine nicht medizinische Verwendung ist.

## Revendications

**1.** Procédé de production d'un extrait de tissu racinaire de *Neobeguea spp.,* le procédé de production de l'extrait comportant au moins l'une des étapes suivantes :

(i) l'isolement d'une fraction liposoluble, et
(ii) l'isolement d'une fraction de masse moléculaire élevée.

**2.** Procédé selon la revendication 1, dans lequel on utilise un solvant lipophile au moins une fois, le solvant lipophile étant de préférence sélectionné dans le groupe incluant pentane, n-pentane, 2-méthylbutane, 2,2-diméthylpropane, dehexane, n-hexane, 2- méthylpentane, 3-méthylpentane, 2,3-diméthylbutane, 2,2-diméthylbutane, heptane, n-heptane, 2-méthylhexane, 3-méthylhexane, 2,2-diméthylpentane, 2,3-diméthylpentane, 2,4-diméthylpentane, 3,3-diméthylpentane, 3-éthylpentane, 2,2,3-triméthylbutane, octane, n-octane, 2-méthylheptane, 3-méthylheptane, 4-méthylheptane, 3-éthylhexane, 2,2-diméthylhexane, 2,3-diméthylhexane, 2,4-diméthylhexane, 2,5-diméthylhexane, 3,3-diméthylhexane, 3,4-diméthylhexane, 2-méthyl-3-éthylpentane, 3-méthyl-3-éthylpentane, 2,2,3-triméthylpentane, 2,2,4-triméthylpentane, isooctane, 2,3,3- triméthylpentane, 2,3,4-triméthylpentane, 2,2,3,3-tétraméthylbutane, cyclohexane, benzonitrile, chlorobenzène, éther diéthylique, éther méthyl-tert-butyle, chlorure de méthylène, dichlorométhane, chloroforme, tétrachlorure de carbone, I, 2-dichloroéthane, perchloroéthylène, trichloroéthylène,1,1, 1-trichloroéthane, trichloroéthylène, perchloroéthylène, tétrachloroéthylène, chlorure de vinyle, acétate d'éthyle, méthyléthylcétone, acétate de propyle, acétate d'isopropyle, lactate de butyle, n-butyle, lactate d'isobutyle, lactate de tert-butyle, lactate sec-butyle, acétate de butyle, n-butyle, acétate d'isobutyle, tert-butyle ,acétate de sec-butyle triacétine,1,2,3-triacetoxypropane, diacétine, glycérol 1,3-diacétate, de glycérol 1,2-diacétate, benzène, toluène, xylène, o-xylène, m xylène, p-xylène, 2,2,4-triméthylpentane, butanone 2-butanone, pentanone-3-one, pentan-2-one, 3-pentanone, 2-pentanone, cyclopentanone, butan-1-ol, butan-2-ol, n-butanol, 1-butanol, sec-butanol, 2-butanol, isobutanol, 2-méthyl-1-propanol, 2-méthyl-2-propanol, pentanol, 1-pentanol, 3-méthyl-1-butanol, 2-méthyl-1-butanol, 2,2-diméthyl-I-propanol, 3-pentanol, 2-pentanol, 3-méthyl-2-butanol, 3-méthyl-2-butanol, 2-méthyl-2-butanol, hexanol, heptanol, hydrocarbure octanol monohydroxylé (s), diéthylène glycol, diméthylsulfoxyde, éther t-butylméthylique, N-méthyl-2-pyrrolidinone, nitrométhane, tétrahydrofuranne, triéthylamine, alcool benzylique, ester méthylique d'acide carboxylique, d'ester éthylique d'acide carboxylique, un radical méthyle d'acide gras ester, huile végétale, huile animale, triglycéride (s), huile minérale, térébenthine de bois, éther de pétrole, naphta, solvant hydrocarboné, solvant hydrocarboné chloré, solvant hydrocarbure fluoré, solvant de type hydrocarbure halogène, fréon,1-bromo-3-chloropropane, 2-(2-butoxyéthoxy) éthyl] acétate d'éthyle, acétate de 2-butoxyéthyle, cumène, cyclohexanol, cyclohexanone, décahydronaphtalène, n-décane, éther dibenzylique, 1,2-dichlorobenzène, 1,3-dichlorobenzène, 1,4-dichlorobenzène,1 , 2-dichloroéthane, carbonate de diméthyle, diéthylène glycol dibutylique, diéthylène glycol diéthylique, diéthylène glycol mono-n-hexylique, éther monobutylique de diéthylène glycol, éther monoéthylique de diéthylène glycol, éther diéthylène glycol monométhylique, éthylbenzène, formate d'éthyle,2-éthyl -1-hexanol, diméthylsulfoxyde, 1,1,1,3,3,3-hexafluoro-2-propanol, acétate d'isoamyle, acétate de 2-méthylbutyle, acétate de 3-méthylbutyle, butyrate d'isoamyle, acétate d'isobutyle, acétate d'isopropyle, méthyle isopropyle cétone, 1,3,5-triméthylbenzène,(I-méthoxy-2-propyle), acétate de méthyle, méthylcyclohexane, méthylcyclohexanol, 5-méthyl-3-heptanone, 3-éthyltétrahydropyranne, 2-méthylpentane, 2-méthyle-1-butanol, n-nonane, nitroéthane, propyle,1,2-propylène glycol diacétate, carbonate de propylène, tétrahydrofuranne, alcool tétrahydrofurfurylique,1,2,3,4-tétrahydronaphtalène, triéthylèneglycol, éther diméthylique, éther éthylique de méthyle, éthane liquide, éthène liquide, propane liquide, n-butane liquide, isobutane liquide, dioxyde de carbone liquide, trifluorométhane liquide, chlorotrifluorométhane liquide, trichlorofluorométhane liquide et ammoniac liquide.

**3.** Procédé selon l'une quelconque des revendications 1 ou 2 pour la production d'extraits à partir d'un tissu racinaire de *Neobeguea spp.,* le procédé comprenant les étapes consistant à :

(A)

(i) extraire le tissu racinaire de *Neobeguea spp.* avec un solvant lipophile pour produire un premier extrait liposoluble, et
(ii) éventuellement, extraire le tissu racinaire de *Neobeguea spp.* de l'étape (i) avec un solvant hydrophile afin de produire un deuxième extrait,
(iii) éventuellement, combiner les extraits des étapes (i) et (ii) pour produire un extrait unifié ;
ou

(B)

(i) extraire le tissu racinaire de *Neobeguea spp.* avec un solvant hydrophile pour produire un premier extrait, et
(ii) extraire le tissu racinaire de *Neobeguea spp.* de l'étape (i) avec un solvant lipophile pour produire un deuxième extrait liposoluble,
(iii) éventuellement, combiner les extraits des étapes (i) et (ii) pour produire un extrait unifié ;
ou

(C)

(i) extraire un tissu racinaire de *Neobeguea spp.* avec un solvant hydrophile pour produire un premier extrait,
(ii) extraire le premier extrait avec un solvant lipophile pour produire un deuxième extrait liposoluble ;
ou

(D)

(i) extraire le tissu racinaire de *Neobeguea spp.* avec un solvant lipophile pour produire une première fraction lipophile, et
(ii) épuiser la première fraction lipophile avec un solvant hydrophile et isoler les matières non solubilisées par le solvant hydrophile, produisant ainsi un extrait liposoluble.

**4.** Procédé selon l'une quelconque des revendications 1 ou 2 pour la production d'extraits à partir d'une tissu racinaire de *Neobeguea spp. ,* le procédé comprenant les étapes consistant à :

A)

(i) extraire un tissu racinaire de *Neobeguea spp.* avec de l'eau pour produire une fraction hydrosoluble, et
(ii) épuiser la fraction hydrosoluble avec un solvant lipophile, de préférence partiellement miscible à l'eau, puis isoler la phase aqueuse pour produire un extrait hydrosoluble, et
(iii) isoler éventuellement la fraction de poids moléculaire élevé à partir de l'extrait hydrosoluble de l'étape (ii) pour produire une fraction de poids moléculaire élevé, et/ou
(iv) extraire éventuellement soit l'extrait hydrosoluble isolé à l'étape (ii) ou la fraction de poids moléculaire élevé isolée à l'étape (iii) avec un solvant lipophile pour produire une fraction liposoluble ; ou

(B)

(i) extraire éventuellement un tissu racinaire de *Neobeguea spp.* avec un solvant lipophile, et
(ii) extraire un tissu racinaire de *Neobeguea spp.* ou le tissu racinaire de *Neobeguea spp.* de l'étape (i) avec un solvant hydrophile, pour produire une fraction, et
(iii) isoler la fraction de poids moléculaire élevé de la fraction provenant de l'étape (ii) pour produire une fraction de poids moléculaire élevé, et
(iv) extraire éventuellement la fraction de poids moléculaire élevé provenant de l'étape (iii) avec un solvant lipophile pour produire une fraction liposoluble.

**5.** Procédé selon l'une quelconque des revendications 1 ou 4, dans lequel la fraction de poids moléculaire élevé est une fraction ayant un poids moléculaire au moins supérieur à 900, de préférence encore supérieur à 1000, de préférence encore supérieur à 1200, encore plus préférablement supérieur à 1500, encore plus préférablement supérieur à 1800, encore plus préférablement supérieur à 2000, encore plus préférablement supérieur à 2500,

encore plus préférablement supérieur à 3000 et le plus préférablement supérieur à 5000 daltons ;
de préférence

(A) dans laquelle la fraction de poids moléculaire élevé est isolée par chromatographie ; ou
(B) dans laquelle la fraction de poids moléculaire élevé est isolée au moyen d'une colonne de Sephadex, le plus préférablement une colonne G-25 ; ou
(C) dans laquelle la fraction de poids moléculaire élevé est isolé par dialyse ou ultrafiltration.

**6.** Procédé (A) selon l'une quelconque des revendications 1 ou 2 pour produire une fraction ayant une activité de stimulation de la fonction sexuelle à partir d'un tissu racinaire de *Neobeguea spp. ,* le procédé comprenant les étapes consistant à :

(i) extraire un tissu racinaire de *Neobeguea spp.* avec un solvant hydrophile et/ou lipophile pour produire un extrait ou une fraction, et
(ii) appliquer l'extrait ou la fraction de l'étape (i) à une colonne chromatographique,
(iii) éluer la matière liée à en fractions,

et sélectionner une fraction éluée ayant une activité de stimulation de la fonction sexuelle, la fraction éluée étant de préférence liposoluble ;
ou
procédé (B) pour produire une fraction ayant une activité de stimulation de la fonction sexuelle à partir d'un tissu racinaire de *Neobeguea spp. ,* le procédé comprenant les étapes consistant à :

(i) appliquer une fraction ou un extrait obtenu par l'un quelconque des procédés des revendications 1 à 5 à une colonne chromatographique, et
(ii) éluer la matière liée à en fractions,

et sélectionner une fraction éluée ayant une activité de stimulation de la fonction sexuelle, la fraction éluée étant de préférence liposoluble.

**7.** Procédé (A) pour produire une fraction liposoluble ayant une activité de stimulation de la fonction sexuelle à partir d'un tissu racinaire de *Neobeguea spp. ,* le procédé comprenant les étapes consistant à :

(i) appliquer une fraction ou un extrait obtenu par l'un quelconque des procédés des revendications 1 à 6 à une colonne d'interaction polaire, et
(ii) éluer la matière liée à en fractions,

et sélectionner une fraction éluée ayant une activité de stimulation de la fonction sexuelle, la fraction éluée étant liposoluble ;
ou
procédé (B) selon l'une quelconque des revendications 1 ou 2 pour la production d'une fraction liposoluble ayant une activité de stimulation de la fonction sexuelle à partir d'un tissu racinaire de *Neobeguea spp. ,* le procédé comprenant les étapes consistant à :

(i) extraire un tissu racinaire de *Neobeguea spp.* avec un solvant hydrophile et/ou lipophile pour produire un premier extrait, et
(ii) appliquer le premier extrait à une colonne d'interaction polaire, et
(iii) éluer la matière liée à en fractions,

et sélectionner une fraction éluée ayant une activité de stimulation de la fonction sexuelle, la fraction éluée étant liposoluble ;
de préférence
dans lequel la phase stationnaire de la colonne d'interaction polaire dans (A) ou (B) est sélectionnée parmi le groupe incluant silice, alumine, hydroxyapatite, cellulose, silice liée à un alcool vinylique, silice liée à une polyamine, silice liée à du silanol, silice liée à du diol, silice liée à un groupe amino, silice liée anionique, silice liée à un amide, silice cationique liée, silice zwitterionique liée, ou toute autre silice liée ;
et/ou
dans lequel la phase mobile dans (A) ou (B) est constituée par un solvant lipophile, ou un mélange de solvants

lipophiles, avec un ou plusieurs additif(s) facultatif(s),
dans lequel le(s) solvant(s) lipophile(s) est (sont) de préférence sélectionné(s) parmi le groupe incluant pentane, n-pentane, 2-méthylbutane, 2,2-diméthylpropane, de hexane, n-hexane, 2-méthylpentane, 3-méthylpentane, 2,3-diméthylbutane, 2,2-diméthylbutane, heptane, n-heptane, 2-méthylhexane, 3-méthylhexane, 2,2-diméthylpentane, 2,3-diméthylpentane, 2,4-diméthylpentane, 3,3-diméthylpentane, 3- éthylpentane, 2, 2,3-triméthylbutane, octane, n-octane, 2-méthylheptane, 3- méthylheptane, 4-méthylheptane, 3-éthylhexane, 2,2-diméthylhexane, 2,3-diméthylhexane, 2,4-diméthylhexane, 2,5-diméthylhexane, 3,3-diméthylhexane, 3,4-diméthylhexane, 2-méthyl-3-éthylpentane, 3-méthyl-3- éthylpentane, 2,2,3-triméthylpentane, 2,2,4-triméthylpentane, isooctane, 2, 3,3- triméthylpentane, 2,3,4-triméthylpentane, 2,2,3,3-tétraméthylbutane, cyclohexane, benzonitrile, chlorobenzène, éther diéthylique, éther méthyl-tert-butyle, chlorure de méthylène, dichlorométhane, chloroforme, tétrachlorure de carbone, 1 , 2-dichloroéthane, perchloroéthylène, trichloroéthylène, 1,1 ,1 trichloroéthane, trichloroéthylène, perchloroéthylène, tétrachloroéthylène, chlorure de vinyle, acétate d'éthyle, méthyléthylcétone, acétate de propyle, acétate d'isopropyle, lactate de butyle, lactate de n-butyle, lactate d'isobutyle, lactate de tert-butyle, lactate de sec-butyle, acétate de butyle, acétate de n-butyle, acétate d'isobutyle, acétate de tert-butyle, acétate de sec-butyle, triacétine, 1,2,3-triacetoxypropane, diacétine, glycérol 1,3- diacétate glycérol 1,2-diacétate, benzène, toluène, xylène, o-xylène, m xylène, p-xylène, 2,2,4-triméthylpentane, butanone, 2-butanone, pentanone-3-one, pentan-2-one, 3-pentanone, 2-pentanone, cyclopentanone, butane-1-ol, butane-2- ol, n-butanol, 1-butanol, sec-butanol, 2-butanol, isobutanol, 2-méthyle-1-ol, 2- méthyle-2-propanol, pentanol, 1-pentanol, 3-méthyl-1-butanol, 2- méthyl-1-butanol, 2,2-diméthyl-1-propanol, 3-pentanol, 2-pentanol, 3-méthyl- 2-butanol, 3-méthyl-2-butanol, 2-méthyl-2-butanol, hexanol, heptanol, hydrocarbure (s) de octanol, monohydroxylés, diéthylène glycol, diméthylsulfoxyde, éther t-butylméthylique, N-méthyl-2-pyrrolidinone, nitrométhane, tétrahydrofuranne, triéthylamine, alcool benzylique, ester méthylique de acide carboxylique, ester éthylique de acide carboxylique, ester méthylique d'acide gras, huile végétale, huile animale, triglycéride (s), huile minérale, térébenthine de bois, éther de pétrole, naphta, solvant hydrocarboné solvant hydrocarboné chloré, un solvant de type hydrocarbure fluoré, un solvant de type hydrocarbure halogène, fréon, 1-bromo-3-chloropropane, [2- (2-butoxyéthoxy) éthyl] acétate d'éthyle, acétate de 2-butoxyéthyle, cumène, cyclohexanol, cyclohexanone, décahydronaphtalène, n- décane, éther dibenzylique, 1,2-dichlorobenzène, 1,3-dichlorobenzène, 1,4-dichlorobenzène, 1,2-dichloroéthane, carbonate de diméthyle, diéthylène glycol dibutylique, éther diéthylique diéthylène glycol, diéthylène glycol, éther mono-n-hexyle , éther monobutylique de diéthylène glycol, éther monoéthylique diéthylène glycol, éther monométhylique de diéthylène glycol, éthylbenzène, formate d'éthyle, 2-éthyl-1-hexanol, diméthylsulfoxyde, 1,1,1,3,3,3-hexafluoro-2-propanol, acétate d'isoamyle, acétate de 2-méthylbutyle, acétate de 3-méthylbutyle, butyrate d'isoamyle, acétate d'isobutyle, acétate d'isopropyle, méthylcétone d'isopropyle, 1,3,5-triméthylbenzène, (1-méthoxy-2-propyle), acétate de méthyle, méthylcyclohexane, méthylcyclohexanol, 5-méthyl-3-heptanone, 3-méthyltétrahydropyranne, 2-méthylpentane, 2-méthyl-1-butanol, n-nonane, nitroéthane, propyle, 1,2-propylène glycol diacétate, carbonate de propylène, tétrahydrofuranne, alcool tétrahydrofurfurylique, 1,2,3,4-tétrahydronaphtalène, triéthylèneglycol, diméthyl ether, éther de méthyle et d'éthyle.

**8.** Procédé (A) pour produire une fraction liposoluble ayant une activité de stimulation de la fonction sexuelle à partir d'un tissu racinaire de *Neobeguea spp.* , le procédé comprenant les étapes consistant à :

(i) appliquer une fraction ou un extrait obtenu par l'un quelconque des procédés des revendications 1 à 7 à une colonne d'interaction hydrophobe, et
(ii) éluer la matière liée en fractions,

et sélectionner une fraction éluée ayant une activité de stimulation de la fonction sexuelle, la fraction éluée étant liposoluble ; ou
procédé (B) selon l'une quelconque des revendications 1 ou 2 pour produire une fraction liposoluble ayant une activité de stimulation de la fonction sexuelle à partir d'un tissu racinaire de *Neobeguea spp.* , le procédé comprenant les étapes consistant à :

(i) extraire un tissu racinaire de *Neobeguea spp.* avec un solvant hydrophile et/ou lipophile solvant pour produire un premier extrait, et
(iii) appliquer le premier extrait à une colonne d'interaction hydrophobe,
(iv) éluer la matière liée en fractions,

et sélectionner une fraction éluée ayant une activité de stimulation de la fonction sexuelle, la fraction éluée étant liposoluble ; ou
procédé (C) selon l'une quelconque des revendications 1 ou 2 pour produire une fraction liposoluble ayant une activité de stimulation de la fonction sexuelle à partir d'un tissu racinaire de *Neobeguea spp.* , le procédé

comprenant les étapes consistant à :

(i) extraire éventuellement un tissu racinaire de *Neobeguea spp.* avec un solvant hydrophile afin de produire un premier extrait,
(ii) extraire un tissu racinaire de *Neobeguea spp.* ou le premier extrait de l'étape (i) avec un solvant lipophile pour produire un deuxième extrait,
(iii) appliquer le deuxième extrait de l'étape (ii) à une colonne d'interaction hydrophobe,
(iv) éluer la matière liée en fractions,

et sélectionner une fraction éluée ayant une activité de stimulation de la fonction sexuelle, la fraction éluée étant liposoluble ;
de préférence, dans lequel la colonne d'interaction hydrophobe dans (A), (B) ou (C) est dérivatisée avec des groupes en $C_{4-22}$, de préférence des groupes en $C_{18}$.

**9.** Procédé selon l'une quelconque des revendications 3 à 8, dans lequel au moins l'un du (des) solvant(s) hydrophile(s) utilisé(s) est (sont) choisi(s) dans groupe incluant méthanol, éthanol, acétonitrile, propionitrile, propanol, propan-1-ol, propan-2 -ol, diméthylsulfoxyde, formamide, diméthylformamide, acétone, tétrahydrofuranne, glycol, glycérol, dioxane, 1,4-dioxane, acide formique, acide acétique, acide propionique, acide butyrique, acide 2-méthylpropanoïque, 3-oxobutanamide, N, N-diéthylacétamide, N, N diéthyl acétoacétamide, propylène glycol, méthylsulfonylméthane, éthanolamine, alcool tert-butyle, diéthylène glycol, diméthyléther, 1,2-diméthoxy éthane, éthylène glycol, hexaméthylphosphoramide, triamide hexaméthylphosphoreux, pyridine, 2-méthyltétrahydrofurane, 3 -méthyltétrahydropyranne, 2- méthylpyridine, 1,3-propanediol, sulfolane, triéthylène glycol, tétraéthylène glycol, alcool tétrahydrofurfurylique, triéthanolamine, phosphate de triéthyle, triéthylène glycol, triéthylène glycol diméthyl éther, éther monométhylique de triéthylèneglycol, N-méthylpyrrolidone, dioxyde de carbone liquide ; et/ou dans lequel, de préférence, au moins l'un des solvant(s) hydrophile(s) utilisé est de l'eau.

**10.** Procédé

(A) selon l'une quelconque des revendications 1 à 9, dans lequel l'extrait ou la fraction a une activité de stimulation de la fonction sexuelle ; ou
(B) selon l'une quelconque des revendications 1 à 9, ou (A) ci-dessus, dans lequel l'extrait ou la fraction contient au moins 0,05%, plus préférablement au moins 0,1%, encore plus préférablement au moins 0,15%, encore plus préférablement au moins 0,2 %, et encore plus préférablement au moins 0,25%, et le plus préférablement au moins 0,3% d'un produit chimique tel que défini dans la revendication 16 ; ou
(C) selon l'une quelconque des revendications 1 à 9, ou (A) ou (B) ci-dessus, dans lequel la fraction ou l'extrait liposoluble est entièrement soluble avec une quantité d'au moins 1 mg/ml, de préférence d'au moins 2 mg/ml, de manière encore plus préférée d'au moins 4 mg/ml, de manière encore plus préférée au moins 8 mg/ml, de manière encore plus préférée d'au moins 12 mg/ml, de manière encore plus préférée d'au moins 16 mg/ml, et le plus préférablement d'au moins 18 mg/ml dans de l'huile de tournesol et/ou dans de l'octan-1-ol à une température de 20 °C ; ou
(D) selon l'une quelconque des revendications 1 à 9, ou (A) - (C) ci-dessus, dans lequel un ou plusieurs des graines, branches, tronc, feuilles ou fruits de *Neobeguea spp.* est (sont) utilisé(s) à la place du tissu racinaire.

**11.** Procédé de fabrication d'une formulation, comprenant la combinaison d'un extrait ou d'une fraction obtenue ou pouvant être obtenue par un procédé tel que revendiqué dans l'une quelconque des revendications 1 à 10, avec l'un quelconque d'une charge, d'un support, d'un excipient, d'un dissolvant, d'un diluant, d'un lubrifiant, d'un agent de glissement, d'un antioxydant et/ou d'un additif pharmaceutiquement acceptable, de préférence ceux qui sont sélectionnés dans le groupe incluant l'huile (par exemple, l'huile d'arachide, l'huile de maïs, l'huile de sésame, l'huile de coton, l'huile d'olive, l'huile de soja), les triglycérides, les diglycérides, le beurre de cacao, la graisse de noix de coco, la graisse hydrogénée, l'huile hydrogénée animale, l'huile végétale hydrogénée, les triglycérides solides, la graisse solide, la graisse malléable, la Fattibase, les bases Wecobee, les bases Witespol, la cire d'ester cétylique, la cire d'abeille, le glycérol, le polysorbate 80, le polyéthylène glycol, le propylène glycol, l'alcool isopropylique, le stéarate de magnésium, l'acide stéarique, le talc, le dioxyde de silicium, le sulfate de lauryle, le glycolate d'amidon sodique, du détergent, un mélange de détergent et d'huile, un mélange d'agent tensio-actif et d'huile, l'huile émulsionnable, un système auto-émulsifiant, Neobee M5, un ester triglycéride tri-caprylique /caprique, le Miglyol 810, le propylèneglycol dicaprylate , le Sefsol 228, les matières grasses végétales éthoxylées, l'éthanol, la polyvinylpyrrolidone, le Tween, le monolaurate de polyoxyéthylènesorbitane, la triacétine, la diacétine, l'acide ascorbique, le palmitate d'ascorbyle, le butylhydroxyanisole, le butylhydroxytoluène, l'acide hypophosphoreux, le monothioglycérol,

le gallate de propyle, l'ascorbate de sodium, le bisulfite de sodium, le formaldéhyde sulfoxylate, le sodium méta bisulfite, le butylparaben, le parahydroxybenzoate d'éthyle, l'acide benzoïque, le parahydroxybenzoate de propyle, le benzoate de sodium, le propionate de sodium, le chlorure de benzalkonium, le chlorure de benzéthonium, l'alcool benzylique, le chlorure de cétylpyridinium, le chlorobutanol, le phénol, l'alcool phényléthylique, le nitrate phénylmercurique, le thimérosal, le lactose, la cellulose microcristalline, l'amidon, le saccharose en poudre, le phosphate de calcium, la gomme arabique, l'acide alginique, la carboxyméthylcellulose sodique, le sucre compressible, l'éthylcellulose, la gélatine, le glucose liquide, la méthylcellulose, la povidone, l'amidon prégélatinisé, la croscarmellose, la crospovidone, la résine échangeuse de cations, la silice, la silice colloïdale, l'amidon de maïs, le stéarate de calcium, l'huile minérale, l'huile d'anis, l'huile de cannelle, le cacao, le menthol, l'huile d'orange, l'essence de menthe poivrée, la vanilline, le gingembre, un colorant pharmaceutiquement acceptable.

**12.** Procédé tel que revendiqué dans l'une quelconque des revendications précédentes, dans lequel la teneur d'un composé chimique tel que défini dans la revendication 16 est analysée ; de préférence, dans lequel la teneur de la substance chimique est analysée par chromatographie et/ou par spectrométrie de masse.

**13.** Composition

(A) comprenant :

(i) un extrait liposoluble à partir d'un tissu racinaire de *Neobeguea spp.* et/ou
(ii) une fraction de poids moléculaire élevé à partir d'un tissu racinaire de *Neobeguea spp.* ; ou

(B) comprenant un extrait ou une fraction obtenue ou pouvant être obtenue par le procédé de l'une quelconque des revendications 1 à 12,

de préférence dans lequel la composition (A) ou (B) comprend en outre l'un quelconque d'une charge, d'un support, d'un excipient, d'un dissolvant, d'un diluant, d'un lubrifiant, d'un agent de glissement, d'un antioxydant et/ou d'un additif, de préférence ceux qui sont sélectionnés dans le groupe qui inclut de huile (par exemple, l'huile d'arachide, l'huile de maïs, l'huile de sésame, l'huile de coton, l'huile d'olive, l'huile de soja), les triglycérides, les diglycérides, le beurre de cacao, la graisse de noix de coco, la graisse hydrogénée, l'huile hydrogénée animale, l'huile végétale hydrogénée, les triglycérides solides, la graisse solide, la graisse malléable, la Fattibase, les bases Wecobee, les bases Witespol, la cire d'ester cétylique, la cire d'abeille, le glycérol, le polysorbate 80, le polyéthylène glycol, le propylène glycol, l'alcool isopropylique, le stéarate de magnésium, l'acide stéarique, le talc, le dioxyde de silicium, le sulfate de lauryle, le glycolate d'amidon sodique, du détergent, un mélange de détergent et d'huile, un mélange d'agent tensio-actif et d'huile, l'huile émulsionnable, un système auto-émulsifiant, Neobee M5, un ester triglycéride tri-caprylique /caprique, le Miglyol 810, le propylèneglycol dicaprylate , le Sefsol 228, les matières grasses végétales éthoxylées, l'éthanol, la polyvinylpyrrolidone, le Tween, le monolaurate de polyoxyéthylènesorbitane, la triacétine, la diacétine, l'acide ascorbique, le palmitate d'ascorbyle, le butylhydroxyanisole, le butylhydroxytoluène, l'acide hypophosphoreux, le monothioglycérol, le gallate de propyle, l'ascorbate de sodium, le bisulfite de sodium, le formaldéhyde sulfoxylate, le sodium méta bisulfite, le butylparaben, le parahydroxybenzoate d'éthyle, l'acide benzoïque, le parahydroxybenzoate de propyle, le benzoate de sodium, le propionate de sodium, le chlorure de benzalkonium, le chlorure de benzéthonium, l'alcool benzylique, le chlorure de cétylpyridinium, le chlorobutanol, le phénol, l'alcool phényléthylique, le nitrate phénylmercurique, le thimérosal, le lactose, la cellulose microcristalline, l'amidon, le saccharose en poudre, le phosphate de calcium, la gomme arabique, l'acide alginique, la carboxyméthylcellulose sodique, le sucre compressible, l'éthylcellulose, la gélatine, le glucose liquide, la méthylcellulose, la povidone, l'amidon prégélatinisé, la croscarmellose, la crospovidone, la résine échangeuse de cations, la silice, la silice colloïdale, l'amidon de maïs, le stéarate de calcium, l'huile minérale, l'huile d'anis, l'huile de cannelle, le cacao, le menthol, l'huile d'orange, l'essence de menthe poivrée, la vanilline, le gingembre, un colorant pharmaceutiquement acceptable.

**14.** Composition comprenant une formulation obtenue ou pouvant être obtenue par le procédé de la revendication 11.

**15.** Composition revendiquée dans l'une quelconque des revendications 13 et 14, se présentant sous la forme de l'une quelconque d'une composition administrable par voie orale, administrable par voie parentérale, administrable par voie systémique, d'une gélule, d'un comprimé, d'une poudre, d'un granulé, d'un suppositoire, d'un insert, d'une pastille, d'un comprimé buccal , d'un comprimé sublingual, d'un comprimé compressé plusieurs fois, d'un comprimé moulé, d'un comprimé à croquer, d'un comprimé effervescent, d'une trituration en comprimé, d'un comprimé enrobé de sucre, d'un comprimé pelliculé, d'un comprimé à enrobage de gélatine, d'un comprimé à enrobage entérique, d'un comprimés de distributeur, d'un comprimé hypodermique, d'un comprimé à libération prolongée, d'un comprimé

à désintégration immédiate, d'un comprimé à libération immédiate, d'une formulation dermique, d'une pommade, d'une crème, d'un gel, d'une formulation transdermique, d'une solutions, d'une teinture, d'une solution injectable, parentérale, d'un implant, d'une formulation pour administration urétrale, d'une formulations pour administration topique, d'une solution ophtalmique, d'une formulation vaginale, d'un inhalant, d'un système dispersé, d'une émulsion, d'un système à comprimés multiples, d'un système de médicament microencapsulé, d'une pompe osmotique, d'un implant sous-cutané, d'un système oculaire, d'un système parentérale, d'un système vaginal, d'un système à granules enrobées, d'une granule, d'une microsphère, d'un système à libération modifiée, d'un système à libération prolongée, d'un système à libération retardée, d'un système à action répétée, d'un système de libération ciblée, d'un système de résine échangeuse d'ions, d'un système à érosion lente et/ou à matrice hydrophile, d'un système noyé à matrice en matière plastique inerte, d'une pommade, d'une crème, d'un gel, d'une solution, d'une solution d'eau, d'une teinture, d'une solution d'huile, d'un sachet de pilules ou d'infusion, ou d'un sachet de thé.

**16.** Composé chimique répondant à la formule générale de la structure III :

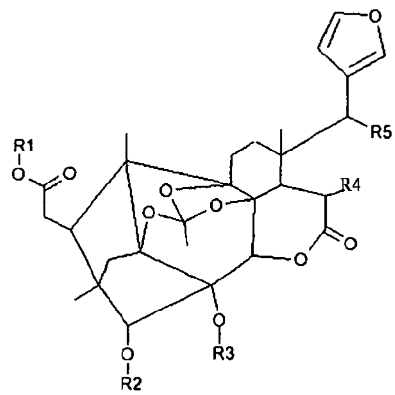

dans lequel RI est un substituant ayant d'un à 30 atomes de n'importe quel type, préférablement d'un à 16 atomes, plus préférablement d'un à 12 atomes et le plus préférablement d'un à 10 atomes, ave, de préférence, des atomes d'hydrogène, d'oxygène, de carbone, de soufre, d'azote, de phosphore et d'halogène ; le substituant R1 étant un atome d'hydrogène et/ou une structure linéaire, ramifiée et/ou cyclique ; le substituant R1 comprenant de préférence de zéro à 10 atomes lourds, encore plus préférablement de zéro à 6 atomes lourds, encore plus préférablement de zéro à 4 atomes lourds, encore plus préférablement de zéro à 3 atomes lourds et le plus préférablement de zéro à 2 atomes lourds ; le plus préférablement le substituant étant constitué de l'une quelconque des substances incluant hydrogène, méthyle, éthyle, propyle, isopropyle, butyle, isobutyle, cyclopropyle, cyclopropényle, cyclobutyle, cyclopentyle, cyclohexyle, alkyle, alkyle cyclique, alkyle halogène, alkyle cyclique halogène, propényle, propényle halogène, alcényle, alcényle halogéné, alcényle cyclique halogène, alcényle, alcényle halogéné, aryle, aryle halogéné, méthyle étant le plus préféré,

et dans lequel R2 est un substituant ayant d'un à 30 atomes de n'importe quel type, plus préférablement d'un à 20 atomes, et plus préférablement d'un à 16 atomes, avec de préférence l'hydrogène, l'oxygène, le carbone, le soufre, l'azote, le phosphore et l'halogène ; le substituant R2 étant de l'hydrogène ou une structure linéaire, ramifiée et/ou cyclique ; le substituant R2 comprenant de préférence de zéro à 10 atomes lourds, encore plus préférablement de zéro à 8 atomes lourds, encore plus préférablement de zéro à 6 atomes lourds, encore plus préférablement de zéro à 5 atomes lourds ; le plus préférablement le substituant étant constitué de l'une quelconque des substances incluant hydrogène, méthyle, éthyle, propyle, isopropyle, cyclopropyle, cyclopropényle, butyle, isobutyle, cyclobutyle, alkyle, alkyle halogéné, alkyle cyclique, alkyle halogéné cyclique, propényle, halogénopropényle, alcényle, alcényle halogéné, alcényle cyclique, alcényle cyclique halogéné, alcynyle, alcynyle halogéné, aryle, aryle halogéné, acétyle, halogénoacétyle, propionyle, halogénopropionyle, butyryle, halogénobutyryle, isobutyryle, halogénoisobutyryle, alkyryle, alkyryle halogéné, alkyryle cyclique, alkyryle cyclique halogéné, benzoyle, aryryle, de l'hydrogène ou un groupe acétyle ou isobutyryle étant le plus préféré,

et dans lequel R3 est un substituant ayant d'un à 30 atomes de n'importe quel type, préférablement d'un à 20 atomes, et le plus préférablement d'un à 16 atomes, l'hydrogène, l'oxygène, le carbone, le soufre, l'azote, le phosphore et l'halogène étant préférés ; le substituant R3 étant un atome d'hydrogène ou une structure linéaire, ramifiée et/ou cyclique; le substituant R3 comportant de préférence de zéro à 10 atomes lourds, encore plus préférablement de zéro à 8 atomes lourds, encore plus préférablement de zéro à 6 atomes lourds, encore plus préférablement de zéro à 5 atomes lourds ; le plus préférablement, le substituant étant constitué de l'une

quelconque des substances incluant hydrogène, méthyle, éthyle, propyle, isopropyle, cyclopropyle, cyclopropényle, butyle, isobutyle, cyclobutyle, alkyle, alkyle halogéné, alkyle cyclique, alkyle cyclique halogéné, propényle, halogénopropényle, alcényle, alcényle halogéné, alcényle cyclique, alcényle cyclique halogéné, alcynyle, alcynyle halogéné, aryle, aryle halogéné, acétyle, halogénoacétyle, propionyle, halogénopropionyle, butyryle, halogénobutyryle, isobutyryle, halogénoisobutyryle, alkyryle, alkyryle halogénés, alkyryle cyclique, alkyryle cyclique halogéné, benzoyle, aryryle, l'hydrogène ou un groupe acétyle ou isobutyryle étant le plus préféré,

et dans lequel R4 représente un substituant relié par une liaison simple ou double ayant d'un à 32 atomes, plus préférablement un à 18 atomes, plus préférablement d'un à 15 atomes, plus préférablement d'un à 12 atomes, et le plus préférablement d'un à 9 atomes, l'hydrogène, l'oxygène, le carbone, le soufre, l'azote, le phosphore et des atomes d'halogène étant préférés ; le substituant R4 étant de préférence un atome d'hydrogène ou une structure linéaire ou ramifié et/ou cyclique; le substituant R4 comprenant de préférence de 0 à 12 atomes lourds, encore plus préférablement de 0 à 11 atomes lourds, encore plus préférablement de 0 à 10 atomes lourds, encore plus préférablement de 0 à 9 atomes lourds, encore plus préférablement de 0 à 8 atomes lourds, encore plus préférablement de 0 à 7 atomes lourds, encore plus préférablement de 0 à 6 atomes lourds, et le plus préférablement de 0 à 5 atomes lourds ; le plus préférablement, le substituant étant constitué de l'une quelconque des substances incluant hydrogène, halogène, oxo, hydroxy, méthoxy, éthoxy, propoxy, isopropoxy, butoxy, isobutoxy, alcoxy, alcoxy halogéné, éthényloxy, propényloxy, alcényloxy, alcényloxy halogéné, méthyle, éthyle, propyle, isopropyle, butyle, isobutyle, alkyle, alkyle halogéné, hydroxyméthyle, hydroxyéthyle, hydroxypropyle, hydroxyispropyl, hydroxybutyle, hydroxyisobutyle, hydroxyalkyle, alkyle halogéné, méthylène, éthényle, propényle, isopropényle , buténylе, isobutényle, alcényle, alcényle halogéné, acétyle, halogénoacétyle, propionyle, butyryle, isobutyryle, alkyryle, alkyryle halogéné, acétyloxy, halogénoacétyloxy, propionyloxy, isopropionyloxy, butyryloxy, isobutyryloxy, alkyryloxy, alkyryloxy halogéné, 2-oxy-2-méthyl-éthyle, hydroxy-oxométhyle, 2-hydroxy-2-oxo-éthyl, 3-hydroxy-3-oxopropionyle, méthoxy-oxométhyle, éthoxy-oxométhyle, propoxy-oxométhyle, oxométhyle isopropoxy, butoxy-oxométhyle, isobutoxy-oxométhyle, alcoxy oxométhyle, 2-méthoxy-2-oxo-éthyl, 2-éthoxy-2-oxoéthyle, 2-propoxy-2-oxoéthyle, 2-isopropoxy-2-oxoéthyle, 2-butoxy-2-oxoéthyle, 2-isobutoxy-2-oxoéthyle, 2-alcoxy-2-oxoéthyle, hydroxyméthylène, 1-hydroxyéthylidène, 1-hydroxypropylidène, 1-hydroxy-2-méthylpropylidène, 1-acétyloxy-2-méthylpropylidène, 1-halogénoacétyloxy-2-méthylpropylidène, 1-alkyryloxy-2-méthylpropylidène, 1-halogénoalkyryloxy-2-méthylpropylidène, l'hydrogène ou l'isobutyryle ou 1-hydroxy-2-méthylpropylidène étant les plus préférés,

et dans lequel R5 est un substituant relié par une liaison simple ou double, ayant d'un à 32 atomes , plus préférablement d'un à 18 atomes , plus préférablement d'un à 15 atomes , plus préférablement d'un à 12 atomes, plus préférablement d'un à 10 atomes, plus préférablement d'un à 8 atomes, et le plus préférablement d'un à 7 atomes, l'hydrogène, l'oxygène, le carbone, le soufre, l'azote, le phosphore et les atomes d'halogène étant préférés ; le substituant R5 étant préférablement un atome d'hydrogène ou un atome d'oxygène ou une structure linéaire ou ramifiée et/ou cyclique ; le substituant R5 comprenant préférablement de 0 à 12 atomes lourds, encore plus préférablement de 0 à 11 atomes lourds, encore plus préférablement de 0 à 10 atomes lourds, encore plus préférablement entre 0 à 9 atomes lourds, encore plus préférablement de 0 à 8 atomes lourds, encore plus préférablement de 0 à 7 atomes lourds, encore plus préférablement de 0 à 6 atomes lourds, plus préférablement de1 à 5 atomes lourds et le plus préférablement de 1 à 4 atomes lourds ; +++ le plus préférablement le substituant étant constitué de l'une des substances incluant hydrogène, halogène, oxo, hydroxy, méthoxy, éthoxy, propoxy, butoxy, isobutoxy, alcoxy, alcoxy halogéné, éthényloxy, propényloxy, alcényloxy, alkyloxy halogéné, méthyle, éthyle, propyle, isopropyle, butyle, isobutyle, alkyle, alkyle halogéné, hydroxyméthyle, hydroxyéthyle, hydroxypropyle, hydroxyisopropyle, hydroxybutyle, hydroxyisobutyle, hydroxyalkyle, méthylène, éthényle, propényle, isopropényle, buténylе, isobutényle, alcényle, alcényle halogéné, acétyle, halogénoacétyle, propionyle, isopropionyle, butyryle, isobutyryle , alkyryle, alkyryle halogéné, acétyloxy, halogénoacétyloxy, propionyloxy, halogénopropionyloxy, butyryloxy, halogénobutyryloxy, isobutyryloxy, halogénoisobutyryloxy, alkyryloxy, alkyryloxy halogène,+++ 2-oxy-2-méthyle-éthyle, hydroxyoxométhyle, 2-hydroxy-2-oxoéthyle, 3-hydroxy-3-oxopropionyle, méthoxy-oxométhyle, éthoxy-oxométhyle, propoxy- oxométhyle, isopropoxy-oxométhyle, butoxy-oxométhyle, isobutoxy-oxométhyle, alcoxy oxométhyle, 2-méthoxy-2-oxoéthyle, 2-éthoxy-2-oxoéthyle, 2-propoxy-2- oxoéthyle, 2-isopropoxy-2-oxoéthyle, 2-butoxy-2-oxoéthyle, 2-isobutoxy-2-oxoéthyle, 2-alcoxy-2-oxoéthyle, hydroxyméthylène, 1-hydroxyéthylidène, 1-hydroxypropylidène, 1-hydroxy-2-méthylpropylidène, oxo et acétyloxy étant préférés,

ou un sel pharmaceutiquement acceptable de ceux-ci,

et qui est éventuellement capable d'induire une effet de stimulation sexuelle ; dans lequel le composé chimique possède de préférence la formule générale de la structure III :

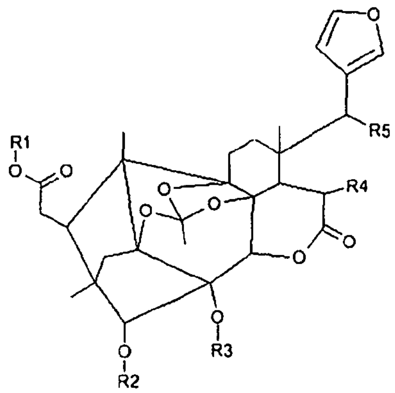

dans lequel R1 est l'un quelconque d'un atome hydrogène, d'un groupe méthyle ou éthyle, le groupe méthyle étant le plus préféré,
et dans lequel R2 est l'un quelconque d'un atome d'hydrogène, d'un groupe méthyle, éthyle, acétyle, halogénoacétyle, propionyle, butyryle, isobutyryle, un atome d'hydrogène ou un groupe acétyle ou isobutyryle étant le plus préféré,
et dans lequel R3 est l'un quelconque d'un atome hydrogène, d'un groupe méthyle, éthyle, acétyle, halogénoacétyle, propionyle, butyryle, isobutyryle, un groupe d'hydrogène ou un groupe acétyle ou isobutyryle étant le plus préféré,
et dans lequel R4 est l'un quelconque d'un atome d'hydrogène, d'un groupe isobutyryle, d'1-hydroxy-2- méthylpropylidène, 1-méthoxy-2-méthylpropylidène, 1-acétoxy-2-méthylpropylidène, 1-halogénoacétyloxy-2-méthylpropylidène, l'atome d'hydrogène ou le groupe isobutyryle ou l'1-hydroxy-2-méthylpropylidène étant le plus préféré,
et dans lequel R5 est l'un quelconque d'un groupe oxo, hydroxy, méthoxy, éthoxy, acétyloxy, halogénoacétyloxy, propionyloxy, butyryloxy,
isobutyryloxy, un groupe oxo et un groupe acétyloxy étant préférés,
ou un sel pharmaceutiquement acceptable de ceux-ci,
et dont la substance chimique (composé chimique) est éventuellement capable d'induire un effet de stimulation sexuelle ; et plus préférablement, dans lequel le composé possède l'une des structures suivantes :

R306

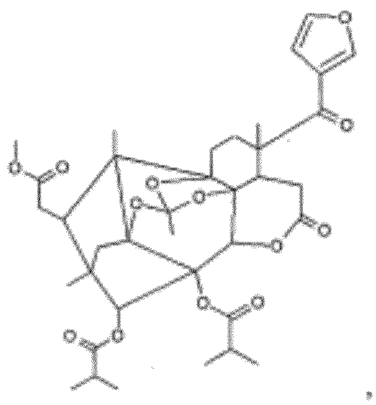

R310A

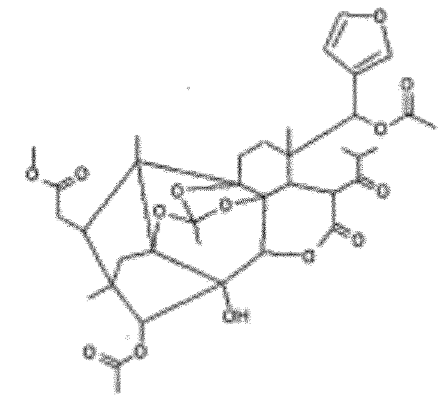

,

R310B

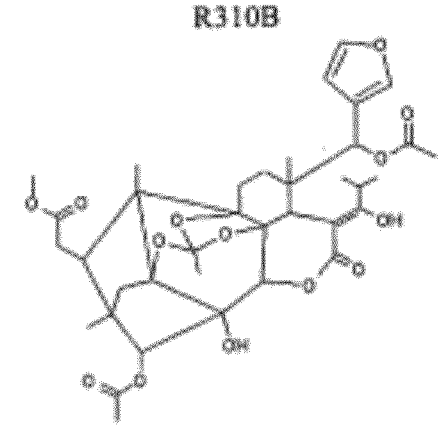

,

R310B1

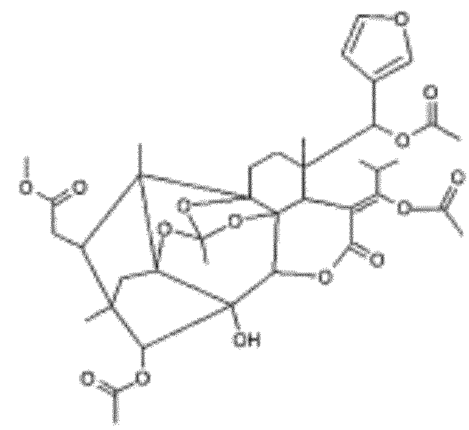

,

R310B2

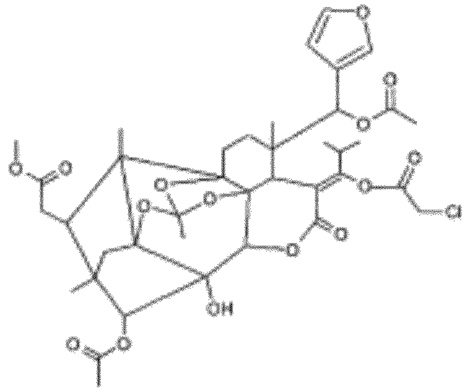

,

R310A3

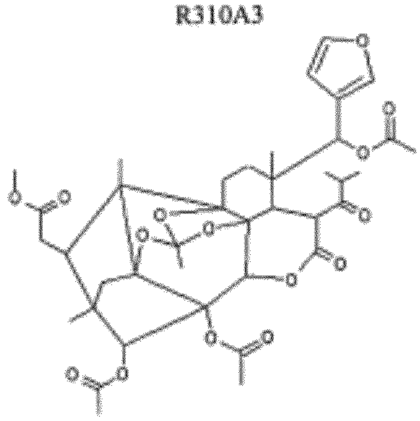

,

R310B3

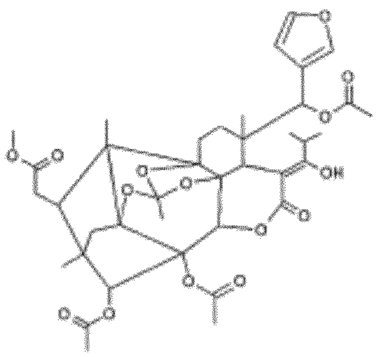

,

R310A4

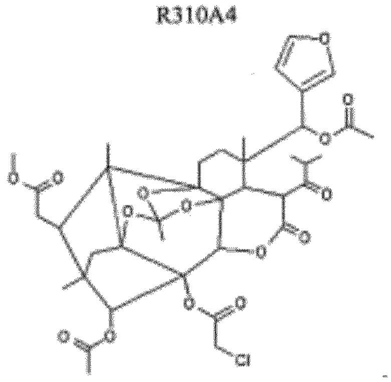

R310B4

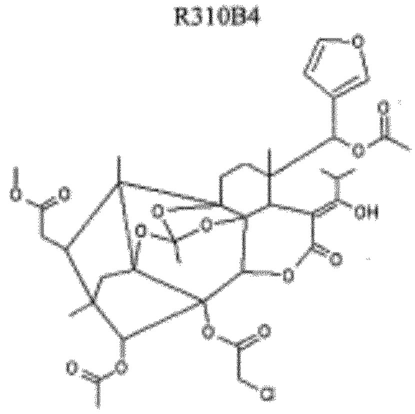

R310A5

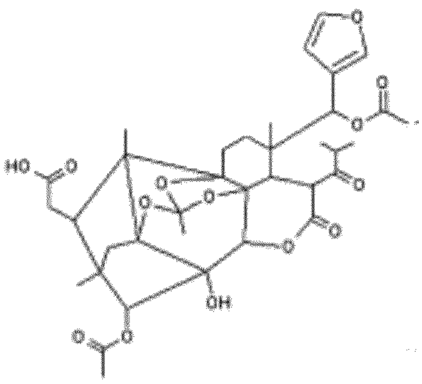

R310B5

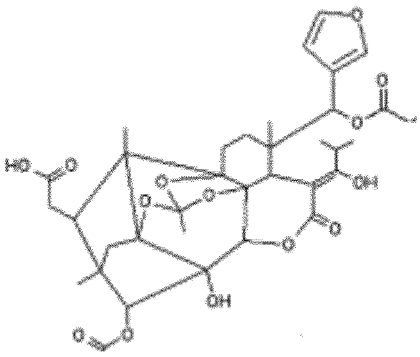

R310B6

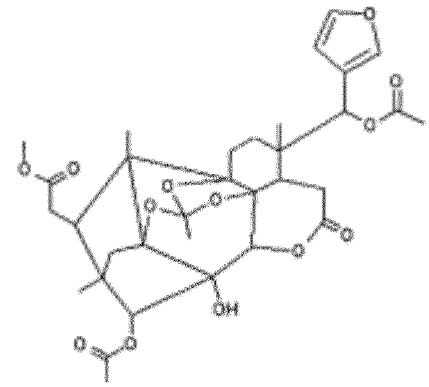

R310B7

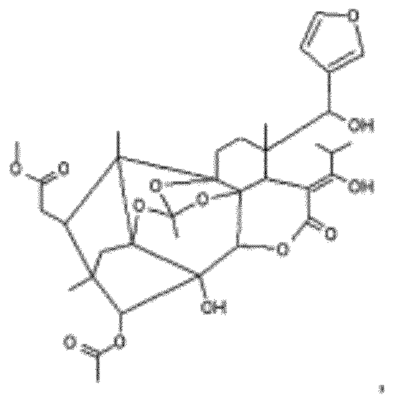

R310B8

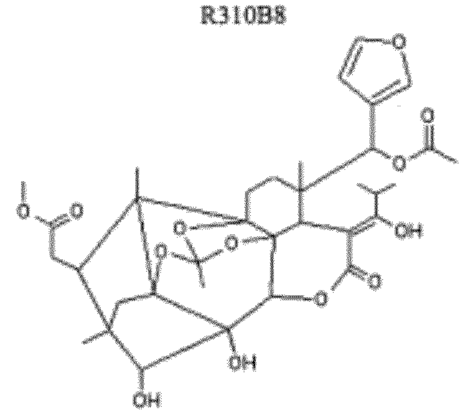

,

R310B9

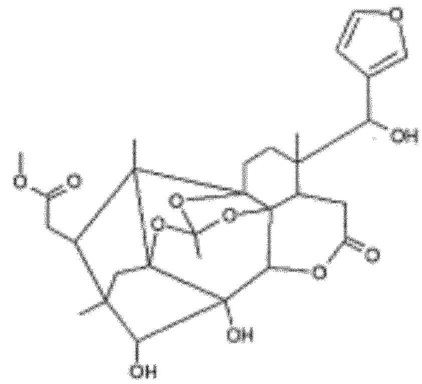

,

R306AB

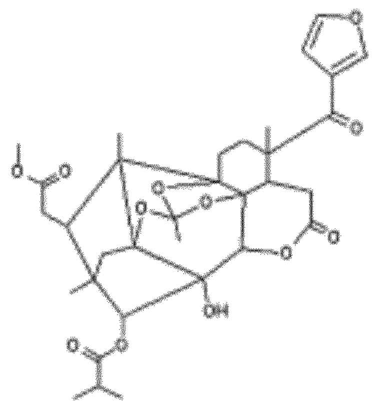

.

R306BA

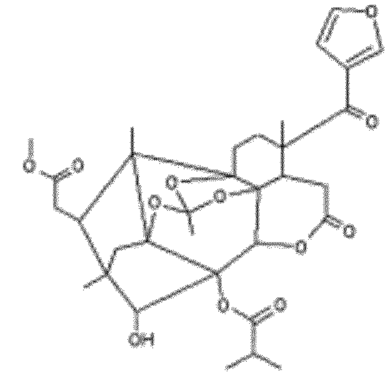

,

R306D

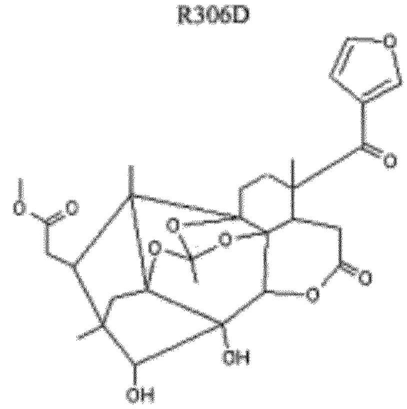

,

R306E

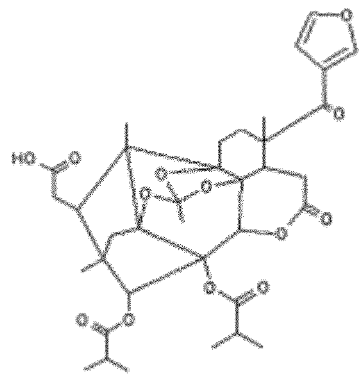

,

R306F

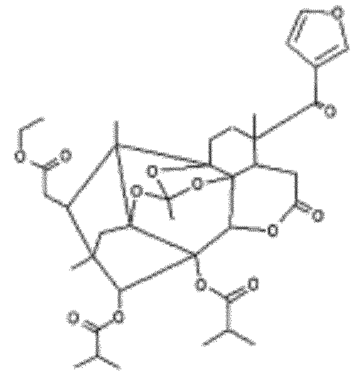

R310B10

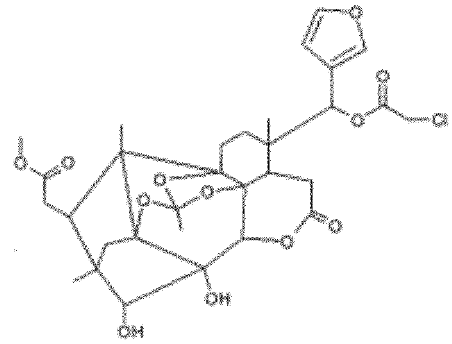

,

R310B11 (R306C)

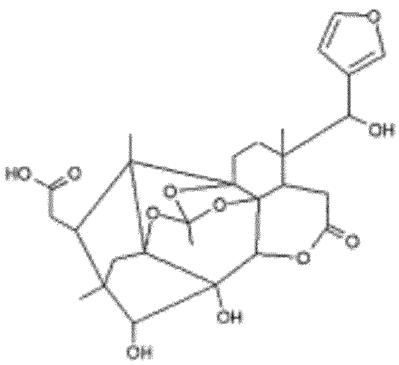

,

R310B12

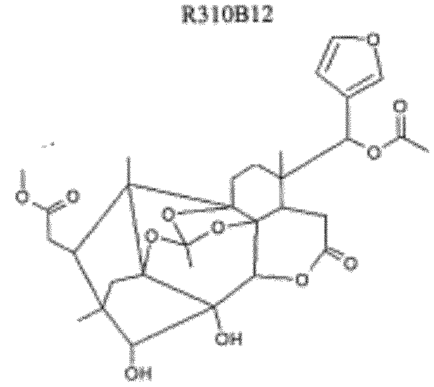

R310B13

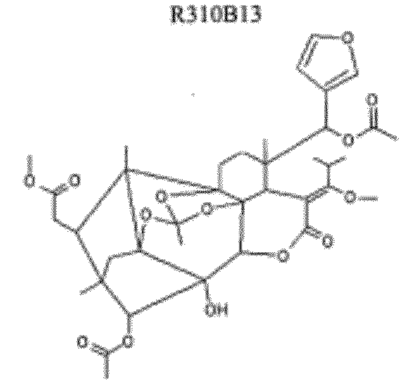

R310B14

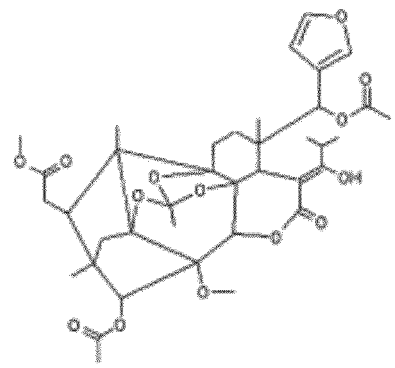

,

R310B15

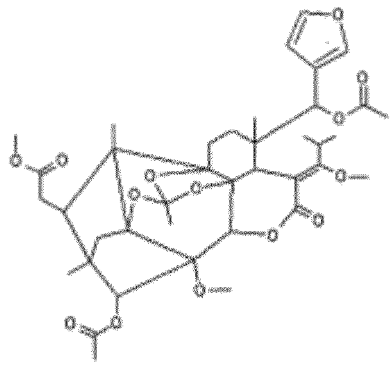

,

R310B16

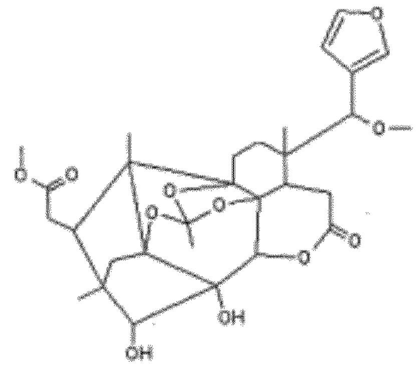

,

R310B17

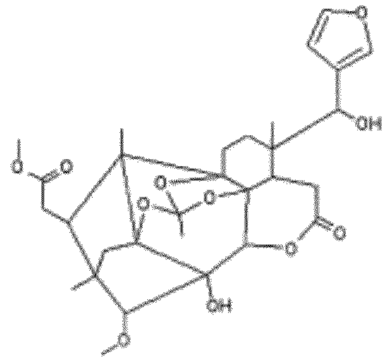

,

R310B18

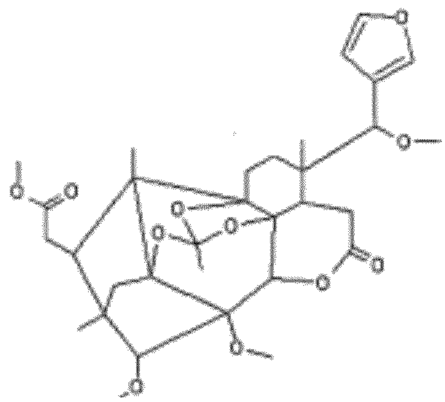

ou un sel pharmaceutiquement acceptable de ceux-ci, et qui est éventuellement capable d'induire un effet de stimulation sexuelle.

**17.** Composé chimique

(A) selon la revendication 16, ayant la formule de synthèse $C_{37}H_{46}O_{13}$ ou la formule de synthèse $C_{37}H_{46}O_{14}$, et qui a éventuellement un effet de stimulation sexuelle ; ou

(B) comprenant un glycone du composé chimique selon l'une quelconque de la revendication 16 ou de (A) ci-dessus, et qui a éventuellement un effet de stimulation sexuelle ; ou

(C) selon l'une quelconque de la revendication 16 ou de (A) - (C) ci-dessus, obtenu à partir de, ou pouvant être

obtenu à partir d'un tissu végétal de *Neobeguea spp.,* et qui a éventuellement un effet de stimulation sexuelle ; ou
(D) selon l'une quelconque de la revendication 16 ou de (A) - (C) ci-dessus, dont l'activité de stimulation de la fonction sexuelle est d'au moins 0,1 U/mg, encore plus préférablement d'au moins 0,3 U/mg, encore plus préférablement d'au moins 1 U/mg, encore plus préférablement d'au moins 3 U/mg et le plus préférablement d'au moins 10 U/mg, l'activité de l'unité étant préférablement estimée à $U_{mnt}$ ; ou
(E) comprenant un promédicament du composé selon l'une quelconque de la revendication 16 ou de (A) - (D) ci-dessus ; ou
(F) selon l'une quelconque de la revendication 16 ou de (A) - (E) ci-dessus, obtenu à partir de, ou pouvant être obtenu à partir d'une source naturelle, la source naturelle étant de préférence une espèce appartenant à la famille des Méliacées ; ou
(G) selon l'une quelconque des revendications 16 ou de (A) - (F) ci-dessus, obtenu par, ou pouvant être obtenu par synthèse chimique ou semi-synthèse chimique ; ou
(H) selon l'une quelconque de la revendication 16 ou de (A) - (G) ci-dessus, qui est synthétisé à partir d'une matière première provenant d'une source naturelle, la source naturelle étant de préférence une espèce appartenant à la famille des Méliacées ; ou
(I) selon l'une quelconque de la revendication 16 ou de (A) - (H) ci-dessus, obtenu par synthèse chimique ou semi-synthèse chimique, par un procédé qui, au moins en partie, implique une hydrolyse et/ou une estérification et/ou une alkylation ; ou
(J) selon l'une quelconque de la revendication 16 ou de (A) - (I) ci-dessus, obtenu à l'aide d'une matière première comprenant l'un quelconque d'un limonoïde, phragmaline, néobéguine, léandréanine A, léandréanine B, léandréanine C, pseudrelone A2, bussein A, B, C, D, E, F, G, H, J, K, L, M, le composé ayant éventuellement un effet de stimulation sexuelle ; ou
(K) selon l'une quelconque de la revendication 16 ou de (A) - (J) ci-dessus, sous forme radioactive.

**18.** Composition pharmaceutique

(A) comprenant le composé chimique selon l'une quelconque des revendications 16 et 17, éventuellement conjointement avec un ou plusieurs adjuvants, supports, dissolvants ou diluants pharmaceutiquement acceptables ; ou
(B) comprenant le composé chimique selon l'une quelconque des revendications 16 et 17, dans de l'huile, de la graisse et/ou des triglycérides pharmaceutiquement acceptables, qui contient de plus, éventuellement, des additifs, adjuvants, supports, dissolvants, conservateurs ou diluants pharmaceutiquement acceptables ;

préférablement, dans lequel la composition pharmaceutique de (A) ou (B) comprend en outre l'un quelconque d'une charge, d'un support, d'un excipient, d'un dissolvant, d'un diluant, d'un lubrifiant, d'un agent de glissement, d'un antioxydant et/ou d'un additif pharmaceutiquement acceptable, préférablement ceux qui sont choisis dans le groupe incluant l'huile (telle que l'huile d'arachide, l'huile de maïs, l'huile de sésame, l'huile de coton, l'huile d'olive, l'huile de soja), les triglycérides, les diglycérides, le beurre de cacao, la graisse de noix de coco, la graisse hydrogénée, l'huile animale hydrogénée, l'huile végétale hydrogénée, les triglycérides solides, les matières grasses solides, la graisse malléable, la Fattibase, les bases Wecobee, les bases Witespol, la cire d'ester cétylique, la cire d'abeille, le glycérol, le polysorbate 80, le polyéthylène glycol, le propylène glycol, l'alcool isopropylique, le stéarate de magnésium, l'acide stéarique, le talc, le dioxyde de silicium, le sulfate de lauryle, le glycolate d'amidon sodique, un détergent,
un mélange de détergent et d'huile, un mélange d'agent tensio-actif, l'huile émulsionnable, un système auto-émulsifiant, Neobee M5, le triester de triglycéride caprique/caprylique, le Miglyol 810, le propylène glycol dicaprylate, le Sefsol 228, les graisses végétales éthoxylées, l'éthanol, la polyvinylpyrrolidone, le Tween, le polyoxyéthylène sorbitol monolaurate, la triacétine, la diacétine, l'acide ascorbique, le palmitate d'ascorbyle, le butylhydroxyanisole, le butylhydroxytoluène, l'acide hypophosphoreux, le monothioglycérol, le gallate de propyle, l'ascorbate de sodium, le bisulfite de sodium, le formaldéhyde sulfoxylate de sodium, le sodium méta bisulfite, le butylparaben, le parahydroxybenzoate d'éthyle, l'acide benzoïque, le parahydroxybenzoate de propyle, le benzoate de sodium, le propionate de sodium, le chlorure de benzalkonium, le chlorure de benzéthonium, l'alcool benzylique, le chlorure de cétylpyridinium, le chlorobutanol, le phénol, l'alcool phényléthylique, le nitrate phénylmercurique, le thimérosal, le lactose, la cellulose microcristalline, l'amidon, le saccharose en poudre, le phosphate de calcium, la gomme arabique, l'acide alginique, la carboxyméthylcellulose sodique, le sucre compressible, l'éthylcellulose, la gélatine, le glucose liquide, la méthylcellulose, la povidone, l'amidon prégélatinisé, la croscarmellose, la crospovidone, de la résine échangeuse de cations, la silice, la silice colloïdale, l'amidon de maïs, le stéarate de calcium, l'huile minérale, l'huile d'anis, l'huile de cannelle, le cacao, le menthol, l'huile d'orange, l'huile de menthe poivrée, la vanilline, le gingembre, un colorant pharmaceutiquement acceptables,

et/ou dans lequel la composition pharmaceutique de (A) ou (B) est sous forme d'administrable par voie orale, administrable par voie parentérale, administrable par voie systémique, d'une gélule, d'un comprimé, d'une poudre, d'un granulé, d'un suppositoire, d'un insert, d'une pastille, d'un comprimé buccal, d'un comprimé sublingual, d'un comprimé compressé plusieurs fois, d'un comprimé moulé, d'un comprimé à croquer, d'un comprimé effervescent, d'une trituration en comprimé, d'un comprimé enrobé de sucre, d'un comprimé pelliculé, d'un comprimé à enrobage de gélatine, d'un comprimé à enrobage entérique, d'un comprimés de distributeur, d'un comprimé hypodermique, d'un comprimé à libération prolongée, d'un comprimé à désintégration immédiate, d'un comprimé à libération immédiate, d'une formulation dermique, d'une pommade, d'une crème, d'un gel, d'une formulation transdermique, d'une solutions, d'une teinture, d'une solution injectable, parentérale, d'un implant, d'une formulation pour administration urétrale, d'une formulations pour administration topique, d'une solution ophtalmique, d'une formulation vaginale, d'un inhalant, d'un système dispersé, d'une émulsion, d'un système à comprimés multiples, d'un système de médicament microencapsulé, d'une pompe osmotique, d'un implant sous-cutané, d'un système oculaire, d'un système parentérale, d'un système vaginal, d'un système à granules enrobées, d'une granule, d'une microsphère, d'un système à libération modifiée, d'un système à libération prolongée, d'un système à libération retardée, d'un système à action répétée, d'un système de libération ciblée, d'un système de résine échangeuse d'ions, d'un système à érosion lente et/ou à matrice hydrophile, d'un système noyé à matrice en matière plastique inerte, d'une pommade, d'une crème, d'un gel, d'une solution, d'une solution d'eau, d'une teinture, d'une solution d'huile,
d'une pilule.

**19.** Tissu racinaire de *Neobeguea spp.* ou un extrait de celle-ci, pour une utilisation comme médicament ou en thérapie ; ou
tissu racinaire de *Neobeguea spp.* ou un extrait de celle-ci, pour une utilisation dans le traitement de l'un quelconque d'un dysfonctionnement sexuel, d'un dysfonctionnement érectile, de troubles de l'éjaculation ou d'un trouble du désir sexuel hypoactif, préférablement dans lequel la composition est administrée par voie systémique ou par voie non-topique.

**20.** Composition selon l'une quelconque des revendications de 13 à 15 ou 18, ou un composé chimique tel que revendiqué dans l'une quelconque des revendications 16 ou 17 pour une utilisation

(A) en tant que médicament ou pour une utilisation thérapeutique ; ou
(B) pour une utilisation dans le traitement de l'un quelconque d'un dysfonctionnement sexuel, d'un dysfonctionnement érectile, de troubles de l'éjaculation ou d'un trouble du désir sexuel hypoactif, préférablement dans lequel la composition (A) ou (B) est administrée par voie systémique ou par voie non-topique.

**21.** Procédé non médical pour induire un effet de stimulation sexuelle, comprenant l'administration à un sujet

(A) d'une quantité pharmaceutiquement efficace d'un tissu racinaire de *Neobeguea spp.* ou d'un extrait de celle-ci ; ou
(B) d'une quantité pharmaceutiquement efficace d'une composition telle que revendiquée dans l'une quelconque des revendications 13 à 15 ou 18, ou d'un composé chimique tel que revendiqué dans l'une quelconque des revendications 16 ou 17 ;\

préférablement, dans lequel le tissu, l'extrait ou la composition est administrée par voie systémique ou par voie non-topique.

**22.** Utilisation de

(A) un tissu racinaire de *Neobeguea spp.* ou un extrait de celle-ci, dans la fabrication d'un médicament destiné au traitement de l'un quelconque d'un dysfonctionnement sexuel, d'un dysfonctionnement érectile, de troubles de l'éjaculation ou d'un trouble du désir sexuel hypoactif ; ou
(B) une composition telle que revendiquée dans l'une quelconque des revendications 13 à 15 ou 18, ou un composé chimique tel que revendiqué dans l'une quelconque des revendications 16 ou 17, dans la fabrication d'un médicament destiné au traitement de l'un quelconque d'un dysfonctionnement sexuel, d'un dysfonctionnement érectile, de troubles de l'éjaculation ou d'un trouble du désir sexuel hypoactif,

préférablement dans lequel le tissu, l'extrait ou la composition (A) ou (B) est administré par voie systémique ou par voie non-topique.

**23.** Utilisation de

(A) un tissu racinaire de *Neobeguea spp.* ou un extrait de celle-ci, pour induire un effet de stimulation sexuelle, ou
(B) une composition telle que revendiquée dans l'une quelconque des revendications 13 à 15 ou 18, ou un composé chimique tel que revendiqué dans l'une quelconque des revendications 16 ou 17, pour induire un effet de stimulation sexuelle,

préférablement dans lequel le tissu, l'extrait ou la composition (A) ou (B) est administré par voie systémique ou par voie non-topique, lorsque l'utilisation est un utilisation non médicale.

Figure 1

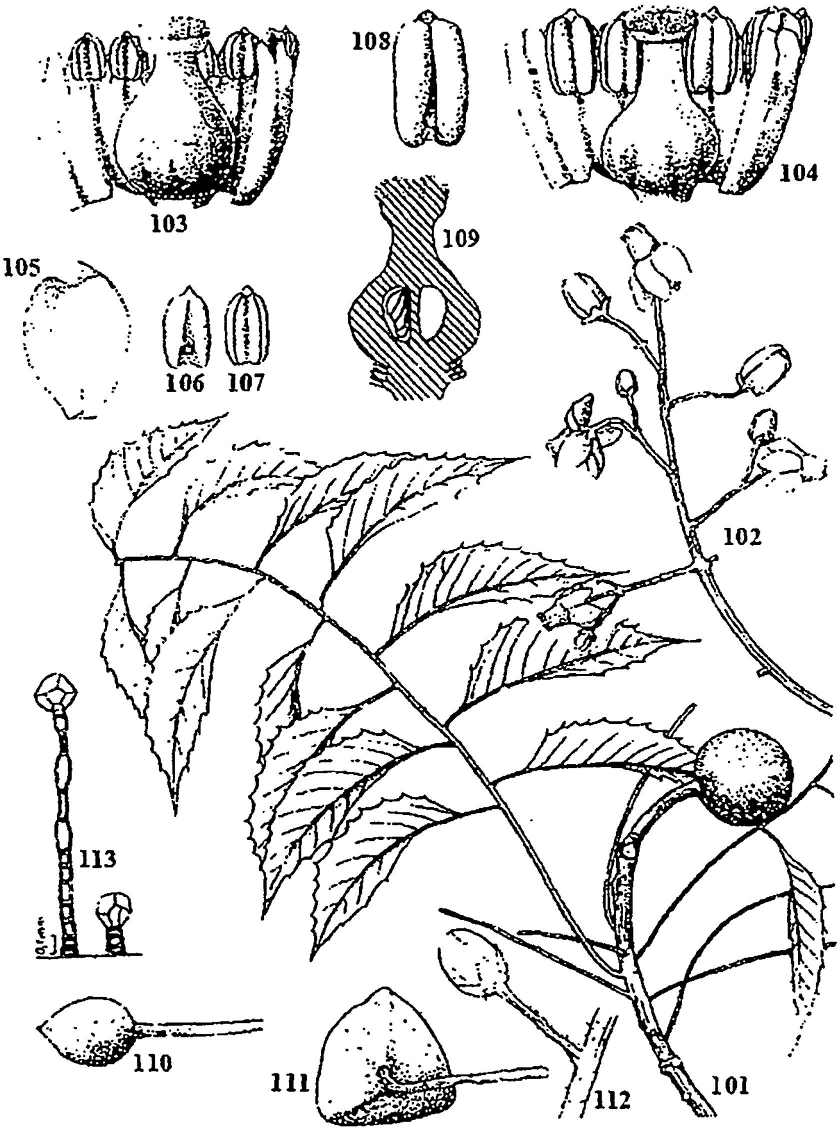

**Figure 2**

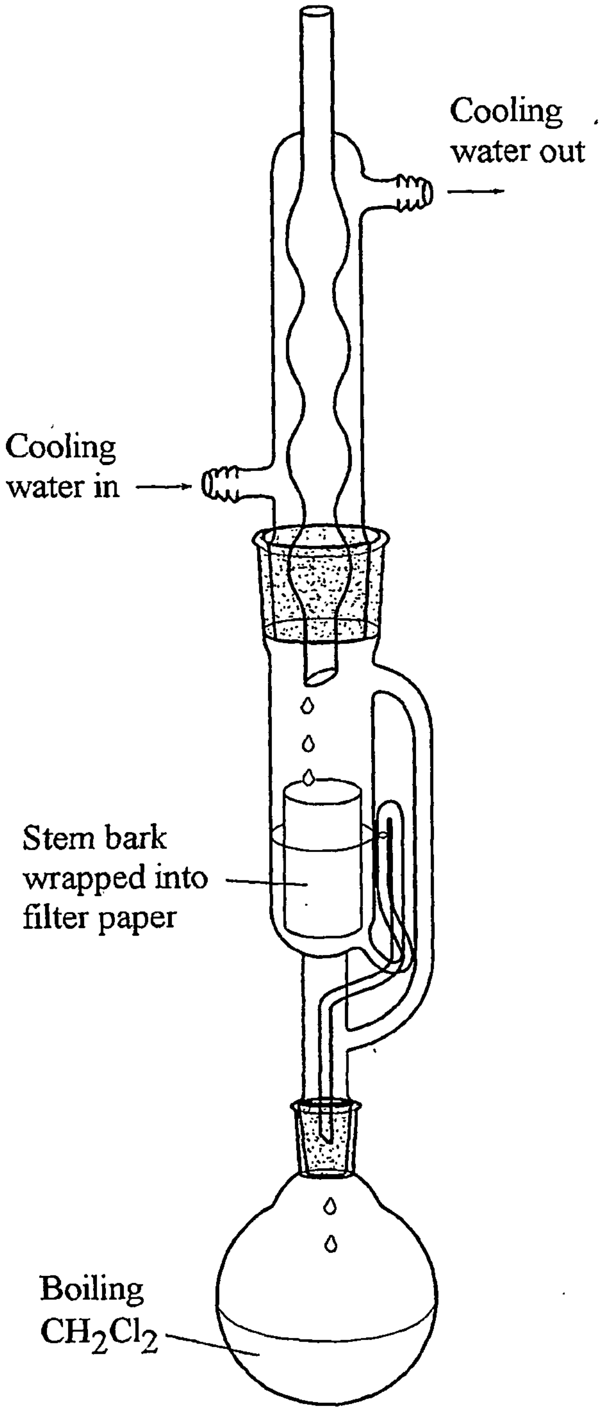
Cooling
water out
Cooling
water in
Stem bark
wrapped into
filter paper
Boiling
$CH_2Cl_2$

Figure 3

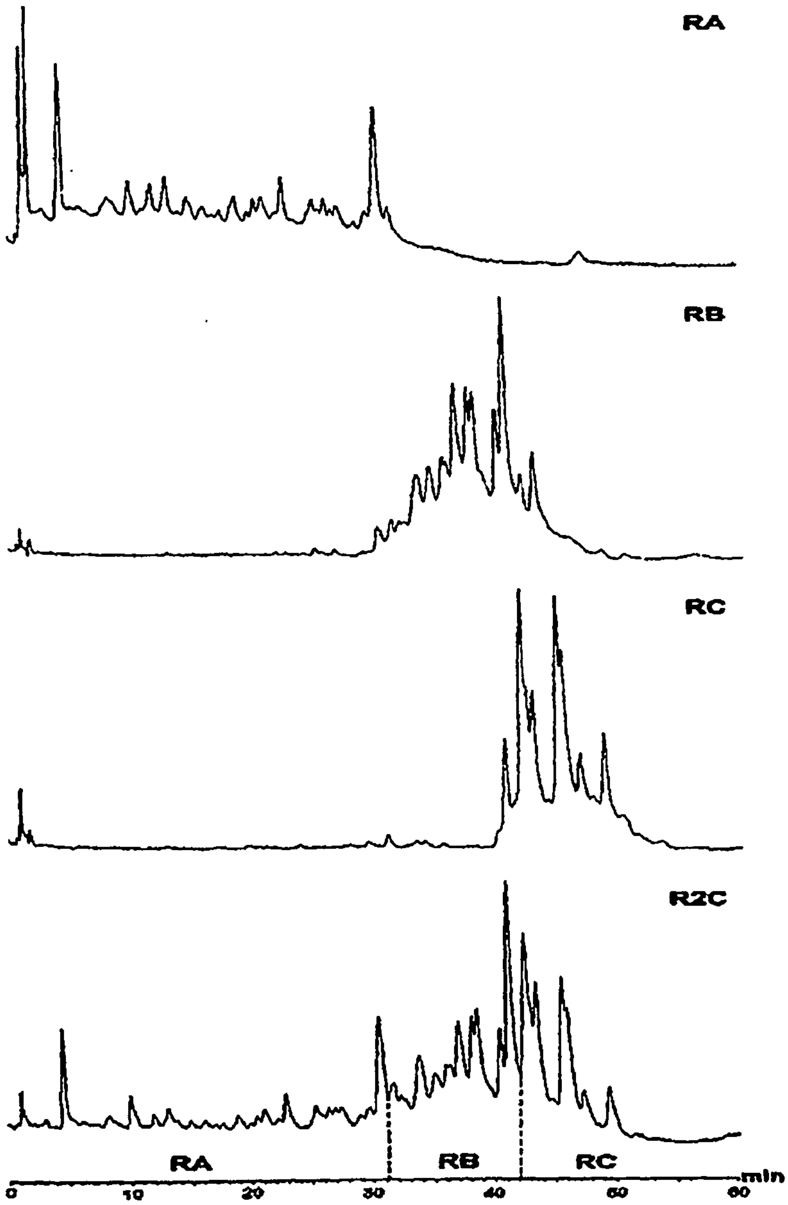

**Figure 4**

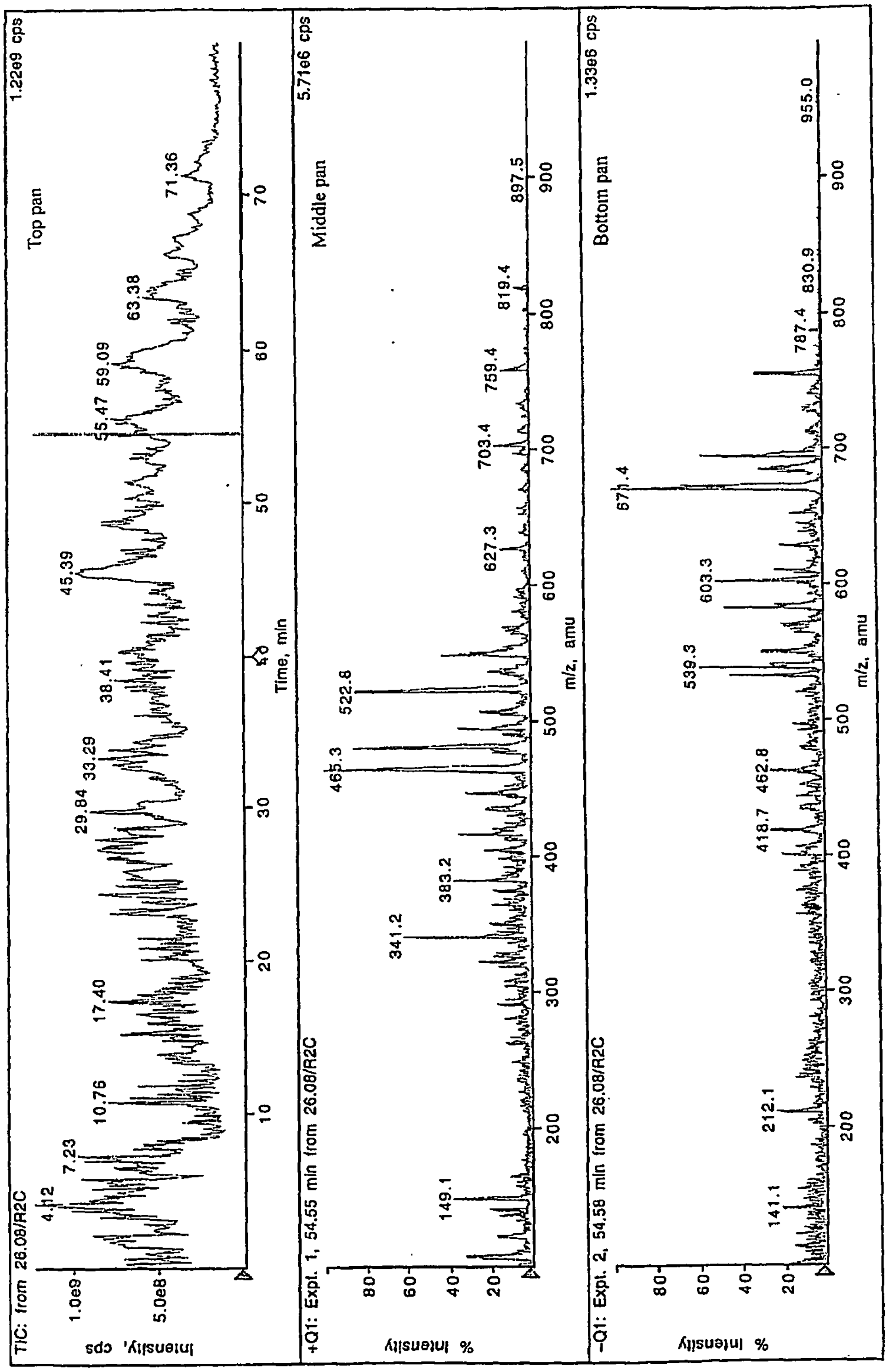

TIC: from 26.08/R2C
1.22e9 cps
Top pan
Intensity, cps
1.0e9
5.0e8
4.12
7.23
10.76
17.40
29.84
33.29
38.41
45.39
55.47
59.09
63.38
71.36
Time, min
+Q1: Expl. 1, 54.55 min from 26.08/R2C
5.71e6 cps
Middle pan
% Intensity
149.1
341.2
383.2
465.3
522.8
627.3
703.4
759.4
819.4
897.5
m/z, amu
-Q1: Expl. 2, 54.58 min from 26.08/R2C
1.33e6 cps
Bottom pan
% Intensity
141.1
212.1
418.7
462.8
539.3
603.3
671.4
787.4
830.9
955.0
m/z, amu

# Figure 5

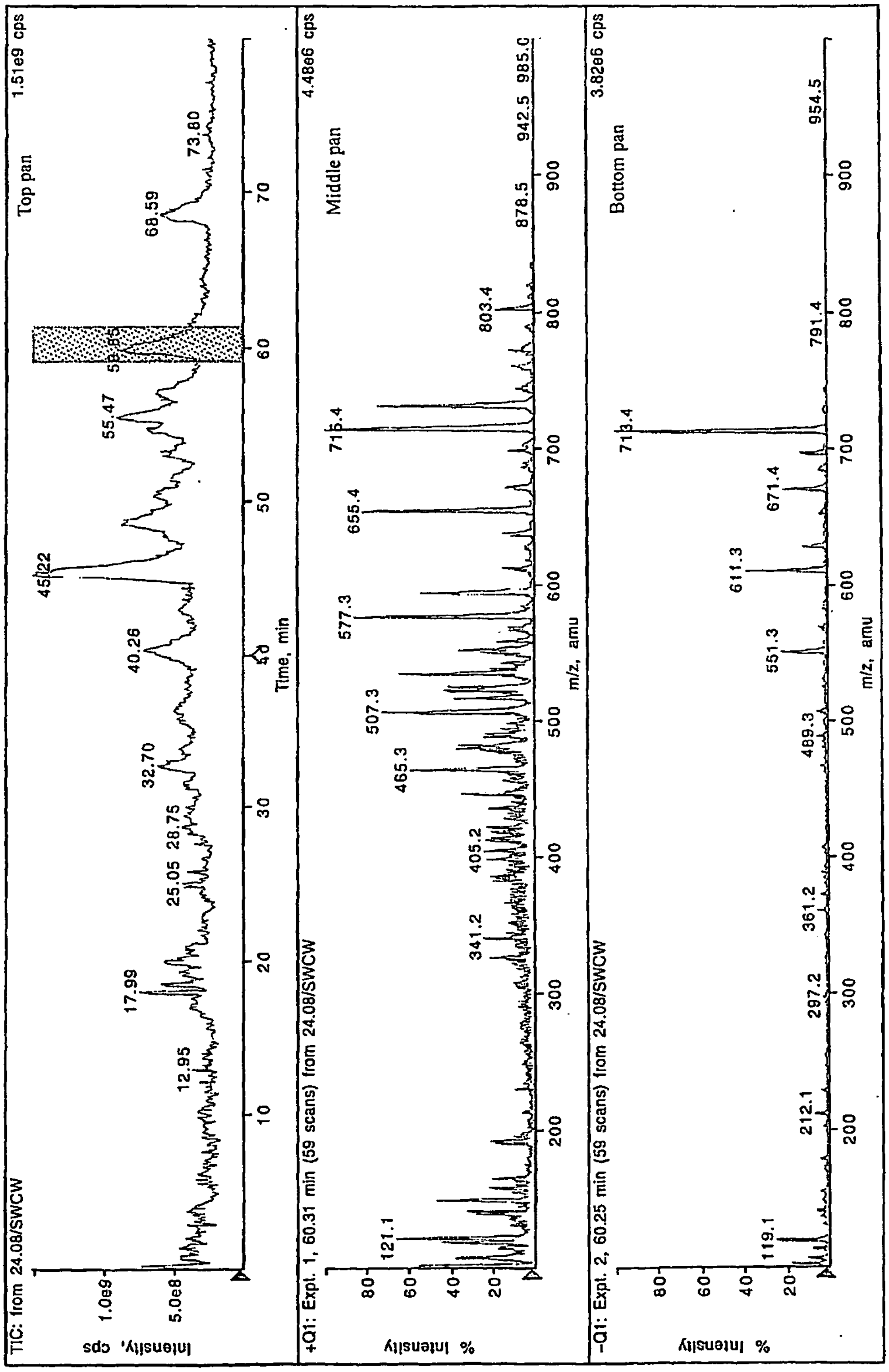
TIC: from 24.08/SWCW
1.51e9 cps
Top pan
Intensity, cps
1.0e9
5.0e8
12.95
17.99
25.05
28.75
32.70
40.26
45.22
55.47
59.85
68.59
73.80
Time, min
+Q1: Expt. 1, 60.31 min (59 scans) from 24.08/SWCW
4.48e6 cps
Middle pan
% Intensity
121.1
341.2
405.2
465.3
507.3
577.3
655.4
715.4
803.4
878.5
942.5
985.0
m/z, amu
-Q1: Expt. 2, 60.25 min (59 scans) from 24.08/SWCW
3.82e6 cps
Bottom pan
% Intensity
119.1
212.1
297.2
361.2
489.3
551.3
611.3
671.4
713.4
791.4
954.5
m/z, amu

**Figure 6**

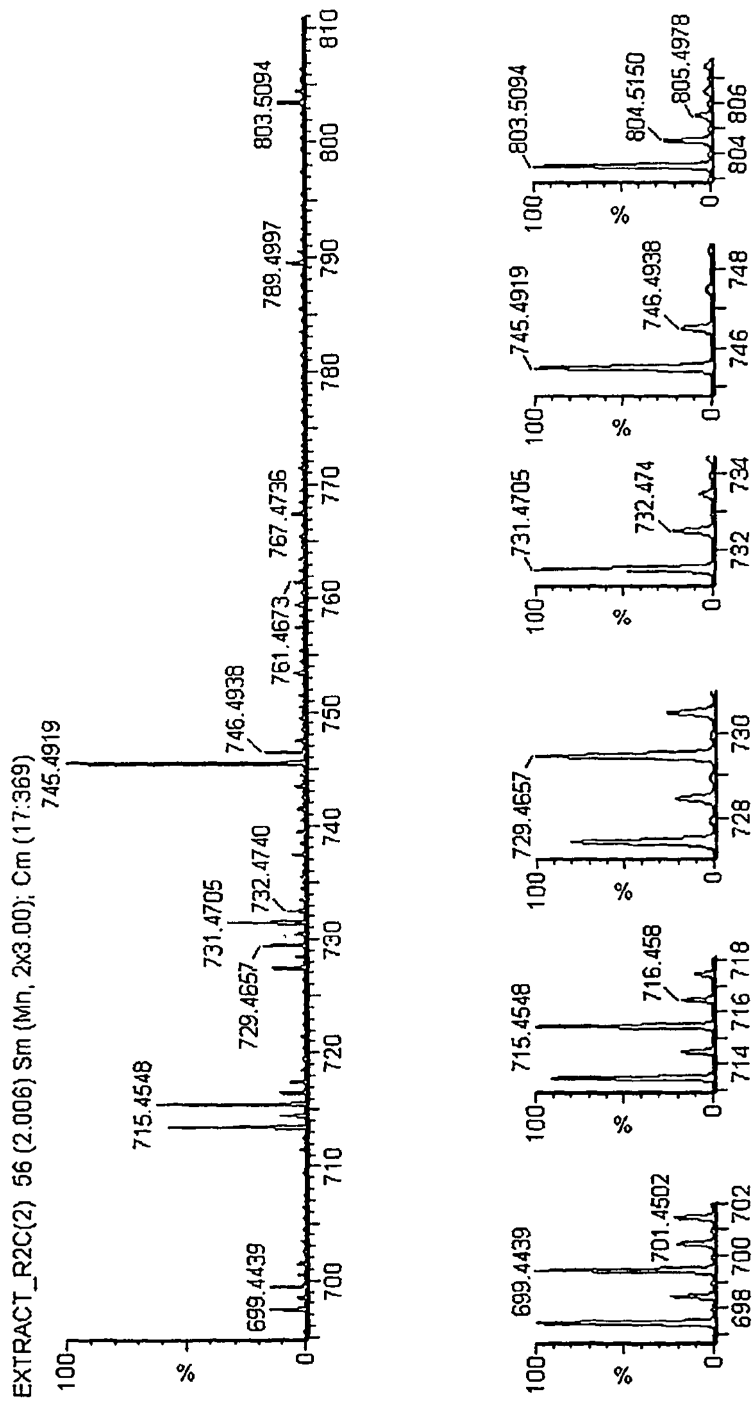
EXTRACT_R2C(2) 56 (2.006) Sm (Mn, 2x3.00); Cm (17:369)
745.4919
715.4548
699.4439
729.4657
731.4705
732.4740
746.4938
761.4673
767.4736
789.4997
803.5094
701.4502
716.458
731.4705
732.474
746.4938
804.5150
805.4978
100
%
0

**Figure 7**

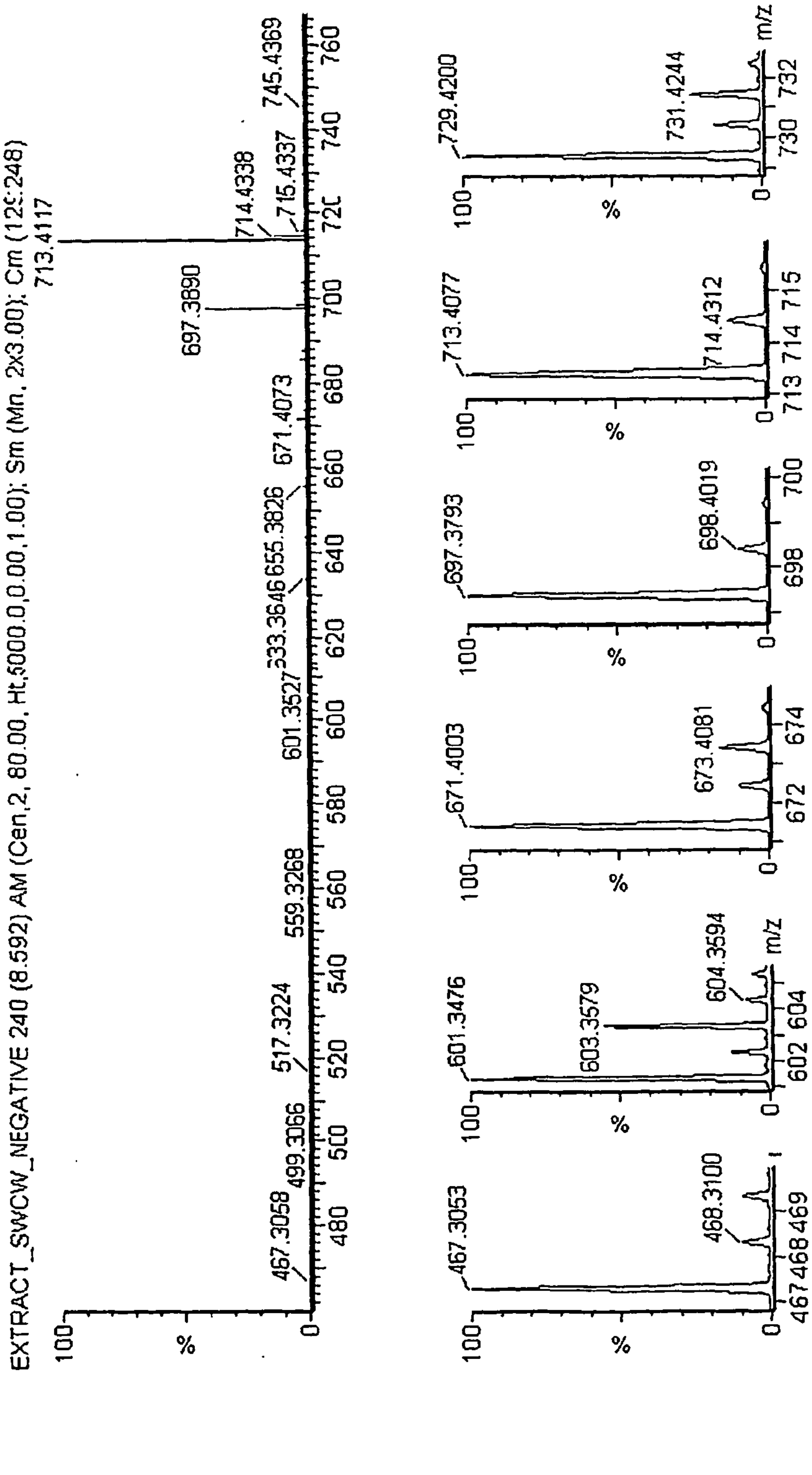
EXTRACT_SWCW_NEGATIVE 240 (8.592) AM (Cen,2, 80.00, Ht,5000.0,0.00,1.00); Sm (Mn, 2x3.00); Cm (129:248)
713.4117
697.3890
714.4338
715.4337
745.4369
671.4073
655.3826
633.3646
601.3527
559.3268
517.3224
499.3066
467.3058
467.3053
468.3100
601.3476
603.3579
604.3594
671.4003
673.4081
697.3793
698.4019
713.4077
714.4312
729.4200
731.4244
%
m/z

**Figure 8**

| Peak number | Retention time, min | UV maxima (nm) | Positive ionization (%) | | Negative ionization (%) | |
|---|---|---|---|---|---|---|
| | | | ESI | APPI | ESI | APPI |
| 1 | 31.59 | 191, 199, 214, 260 (shoulder) | 687.2684 | 687.2720 | 713.2845 (100)<br>697.2579 (70) | 635.2375 (100)<br>685.2584 (60)<br>701.2443 (60) |
| 2 | 32.51 | 199, 216, 260 (shoulder) | 781.2695 (100)<br>757.3043 (30)<br>777.3041 (30) | 757.3044 (100)<br>777.2954 (100)<br>833.2825 (100) | 715.2991 (100)<br>713.2837 (100)<br>697.2562 (100) | 731.2914 (100) |
| 3 | 33.90 | 199, 216, 260 | 713.2837 (100) | 663.4538 (100)<br>713.2867 (15)<br>731.2950 (15) | 699.2670 (100)<br>717.2317 (60)<br>729.2712 (60) | 631.2452 (100)<br>729.2827 (60) |
| 4 | 34.46 | 199, 216, 260 (shoulder) | 759.2906 (100)<br>729.2780 (30) | 607.5648 (100)<br>729.2770 (20)<br>759.2868 (10)<br>815.3124 (10) | 671.2945 | 715.2625 (100)<br>677.2615 (50)<br>727.2664 (40) |
| 5 | 34.96 | 198, 214, 263 (shoulder) | 697.2883 | 697.2856 (100)<br>759.2897 (30)<br>683.2740 (30) | 713.2840 (100)<br>671.2746 (40) | 645.2507 (100) |
| 6 | 35.16 | 198, 214, 256 | 761.3022 (100)<br>703.2987 (20) | 761.3002 (100)<br>703.2965 (60) | 697.2562 (100)<br>655.2738 (80)<br>713.2880 (70) | 745.2903 (100)<br>703.2686 (80)<br>701.2740 (60)<br>759.2914 (60)<br>685.2657 (60) |
| 7 | 35.91 | 198, 216, 266 | 727.2975 | 727.3070 (100)<br>761.3231 (60) | 743.2921 | 743.2920 (100)<br>601.2740 (100) |

**Figure 9**

| | | | | | | |
|---|---|---|---|---|---|---|
| 7 (continued) | | (shoulder) | | 629 3130 (40) | | 583.2593 (90)<br>549 2480 (80) |
| 8 | 36.46 | 198, 211, 255 | 715 2982 | 715 2943 (100)<br>583 2175 (50)<br>699 2992 (50) | 713 2840 | 663.2630 (100)<br>643 2410 (75)<br>713.2805 (70)<br>729.2802 (50)<br>681.2554 (50)<br>610.2440 (50) |
| 9 | 36.84 | 198, 216, 258 | 699.3026 | 699.2991 (100)<br>715 2953 (30) | 713 2840 | 647.2702 (100)<br>685.2828 (30)<br>717 2990 (25)<br>701.2825 (25) |
| 10 | 39 83 | 193, 198, 219,<br>267 (shoulder) | 727 3017 | 741 3110 (100)<br>727 2964 (40) | 725 2812 | 615 2123 (100) |

**Figure 10**

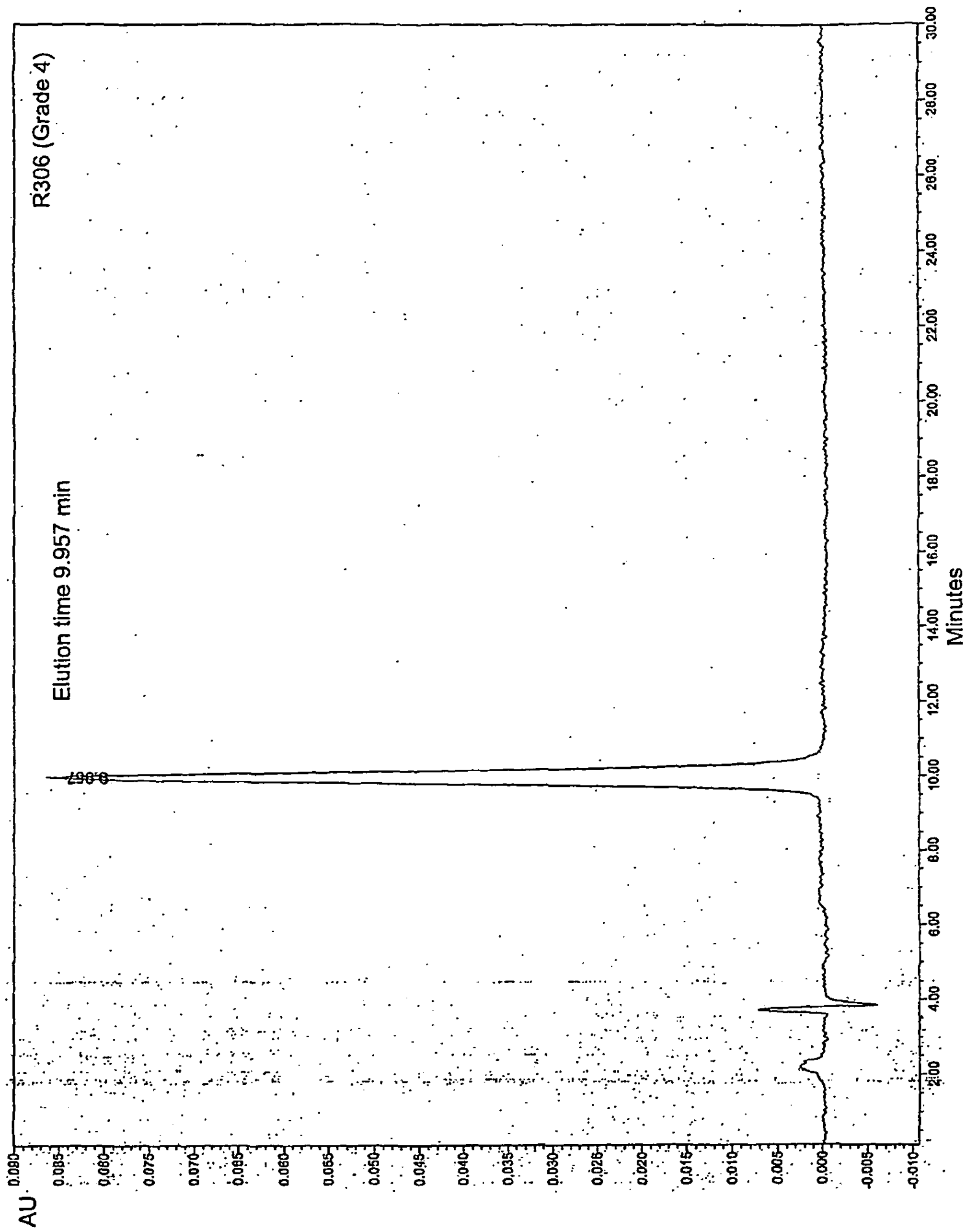

**Figure 11**

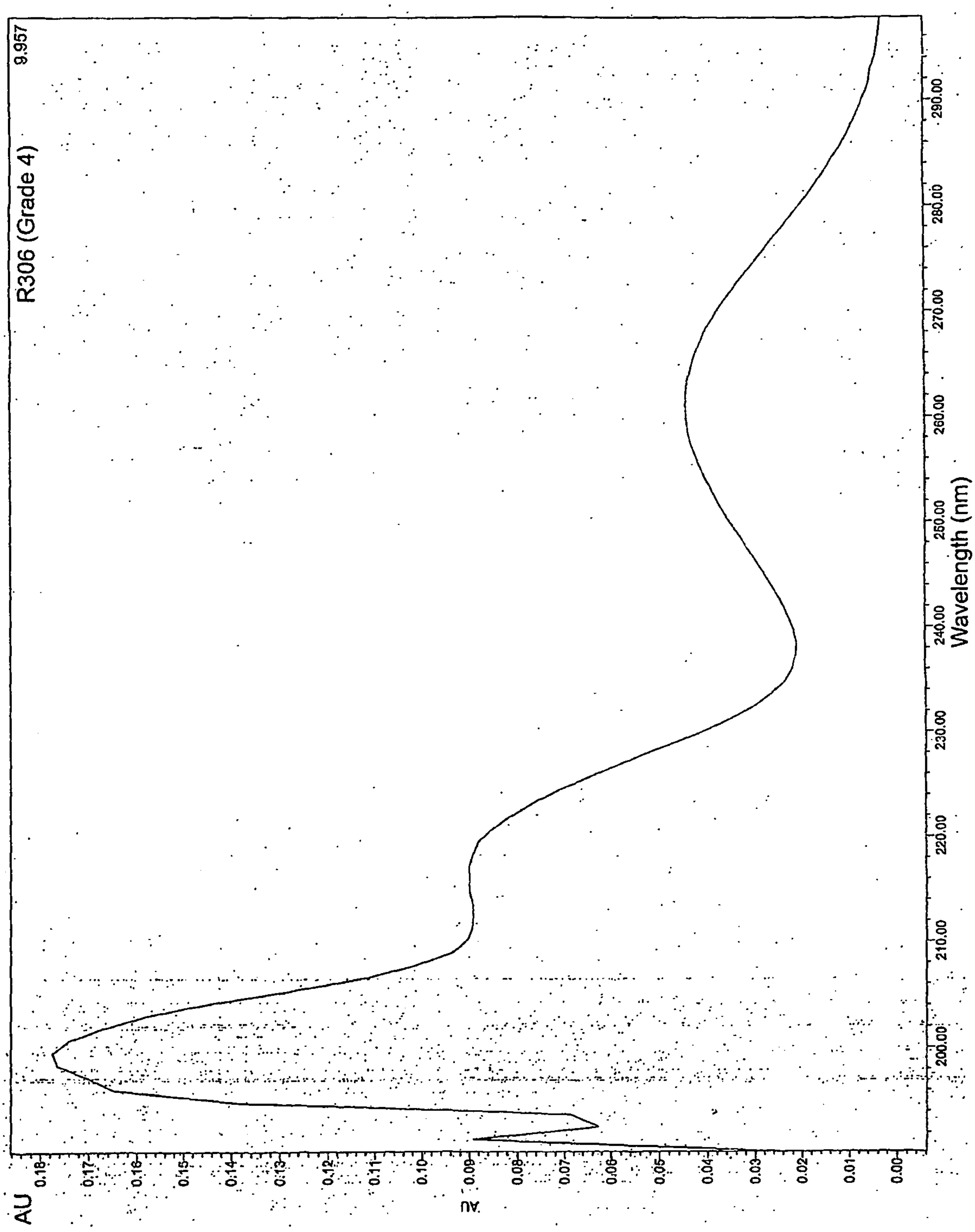
R306 (Grade 4)
9.957
AU
0.18
0.17
0.16
0.15
0.14
0.13
0.12
0.11
0.10
0.09
0.08
0.07
0.06
0.05
0.04
0.03
0.02
0.01
0.00
200.00
210.00
220.00
230.00
240.00
250.00
260.00
270.00
280.00
290.00
Wavelength (nm)

**Figure 12**

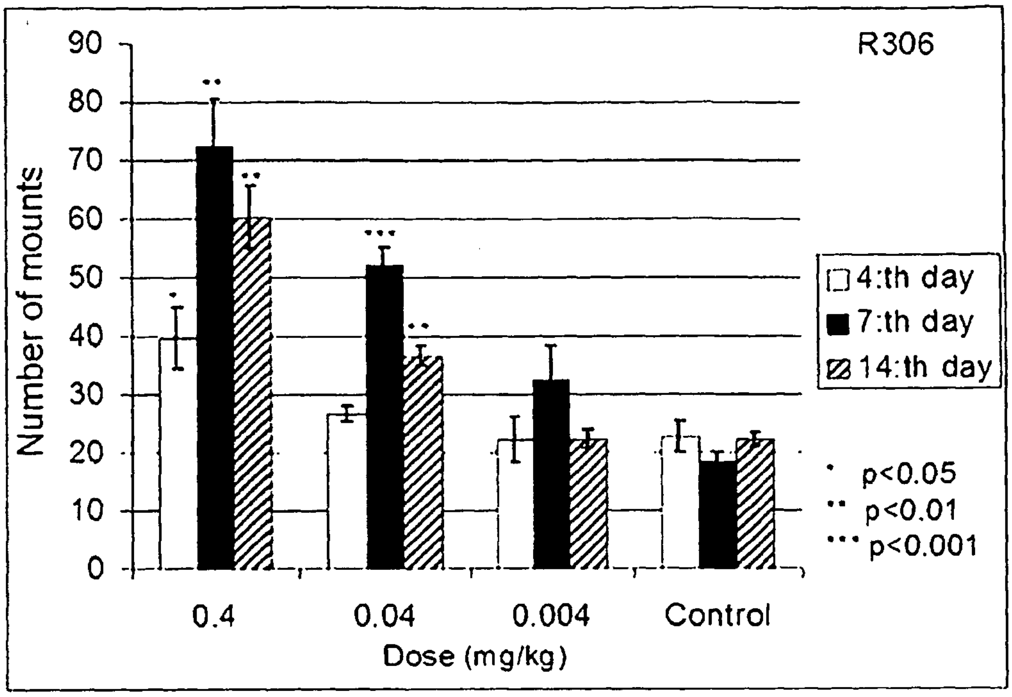
R306
Number of mounts
90
80
70
60
50
40
30
20
10
0
0.4
0.04
0.004
Control
Dose (mg/kg)
4:th day
7:th day
14:th day
* p<0.05
** p<0.01
*** p<0.001

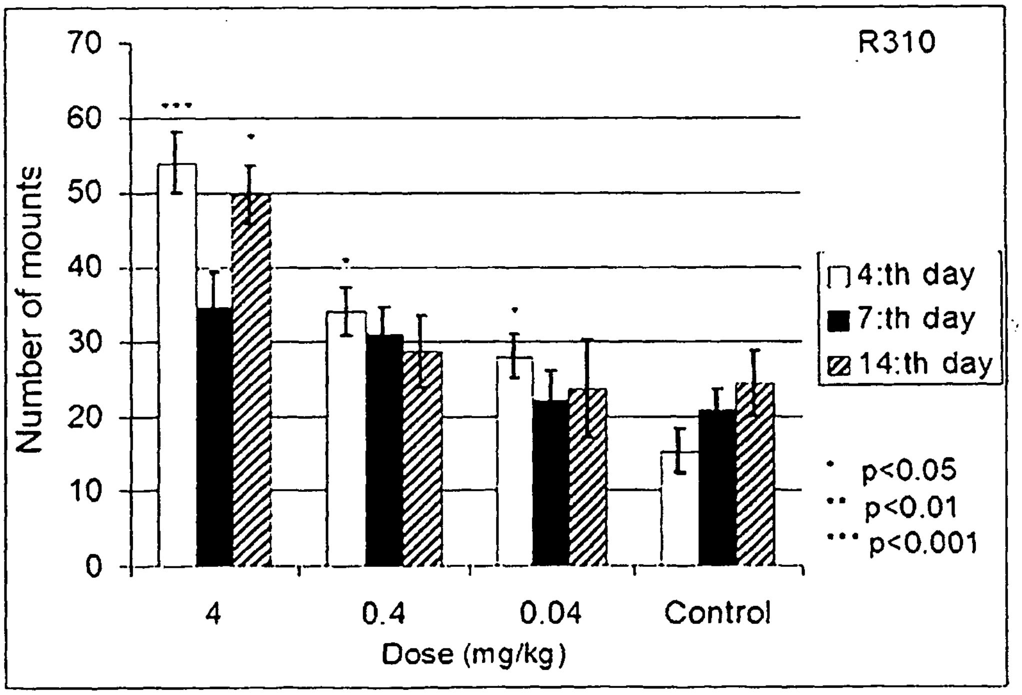
R310
Number of mounts
70
60
50
40
30
20
10
0
4
0.4
0.04
Control
Dose (mg/kg)
4:th day
7:th day
14:th day
* p<0.05
** p<0.01
*** p<0.001

**Figure 13**

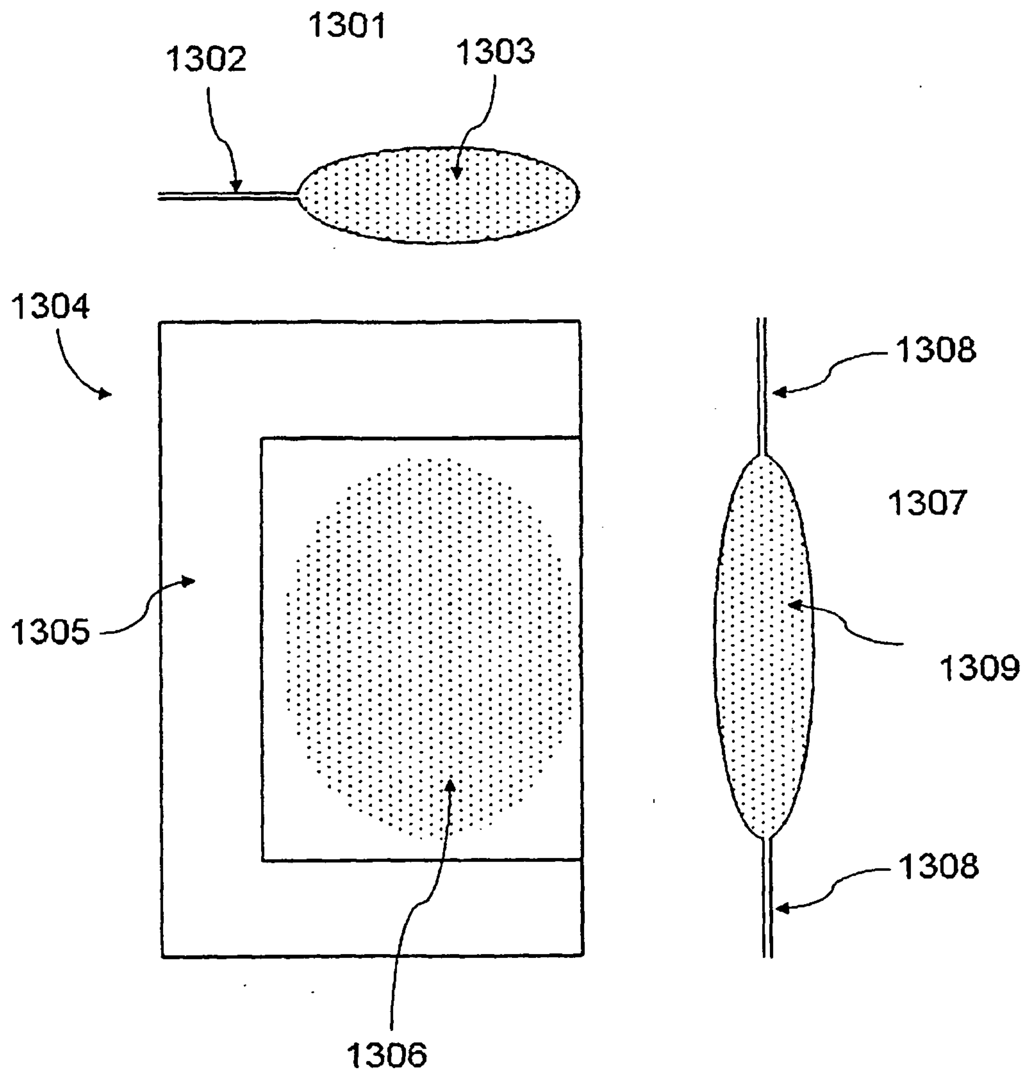

Figure 14

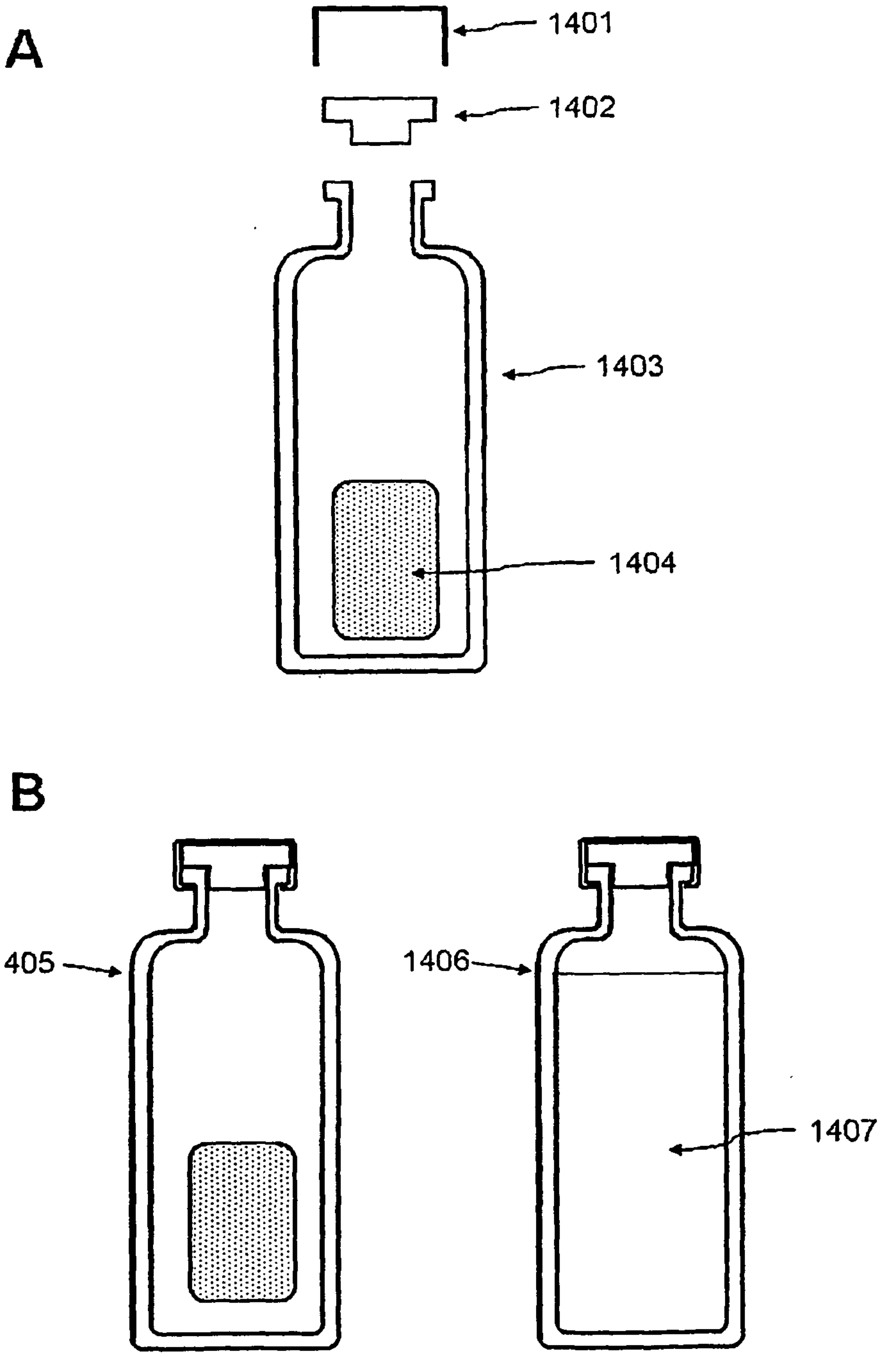

Figure 15

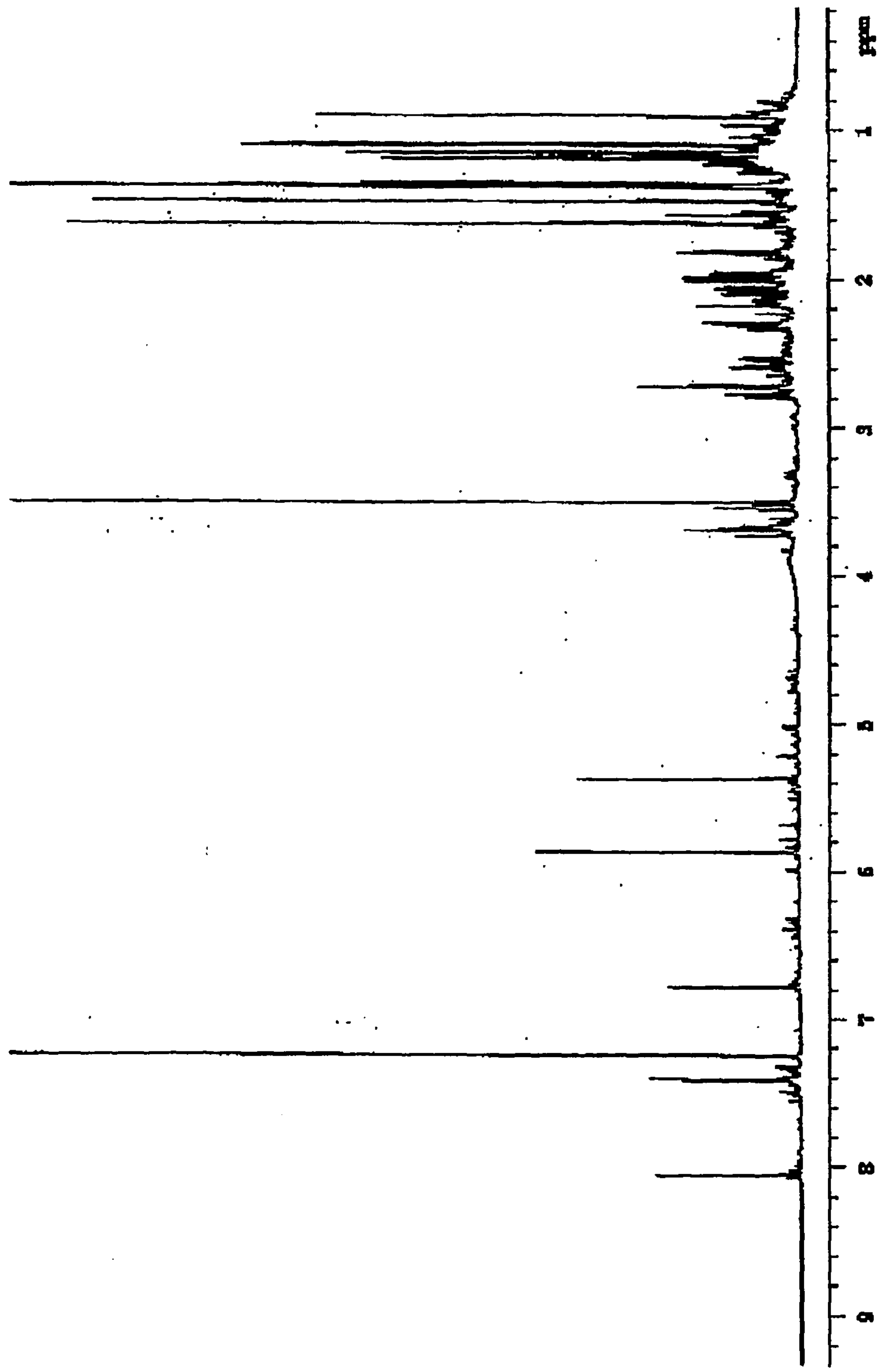

**Figure 16**

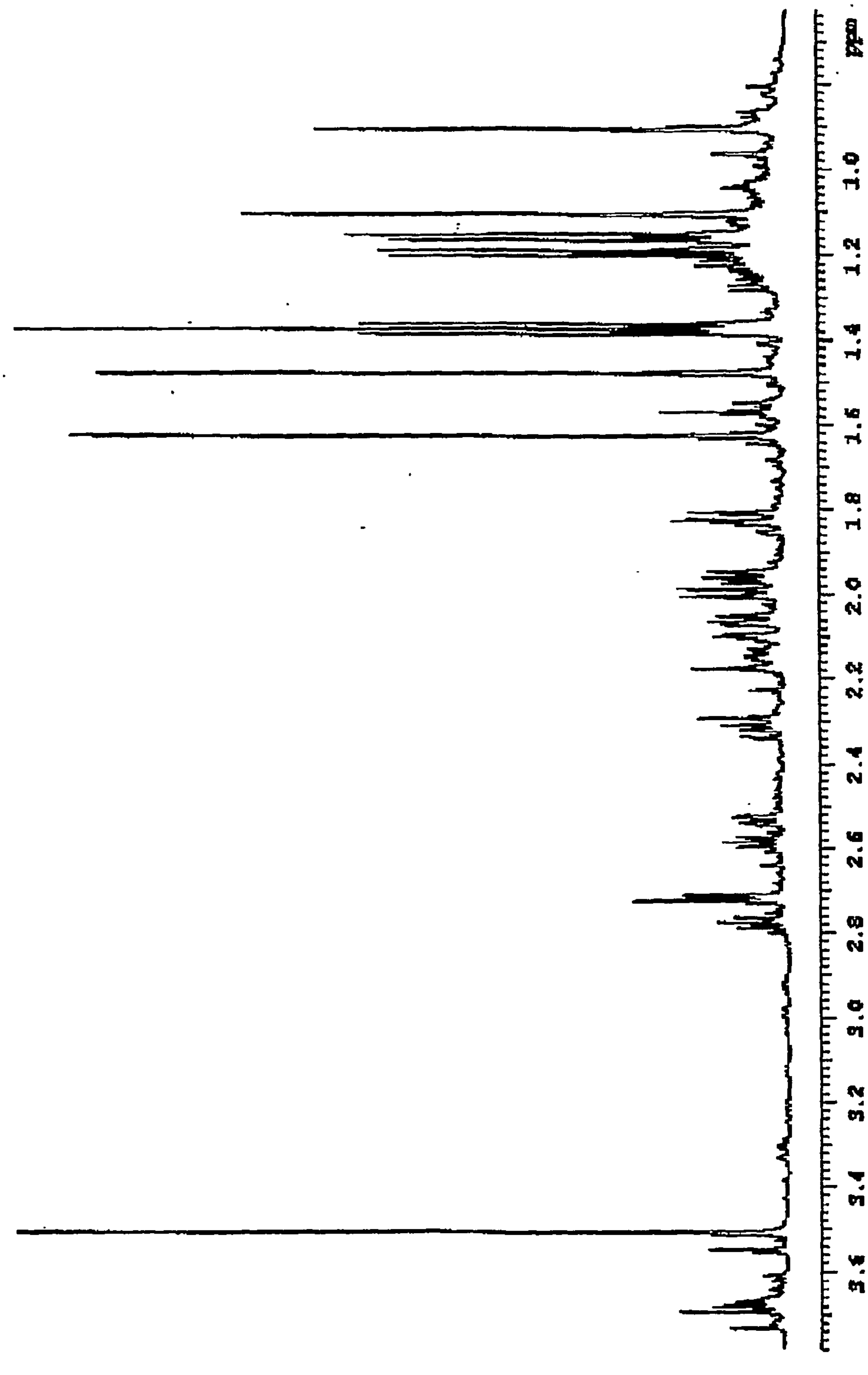

**Figure 17**

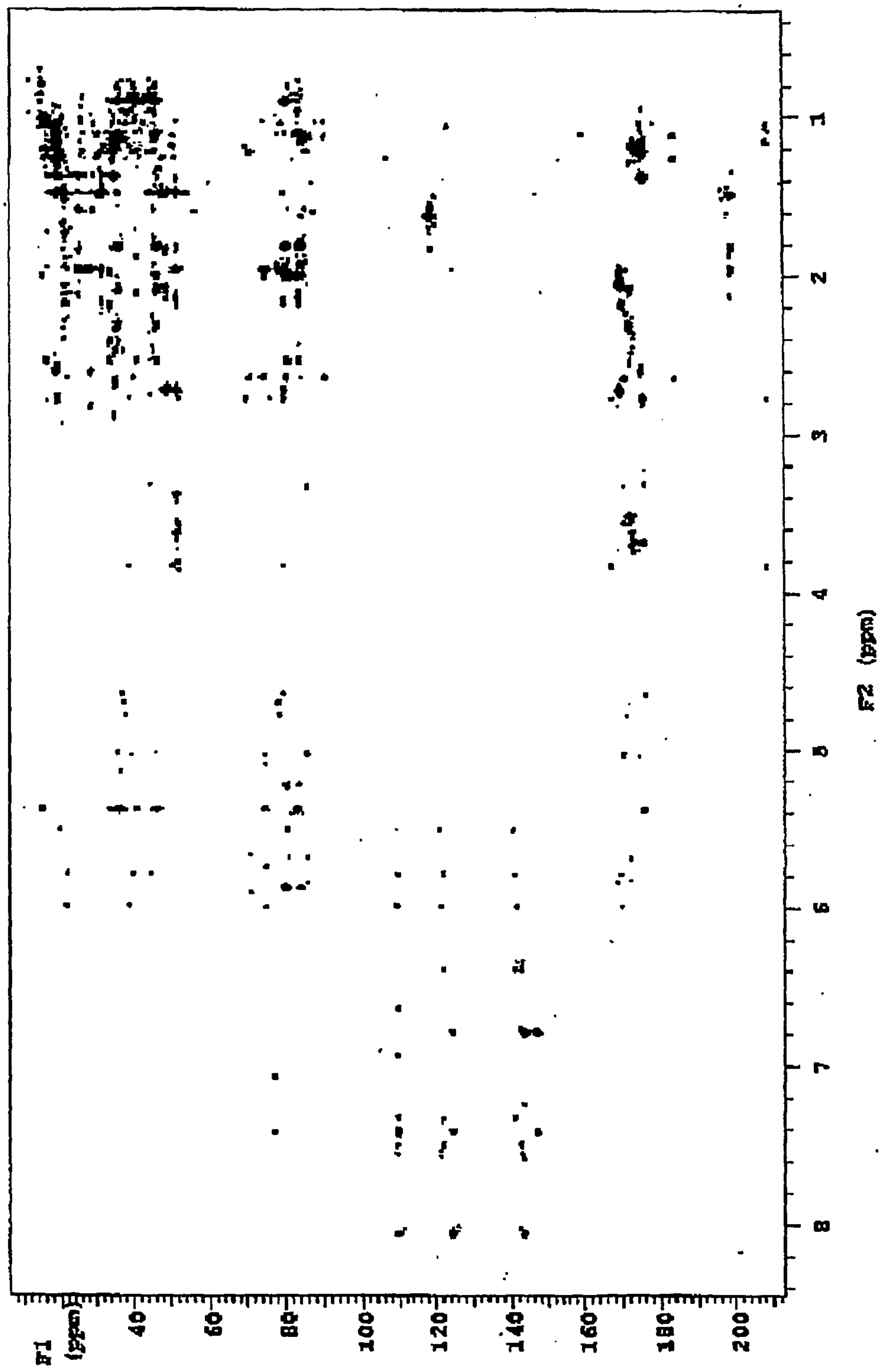

Figure 18

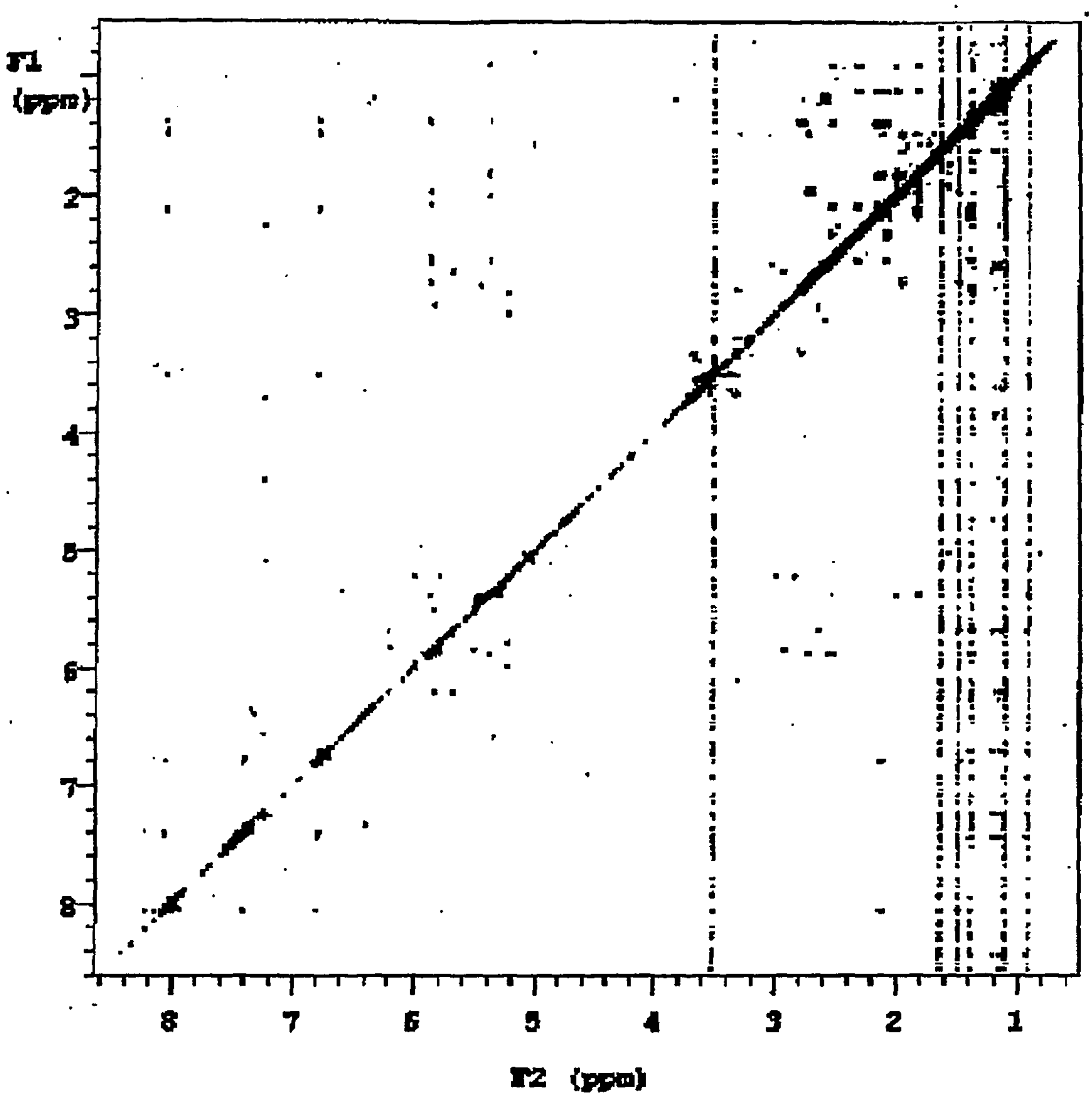

# Figure 19

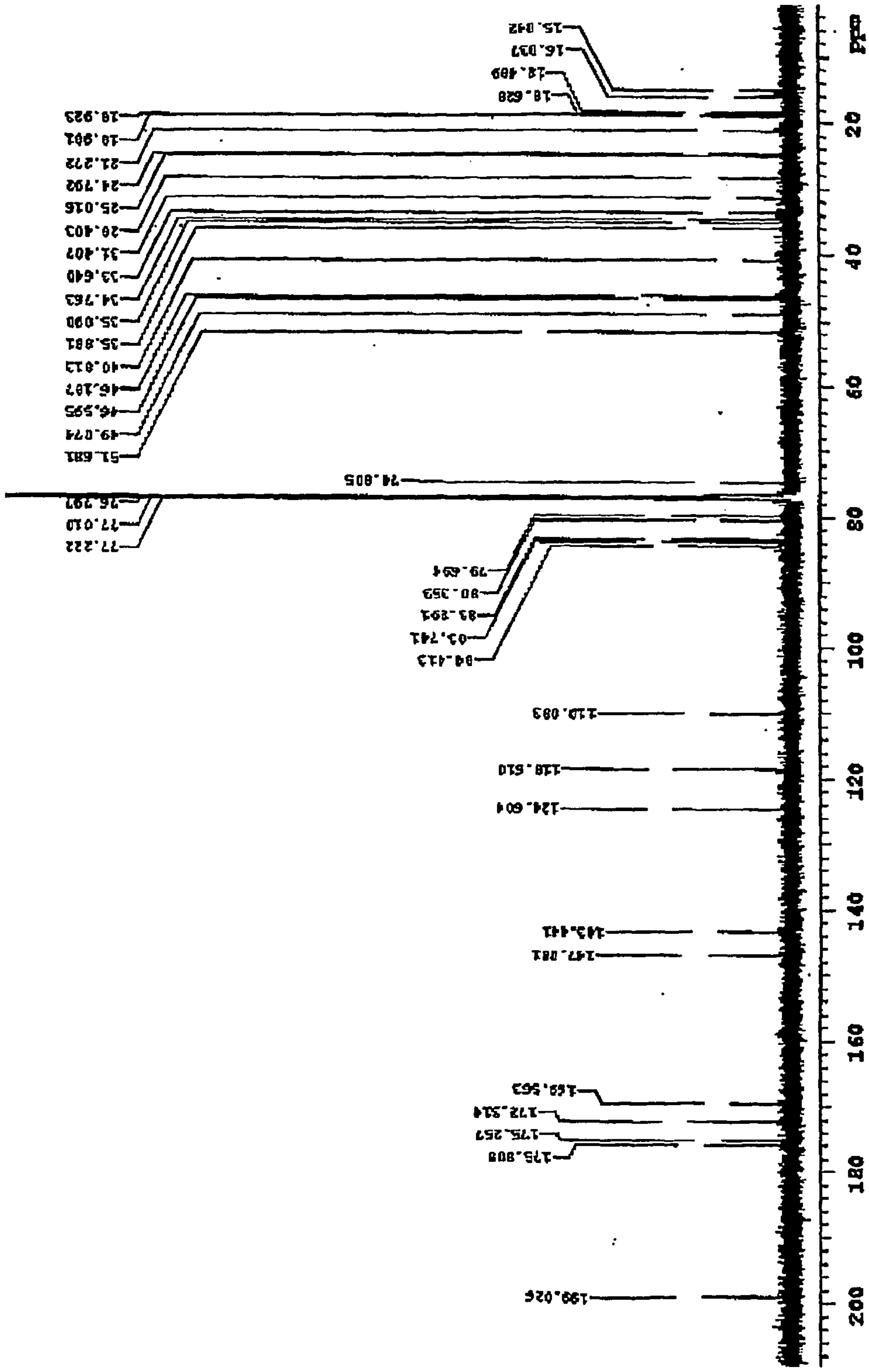

**Figure 20**

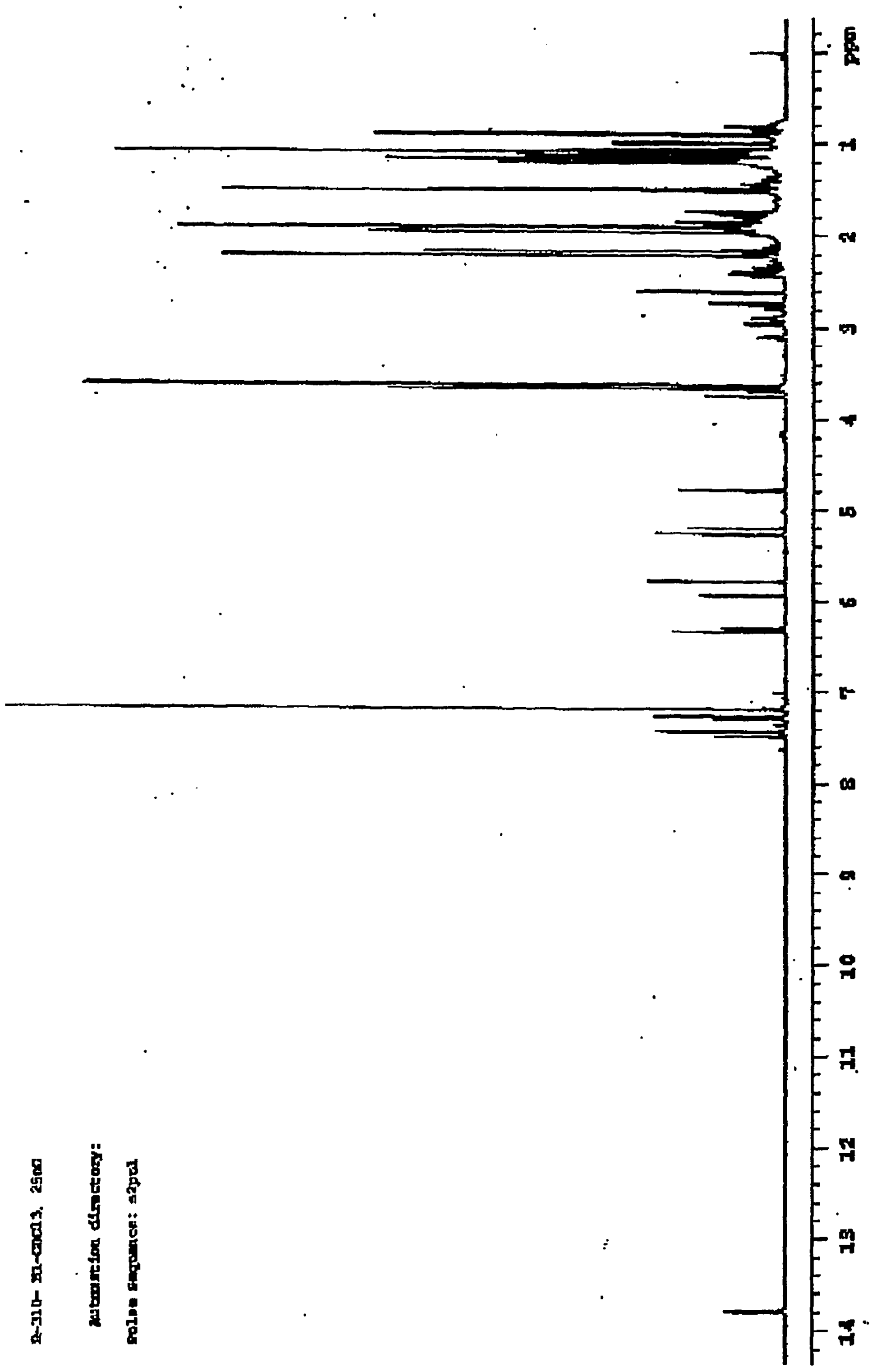

**Figure 21**

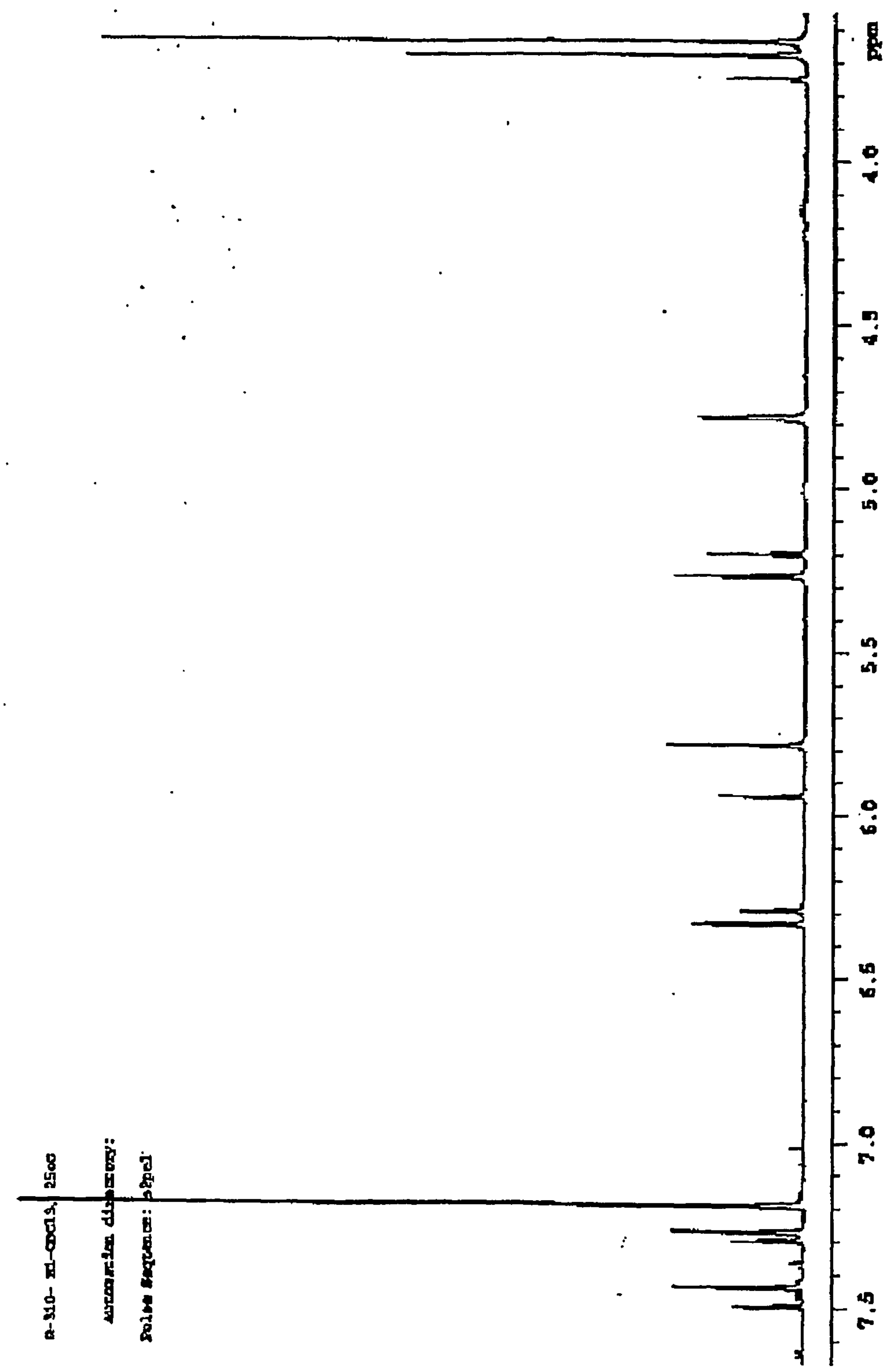

# Figure 22

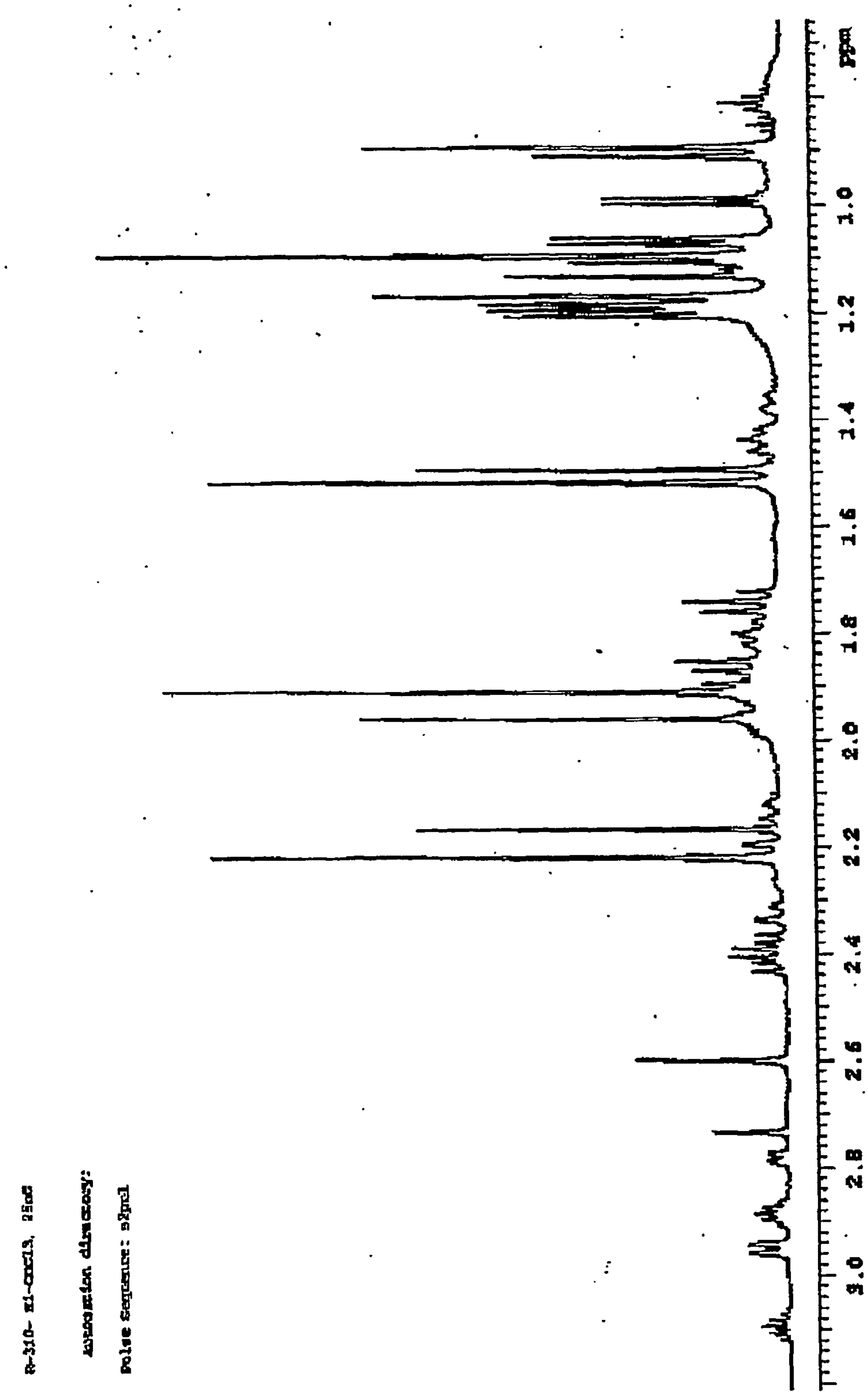

**Figure 23**

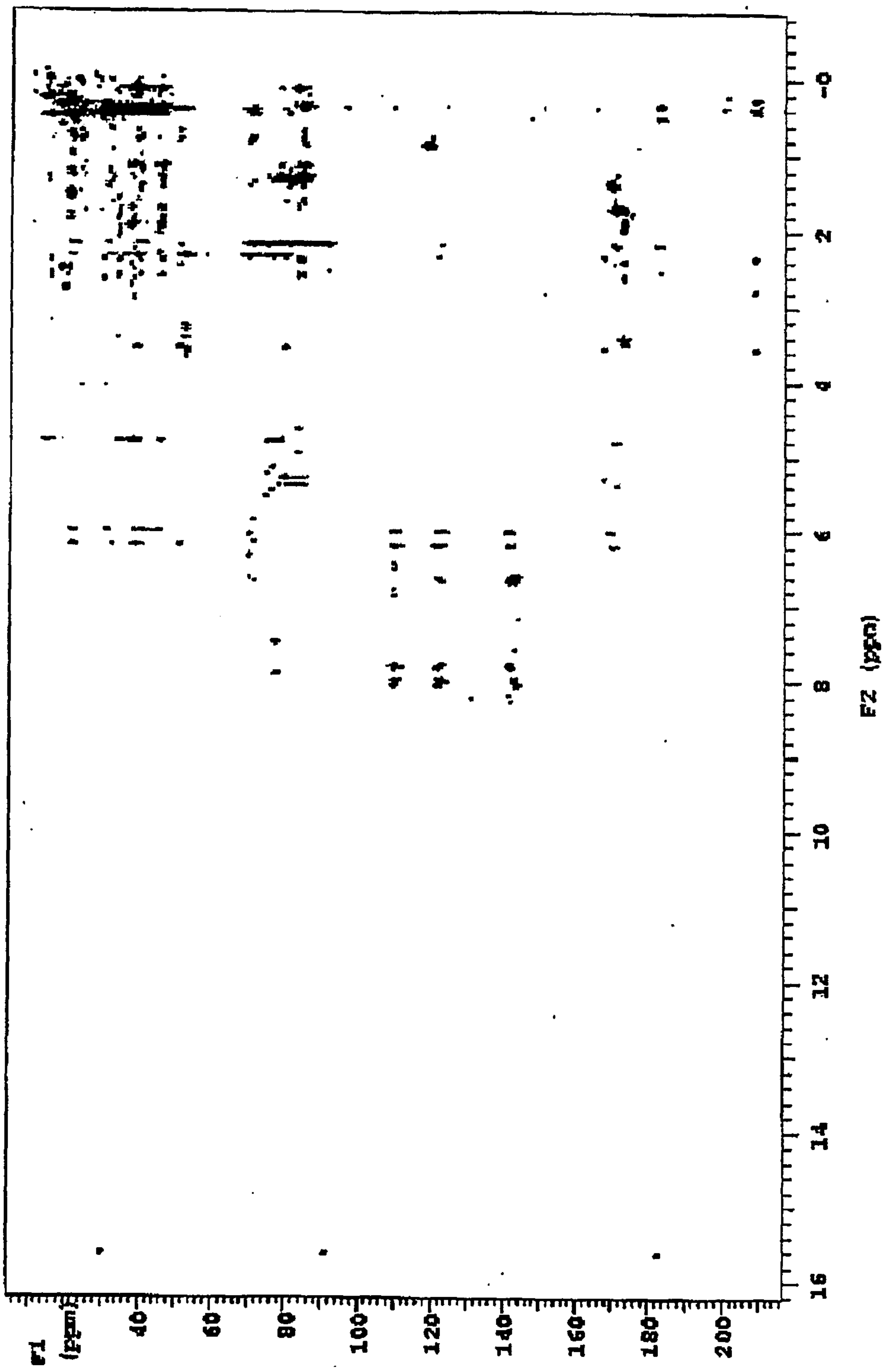

Figure 24

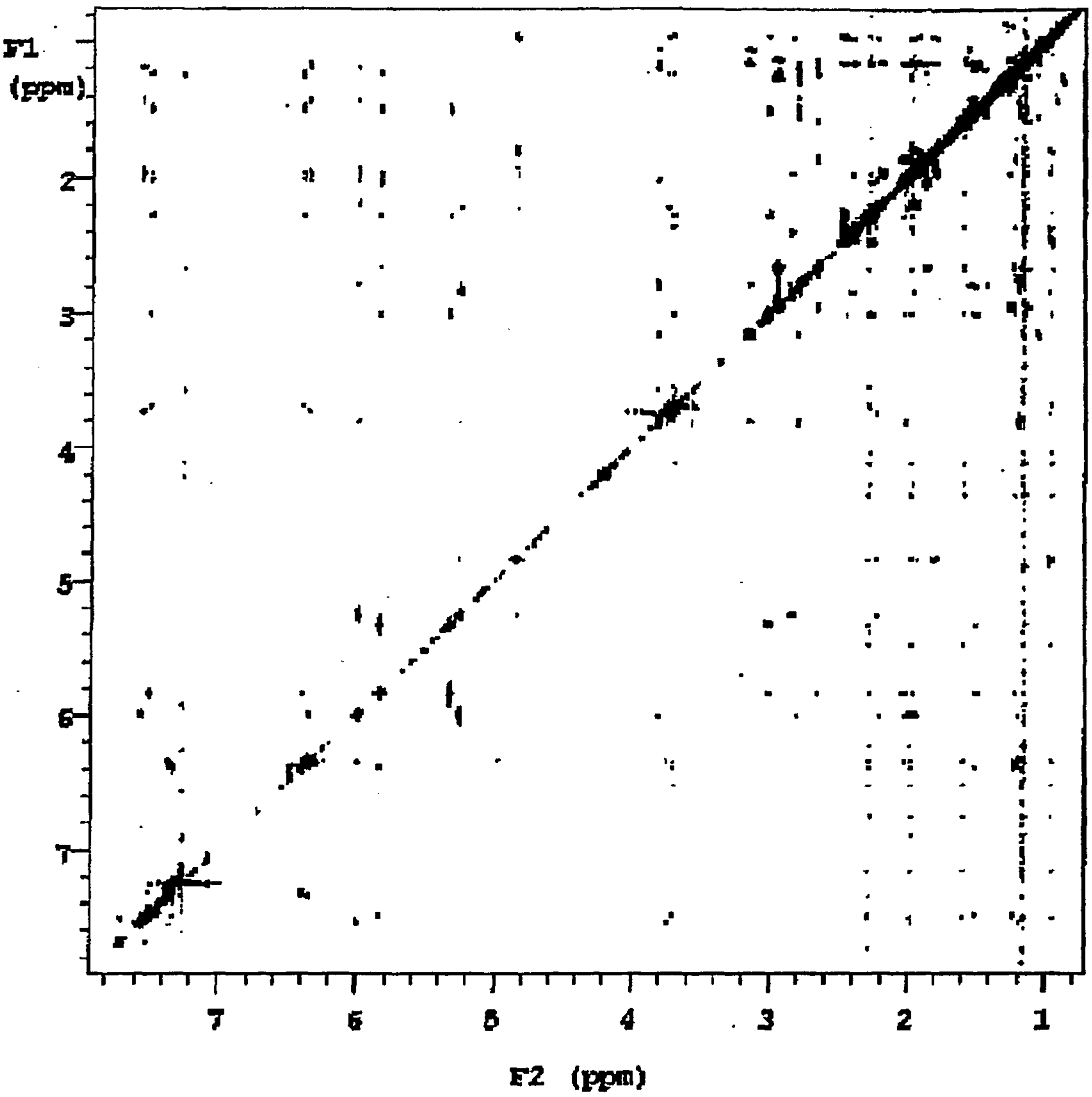

Figure 25

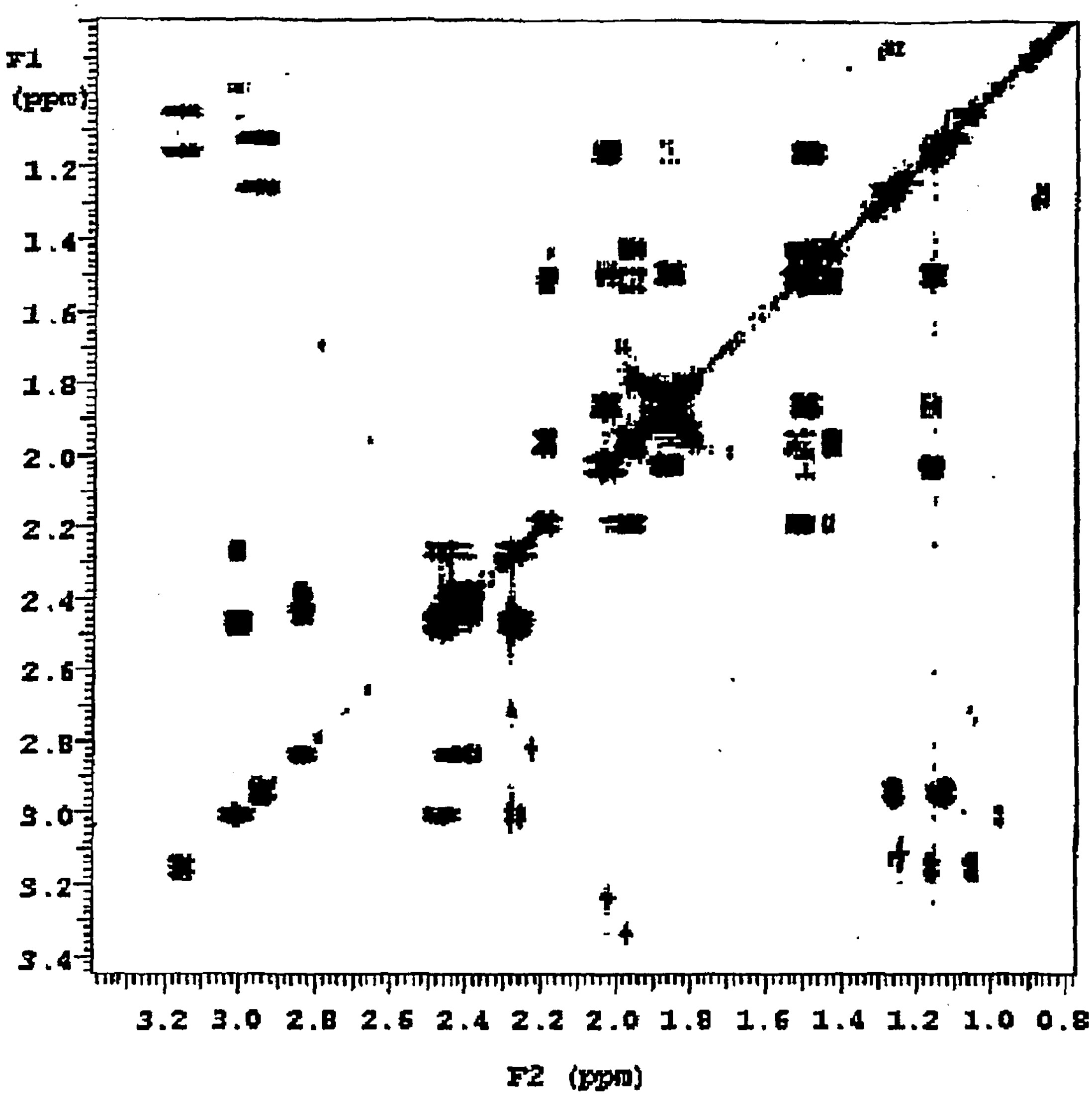
F1
(ppm)
1.2
1.4
1.6
1.8
2.0
2.2
2.4
2.6
2.8
3.0
3.2
3.4
3.2 3.0 2.8 2.6 2.4 2.2 2.0 1.8 1.6 1.4 1.2 1.0 0.8
F2 (ppm)

Figure 26

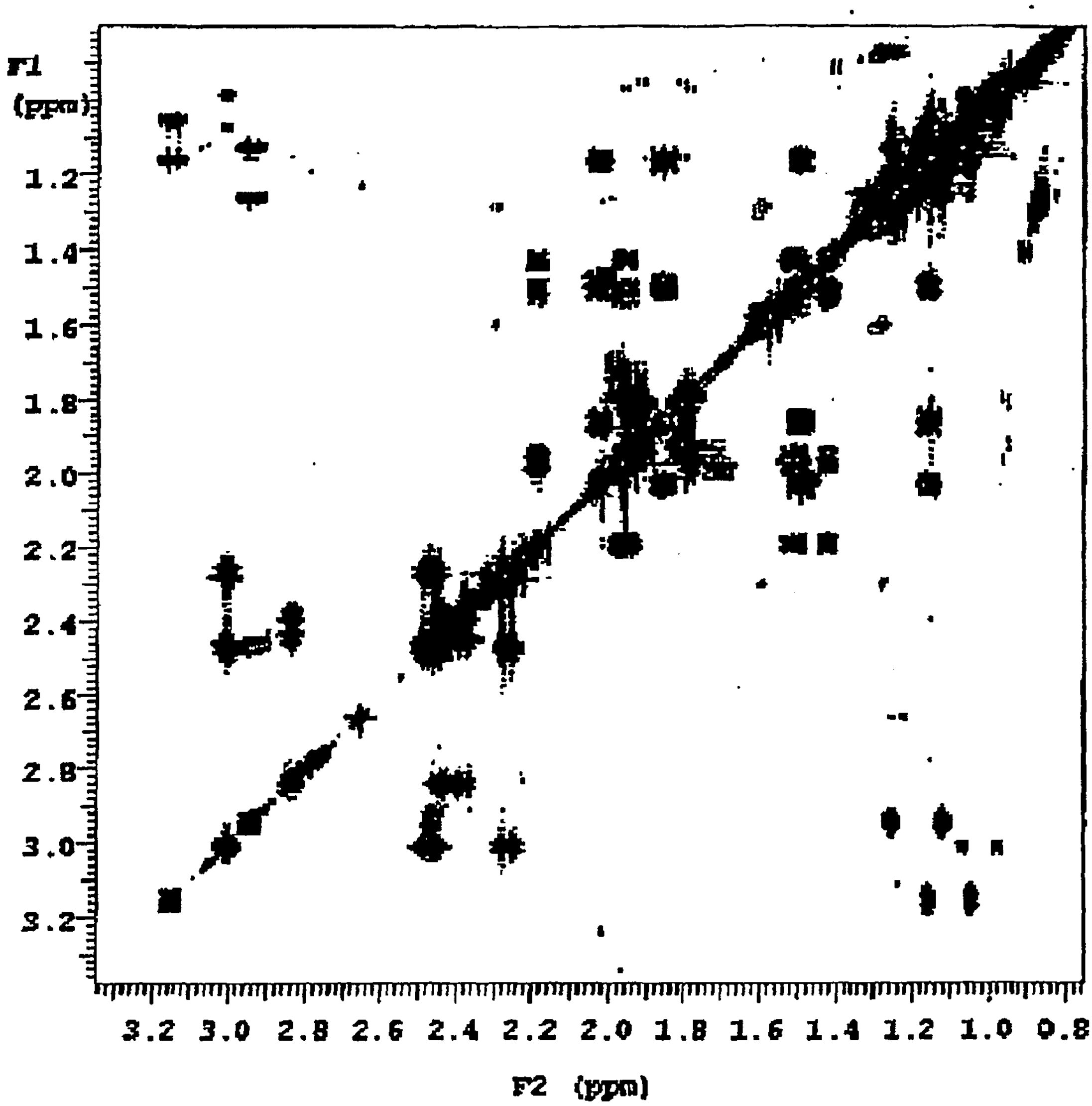
F1
(ppm)
1.2
1.4
1.6
1.8
2.0
2.2
2.4
2.6
2.8
3.0
3.2
3.2 3.0 2.8 2.6 2.4 2.2 2.0 1.8 1.6 1.4 1.2 1.0 0.8
F2 (ppm)

Figure 27

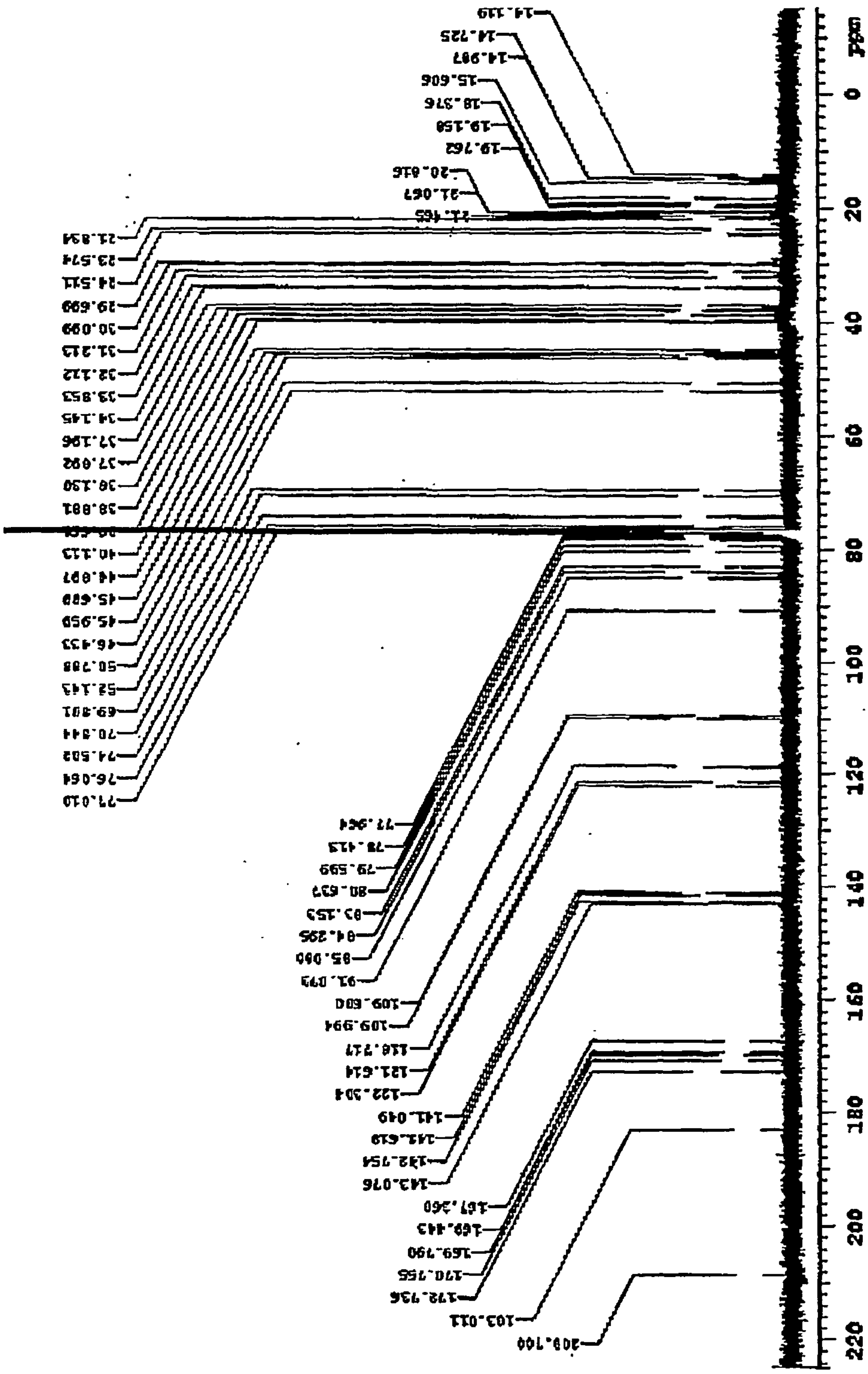

**Figure 28**

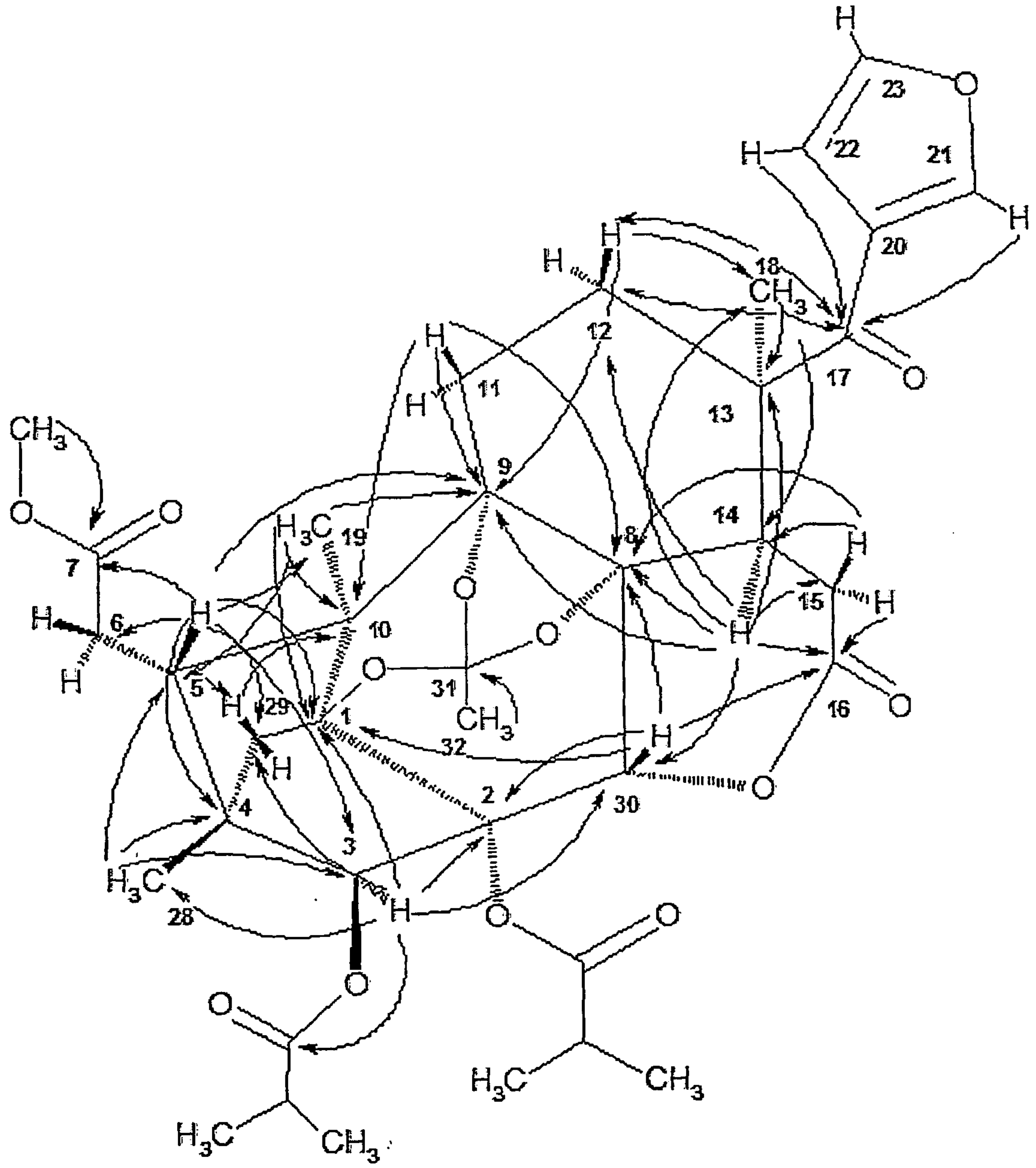

Figure 29

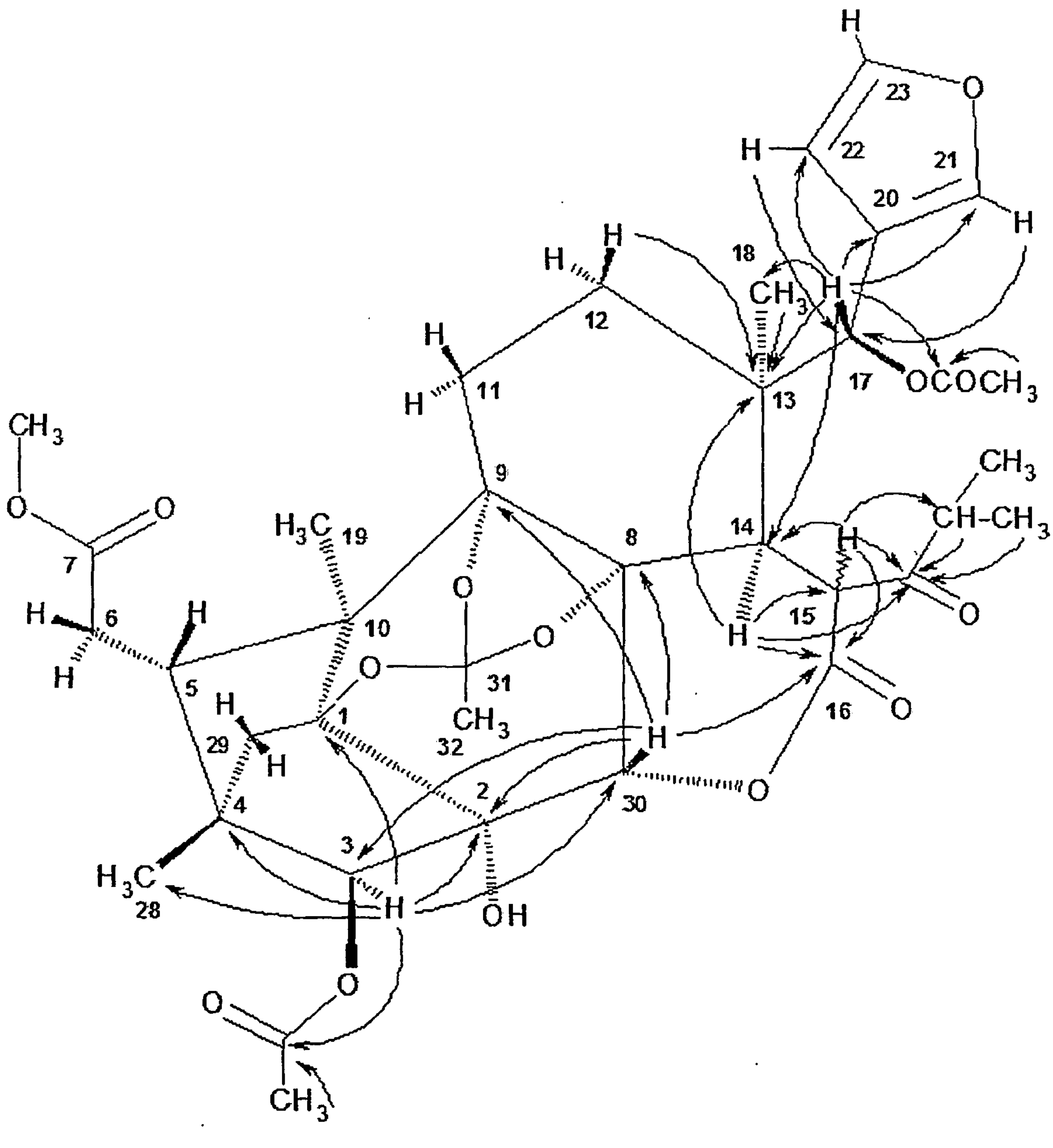

**Figure 30**

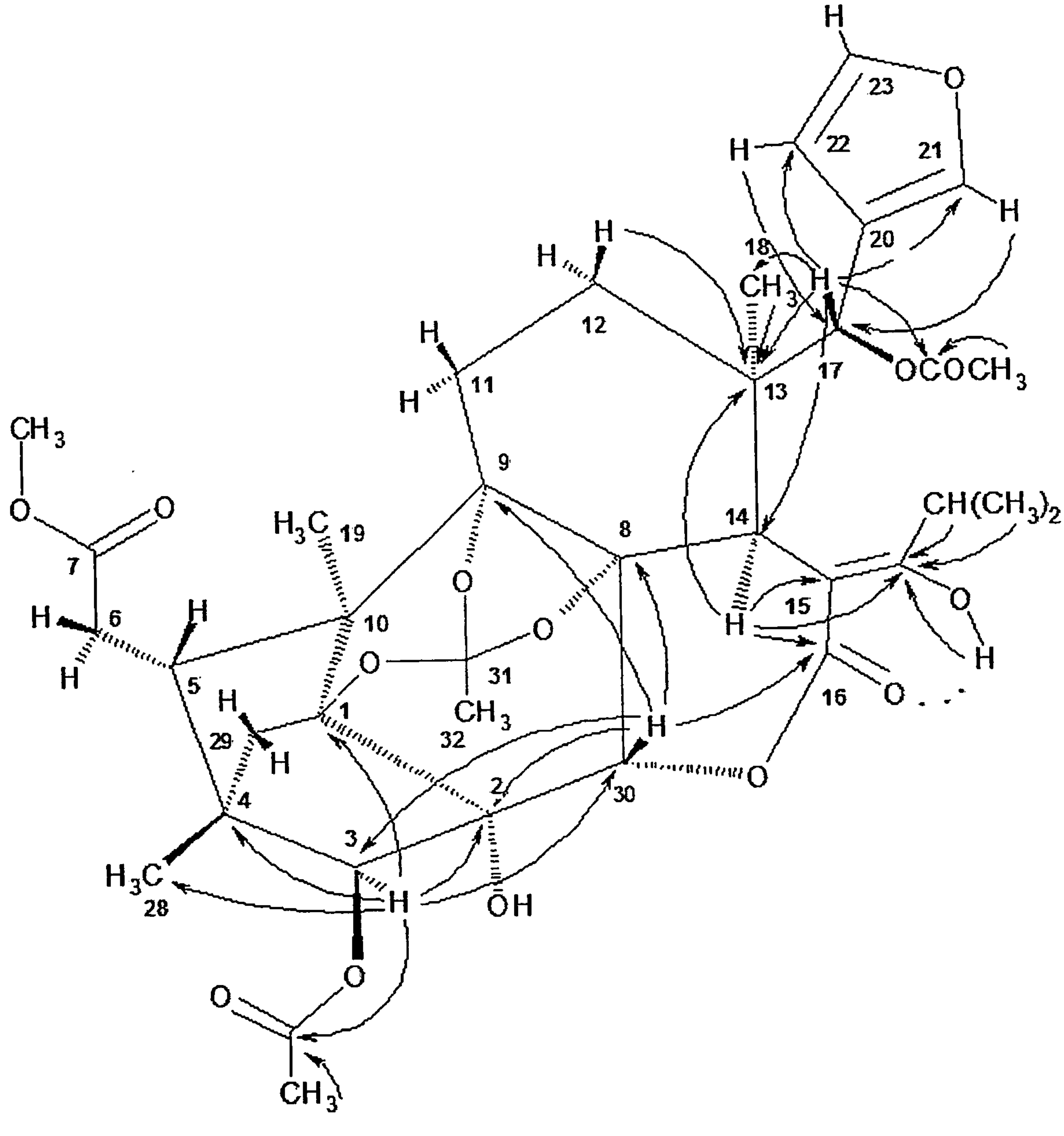

**Figure 31**

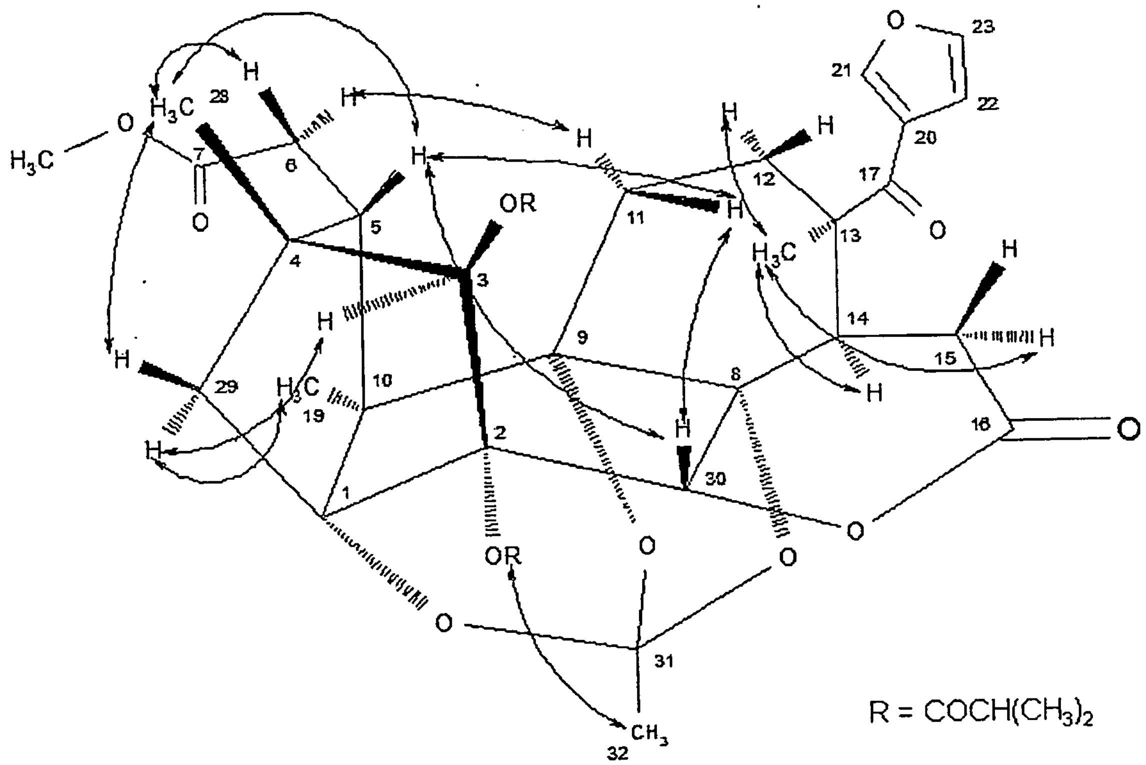

R = $COCH(CH_3)_2$

**Figure 32**

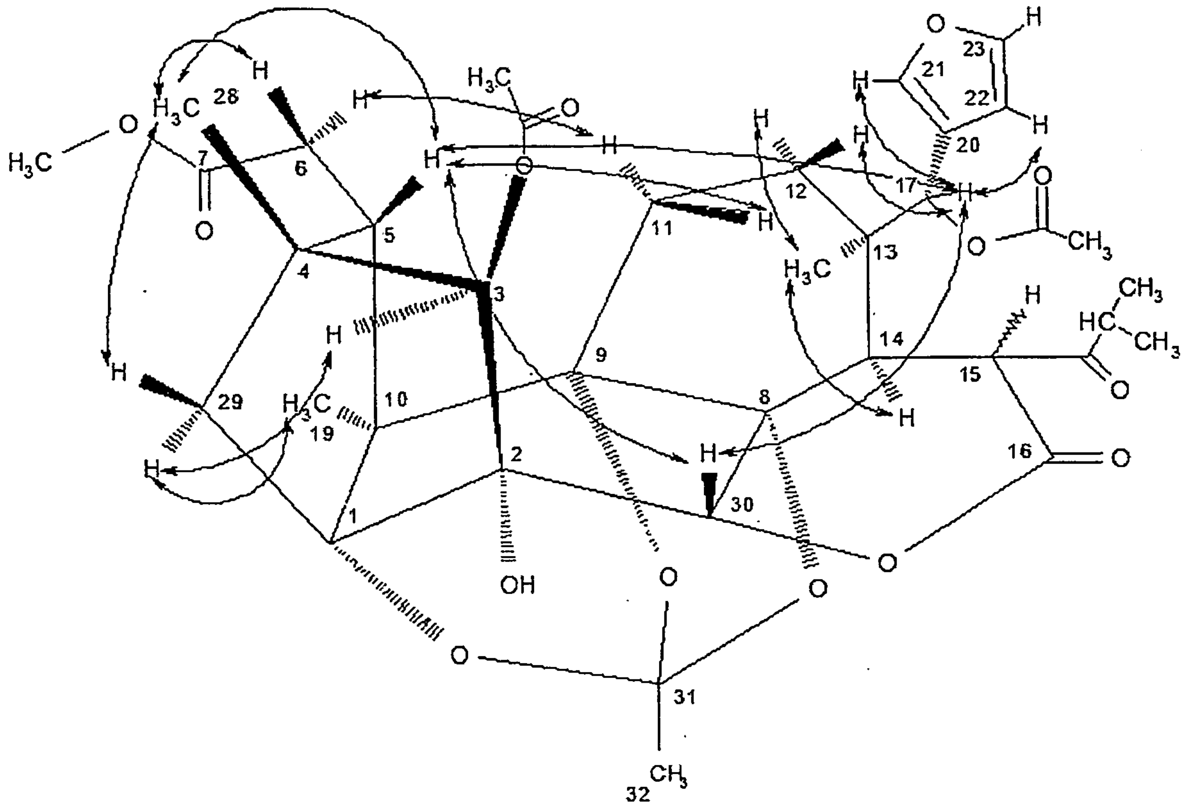

**Figure 33**

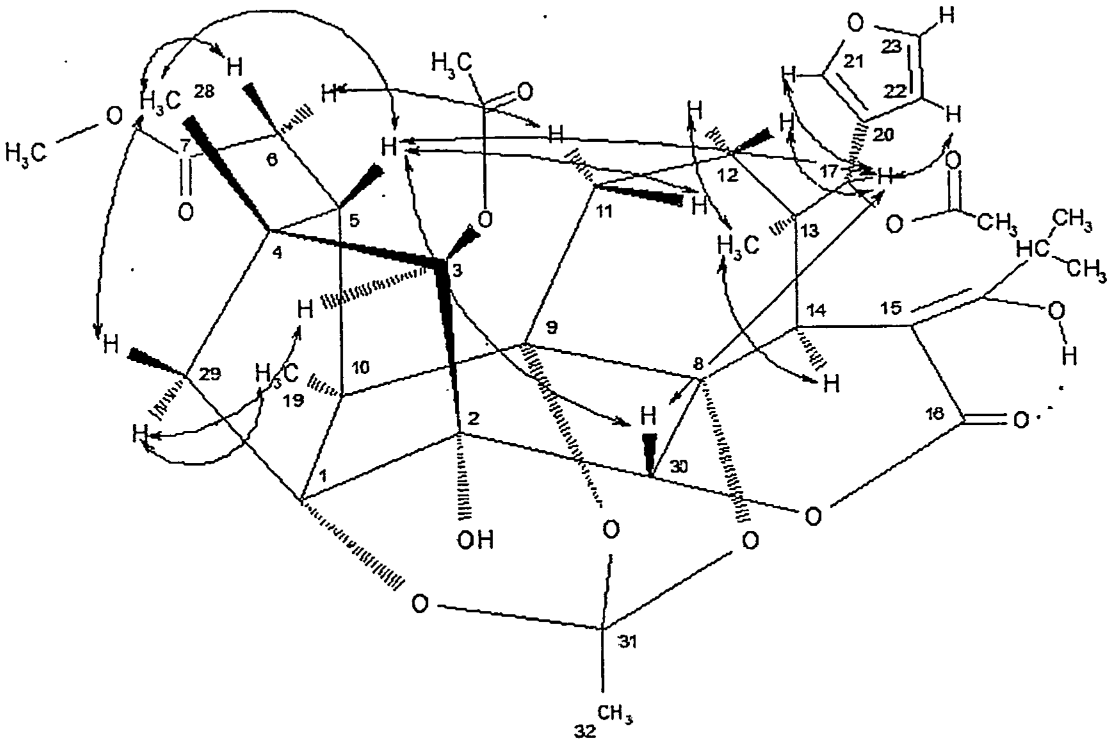

# Figure 34

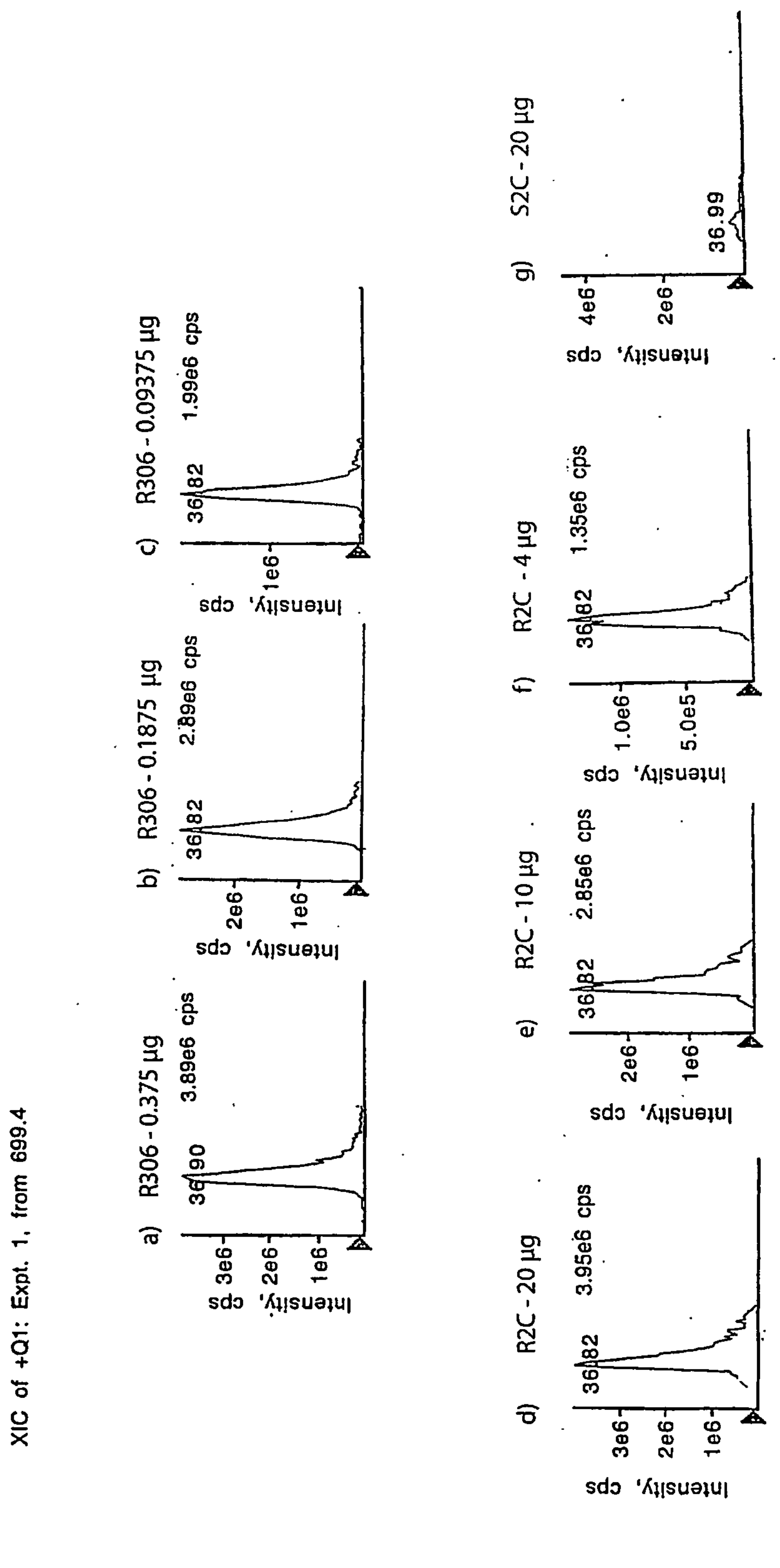
XIC of +Q1: Expt. 1, from 699.4
a) R306 - 0.375 µg
3.89e6 cps
36.90
b) R306 - 0.1875 µg
2.89e6 cps
36.82
c) R306 - 0.09375 µg
1.99e6 cps
36.82
d) R2C - 20 µg
3.95e6 cps
36.82
e) R2C - 10 µg
2.85e6 cps
36.82
f) R2C - 4 µg
1.35e6 cps
36.82
g) S2C - 20 µg
36.99
Intensity, cps

Figure 35

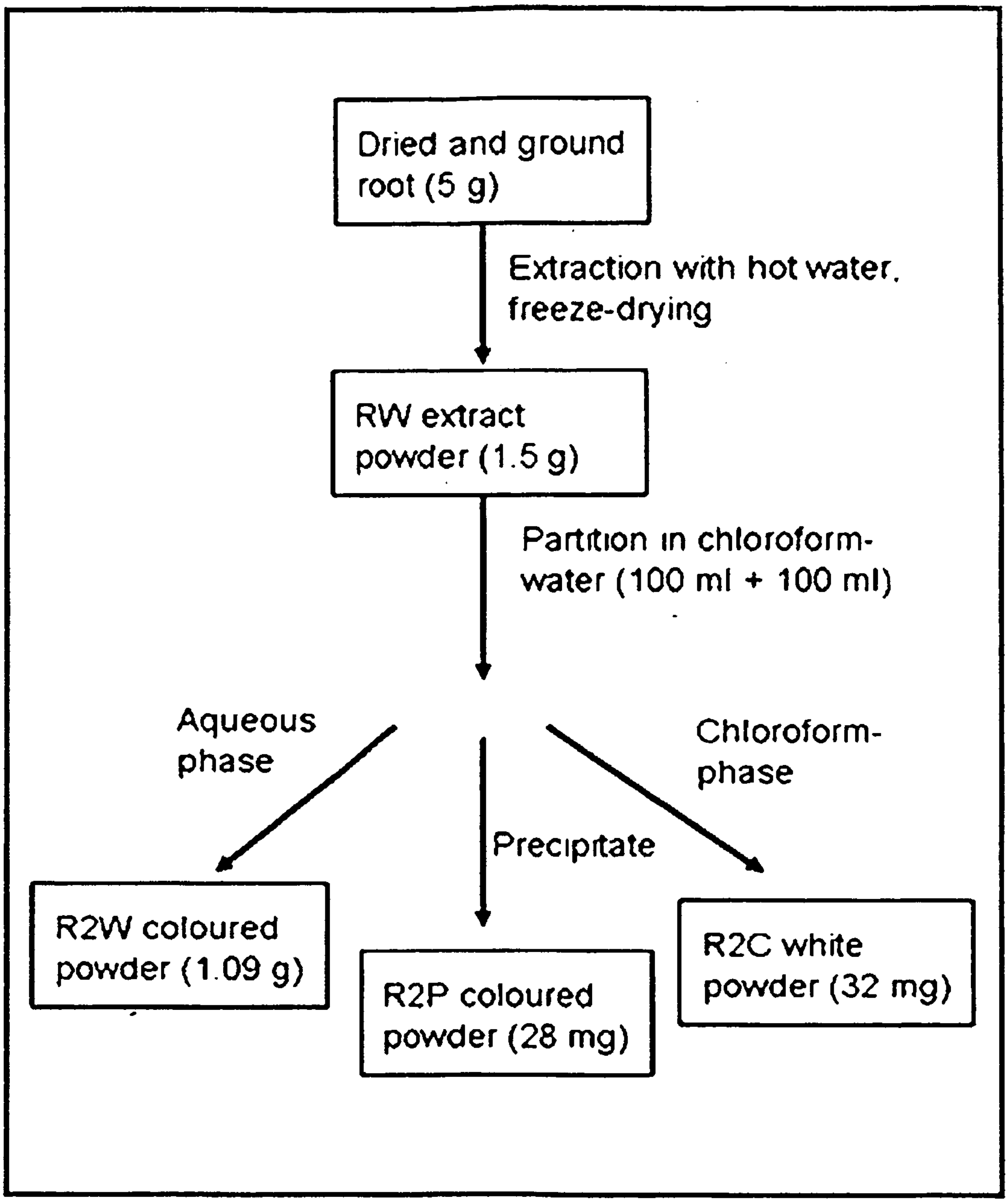
Dried and ground root (5 g)
Extraction with hot water, freeze-drying
RW extract powder (1.5 g)
Partition in chloroform-water (100 ml + 100 ml)
Aqueous phase
Precipitate
Chloroform-phase
R2W coloured powder (1.09 g)
R2P coloured powder (28 mg)
R2C white powder (32 mg)

**Figure 36**

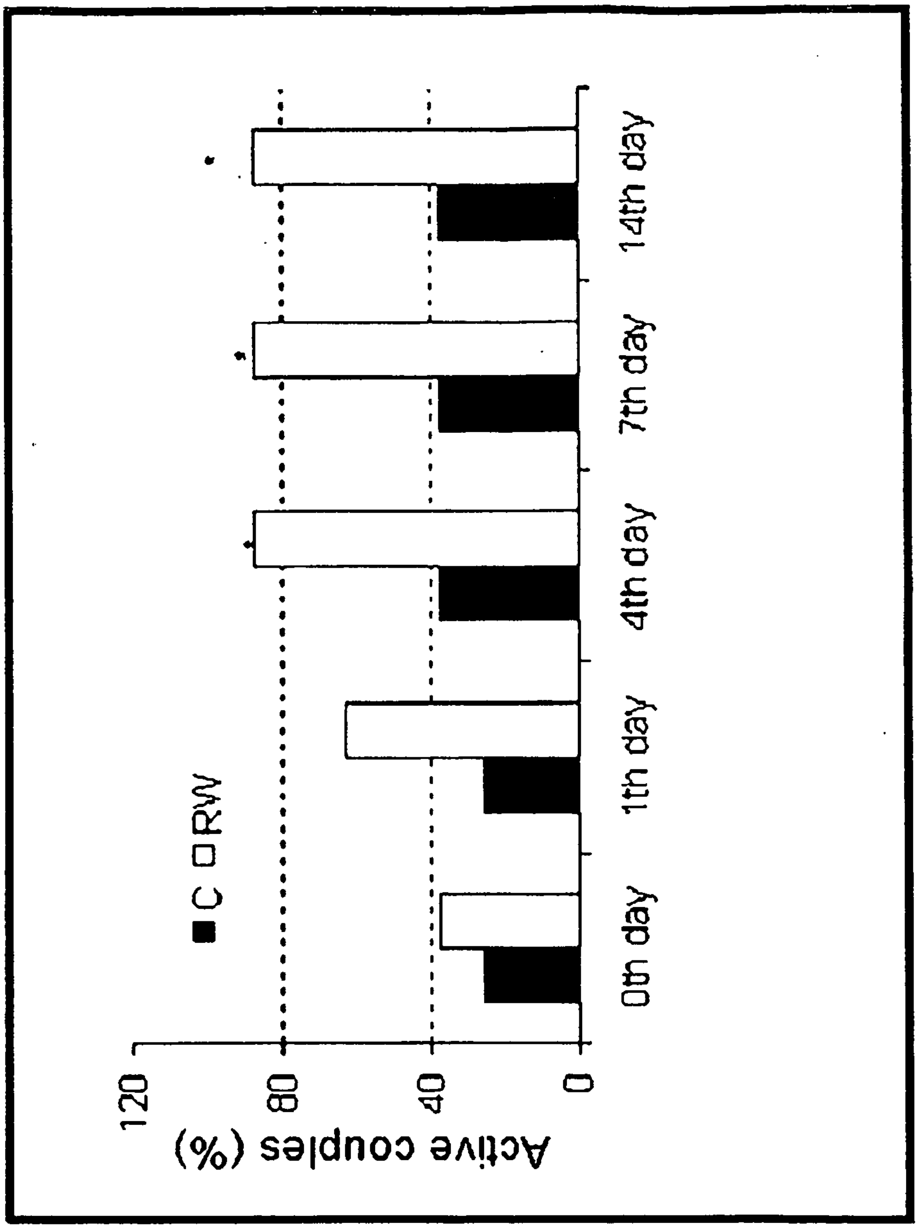
Active couples (%)
120
80
40
0
0th day
1th day
4th day
7th day
14th day
C
RW

**Figure 37**

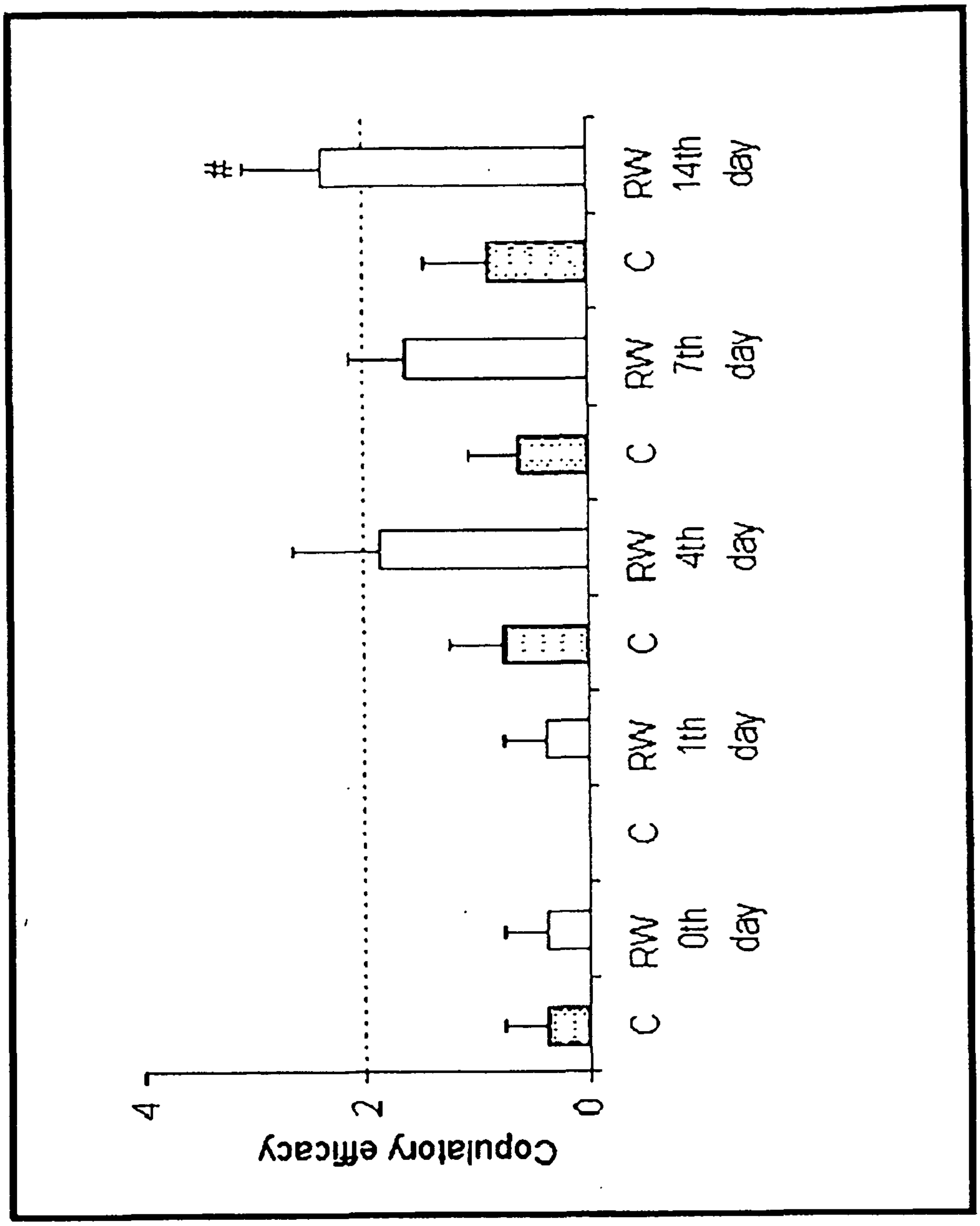
Copulatory efficacy
0
2
4
C
RW
0th
day
C
RW
1th
day
C
RW
4th
day
C
RW
7th
day
C
RW
14th
day
#

**Figure 38**

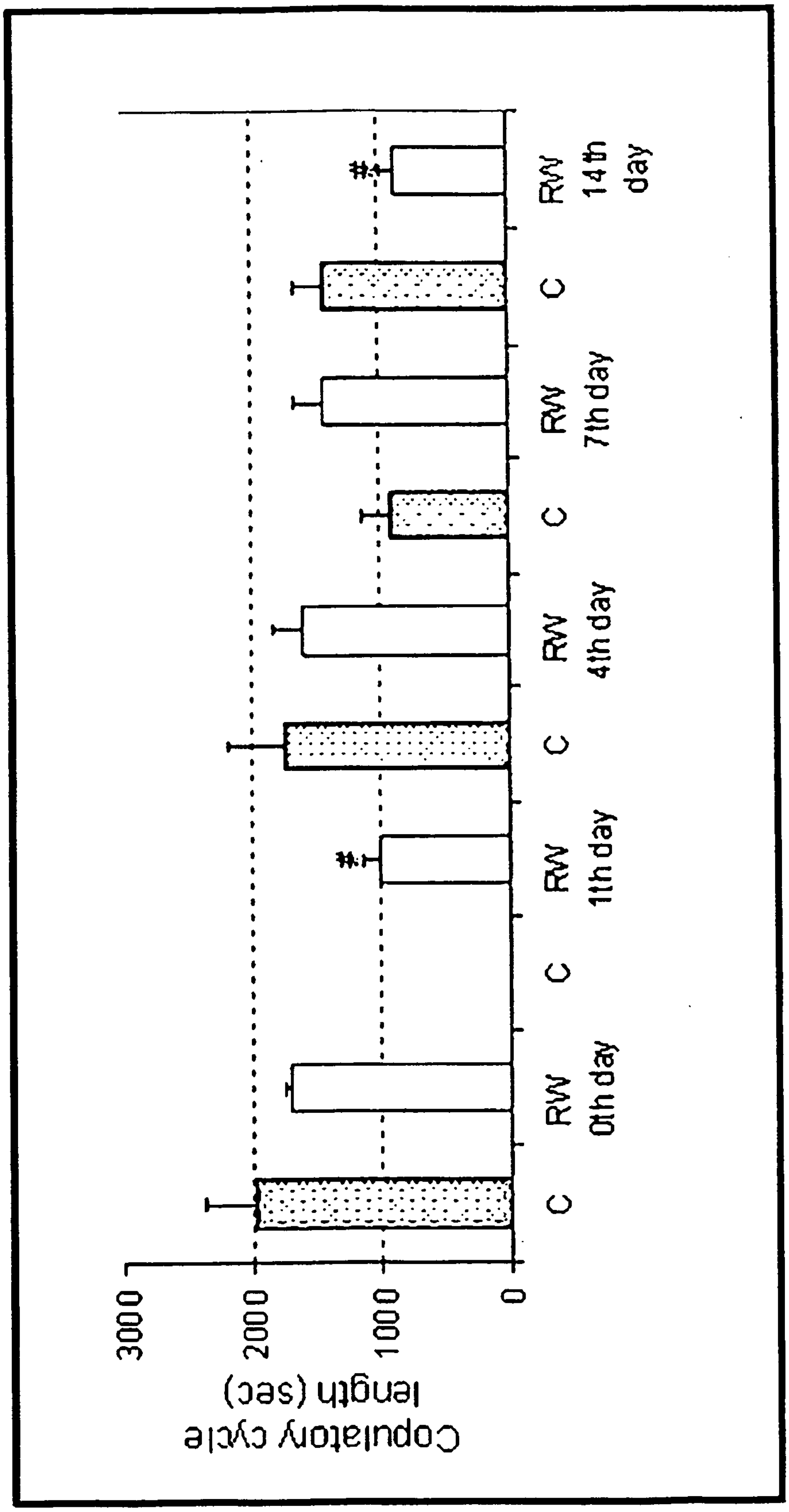
Copulatory cycle length (sec)
3000
2000
1000
0
C
RW
0th day
C
RW
1th day
C
RW
4th day
C
RW
7th day
C
RW
14 th day

**Figure 39**

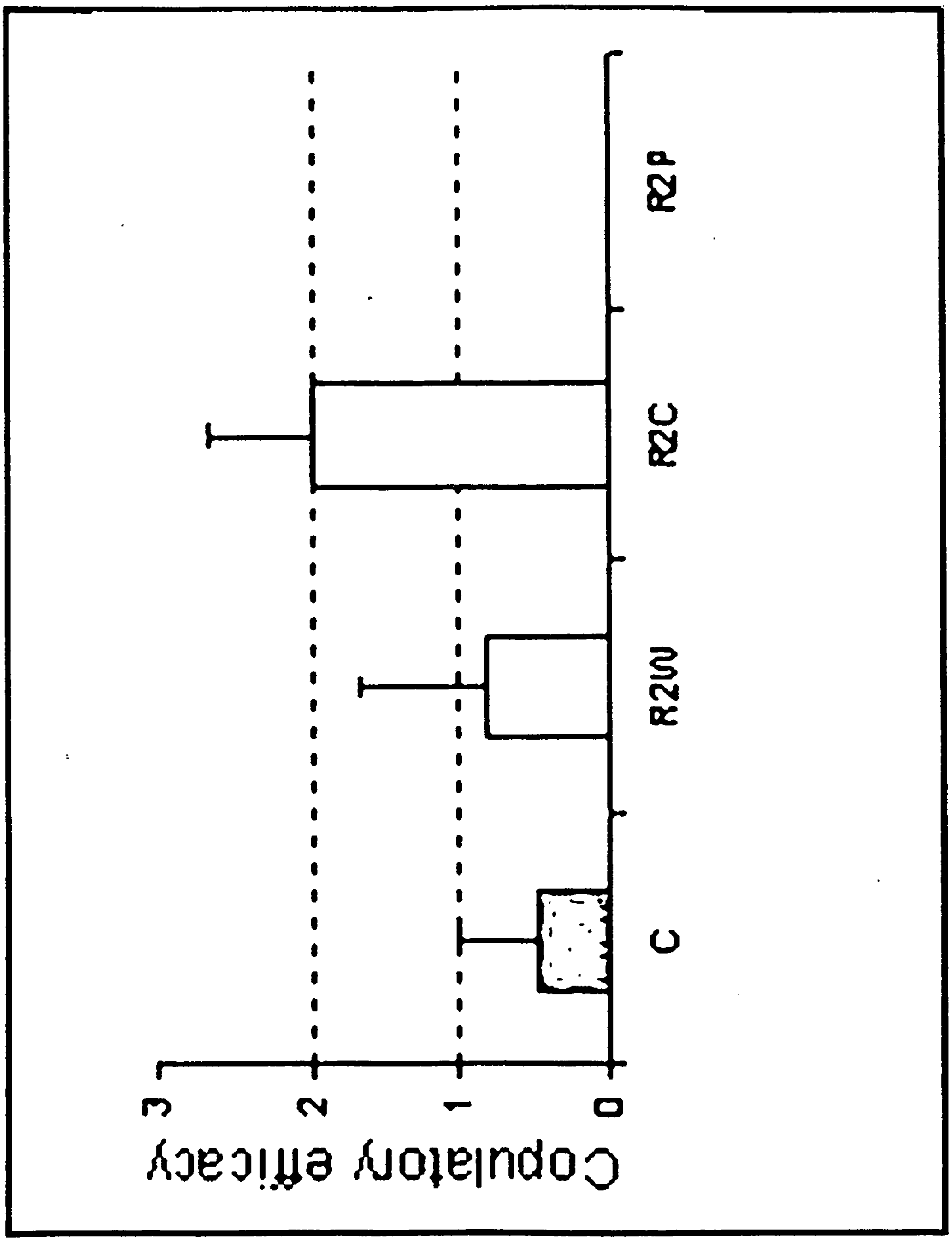

Figure 40

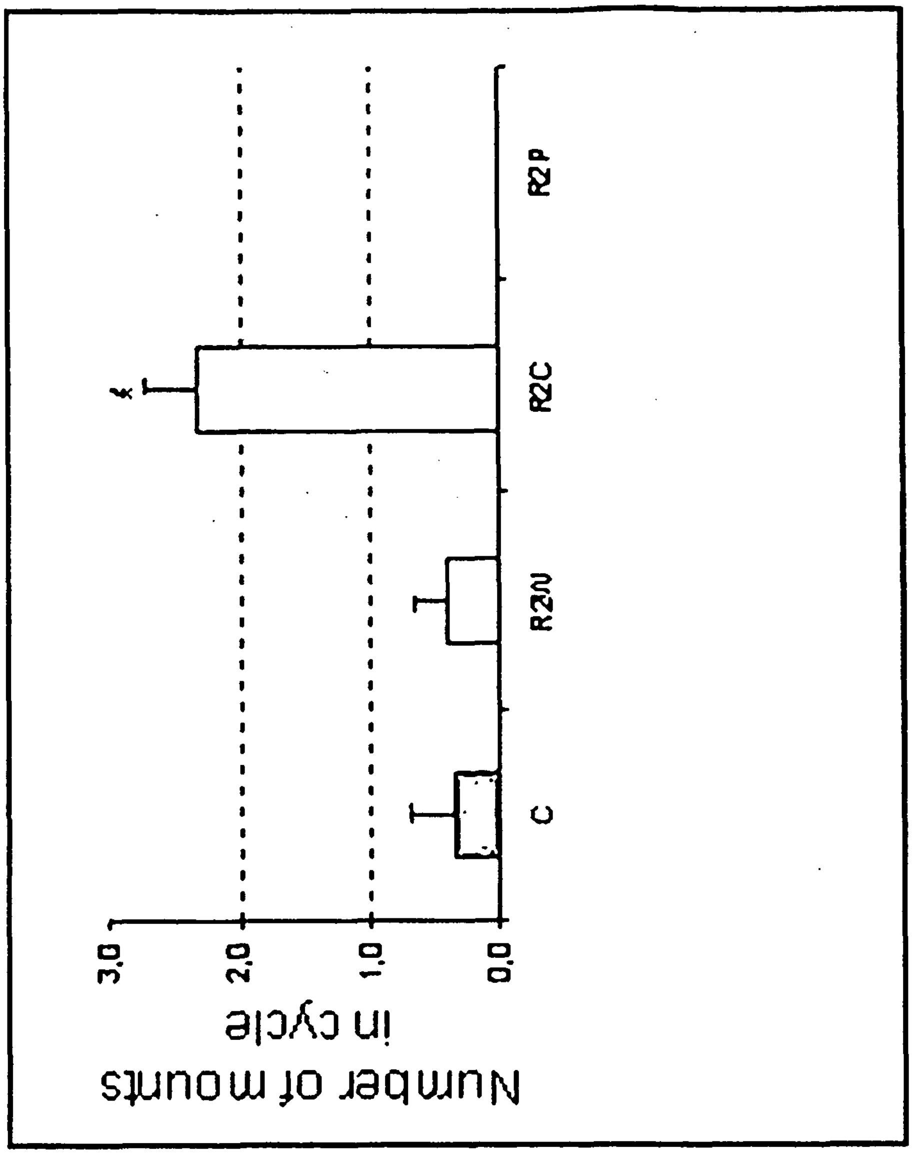

Figure 41

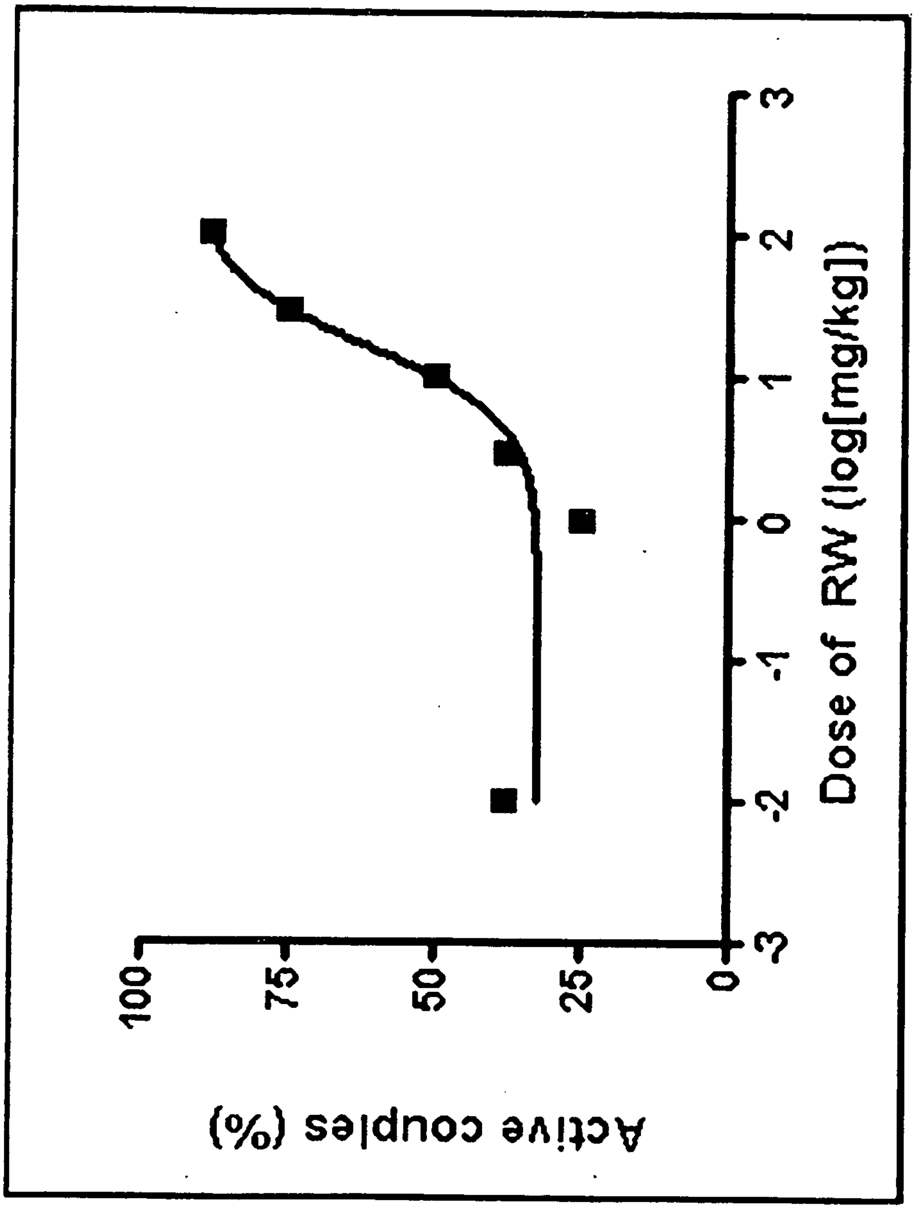

Figure 42

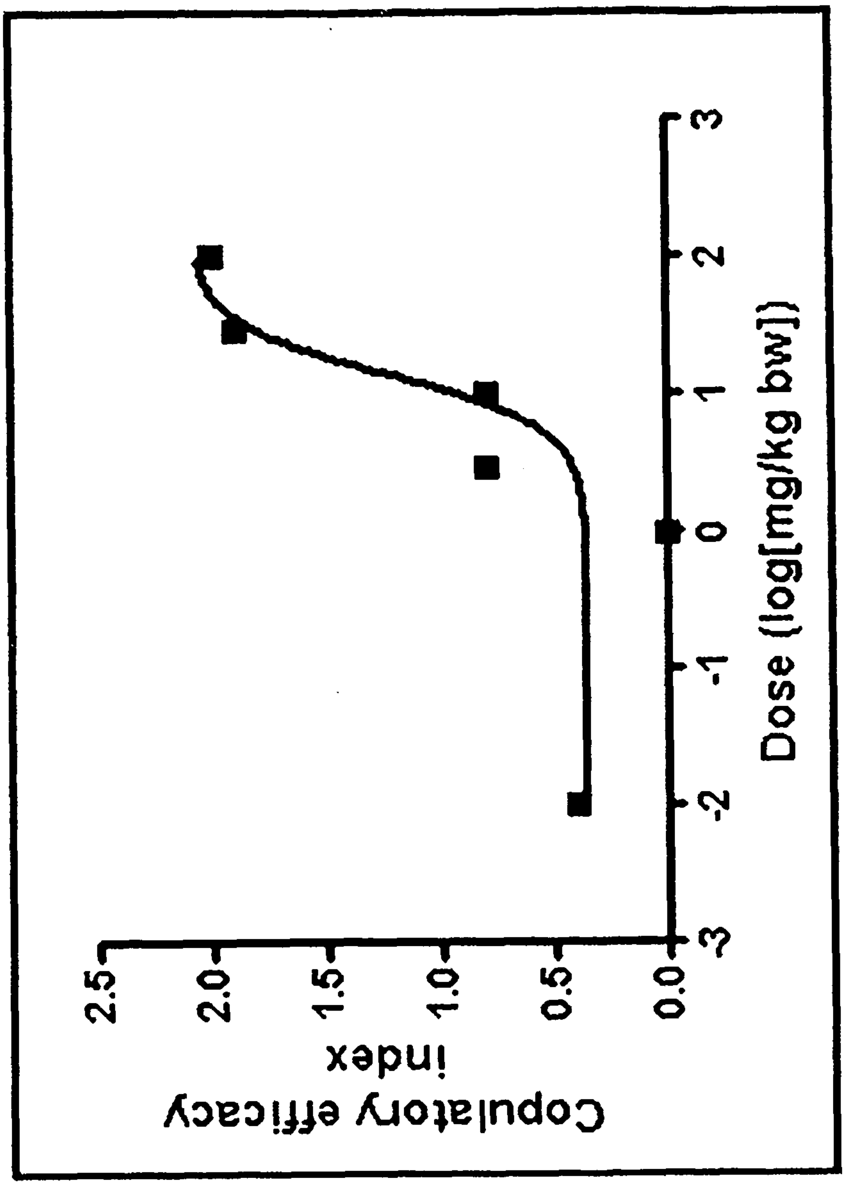
Copulatory efficacy index
2.5
2.0
1.5
1.0
0.5
0.0
-3
-2
-1
0
1
2
3
Dose (log[mg/kg bw])

**Figure 43**

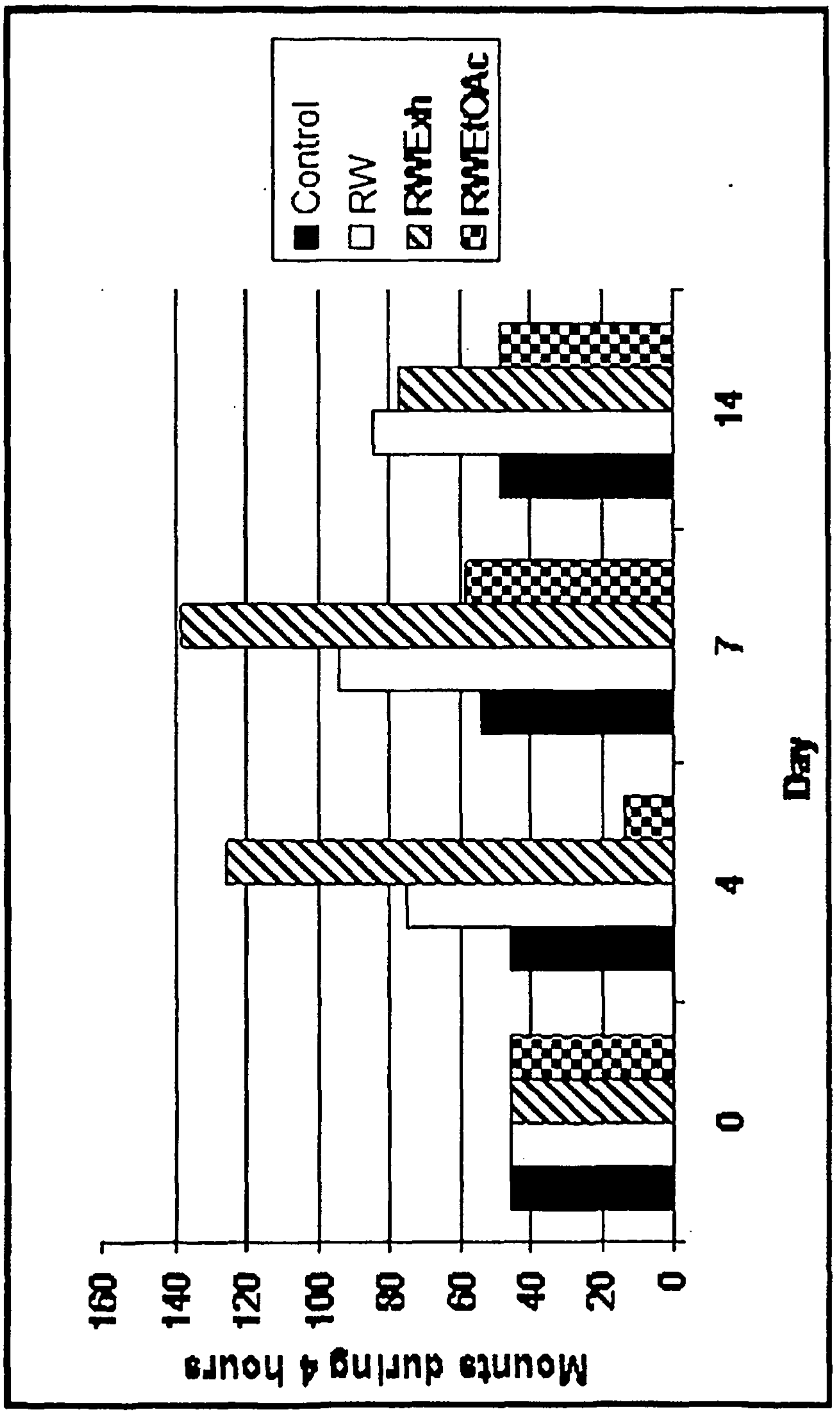
Mounts during 4 hours
0
20
40
60
80
100
120
140
160
Day
0
4
7
14
Control
RW
RWExh
RWEtOAc

**Figure 44**

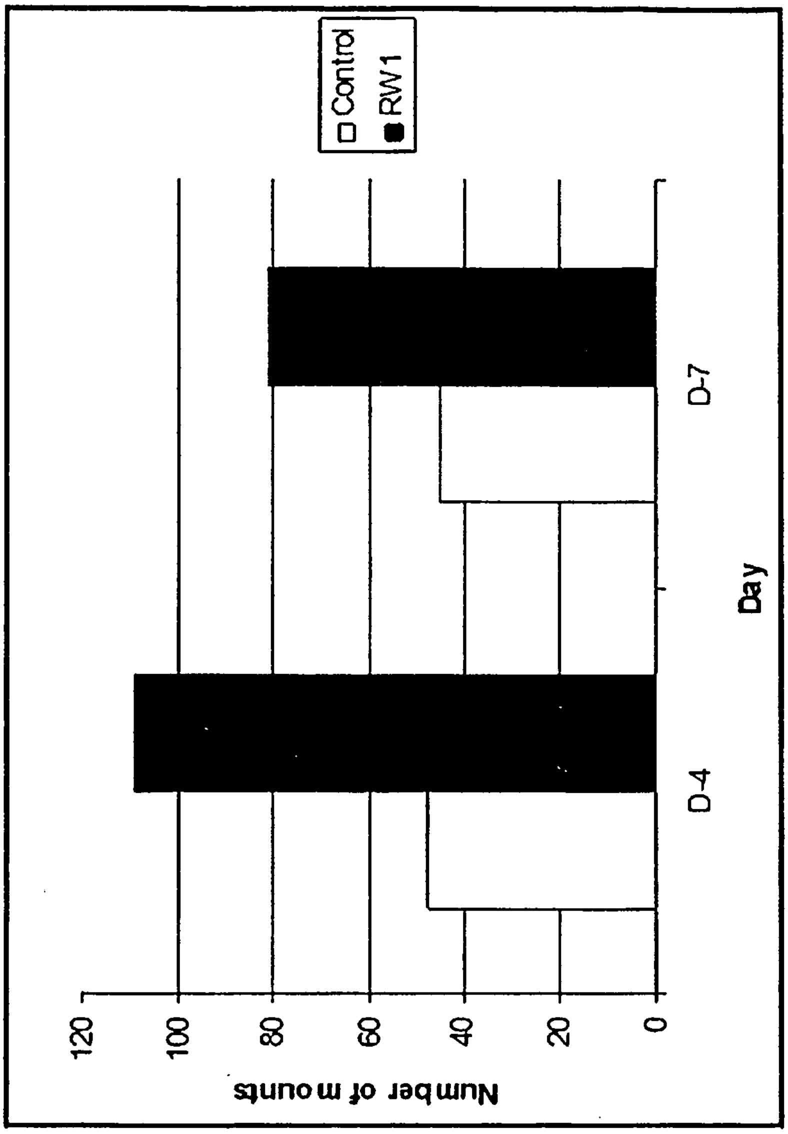

Figure 45

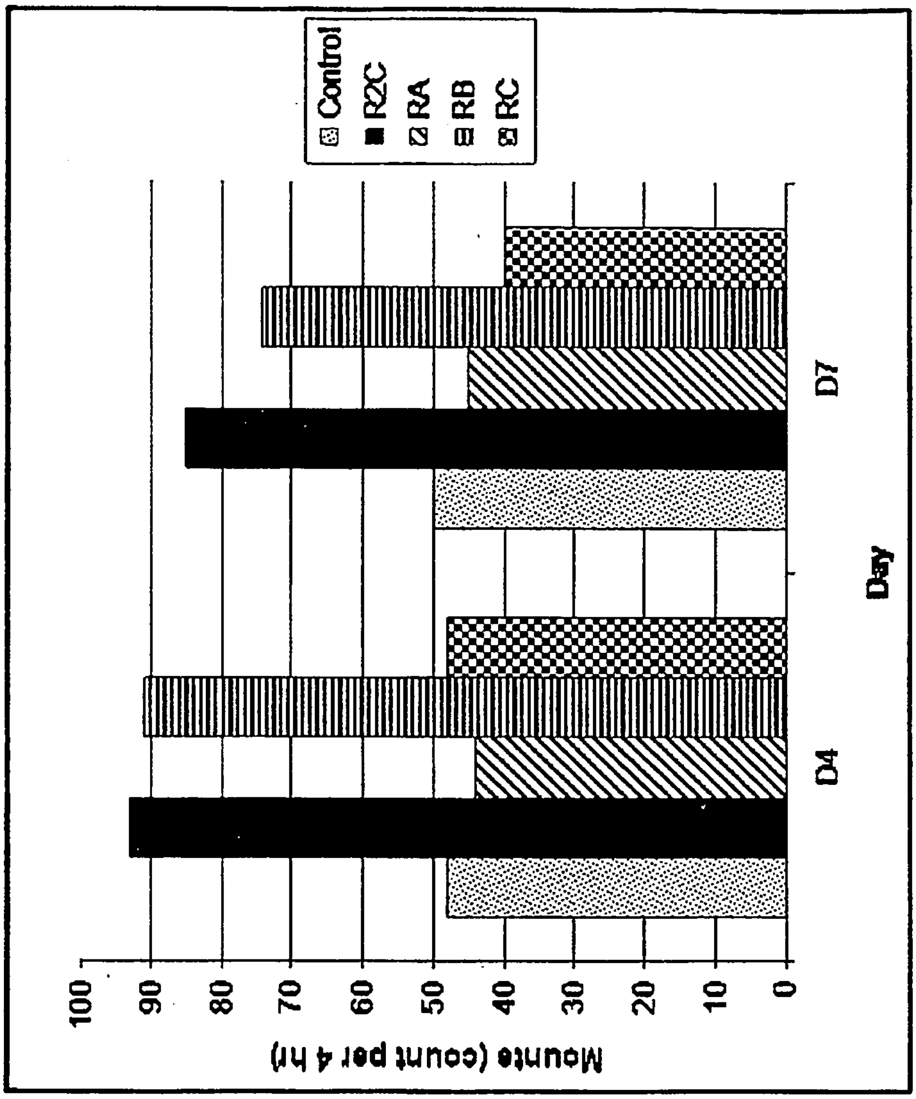
Control
R2C
RA
RB
RC
Mounts (count per 4 hr)
100
90
80
70
60
50
40
30
20
10
0
D4
D7
Day

**Figure 46**

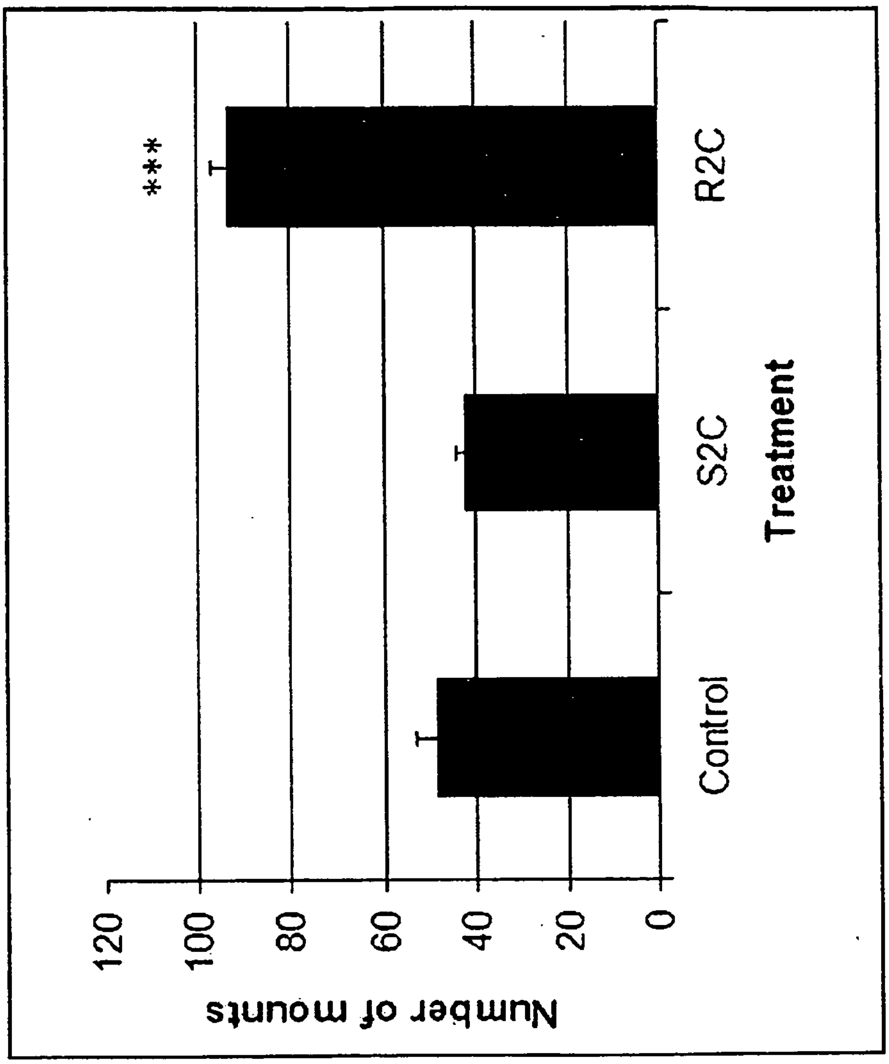
***
120
100
80
60
40
20
0
Number of mounts
Control
S2C
R2C
Treatment

Figure 47

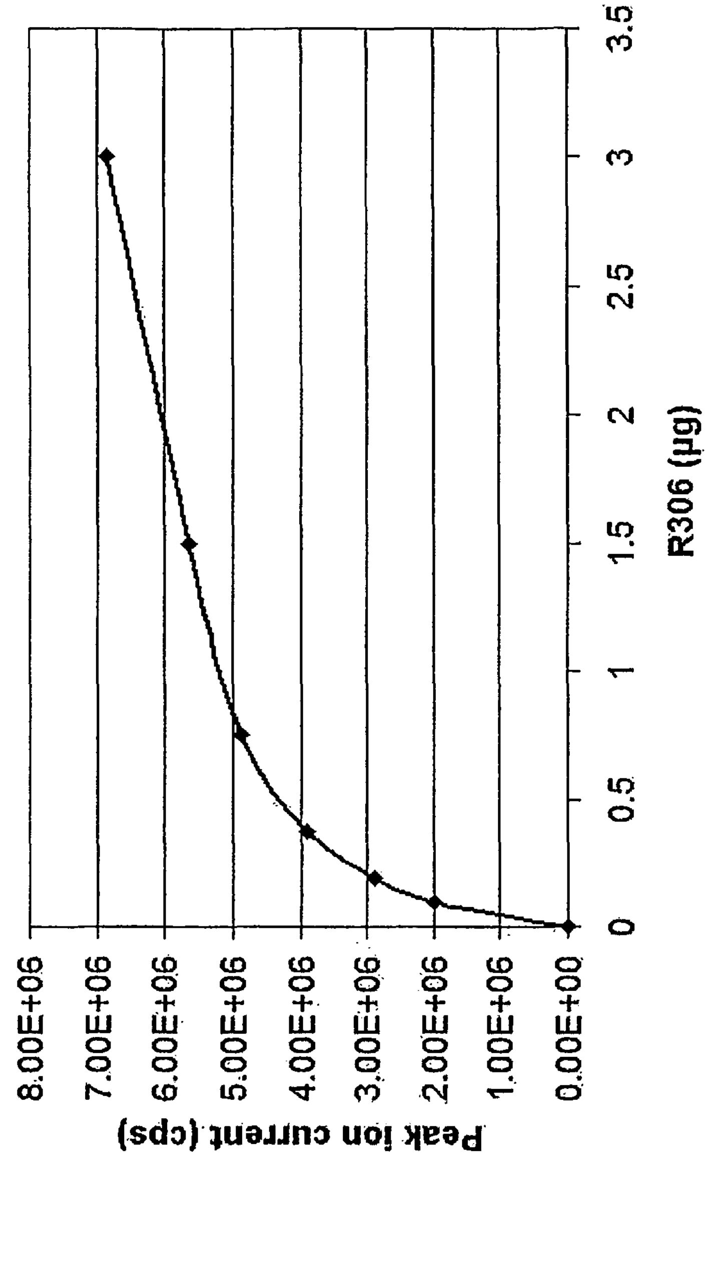
Peak ion current (cps)
8.00E+06
7.00E+06
6.00E+06
5.00E+06
4.00E+06
3.00E+06
2.00E+06
1.00E+06
0.00E+00
0
0.5
1
1.5
2
2.5
3
3.5
R306 (µg)

Figure 48

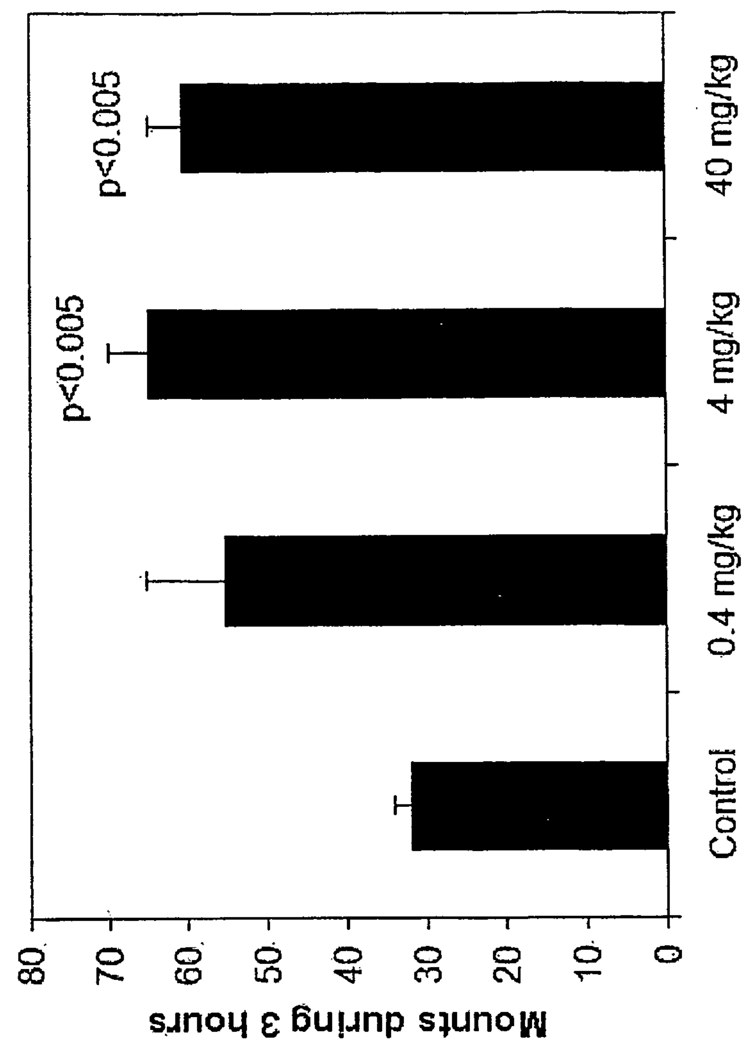
Mounts during 3 hours
0
10
20
30
40
50
60
70
80
Control
0.4 mg/kg
4 mg/kg
40 mg/kg
p<0.005
p<0.005

**Figure 49**

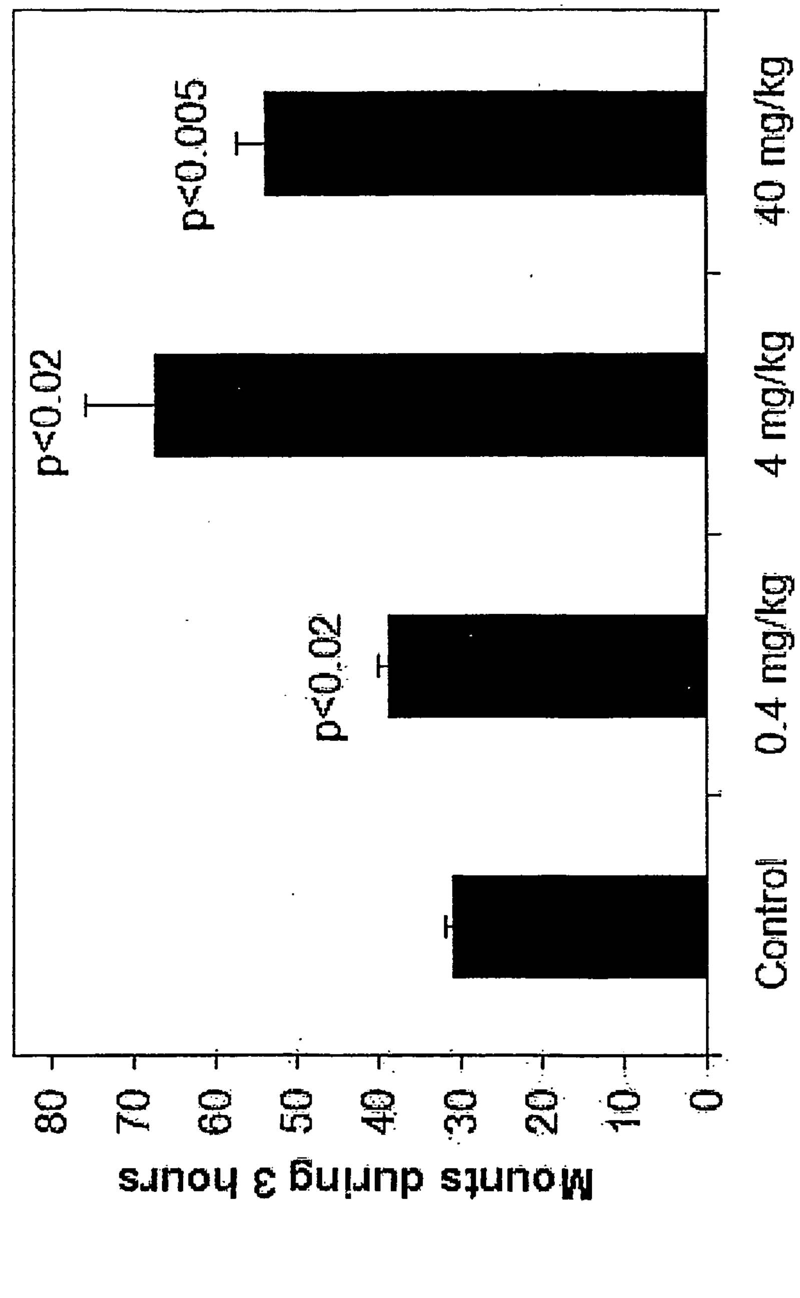
Mounts during 3 hours
0
10
20
30
40
50
60
70
80
Control
0.4 mg/kg
4 mg/kg
40 mg/kg
p<0.02
p<0.02
p<0.005

Figure 50

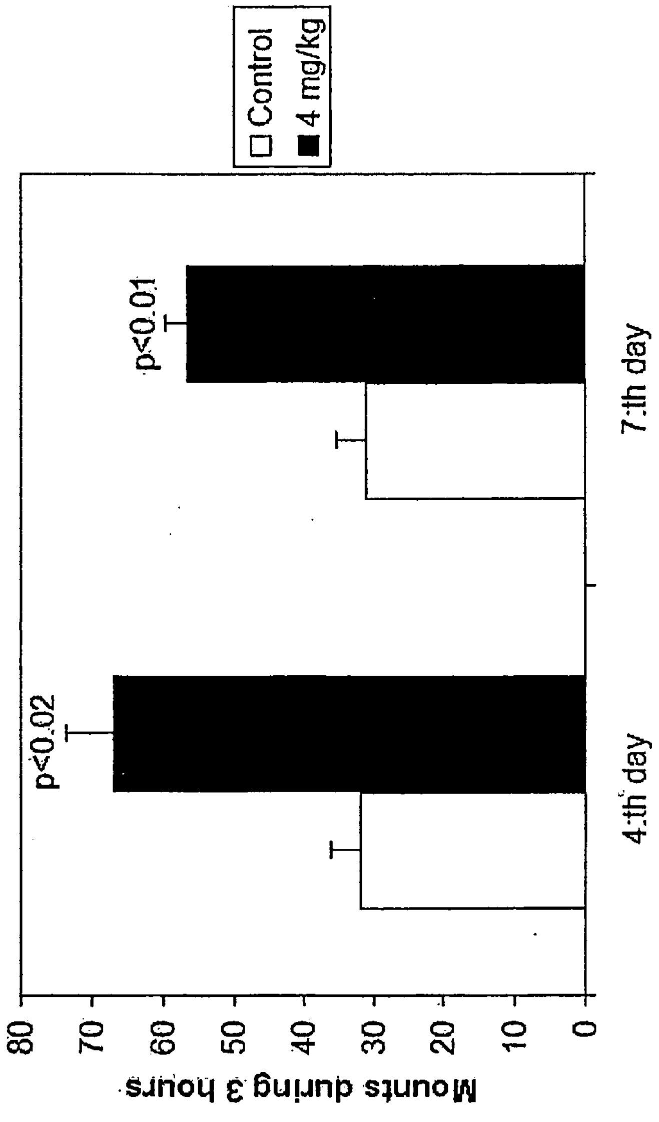
Control
4 mg/kg
p<0.02
p<0.01
4:th day
7:th day
Mounts during 3 hours
80
70
60
50
40
30
20
10
0

Figure 51

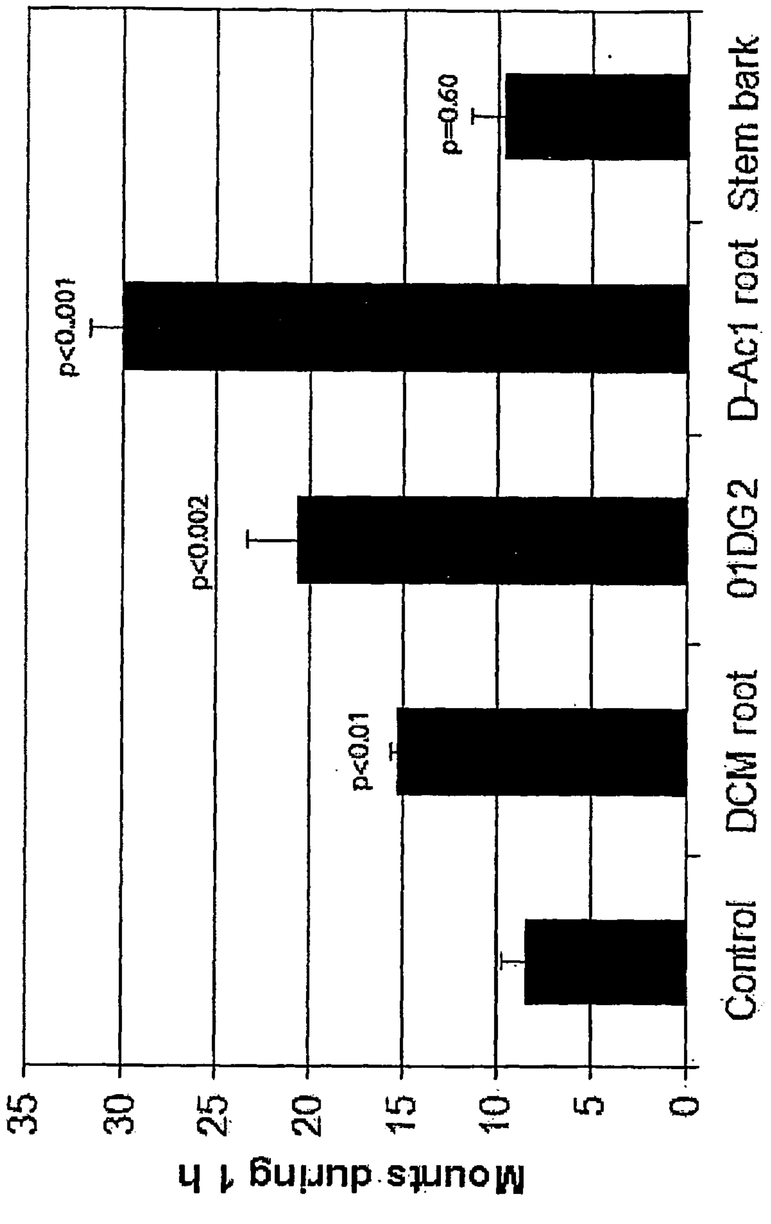
Mounts during 1 h
35
30
25
20
15
10
5
0
Control
DCM root
01DG2 root
D-Ac1 root
Stem bark
p<0.01
p<0.002
p<0.001
p=0.60

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005213202 A
- WO 2007031830 A2


### Non-patent literature cited in the description


- **LIAO et al.** Plant orthoesters. *Chemical Reviews,* vol. 109 (3), 1092-1140
- **KOTSOS.** *PhD thesis,* 1997, http://hdl.handle.net110413/278
- Dietary Supplement Health and Education Act. US Food and Drug Administration, 1994
- **J.F. LEROY.** Compt. Rend. Acad. Sci. Paris ccxlvi 2640. *Journ. Agric. & Bot. Appliq.,* 1958
- *Comp. Rend. Acad. Sci. Paris ccxlvi. 2640,* 1958
- *Journ. Agric. & Bot. Appliq. v 504,* 1958
- **J.F. LEROY.** *Adansonia,* 1976, vol. 16 (2), 172
- **GRUMBACH et al.** Hydrophilic interaction chromatography using silica columns for the retention of polar analytes and enhanced ESI-MS sensitivity. *LCGS Asia Pacific,* 04 November 2004, vol. 7
- **MUELLNER AN et al.** Molecular phylogenetics of Meliaceae (Sapindales) based on nuclear and plastid DNA sequences. *Am. J. Botany.,* 2003, vol. 90 (3), 471-480
- **BERNASCONI et al.** *J. Am. Chem. Soc.,* 1981, vol. 103 (16), 4852
- **BERNASCONI et al.** *J. Am. Chem. Soc.,* 1981, vol. 103 (16), 4850-4860
- **DESS, DB ; MARTIN, JC.** *J. Am. Chem. Soc.,* 1991, vol. 113, 7277-7287
- **MILLER et al.** *J. Am. Chem. Soc.,* 2006, vol. 128 (51), 17057-17062
- *Turkish J Chemistry,* 2005, vol. 29 (6), 635-639
- *Synlett.,* 2005, (8), 2826-2828
- **DÁLAIGH et al.** *Org. Biomol. Chem.,* 2006, (4), 2785-2793
- **MACKAY ; VEDEJS.** *J. Org. Chem,* 2004, vol. 69, 6934-6937
- **MASTER et al.** *Bioorganic & Medicinal Chemistry Letters,* 2003, vol. 13, 1249-1251
- **OUK et al.** *Green Chemistry,* 2002, vol. 4, 431-435
- **TAMBURLIN-THUMIN et al.** *Eur. J. Med. Chem.,* 2001, vol. 36, 561-568
- **B. NEISES ; W. STEGLICH.** Simple Method for the Esterification of Carboxylic Acids. *Angew. Chem. Int. Ed.,* 1978, vol. 17, 522-524
- Chemoinformatics. Wiley-VCH GmbH & Co,
- **BERGE et al.** *J. PHARM. SCI.,* 1977, vol. 66, 1-19
- Harms ex Engl. Publication. *Engl. Pflanzenw. Afr. iii. 1. (Engl. & Drude, Veg. der Erde, ix.),* 1915, 807
- *Tetrahedron,* 1972, vol. 28, 2333-2340
- **ANSEL, H.C. ; L.V. ALLEN, JR ; N.G. POPOVICH.** Pharmaceutical Dosage forms and drug delivery systems. Lippincott Williams & Wilkins, 1999
- **BALDWIN, DS.** Sexual dysfunction associated with antidepressant drugs. *Expert Opinion Drug Safety,* 2004, vol. 3 (5), 457-470
- **SHABBIR, M. ; D.M. MIHAILIDIS ; R.J. MORGAN.** Erectile dysfunction: an underdiagnosed condition associated with multiple risk factors. *Current Medical Research and Opinions,* 2004, vol. 20 (5), 603-606
- **HAFEZ, ESE ; S.D. HAFEZ.** Erectile dysfunction: Anatomical parameters, etiology, diagnosis and therapy. *Archives of Andrology,* 2005, vol. 51, 15-31
- **GIULIANO, F. ; O. RAMPIN.** Neural control of erection. *Physiology & Behaviour.,* 2004, vol. 83, 189-201
- **ANDERSSON, K-E.** Pharmacology of penile erection. *Pharmacological Reviews,* 2001, vol. 53 (3), 417-450
- **KENDIRCI, M. ; S. NOWFAR ; W.J.G. HELLSTROM.** The impact of vascular risk factors on erectile function. *Drugs of Today. 005,* vol. 41 (1), 65-74
- **RALPH, D.J. ; K.R. WYLIE.** Ejaculatory disorders and sexual function. *BJU International,* June 2005, vol. 95 (9), 1181-1186
- **MEULEMAN, E.J.H ; J.J.D.M. LANKVELD.** Hypoactive sexual desire disorder: an underestimated condition in men. *BJU International,* 2005, vol. 95, 291-295
- **ENSERINK, M.** Let's talk about sex - and drugs. *Science,* 2005, vol. 308, 1578-1580
- **BOLOUR, S ; G. BRAUNSTEIN.** Testosterone thrapy in women: a review. *International Journal of Impotence Research,* 2005, 1-10

- **BASSON, R.** Female sexual response: the role of drugs in the management of sexual dysfunction. *Obstet. Gynecol.,* 2001, vol. 98 (2), 350-353
- **ROSEN, R.C. ; A. RILEY ; G. WAGNE ; I.H. OSTERLOH ; J. KIRKPATRICK ; A. MISHRA.** The international index of erectile function (IIEF): a multidimensional scale for assessment of erectile dysfunction. *Urology,* June 1997, vol. 49 (6), 822-30
- **CAPPELLERI, J.C. ; R.C. ROSEN.** The sexual health inventory for men (SHIM): a 5-year review of research and clinical experience. *International Journal of Impotense research,* 2005, vol. 17, 307-319
- **DAVIS , S.R. ; S.L. DAVISON ; S. DONATH ; R.J. BELL.** Circulating androgen levels and self-reported sexual function in women. *JAMA,* 2005, vol. 294 (1), 91-96
- **SANDRONI, P.** Aphrodisiacs past and present: a historical review. *Clin Auton Res.,* October 2001, vol. 11 (5), 303-7
- **PHILIPPE RASOANAIVO ; ALAIN PETITJEAN ; SUZANNE RATSIMAMANGA-URVERG ; ALBERT RAKOTO-RATSIMAMANGA.** Medicinal plants used to treat malaria in Madagascar. *Journal of Ethnopharmacology,* 1992, vol. 37, 117-127
- **DAVIDRA H. RAJAONATAHINA.** Médecine traditionnelle, les croyances, la tradition et les maladies transmissibles. *These de Doctorat en Médecine,* 1992, (2693
- **M. DEBRAY.** Contribution à l'inventaire des plantes médicinales de Madagascar: Region Sud-Ouest. *Documents de l'ORSTOM,* 1971, 21
- **PARIS, RR ; DEBRAY, M.** Sur les polypenols (acides-phénols, flavinoides) des feulles de deuc mélicées malagaches: Cedrelopis grevei Baillon et Neobeguea mahafalensis Leroy. *Plantes medicinales et phytothérapie,* 1972, (4), 311-319
- Fiches dendrologiques. Centre de Formation Professionnelle Forestiere' FOFAMPIALA, October 1991
- Apercu sur les plantes medicinales dans le sud de Madagascar. **DIANA RALANTONIRINA.** Etude faite sur les adultes dans le péromètre de la reserve spéciale de Bexa - Mahafaly (These pour l'obtention du doctorat en medicine). Univesite D'Antanarivo, Faculte de Medecine, 1993
- Recueil Botanique de 200 espèces Forestières. Ministère de l'Agriculture et du Développement Rural, 1996
- **PIERRE BOITEAU.** Dictionnaire des Noms Malgaches des Végétaux. 1999, vol. I
- **LEROY, J.-F.** Contributions à l'étude des forets de Madagascar, I. Les acajous de Madagascar (Khaya et Neobeguea). *Journal d'Agronomie Tropicale et de Botanique Appliquée,* 1958, vol. 5, 593-595
- **LEROY, J.-F.** Essais de taxonomie syncrétique, 1. *Etude sur les Meliaceae de Madagascar,* 1976, vol. 16 (2), 167-203
- Fiches dendrologiques. Centre de Formation Professionnelle Forestière' FOFAMPIALA, October 1991
- **D. NAIDOO ; D. A. MULHOLLAND ; M. RANDRIANARIVELOJOSIA ; P. H. COOMBES.** Limonoids and triterpenoids from the seed of Neobegueaea mahafalensis. *Biochemical Systematics and Ecology,* 2003, vol. 31, 1047-1050
- **ANSEL, H.C. ; L.V. ALLEN, JR ; N.G. POPOVICH.** Pharmaceutical Dosage forms and drug delivery systems. Lippincott Williams & Wilkins, 1999
- **YANG, CC ; PORTER, MP ; PENSON, DF.** Comparison of the international index of erectile function erectile domain scores and nocturnal penile tumescence and rigidity measurements: does one predict the other?. *BJU Int.,* July 2006, vol. 98 (1), 105-9
- **VON SOXHLET, FR.** Die gewichtsanalytische Bestimmung des Milchfettes. *Polytechnisches J. (Dingler's),* vol. 232, 461
- Phase Equilibrium of Mixtures Consisting of Molecules with Large Size and Shape Differences (Thermodynamic Modeling of Supercritical Fluid Extraction and Retrograde Condensation, SFE/RC). **MANSOORI, G.A.** GRI Document Number: GRI-88/0360. Gas Research Institute, August 1988, 88
- **MANSOORI, G.A. ; SCHULZ, K. ; MARTINELLI, E.** Bioseparation Using Supercritical Fluid Extraction Retrograde Condensation. *BIO/TECHNOLOGY,* 1988, vol. 6, 393-396
- **RUIZ-JIMENEZ J et al.** *Anal Chim Acta,* 2004, vol. 502, 75
- **RUIZ-JIMENEZ J et al.** *Anal Chim Acta,* 2004, vol. 525, 159
- **CHARMAN SA ; CHARMAN WN ; ROGGE MC ; WILSON TD ; DUTKO FJ ; POUTON CW.** Self-emulsifying drug delivery systems: formulation and biopharmaceutic evaluation of an investigational lipophilic compound. *Pharmaceutical Research,* January 1992, vol. 9 (1), 87-93
- **RANDRIANARIVELOJOSIA, M ; KOTSOS, MP ; MULHOLLAND, DA.** A limonoid from Neobeguea mahafalensis. *Phytochemistry,* 1999, vol. 52, 1141-1143
- **MULHOLLAND, DA ; TAYLOR, DAH.** Limonoid extractives from the genera Capuronanthus, Neobeguea and Quivisanthe. *Phytochemistry,* 1988, vol. 27, 1741-1743
- **GUEX, MATTHIAS ; TAMM, CHRISTOPH.** Selective reactions of the tetranortriterpenes busseins A and B. *Helvetica Chimica Acta,* 1985, vol. 68 (2), 522-33

- **COOMBES, PH ; DA MULHOLLAND ; M RANDRIANARIVELOJOSIA.** Phragmalin limonoids from the Madagascan Meliaceae Neobeguea leandreana. *Journal of Natural Products,* 2003, vol. 66, 735-738
- **GUEX, M ; TAMM, C.** Die busseine C, D, E, F, G, H, J, K, L und M, zehn neue tetratriterpene aus Entandrophramga bussei Harms. *Helvetica chimica Acta,* 1984, vol. 67 (99), 885-901
- **ABDELGAKEIL, SAM ; MAKANTI, M.** Antifeeding activity of limonoids from Khaya psenegalesis (Meliaceae). *J. Appl. Ent.,* 2003, vol. 127, 236-239
- **BOTO, A ; BETABCOR, C ; SUÁREZ, E.** Hypervalent iodine reagents: Synthesis of a steroidal orthoacetate by a radical reaction. *Tetrahedron Letters,* 1994, vol. 35 (37), 6933-6936
- **MULHOLLAND, SA ; PAREL, B ; COOMBES, PH.** The chemistry of the meliaeae and ptaeroxulaceae of southern and eastern africa and Madagascar. *Curent Organic Chemistry,* 2000, vol. 4, 1011-1054
- **NAIDOO, D. ; D.A. MULHOLLAND ; M. RANDRIANARIVELOJOSIA ; P.H. COOMBES.** Limonoids and triterpenoids from the seed of Neobeguea mahafalensis. *Biochemical Systematics and Ecology,* 2003, vol. 31, 1047-1050